# EUROPEAN PATENT SPECIFICATION

(11) **EP 1 140 814 B1**
(45) Date of publication and mention of the grant of the patent: **25.05.2005**
(21) Application number: 99966709.0
(22) Date of filing: 29.12.1999
(51) Int. Cl.: C07C 323/00

(54) **CELL ADHESION-INHIBITING ANTIINFLAMMATORY AND IMMUNE-SUPPRESSIVE COMPOUNDS**
ZELLADHÄSION- UND ENTZÜNDUNGSHEMMENDE IMMUNOSUPPRESSIVE VERBINDUNGEN
COMPOSES ANTI-INFLAMMATOIRES ET IMMUNOSUPPRESSEURS INHIBANT L'ADHESION CELLULAIRE

(30) Priority: 29.12.1998 US 222491
(43) Date of publication of application: 10.10.2001
(73) Proprietor: ABBOTT LABORATORIES, Abbott Park, IL 60064-6050 (US)
(72) Inventor: LINK, James, Evanston, IL 60201 (US); LIU, Gang, Gurnee, IL 60031 (US); PEI, Zhonghua, Libertyville, IL 60048 (US); VON GELDERN, Tom, Richmond, IL 60071 (US); WINN, Martin, Deerfield, IL 60015 (US); XIN, Zhili, Lake Bluff, IL 60044 (US); BOYD, Steven, A., Mundelein, IL 60060 (US); JAE, Hwan-Soo, Glencoe, IL 60022 (US); LYNCH, John, K., Kenosha, WI 53142 (US); ZHU, Gui-Dong, Gurnee, IL 60031 (US); FREEMAN, Jennifer, C., Grayslake, IL 60030 (US); GUNAWARDANA, Indrani, W., Libertyville, IL 60048 (US); STAEGER, Michael, A., Greenfield, WI 53221 (US)
(74) Representative: Helbing, Jörg, Dr. Dipl.-Chem.
(86) International application number: PCT/US1999/031162
(87) International publication number: WO 2000/039081

(56) References cited:
- EP-A- 0 835 867
- EP-A- 0 887 340
- WO-A-98/54207
- GB-A- 2 117 760
- A. FRANKE ET AL: HELVETICA CHIMICA ACTA, vol. 58(1), no. 32, 1975, pages 268-278, XP002016692
- DATABASE CHEMABS [Online] CHEMICAL ABSTRACTS SERVICE, COLUMBUS, OHIO, US; STN, CAPLUS accession no. 1987:196262, XP002159034 -& CHEMICAL ABSTRACTS, vol. 106, no. 23, 8 June 1987 (1987-06-08) Columbus, Ohio, US; abstract no. 196262, XP002159158 & JP 62 012757 A (YOSHITOMO PHARMACEUTICAL INDUSTRIES LTD) 21 January 1987 (1987-01-21)

## Description

### Technical Field

The present invention relates to compounds that are useful for treating inflammatory and immune diseases, to pharmaceutical compositions comprising these compounds, and to the use of said compounds for preparing a medicament for inhibiting inflammation or suppressing immune response in a mammal.

### Background

Inflammation results from a cascade of events that includes vasodilation accompanied by increased vascular permeability and exudation of fluid and plasma proteins. This disruption of vascular integrity precedes or coincides with an infiltration of inflammatory cells. Inflammatory mediators generated at the site of the initial lesion serve to recruit inflammatory cells to the site of injury. These mediators (chemokines such as IL-8. MCP-1, MIP-1, and RANTES, complement fragments and lipid mediators) have chemotactic activity for leukocytes and attract the inflammatory cells to the inflamed lesion. These chemotactic mediators which cause circulating leukocytes to localize at the site of inflammation require the cells to cross the vascular endothelium at a precise location. This leukocyte recruitment is accomplished by a process called cell adhesion.

Cell adhesion occurs through a coordinately regulated series of steps that allow the leukocytes to first adhere to a specific region of the vascular endothelium and then cross the endothelial barrier to migrate to the inflamed tissue (Springer, T.A., 1994, Traffic Signals for Lymphocyte Recirculation and Leukocyte Emigration: The Multistep Paradigm, Cell 76: 301-314; Lawrence, M. B., and Springer, T. A., 1991, Leukocytes' Roll on a Selectin at Physiologic Flow Rates: Distinction from and Prerequisite for Adhesion Through Integrins, Cell.65: 859-873; von Adrian, U., Chambers, J. D., McEnvoy, L.M., Bargatze, R.F., Arfos, K.E, and Butcher, E.C., 1991, Two-Step Model of Leukocyte-Endothelial Cell Interactions in Inflammation, Proc. Natl. Acad. Sci. USA 88: 7538-7542; and Ley, K., Gaehtgens, P., Fennie, C., Singer, M.S., Lasky, L.H. and Rosen, S.D.,1991, Lectin-Like Cell Adhesion Molecule 1 Mediates Rolling in Mesenteric Venules *in vivo*, Blood 77: 2553-2555). These steps are mediated by families of adhesion molecules such as integrins, lg supergene family members, and selectins which are expressed on the surface of the circulating leukocytes and on thc vascular endothelial cells. The first step consists of leukocytes rolling along the vascular endothelial cell lining in the region of inflammation. The rolling step is mediated by an interaction between a leukocyte surface oligosaccharide, such as Sialylated Lewis-X antigen (SLe^{x}), and a selectin molecule expressed on the surface of the endothelial cell in the region of inflammation. The selectin molecule is not normally expressed on the surface of endothelial cells but rather is induced by the action of inflammatory mediators such as TNF-α and interleukin-1. Rolling decreases the velocity of the circulating leukocyte in the region of inflammation and allows the cells .to more firmly adhere to the endothelial cell. The firm adhesion is accomplished by the interaction of integrin molecules that are present on the surface of the rolling leukocytes and their counter-receptors (the Ig superfamily molecules) on the surface of the endothelial cell. The Ig superfamily molecules or CAMs (Cell Adhesion Molecules) are either not expressed or are expressed at low levels on normal vascular endothelial, cells. The CAM's, like the selectins, are induced by the action of inflammatory mediators like TNF-alpha and IL-1. The final event in the adhesion process is the extravasation of leukocytes through the endothelial cell barrier and their migration along a chemotactic gradient to the site of inflammation. This transmigration is mediated by the conversion of the leukocyte integrin from a low avidity state to a high avidity state. The adhesion process relies on the induced expression of selectins and CAM's on the surface of vascular endothelial cells to mediate the rolling and firm adhesion of leukocytes to the vascular endothelium.

The interaction of the intercellular adhesion molecule ICAM-1 (cd54) on endothelial cells with the integrin LFA-1 on leukocytes plays an important role in endothelial-leukocyte contact. Leukocytes bearing high-affinity LFA-1 adhere to endothelial cells through interaction with ICAM-1. initiating the process of extravasation from the vasculature into the surrounding tissues. Thus, an agent which blocks the ICAM-1/LFA-1 interaction suppresses these early steps in the inflammatory response. Consistent with this background, ICAM-1 knockout mice have numerous abnormalities in their inflammatory responses.

The present invention discloses compounds which bind to the intcraction-domain (I-domain) of LFA-1, thus interrupting endothelial cell-leukocyte adhesion by blocking the interaction of LFA-1 with ICAM-1, ICAM-3, and other adhesion molecules. These compounds are useful for the treatment or prophylaxis of diseases in which leukocyte trafficking plays a role, notably acute and chronic inflammatory diseases, autoimmune diseases, tumor metastasis, allograft rejection, and reperfusion injury. The compounds of this invention are diaryl sulfides, which are substituted with a cinnamide moiety. The cinnamide functionality may be placed either ortho- or para- to the linking sulfur atom, although para-substitution is preferable. Appropriate substitution of both aromatic rings is tolerated, and can be used to modulate a variety of biochemical, physicochemical and pharmacokinetic properties. In particular the amide moiety is readily modified; a variety of secondary and tertiary amides are active, and alternatively a heterocyclic ring may be attached at this position. Modifications of this amide functionality are particularly useful in modulating physicochemical and pharmacokinetic properties.

Similar cinnamide compounds are known in the art (e.g. from A. Franke et al., Helv. Chimica Acta, vol. 58(1), no. 32, 263-278 (1975) and GB-A-2117760) but are described as being suitable for different purposes.

Furthermore, heterocyclic compounds suitable for treating autoimmune diseases and inflammatory disorders are known from WO98/54207, EP-A-835867, EP-A-887340 and JP-A-62 012757.

### Summary of The Invention

The present invention provides compounds of formula **I**, below, 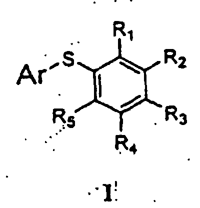 wherein R₁, R₂, R₃, R₄ and R₅ are independently selected from
a. hydrogen,
b. halogen,
c. alkyl,
d. haloalkyl,
e. alkoxy,
f. cyano,
g. nitro,
h. carboxaldehyde, and
with the proviso that at least one of R₁ to R₃ is a "*cis*-cinnamide" or a "*trans*-cinnamide", defined as 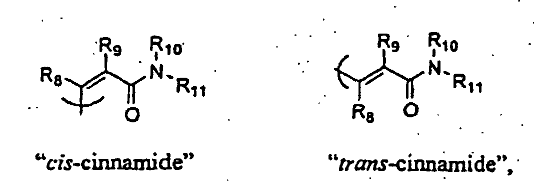 wherein R₈ and R₉ are independently selected from
a. hydrogen,
b. alkyl,
c. carboxy alkyl,
d. alkylaminocarbonyl alkyl, and
e. dialkylaminocarbonyl alkyl,
and R₁₀ and R₁₁ are independently selected from
a. hydrogen,
b. alkyl,
c. cycloalkyl,
d. alkoxycarbonylalkyl,
e. hydroxyalkyl,
f. heterocyclyl representing a 4-, 5-, 6- or 7-membered ring which contains one, two or three heteroatoms independently selected from nitrogen, oxygen and sulfur, which can be fused to one or two rings independently selected from an aryl ring as defined below, a cyclohexane ring, a cyclohexene ring, a cyclopentane ring, a cyclopentene ring or another monocyclic heterocyclic ring,
g. heterocyclylalkyl, where the heterocyclyl is as defined above,
h. heterocyclylamino, where the heterocyclyl is as defined above,
i. a heterocyclyl group as defined in f. above having substituents independently selected from alkyl, halogen, hydroxy and alkoxy;
j. a heterocyclylalkyl group as defined in g. above having substituents independently selected from alkyl, halogen, hydroxy and alkyoxy;
or where NR₁₀R₁₁ is a heterocyclyl or substituted heterocyclyl group where the heterocyclyl group is as defined in f. above and the substituents are independently selected from
1) alkyl,
2) alkoxy,
3) alkoxyalkyl,
4) cycloalkyl,
5) an aryl group being a mono- or bicyclic carbocyclic ring system having one or two aromatic rings which can be fused to a cyclohexane, cyclohexene, cyclopentane or cyclopentene ring,
6) a heterocyclyl group as defined above,
7) heterocyclylcarbonyl, where the heterocyclyl is as defined above,
8) heterocyclylalkylaminocarbonyl, where the heterocyclyl is as defined above,
9) hydroxy,
10) hydroxyalkyl,
11) hydroxyalkoxyalkyl,
12) carboxy,
13) carboxyalkyl,
14) carboxycarbonyl,
15) carboxaldehyde,
16) alkoxycarbonyl,
17) arylalkoxycarbonyl, where the aryl is as defined above,
18) aminoalkyl,
19) aminoalkanoyl,
20) carboxamido,
21) alkoxycarbonylalkyl,
22) carboxamidoalkyl,
23) cyano,
24) tetrazolyl,
25) substituted tetrazolyl,
26) alkanoyl,
27) hydroxyalkanoyl,
28) alkanoyloxy,
29) alkanoylamino,
30) alkanoyloxyalkyl,
31) alkanoylaminoalkyl,
32) sulfonate,
33) alkylsulfonyl,
34) alkysulfonylaminocarbonyl,
35) arylsulfonylaminocarbonyl, where the aryl is as defined above, and
36) heterocyclylsulfonylaminocarbonyl, where the heterocyclyl group is as defined above,
and wherein Ar is a substituted aryl or substituted heteroaryl group, which has one or two aromatic rings and which can be fused to a cyclohexane, cyclohexene, cyclopentane or cyclopentene ring, where substitutions are independently selected from
a. hydrogen,
b. halogen,
c. alkyl,
d. an aryl group as defined above,
e. haloalkyl,
f. hydroxy,
g. alkoxy,
h. alkoxyalkyl,
i. alkoxycarbonyl,
j. alkoxyalkoxy,
k. hydroxyalkyl,
l. aminoalkyl,
m. aminocarbonyl,
n. alkyl(alkoxycarbonylalkyl)aminoalkyl,
o. a heterocyclyl group as defined above,
p. a heterocyclylalkyl group as defined above,
q. a heterocyclylalkyl group as defined above having substituents independently selected from alkyl, halogen, hydroxy and alkoxy;
r. carboxaldehyde,
s. carboxaldehyde hydrazone,
t. carboxamide,
u. alkoxycarbonylalkyl,
v. carboxy,
w. carboxyalkyl,
x. hydroxycarbonylalkyl(carboxyalkyl),
y. hydroxyalkylaminocarbonyl,
z. cyano,
aa. amino,
bb. heterocyclylalkylamino, where the heterocyclyl is as defined above,
cc. heterocyclylaminocarbonyl, where the heterocyclyl is as defined above, and
dd. "*trans*-cinnamide",
provided that
(i) if Ar is phenyl and R₁, R₂, R₄, and R₅ are simultaneously hydrogen, then R₃ is not a *cis*-cinnamide or trans-cinnamide where R₈ is methyl, R₉ is hydrogen and R₁₀ and R₁₁ are simultaneously -C₂H₅, and
(ii) if Ar is pyridyl or C₁₋₃ alkyl substituted pyridyl, and R₁, R₂, R₄, and R₅ are simultaneously hydrogen, then R₃ is not a *cis*-cinnamide or *trans*-cinnamide where R₈ is hydrogen, R₉ is hydrogen or C₁₋₃ alkyl, and R₁₀ and R₁₁ are independently hydrogen, C₁₋₄ alkyl, or C₃₋₆ cycloalkyl,
or a pharmaceutically acceptable salt thereof.

Additionally provided is the use of a compound of formula I for preparing a medicament for treatment or prophylaxis or inhibition of inflammation or suppression of immune response.
Still further provided are pharmaceutical compositions containing compounds of formula I.
Still further provided is a process for preparing compounds of formula I, which process comprises activating the acid group in a compound of the formula 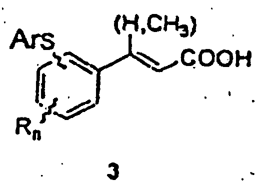 and subsequently reacting it with a primary or secondary amine of the formula R₁₀R₁₁, where all the variables are as defined above.

### Detailed Description

The term "alkanoyl" as used herein refers to an alkyl group attached to the parent molecular group through a carbonyl group.

The term "alkanoylamino" as used herein refers to an alkanoyl group attached to the parent molecular group though an amino group.

The term "alkanoylaminoalkyl" as used herein refers to an alkanoylamino group attached to the parent molecular group through an alkyl group.

The term " alkanoyloxy" as used herein refers to an alkanoyl group attached to the parent molecular group through an oxygen radical.

The term "alkanoyloxyalkyl" as used herein refers to an atkanoyloxy group attached to the parent molecular group through an alkyl group.

The term "alkoxy" as used herein refers to an alkyl group attached to the parent molecular group through an oxygen atom.

The term "alkoxyalkoxy" as used herein refers to an alkoxy group attached to the parent molecular group through an alkoxy group.

The term "alkoxyalkyl" as used herein refers to an alkoxy group attached to the parent molecular group through an alkyl group.

The term "alkoxycarbonyl" as used herein refers to an alkoxy group attached to the parent molecular group through a carbonyl group.

The term "alkoxycarbonylalkyl" as used herein refers to an alkoxycarbonyl group attached to the parent molecular group through an alkyl group.

The term "alkyl" as used herein refers to a saturated straight or branched chain group of 1-10 carbon atoms derived from an alkane by the removal of one hydrogen atom.

The term "alkyl(alkoxycarbonylalkyl)amino" as used herein refers to an amino group substituted with one alkyl group and one alkoxycarbonylalkyl group.

The term "alkyl(alkoxycarbonylalkyl)aminoalkyl" as used herein refers to an alkyl(alkoxycarbonylalkyl)amino group attached to the parent molecular group through an alkyl group.

The term "alkylene" as used herein refers to a divalent group of 1-10 carbon atoms derived from a straight or branched chain alkane by the removal of two hydrogen atoms.

The term "alkylsulfonyl" as used herein refers to an alkyl radical attached to the parent molecular group through an -SO₂- group.

The term "alkylsulfonylaminocarbonyl" as used herein refers to an alkylsulfonyl group attached to the parent molecular group through an aminocarbonyl group.

The term "amino" as used herein refers to a radical of the form -NR₁₈R₁₉, or to to a radical of the form -NR₁₈-, where R₁₈ and R₁₉ are independently selected from hydrogen, alkyl or cycloalkyl.

The term "aminoalkanoyl" as used herein refers to to an amino group attached to the parent molecular group through an alkanoyl group.

The term "aminoalkyl" as used herein refers to an amino group attached to the parent molecular group through an alkyl group.

The term "aminocarbonyl" as used herein refers to an amino group attached to the parent molecular group through a carbonyl group.

The term "aryl" as used herein refers to a mono- or bicyclic carbocyclic ring system having one or two aromatic rings. The aryl group can also be fused to a cyclohexane, cyclohexene, cyclopentane or cyclopentene ring. The aryl groups of this invention can be optionally substituted with alkyl, halogen, hydroxy, or alkoxy substituents.

The term "arylalkoxy" as used herein refers to an aryl group attached to the parent molecular group through an alkoxy group.

The term "arylalkoxycarbonyl" as used herein refers to an arylalkoxy group attached to the parent molecular group through a carbonyl group.

The term "arylsulfonyl" as used herein refers to an aryl radical attached to the parent molecular group through an -SO₂- group.

The term "arylsulfonylaminocarbonyl" as used herein refers to an arylsulfonyl group attached to the parent molecular group through an aminocarbonyl group.

The term "carboxaldehyde" as used herein refers to the radical -CHO.

The term "carboxaldehyde hydrazone" as used herein refers to the radical -CH=N-NR₂₀R₂₁, where R₂₀ and R₂₁ are independently selected from hydrogen, alkyl or cycloalkyl.

The terms "carboxamide" or "carboxamido" as used herein refer to an amino group attached to the parent molecular group through a carbonyl group.

The term "carboxamidoalkyl" as used herein refers to a carboxamido group attached to the parent molecular group through an alkyl group.

The term "carboxy" as used herein refers to the radical -COOH.

The term "carboxyalkyl" as used herein refers to a carboxy group attached to the parent molecular group through a alkyl group.

The term "carboxycarbonyl" as used herein refers to a carboxy group attached to the parent molecular group through a carbonyl group.

The term "cyano" as used herein refers to the radical -CN.

The term "cycloalkyl" as used herein refers to a monovalent saturated cyclic or bicyclic hydrocarbon group of 3-12 carbons derived from a cycloalkane by the removal of a single hydrogen atom. Cycloalkyl groups may be optionally substituted with alkyl, alkoxy, halo, or hydroxy substituents.

The terms "halo" or "halogen" as used herein refers to F, Cl, Br, or I.

The term "haloalkyl" as used herein refers to an alkyl group substituted with one or more halogen atoms.

The terms "heterocycle" or "heterocyclyl" represent a 4-, 5-, 6- or 7-membered ring containing one, two or three heteroatoms independently selected from the group consisting of nitrogen, oxygen and sulfur. The 4- and 5-membered rings have zero to two double bonds and the 6- and 7-membered rings have zero to three double bonds. The term "heterocycle" or "heterocyclic" as used herein additionally refers to bicyclic, tricyclic and tetracyclic groups in which any of the above heterocyclic rings is fused to one or two rings independently selected from an aryl ring, a cyclohexane ring, a cyclohexene ring, a cyclopentane ring, a cyclopentene ring or another monocyclic heterocyclic ring. Heterocycles include acridinyl, benzimidazolyl, benzofuryl, benzothiazolyl, benzothienyl, benzoxazolyl, biotinyl, cinnolinyl, dihydrofuryl, dihydroindolyl, dihydropyranyl, dihydrothienyl, dithiazolyl, furyl, homopiperidinyl, imidazolidinyl, imidazolinyl, imidazolyl, indolyl, isoquinolyl, isothiazolidinyl, isothiazolyl, isoxazolidinyl, isoxazolyl, morpholinyl, oxadiazolyl, oxazolidinyl, oxazolyl, piperazinyl, piperidinyl, pyranyl, pyrazolidinyl, pyrazinyl, pyrazolyl, pyrazolinyl, pyridazinyl, pyridyl, pyrimidinyl, pyrimidyl, pyrrolidinyl, pyrrolidin-2-onyl, pyrrolinyl, pyrrolyl, quinolinyl, quinoxaloyl, tetrahydrofuryl, tetrahydroisoquinolyl, tetrahydroquinolyl, tetrazolyl, thiadiazolyl, thiazolidinyl, thiazolyl, thienyl, thiomorpholinyl, triazolyl, and the like.

Heterocyclics also include bridged bicyclic groups where a monocyclic heterocyclic group is bridged by an alkylene group such as 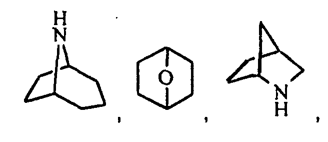 and the like.

Heterocyclics also include compounds of the formula 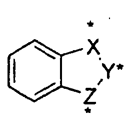 where X* and Z* are independently selected from -CH₂-, -CH₂NH-, -CH₂O-, -NH- and -O-, with the proviso that at least one of X* and Z* is not -CH₂-, and Y* is selected from -C(O)- and -(C(R")₂)ᵥ -, where R" is hydrogen or alkyl of one to four carbons, and v is 1-3. These heterocycles include 1,3-benzodioxolyl, 1,4-benzodioxanyl, 1,3-benzimidazol-2-one and the like. The heterocycle groups of this invention can be optionally substituted with alkyl, halogen, hydroxy or alkoxy substituents.

The term "heterocyclylalkyl" as used herein refers to an heterocyclic group attached to the parent molecular group through an alkyl group.

The term "heterocyclylalkylamino" as used herein refers to an heterocyclylalkyl group attached to the parent molecular group through an amino group.

The term "heterocyclylalkylaminocarbonyl" as used herein refers to a heterocyclylalkylamino group attached to the parent molecular group through a carbonyl group.

The term "heterocyclylamino" as used herein refers to a heterocyclyl group attached to the parent molecular group through a amino group.

The term "heterocyclylcarbonyl" as used herein refers to a heterocyclyl group attached to the parent molecular group through a carbonyl group.

The term "heterocyclylsulfonyl" as used herein refers to a heterocyclyl radical attached to the parent molecular group through an -SO₂- group.

The term "heterocyclylsulfonylaminocarbonyl" as used herein refers to a heterocyclylsulfonyl group attached to the parent molecular group through an aminocarbonyl group.

The term "hydroxyalkanoyl" as used herein refers to an hydroxy radical attached to the parent molecular group through an alkanoyl group.

The term "hydroxyalkoxy" as used herein refers to an hydroxy radical attached to the parent molecular group through an alkoxy group.

The term "hydroxyalkoxyalkyl" as used herein refers to an hydroxyalkoxy group attached to the parent molecular group through an alkyl group.

The term "hydroxyalkyl" as used herein refers to an hydroxy radical attached to the parent molecular group through an alkyl group.

The term "hydroxyalkylaminocarbonyl" as used herein refers to an hydroxyalkyl group attached to the parent molecular group through an aminocarbonyl group.

The term "perfluoroalkyl" as used herein refers to an alkyl group in which all of the hydrogen atoms have been replaced by fluoride atoms.

The term "phenyl" as used herein refers to a monocyclic carbocyclic ring system having one aromatic ring. The phenyl group can also be fused to a cyclohexane or cyclopentane ring. The phenyl groups of this invention can be optionally substituted with alkyl, halogen, hydroxy or alkoxy substituents.

The term "pharmaceutically-acceptable prodrugs" as used herein represents those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention.

The term "prodrug," as used herein, represents compounds which are rapidly transformed *in vivo* to the parent compound of the above formula, for example, by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

The term "sulfonate" as used herein refers to the radical -SO₃H

The term "tetrazole" or "tetrazolyl" as used herein refers to the heterocyclic radical -CN₄H..

The term "thioalkoxy" as used herein refers to an alkyl group attached to the parent molecular group through a sulfur atom.

Compounds of the present invention can exist as stereoisomers wherein asymmetric or chiral centers are present. These compounds are designated by the symbols "R" or "S," depending on the configuration of substituents around the chiral carbon atom. The present invention contemplates various stereoisomers and mixtures thereof. Stereoisomers include enantiomers and diastereomers, and mixtures of enantiomers or diastereomers are designated (±). Individual stereoisomers of compounds of the present invention can be prepared synthetically from commercially available starting materials which contain asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and liberation of the optically pure product from the auxiliary, (2) salt formation employing an optically active resolving agent, or (3) direct separation of the mixture of optical enantiomers on chiral chromatographic columns.

Geometric isomers can also exist in the compounds of the present invention. The present invention contemplates the various geometric isomers and mixtures thereof resulting from the arrangement of substituents around a carbon-carbon double bond or arrangement of substituents around a carbocyclic ring. Substituents around a carbon-carbon double bond are designated as being in the Z or E configuration wherein the term "Z" represents substituents on the same side of the carbon-carbon double bond and the term "E" represents substituents on opposite sides of the carbon-carbon double bond. The arrangement of substituents around a carbocyclic ring are designated as cis or trans wherein the term "cis" represents substituents on the same side of the plane of the ring and the term "trans" represents substituents on opposite sides of the plane of the ring. Mixtures of compounds wherein the substituents are disposed on both the same and opposite sides of plane of the ring are designated cis/trans.

As is apparent from the foregoing descriptions, the compounds of Formula 1 are useful in a variety of forms, i.e., with various substitutions as identified. Examples of particularly desirable compounds are quite diverse, and many are mentioned herein. Included are compounds in which R₁ is a "cis-cinnamide" or a "trans-cinnamide", and R₃ is hydrogen; or where R₃ is a "cis-cinnamide" or a "trans-cinnamide", and R₁ is hydrogen, or R₁, R₂, and R₄ are each independently hydrogen or alkyl, and R₅ is halogen, haloalkyl or nitro. Further preferred compounds include those as above wherein R₁₀ and R₁₁ are each independently hydrogen, alkyl, cycloalkyl, alkoxycarbonylaalkyl, hydroxyalkyl, or heterocyclylalkyl, or where NR₁₀R₁₁ is heterocyclyl or substituted heterocyclyl, and where **Ar** is aryl, substituted aryl, heteroaryl, or substituted heteroaryl.

Compounds of the present invention include:
(2,4-Dichlorophenyl)[2-(*E*-((6-hydroxyhexylamino)carbonyl)ethenyl)phenyl]sulfide;
(2,4-Dichlorophenyl)[2-(*E*-((3-(1-imidazolyl)propylamino)carbonyl)ethenyl)phenyl]sulfide;
(2,4-Dichlorophenyl)[2-chloro-4-(*E*-((2-hydroxyethylamino)carbonyl)ethenyl)phenyl]sulfide;
(2,4-Dichlorophenyl)[2-chloro-4-(*E*-((6hydroxyhexylamino)carbonyl)ethenyl)phenyl]sulfide;
(2,4-Dichlorophenyl)[2-chloro-4-(*E*-((bis-(2-hydroxyethyl)amino)carbonyl)ethenyl)phenyl]sulfide;
(2,4-Dichlorophenyl)[2-chloro-4-(*E*-((3-(1-pyrrolidin-2-only)propylamino)carbonyl) ethenyl)phenyl]sulfide;
(2,4-Dichlorophenyl)[2-chloro-4-(*E*-((1-morpholinyl)carbonyl)ethenyl)phenyl]sulfide;
(2,4-Dichlorophenyl)[2-chloro-4-(*E*-((4-methylpiperazin-1-yl)carbonyl)ethenyl)phenyl]sulfide;
(2,4-Dichlorophenyl)[2-chloro-4-(*E*-((4-acetylpiperazin-1-yl)carbonyl)ethenyl)phenyl]sulfide;
(2,4-Dichlorophenyl)[2-chloro-4-(*E*-((4-(2-pyridyl)piperazin-1-yl)carbonyl) ethenyl)phenyl]sulfide;
(2-(Hydroxymethyl)phenyl)[2-chloro-4-(*E*-((1-morpholinyl)carbonyl) ethenyl)phenyl]sulfide;
(2-Bromophenyl)[2-chloro-4-(*E*-((1-morpholinyl)carbonyl)ethenyl)phenyl]sulfide;
(2,4-Dichlorophenyl)[2-chloro-4-(*E*-((4-(2-hydroxyethyl)piperazin-1-yl)carbonyl) ethenyl)phenyl]sulfide;
(2,4-Dichlorophenyl)[2-chloro-4-(*E*-((4-(2-hydroxyethoxyethyl)piperazin-1-yl)carbonyl)ethenyl)phenyl]sulfide;
(2-Bromophenyl)[2-chloro-4-(*E*-((3-(hydroxymethyl)piperidin-1-yl)carbonyl) ethenyl)phenyl]sulfide;
(2-Bromophenyl)[2-chloro-4-(*E*-((2-(hydroxymethyl)piperidin-1-yl)carbonyl) ethenyl)phenyl]sulfide;
(2-Bromophenyl)[2-chloro-4-(*E*-((3-acetamidopyrrolidin-1-yl)carbonyl)ethenyl)phenyl]sulfide;
(2-Bromophenyl)[2-chloro-4-(*E*-((4-hydroxypiperidin-1-yl)carbonyl)ethenyl)phenyl]sulfide;
(2-Bromophenyl)[2-chloro-4-(*E*-((piperidin-1-yl)carbonyl)ethenyl)phenyl]sulfide;
(2,4-Dichlorophenyl)[2-chloro-4-(*E*-((3-carboxypiperidin-1-yl)carbonyl)ethenyl)phenyl]sulfide;
(2,4-Dichlorophenyl)[2-chloro-4-(*E*-((4-carboxypiperidin-1-yl)carbonyl)ethenyl)phenyl]sulfide;
(2-Bromophenyl)[2-chloro-4-(*E*-((4-acetylhomopiperazin-1-yl)carbonyl)ethenyl)phenyl]sulfide;
(2-Bromophenyl)[2-chloro-4-(*E*-((thiomorpholin-1-yl)carbonyl)ethenyl)phenyl]sulfide;
(2-Bromophenyl)[2-chloro-4-(*E*-((4-(1-benzimidazol-2-only)piperidin-1-yl)carbonyl) ethenyl)phenyl]sulfide;
(2-Bromophenyl)[2-chloro-4-(*E*-((2-tetrahydroisoquinolinyl)carbonyl)ethenyl)phenyl]sulfide;
(2-Methylphenyl)[2-trifluoromethyl-4-(*E*-((4-acetylpiperazin-1-yl)carbonyl) ethenyl)phenyl]sulfide;
(2-Methylphenyl)[2-trifluoromethyl-4-(*E*-((1-morpholinyl)carbonyl)ethenyl)phenyl]sulfide;
(2-Methylphenyl)[2-trifluoromethyl-4-(*E*-((2-(1-morpholinyl)ethylamino)carbonyl) ethenyl)phenyl]sulfide;
(2-Methylphenyl)[2-trifluoromethyl-4-(*E*-((4-phenylpiperazin-1-yl)carbonyl) ethenyl)phenyl]sulfide;
(2-Methylphenyl)[2-trifluoromethyl-4-(*E*-((3-(1-pyrrolidin-2-onyl)propylamino)carbonyl)ethenyl)phenyl]sulfide;
(2-Methylphenyl)[2-trifluoromethyl-4-(*E*-((cyclopropylamino)carbonyl)ethenyl)phenyl]sulfide;
(2,4-Dichlorophenyl)[2-nitro-4-(*E*-((4-acetylpiperazin-1-yl)carbonyl)ethenyl)phenyl]sulfide;
(2,4-Dichlorophenyl)[2-nitro-4-(*E*-((3-(1-pyrrolidin-2-only)propylamino)carbonyl) ethenyl)phenyl]sulfide;
(2,3-Dichlorophenyl)[2-nitro-4-(*E*-((4-acetylpiperazin-1-yl)carbonyl)ethenyl)phenyl]sulfide;
(4-Bromophenyl)[2-nitro-4-(*E*-((4-acetylpiperazin-1-yl)carbonyl)ethenyl)phenyl]sulfide;
(4-Methylphenyl)[2-nitro-4-(*E*-((4-acetylpiperazin-1-yl)carbonyl)ethenyl)phenyl]sulfide;
(2,4-Dichlorophenyl)[2-nitro-4-(*E*-((4-(*tert*-butoxycarbonyl)piperazin-1-yl)carbonyl) ethenyl)phenyl]sulfide;
(2,4-Dichlorophenyl)[2-nitro-4-(*E*-((4-(2-furoylcarbonyl)piperazin-1-yl)carbonyl) ethenyl)phenyl]sulfide;
(2,4-Dichlorophenyl)[2-nitro-4-(*E*-((4-(methanesulfonyl)piperazin-1-yl)carbonyl) ethenyl)phenyl]sulfide;
(2,4-Dichlorophenyl)[2-nitro-4-(*E*-((4-(diethylaminocarbonylmethyl)piperazin-1-yl)carbonyl)ethenyl)phenyl]sulfide;
(2,4-Dichlorophenyl)[2-nitro-4-(*E*-((4-(diethylaminocarbonyl)piperazin-1-yl)carbonyl)ethenyl)phenyl]sulfide;
(2,4-Dichlorophenyl)[2-nitro-4-(*E*-((4-(*tert*-butoxycarbonylmethyl)piperazin-1-yl)carbonyl)ethenyl)phenyl]sulfide;
(2,4-Dichlorophenyl)[2-nitro-4-(*E*-((4-(carboxycarbonyl)piperazin-1-yl)carbonyl) ethenyl)phenyl]sulfide;
(2,4-Dichlorophenyl)[2-nitro-4-(*E*-((4-(carboxymethyl)piperazin-1-yl)carbonyl) ethenyl)phenyl]sulfide;
(2-Methylphenyl)[2-nitro-4-(*E*-((4-acetylpiperazin-1-yl)carbonyl)ethenyl)phenyl]sulfide;
(2-Chlorophenyl)[2-nitro-4-(*E*-((4-acetylpiperazin-1-yl)carbonyl)ethenyl)phenyl]sulfide;
(2-Aminophenyl)[2-nitro-4-(*E*-((4-acetylpiperazin-1-yl)carbonyl)ethenyl)phenyl]sulfide;
(2-Hydroxymethylphenyl)[2-nitro-4-(*E*-((4-acetylpiperazin-1-yl)carbonyl)ethenyl)phenyl]sulfide;
(2-Ethylphenyl)[2-nitro-4-(*E*-((4-acetylpiperazin-1-yl)carbonyl)ethenyl)phenyl]sulfide;
(2-*iso*-Propylphenyl)[2-nitro-4-(*E*-((4-acetylpiperazin-1-yl)carbonyl)ethenyl)phenyl]sulfide;
(2-*tert*-Butylphenyl)[2-nitro-4-(*E*-((4-acetylpiperazin-1-yl)carbonyl)ethenyl)phenyl]sulfide;
(2-Chlorophenyl)[2-chloro-4-(*E*-((4-acetylpiperazin-1-yl)carbonyl))2-propenyl)phenyl]sulfide;
(2-(1-Morpholinylmethyl)phenyl)[2-chloro-4-(*E*-((1-morpholinyl)carbonyl)ethenyl)phenyl]sulfide;
(2-(4-(1,3-Benzodioxolyl-5-methyl)piperazin-1-ylmethyl)phenyl)[2-chloro-4-(*E*-((1-morpholinyl)carbonyl)ethenyl)phenyl]sulfide;
(2-(4-(*iso*-Propylaminocarbonylmethyl)piperazin-1-ylmethyl)phenyl)[2-chloro-4-(*E*-((1-morpholinyl)carbonyl)ethenyl)phenyl]sulfide;
(2-((*N*-Ethoxycarbonylmethyl-*N*-methyl)aminomethyl)phenyl)[2-chloro-4-(*E*-((1-morpholinyl)carbonyl)ethenyl)phenyl]sulfide;
(2-Formylphenyl)[2-chloro-4-(*E*-((1-morpholinyl)carbonyl)ethenyl)phenyl]sulfide;
(2-(4-Formylpiperazin-1-ylmethyl)phenyl)[2-chloro-4-(*E*-((1-morpholinyl)carbonyl) ethenyl)phenyl]sulfide;
(2-(*E*-((1-Morpholinyl)carbonyl)ethenyl)phenyl)[2-chloro-4-(*E*-((1-morpholinyl) carbonyl)ethenyl)phenyl]sulfide;
(2-Formylphenyl)[2-nitro-4-(*E*-((4-acetylpiperazin-1-yl)carbonyl)ethenyl)phenyl]sulfide;
(2-Formylphenyl)[2-chloro-4-(*E*-((1-morpholinyl)carbonyl)ethenyl)phenyl]sulfide, N,N-dimethyl hydrazone;
(2-((3-(1-Morpholinyl)propyl)-1-amino)phenyl)[2-chloro-4-(*E*-((1-morpholinyl)carbonyl)ethenyl)phenyl]sulfide;
(2,4-Dichlorophenyl)[2-bromo-4-(*E*-((3-(1-pyrrolidin-2-only)propylamino)carbonyl) ethenyl)phenyl]sulfide;
(2,4-Dichlorophenyl)[2-formyl-4-(*E*-((1-morpholinyl)carbonyl)ethenyl)phenyl]sulfide;
(2-Chloro-6-formylphenyl)[2-chloro-4-(*E*-((4-acetylpiperazin-1-yl)carbonyl)ethenyl)phenyl]sulfide;
(2-Cyanophenyl)[2-chloro-4-(*E*-((4-acetylpiperazin-1-yl)carbonyl) ethenyl)phenyl]sulfide;
(2-Isopropylphenyl)[2-cyano-4-(*E*-((morpholin-1-yl)carbonyl) ethenyl)phenyl]sulfide;
(2-Bromophenyl)[2-nitro-4-(*E*-((4-acetylpiperazin-1-yl)carbonyl) ethenyl)phenyl]sulfide;
(2-(Pyrrolidin-1-yl)phenyl)[2-chloro-4-(*E*-((morpholin-1-yl)carbonyl) ethenyl)phenyl]sulfide;
(2-Methoxyphenyl)-[2-chloro-4(*E*-[(morpholin-1-yl)carbonyl]ethenyl)phenyl]sulfide;
(2-Isopropylphenyl)[2-nitro-4-(*E*-((3-carbomethoxypiperazin-1-yl)carbonyl) ethenyl)phenyl]sulfide;
(2-Methylphenyl)[2-nitro-4-(*E*-((3-carboxamido-4-carbobenzoxypiperazin-1-yl)carbonyl)ethenyl)phenyl]sulfide;
(2-Isopropylphenyl)[2-nitro-4-(*E*-((2-carbomethoxy-4-*tert*-butoxycarbonylpiperazin-1-yl)carbonyl)ethenyl)phenyl]sulfide;
(2-Isopropylphenyl)[2-nitro-4-(*E*-((2-carboxy-4-*tert*-butoxycarbonylpiperazin-1-yl)carbonyl)ethenyl)phenyl]sulfide;
(2-Isopropylphenyl)[2-trifluoromethyl-4-(*E*-((4-acetylpiperazin-1-yl)carbonyl) ethenyl)phenyl]sulfide;
(2-Isopropylphenyl)[2-trifluoromethyl-4-(*E*-((morpholin-1-yl)carbonyl) ethenyl)phenyl]sulfide;
(2-Isopropylphenyl)[2-trifluoromethyl-4-(*E*-((3-(pyrrolidin-2-on-1-yl)prop-1-ylamino)carbonyl)ethenyl)phenyl]sulfide;
(2-Isopropylphenyl)[2-trifluoromethyl-4-(*E*-((cyclobutylamino)carbonyl) ethenyl)phenyl]sulfide;
(2-Isopropylphenyl)[2-trifluoromethyl-4-(*E*-((cyclopentylamino)carbonyl) ethenyl)phenyl]sulfide;
(2-Isopropylphenyl)[2-trifluoromethyl-4-(*E*-((5-hydroxypent-1-ylamino)carbonyl) ethenyl)phenyl]sulfide;
(2-Isopropylphenyl)[2-nitro-4-(*E*-((3-carbomethoxy-4-acetylpiperazin-1-yl)carbonyl)ethenyl)phenyl]sulfide;
(2-Biphenyl)[2-chloro-4-(*E*-((morpholin-1-yl)carbonyl) ethenyl)phenyl]sulfide;
(3,4-Dimethylphenyl)[2-nitro-4-(*E*-((4-acetylpiperazin-1-yl)carbonyl)ethenyl)phenyl]sulfide;
(2-Bromophenyl)[2-trifluoromethyl-4-(*E*-((4-acetylpiperazin-1-yl)carbonyl) ethenyl)phenyl]sulfide;
(5-Indolyl)[2-chloro-4-(*E*-((4-acetylpiperazin-1-yl)carbonyl) ethenyl)phenyl]sulfide;
(5-Benzodioxolyl)[2-chloro-4-(*E*-((4-acetylpiperazin-1-yl)carbonyl) ethenyl)phenyl]sulfide;
(2-Isopropylphenyl)[2-nitro-4-(*E*-((2-carbomethoxypiperazin-1-yl)carbonyl)ethenyl)phenyl]sulfide;
(2,3-Dimethoxyphenyl)-[2-chloro-4(*E*-[(morpholin-1-yl)carbonyl]ethenyl)phenyl]sulfide;
(2-Fluorophenyl)[2-nitro-4-(*E*-((4-acetylpiperazin-1-yl)carbonyl)ethenyl)phenyl]sulfide;
(2-Bromophenyl)[2-trifluoromethyl-4-(*E*-((4-(*tert*-butoxycarbonyl)piperazin-1-yl)carbonyl)ethenyl)phenyl]sulfide;
(2-(Pyrrolidin-1-yl)phenyl)[2-trifluoromethyl-4-(*E*-((4-(*tert*-butoxycarbonyl)piperazin-1-yl)carbonyl)ethenyl)phenyl]sulfide;
(3-Carboxamidophenyl)[2-nitro-4-(*E*-((4-acetylpiperazin-1-yl)carbonyl) ethenyl)phenyl]sulfide;
(3-(Hydroxymethyl)phenyl)[2-nitro-4-(*E*-((4-acetylpiperazin-1-yl)carbonyl) ethenyl)phenyl]sulfide;
Phenyl[2-trifluoromethyl-4-(*E*-((4-(*tert*-butoxycarbonyl)piperazin-1-yl)carbonyl)ethenyl)phenyl]sulfide;
(2-Isopropylphenyl)[2-trifluoromethyl-4-(*E*-((2-carbomethoxy-4-(*tert*-butoxycarbonyl)piperazin-1-yl)carbonyl)ethenyl)phenyl]sulfide;
(2-Isopropylphenyl)[2-nitro-4-(*E*-((3-(pyridine-4-methylaminocarbonyl)-4-*tert*-butoxycarbonylpiperazin-1-yl)carbonyl)ethenyl)phenyl]sulfide;
(2-Ethoxyphenyl)-[2-chloro-4(*E*-[(morpholin-1-yl)carbonyl]ethenyl)phenyl]sulfide;
(2-Methoxyphenyl)[2-nitro-4-(*E*-((4-acetylpiperazin-1-yl)carbonyl)ethenyl)phenyl]sulfide;
(2-(Azetidin-1-yl)phenyl)[2-trifluoromethyl-4-(*E*-((4-(*tert*-butoxycarbonyl)piperazin-1-yl)carbonyl)ethenyl)phenyl]sulfide;
(2-(Piperidin-1-yl)phenyl)[2-trifluoromethyl-4-(*E*-((4-(*tert*-butoxycarbonyl)piperazin-1-yl)carbonyl)ethenyl)phenyl]sulfide;
(3-Chloro-2-formylphenyl)[2-chloro-4-(*E*-((4-acetylpiperazin-1-yl)carbonyl) ethenyl)phenyl]sulfide;
(2-Trifluoromethylphenyl)[2-trifluoromethyl-4-(*E*-((4-acetylpiperazin-1-yl)carbonyl) ethenyl)phenyl]sulfide;
(3-Bromophenyl)[2-trifluoromethyl-4-(*E*-((4-acetylpiperazin-1-yl)carbonyl) ethenyl)phenyl]sulfide;
(3,5-Dimethylphenyl)[2-trifluoromethyl-4-(*E*-((4-acetylpiperazin-1-yl)carbonyl) ethenyl)phenyl]sulfide;
(2-Isopropylphenyl)[2-nitro-4-(*E*-((3-dimethylaminocarbonyl-4-(pyridine-4-carbonyl)piperazin-1-yl)carbonyl)ethenyl)phenyl]sulfide;
(2-Isopropylphenyl)[2-nitro-4-(*E*-((3-dimethylaminocarbonyl-4-carbomethoxypiperazin-1-yl)carbonyl)ethenyl)phenyl]sulfide;
(2-Isopropylphenyl)[2-nitro-4-(*E*-((3-dimethylaminocarbonyl-4-acetylpiperazin-1-yl)carbonyl)ethenyl)phenyl]sulfide;
(2-Isopropylphenyl)[2-nitro-4-(*E*-((3-(1-morpholinocarbonyl)-4-*tert*-butoxycarbonylpiperazin-1-yl)carbonyl)ethenyl)phenyl]sulfide;
(2-Isopropylphenyl)[2-nitro-4-(*E*-((3-(pyridine-4-methylaminocarbonyl)piperazin-1-yl)carbonyl)ethenyl)phenyl]sulfide;
(2-Isopropylphenyl)[2-nitro-4-(*E*-(((3-dimethylaminacarbonyl)piperazin-1-yl)carbonyl)ethenyl)phenyl]sulfide;
(2-Isopropylphenyl)[2-nitro-4-(*E*-((3-(benzylaminocarbonyl)-4-*tert*-butoxycarbonylpiperazin-1-yl)carbonyl)ethenyl)phenyl]sulfide;
(2-Isopropylphenyl)[2-nitro-4-(*E*-((3-(dimethylaminocarbonyl)-4-*tert*-butoxycarbonylpiperazin-1-yl)carbonyl)ethenyl)phenyl]sulfide;
(2-Bromophenyl)[2-chloro-4-(*E*-((3-(5*S*-hydroxymethyl-pyrrolidin-2-on-1-yl)prop-1-ylamino)carbonyl)ethenyl)phenyl]sulfide;
(2-Bromophenyl)[2-chloro-4-(*E*-((3-(pyrrolidin-2-on-1-yl)prop-1-ylamino)carbonyl) ethenyl)phenyl]sulfide;
(2-Bromophenyl)[2-chloro-4-(*E*-(N-methyl-N-(3-(pyrrolidin-2-on-1-yl)prop-1-yl)amino)carbonyl)ethenyl)phenyl]sulfide;
(2-[2-Methoxy]ethoxyphenyl)-[2-chloro-4(*E*-[(morpholin-1-yl)carbonyl]ethenyl)phenyl]sulfide;
(2-Isopropylphenyl)[2-nitra-4-(*E*-((3-(morpholinocarbonyl)piperazin-1-yl)carbonyl)ethenyl)phenyl]sulfide;
(2-Isopropylphenyl)[2-nitro-4-(*E*-((4-*tert-*butoxycarbonylpiperazin-1-yl)carbonyl)ethenyl)phenyl]sulfide;
(2-Isopropylphenyl)[2-nitro-4-(*E*-((4-methoxycarbonylpiperazin-1-yl)carbonyl)ethenyl)phenyl]sulfide;
(2-Isopropylphenyl)[2-nitro-4-(*E*-(4-(pyridine-4-carbonyl)piperazin-1-yl)carbonyl)ethenyl)phenyl]sulfide;
(2-Isopropylphenyl)[2-nitro-4-(*E*-((3-(pyridine-3-methylaminocarbonyl)-4-*tert*-butoxycarbonylpiperazin-1-yl)carbonyl)ethenyl)phenyl]sulfide;
(2-Isopropylphenyl)[2-nitro-4-(*E*-((3-(pyridine-2-methylaminocarbonyl)piperazin-1-yl)carbonyl)ethenyl)phenyl]sulfide;
(2-Isopropylphenyl)[2-nitro-4-(*E* ((3-(pyridine-3-methylaminocarbonyl)piperazin-1-yl)carbonyl)ethenyl)phenyl]sulfide;
(4-Hydroxyphenyl)[2-nitro-4-(*E*-((4-acetylpiperazin-1-yl)carbonyl)ethenyl)phenyl]sulfide;
(3.5-Dichlorophenyl)[2-nitro-4-(*E*-((4-acetylpiperazin-1-yl)carbonyl)ethenyl)phenyl]sulfide;
(2-Bromophenyl)[2-chloro-4-(*E*-((3-(5*S*-acetoxymethyl-pyrrolidin-2-on-1-yl)prop-1-ylamino)carbonyl)ethenyl)phenyl]sulfide;
(2-Bromophenyl)[2-chloro-4-(*E*-((3-(5*S*-methoxymethyl-pyrrolidin-2-on-1-yl)prop-1-ylamino)carbonyl)ethenyl)phenyl]sulfide;
(2-Bromophenyl)[2-chloro-4-(*E*-((3-(4*R*-hydroxymethyl-pyrrolidin-2-on-1-yl)prop-1-ylamino)carbonyl)ethenyl)phenyl]sulfide;
Phenyl[2-nitro-4-(*E*-((4-acetylpiperazin-1-yl)carbonyl)ethenyl)phenyl]sulfide; (2-Dimethylaminophenyl)[2-nitro-4-(*E*-((4-acetylpiperazin-1-yl)carbonyl) ethenyl)phenyl]sulfide;
(3-((2-Hydroxyethyl)aminocarbonyl)phenyl)[2-nitro-4-(*E*-((4-acetylpiperazin-1-yl)carbonyl)ethenyl)phenyl]sulfide;
(3-((3-(1-Imidazolyl)propyl)aminocarbonyl)phenyl)[2-nitro-4-(*E*-((4-acetylpiperazin-1-yl)carbonyl)ethenyl)phenyl)sulfide;
(3-((2-(1-Morpholinyl)ethyl)aminocarbonyl)phenyl)[2-nitro-4-(*E*-((4-acetylpiperazin-1-yl)carbonyl)ethenyl)phenyl]sulfide;
(2-Isopropylphenyl)[2-nitro-4-(*E*-((3-hydroxymethyl-4-*tert*-butoxycarbonylpiperazin-1-yl)carbonyl)ethenyl)phenyl]sulfide;
(2-Isopropylphenyl)[2-nitro-4-(*E*-((4-formylpiperazin-1-yl)carbonyl)ethenyl)phenyl]sulfide;
(2-Isopropylphenyl)[2-nitro-4-(*E*-((2-hydroxymethyl-4-*tert*-butoxycarbonylpiperazin-1-yl)carbonyl)ethenyl)phenyl]sulfide;
(2-Ethoxyphenyl)-[2-chloro-4(*E*-[(3-ethoxycarbonylpiperidin-1-yl)carbonyl]ethenyl)phenyl]sulfide;
(3- Aminophenyl)[2-nitro-4-(*E*-((4-acetylpiperazin-1-yl)carbonyl)ethenyl)phenyl]sulfide;
(4-Aminophenyl)[2-nitro-4-(*E*-((4-acetylpiperazin-1-yl)carbonyl)ethenyl)phenyl]sulfide;
(2,4-Dimethylphenyl)[2- nitro-4-(*E*-((4-acetylpiperazin-1-yl)carbonyl)ethenyl)phenyl]sulfide;
(2,5-Dimethylphenyl)(2- nitro-4-(*E*-((4-acetylpiperazin-1-yl)carbonyl)ethenyl)phenyl]sulfide;
(4-Methoxyphenyl)[2-nitro-4-(*E*-((4-acetylpiperazin-1-yl)carbonyl)ethenyl)phenyl]sulfide;
(3-Chlorophenyl)[2-nitro-4-(*E*-((4-acetylpiperazin-1-yl)carbonyl)ethenyl)phenyl]sulfide;
(2-Chloro, 4,5-diaminophenyl)[2-chloro-4-(*E*-((4-acetylpiperazin-1-yl)carbonyl)ethenyl)phenyl]sulfide;
(3,4-Diaminophenyl)[2-chloro-4-(*E*-((4-acetylpiperazin-1-yl)carbonyl)ethenyl)phenyl]
(6-Chlorobenzimidazol-2-on-5-yl)[2-chloro-4-(*E*-((4-acetylpiperazin-1-yl)carbonyl)ethenyl)phenyl]sulfide;
(1-Methylindol-7-yl)[2-chloro-4-(*E*-((4-acetylpiperazin-1-yl)carbonyl)ethenyl)phenyl]sulfide;
(2-Hydroxy, 4-aminophenyl)[2-chloro-4-(*E*-((4-acetylpiperazin-1-yl)carbonyl)ethenyl)phenyl]sulfide;
(2-Isopropylphenyl)[2-nitro-4-(*E*-((4-methylpiperazin-1-yl)carbonyl)ethenyl)phenyl]sulfide;
(2-Isopropylphenyl)[2-nitro-4-(*E*-((4-(pyridine-2-carbonyl)piperazin-1-yl)carbonyl)ethenyl)phenyl]sulfide;
(2-Isopropylphenyl)[2-nitro-4-(*E*-((4-(pyridine-3-carbonyl)piperazin-1-yl)carbonyl)ethenyl)phenyl]sulfide;
(2-Isopropylphenyl)[2-nitro-4-(*E*-((2-carbomethoxy-4-methoxycarbonylpiperazin-1-yl)carbonyl)ethenyl)phenyl]sulfide;
(2-Isopropylphenyl)[2-nitro-4-(*E*-((2-carboxy-4-methoxycarbonylpiperazin-1-yl)carbonyl)ethenyl)phenyl]sulfide;
(2-Isopropylphenyl)[2-nitro-4-(*E*-((3-carbomethoxy-4-methylpiperazin-1-yl)carbonyl)ethenyl)phenyl]sulfide;
(2-Ethoxyphenyl)-[2-chloro-4(*E*-[(3-carboxypiperidin-1-yl)carbonyl]ethenyl)phenyl]sulfide;
(2-Ethoxyphenyl)-[2-chloro-4(*E*-[(3-carboxypiperidin-1-yl)carbonyl]ethenyl)phenyl]sulfide;
(2-Ethoxyphenyl)-[2-chloro-4(*E*-[(2-ethoxycarbonylpiperidin-1-yl)carbonyl]ethenyl)phenyl]sulfide;
(2-Ethoxyphenyl)[2-trifluoromethyl-4-(*E*-((1-(*tert*-butoxycarbonyl)-4-hydroxypyrrolidin-3-ylamino)carbonyl)ethenyl)phenyl]sulfide;
(2-Ethoxyphenyl)-[2-chloro-4(*E*-[(2-carboxypiperidin-1-yl)carbonyl]ethenyl)phenyl]sulfide;
(2-Ethoxyphenyl)[2-trifluoromethyl-4-(*E*-(((pyrrol-3-in-1-yl)carbonyl)ethenyl)phenyl]sulfide;
(2-Ethoxyphenyl)[2-trifluoromethyl-4-(*E*-((3-(pyrrolidin-2-on-1-yl)prop-1-ylamino)carbonyl)ethenyl)phenyl]sulfide;
(2-Ethoxyphenyl)(2-trifluoromethyl-4-(*E*-((4-acetylpiperazin-1-yl)carbonyl)ethenyl)phenyl]sulfide;
(2-Ethoxyphenyl)[2-trifluoromethyl-4-(*E*-((4-(ethoxycarbonyl)piperazin-1-yl)carbonyl)ethenyl)phenyl]sulfide;
(2-Ethoxyphenyl)[2-trifluoromethyl-4-(*E*-((4-(2-furylcarbonyl)piperazin-1-yl)carbonyl)ethenyl)phenyl]sulfide;
(2-Ethoxyphenyl)-[2-chloro-4(*E*-[(3-ethoxycarbonylpiperidin-1-yl)carbonyl]ethenyl)phenyl]sulfide;
(2-Ethoxyphenyl)-[2-chloro-4(*E*-[(4-carboxypiperidin-1-yl)carbonyl]ethenyl)phenyl]sulfide;
(Benzodioxan-6-yl)[2-chloro-4-(*E*-((4-acetylpiperazin-1-yl)carbonyl)ethenyl)phenyl]sulfide;
(2-Isopropylphenyl)[2-nitro-4-(*E*-((4-ethoxycarbonylpiperazin-1-yl)carbonyl)ethenyl)phenyl]sulfide;
(2-Isopropylphenyl)[2-nitro-4-(*E*-((4-isopropoxycarbonylpiperazin-1-yl)carbonyl)ethenyl)phenyl]sulfide;
(2-Isopropylphenyl)[2-nitro-4-(*E*-((4-isobutoxycarbonylpiperazin-1-yl)carbonyl)ethenyl)phenyl]sulfide;
(2-Isopropylphenyl)[2-nitro-4-(*E*-((4-((1-propen-2-oxy)carbonyl)piperazin-1-yl)carbonyl)ethenyl)phenyl]sulfide;
(2-Isopropylphenyl)[2-nitro-4-(*E*-((4-propionylpiperazin-1-yl)carbonyl)ethenyl)phenyl]sulfide;
(2-Isopropylphenyl)[2-nitro-4-(*E*-((4-carboxamidopiperazin-1-yl)carbonyl)ethenyl)phenyl]sulfide;
(2-Isopropylphenyl)[2-nitro-4-(*E*-((4-methylaminocarbonylpiperazin-1-yl)carbonyl)ethenyl)phenyl]sulfide;
(2-Isopropylphenyl)[2-nitro-4-(*E*-((4-(pyrimidin-2-yl)piperazin-1-yl)carbonyl)ethenyl)phenyl]sulfide;
(2-Isopropylphenyl)[2-nitro-4-(*E*-((4-hydroxyacetylpiperazin-1-yl)carbonyl)ethenyl)phenyl]sulfide;
(2-Isopropylphenyl)[2-nitro-4-(*E*-((4-(pyrazine-2-carbonyl)piperazin-1-yl)carbonyl)ethenyl)phenyl]sulfide;
(2-Isopropylphenyl)[2-trifluoromethyl-4-(*E*-(((2-carboxypyrrol-3-in-1-yl)carbonyl)ethenyl)phenyl]sulfide;
(2-Isopropylphenyl)[2-nitro-4-(*E*-((3-hydroxymethyl-4-methylpiperazin-1-yl)carbonyl)ethenyl)phenyl]sulfide;
(2-Isopropylphenyl)[2-trifluoromethyl-4-(*E*-(((2-carboxypyrrol-3-in-1-yl)carbonyl)ethenyl)phenyl]sulfide;
(2-Isopropylphenyl)(2-trifluoromethyl-4-(*E*-(((2-hydroxymethylpyrrolidin-1-yl)carbonyl)ethenyl)phenyl]sulfide;
(2-Isopropylphenyl)[2-nitro-4-(*E*-((3-methylaminocarbonyl)piperazin-1-yl)carbonyl)ethenyl)phenyl]sulfide;
(2-Isopropylphenyl)[2-nitro-4-(*E*-(((3-cyclopropylaminocarbonyl)piperazin-1-yl)carbonyl)ethenyl)phenyl]sulfide;
(2-Isopropylphenyl)[2-nitro-4-(*E*-((3-carboxamidopiperazin-1-yl)carbonyl)ethenyl)phenyl]sulfide;
(2-Isopropylphenyl)[2-nitro-4-(*E*-((3-carbomethoxy-4-oxopiperidin-1-yl)carbonyl)ethenyl)phenyl]sulfide;
(2-Isopropylphenyl)[2-nitro-4-(*E*-((3,5-dimethylpiperazin-1-yl)carbonyl)ethenyl)phenyl]sulfide;
(1-Ethylindol-7-yl)[2-chloro-4-(*E*-((4-acetylpiperazin-1-yl)carbonyl)ethenyl)phenyl]sulfide;
(3-[2-Methoxy]ethoxyphenyl)-[2-chloro-4(*E*-[(morpholin-1-yl)carbonyl]ethenyl)phenyl]sulfide;
(2-Bromophenyl)[2-chloro-4-(*E*-((4,4'-*S*-dioxythiomorpholin-1-yl)carbonyl) ethenyl)phenyl]sulfide;
(2-Bromophenyl)[2-chloro-4-(*E*-(N-carbomethoxymethyl-N-(3-(pyrrolidin-2-on-1-yl)prop-1-yl)amino)carbonyl)ethenyl)phenyl]sulfide;
(2-Bromophenyl)[2-chloro-4-(*E*-((4-*S*-oxythiomorpholin-1-yl)-2-pyrrolidinone)carbonyl)ethenyl)phenyl]sulfide;
(2-Methoxy-5-chlorophenyl)[2-nitro-4-(*E*-((4-acetylpiperazin-1-yl)carbonyl)ethenyl)phenyl]sulfide;
(2-Isopropylphenyl)[2-nitro-4-(*E*-((3-acetoxymethyl)piperazin-1-yl)carbonyl)ethenyl)phenyl]sulfide;
(2-Isopropylphenyl)[2-nitro-4-(*E*-((3,5-dimethyl-4acetylpiperazin-1-yl)carbonyl)ethenyl)phenyl]sulfide;
(1-Methylindol-5-yl)[2-chloro-4-(*E*-((4-acetylpiperazin-1-yl)carbonyl)ethenyl)phenyl]sulfide;
(Benzodioxan-6-yl)[2-nitro-4-(*E*-((4-acetylpiperazin-1-yl)carbonyl)ethenyl)phenyl]sulfide;
(Benzodioxan-6-yl)[2-nitro-4-(*E*-((3-(pyrrolidin-2-on-1-yl)prop-1-ylamino)carbonyl) ethenyl)phenyl)sulfide;
(Benzodioxan-6-yl)[2-nitro-4-(*E*-((3-carboethoxypiperidin-1-yl)carbonyl)ethenyl)phenyl]sulfide;
(Benzodioxan-6-yl)[2-nitro-4-(*E*-((4-carboethoxypiperidin-1-yl)carbonyl)ethenyl)phenyl]sulfide;
(2-Ethoxyphenyl)[2-trifluoromethyl-4-(*Z*-((4-acetylpiperazin-1-yl)carbonyl)ethenyl)phenyl]sulfide;
(2-Ethoxyphenyl)[2-trifluoromethyl-4-(*E*-((6-methylpyrid-2-ylamino)carbonyl)ethenyl)phenyl]sulfide;
(2-Methyl-3-chlorophenyl)[2-nitro-4-(*E*-((4-acetylpiperazin-1-yl)carbonyl)ethenyl)phenyl]sulfide;
(Benzodioxan-6-yl)[2-nitro-4-(*E*-((3-carboxamidopiperidin-1-yl)carbonyl)ethenyl)phenyl]sulfide;
(Benzodioxan-6-yl)[2-nitro-4-(*E*-((2-carboethoxypiperidin-1-yl)carbonyl)ethenyl)phenyl]sulfide;
(Benzodioxan-6-yl)[2-nitro-4-(*E*-((4-carboxamidopiperidin-1-yl)carbonyl)ethenyl)phenyl]sulfide;
(Benzodioxan-6-yl)[2-nitro-4-(*E*-((4-*tert*-butoxycarbonylpiperazin-1-yl) carbonyl)ethenyl)phenyl]sulfide;
(2-Isopropylphenyl)[2-nitro-4-(*E*-((*syn*-3,5-dimethylmorpholin-1-yl)carbonyl)ethenyl)phenyl]sulfide;
(2-Isopropylphenyl)[2-nitro-4-(*E*-((*anti*-3,5-dimethylmorpholin-1-yl)carbonyl)ethenyl)phenyl]sulfide;
(2-Isopropylphenyl)[2-nitro-4-(*E*-((3-carboethoxypiperazin-1-yl)carbonyl)ethenyl)phenyl]sulfide;
(2-Isopropylphenyl)[2-nitro-4-(*E*-((3-isopropoxycarbonylpiperazin-1-yl)carbonyl)ethenyl)phenyl]sulfide;
(2-Isopropylphenyl)[2-nitro-4-(*E*-((3-(dimethylaminocarbonyl)-4-methylpiperazin-1-yl)carbonyl)ethenyl)phenyl]sulfide;
(2-Isopropylphenyl)[2-nitro-4-(*E*-((3-carbomethoxy-4-hydroxypiperidin-1-yl)carbonyl)ethenyl)phenyl]sulfide;
(2-Isopropylphenyl)[2-nitro-4-(*E*-((3-hydroxymethyl-4-hydroxypiperidin-1-yl)carbonyl)ethenyl)phenyl]sulfide;
(2-Ethoxyphenyl)[2-trifluoromethyl-4-(*E*-((2-carbomethoxy-4-(methoxycarbonyl)piperazin-1-yl)carbonyl)ethenyl)phenyl]sulfide;
(2-Ethoxyphenyl)[2-trifluoromethyl-4-(*E*-((2-carbomethoxy-4-methylpiperazin-1-yl)carbonyl)ethenyl)phenyl]sulfide;
(2-Ethoxyphenyl)[2-trifluoromethyl-4-(*E*-((2-carboxy-4-(methoxycarbonyl)piperazin-1-yl)carbonyl)ethenyl)phenyl]sulfide;
(Indol-6-yl)[2-chloro-4-(*E*-((4-acetylpiperazin-1-yl)carbonyl)ethenyl)phenyl]sulfide;
(1-Ethyl,3-(dimethylaminomethyl)indol-7-yl)[2-chloro-4-(*E*-((4-acetylpiperazin-1-yl)carbonyl)ethenyl)phenyl]sulfide;
(5-Ethoxybenzodioxan-6-yl)[2-chloro-4-(*E*-((4-acetylpiperazin-1-yl)carbonyl)ethenyl)phenyl]sulfide;
(2-Ethyl-4-bromophenyl)[2-nitro-4-(*E*-((4-acetylpiperazin-1-yl)carbonyl)ethenyl)phenyl]sulfide;
(Benzodioxan-6-yl)[2-nitro-4-(*E*-((2-carboxypiperidin-1-yl)carbonyl)ethenyl)phenyl]sulfide;
(Benzodioxan-6-yl)[2-nitro-4-(*E*-((4-carboxymethylpiperazin-1-yl) carbonyl)ethenyl)phenyl]sulfide;
(3-Morpholinophenyl)[2-nitro-4-(*E*-((4-acetylpiperazin-1-yl)carbonyl)ethenyl)phenyl]sulfide;
(5-Ethoxybenzodioxan-8-yl)[2-chloro-4-(*E*-((4-acetylpiperazin-1-yl)carbonyl)ethenyl)phenyl]sulfide;
(5-Chloro-8-ethoxyquinolin-7-yl)[2-chloro-4-(*E*-((4-acetylpiperazin-1-yl)carbonyl)ethenyl)phenyl]sulfide;
(2-Isopropylphenyl)[2-nitro-4-(*E*-((3-carboethoxypiperidin-1-yl)carbonyl)ethenyl)phenyl]sulfide;
(2-Isopropylphenyl)[2-nitro-4-(*E*-((3-carboxypiperidin-1-yl)carbonyl)ethenyl)phenyl]sulfide;
(2-Isopropylphenyl)[2-nitro-4-(*E*-(((3-ethanesulfonylaminocarbonyl)piperidin-1-yl)carbonyl)ethenyl)phenyl]sulfide;
(2-Isopropylphenyl)[2-nitro-4-(*E*-(((3-(4-methylpiperazine) sulfonylaminocarbonyl)piperidin-1-yl)carbonyl)ethenyl)phenyl]sulfide;
(2-Isopropylphenyl)[2-nitro-4-(*E*-(((3-*p*-toluenesulfonylaminocarbonyl)piperidin-1-yl)carbonyl)ethenyl)phenyl]sulfide;
(2-Isopropylphenyl)[2-nitro-4-(*E*-((3-methyl-4-acetylpiperazin-1-yl)carbonyl)ethenyl)phenyl]sulfide;
(2-Hydroxyphenyl)-[2-chloro-4(*E*-[(morpholin-1-yl)carbonyl]ethenyl)phenyl]sulfide
(1-(Carboxymethyl)indol-5-yl)[2-chloro-4-(*E*-((4-acetylpiperazin-1-yl)carbonyl)ethenyl)phenyl]sulfide;
(Benzodioxan-6-yl)[2-trifluoromethyl-4-(*E*-((4-acetylpiperazin-1-yl)carbonyl)ethenyl)phenyl]sulfide;
(2-Isopropylphenyl)[2-nitro-4-(*E*-((3-(1-pyrrolidin-2-onyl)prop-1-ylamino) carbonyl)ethenyl)phenyl]sulfide;
(3-(2-Morpholinoethylamino)phenyl)[2-trifluoromethyl-4-(*E*-((4-acetylpiperazin-1-yl)carbonyl)ethenyl)phenyl]sulfide;
(2-Pyrrolidin-1-ylphenyl)[2-nitro-4-(*E*-((4-acetylpiperazin-1-yl)carbonyl)ethenyl)phenyl]sulfide;
(3-Bromophenyl)[2-nitro-4-(*E*-((3-carboethoxypyrrolidin-1-yl)carbonyl)ethenyl)phenyl]sulfide;
(3-Bromophenyl)[2-nitro-4-(*E*-((4-carboethoxypyrrolidin-1-yl)carbonyl)ethenyl)phenyl]sulfide;
(2-(Hydroxymethyl)-benzodioxan-6-yl)[2-chloro-4-(*E*-((4-acetylpiperazin-1-yl)carbonyl)ethenyl)phenyl]sulfide;
(Benzodioxan-6-yl)[2-trifluoromethyl-4-(*E*-((3-(pyrrolidin-2-on-1-yl)prop-1-ylamino)carbonyl)ethenyl)phenyl]sulfide;
(3-(Dimethylaminomethyl)indol-5-yl)[2-chloro-4-(*E*-((4-acetylpiperazin-1-yl)carbonyl)ethenyl)phenyl]sulfide;
(2-Isopropylphenyl)[2-nitro-4-(*E*-((2-carboethoxypiperidin-1-yl)carbonyl)ethenyl)phenyl]sulfide;
(2-Isopropylphenyl)[2-nitro-4-(*E*-((2-carboxypiperidin-1-yl)carbonyl)ethenyl)phenyl]sulfide;
(2-Isopropylphenyl)[2-nitro-4-(*E*-((4-carboethoxypiperidin-1-yl)carbonyl)ethenyl)phenyl]sulfide;
(2-Isopropylphenyl)[2-nitro-4-(*E*-((4-carboxypiperidin-1-yl)carbonyl)ethenyl)phenyl]sulfide;
(2-Isopropylphenyl)[2-nitro-4-(*E*-(((4-*p*-toluenesulfonylaminocarbonyl)piperidin-1-yl)carbonyl)ethenyl)phenyl]sulfide;
(2-Isopropylphenyl)[2-nitro-4-(*E*-((3-carboxy-4-hydroxypiperidin-1-yl)carbonyl)ethenyl)phenyl]sulfide;
(Benzodioxan-6-yl)[2-trifluoromethyl-4-(*E*-((3-carboethoxypiperidin-1-yl) carbonyl)ethenyl)phenyl]sulfide;
(Benzodioxan-6-yl)[2-trifluoromethyl-4-(*E*-((2-carboethoxypiperidin-1-yl) carbonyl)ethenyl)phenyl]sulfide;
(Benzodioxan-6-yl)[2-nitro-4-(*E*-((4-carboxypiperidin-1-yl)carbonyl)ethenyl)phenyl]sulfide;
(Benzodioxan-6-yl)[2-trifluoromethyl-4-(*E*-((3-carboxypyrrolidin-1-yl)carbonyl)ethenyl)phenyl]sulfide;
(Benzodioxan-6-yl)[2-trifluoromethyl-4-(*E*-((4-carboethoxypiperidin-1-yl) carbonyl)ethenyl)phenyl]sulfide;
(Benzodioxan-6-yl)[2-trifluoromethyl-4-(*E*-((2-carbomethoxy-4-*tert*-butoxycarbonylpiperazin-1-yl)carbonyl)ethenyl)phenyl]sulfide;
(Benzodioxan-6-yl)[2-trifluoromethyl-4-(*E*-((2-carbomethoxy-4-methoxycarbonylpiperazin-1-yl)carbonyl)ethenyl)phenyl]sulfide;
(Benzodioxan-6-yl)[2-trifluoromethyl-4-(*E*-((2-carbomethoxypiperazin-1-yl) carbonyl)ethenyl)phenyl]sulfide;
(2-Methyl-3-(carboethoxymethyl)indol-5-yl)[2-trifluoromethyl-4-(*E*-((morpholin-1-yl)carbonyl)ethenyl)phenyl]sulfide;
(1-(2-Methoxyethyl)indol-5-yl)[2-chloro-4-(*E*-((4-acetylpiperazin-1-yl)carbonyl)ethenyl)phenyl]sulfide;
(2-Isopropylphenyl)[2-nitro-4-(*E*-((3-acetoxymethyl-4-hydroxypiperidin-1-yl)carbonyl)ethenyl)phenyl]sulfide;
(2-Isopropylphenyl)[2-nitro-4-(*E*-((3-(dimethylaminocarbonyl)-4-hydroxypiperidin-1-yl)carbonyl)ethenyl)phenyl]sulfide;
(2-Isopropylphenyl)[2-nitro-4-(*E*-((3-cyanomorpholin-1-yl)carbonyl)ethenyl)phenyl]sulfide;
(2-Isopropylphenyl)[2-nitro-4-(*E*-((3-carboethoxymorpholin-1-yl)carbonyl)ethenyl)phenyl]sulfide;
(2-Isopropylphenyl)[2-nitro-4-(*E*-((3-(tetrazol-5-yl)morpholin-1-yl)carbonyl)ethenyl)phenyl]sulfide;
(Benzodioxan-6-yl)[2-trifluoromethyl-4-(*E*-((4-carboxypiperidin-1-yl) carbonyl)ethenyl)phenyl]sulfide;
(Benzodioxan-6-yl)[2-trifluoromethyl-4-(*E*-((2-carboxypiperidin-1-yl) carbonyl)ethenyl)phenyl]sulfide;
(Benzodioxan-6-yl)[2-trifluoromethyl-4-(*E*-((4-carbomethoxypiperazin-1-yl) carbonyl)ethenyl)phenyl]sulfide;
(Benzodioxan-6-yl)[2-trifluoromethyl-4-(*E*-((3-aza-6,9-diooxaspiro[5.4]decan-1-yl)carbonyl)ethenyl)phenyl]sulfide;
(Benzodioxan-6-yl)[2-trifluoro-4-(*E*-((4-(benzimidazolon-1-yl)piperidin-1-yl)carbonyl)ethenyl)phenyl]sulfide;
(Benzodioxan-6-yl)[2-trifluoromethyl-4-(*E*-((4-(methylaminocarbonyl)piperidin-1-yl)carbonyl)ethenyl)phenyl]sulfide;
(Benzodioxan-6-yl)[2-trifluoromethyl-4-(*E*-((3-carbomethoxy-4-methoxycarbonylpiperazin-1-yl)carbonyl)ethenyl)phenyl]sulfide;
(2-Isopropylphenyl)[2-nitro-4-(*E*-((3-carboxymorpholin- 1-yl)carbonyl)ethenyl)phenyl]sulfide;
(Benzodioxan-6-yl)[2-trifluoromethyl-4-(*E*-((2-carboxy-4-methoxycarbonylpiperazin-1-yl)carbonyl)ethenyl)phenyl]sulfide;
(Benzodioxan-6-yl)[2-trifluoromethyl-4-(*E*-((morpholin-1-yl)carbonyl)ethenyl)phenyl]sulfide;
(Benzodioxan-6-yl)[2-trifluoromethyl-4-(*E*-((4-(pyrrolidin-1-yl)piperidin-1-yl)carbonyl)ethenyl)phenyl]sulfide;
(2-Isopropylphenyl)[2-nitro-4-(*E*-((3-aza-6,9-diooxaspiro[5.4]decan-1-yl)carbonyl)ethenyl)phenyl]sulfide;
(2-Isopropylphenyl)[2-nitro-4-(*E*-((2-(dimethylaminomethyl)piperidin-1-yl)carbonyl)ethenyl)phenyl]sulfide;
(2-Isopropylphenyl)[2-nitro-4-(*E*-((piperidin-1-ylamino)carbonyl)ethenyl)phenyl]sulfide;
(Benzodioxan-6-yl)[2-trifluoromethyl-4-(*E*-((3-carboxy-4-methoxycarbonylpiperazin-1-yl)carbonyl)ethenyl)phenyl]sulfide;
(2-(Dimethylaminocarbonyl)-benzodioxan-6-yl)[2-chloro-4-(*E*-((4-acetylpiperazin-1-yl)carbonyl)ethenyl)phenyl]sulfide;;
(2-Isopropylphenyl)[2-nitro-4-(*E*-((3-(2-(methoxymethyl)tetrazol-5-yl) piperidin-1-yl)carbonyl)ethenyl)phenyl]sulfide
(2-Isopropylphenyl)[2-nitro-4-(*E*-((3-(1-(methoxymethyl)tetrazol-5-yl) piperidin-1-yl)carbonyl)ethenyl)phenyl]sulfide;
(1-Methylindol-5-yl)[2-chloro-4-(*E*-((3-(1-pyrrolidin-2-onyl)propylamino) carbonyl)ethenyl)phenyl]sulfide;
(2-Isopropylphenyl)[2-nitro-4-(*E*-((3-(tetrazol-5-yl) piperidin-1-yl)carbonyl)ethenyl)phenyl]sulfide;
(1-Methylindol-5-yl)[2-chloro-4-(*E*-((3-carboethoxypiperidin-1-yl)carbonyl)ethenyl)phenyl]sulfide
(1-Methylindol-5-yl)[2-chloro-4-(*E*-((3-carboxypiperidin-1-yl)carbonyl)ethenyl)phenyl]sulfide;
(1-Methylindol-5-yl)[2-chloro-4-(*E*-((4-carboethoxypiperidin-1-yl)carbonyl)ethenyl)phenyl]sulfide;
(1-Methylindol-5-yl)[2-chloro-4-(*E*-((3-carboxypiperidin-1-yl)carbonyl)ethenyl)phenyl]sulfide;
(2-Isopropylphenyl)[2-nitro-4-(*E*-((2-(1-methylpyrrolidin-2-yl)ethylamino)carbonyl)ethenyl)phenyl]sulfide;
(2-Isopropylphenyl)[2-nitro-4-(*E*-((4-(pyrrolidin-1-yl)piperidin-1-yl)carbonyl)ethenyl)phenyl]sulfide;
(2-Isopropylphenyl)[2-nitro-4-(*E*-((4-sulfopiperidin-1-yl)carbonyl)ethenyl)phenyl]sulfide;
(2-Isopropylphenyl)[2-nitro-4-(*E*-((3-hydroxypiperidin-1-yl)carbonyl)ethenyl)phenyl]sulfide;
(Benzodioxan-6-yl)[2-trifluoromethyl-4-(*E*-((3-((ethanesulfonylamino)carbonyl)piperidin-1-yl)carbonyl)ethenyl)phenyl]sulfide;
(Benzodioxan-6-yl)[2-trifluoromethyl-4-(*E*-((3-((*p*-toluenesulfonylamino)carbonyl)piperidin-1-yl)carbonyl)ethenyl)phenyl]sulfide;
(Benzodioxan-6-yl)[2-trifluoromethyl-4-(*E*-((4-((ethanesulfonylamino)carbonyl)piperidin-1-yl)carbonyl)ethenyl)phenyl]sulfide;
(Benzodioxan-6-yl)[2-trifluoromethyl-4-(*E*-((2(tetrazot-5-yl)morpholin-1-yl)carbonyl)ethenyl)phenyl]sulfide;
(2-Isopropylphenyl)[2-nitro-4-(*E*-((2-butyl, 5-(tetrazol-5-yl)morpholin-1-yl)carbonyl)ethenyl)phenyl]sulfide;
(2-(and 3-)(Hydroxymethyl)-benzodioxan-6-yl)[2-nitro-4-(*E*-((4-acetylpiperazin-1-yl)carbonyl)ethenyl)phenyl]sulfide;
(2-(and 3-)(Hydroxymethyl)-benzodioxan-6-yl)[2-nitro-4-(*E*-((3-(pyrrolidin-2-on-1-yl)prop-1-ylamino)carbonyl)ethenyl)phenyl]sulfide;
(2-(and 3-)(Hydroxymethyl)-benzodioxan-6-yl)[2-trifluoromethyl-4-(*E*-((3-(pyrrolidin-2-on-1-yl)prop-1-ylamino)carbonyl)ethenyl)phenyl]sulfide;
(3-Hydroxymethyl)-benzodioxan-6-yl)[2-nitro-4-(*E*-((3-(pyrrolidin-2-on-1-yl)prop-1-ylamino)carbonyl)ethenyl)phenyl]sulfide;
(Benzodioxan-6-yl)[2-chloro-4-(*E*-((3-carboxypiperidin-1-yl)carbonyl)ethenyl)phenyl]
(2-(and 3-)(Aminomethyl)-benzodioxan-6-yl)[2-trifluoromethyl-4-(*E*-((3-(pyrrolidin-2-on-1-yl)prop-1-ylamino)carbonyl)ethenyl)phenyl]sulfide;
(2-Isopropylphenyl)[2-nitro-4-(*E*-((3-(methylaminocarbonyl)morpholin-1-yl)carbonyl)ethenyl)phenyl]sulfide;
(2-Isopropylphenyl)[2-nitro-4-(*E*-((3-(hydroxymethyl)morpholin-1-yl)carbonyl)ethenyl)phenyl]sulfide;
(2-Isopropylphenyl)[2-nitro-4-(*E*-((3-(acetoxymethyl)morpholin-1-yl)carbonyl)ethenyl)phenyl]sulfide;
(2-Isopropylphenyl)[2-nitro-4-(*E*-((3-(aminomethyl)morpholin-1-yl)carbonyl)ethenyl)phenyl]sulfide;
(2-Isopropylphenyl)[2-nitro-4-(*E*-((3-(acetamidomethyl)morpholin-1-yl)carbonyl)ethenyl)phenyl]sulfide;
(Benzodioxan-6-yl)[2-chloro-4-(*E*-((3-(pyrrolidin-2-on-1-yl)prop-1-ylamino)carbonyl)ethenyl)phenyl]sulfide;
(Benzodioxan-6-yl)[2-chloro-4-(*E*-((3-carboethoxypiperidin-1-yl)carbonyl)ethenyl)phenyl]sulfide;
(Benzodioxan-6-yl)[2-chloro-4-(*E*-((2-carboethoxypiperidin-1-yl)carbonyl)ethenyl)phenyl]sulfide
(2-Methoxyphenyl)-[2,3-dichloro-4(*E*-[(morpholin-1-yl)carbonyl]ethenyl)phenyl]sulfide;
(2-Methoxyphenyl)-[2,3-dimethyl-4(*E*-[(morpholin-1-yl)carbonyl]ethenyl)phenyl]sulfide;
(2-Isopropylphenyl)[2-nitro-4-(*E*-((indol-5-ylamino)carbonyl)ethenyl)phenyl]sulfide;
(Benzodioxan-6-yl)[2-chloro-4-(*E*-((3-carboxypiperidin-1-yl)carbonyl)ethenyl)phenyl]sulfide;
(Benzodioxan-6-yl)[2-chloro-4-(*E*-((3-(tetrazol-5-yl)piperidin-1-yl)carbonyl)ethenyl)phenyl]sulfide;
(Benzodioxan-6-yl)[2-chloro-4-(*E*-((4-(*tert*-butoxycarbonyl)piperazin-1-yl) carbonyl)ethenyl)phenyl]sulfide;
(Benzodioxan-6-yl)[2-chloro-4-(*E*-((2-carboxypiperidin-1-yl)carbonyl)ethenyl)phenyl]sulfide;
(Benzodioxan-6-yl)[2-chloro-4-(*E*-((3-(tetrazol-5-yl)morpholin-1-yl) carbonyl)ethenyl)phenyl]sulfide;
(Benzodioxan-6-yl)[2-chloro-4-(*E*-((4-(methylaminocarbonyl)piperazin-1-yl) carbonyl)ethenyl)phenyl]sulfide;
(2-Methoxyphenyl)-[2,3-dichloro-4(*E*-[(4-carboxypiperidin-1-yl)carbonyl]ethenyl)phenyl]sulfide;
(Benzodioxan-6-yl)[2-chloro-4-(*E*-((4-(tetrazol-5-yl)piperidin-1-yl)carbonyl)ethenyl)phenyl]sulfide;
(2-Methoxyphenyl)-[3-chloro-4(*E*-[(morpholin-1-yl)carbonyl]ethenyl)phenyl]sulfide;
(2-Isopropylphenyl)[2-nitro-4-(*E*-((4-oxopiperidin-1-yl)carbonyl)ethenyl)phenyl]sulfide;
(Benzodioxan-6-yl)[2-trifluoromethyl-4-(*E*-((3-*R*-carboethoxypiperidin-1-yl)carbonyl)ethenyl)phenyl]sulfide;
(Benzodioxan-6-yl)[2-trifluoromethyl-4-(*E*-((3-*R*-carboxypiperidin-1-yl)carbonyl)ethenyl)phenyl]sulfide;
(Benzodioxan-6-yl)[2,3-dichloro-4-(*E*-((3-(pyrrolidin-2-on-1-yl)prop-1-ylamino)carbonyl)ethenyl)phenyl]sulfide;
(Benzodioxan-6-yl)[2,3-dichloro-4-(*E*-((4-acetylpiperazin-1-yl)carbonyl)ethenyl)phenyl]sulfide;
(Benzodioxan-6-yl)[2,3-dichloro-4-(*E*-((3-carboethoxypiperidin-1-yl) carbonyl)ethenyl)phenyl]sulfide;
(Benzodioxan-6-yl)[2,3-dichloro-4-(*E*-((4-carboethoxypiperidin-1-yl) carbonyl)ethenyl)phenyl]sulfide;
(Benzodioxan-6-yl)[2,3-dichloro-4-(*E*-((3-carboxypiperidin-1-yl)carbonyl)ethenyl)phenyl]sulfide;
(Benzodioxan-6-yl)[2,3-dichloro-4-(*E*-((4-carboxypiperidin-1-yl)carbonyl)ethenyl)phenyl]sulfide;
(2-Isopropylphenyl)[2,3-dichloro-4-(*E*-((3-(1-pyrrolidin-2-onyl)propylamino) carbonyl)ethenyl)phenyl]sulfide;
(2-Isopropylphenyl)[2,3-dichloro-4-(*E*-((4-acetylpiperazin-1-yl)carbonyl)ethenyl)phenyl]sulfide;
(2-Isopropylphenyl)[2,3-dichloro-4-(*E*-((3-carboethoxypiperidin-1-yl) carbonyl)ethenyl)phenyl]sulfide;
(2-Isopropylphenyl)[2,3-dichloro-4-(*E*-((4-carboethoxypiperidin-1-yl) carbonyl)ethenyl)phenyl]sulfide;
(2-Isopropylphenyl)[2,3-dichloro-4-(*E*-((3-carboxypiperidin-1-yl)carbonyl)ethenyl)phenyl]sulfide;
(2-Isopropylphenyl)[2,3-dichloro-4-(*E*-((4-carboxypiperidin-1-yl)carbonyl)ethenyl)phenyl]sulfide;
(1-Methylindol-5-yl)[2,3-dichloro-4-(*E*-((3-carboethoxypiperidin-1-yl) carbonyl)ethenyl)phenyl]sulfide;
(1-Methylindol-5-yl)[2,3-dichloro-4-(*E*-((3-carboxypiperidin-1-yl)carbonyl)ethenyl)phenyl]sulfide;
(1-Methylindol-5-yl)[2,3-dichloro-4-(*E*-((4-carboethoxypiperidin-1-yl) carbonyl)ethenyl)phenyl]sulfide;
(1-Methylindol-5-yl)[2,3-dichloro-4-(*E*-((4-carboxypiperidin-1-yl)carbonyl)ethenyl)phenyl]sulfide;
(2-Ethoxyphenyl)-[2,3-dichloro-4(*E*-[(4-carboxypiperidin-1-yl)carbonyl]ethenyl)phenyl]sulfide;
(2-Ethoxyphenyl)-[2,3-dichloro-4(*E*-[(morpholin-1-yl)carbonyl]ethenyl)phenyl]sulfide;
(2-Ethoxyphenyl)-[2,3-dichloro-4(*E*-[(3-carboxypiperidin-1-yl)carbonyl]ethenyl)phenyl]sulfide;
(2-Isopropylphenyl)[2-nitro-4-(*E*-((3-carboethoxypyrrolidin-1-yl)carbonyl)ethenyl)phenyl]sulfide;
(2-Isopropylphenyl)[2-nitro-4-(*E*-((3-carboxypyrrolidin-1-yl)carbonyl)ethenyl)phenyl]sulfide;
(2-Isopropylphenyl)[2,3-difluoro-4-(*E*-((3-carboethoxypiperidin-1-yl)carbonyl)ethenyl)phenyl]sulfide;
(2-Isopropylphenyl)[2,3-difluoro-4-(*E*-((3-carboxypiperidin-1-yl)carbonyl)ethenyl)phenyl]sulfide;
(2-Isopropylphenyl)[2,3-difluoro-4-(*E*-((4-carboxypiperidin-1-yl)carbonyl)ethenyl)phenyl]sulfide;
(Benzodioxan-6-yl)[2-trifluoromethyl-4-(*E*-((3-ethoxycarbonylpyrrolidin-1-yl)carbonyl)ethenyl)phenyl]sulfide
(Benzodioxan-6-yl)[2-trifluoromethyl-4-(*E*-((3-carboxypyrrolidin-1-yl)carbonyl)ethenyl)phenyl]sulfide;
(2-Methoxyphenyl)[2-chloro-3-trifluoromethyl-4-(*E*-((4-carboethoxypiperidin-1-yl)carbonyl)ethenyl)phenyl]sulfide;
(2-Methoxyphenyl)[2-chloro-3-trifluoromethyl-4-(*E*-((4-carboethoxypiperidin-1-yl)carbonyl)ethenyl)phenyl]sulfide;
(2-Methoxyphenyl)[2-chloro-3-trifluoromethyl-4-(*E*-((morpholin-1-yl)carbonyl)ethenyl)phenyl]sulfide;
(Benzodioxan-6-yl) [ 4-(*E*-((4-carboxypiperidin-1-yl)carbonyl)ethenyl)naphthyl] sulfide;
(2-Methoxyphenyl) [2,3-dichloro-4-(*E*-((4-(spiro-hydantoin-5-yl)-piperidin-1-yl)carbonyl)ethenyl)phenyl]sulfide;
(2-Methoxyphenyl) [2,3-dichloro-4-(*E*-(4-(2-(2-hydroxyethoxy)ethyl)piperazin-1-yl)carbonyl)ethenyl)phenyl]sulfide;
(2-Methoxyphenyl)[ 2,3-dichloro-4-(*E*-((4-ethylpiperazin-1-yl)carbonyl)ethenyl)phenyl]sulfide;
(2-Isopropylphenyl)[ 2,3-dichloro-4-(*E*-((4-(2-(2-hydroxyethoxy)ethyl)piperazin-1-yl)carbonyl)ethenyl)phenyl]sulfide;
(Benzodioxan-6yl)[2,3-bis(trifluoromethyl)-4-(*E*-((4-carboxypiperidin-1-yl)carbonyl)ethenyl)phenyl]sulfide;
(2-Methoxyphenyl) [2,3-dichloro-4-(*E*-((4-(carboxymethylamino)carbonyl-piperidin-1-yl)carbonyl)ethenyl)phenyl]sulfide;
(2-Methoxyphenyl) [2,3-bis(trifluoromethyl)-4-(*E*-((4-carboxymethylpiperazin-1-yl) carbonyl)ethenyl)phenyl]sulfide;
(2-Methoxyphenyl) [2,3-bis(trifluoromethyl)-4-(*E*-((4-N-(2-hydroxyethyl)piperazin-1-yl)carbonyl)ethenyl)phenyl]sulfide;
(1-Methylindol-5-yl)[2,3-dichloro-4-(*E*-((4-(carbo-2,3-dihydroxypropylamino)piperidin-1-yl)carbonyl)ethenyl)phenyl]sulfide;
(2-Methoxyphenyl) [2,3-dichloro-4-(*E*-(4-(2,3-dihydroxypropionyl)piperazin-1-yl)carbonyl)ethenyl)phenyl]sulfide;
(2-Methoxyphenyl) [2,3-dichloro-4-(*E*-(4-(2,3-dihydroxy-3-carboxypropionyl)piperazin-1-yl)carbonyl)ethenyl)phenyl]sulfide;
(1-Methylindol-5-yl)[2,3-dichloro-4-(*E*-((4-(carboxymethylamino)carbonylpiperidin-1-yl)carbonyl)ethenyl)phenyl]sulfide;
(1-Methylindol-5-yl)[2,3-dichloro-4-(*E*-((4-sulfopiperidin-1-yl)carbonyl)ethenyl)phenyl]sulfide;
(1-Methylindol-5-yl)[2,3-dichloro-4-(*E*-(4-methylhomopiperazin-1-ylcarbonyl)ethenyl)phenyl]sulfide;
(1-Methylindol-5-yl)[2,3-dichloro-4-(*E*-(4-tetrohydrofuroylpiperazin-1-yl)carbonyl)ethenyl)phenyl]sulfide;
(2-Methoxyphenyl) [2,3-dichloro-4-(*E*-((4-amino-4-carboxypiperidin-1-yl)carbonyl)ethenyl)phenyl]sulfide;
(2-Methoxyphenyl)[2,3-dichloro-4-((4-furoylpiperazin-1-yl)carbonyl)ethenyl)phenyl]sulfide;
(1-Methylindol-5-yl)[2,3-dichloro-4-(*E*-(4-(carbo-3-sulfopropylamino)piperadin-1-yl)carbonyl)ethenyl)phenyl]sulfide;
(2-Methoxyphenyl)[ 2,3-dichloro-4-(*E*-(4-acetylamino-4-carboxypiperidin-1-ylcarbonyl)ethenyl)phenyl]sulfide;
(2-Methoxyphenyl) [2,3-bis(trifluoromethyl)-4-(*E*-((4-carboxypiperidin-1-yl)carbonyl)ethenyl)phenyl]sulfide;
(2-Methoxyphenyl) 5-[8-(*E*-((4-(aminocarbonyl)piperidin-1-yl)carbonyl)ethenyl)quinolinyl]sulfide;
(2-Methoxyphenyl) [2-trifluoromethyl-4-(*E*-((4-carboxypiperidin-1-yl)carbonyl)ethenyl)phenyl]sulfide;
(1-Methylindol-5-yl) [ 2,3-dichloro-4-(*E*/*Z*-((1S,4S)-2,5-diazabycyclo(2,2.1)heptan-2-ylcarbonyl)ethenyl)-2,3-dichlorophenyl]sulfide;
(1-Methylindol-5-yl) [2,3-dichloro-4-(*E*-(4-hydroxy-3-carboxypiperadin-1-ylcarbonyl)ethenyl)phenyl]sulfide;;
(1-Methylindol-5-yl)[ 2,3-dichloro-4-(*E*-(S-oxothiomorpholin-1-ylcarbonyl)ethenyl)phenyl]sulfide;
(2-Methoxyphenyl) [2,3-dichloro-4-(*E*-((4-sulfophenylamino)carbonyl)ethenyl)phenyl]sulfide;
(2-Methoxyphenyl) [2,3-dichloro-4-(*E*-((4-carboxyphenylamino)carbonyl)ethenyl)phenyl]sulfide; and
[3-(4-Morpholino)phenyl] [2,3-dichloro-4-(*E*-[(4-carboxypiperidin-1-yl)carbonyl]ethenyl)phenyl]sulfide.

### Pharmaceutical Compositions and Methods of Treatment

The present invention also provides pharmaceutical compositions which comprise compounds of the present invention formulated together with one or more pharmaceutically-acceptable carriers. The pharmaceutical compositions may be specially formulated for oral administration in solid or liquid form, for parenteral injection, or for rectal administration.

The pharmaceutical compositions of this invention can be administered to humans and other animals orally, rectally, parenterally, intracisternally, intravaginally, intraperitoneally, topically (as by powders, ointments, or drops), bucally, or as an oral or nasal spray. The term "parenteral" administration as used herein refers to modes of administration which include intravenous, intramuscular, intraperitoneal, intrasternal, subcutaneous and intraarticular injection and infusion.

Pharmaceutical compositions of this invention for parenteral injection comprise pharmaceutically-acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like, Prolonged absorption of the injectable pharmaceutical form may be brought about by the inclusion of agents which delay absorption such as aluminum monostearate and gelatin.

In some cases, in order to prolong the effect of the drug, it is desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissues.

The injectable formulations can be sterilized, for example, by filtration through a bacterial-retaining filter, or by incorporating sterilizing agents in the form of sterile solid compositions which can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically-acceptable excipient or carrier such as sodium citrate or dicalcium phosphate and/or (a) fillers or extenders such as starches, lactose, sucrose, glucose, mannitol, and silicic acid, (b) binders such as, for example, carboxymethylcellulose, alginates, gelatin, polyvinylpyrrolidone, sucrose, and acacia, (c) humectants such as glycerol, (d) disintegrating agents such as agar-agar, calcium carbonate, potato or tapioca starch, alginic acid, certain silicates, and sodium carbonate, (e) solution retarding agents such as paraffin, (f) absorption accelerators such as quaternary ammonium compounds, (g) wetting agents such as, for example, cetyl alcohol and glycerol monostearate, (h) absorbents such as kaolin and bentonite clay, and (I) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

The active compounds can also be in micro-encapsulated form, if appropriate, with one or more of the above-mentioned excipients.

Liquid dosage forms for oral administration include pharmaceutically-acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, com, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

Compositions for rectal or vaginal administration are preferably suppositories which can be prepared by mixing the compounds of this invention with suitable nonirritating excipients or carriers such as cocoa butter, polyethylene glycol or a suppository wax which are solid at room temperature but liquid at body temperature and therefore melt in the rectum or vaginal cavity and release the active compound.

Compounds of the present invention can also be administered in the form of liposomes. As is known in the art, liposomes are generally derived from phospholipids or other lipid substances. Liposomes are formed by mono- or multilamellar hydrated liquid crystals that are dispersed in an aqueous medium. Any nontoxic, physiologically-acceptable and metabolizable lipid capable of forming liposomes can be used. The present compositions in liposome form can contain, in addition to a compound of the present invention, stabilizers, preservatives, excipients, and the like. The preferred lipids are the phospholipids and the phosphatidyl cholines (lecithins), both natural and synthetic.

Methods to form liposomes are known in the art. See, for example, Prescott, Ed., Methods in Cell Biology, Volume XIV, Academic Press, New York, N.Y. (1976), p. 33 et seq.

The compounds of the present invention may be used in the form of pharmaceutically-acceptable salts derived from inorganic or organic acids. By "pharmaceutically-acceptable salt" is meant those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically-acceptable salts are well-known in the art. For example, S. M. Berge, *et al.* Describe pharmaceutically-acceptable salts in detail in J. Pharmaceutical Sciences, 1977, 66: 1 et seq. The salts may be prepared *in situ* during the final isolation and purification of the compounds of the invention or separately by reacting a free base function with a suitable acid. Representative acid addition salts include acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethanesulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quatemized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which may be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

Basic addition salts can be prepared *in situ* during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically-acceptable basic addition salts include cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylamine, dimethylamine, trimethylamine, triethylamine, diethylamine, ethylamine and the like. Other representative organic amines useful for the formation of base addition salts include ethylenediamine, ethanolamine, diethanolamine, piperidine, piperazine and the like.

Dosage forms for topical administration of a compound of this invention include powders, sprays, ointments and inhalants. The active compound is mixed under sterile conditions with a pharmaceutically-acceptable carrier and any needed preservatives, buffers, or propellants which may be required. Opthalmic formulations, eye ointments, powders and solutions are also contemplated as being within the scope of this invention.

Actual dosage levels of active ingredients in the pharmaceutical compositions of this invention may be varied so as to obtain an amount of the active compound(s) that is effective to achieve the desired therapeutic response for a particular patient, compositions, and mode of administration. The selected dosage level will depend upon the activity of the particular compound, the route of administration, the severity of the condition being treated, and the condition and prior medical history of the patient being treated. However, it is within the skill of the art to start doses of the compound at levels lower than required for to achieve the desired therapeutic effect and to gradually increase the dosage until the desired effect is achieved.

Generally dosage levels of about 0.1 to about 50 mg, more preferably of about 5 to about 20 mg of active compound per kilogram of body weight per day are administered orally or intravenously to a mammalian patient. If desired, the effective daily dose may be divided into multiple doses for purposes of administration, e.g. two to four separate doses per day.

### Preparation of Compounds of the Invention

The compounds and processes of the present invention may be better understood in connection with the following synthetic Schemes which illustrate the methods by which the compounds of the invention can be prepared.

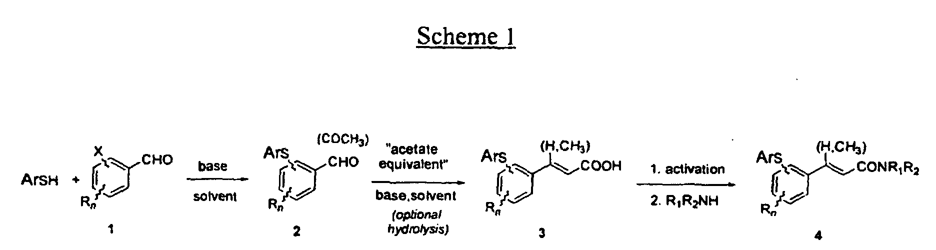

Scheme 1 describes the synthesis of a typical cinnamide-substituted diaryl sulfide **4** through an aldehyde intermediate **2**. Aldehyde 2 is prepared by reaction of a thiophenol (for example 2,4-dichlorothiophenol, 2-bromothiophenol. or the like) with halo-substituted benzaldehyde derivative **1** (e.g. 2-chlorobenzaldehyde, 3-chloro,4-fluorobenzaldehyde, or the like) in the presence of base (e.g. sodium carbonate, triethylamine, or the like) and a polar solvent (e.g. dimethylformamide, dimethylsulfoxide, or the like). The aldehyde group is homologated to the corresponding cinnamic acid **3**, using an acetate equivalent (for example, malonic acid, triethoxyphosphonoacetate, or the like) in the presence of an appropriate base and solvent. In some cases, it may be necessary to hydrolyze an intermediate ester (for example using sodium hydroxide in alcohol). The acid group is activated (for example using thionyl chloride, or dicyclohexylcarbodiimide and N-hydroxysuccinimide, or the like) and reacted with a primary or secondary amine (for example, 6-aminohexanol, pyrrolidone-3-propylamine, or the like) to provide the desired analog **4**. In one variant, a halo-acetophenone can replace benzaldehyde **2**; the resultant cinnamides **4** are substituted with a methyl group at the 3-position.

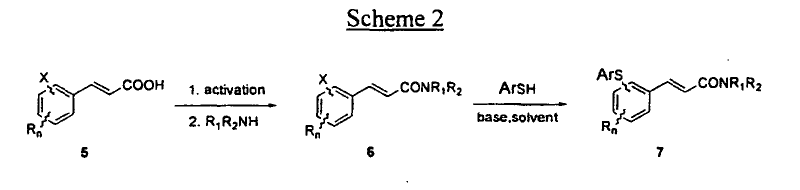

Alternatively, the order of these coupling steps may be reversed (Scheme 2). A substituted halocinnamic acid **5** (e.g. 3-chloro-2-nitrocinnamic acid or the like) may be coupled with a primary or secondary amine (e.g. N-acetylpiperazine or the like) as described above to give the corresponding amide **6**. The halo-group can then be displaced with a substituted thiophenol in the presence of base to provide the product **7**.

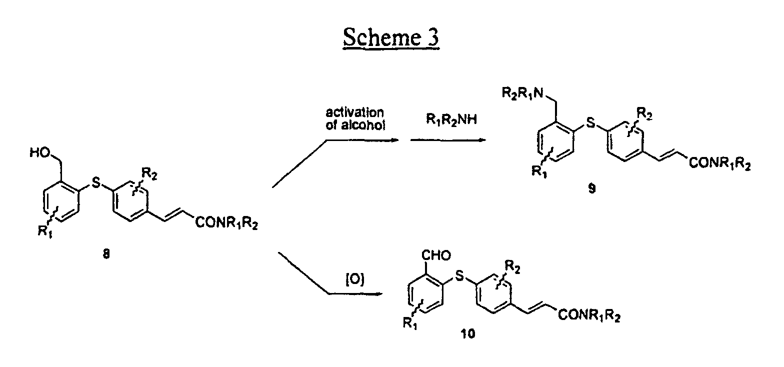

A number of the compounds described herein may be prepared from intermediate benzylic alcohols like **8** (Scheme 3) Activation of the alcohol moiety (for example, using phosphorus tribromide or methanesulfonyl chloride and lithium halide in dimethylformamide) and displacement with a primary or secondary amine (e.g. morpholine, N-formylpiperazine or the like) provides analogs with structures related to **9**. Alternatively the alcohol may be oxidized (for example using TPAP or PCC or the like) to give aldehyde **10**.

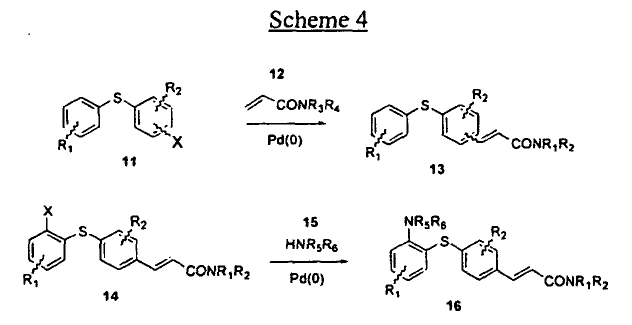

Cinnamides like **13** may be prepared from halo-substituted derivatives **11** by palladium-mediated coupling [e.g. using tetrakis (o-tolyl phosphine) palladium (0), Pd₂(dba)₃, or the like] with acrylamide derivatives **12** (Scheme 4). In similar manner, anilino-cinnamides like **16** can be prepared by palladium-mediated coupling of amines **15** with halo-cinnamides **14**.

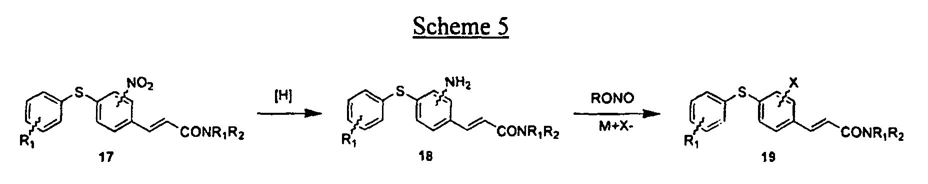

In some cases, functional groups on the aromatic rings can be modified to produce new analogs (Scheme 5). For example, a nitro group in compounds like **17** may be reduced (for example, with tin(II) chloride, or by catalytic hydrogenation, or the like) to the corresponding amine **18**. This amine may then itself be converted to a halogen, for example by diazotization using nitrous acid or t-butyl nitrite in the presence of a metal halide salt like cupric bromide, providing analog **19**.

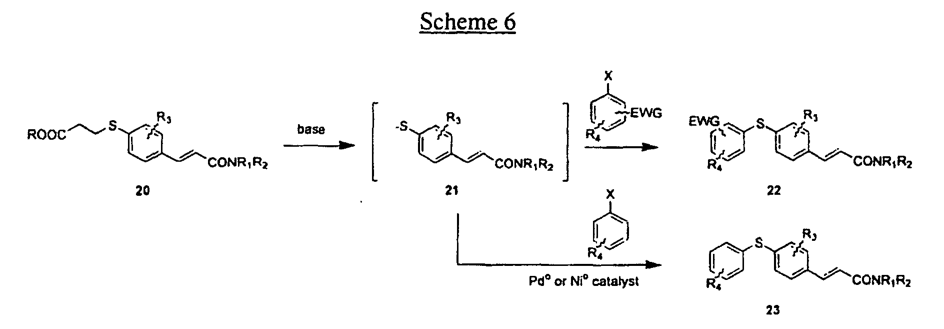

It is also possible to assemble cinnamide-substituted diaryl sulfides in a "reverse" sense (Scheme 6). Thus, for example, compound **20**, prepared as described in Scheme 1, may be deprotected by treatment with base (e.g. potassium t-butoxide or the like) to provide thiolate anion **21**, which may be reacted with an activated haloarene (e.g. 2,3-dichlorobenzaldehyde, 3-chloro,4-fluorobenzaldehyde or the like) to provide the corresponding product **22.** Alternatively, this same thiolate anion may be coupled with unactivated aryl halides (e.g. aryl bromide or Aryl iodides) using a metal-catalyzed Ullman coupling procedure (for example, using a palladium or nickel catalyst) to give product **23**.

A further method for producing diarylsulfide cinnamides is shown in Scheme 7, wherein the diaryl sulfide is formed through coupling of a suitably protected aryl thiol **28** to an activated cinnamate ester **27**. Substituted phenol **24** may be brominated to give bromophenol **25**. Heck-type coupling of bromide **25** with an appropriate olefinic substrate, for example methyl acrylate, is effected with palladium catalysis, leading to the cinnamate ester **26**. The phenol is then activated towards further reaction, for example by conversion to the corresponding triflate **27** under standard conditions. The required protected thiol **28** may be prepared by the method of XXX (Tetrahedron *Lett*. **1994**, *35*, 3221-3224), by coupling an aryl halide or triflate with triisopropylsilyl thiol under palladium catalysis. The two partners **27** and **28** are then reacted in the presence of a fluoride source, for example cesium fluoride, to provide the diarylsulfide cinnamate **29**. Hydrolysis is accomplished by basic media. such as lithium or sodium hydroxide in water-THF, and the resulting acid **30** is coupled to amines under standard amide-bond forming conditions (for example, EDC/HOBt) to produce the amides **31**.

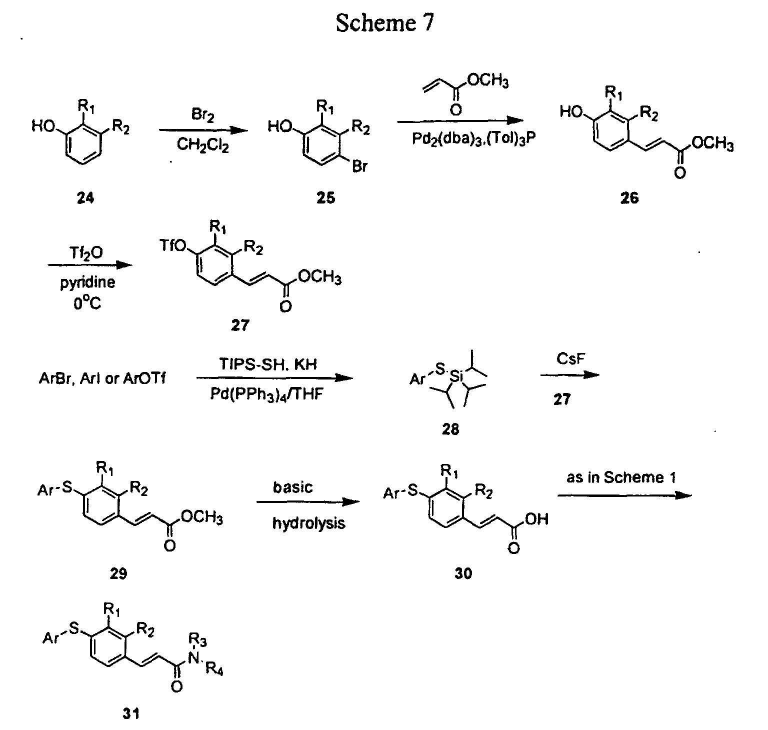

A method for preparing cinnamides bearing two arylthio groups is outlined in Scheme 8. Commercially available difluoro cinnamic acid **32** was coupled with an amine, using standard conditions, and this derived amide **33** was reacted with excess aryl thiol to provide the bis-sulfide **34**.

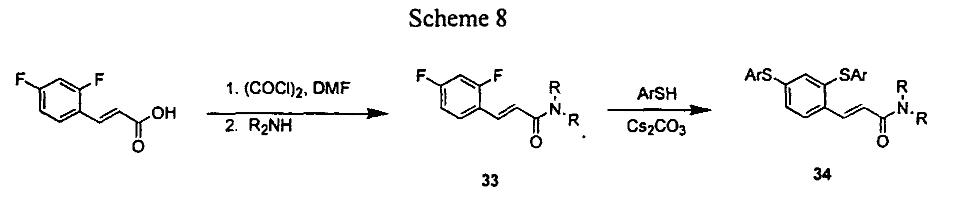

Compounds which contain trifluoromethyl groups on the cinnamide-portion of inhibitors were made by the method shown in Scheme 9. According to the method of XXX (Ref), Diels-Alder reaction between 1,1,1,4,4,4-hexafluoro-2-butyne and 2-methylfuran led to bicyclic ether **35,** which was rearranged with Lewis acid (for example, boron trifluoride etherate) to the phenol **36**. The methyl group is then converted to the corresponding aldehyde **37** by bromination followed by reaction with dimethylsulfoxide. Using the analogous procedures described for Scheme 1 above, the phenol was activated and condensed with thiols under basic conditions to afford diarylsulfide aldehydes **38**, and further converted to cinnamides **39** by the previously described procedures.

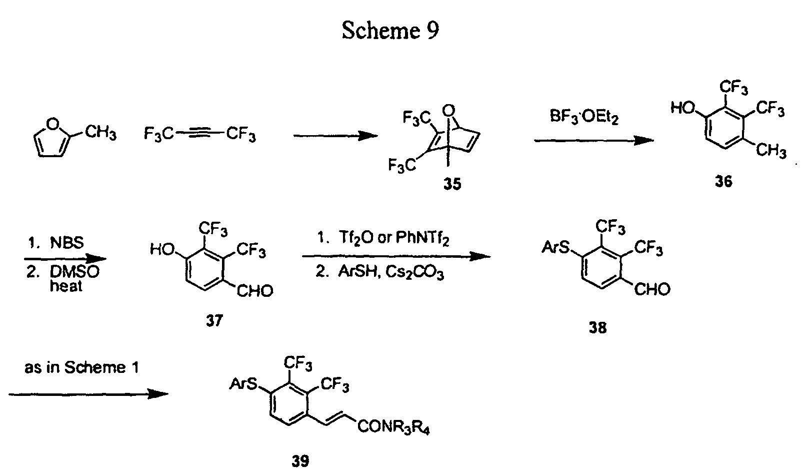

Cinnamides bearing more complex substituted piperidine amides can be produced by the methods outlined in Scheme 10 and 11. Cinnamic acids **40** are coupled to spiro-hydantoin piperidine **41**, and the derived amide **42** is first reacted with an activating reagent (for example di-tert-butyl dicarbonate), and then hydrolyzed to the amino acid **43**. The derived amino group may then be reacted further, for example with acid anhydrides or acid chlorides, to produce amides **44**.

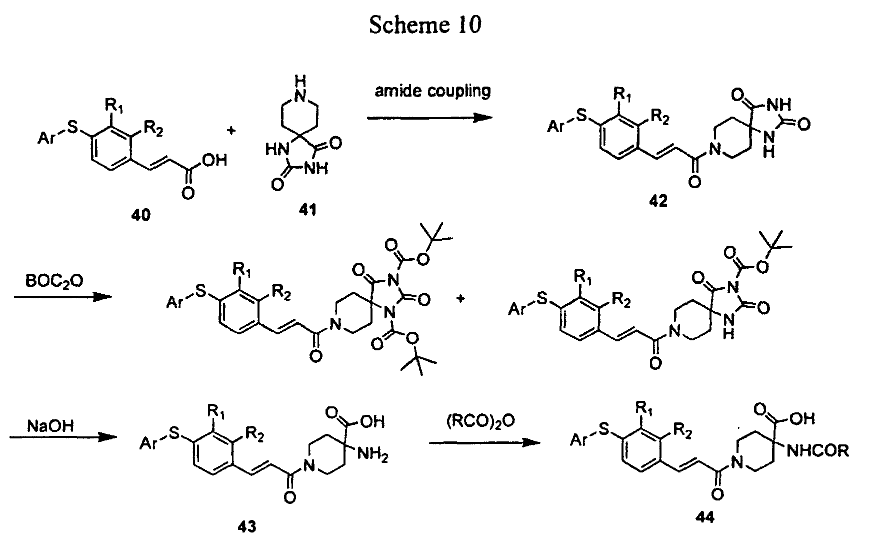

Further derivatives of piperidine amides can be obtained by coupling of piperidinone **45** with cinnamic acids **40,** as shown in Scheme 11. Standard coupling conditions lead to amide **46**, which is first reduced to the corresponding alcohol, then hydrolyzed to afford hydroxy acid **47**.

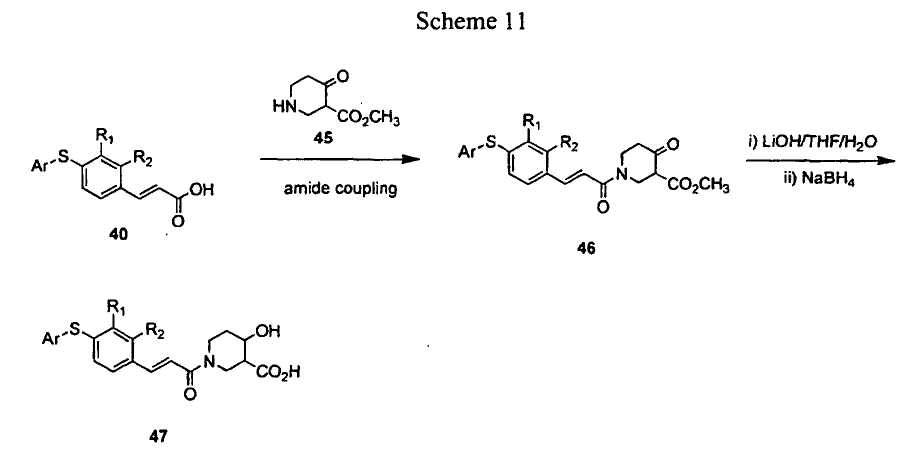

Also included in this invention are compounds derived from coupling of amines, or amino acid derivatives (such as a-amino esters) to the carboxylic acid group of cinnamides **48,** using standard coupling and hydrolysis methods, as outlined in Scheme 12. Thus, amides **49** are produced directly from amine coupling reactions. Amino acid esters are coupled to **48**, and the derived esters are hydrolyzed to the corresponding acids **50**.

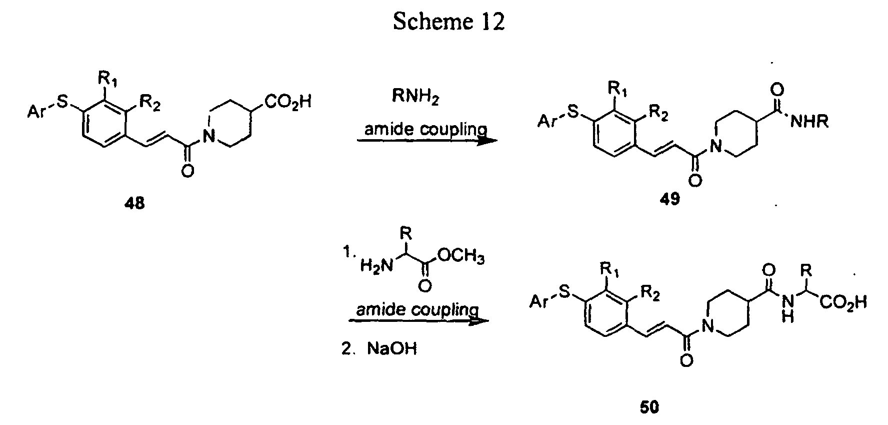

Inhibitors bearing substituted piperazine (or homopiperazine) cinnamides may be produced by the methods described in Scheme 13. The methods described may be utilized to produce piperazine amide **51**. Secondary amine **51** then serves as educt for preparing amides **52**, through standard coupling reactions. Alternatively, **51** may be converted to tertiary amines **53**, through standard reductive alkylation methods (for example, condensation with an aldehyde in the presence of a reducing agent such as sodium triacetoxyborohydride).

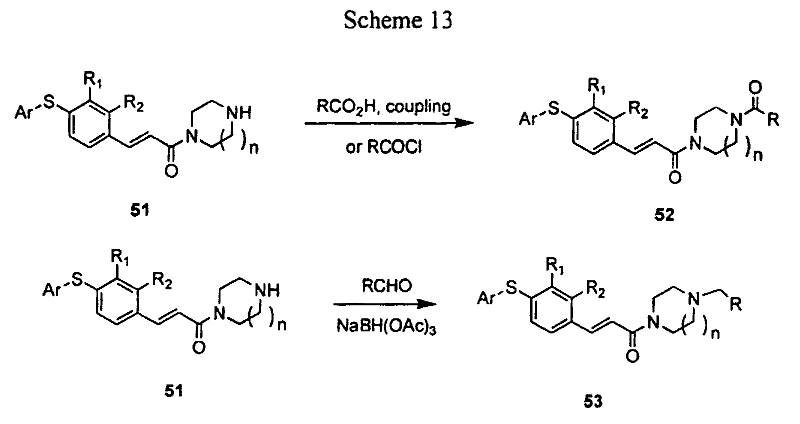

A process for preparing analogs with amino substitutions of the aryl portion of the sulfides is illustrated in Scheme 14. The intermediate triflate **27** is reacted with halo-substituted thiophenols **54** (X = Br, Cl, OTf, OTs) under basic catalysis, to provide the sulfide derivative **55**. The halogen or activated hydroxyl is then substituted with an amine, using the method of Buchwald (Old, D. W.; Wolfe, J. P.; Buchwald, S. L. *J. Am. Chem. Soc.* **1998,** *120*, 9722-9723). Similar transition-metal catalyzed reactions may be applied, for example, the method of Hartwig {Hamann, B. C.; Hartwig, J. F. *J. Am. Chem. Soc.* **1998,** *120*, 7369-7370). The NR₃R₄ group may constitute a cyclic or acyclic group, optionally substituted with additional functionalities that may enhance the activities of the compounds, and that further synthetic transformations familiar to those skilled in the art may be applied. For instance, ester groups may be hydrolyzed to the corresponding carboxylic acids or amides. The derived anilino sulfides may then be processed as described above to produce the cinnamides **56**.

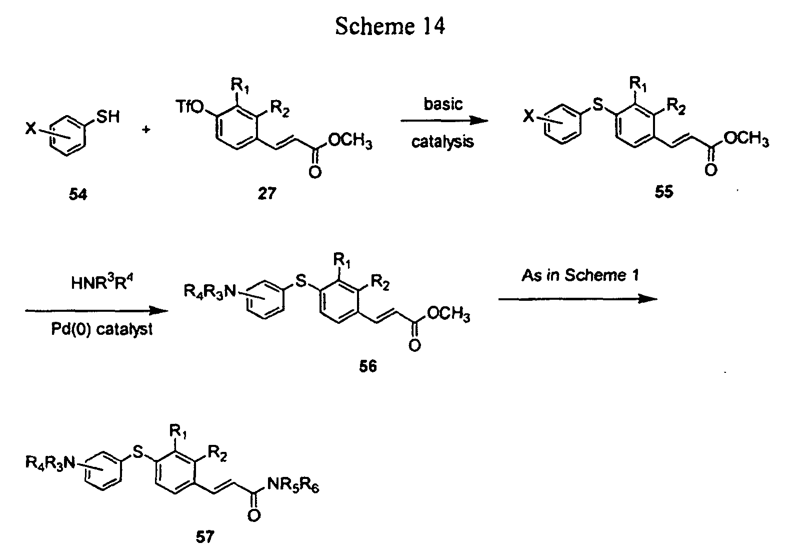

### EXAMPLES

The compounds and processes of the present invention may be better understood in connection with the following Examples, which are intended as an illustration of and not a limitation upon the scope of the invention.

### Example 1

### (2,4-Dichlorophenyl)[2-(E-((6-hydroxyhexylamino)carbonyl)ethenyl)phenyl]sulfide

### Example 1A

### 2-[(2,4-Dichlorophenyl)thio]benzaldehyde

To a stirred solution of 2,4-dichlorothiophenol (2.0 g, 11.2 mmol) in 25 mL of anhydrous DMF was added potassium carbonate (3.09 g, 22.4 mmol), followed by 2-chlorobenzaldehyde (1.26 mL, 11.3 mmol). The mixture was then heated under nitrogen atmosphere at 70 °C for 5 hours. The reaction mixture was then allowed to cool to room temperature and partitioned between ether and water. The aqueous layer was extracted with ether once and the combined organic layer was washed with water and brine, dried over sodium sulfate and condensed in vacuo. The crude product was purified via silica gel flash chromatography, eluting with 5-10 % ether/hexanes, to give 2.62 g (9.25 mmol, 83%) of the desired aldehyde as a colorless oil, which solidified slowly upon standing at room temperature.

### Example 1B

### trans-2-[(2,4-Dichlorophenyl)thio]cinnamic acid

A mixture of the aldehyde (1.50 g, 5.3 mmol) from Example 1A, malonic acid (1.21 g, 11.6 mmol), piperidine (78.6 µL, 0.80 mmol) in 8.0 mL of anhydrous pyridine was heated at 110 °C for 2 hours. Gas evolution ceased during this period. Pyridine was then removed under vacuum. Water and 3N aq. HCI were then added with stirring. The desired cinnamic acid was then collected through filtration, washed with cold water and dried in a vacuum oven overnight to give 1.56 g (4.8 mmol, 91 %) of white solid.

### Example 1C

### (2,4-Dichlorophenyl)[2-(E-((6-hydroxyhexylamino)carbonyl)ethenyl)phenyl]sulfide

A suspension of the acid (284 mg, 0.87 mmol) from Example 1B in 5 mL of methylene chloride was stirred with (COCl)₂ (84 µL, 0.97 mmol), and one drop of DMF under nitrogen atmosphere for 90 minutes. The solvent was then removed under vacuum. The residue (COCl)₂ was removed with benzene (2x) in vacuo. To a separate flask, previously filled with 6-amino-1-hexanol (12 mg, 0.10 mmol), Hunig's base (22.8 µL, 0.13 mmol) and DMAP (1.1 mg, 0.008 mmol) in 2.0 mL of CH₂Cl₂, the acid chloride (30 mg, 0.087 mmol) in 1.0 mL of CH₂Cl₂ was then dropped in slowly. After 30 minutes, the reaction mixture was poured into 3N HCl and extracted with ethyl aceetate (EtOAc). The organic layer was washed with brine, dried with Na₂SO₄, condensed under reduced pressure. The crude product was purified by preparative TLC to give 21.0 mg (90 %) of the title compound as a colorless oil. ¹H NMR (CDCl₃, 300 MHz) δ 1.31-1.48 (m, 4H), 1.48-1.70 (m, 4H), 3.37 (q, *J* = 6.7 Hz, 2H), 3.65 (t, *J* = 6.3 Hz, 2H), 5.63 (br s, 1H), 6.36 (d, *J* = 15.9 Hz, 1H), 6.71 (d. *J* = 9.3 Hz, 1H), 7.05 *(*dd, *J* = 2.4, 8.7 Hz, 1H), 7.31-7.49 (m, 4H), 7.65 (dd, *J* = 2.1, 7.5 Hz, 1H), 7.99 (d, *J* = 15.9 Hz, 1H). MS (DCI/NH₃) (M+NH₄)⁺ at *m*/*z* 441, 443, 445.

### Example 2

### (2,4-Dichlorophenyl)[2-(E-((3-(1-imidazolyl)propylamino)carbonyl)ethenyl)phenyl]sulfide

The title compound was prepared by the procedures described in Example 1C substituting 6-amino-1-hexanol with 1-(3-aminopropyl)imidazole. White powder; ¹H NMR (d⁶-DMSO, 300 MHz) δ 1.88 (p, *J* = 7.7 Hz, 2H), 3.11 (q, *J* = 7.7 Hz, 2H), 3.97 (t, *J* = 7.7 Hz, 2H), 6.63 (d, *J* = 15.9 Hz, 1H), 6.70 (d, *J* = 8.7 Hz, 1H), 6.89 (d. *J* = 0.9 Hz, 1H), 7.17 *(*d, *J* = 0.9 Hz, 1H), 7.33 (dd, *J* = 2.7. 8.7 Hz, 1H), 7.46-7.65 (m. 4H), 7.72 (d, *J* = 2.7 Hz, 1H), 7.78 (d, *J* = 15.9 Hz, 1H), 7.80 (d, *J* = 8.7 Hz, 1H), 8.24 (t, *J* = 5.9 Hz, 1H). MS (DCI/NH₃) (M+H)⁻ at *m*/*z* 448. 450, 452. Analysis calculated for C₂₁H₁₉N₃O₁Cl₂S₁·0.87 H,O: C, 56.30; H, 4.67; N. 9.38. Found: C, 56.30; H, 4.56; N, 9.27.

### Example 3

### (2,4-Dichlorophenyl)[2-chloro-4-(E-((2-hydroxyethylamino)carbonyl)ethenyl)phenyl]sulfide

The title compound was prepared by the procedures described in Example I substituting 2-chlorobenzaldehyde with 3-chloro-4-fluoro-benzadehyde, and 6-amino-1-hexanol with ethanolamine. Colorless oil; ¹H NMR (CDCl₃, 300 MHz) δ 3.57 (q, *J* = 7.65 Hz, 2H), 3.71 (q, *J* = 7.65 Hz, 2H), 6.06 (br s, 1H), 6.40 (d, *J* = 15.3 Hz, 1H), 6.96 (d, *J* = 8.7 Hz, 1H), 7.22-7.30 (m, 4H), 7.49-7.60 (m, 1H), 7.55 (d, *J* = 15.3 Hz,1H). MS (APCI) (M+H)⁺ at *m*/*z* 402, 404, 406, 408. Analysis calculated for C₁₇H₁₄N₁O₂Cl₃S₁·0.25H₂O: C, 50.14; H, 3.59; N, 3.44. Found: C, 50.16; H. 3.62; N, 3.29.

### Example 4

### (2,4-Dichlorophenyl)[2-chloro-4-(E-((6-hydroxyhexylamino)carbonyl)ethenyl)phenyl]sulfide

The title compound was prepared by the procedures described in Example 1 substituting 2-chlorobenzaldehyde with 3-chloro-4-fluoro-benzadehyde. Colorless oil; ¹H NMR (CDCl₃, 300 MHz) δ 1.42 (m, 4H), 1.58 (m, 4H), 3.40 (q, J = 6.7 Hz, 2H), 3.65 (br m, 2H), 5.60 (br t, 1H), 6.35 (d, J = 15.3 Hz, 1H), 6.98 (d, *J* = 8.7 Hz, 1H), 7.22-7.30 (m, 4H), 7.49-7.60 (m, 1H), 7.55 (d, J = 15.3 Hz, 1H). MS (APCI) (M+H)⁻ at m/z 458, 460, 462, 464. Analysis calculated for C₂₁H₂₂N₁O₂Cl₃S₁·0.27H₂O: C. 54.39; H, 4.90; N, 3.02. Found: C, 54.40; H, 4.85; N, 2.71.

### Example 5

### (2,4-Dichlorophenyl)[2-chloro-4-(E-((bis-(2-hydroxyethyl)amino)carbonyl)ethenyl)phenyl]sulfide

The title compound was prepared by the procedures described in Example 1 substituting 2-chlorobenzaldehyde with 3-chloro-4-fluoro-benzadehyde, and 6-amino-1-hexanol with diethanolamine. Colorless oil; ¹H NMR (CDCl₃, 300 MHz) δ 2.99 (br s, 2H), 3.67 (br m, 4H), 3.88 (t, *J* = 5.1 Hz, 2H), 3.94 (t, *J* = 5.1 Hz, 2H), 6.94 (d, *J* = 15.3 Hz, 1H), 6.97 (d, *J* = 8.7 Hz, 1H), 7.21-7.32 (m, 3H), 7.50-7.54 (m, 1H), 7.58 (d, *J =* 2.4 Hz, 1H), 7.58 (d, *J* = 15.3 Hz, 1H). MS (APCI) (M+H)⁺ at *m*/*z* 446, 448, 450, 452. Analysis calculated for C₁₉H₁₈N₁O₃Cl₃S₁·1.09H₂O: C, 48.93; H, 4.36; N, 3.00. Found: C, 48.88; H, 4.00; N, 3.01.

### Example 6

### (2,4-Dichlorophenyl)[2-chloro-4-(E-((3-(1-pyrrolidin-2-only)propylamino)carbonyl)ethenyl)phenyl]sulfide

The title compound was prepared by the procedures described in Example 1 substituting 2-chlorobenzaldehyde with 3-chloro-4-fluoro-benzadehyde, and 6-amino-1-hexanol with 1-(3-aminopropyl)-2-pyrrolidinone. Colorless oil; ¹H NMR (CDCl₃, 300 MHz) δ 1.74 (qu, *J =* 6.0 Hz, 2H), 2.09 (qu, *J =* 7.5 Hz, 2H), 2.45 (t, *J =* 8.25 Hz, 2H), 3.33 *(q, J =* 6.0 Hz, 2H), 3.42 (q. *J* = 8.25 Hz, 4H), 6.46 (d, *J* = 15.6 Hz, 1H), 7.02 *(d, J =* 8.7 Hz, 1H), 7.14-7.23 (m, 2H), 7.30 *(dd, J =* 2.4, 8.7 Hz, 1H), 7.51 *(d, J* = 2.4 Hz, 1H), 7.51 (d, *J* = 15.6 Hz, 1H), 7.60 (d, *J* = 2.1 Hz, 1H). MS (DCl/NH₃) (M+H)⁺ at m/z 483, 485, 487, 489. Analysis calculated for C₂₂H₂₁N₂O₂Cl₃S₁·0.57H₂O: C, 53.48; H, 4.52; N, 5.67. Found: C, 53.49; H, 4.60; N, 5.65.

### Example 7

### (2,4-Dichlorophenyl)[2-chloro-4-(E-((1-morpholinyl)carbonyl)ethenyl)phenyl]sulfide

The title compound was prepared by the procedures described in Example 1 substituting 2-chlorobenzaldehyde with 3-chloro-4-fluoro-benzadehyde, and 6-amino-1-hexanol with morpholine. White solid; ¹H NMR (CDCl₃, 300 MHz) δ 3.59-3.80 (m, 8H), 6.83 (d, *J* = 15.6 Hz, 1H), 6.97 (d, *J* = 8.7 Hz, 1H), 7.16-7.32 (m, 3H), 7.49-7.53 (m, 1H), 7.59 (d, *J* = 2.4 Hz, 1H), 7.59 (d, *J* = 15.6 Hz, 1H). MS (DCI/NH₃) (M+H)⁺ at *m*/*z* 428, 430, 432, 434. Analysis calculated for C₁₉H₁₆N₁O₂Cl₃S₁·0.46H₂O: C, 52.22; H, 3.90; N, 3.20. Found: C, 52.20; H, 3.76; N, 3.12.

### Example 8

### (2,4-Dichlorophenyl)[2-chloro-4-(E-((4-methylpiperazin-1-yl)carbonyl)ethenyl)phenyl]sulfide

The title compound was prepared by the procedures described in Example 1 substituting 2-chlorobenzaldehyde with 3-chloro-4-fluoro-benzadehyde, and 6-amino-1-hexanol with 1-methylpiperazine. Colorless oil; ¹H NMR (CDCl₃, 300 MHz) δ 2.37 (s, 3H), 2.51 (br m, 4H), 3.63-3.87 (br m, 4H), 6.85 (d, J = 15.6 Hz, 1H), 6.98 (d, J = 8.7 Hz, 1H), 7.19-7.25 (m, 2H), 7.27 (dd, *J* = 2.1, 8.7 Hz, 1H). 7.52 (t, J = 0.9 Hz, 1H), 7.57 (d, *J* = 15.6 Hz, 1H), 7.60 (d, *J* = 2.1 Hz, 1H). MS (DCI/NH₃) (M+H)⁺ at *m*/*z* 441, 443, 445, 447. Analysis calculated for C₂₀H₁₉N₂O₁Cl₃S₁·0.45H₂O: C, 53.39; H, 4.46; N, 6.23. Found: C, 53.37; H, 4.46; N, 6.07.

### Example 9

### (2,4-Dichlorophenyl)[2-chloro-4-(E-((4-acetylpiperazin-1-yl)carbonyl)ethenyl)phenyl]sulfide

The title compound was prepared by the procedures described in Example I substituting 2-chlorobenzaldehyde with 3-chloro-4-fluoro-benzadehyde, and 6-amino-1-hexanol with 1-acetylpiperazine. White solid; ¹H NMR (CDCl₃, 300 MHz) δ 2.15 (s, 3H), 3.50-3.58 (m, 2H), 3.58-3.85 (m, 6H), 6.85 (d, *J* = 15.3 Hz, 1H), 6.96 (d, *J* = 8.7 Hz, 1H), 7.24-7.36 (m, 3H), 7.54 (d, *J*= 2.4 Hz, 1H), 7.61 (d, *J* = 15.3 Hz, 1H), 7.61 (d, *J* = 2.1 Hz, 1H). MS (DCI/NH₃) (M+H)⁻ at *m*/*z* 486, 488, 490, 492. Analysis calculated for C₂₁H₁₉N₂O₂Cl₃S₁·0.85H₂O: C, 51.99; H, 4.30; N, 5.77. Found: C, 52.03; H, 4.27; N, 5.67.

### Example 10

### (2,4-Dichlorophenyl)[2-chloro-4-(E-((4-(2-pyridyl)piperazin-1-yl)carbonyl)ethenyl)phenyl]sulfide

The title compound was prepared by the procedures described in Example I substituting 2-chlorobenzaldehyde with 3-chloro-4-fluoro-benzadehyde, and 6-amino-1-hexanol with 1-(2-pyridyl)piperazine. White solid; ¹H NMR (CDCl₃, 300 MHz) δ 3.59 (br m, 2H), 3.69 (br m, 2H), 3.78 (br m, 2H), 3.86 (br m, 2H), 6.64-6.72 (m, 2H), 6.90 (d, *J* = 15.6 Hz, 1H), 6.99 (d, *J* = 8.7 Hz, 1H), 7.22-7.25 (m, 2H), 7.31 (dd, *J* = 2.4, 8.7 Hz, 1H), 7.49-7.57 (m, 2H), 7.61 (d, *J* = 15.6 Hz, 1H), 7.62 (d, *J* = 2.4 Hz, 1H), 8.19-8.24 (m, 1H). MS (DCI/NH₃) (M+H)⁺ at *m*/*z* 504, 506, 508, 510. Analysis calculated for C₂₄H₂₀N₃O₁Cl₃S₁: C, 57.10; H, 3.99; N, 8.32. Found: C, 57.12; H, 4.06; N, 8.29.

### Example 11

### (2-(Hydroxymethyl)phenyl)[2-chloro-4-(E-((1-morpholinyl)carbonyl) ethenyl)phenyl]sulfide

The title compound was prepared by the procedures described in Example 1 substituting 2,4-dichlorothiophenol with 2-mercaptobenzyl alcohol, 2-chlorobenzaldehyde with 3-chloro-4-fluoro-benzadehyde, and 6-amino-1-hexanol with morpholine. White solid; ¹H NMR (CDCl₃, 300 MHz) δ 3.50-3.62 (br m, 6H), 3.65-3.74 (br m, 2H), 4.54 (d, *J* = 5.7 Hz, 2H), 5.33 (t, *J* = 5.7 Hz, 1H), 6.62 (d, *J* = 8.7 Hz, 1H), 7.28 (d, *J* = 15.0 Hz, 1H), 7.36 (d, *J* = 7.8 Hz, 1H), 7.42 (d, *J* = 15.0 Hz, 1H), 7.43 (dd, *J* = 1.8, 8.7 Hz, 1H), 7.50 (dd, *J* = 2.1, 8.7 Hz, 1H), 7.55 (dd. *J* = 2.1, 7.8 Hz, 1H), 7.68 (dd, *J* = 1.5, 8.1 Hz, 1H), 8.02 (d, *J* = 2.1 Hz, 1H). M S (DCI/NH₃) (M+H)⁺ at m/z 390, 392. Analysis calculated for C₂₀H₂₀N₁O₃Cl₁S₁ ·0.09H₂O: C, 61.35; H, 5.20; N, 3.58. Found: C, 61.37; H, 5.48; N, 3.81.

### Example 12

### (2-Bromophenyl)[2-chloro-4-(E-((1-morpholinyl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with morpholine. White solid; ¹H NMR (d⁶-DMSO, 300 MHz) δ 3.50-3.66 (br m, 6H), 3.66-3.79 (br m, 2H), 7.05 (d, *J* = 8.7 Hz, 1H), 7.26 (dd, *J* = 2.1, 8.1 Hz, 1H), 7.33 (dd, *J* = 2.1, 8.1 Hz, 1H), 7.36 (d, *J* = 15.6 Hz, 1H), 7.39 (dd, *J*= 1.8, 12.0 Hz, 1H), 7.45 (dd, *J* = 1.8, 6.3 Hz, 1H), 7.48 (d, *J* = 15.6 Hz,1H), 7.64 (dd, *J* = 2.1, 8.7 Hz, 1H), 7.80 (dd, *J* = 2.8, 8.7 Hz, 1H), 8.09 (d, *J* = 2.1 Hz, 1H). MS (DCI/NH₃) (M+H)⁺ at *m*/*z* 438, 440, 442.

### Example 13

### (2,4-Dichlorophenyl)[2-chloro-4-(E-((4-(2-hydroxyethyl)piperazin-1-yl)carbonyl)ethenyl)phenyl]sulfide

The title compound was prepared by the procedures described in Example 1 substituting 2-chlorobenzaldehyde with 3-chloro-4-fluoro-benzadehyde, and 6-amino-1-hexanol with 1-hydroxyethylpiperazine. Colorless oil; ¹H NMR (CDCl₃, 300 MHz) δ 2.85-3.20 (br m, 6H), 3.84-4.19 (m, 6H), 6.80 (d, *J* = 15.3 Hz, 1H), 6.94 (d, *J* = 8.7 Hz, 1H), 7.22-7.38 (m, 3H), 7.50-7.56 (m, 1H), 7.56-7.62 (m, 1H), 7.60 (d. *J* = 15.3 Hz, 1H). MS (DCI/NH₃) (M+H)⁺ at *m*/*z* 471, 473, 475, 477.

### Example 14

### (2,4-Dichlorophenyl)[2-chloro-4-(E-((4-(2-hydroxyethoxyethyl)piperazin-1-yl)carbonyl)ethenyl)phenyl]sulfide

The title compound was prepared by the procedures described in Example 1 substituting 2-chlorobenzaldehyde with 3-chloro-4-fluoro-benzadehyde, and 6-amino-1-hexanol with 1-[2-(2-hydroxyethoxy)ethyl]piperazine. Colorless oil; ¹H NMR (CDCl₃, 300 MHz) δ 2.73 (br m, 6H), 3.58-3.68 (m, 2H), 3.68-4.00 (m, 8H), 6.84 (d, *J* = 15.3 Hz, 1H), 6.97 (d, *J* = 8.7 Hz, 1H), 7.20-7.34 (m, 3H), 7.54 (d, *J* = 7.5 Hz, 1H), 7.58 (d, *J* = 15.3 Hz, 1H), 7.58-7.65 (overlapping d, 1H). MS (DCI/NH₃) (M+H)⁺ at *m*/*z* 515, 517, 519, 521.

### Example 15

### (2-Bromophenyl)[2-chloro-4-(E-((3-(hydroxymethyl)piperidin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with 3-hydroxymethylpiperidine. ¹H NMR (DMSO-d₆, 300MHz) δ 8.07 (d, J = 17.7 Hz, 1H), 7.80 (d, J = 7.7 Hz, 1H), 7.63 (br d, J = 7.7 Hz, 1H), 7.44 (d, J = 7.0 Hz, 1H), 7.40 (br s, 2H), 7.35 (m, 1H), 7.25 (dd 7.7, 1.5, 1H), 7.06 (dd, J = 8.1, 2.9, 1H), 4.57 (m, 1H), 4.45 (m, 1H), 4.16 (br m, 2H), 1.2 - 1.8 (m, 8H). HRMS calculated for C₂₁H₂₁N₁O₂S₁Br₁Cl₁: 466.0243. Observed: 466.0247.

### Example 16

### (2-Bromophenyl)[2-chloro-4-(E-((2-(hydroxymethyl)piperidin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with 2-hydroxymethylpiperidine. ¹H NMR (DMSO-d₆, 300MHz) δ 8.03 (m, 1H), 7.79 (d, *J* = 7.8 Hz, 1H), 7.61 (m, 1H), 7.30 - 7.45 (m, 4H), 7.23 (m, 1H), 7.07 (m, 1H), 4.79 (m, 2H), 4.61 (m, 2H), 4.10 (m, 1H), 1.50 (m, 6H). HRMS calculated for C₂₁H₂₁N₁O₂S₁Br₁Cl₁: 466.0243. Observed: 466.0247.

### Example 17

### (2-Bromophenyl)[2-chloro-4-(E-((3-acetamidopyrrolidin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with 3-acetamidopyrrolidine. ¹H NMR (DMSO-d₆, 300MHz) δ 8.14 (m, 1H), 8.07 (dd, *J* = 9.8, 1.7 Hz, 1H), 7.80 (d, *J* = 7.8 Hz, 1H), 7.64 (dd, *J* = 8.1, 1.7 Hz, 1H), 7.25 - 7.47 (m, 4H), 7.10 (t, *J* = 7.8 Hz, 1H), 7.03 (dd, *J* = 8.1, 1.7 Hz, 1H), 3.45 - 4.34 (m, 6H), 2.02 (m, 2H), 1.81 (ap d, *J* = 1.4 Hz, 1H). HRMS calculated for C₂₁H₂₀N₂O₂S₁Br₁Cl₁: 479.0196. Observed: 479.0183.

### Example 18

### (2-Bromophenyl)[2-chloro-4-(E-((4-hydroxypiperidin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with 4-hydroxypiperidine. ¹H NMR (DMSO-d₆, 300MHz) δ 8.08 (d, *J* = 1.7 Hz, 1H), 7.80 (dd, *J* = 8.0, 1.5 Hz, 1H), 7.63 (dd, *J* = 8.3, 1.9 Hz, 1H), 7.44 (ap dd, *J* = 7.5. 1.4 Hz, 2H), 7.40 (ap d, *J* = 3.7 Hz, 2H), 7.34 (dt, *J* = 7.6, 1.8 Hz, 1H), 7.25 (dd, *J* = 7.5,1.7 Hz 1H), 7.05 (d, *J* = 8. 1 Hz, 1H), 4.76 (br s, 1H), 4.01 (m, 2H), 3.72 (m, 1H), 3.12 (m, 1H). 1.75 (m, 2H), 1.32 (m, 2H), HRMS calculated for C₂₀H₁₉N₁O₂S₁Br₁Cl₁: 452.0087. Observed: 452.0076.

### Example 19

### (2-Bromophenyl)[2-chloro-4-(E-((piperidin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with piperidine. ¹H NMR (DMSO-d₆, 300MHz) δ 8.08 (d, *J* = 1.7 Hz, 1H), 7.80 (dd, *J* = 8.1, 1.4 Hz, 1H), 7.63 (dd, *J* = 8.1, 1.7 Hz, 1H), 7.44 (ap dd, *J* = 7.6, 1.5 Hz, 1H), 7.39 (ap d. *J* = 4.8 Hz, 2H), 7.34 (dt, *J* = 7.5, 1.6, 1H), 7.24 (dd, *J* = 7.5, 1.7, 1H), 7.05 (d, *J* = 8.1 Hz. 1H), 3.65 (br m, 2H), 3.53 (br m, 2H), 1.62 (br m, 2H), 1.50 (br m, 4H). HRMS calculated for C₂₀H₁₉N₁O₁S₁Br₁Cl₁: 436.0130. Observed: 436.0122.

### Example 20

### (2,4-Dichlorophenyl)[2-chloro-4-(E-((3-carboxypiperidin-1-yl)carbonyl)ethenyl)phenyl]sulfide

The title compound was prepared by the procedures described in Example I substituting 2-chlorobenzaldehyde with 3-chloro-4-fluoro-benzadehyde, and 6-amino-1-hexanol with nipecotic acid. Colorless oil; ¹H NMR (CDCl₃, 300 MHz) δ 1.44-1.68 (br m, 1H), 1.68-2.00 (br m, 2H), 2.51-2.67 (br m, 1H), 3.13-3.37 (br m, 1H), 3.80-4.12 (br m, 1H), 4.30-5.00 (br m, 3H), 6.86 (d, *J* = 15.3 Hz, 1H), 6.99 (d, *J* = 8.7 Hz, 1H), 7.16-7.24 (m, 2H), 7.29 (d, *J* = 8.7 Hz, 1H), 7.47-7.55 (m, 1H), 7.55 (d, I= 15.3 Hz, 1H), 7.60 (br d, 1H). MS (APCI) (M+H)⁺ at *m*/*z* 470, 472, 474, 476.

### Example 21

### (2,4-Dichlorophenyl)[2-chloro-4-(E-((4-carboxypiperidin-1-yl)carbonyl)ethenyl)phenyl]sulfide

The title compound was prepared by the procedures described in Example 1 substituting 2-chlorobenzaldehyde with 3-chloro-4-fluoro-benzadehyde, and 6-amino-I-hexanol with isonipecotic acid. Colorless oil; ¹H NMR (CDCl₃, 300 MHz) δ 1.68-1.85 (m, 2H), 1.98-2.09 (m, 2H), 2.60-2.72 (m, 1H), 2.90-3.13 (br m, 1H), 3.17-3.38 (br m, 1H), 3.93-4.12 (br m, 1H), 4.38-4.59 (br m, 1H), 6.86 (d, *J* = 15.3 Hz, 1H), 6.99 (dd, *J* = 8.7 Hz, 1H), 7.20-7.25 (m, 2H), 7.28 (dd, *J* = 1.8, 8.7 Hz, 1H), 7.49-7.53 (m, 1H), 7.56 (d, *J* = 15.3 Hz, 1H), 7.60 (d, *J* = 1.8 Hz, 1H). MS (APCI) (M+H)⁺ at *m*/*z* 470, 472, 474, 476.

### Example 22

### (2-Bromophenyl)[2-chloro-4-(E-((4-acetylhomopiperazin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with 4-acetylhomopiperazine. ¹H NMR (DMSO-d₆, 300MHz) δ 8.10 (m, 1H), 7.81 (d, *J* = 7.7 Hz, 1H), 7.64 (m, 1H), 7.24 - 7.51 (m, 5H), 7.05 (m, 1H), 3.39 - 3.77 (m, 8H), 1.97 (m, 3H), 1.68 (m, 2H). HRMS calculated for C₂₂H₂₂N₂O₂S₁Br₁Cl₁: 493.0352. Observed: 493.0352.

### Example 23

### (2-Bromophenyl)[2-chloro-4-(E-((thiomorpholin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with thiomorpholine. ¹H NMR (DMSO-d₆, 300MHz) δ 8.10 (d, *J* = 1.5 Hz, 1H), 7.80 (d, *J* = 8.5 Hz, 1H), 7.64 (dd, *J* = 8.1, 1.5 Hz, 1H), 7.31 - 7.48 (m, 4H), 7.36 (m. 1H). 7.26 (dd, *J* = 8.1, 1.8 Hz, 1H), 7.05 (d *J* = 8.1 Hz, 1H), 3.96 (m, 2H), 3.82 (m, 2H), 2.62 (m, 4H). HRMS calculated for C₁₉H₁₇N₁O₁S₂Br₁Cl₁: 455.9681. Observed: 455.9676.

### Example 24

### (2-Bromophenyl)[2-chloro-4-(E-((4-(1-benzimidazol-2-only)piperidin-1-yl)carbonyl) ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with 4-(1-benzimidazol-2-only)piperidine. ¹H NMR (DMSO-d₆, 300MHz) δ 8.14 (d, *J* = 1.5 Hz, 1H), 7.80 (dd, *J* = 7.9, 1.3 Hz, 1H), 7.67 (dd, *J* = 8.1, 1.8 Hz, 1H), 7.48 (ap s, 2H), 7.44 (dt, *J =* 7.5, 1.2, 1H), 7.34 (dt, *J* =7.6, 1.6, 1H), 7.26 (dd, *J* = 7.7, 1.8 Hz. 1H), 7.22 (m, 1H), 7.06 (d, *J* = 8.1, 1H), 6.97 (ap d, *J* = 2.6, 3H), 4.64 (m, 1H), 4.48 (m, 2H), 2.79 (m, 2H), 2.29 (m, 2H), 1.78 (m, 2H). HRMS calculated for C₂₇H₂₃N₃O₂S₁Br₁Cl₁: 568.0461. Observed: 568.0477.

### Example 25

### (2-Bromophenyl)[2-chloro-4-(E-((2-tetrahydroisoquinolinyl)carbonyl)ethenyl)phenyl]sulfide

The title compound was prepared by the procedures described in Example 1 substituting 2,4-dichlorothiophenol with 2-bromothiophenol, 2-chlorobenzaldehyde with 3-chloro-4-fluoro-benzadehyde, and 6-amino-1-hexanol with tetrahydroisoquinoline. ¹H NMR (DMSO-d₆, 300MHz) δ 8.12 (d, *J* = 7.4 Hz, 1H), 7.81 (dd, *J* = 7.7, 1.1 Hz, 1H), 7.67 (dd, *J* = 8.3, 1.3 Hz, 1H), 7.47 (m, 2H), 7.43 *(dd, J* = 7.5, 1.3 Hz, 2H), 7.34 (dt, *J* = 7.6, 1.7 Hz, 1H), 7.27 (d 7.7 Hz, 1H), 7.19 (m, 4H), 7.05 (d, *J* = 8.1 Hz, 1H), 4.92 (s, 1H), 4.72 (s, 1H), 3.95 (t, *J* = 5.9 Hz, 1H), 3.78 (t, *J* = 5.7 Hz, 1H), 2.89 (t, *J*=5.3 Hz, 1H), 2.83 (t, *J*=3.7, 1H). HRMS calculated for C₂₄H₁₉N₁O₂S₁Br₁Cl₁: 484.0138. Observed: 484.0128.

### Example 26

### (2-Methylphenyl)[2-trifluoromethyl-4-(E-((4-acetylpiperazin-1-yl)carbonyl) ethenyl)phenyl]sulfide

The title compound was prepared by the procedures described in Example 1 substituting 2,4-dichlorothiophenol with 2-methylthiophenol, 2-chlorobenzaldehyde with 4-fluoro-3-trifluoromethylbenzadehyde, and 6-amino-1-hexanol with 1-acetylpiperazine. ¹H NMR (CDCl₃, 300MHz) δ 7.79 (s, 1H); 7.63 (d, J = 15.4Hz, 1H); 7.51 (d, J = 6.8 Hz, 1H); 7.41-7.33 (m, 3H); 7.28 (m, 1H); 6.83 (d, J = 15.4 Hz, 1H); 6.79 (d, J = 6.8 Hz, 1H); 3.80-3.60 (m, 6H); 3.57-3.50 (m, 2H); 2.34 (s, 3H); 2.14 (s, 3H). MS (ESI) *m*/*z* 919 (2M+Na)⁺, 897 (2M+H)⁺, 471 (M+Na)⁺, 449 (M+H)⁺.

### Example 27

### (2-Methylphenyl)[2-trifluoromethyl-4-(E-((1-morpholinyl)carbonyl)ethenyl)phenyl]sulfide

The title compound was prepared by the procedures described in Example I substituting 2,4-dichlorothiophenol with 2-methylthiophenol, 2-chlorobenzaldehyde with 4-fluoro-3-trifluoromethylbenzadehyde, and 6-amino-1-hexanol with morpholine. ¹H NMR (CDCl₃, 300MHz) δ 7.79 (s, 1H); 7.63 (d, J = 14.0 Hz, 1H); 7.52 (d, J = 7.6 Hz, 1H); 7.40-7.30 (m, 3H); 7.28 (m, 1H); 6.87 (d, J = 14.0 Hz, 1H); 6.84 (d, J = 7.6 Hz, 1H); 3.73 (br s, 8H); 2.34 (s, 3H). MS (ESI) *m*/*z* 837 (2M+Na)⁺, 815 (2M+H)⁻, 408 (M+H)⁺.

### Example 28

### (2-Methylphenyl)[2-trifluoromethyl-4-(E-((2-(1-morpholinyl)ethylamino)carbonyl)ethenyl)phenyl]sulfide

The title compound was prepared by the procedures described in Example 1 substituting 2,4-dichlorothiophenol with 2-methylthiophenol, 2-chlorobenzaldehyde with 4-fluoro-3-trifluoromethylbenzadehyde, and 6-amino-1-hexanol with 2-(1-morpholinyl)ethylamine. ¹H NMR (CDCl₃, 300MHz) δ 7.80 (s, 1H); 7.56 (d, J = 15.8 Hz, 1H); 7.50 (d, J = 8.1 Hz, 1H); 7.40-7.32 (m, 3H); 7.28 (m, 1H); 6.79 (d. J = 15.8 Hz, 1H); 6.40 (d. J = 8.1 Hz, 1H); 3.75 (t, J = 4.6 Hz, 4H); 3.51 (q. J = 5.5 Hz, 2H), 2.57 (t, J = 5.8 Hz, 2H); 2.55-2.48 (m, 4H); 2.34 (s, 3H). MS (ESI) *m*/*z* 923 (2M+Na)⁺, 473 (M+Na)⁺, 451 (M+H)⁺.

### Example 29

### (2-Methylphenyl)[2-trifluoromethyl-4-(E-((4-phenylpiperazin-1-yl)carbonyl) ethenyl)phenyl]sulfide

The title compound was prepared by the procedures described in Example 1 substituting 2,4-dichlorothiophenol with 2-methylthiophenol, 2-chlorobenzaldehyde with 4-fluoro-3-trifluoromethylbenzadehyde, and 6-amino-1-hexanol with 4-phenylpiperazine. ¹H NMR (CDCl₃, 300MHz) δ 7.81 (s, 1H); 7.64 (d, J = 16.0 Hz, 1H); 7.51 (d, J = 8.2 Hz, 1H); 7.40-7.27 (m, 6H); 6.98-6.90 (m, 4H); 6.80 (d, J = 8.2 Hz, 1H); 3.88 (br s, 4H); 2.23 (br s, 4H); 2.34 (s, 3H). MS (ESI) *m*/*z* 987 (2M+Na)⁺, 965 (2M+H)⁺, 505 (M+Na)⁺, 483 (M+H)⁺, 451.

### Example 30

### (2-Methylphenyl)[2-trifluoromethyl-4-(E-((3-(1-pyrrolidin-2-only)propylamino)carbonyl)ethenyl)phenyl]sulfide

The title compound was prepared by the procedures described in Example I substituting 2,4-dichlorothiophenol with 2-methylthiophenol, 2-chlorobenzaldehyde with 4-fluoro-3-trifluoromethylbenzadehyde, and 6-amino-1-hexanol with 1-pyrrolidin-2-only)propylamine. ¹H NMR (CDCl₃, 300MHz) δ 7.78 (s, 1H); 7.53 (d, J = 15.6 Hz, 1H); 7.49 (d, J = 7.2 Hz, 1H); 7.40-7.33 (m, 3H); 7.14 (m, 1H); 6.80 (d, J = 8.2 Hz, 1H); 6.43 (d, J = 15.6 Hz, 1H); 3.41 (m, 4H); 3.32 (q, J = 6.1 Hz, 2H); 2.43 (t, J = 6.6 Hz, 2H); 2.34 (s, 3H), 2.08 (m, 2H), 1.75 (m, 2H). MS (ESI) *m*/*z* 947 (2M+Na)⁺, 925 (2M+H)⁻, 485 (M+Na)⁺, 463 (M+H)⁺.

### Example 31

### (2-Methylphenyl)[2-trifluoromethyl-4-(E-((cyclopropylamino)carbonyl) ethenyl)phenyl]sulfide

The title compound was prepared by the procedures described in Example 1 substituting 2,4-dichlorothiophenol with 2-methylthiophenol, 2-chlorobenzaldehyde with 4-fluoro-3-trifluoromethylbenzadehyde, and 6-amino-1-hexanol with cyclopropylamine. ¹H NMR (CDCl₃, 300MHz) δ 7.76 (s, 1H); 7.56 (d, J = 15.4 Hz, 1H); 7.50 (d, J = 8.4 Hz, 1H); 7.40-7.30 (m, 3H); 7.28 (m, 1H); 6.88 (d, J = 8.4 Hz, 1H); 6.30 (d, J = 15.4 Hz, 1H); 5.70 (br s, 1H), 2.95 (m, 1H); 2.34 (s, 3H); 0.85 (m, 2H); 0.57 (m, 2H). MS (ESI) *m*/*z* 777 (2M+Na)⁺, 755 (2M+H)⁺, 400 (M+Na)⁺, 378 (M+H)⁺.

### Example 32

### (2,4-Dichlorophenyl)[2-nitro-4-(E-((4-acetylpiperazin-1-y)carbonyl)ethenyl)phenyl]sulfide

### Example 32A

### 1-Chloro-2-nitro-4-(E-((4-acetylpiperazin-1-yl)carbonyl)ethenyl)benzene

To a stirred solution of trans-4-chloro-3-nitrocinnamic acid (1.50 g, 6.59 mmol) and 1-acetylpiperazine (0.89 g, 6.94 mmol) in 20 mL of DMF at room temperature was added EDAC (1.4 g, 7.30 mmol). The mixture was then stirred at room temperature for 2 hours. TLC indicated the complete consumption of the acid. Water was then added to quench the reaction and to precipitate out the product. Cinnamide was then collected through filtration and washed with cold water. The light yellow product was dried in vacuum oven overnight at 40 °C to give 2.04 g (6.03 mmol. 91.6 %) of the title compound.

### Example 32B

### (2,4-Dichlorophenyl)[2-nitro-4-(E-((4-acetylpiperazin-1-yl)carbonyl)ethenyl)phenyl]sulfide

To a stirred solution of 4-chloro-3-nitro-cinnamide (275 mg, 0.814 mmol) from Example 32A in 1.0 mL of DMF was added potassium carbonate (169 mg. 1.22 mmol), followed by the dropwise addition of 2,4-dichlorothiophenol (146 mg, 0.815 mmol). The mixture was then stirred at room temperature for 60 minutes. Completion of the reaction was indicated by the TLC. Water was then added to precipitate the product. Filtration, washing with cold water, and drying in a vacuum oven afforded 350 mg (0.728 mmol, 89%) of the titled compound as light yellow solid. ¹H NMR (d⁶-DMSO, 300 MHz) δ 2.05 (s, 3H), 3.42-3.50 (br m, 4H). 3.50-3.64 (br m, 2H), 3.64-3.79 (br m, 2H), 6.83 (d, *J* = 8.7 Hz, 1H), 7.44 (d, *J* = 15.3 Hz. 1H), 7.55 (d, *J* = 15.3 Hz, 1H), 7.63 (dd, *J* = 2.7, 8.7 Hz, 1H), 7.83 (d, *J* = 8.7 Hz, 1H), 7.93 (d, *J* = 8.7 Hz, 1H), 7.96 (d, *J* = 2.7 Hz, 1H), 8.69 (d, *J* = 1.8 Hz, 1H). MS (DCI/NH₃) (M+H)⁺ at *m*/*z* 497, 499, 501. Analysis calculated for C₂₁H₁₉N₃O₄ Cl₂ S₁·0.82H₂O: C, 50.94; H, 4.20; N, 8.49. Found: C, 50.91; H, 4.21; N, 8.69.

### Example 33

### (2,4-Dichlorophenyl)[2-nitro-4-(E-((3-(1-pyrrolidin-2-only)propylamino)carbonyl) ethenyl)phenyl]sulfide

The title compound was prepared by the procedures described in Example 32 substituting 1-acetylpiperazine with 1-(3-aminopropyl)-2-pyrrolidinone. Light-yellow powder; ¹H NMR (d⁶-DMSO, 300 MHz) δ 1.64 (p, *J* = 7.1 Hz, 2H), 1.91 (p, *J* = 7.5 Hz, 2H), 2.21 (t, *J* = 8.3 Hz, 2H), 3.15 (q, *J* = 6.3 Hz, 2H), 3.21 (dd, *J* = 9.9, 17.7 Hz, 2H), 3.32 (overlapping t, *J* = 8.4 Hz, 2H), 6.72 (d, *J* = 15.6 Hz, 1H), 6.86 (d, *J* = 8.7 Hz, 1H), 7.46 (d, *J* = 15.6 Hz, 1H), 7.63 (dd, *J* = 2.4, 8.1 Hz, 1H), 7.79 (dd, *J* = 2.4, 8.7 Hz, 1H), 7.84 (d, *J* = 8.7 Hz, 1H), 7.96 (d, *J* = 2.4 Hz, 1H), 8.18 (t, *J* = 6.0 Hz, 1H), 8.46 (d, *J* = 2.1 Hz, 1H). MS (DCI/NH₃) (M+H)⁺ at *m*/*z* 494, 496.

### Example 34

### (2.3-Dichlorophenyl)[2-nitro-4-(E-((4-acetylpiperazin-1-yl)carbonyl)ethenyl)phenyl]sulfide

The title compound was prepared by the procedures described in Example 32B substituting 2,4-dichlorothiophenol with 2,3-dichlorothiophenol. Light-yellow powder; ¹H NMR (d⁶-DMSO, 300 MHz) δ 2.04 (s, 3H), 3.42-3.50 (br m, 4H), 3.50-3.64 (br m, 2H), 3.64-3.79 (br m, 2H), 6.88 (d, *J* = 8.7 Hz, 1H), 7.45 (d, *J* = 15.6 Hz, 1H), 7.55 (t, *J* = 7.65 Hz, 1H), 7.57 (d, *J* = 15.6 Hz, 1H), 7.78 (dd, *J* = 1.8, 8.1 Hz, 1H), 7.87 (dd, *J* = 1.8, 8.1 Hz, 1H), 7.95 (dd, *J*= 2.7, 9.0 Hz, 1H), 8.69 (d, *J*= 1.8 Hz, 1H). MS (DCI/NH₃) (M+H)⁻ at *m*/*z* 497, 499, 501.

### Example 35

### (4-Bromophenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with 4-bromothiophenol. Light-yellow powder; ¹H NMR (d⁶-DMSO, 300 MHz) δ 2.04 (s, 3H), 3.47 (br m, 4H), 3.52 (br m, 1H), 3.60 (br m, 1H), 3.68 (br m, 1H), 3.74 (br m, 1H), 6.90 (d, *J* = 8.7 Hz, 1H), 7.43 (d, *J* = 15.0 Hz, 1H), 7.54 (d, *J* = 15.0 Hz, 1H), 7.58 (d, *J* = 9.0 Hz, 2H), 7.78 (d, *J* = 9.0 Hz, 2H), 7.92 (dd, *J* = 2.1, 9.0 Hz, 1H), 8.65 (d, *J* = 2.1 Hz, 1H). MS (DCI/NH₃) (M+H)⁺ at *m*/*z* 507,509.

### Example 36

### (4-Methylphenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with *p*-thiocresol. Light-yellow powder; ¹H NMR (d⁶-DMSO, 300 MHz) δ 2.04 (s, 3H), 2.39 (s, 3H), 3.47 (br m, 4H), 3.52 (br m, 1H), 3.60 (br m, 1H), 3.68 (br m, 1H), 6.89 (d, *J* = 8.7 Hz, 1H), 7.20 (d. *J* = 8.1 Hz, 1H), 7.39 (d, *J* = 8.4 Hz, 2H), 7.40 (d, *J* = 15.0 Hz, 1H), 7.53 (d, *J* = 15.0 Hz, 1H), 7.54 (d, *J* = 8.4 Hz, 2H), 7.89 (dd, *J* = 2.1, 8.7 Hz, 1H), 8.64 (d, *J* = 2.1 Hz, 1H). MS (DCI/NH₃) (M+NH₄)⁺ at *m*/*z* 443.

### Example 37

### (2,4-Dichlorophenyl)[2-nitro-4-(E-((4-(tert-butoxycarbonyl)piperazin-1-yl)carbonyl)ethenyl)phenyl]sulfide

The title compound was prepared by the procedures described in Example 32 substituting 1-acetylpiperazine with *tert*-butyl piperazine carboxylate. Light-yellow powder; ¹H NMR (d⁶-DMSO, 300 MHz) δ 1.42 (s, 9H), 3.36 (overlapping m, 4H), 3.55 (br m, 2H), 3.70 (br m, 2H), 6.83 (d, *J* = 8.7 Hz, 1H), 7.42 (d, *J* = 15.6 Hz, 1H), 7.54 (d, *J* = 15.6 Hz, 1H), 7.63 *(dd, J =* 2.4, 8.4 Hz, 1H), 7.83 (d, *J* = 8.7 Hz, 1H), 7.92 (dd, *J* = 2.4, 8.7 Hz, 1H), 7.96 (d, *J* = 2.7 Hz, 1H), 8.68 (d, *J* = 2.4 Hz, 1H). MS (APCI) (M+H)⁺ at *m*/*z* 538, 540, 542.

### Example 38

### (2,4-Dichlorophenyl)[2-nitro-4-(E-((4-(2-furoylcarbonyl)piperazin-1-yl)carbonyl)ethenyl)phenyl]sulfide

### Example 38A

### (2,4-Dichlorophenyl)[2-nitro-4-(E-((piperazin-1-yl)carbonyl)ethenyl)phenyl]sulfide Trifluoroacetic Acid Salt

The compound (100 mg, 0.186 mmol) from Example 37 was dissolved in 0.5 mL of neat trifluoroacetic acid (TFA). The mixture was stirred at room temperature for 1 hour. The TFA was then removed under vacuum to give the title compound (105 mg) as a yellow solid.

### Example 38B

### (2,4-Dichlorophenyl)[2-nitro-4-(E-((4-(2-furoylcarbonyl)piperazin-1-yl)carbonyl)ethenyl)phenyl]sulfide

To a stirred solution of piperazine TFA salt (35 mg, 0.067 mmol) from Example 38A in 2.0 mL of CH₂Cl₂ was added Et₃N (23 µL, 0.17 mmol), 4-dimethylaminopyridine (DMAP) (1.0 mg, 0.0082 mmol), and furyl chloride (8.0 µL, 0.080 mmol). The mixture was then stirred at room temperature for 30 minutes before the solvent was removed. The crude product was purified with Gilson HPLC system, YMC C-18 column, 75x30 mm I.D., S-5 µM, 120 Å, and a flow rate of 25 mL/min, λ=214, 245 nm; mobile phase A, 0.05 M NH₄Oac, and B, CH₃CN; linear gradient 20-100% of B in 20 minutes to give the title compound (24 mg, 67%) as light-yellow powder; ¹H NMR (d⁶-DMSO, 300 MHz) δ 3.62-3.87 (br m, 8H), 6.66 (q, *J*= 2.1 Hz, 1H), 6.84 (d, *J* = 8.7 Hz, 1H), 7.04 (d, *J* = 3.3 Hz, 1H), 7.44 (d, *J* = 15.3 Hz, 1H), 7.56 (d, *J* = 15.3 Hz, 1H), 7.63 (dd, *J* = 2.4, 8.1 Hz, 1H), 7.83 (d, *J* = 8.4 Hz, 1H), 7.87 (d, *J* = 2.1 Hz, 1H), 7.92 (dd, *J* = 2.1, 12.0 Hz, 1H), 7.96 (d, *J* = 2.1 Hz, 1H), 8.70 (d, *J* = 2.1 Hz, 1H). MS (APCI) (M+H)⁺ at *m*/*z* 532, 534, 536.

### Example 39

### (2,4-Dichlorophenyl)[2-nitro-4-(E-((4-(methanesulfonyl)piperazin-1-yl)carbonyl)ethenyl)phenyl]sulfide

The title compound was prepared by the procedures described in Example 38B substituting furoyl chloride with methanesulfonyl chloride. Light-yellow powder; ¹H NMR (d⁶-DMSO, 300 MHz) δ 2.90 (s, 3H), 3.25 (br m, 4H), 3.68 (br m, 2H), 3.83 (br m, 2H), 6.84 (d, *J* = 9.0 Hz, 1H), 7.45 (d, *J* = 15.6 Hz, 1H), 7.56 (d, *J* = 15.6 Hz, 1H), 7.63 (dd, *J* = 2.4, 8.7 Hz, 1H), 7.83 (d, *J* = 9.0 Hz, 1H), 7.93 (dd, *J* = 2.1, 9.0 Hz, 1H), 7.95 (d, *J =* 2.7 Hz, 1H), 8.70 (d, *J* = 2.1 Hz, 1H). MS (ESI) (M+H)⁺ at *m*/*z* 516, 518, 520.

### Example 40

### (2.4-Dichlorophenyl)[2-nitro-4-(E-((4-(diethylaminocarbonylmethyl)piperazin-1-yl)carbonyl)ethenyl)phenyl]sulfide

The title compound was prepared by the procedures described in Example 38B substituting furoyl chloride with 2-chloro-*N,N*-diethylacetamide. Light-yellow powder; ¹H NMR (d⁶-DMSO, 300 MHz) δ 1.01 (t, *J* = 7.2 Hz, 3H), 1.13 (t, *J* = 7.2 Hz, 3H), 2.46 (br m, 4H), 3.16 (s, 2H), 3.24 (q, *J* = 7.2 Hz, 2H), 3.37 (q. *J* = 7.2 Hz, 2H), 3.56 (br m, 2H), 3.69 (br m, 2H), 6.83 (d, *J* = 9.0 Hz, 1H), 7.46 (d. *J* = 15.3 Hz, 1H), 7.52 (d, *J* = 15.3 Hz, 1H), 7.62 (dd, *J* = 2.4, 8.7 Hz, 1H), 7.82 (d, *J* = 9.0 Hz, 1H), 7.92 (dd, *J* = 2.1, 9.0 Hz, 1H), 7.95 (d, *J* = 2.7 Hz, 1H), 8.67 (d, *J* = 2.1 Hz, 1H). MS (ESI) (M+NH₄)⁻ at *m*/*z* 573, 575, 577.

### Example 41

### (2,4-Dichlorophenyl)[2-nitro-4-(E-((4-(diethylaminocarbonyl)piperazin-1-yl)carbonyl)ethenyl)phenyl]sulfide

The title compound was prepared by the procedures described in Example 38B substituting furoyl chloride with *N,N*-diethylcarbamyl chloride. Light-yellow powder; ¹H NMR (d⁶-DMSO, 300 MHz) δ 1.06 (t, *J* = 6.9 Hz, 6H), 3.12 (br m, 4H), 3.15 (q, *J* = 6.9 Hz, 4H), 3.58 (br m, 2H), 3.72 (br m, 2H), 6.83 (d, *J* = 8.7 Hz, 1H), 7.42 (d, *J* = 15.6 Hz, 1H), 7.53 (d, *J* = 15.6 Hz, 1H), 7.63 (dd, *J* = 2.7, 9.0 Hz, 1H), 7.82 (d, *J* = 8.7 Hz, 1H), 7.92 (dd, *J* = 2.4, 8.7 Hz, 1H), 7.95 (d, *J* = 2.7 Hz, 1H), 8.68 (d, *J* = 2.1 Hz, 1H). MS (APCI) (M+H)⁺ at *m*/*z* 537, 539, 541.

### Example 42

### (2,4-Dichlorophenyl)[2-nitro-4-(E-((4-(tert-butoxycarbonylmethyl)piperazin-1-yl)carbonyl)ethenyl)phenyl]sulfide

The title compound was prepared by the procedures described in Example 38B substituting CH₂CL₂ with CH₃CN as solvent, and furoyl chloride with tert-butyl bromoacetate. Light-yellow powder; ¹H NMR (CDCl₃, 300 MHz) δ 1.47 (s, 9H), 2.70 (br m, 4H), 3.21 (s, 2H), 3.74 (br m, 2H), 3.82 (br m, 2H), 6.73 (d, *J* = 8.7 Hz, 1H), 6.92 (d, *J* = 15.0 Hz, 1H), 7.39 (dd, *J* = 2.4, 8.7 Hz, 1H), 7.47 (d, *J* = 8.7 Hz, 1H), 7.61 (d, *J* = 15.0 Hz, 1H), 7.62 (d, *J* = 2.4 Hz, 1H), 7.66 (d, *J* = 8.7 Hz, 1H), 8.43 (br d, 1H). MS (APCI) (M+H)⁺ at *m*/*z* 552, 554, 556.

### Example 43

### (2,4-Dichlorophenyl)[2-nitro-4-(E-((4-(carboxycarbonyl)piperazin-1-yl)carbonyl)ethenyl)phenyl]sulfide

### Example 43A

### (2,4-Dichlorophenyl)[2-nitro-4-(E-((4-(carbethoxycarbonyl)piperazin-1-yl)carbonyl)ethenyl)phenyl]sulfide

The title compound was prepared by the procedures described in Example 38B substituting furoyl chloride with ethyl oxalyl chloride.

### Example 43B

### (2,4-Dichlorophenyl)[2-nitro-4-(E-((4-(carboxycarbonyl)piperazin-1-yl)carbonyl)ethenyl)phenyl]sulfide

To a stirred solution of the ethyl ester (40 mg, 0.074 mmol) from Example 43A in 2 mL of ethanol was added saturated LiOH (0.25 mL). The mixture was then stirred at room temperature for 2 hours. Water (2 mL) was then added to the reaction mixture, which was then acidified to pH = 2 with concentrated HCl. The precipitates were collected through filtration, washed with cold water, dried under vacuum to give the titled compound (30 mg, 79%) as light yellow solid. ¹H NMR (d⁶-DMSO, 300 MHz) δ 3.52 (br m, 4H), 3.62 (br m, 2H), 3.76 (br m, 2H), 6.84 (d, *J* = 9.0 Hz, 1H), 7.46 (d, *J* = 15.3 Hz, 1H), 7.56 (d, *J* = 15.3 Hz, 1H), 7.63 (dd, *J* = 2.7, 8.7 Hz, 1H), 7.83 (d, *J* = 9.0 Hz, 1H), 7.93 (d, *J* = 9.0 Hz, 1H), 7.96 (d, *J* = 2.7 Hz, 1H), 8.70 (br d, 1H). MS (APCI) (M-COO)⁺ at *m*/*z* 466, 468, 470.

### Example 44

### (2,4-Dichlorophenyl)[2-nitro-4-(E-((4-(carboxymethyl)piperazin-1-yl)carbonyl)ethenyl)phenyl]sulfide

The title compound was prepared by the procedures described in Example 38A substituting compound from Example 37 with compound from Example 42. Light-yellow powder; ¹H NMR (d⁶-DMSO, 300 MHz) δ 3.14 (s, 2H), 3.40 (overlapping br m, 4H), 3.44 (br m, 1H), 3.51 (br m, 1H), 3.57 (br m, 1H), 3.71 (br m, 1H), 6.82 (d, *J* = 8.7 Hz, 1H), 7.42 (d, *J* = 15.6 Hz, 1H), 7.52 (d, *J* = 15.6 Hz, 1H), 7.63 (dd, *J* = 2.4, 8.7 Hz, 1H), 7.83 (d, *J* = 8.7 Hz, 1H), 7.92 (dd, *J* = 2.4, 8.7 Hz, 1H), 7.96 (d, *J* = 2.4 Hz, 1H), 8.68 (d, *J* = 2.4 Hz, 1H). MS (APCI) (M+H)⁺ at *m*/*z* 496, 498, 500.

### Example 45

### (2-Methylphenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with o-thiocresol. Light-yellow powder; ¹H NMR (d⁶-DMSO, 300 MHz) δ 2.03 (s, 3H), 2.29 (s, 3H), 3.47 (br m, 4H), 3.53 (br m, 1H), 3.60 (br m, 1H), 3.67 (br m, 1H), 3.83 (br m, 1H), 6.64 (d, *J =* 8.7 Hz, 1H), 7.40 *(d, J* = 15.0 Hz, 1H), 7.36-7.42 (m, 1H), 7.46-7.57 (m, 3H), 7.63 (d, *J* = 6.9 Hz, 1H), 7.89 (dd, *J* = 2.4, 9.0 Hz, 1H), 8.66 (d, *J* = 2.4 Hz, 1H). MS (APCI) (M+H)⁺ at *m*/*z* 426.

### Example 46

### (2-Chlorophenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with 2-chlorothiophenol. Light-yellow powder; ¹H NMR (d⁶-DMSO, 300 MHz) δ 2.04 (s, 3H), 3.47 (br m, 4H), 3.52 (br m, 1H), 3.60 (br m, 1H), 3.68 (br m, 1H), 3.73 (br m, 1H), 6.75 (d, *J* = 9.0 Hz, 1H), 7.43 (d, *J* = 15.3 Hz, 1H), 7.54 (d, *J* = 15.3 Hz, 1H), 7.55 (dd, *J* = 1.8, 8.1 Hz, 1H), 7.64 (t, *J* = 1.8, 8.1 Hz, 1H), 7.76 (d, *J* = 1.8, 8.1 Hz, 1H), 7.82 (d, *J* = 1.8, 8.1 Hz, 1H), 7.93 (dd, *J* = 2.4, 9.0 Hz, 1H), 8.68 (d, *J*= 2.4 Hz, 1H). MS (APCI) (M+H)⁺ at *m*/*z* 446, 448, 450.

### Example 47

### (2-Aminophenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with 2-aminothiophenol. Light-yellow powder; ¹H NMR (d⁶-DMSO, 300 MHz) δ 2.04 (s, 3H), 3.47 (br m, 4H), 3.52 (br m, 1H), 3.60 (br m, 1H), 3.68 (br m, 1H), 3.74 (br m, 1H), 5.58 (s, 2H), 6.65 (td, *J* = 1.5, 15.0 Hz, 1H), 6.72 (dd, *J* = 1.5, 8.7 Hz, 1H), 7.00 (dd, *J* = 1.8, 8.7 Hz, 1H), 7.27 (t, *J* = 1.5, 8.6 Hz, 1H), 7.36 (dd, *J* = 1.5, 8.7 Hz, 1H), 7.39 (d, *J* = 15.3 Hz, 1H), 7.53 (d, *J* = 15.3 Hz, 1H), 7.89 (dd, *J* = 1.8, 8.7 Hz, 1H), 8.64 (d, *J* = 1.8 Hz, 1H). MS (APCI) (M+H)⁺ at *m*/*z* 427.

### Example 48

### (2-Hydroxymethylphenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with 2-mercaptobenzyl alcohol. Light-yellow powder; ¹H NMR (d⁶-DMSO, 300 MHz) δ 2.03 (s, 3H), 3.47 (br m, 4H), 3.52 (br m, 1H), 3.60 (br m, 1H), 3.67 (br m, 1H), 3.73 (br m, 1H), 4.53 (d, *J* = 5.7 Hz, 1H), 5.34 (t, J = 5.7 Hz, 1H), 6.65 (d, *J* = 8.7 Hz, 1H), 7.40 (d, *J* = 15.3 Hz, 1H), 7.46 (d, *J* = 7.8 Hz, 1H), 7.53 (d, *J* = 15.3 Hz, 1H), 7.59 (d, *J* = 7.5 Hz. 1H), 7.64 (d, *J* = 7.5 Hz, 1H), 7.87 (dd, *J* = 2.1, 8.7 Hz, 1H), 8.65 (d, *J* = 2.1 Hz, 1H). MS (APCI) (M+NH₄)⁺ at *m*/*z* 459.

### Example 49

### (2-Ethylphenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with 2-ethylthiophenol. Light-yellow powder; ¹H NMR (d⁶-DMSO, 300 MHz) δ 1.01 (t, *J* = 7.65 Hz, 3H), 2.04 (s, 3H), 2.69 (q, *J =* 7.65 Hz, 2H), 3.47 (br m, 4H), 3.52 (br m, 1H), 3.59 (br m, 1H), 3.67 (br m, 1H), 3.73 (br m, 1H), 6.64 (d, *J* = 8.7 Hz, 1H), 7.38 (dd, *J* = 2.4, 7.5 Hz, 1H), 7.40 (d, *J* = 15.6 Hz, 1H), 7.50-7.61 (m, 3H), 7.53 (d, *J* = 15.6 Hz, 1H), 7.89 (dd, *J* = 2.4. 8.7 Hz, 1H), 8.64 (d, *J* = 2.4 Hz, 1H). MS (APCI) (M+Cl)⁻ at *m*/*z* 474, 476.

### Example 50

### (2-iso-Propylphenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with 2-isopropylthiophenol. Light-yellow powder; ¹H NMR (d⁶-DMSO, 300 MHz) δ 1.05 (d, *J* = 6.9 Hz, 6H), 2.04 (s, 3H), 3.47 (br m, 4H), 3.52 (br m, 1H), 3.60 (br m, 1H), 3.67 (br m, 1H). 3.72 (br m. 1H). 6.64 (d, *J =*_{*.*} 8.4 Hz, 1H), 7.34-7.41 (m, 2H), 7.39 (d, *J* = 15.3 Hz, 1H), 7.52 (d, *J* = 15.3 Hz, 1H), 7.56-7.73 (m, 2H), 7.90 (dd, *J* = 2.1, 8.7 Hz, 1H), 8.64 (d, *J* = 2.1 Hz, 1H). MS (APCI) (M+NH₄)⁻ at *m*/*z* 471. Analysis calculated for C₂₄H₂₇N₃O₄ S₁·0.21 H₂O: C, 63.03; H, 5.96; N, 9.13. Found: C, 63.03; H, 6.04; N, 9.19.

### Example 51

### (2-tert-Butylphenyl)[2-nitro-4-(E-((4-acetylpiperazin-1-yl)carbonyl)ethenyl)phenyl]sulfide

The title compound was prepared by the procedures described in Example 32 substituting 2,4-dichlorothiophenol with 2-*tert*-butylthiophenol. Light-yellow powder; ¹H NMR (d⁶-DMSO, 300 MHz) δ 1.46 (s, 9H), 2.04 (s, 3H), 3.47 (br m, 4H). 3.52 (br m, 1H), 3.60 (br m, 1H), 3.67 (br m, 1H), 3.73 (br m, 1H), 6.68 (d, *J* = 8.7 Hz, 1H), 7.35 (t, *J* = 7.5 Hz, 1H), 7.39 (d, *J* = 15.3 Hz, 1H), 7.45-7.57 (m, 2H), 7.50 (d, *J* = 15.3 Hz, 1H), 7.65 (d, *J* = 8.1 Hz, 1H), 7.88 (dd, *J* = 2.4, 8.7 Hz, 1H), 8.64 (d, *J* = 2.4 Hz, 1H). MS (APCI) (M+NH₄)⁺ at *m*/*z* 485.

### Example 52

### (2-Chlorophenyl)[2-chloro-4-(E-((4-acetylpiperazin-1-yl)carbonyl))2-propenyl)phenyl]sulfide

### Example 52A

### 3'-Chloro-4'-[(2-chlorophenyl)thio]acetophenone

The title compound was prepared by the procedures described in Example I A substituting 2,4-dichlorothiophenol with 2-chlorothiophenol, and 2-chlorobenzaldehyde with 4'-fluoro-3'-chloroacetophenone.

### Example 52B

### (2-Chlorophenyl)[2-chloro-4-(E-(1-ethoxycarbonyl)2-propenyl)phenyl]sulfide

To a stirred suspension of NaH (60% in mineral oil, 121 mg, 3.03 mmol) in 20 mL of anhydrous THF under nitrogen atmosphere was added triethyl phosphonoacetate dropwise. After 20 minutes, the acetophenone (600 mg, 2.02 mmol) from Example 52A in THF (5 mL) was added in one portion. The resulting clear solution was then stirred at room temperature for 7 hours. Reaction was then stopped, most of the solvent was evaporated, and the residue was partitioned between EtOAc (2x20 mL) and water. The combined organic layer was washed with water and brine, dried over Na₂SO₄, concentrated in vacuo. The crude product was purified using silica gel flash column chromatography eluting with 5-10% Et₂O in hexanes to give the (*E*)-isomer of the cinnamate (500 mg, 68%) as a white solid.

### Example 52C

### (2-Chlorophenyl)[2-chloro-4-(E-(1-carboxy)2-propenyl)phenyl]sulfide

A mixture of the cinnamate (500 mg, 1.37 mmol) from Example 52B in 5 mL of EtOH/THF (4:1) was stirred with sat. LiOH solution (0.50 mL) at 50 °C for 2 hours. The mixture was then acidified with 3N HCl and extracted with CH₂Cl₂ (3x10 mL). The combined organic layer was dried over MgSO₄, concentrated under reduced pressure to give the titled compound (450mg, 97%) as a white solid.

### Example 52D

### (2-Chlorophenyl)[2-chloro-4-(E-((4-acetylpiperazin-1-yl)carbonyl))2-propenyl)phenyl]sulfide

The title compound was prepared using the cinnamic acid from Example 52C by the procedures described in Example 1C substituting 6-amino-1-hexanol with 1-acetylpiperazine. White solid; ¹H NMR (CDCl₃, 300 MHz) δ 2.10-2.20 (m. 3H), 2.25 (s, 3H), 3.40-3.80 (m, 8H), 6.28 (s, 1H), 7.00 (d, *J* = 8.7 Hz, 1H), 7.19-7.36 (m, 4H), 7.46-7.56 (m, 2H). MS (APCI) (M+NH₄)⁺ at *m*/*z* 466, 468, 470.

### Example 53

### (2-(1-Morpholinylmethyl)phenyl)[2-chloro-4-(E-((1-morpholinyl)carbonyl)ethenyl)phenyl]sulfide

### Example 53A

### (2-(1-Bromomethyl)phenyl)[2-chloro-4-(E-((1-morpholinyl)carbonyl)ethenyl)phenyl]sulfide

To a stirred solution of benzyl alcohol (195 mg, 0.32 mmol) from Example 11 in 2.0 mL of anhydrous DMF was added LiBr (48 mg, 0.35 mmol). The mixture was then cooled in an ice-water bath, and PBr₃ (60 µL, 0.40 mmol) was dropped in slowly. The ice bath was then removed and the mixture was stirred at room temperature for 1 hour. Water was then added, the mixture was then partitioned between EtOAc and aqueous NaHCO₃. The aqueous layer was extracted with EtOAc once. The combined organic layer was washed with water and brine, dried over Na₂SO₄, concentrated on a rotavap. The crude bromide (230mg) was used directly for the alkylation without purification.

### Example 53B

### (2-(1-Morpholinylmethyl)phenyl)[2-chloro-4-( E-((1-morpholinyl)carbonyl)ethenyl)phenyl]sulfide

To a stirred solution of morpholine (10 µL, 0.11 mmol) in 0.5 mL of CH₃CN was added Hunig's base (23.7 µL, 0.14 mmol), followed by the bromide (40 mg, 0.091 mmol). The mixture was then stirred at room temperature for 2 hours. Solvent was then removed and the crude product was purified with Gilson Preparative HPLC as described in Example 38B to give the titled compound as a white solid. ¹H NMR (d⁶-DMSO, 300 MHz) δ 2.33 (br t, 4H), 3.45 (br t, 4H), 3.50-3.65 (m, 6H), 3.56 (s, 2H), 3.65-3.80 (br m, 2H), 6.74 (d, *J* = 8.7 Hz, 1H), 7.30 (d, *J* = 15.3 Hz, 1H), 7.35-7.41 (m, 2H), 7.43 (d, *J* = 15.3 Hz, 1H), 7.46 (td, *J* = 2.4, 8.1 Hz, 1H), 7.52 (dd, *J* = 2.1, 8.7 Hz, 1H), 7.56 (d, *J* = 8.1 Hz, 1H), 8.02 (d, *J* = 2.1 Hz, 1H). MS (DCI/NH₃) (M+H)⁺ at *m*/*z* 459, 461.

### Example 54

### (2-(4-(1,3-Benzodioxolyl-5-methyl)piperazin-1-ylmethyl)phenyl)[2-chloro-4-(E-((1-morpholinyl)carbonyl)ethenyl)phenyl]sulfide

The title compound was prepared by the procedures described in Example 53B substituting morpholine with 1-piperonylpiperazine. White solid; ¹H NMR (d⁶-DMSO, 300 MHz) δ 2.13-2.40 (br m, 8H), 3.28 (s, 2H), 3.49-3.64 (br m, 6H), 3.54 (s, 2H), 3.70 (br m, 2H), 5.97 (s, 2H), 6.69 (dd, *J* = 1.8, 8.1 Hz, 1H), 6.74 (d, *J* = 8.7 Hz, 1H), 6.79 (d, *J* = 1.8 Hz, 1H), 6.81 (d, *J* = 8.1 Hz, 1H), 7.39 (d, *J* = 15.3 Hz, 1H), 7.33-7.38 (m, 2H), 7.38-7.50 (m, 2H), 7.43 (d, *J* = 15.3 Hz, 1H), 7.53 (d, *J* = 8.4 Hz, 1H), 8.00 (d, *J* = 2.1 Hz, 1H). MS (DCI/NH₃) (M+H)⁺ at *m*/*z* 592, 594.

### Example 55

### (2-(4-(iso-Propylaminocarbonylmethyl)piperazin-1-ylmethyl)phenyl)[2-chloro-4-(E-((1-moepholinyl)carbonyl)ethenyl)phenyl]sulfide

The title compound was prepared by the procedures described in Example 53B substituting morpholine with *N*-isopropyl-1-piperazineacetamide. White solid; ¹H NMR (d⁶-DMSO, 300 MHz) δ 1.04 (d, *J* = 6.3 Hz, 6H), 2.20-2.42 (br m, 8H), 2.78 (s, 2H), 3.47-3.64 (br m, 6H), 3.56 (s, 2H), 3.64-3.76 (br m, 2H), 3.85 (qd, *J* = 6.3, 8.1 Hz, 1H), 6.73 (d, *J* = 8.7 Hz, 1H), 7.29 (d, *J* = 15.6 Hz, 1H), 7.31-7.39 (m. 2H), 7.43 (d, *J* = 15.6 Hz, 1H), 7.45 (td, *J* = 2.7, 6.3 Hz, 1H), 7.50 (dd, *J* = 2.1, 8.7 Hz, 1H), 7.55 (d, *J* = 7.8 Hz, 1H), 8.00 (d, *J* = 2.1 Hz, 1H). MS (DCI/NH₃) (M+H)⁻ at *m*/*z* 557, 559.

### Example 56

### (2-((N-Ethoxycarbonylmethyl-N-methyl)aminomethyl)phenyl)[2-chloro-4-(E-((1-morpholinyl)carbonyl)ethenyl)phenyl]sulfide

The title compound was prepared by the procedures described in Example 53B substituting morpholine with ethyl sarcosinate hydrochloride. White solid; ¹H NMR (d⁶-DMSO, 300 MHz) δ 1.16 (t, *J* = 7.2 Hz, 3H), 2.27 (s, 2H), 3.30 (s, 2H). 3.51-3.66 (br m, 6H), 3.66-3.75 (br m, 2H), 3.78 (s, 2H), 4.05 (q; *J* = 7.2 Hz, 2H), 6.75 (d, *J* = 8.7 Hz, 1H), 7.30 (d, *J* = 15.3 Hz, 1H), 7.33-7.38 (m, 2H), 7.42-7.50 (m, 2H), 7.43 (d, *J* = 15.3 Hz, 1H), 7.53 (dd, *J* = 2.1, 8.7 Hz, 1H), 7.60 (d, *J* = 7.8 Hz, 1H). 8.02 (d, *J* = 2.1 Hz, 1H). MS (DCI/NH₃) (M+H)⁺ at *m*/*z* 489,491.

### Example 57

### (2-Formylphenyl)[2-chloro-4-(E-((1-morpholinyl)carbonyl)ethenyl)phenyl]sulfide

To a stirred solution of the alcohol (368 mg, 0.94 mmol) from Example 11 in 5 mL of anhydrous acetonitrile was added activated 4Å molecular sieves, TPAP (3.3 mg, 0.0094 mmol), and NMO (110 mg, 1.03 mmol). The mixture was then stirred at room temperature for 3 hours. The reaction mixture was then quenched with dimethyl sulfide (100 µL). The crude product was filtered through celite, washed with acetonitrile, condensed in vacuo. The titled compound was purified by silica gel column chromatography to give a white solid (216 mg, 59 %). ¹H NMR (d⁶-DMSO, 300 MHz) δ 3.60 (br m, 6H), 3.73 (br m, 2H), 7.00 (d, *J* = 8.4 Hz, 1H), 7.40 (d, *J* = 15.3 Hz, 1H), 7.42 (d, *J* = 8.4 Hz, 1H), 7.51 (d, *J* = 15.3 Hz, 1H). 7.52 (td, J = 1.8, 8.1 Hz, 1H), 7.61 (td, *J* = 1.8. 8.1 Hz, 1H), 7.71 (dd, *J* = 2.1, 8.4 Hz, 1H), 8.02 (dd, *J =* 2.1, 8.4 Hz, 1H), 8.14 (d, *J* = 2.1 Hz, 1H). MS (DCI/NH₃) (M+H)⁺ at *m*/*z* 388, 390.

### Example 58

### (2-(4-Formylpiperazin-1-ylmethyl)phenyl)[2-chloro-4-(E-((1-morpholinyl)carbonyl)ethenyl)phenyl]sulfide

The title compound was prepared by the procedures described in Example 53B substituting morpholine with 1-formyl piperazine. White solid; ¹H NMR (d⁶-DMSO, 300 MHz) δ 2.20-2.32 (m. 6H), 2.74 (br m, 2H), 3.48 (s, 2H), 3.59 (m, 6H), 3.70 (br m, 2H), 6.74 (d, *J* = 8.7 Hz, 1H), 7.29 *(d, J =* 15.6 Hz, 1H), 7.35-7.41 (m, 2H), 7.42 (d,*J* = 15.6 Hz, 1H), 7.45-7.52 (m, 3H), 7.98 (d, *J* = 2.1, 1H). MS (DCI/NH₃)(M+H)⁺ at *m*/*z* 486, 488.

### Example 59

### (2-(E-((1-Morpholinyl)carbonyl)ethenyl)phenyl)[2-chloro-4-(E-((1-morpholinyl) carbonyl)ethenyl)phenyl]sulfide

A mixture of bromide (80 mg, 0.18 mmol) from Example 12, acryloylmorpholine (33 mg, 0.23 mmol), Pd(OAc)₂ (2.0 mg, 0.009 mmol), P(*o*-tolyl)₃ (17 mg, 0.056 mmol), Et₃N (39 µL, 0.27 mmol), and anhydrous DMF (1.0 mL) in a pressure tube was flushed with nitrogen for 5 minutes before it capped and heated at 110 °C over night. TLC indicated almost complete consumption of the starting bromide. The reaction mixture was then allowed to cool down to room temperature, partitioned between EtOAc and water. The aqueous layer was extracted once with EtOAc. The combined organic layer was washed with water and brine, dried over Na₂SO₄, condensed under reduced pressure. The crude product was purified with Gilson Preparative HPLC as described in Example 38B to give the titled compound as a light-brown solid (35 mg, 39%). ¹H NMR (d⁶-DMSO, 300 MHz) δ 3.43-3.88 (m, 16H), 6.58 (d, *J* = 8.7 Hz, 1H), 7.30 (d, *J* = 15.3 Hz, 2H), 7.43 (d, *J*= 15.3 Hz, 1H), 7.47-7.64 (m, 4H), 7.86 (d, *J* = 15.3 Hz, 1H), 8.06 (d, *J* = 2.1 Hz, 1H), 8.14 (d, *J* = 7.5 Hz, 1H). MS (DCI/NH₃) (M+NH₄)⁺ at *m*/*z* 516, 518. Analysis calculated for C₂₆H₂₇N₂O₄Cl₁S₁·0.46H₂O: C, 61.56; H, 5.55; N, 5.21. Found: C, 61.56; H, 5.50; N, 5.43.

### Example 60

### (2-Formylphenyl)[2-nitro-4-(E-((4-acetylpiperazin-1-yl)carbonyl)ethenyl)phenyl]sulfide

The title compound was prepared by the procedures described in Example 57 substituting compound from Example 11 with compound from Example 48. Yellow solid; ¹H NMR (d⁶-DMSO, 300 MHz) δ 2.04 (s, 3H), 3.47 (br m, 4H), 3.52 (br m, 1H), 3.60 (br m, 1H), 3.68 (br m, 1H), 3.74 (br m, 1H), 6.85 (d, *J* = 8.4 Hz. 1H), 7.44 (d, *J* = 15.6 Hz, 1H), 7.55 (d, *J* = 15.6 Hz, 1H), 7.61 (d, *J* = 7.5 Hz, 1H). 7.73 (t, *J* = 7.5 Hz, 1H), 7.80 (td, *J* = 2.4, 7.5 Hz, 1H), 7.92 (dd, *J* = 2.1, 9.0 Hz, 1H), 8.04 (dd. *J* = 2.4, 7.5 Hz, 1H), 8.66 (d, *J* = 2.1 Hz, 1H), 10.29 (s, 1H). MS (APCI) (M+Cl)⁻ at *m*/*z* 474.476.

### Example 61

### (2-Formylphenyl)[2-chloro-4-(E-((1-morpholinyl)carbonyl)ethenyl)phenyl]sulfide, N,N-dimethyl hydrazone

A mixture of the aldehyde (20 mg, 0.052 mmol) from Example 57, 1,1-dimethyl hydrazine (3.9 µL, 0.052 mmol) in 0.5 mL of EtOH with a tiny amount of AcOH was stirred at room temperature over night. The solvent was then removed and the product was purified by preparative TLC to give the titled compound (20 mg, 90%) as a white solid. ¹H NMR (CDCl₃, 300 MHz) δ 2.91 (s, 6H), 3.55-3.82 (br m, 8H), 6.64 (d, *J* = 8.7 Hz, 1H), 6.76 (d, *J* = 15.3 Hz, 1H), 7.05 (dd, *J* = 1.8, 8.7 Hz, 1H), 7.26 (td, *J* = 1.8, 7.8 Hz, 1H), 7.43 (t, *J* = 7.8 Hz, 1H), 7.47-7.57 (m, 2H), 7.54 (m, 2H), 8.04 (dd, *J* = 1.8, 8.7 Hz, 1H). MS (DCI/NH₃) (M+H)⁺ at *m*/*z* 430,432,434, 436.

### Example 62

### (2-((3-(1-Morpholinyl)propyl)-1-amino)phenyl)[2-chloro-4-(E-((1-morpholinyl)carbonyl)ethenyl)phenyl]sulfide

A mixture of bromide (60 mg, 0.14 mmol) from Example 12, aminopropylmorpholine (24 µL, 0.17 mmol), Pd₂(dba)₃ (1.2 mg, 0.0013mmol), BINAP (2.5 mg, 0.004 mmol), NaO*t*-Bu (19 mg, 0.20 mmol), 18-crown-6 (50 mg, 0.20 mmol), and anhydrous toluene (1 mL) in a pressure tube was flushed with nitrogen for 3 minutes before it was capped and heated at 80 °C over night. The reaction was then stopped, and allowed to cool down to room temperature. The reaction mixture was partitioned between EtOAc and water, and the aqueous layer was extracted once with EtOAc. The combined organic layer was then washed with water and brine, dried over Na₂SO₄, condensed under reduced pressure. The crude product was purified with Gilson Preparative HPLC as described in Example 38B to give the titled compound as a light-brown oil (30 mg, 44%). ¹H NMR (d⁶-DMSO, 300 MHz) δ 1.62 (quintet, *J* = 6.5 Hz, 2H), 2.15-2.26 (m, 8H), 3.17 (q, *J* = 6.5 Hz, 2H), 3.22-3.76 (m, 12 H), 3.50 (t, *J* = 6.5 Hz, 2H), 5.72 (t, *J* = 5.7 Hz, 1H), 6.47 (d, *J* = 8.7 Hz, 1H), 6.68 *(t, J =* 7.2 Hz, 1H), 6.81 (d, *J* = 8.4 Hz, 1H), 7.26 (d, *J* = 15.6 Hz, 1H), 7.35-7.42 (m, 2H), 7.43 (d, *J* = 15.6 Hz, 1H), 7.44 (d, *J* = 8.4 Hz, 1H), 7.49 (d, *J* = 8.4 Hz, 1H), 8.00 (d, *J* = 2. 1 Hz, 1H). MS (APCI) (M+H)⁺ at *m*/*z* 502, 504.

### Example 63

### (2,4-Dichlorophenyl)[2-bromo-4-(E-((3-(1-pyrrolidin-2-only)propylamino)carbonyl)ethenyl)phenyl]sulfide

### Example 63A

### (2,4-Dichlorophenyl)[2-amino-4-(E-((3-(1-pyrrolidin-2-only)propylamino)carbonyl)ethenyl)phenyl]sulfide

A mixture of nitro compound (780 mg, 1.58 mmol) from Example 33, SnCl₂ (1.50 g, 7.91 mmol) in 25 mL of anhydrous EtOH was refluxed under nitrogen atmosphere for 90 minutes. The reaction was then allowed to cool down to room temperature, quenched with sat. NaHCO₃, extracted with EtOAc (2x50 mL). The combined organic layer was washed with water and brine, dried over Na₂SO₄, condensed in vacuo to give the crude aniline as yellowish brown solid, which was converted to the bromide without purification.

### Example 63B

### (2,4-Dichlorophenyl)[2-bromo-4-(E-((3-(1-pyrrolidin-2-only)propylamino)carbonyl) ethenyl)phenyl]sulfide

To a stirred solution of *t*-butyl nitrite (57 µL, 0.48 mmol), CuBr₂ (87 mg, 0.39 mmol) in 2.0 mL of CH₃CN at room temperature was added a solution of aniline from Example 63A (150 mg, 0.323 mmol) in 1.0 mL of CH₃CN. The dark green solution was then heated at 65 °C under nitrogen atmosphere for 90 minutes. The reaction mixture was then allowed to cool down to room temperature, partitioned between EtOAc and 3N HCl. The organic layer was then washed with brine, dried over Na₂SO₄, condensed in vacuo. The crude product was then purified with Gilson Preparative HPLC as described in Example 38B to give the titled compound as a light-brown solid (50 mg, 29%). Colorless oil; ¹H NMR (d⁶-DMSO, 300 MHz) δ 1.63 (quintet, *J* = 7.2 Hz, 2H), 1.91 (quintet, *J* = 8.4 Hz, 2H), 2.22 (t, *J* = 8.4 Hz, 2H), 3.09-3.47 (m, 6H), 6.67 (d, *J* = 15.3 Hz, 1H), 7.07 (d, *J* = 8.4 Hz, 1H), 7.32 (d, *J* = 8.7 Hz, 1H), 7.38 (d, *J* = 15.3 Hz, 1H), 7.50 (dd, *J* = 2.4, 8.7 Hz, 1H), 7.57 (dd, *J* = 2.1, 8.4 Hz, 1H), 7.86 (d, *J* = 2.4 Hz, 1H), 7.96 (d, *J* = 2.1 Hz, 1H), 8.13 (t, *J* = 6.0 Hz, 1H). MS (ESI) (M+H)⁺ at *m*/*z* 527, 529, 531, 533.

### Example 64

### (2,4-Dichlorophenyl)[2-formyl-4-(E-((1-morpholinyl)carbonyl)ethenyl)phenyl]sulfide

### Example 64A

### [1-Fluoro-2-formyl-4-(E-((1-morpholinyl)carbonyl)ethenyl)benzene

The title compound was prepared by the procedures described in Example 59 substituting the bromide from Example 12 with 2-fluoro-5-bromobenzaldehyde.

### Example 64B

### (2,4-Dichlorophenyl)[2-formyl-4-(E-((1-morpholinyl)carbonyl)ethenyl)phenyl]sulfide

The title compound was prepared by the procedures described in Example 32 substituting 4-chloro-3-nitro-cinnamide with the compound from Example 64A. White solid; ¹H NMR (d⁶-DMSO, 300 MHz) δ 3.60 (br m, 6H), 3.71 (br m, 2H), 6.82 (d, *J* = 8.7 Hz, 1H), 7.35 (d, *J* = 15.6 Hz, 1H), 7.54 (d, *J* = 15.6 Hz, 1H), 7.55 (dd, *J* = 2.4, 8.7 Hz, 1H), 7.61 (d, *J* = 8.7 Hz, 1H), 7.86 (dd, *J* = 2.4, 8.4 Hz, 1H), 7.91 (d, *J* = 2.4 Hz, 1H), 8.41 (d, *J* = 2.1 Hz, 1H), 10.19 (s, 1H). MS (DCI/NH₃) (M+H)⁺ at *m*/*z* 422, 424, 426, 428.

### Example 65

### (2-Chloro-6-formylphenyl)[2-chloro-4-(E-((4-acetylpiperazin-1-yl)carbonyl)ethenyl)phenyl]sulfide

### Example 65A

### (2-Carbomethoxyethyl)[2-chloro-4-(E-((4-acetylpiperazin-1-yl)carbonyl)ethenyl)phenyl]sulfide

The title compound was prepared by the procedures described in Example I substituting 2,4-dichlorothiophenol with methyl 3-mercaptopropionate, and 6-amino-1 -hexanol with 1-acetyl piperazine.

### Example 65B

### (2-Chloro-6-formylphenyl)[2-chloro-4-(E-((4-acetylpiperazin-1-yl)carbonyl)ethenyl)phenyl]sulfide

To a stirred solution of the compound (105 mg, 0.26 mmol) from Example 65A in 2 mL of THF under nitrogen atmosphere at 0 °C was added *t*-BuOK solution (1.0M, 281 µL, 0.29 mmol). Light orange precipitates appeared immediately. After completion of the addition, the reaction mixture was stirred at room temperature for 1 hour before the solvent was removed on a rotavap under reduced pressure.

The yellow thiolate thus obtained was dissolved in 0.5 mL of DMF, and 2,3-dichlorobenzaldehyde was then added. The mixture was then heated at 80 °C under nitrogen for 2 hours. Reaction was then stopped and the solvent was removed under vacuum. The crude product was purified with Gilson Preparative HPLC as described in Example 38B to give the titled compound as a white solid (25 mg, 21 %). ¹H NMR (CDCl₃, 300 MHz) δ 2.05 (s, 3H), 3.48-3.58 (m, 2H), 3.58-3.84 (m, 6H), 6.53 (d, *J* = 8.7 Hz, 1H), 6.80 (d, *J* = 15.3 Hz, 1H), 7.19 (dd, *J* = 1.8, 8.7 Hz, 1H), 7.51-7.62 (m, 2H), 7.60 (d, *J* = 15.3 Hz, 1H), 7.84 (dd, *J* = 1.8, 8.4 Hz, 1H), 7.99 (dd, *J* = 1.8, 8.4 Hz, 1H). MS (APCI) (M+NH₄)⁺ at *m*/*z* 480, 482, 484.

### Example 66

### (2-Cyanophenyl)[2-chloro4-(E-((4-acetylpiperazin-1-yl)carbonyl)ethenyl)phenyl]sulfide

The title compound was prepared by the procedures described in Example 65B substituting 2,3-dichlorobenzaldehyde with 2-fluorobenzonitrite, giving a white solid. ¹H NMR (CDCl₃, 300 MHz) δ 2.15 (s, 3H), 3.48-3.57 (m, 2H), 3.59-3.84 (m, 6H), 6.86 (d, J = 15.6 Hz, 1H), 7.12 (d, J = 8.4 Hz, 1H), 7.32 (d, J = 8.4 Hz, 1H), 7.41 (d, J = 6.6 Hz, 1H), 7.46 (dd, J = 1.8, 8.4 Hz, 1H), 7.55 (dd, J = 1.8, 8.1 Hz, 1H), 7.61 (d, J = 15.6 Hz, 1H), 7.64 -(d, J = 1.8 Hz, 1H), 7.75 (dd, J = 1.8, 8.4 Hz, 1H). MS (DCI/NH₃) (M+NH₄)⁺ at *m*/*z* 443.

### Example 67

### (2-Isopropylphenyl)[2-cyano-4-(E-((morpholin-1-yl)carbonyl)ethenyl)phenyl]sulfide

### Example 67A

### (2-Isopropylphenyl)(4-bromo-2-cyanophenyl)sulfide

The title compound was prepared by the procedures described in Example 1A substituting 2,4-dichlorothiophenol with isopropylthiophenol, and 2-chlorobenzaldehyde with 2-fluorobenzonitrile.

### Example 67B

### (2-Isopropylphenyl)[2-cyano-4-(E-((morpholin-1-yl)carbonyl)ethenyl)phenyl]sulfide

The title compound was prepared by the procedures described in Example 59 substituting the bromide from Example 12 with the bromide from Example 67A, giving a white solid. ¹H NMR (CDCl₃, 300 MHz) δ 1.19 (d, *J* = 6.9 Hz, 6H), 3.49 (septet, *J* = 6.9 Hz, 1H), 3.58-3.87 (m, 8H), 6.73 (d, *J* = 8.4 Hz, 1H), 6.83 (d, *J* = 15.6 Hz, 1H), 7.20-7.30 (m, 1H), 7.42 (dd, *J* = 2.4, 8.4 Hz, 1H), 7.46 (d, *J* = 3.0 Hz, 2H), 7.49 (dd, *J* = 1. 8, 6.9 Hz, 1H), 7.57 (d, *J* = 15.6 Hz, 1H), 7.76 (d, *J* = 1.8 Hz, 1H). MS (APCI⁺) (M+H)⁺ at *m*/*z* 393.

### Example 68

### (2-Bromophenyl)[2-nitro-4-(E-((4-acetylpiperazin-1-yl)carbonyl)ethenyl)phenyl]sulfide

The title compound was prepared by the procedures described in Example 32B substituting 2,4-dichlorothiophenol with 2-bromothiophenol, providing a light-yellow solid; ¹H NMR (d⁶-DMSO, 300 MHz) δ 2.04 (s, 3H), 3.40-3.65 (m, 8H), 6.75 (d, *J* = 8.7 Hz, 1H), 7.42 (d, *J* = 15.6 Hz, 1H), 7.51 (dd, *J* = 2.1, 6.9 Hz ,1H), 7.54 (d, *J* = 15.6 Hz, 1H), 7.55 (t, *J* = 2.1 Hz, 1H), 7.59 (dd, *J* = 2.1, 6.9 Hz ,1H), 7.82 (dd, *J* = 2.4, 7.8 Hz, 1H), 7.92(td, *J =* 2.4, 8.4 Hz, 1H), 8.67 (d, *J =* 2.4 Hz, 1H). MS (APCI⁻) (M+Cl)⁻ at *m*/*z* 524, 526, 528.

### Example 69

### (2-(Pyrrolidin-1-yl)phenyl)[2-chloro-4-(E-((morpholin-1-yl)carbonyl)ethenyl)phenyl]sulfide

To a stirred solution of bromide (75 mg, 0.17 mmol) from Example 12 in toluene in a sealed tube was added sequentially pyrrolidine (18.4 mL, 0.22 mmol), Pd₂(dba)₃ (3.0 mg, 0.0034mmol), BINAP (6.0 mg, 0.010mmol), followed by NaO*t*-Bu (26 mg, 0.27 mmol). The resulting mixture was then flushed with anhydrous N₂ for 2 min before it was capped and heated at 90 °C for 24 h. The reaction mixture was then allowed to cool down to room temperature and partitioned between ethyl acetate and brine. The organic layer was then dried with Na₂SO₄, filtered, and concentrated in vacuo. The crude product was purified using Gilson Preparative HPLC as described in Example 38B to give the title compound (40 mg, 55% yield) as a white solid; ¹H NMR (CDCl₃, 300 MHz) δ 1.83 (br s, 4H), 3.40 (br s, 4H), 3.56-3.80 (m, 8H). 6.57 (d, *J* = 8.4 Hz, 1H), 6.75 (d, *J* = 15.6 Hz, 1H), 6.8 1 (br t, *J* = 8.4 Hz, 1H), 6.90 (br s, 1H), 7.15 (dd, *J* = 2.1, 8.4 Hz, 1H), 7.18-7.27 (m, 1H), 7.32 (td, *J* = 1.8, 8.4 Hz, 1H), 7.42 (dd, *J* = 1.8, 7.8 Hz, 1H), 7.50 (d, *J* = 1.8 Hz, 1H), 7.55 (d, *J* = 15.6 Hz. 1H). MS (APCI⁺) (M+H)⁺ at *m*/*z* 429, 431.

### Example 70

### (2-Methoxyphenyl)-[2-chloro-4(E-[(morpholin-1-yl)carbonyl]ethenyl)phenyl]sulfide

The title compound was prepared according to the procedures of Example 1, giving a white solid, m.p. 162-164C. ¹H NMR (CDCl₃, 300 MHz) δ 3.60-3.78 (m, 8H), 3.84 (s, 3H), 6.72 (d, J =9Hz, 1H), 6.78 (d, J =16Hz, 1H), 6.96-7.04 (m. 2H), 7.16 (dd, J =9Hz, 2Hz, 1H), 7.40-7.46 (, 2H), 7.55 (d, J =2H, 1H), 7.58 (d, J =16Hz. 1H). Anal. Calcd. for C₂₀H₂₀ClNO₃S: C, 61.61; H, 5.17; N, 3.59. Found: C, 61.53, H, 5.22; N, 3.50.

### Example 71

### (2-Isopropylphenyl)[2-nitro-4-(E-((3-carbomethoxypiperazin-1-yl)carbonyl)ethenyl)phenyl]sulfide

### Example 71 A

### 1-tert-Butyoxycarbonyl-2-carbomethoxypiperazine

2-Carbomethoxypiperazine was treated with benzyl chloroformate (1.0 eq) in aqueous NaHCO₃ to give 1-benzyloxycarbonyl-3-carbomethoxypiperazine. This material was treated with di-*tert*-butyldicarbonate (1.1 eq) and triethylamine (1.0 eq) in THF to produce 1-*tert*-butyoxycarbonyl-4-benzyloxycarbonyl-2-carbomethoxypiperazine. Hydrogenation of this compound in methanol using 10% Pd-C gives the title compound after filtration and solvent removal.

### Example 71B

### (2-Isopropylphenyl)[2-nitro-4-(E-((3-carbomethoxypiperazin-1-yl)carbonyl)ethenyl)phenyl]sulfide

A mixture of (2-isopropylphenyl)[2-nitro-4-*E*-(carboxyethenyl)phenyl]sulfide (prepared according to the procedures of Example 32), the amine from Example 71A (1.0 eq), 2-(1H-benzotriazol-1-yl)-1,1,3,3-tetramethyluronium tetrafluoroborate (1.0 eq), and diisopropylethylamine (2.0 eq) in DMF was stirred at ambient temperature for 4 hr. Ethyl acetate was added, and the mixture was washed sequentially with 1N HCl, bicarb, and brine. The resultant yellow solid was treated with 1:1 TFA/dichloromethane at ambient temperature to give the title compound as a yellow solid. ¹H NMR (DMSO-d₆, 300MHz) δ 1.15 (d, J = 6.6 Hz, 6H); 2.52-3.16 (br m, 4H); 3.25-3.47 (m, 1H); 3.60-3.65 (br d, 3H); 3.60, 3.66 (br s, br s, 3H); 6.61-6.67 (br m, 1H); 7.30-7.62 (m, 6H); 7.88-7.93 (br m, 1H); 8.58-8.65 (br m, 1H). MS (APCI) (M+H)⁺ at m/z 470. Anal calcd for C₂₄H₂₇N₃S₁O₅: C, 61.39; H, 5.80; N, 8.95. Found: C, 61.51; H, 5.87; N, 8.68.

### Example 72

### (2-Methylphenyl)[2-nitro-4-(E-((3-carboxamido-4-carbobenzoxypiperazin-1-yl)carbonyl)ethenyl)phenyl]sulfide

Prepared according to the procedures of Example 71, giving a yellow solid. ¹H NMR (DMSO-d₆, 300MHz) δ 2.30 (s, 3H); 2.80-4.80 (br m, 7H); 5.05-5.15 (br m, 2H); 6.61-6.67 (br m, 1H); 7.02-7.64 (m, 13H); 7.80-7.90 (br m, 1H); 8.56-8.65 (br m, 1H). MS (APCI) (M+H)- at m/z 561. Anal calcd for C₂₉H₂₈N₄S₁O₆·0.42CH₃COOCH₂CH₃: C, 61.66; H, 5.29; N, 9.38. Found: C, 61.41; H, 5.28; N, 9.53.

### Example 73

### (2-Isopropylphenyl)[2-nitro-4-(E-((2-carbomethoxy-4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.13 (d, J = 6.6 Hz, 6H); 1.40, 1.41 (s, s, 9H); 2.72-3.08 (br m, 1H); 3.17-3.24 (m, 1H); 3.30-3.40 (m, 1H); 3.68 (br s, 3H); 3.79-4.51 (br m, 4H); 5.06, 5.36 (br s, br s, 1H); 6.61-6.67 (m, 1H); 7.30-7.62 (m, 6H); 7.85-7.93 (br m, 1H); 8.64-8.69 (br m, 1H). MS (APCI) (M+H)⁺ at m/z 570. Anal calcd for C₂₉H₃₅N₃S₁O₇^{·}0.15C₆H₁₄: C, 61.66; H, 6.43; N, 7.21. Found: C, 61.69; H, 6.35; N, 7.02.

### Example 74

### (2-Isopropylphenyl)[2-nitro-4-(E-((2-carboxy-4-tert-butoxycarbonylpiperazin-1-yl)carbonyl)ethenyl)phenyl]sulfide

Prepared according to the procedures of Example 71, giving a yellow solid. ¹H NMR (CDCl₃, 300MHz) δ 1.14 (d, J = 6.6 Hz, 6H); 1.45 (s, 9H); 2.72-4.75 (br m, 6H); 3.38-3.49 (m, 1H); 5.78 (br s, 1H); 6.68, 6.72 (s, s, 1H); 6.88, 6.94 (br s, br, s, 1H); 7.26-7.71 (m, 6H); 8.44 (br s, 1H). MS (APCI) (M-H)⁺ at m/z 554. Anal calcd for C₂₈H₃₃N₃S₁O₇: C, 60.53; H, 5.99; N, 7.56. Found: C, 60.42; H, 6.21; N, 7.31.

### Example 75

### (2-Isopropylphenyl)[2-trifluoromethyl-4-(E-((4-acetylpiperazin-1-yl)carbonyl)ethenyl)phenyl]sulfide

The title compound was prepared according to the procedures of Example 1. ¹H NMR (CDCl₃, 300 MHz) δ 7.78 (s, 1H), 7.62 (d, 1H, J = 15.5 Hz), 7.43-7.49 (m, 3H), 7.37 (d, 1H, J = 8.1 Hz), 7.23 (m, 1H), 6.85 (d, 1H, J = 15.5 Hz), 6.82 (d, 1H, J = 8.5 Hz), 3.63-3.77 (m. 6H), 3.45-3.55 (m, 3H), 2.14 (s, 3H), 1.17 (d, 6H, J = 6.6 Hz). MS (ESI) *m*/*z* 477, 499, 975, 953. Anal. Calcd for C₂₅H₂₇F₃N₂O₂S · 0.5 EtOAc: C, 62.29; H, 6.00; N, 5.38. Found: C, 62.40; H, 6.21; N, 5.35.

### Example 76

### (2-Isopropylphenyl)(2-trifluoromethyl-4-(E-((morpholin-1-yl)carbonyl)ethenyl)phenyl]sulfide

The title compound was prepared according to the procedures of Example 1. ¹H NMR (CDCl₃, 300 MHz) 7.78 (s, 1H), 7.62 (br, 1H), 7.33-7.48 (m, 3H), 7.22 (m, 1H), 6.85 (m, 1H), 6.80 (d, 1H, J = 8.5 Hz). 3.73 (br, 8H), 3.49 (dq, 1H, J₁ = J₂ = 6.9 Hz), 1.17 (d, 6H, J = 7.1 Hz). MS (ESI) *m*/*z* 436, 871,893. Anal. Calcd for C₂₃H₂₄F₃N₁O₂S: C, 63.43; H, 5.55; N, 3.22. Found: C,63.12; H, 5.81, N, 3.10.

### Example 77

### (2-Isopropylphenyl)[2-trifluoromethyl-4-(E-((3-(pyrrolidin-2-on-1-yl)prop-1-ylamino)carbonyl)ethenyl)phenyl]sulfide

The title compound was prepared according to the procedures of Example 1. ¹H NMR (CDCl₃, 300 MHz) δ 7.77 (s, 1H). 7.52(d, 1H, J = 15.4 Hz), 7.43-7.51 (m, 3H), 7.36 (d, 1H, J = 8.8 Hz), 7.22 (m, 1H). 7. 10 (br, 1H), 6.80 (d, 1H, J = 8.4 Hz), 6.44 (d, 1H, J = 15.4 Hz), 3.49 (dq, 1H, J₁ = J₁ = 6.9 Hz), 3.40 (m, 4H), 3.3 1 (dd, 2H, J₁ = 5.7 Hz, J₂ = 12.0 Hz), 2.44 (t, 2H, J = 8.1 Hz), 2.08 (tt, 2H, J₁ = J₂ = 7.5 Hz), 1.74 (m, 2H), 1.18 (d, 6H, J = 6.9 Hz). MS (ESI) *m*/*z* 491, 513, 981, 1003. Anal. Calcd for C₂₆H₂₉F₃N₂O₂S: C, 63.66; H, 5.96; N, 5.71. Found: C,64.00; H, 6.12, N, 5.68.

### Example 78

### (2-Isopropylphenyl)[2-trifluoromethyl-4-(E-((cyclobutylamino)carbonyl)ethenyl)phenyl]sulfide

The title compound was prepared according to the procedures of Example 1. ¹H NMR (CDCl₃, 300 MHz) δ 7.76 (s, 1H), 7.52 (d, 1H, J = 15.4 Hz), 7.43-7.49 (m, 3H), 7.33 (d, 1H, J = 7.7 Hz), 7.22 (m, 1H), 6.79 (d, 1H, J = 8.1 Hz), 6.33 (d, 1H, J = 15.4 Hz), 5.72 (br, 1H), 4.52 (m, 1H), 3.49 (dq, 1H, J₁ = J₂ = 6.9 Hz), 2.40 (m, 2H), 1.90 (m, 2H), 1.74 (m, 2H), 1.17 (d, 6H, J = 6.6 Hz). MS (ESI) *m*/*z* 420, 839, 861. Anal. Calcd for C₂₃H₂₄F₃N₁O₁S: C, 65.85; H, 5.77; N, 3.34. Found: C,65.53; H, 5.83, N, 3.21.

### Example 79

### (2-Isopropylphenyl)[2-trifluoromethyl-4-(E-((cyclopentylamino)carbonyl)ethenyl)phenyl]sulfide

The title compound was prepared according to the procedures of Example 1. ¹H NMR (CDCl₃, 300 MHz) δ 7.77 (s, 1H), 7.52 (d, 1H, J = 15.5 Hz), 7.43-7.48 (m, 3H), 7.33 (d, 1H, J = 8.8 Hz), 7.22 (m, 1H), 6.79 (d, 1H, J = 8.1 Hz), 6.33 (d, 1H, J = 15.5 Hz), 5.54 (d, J = 7.7, 1H), 4.35 (m, 1H), 3.49 (dq, 1H, J₁ = J₂ = 6.9 Hz). 2.05 (m, 2H), 1.68 (m, 4H), 1.44(m. 2H), 1.17 (d, 6H, J = 7.0 Hz). MS (ESI) *m*/*z* 434, 867, 889. Anal. Calcd for C₂₄H₂₆F₃N₁O₁S: C, 66.49; H, 6.04; N, 3.23. Found: C, 66.24; H, 6.14, N, 3.06.

### Example 80

### (2-Isopropylphenyl)[2-trifluoromethyl-4-(E-((5-hydroxypent-1-ylamino)carbonyl)ethenyl)phenyl]sulfide

The title compound was prepared according to the procedures of Example 1. ¹H NMR (CDCl₃, 300 MHz) δ 7.77 (s, 1H), 7.54 (d, 1H, J = 15.5 Hz), 7.43-7.49 (m, 3H), 7.33 (d, 1H, J = 8.0 Hz), 7.22 (m, 1H), 6.79 (d, 1H, J = 8.4 Hz), 6.35 (d, 1H, J = 15.6 Hz), 5.67 (br, 1H), 3.67 (t, 2H, J = 6.4 Hz), 3.49 (dq, 1H, J₁ = J₂ = 6.9 Hz), 3.40 (m, 2H), 2.40 (m, 2H), 1.45-1.62 (m, 6H), 1.17 (d, 6H, J = 7.0 Hz). MS (ESI) *m*/*z* 452, 474, 903, 925. Anal. Calcd for C₂₄H₂₈F₃NO₂S · 0.56 EtOAc: C, 62.92; H, 6.54; N, 2.80. Found: C, 62.86; H, 6.53; N, 2.96.

### Example 81

### (2-Isopropylphenyl)[2-nitro-4-(E-((3-carbomethoxy-4-acetylpiperazin-1-yl)carbonyl)ethenyl)phenyl]sulfide

Prepared according to the procedures of Example 71, giving a yellow solid. ¹H NMR (CDCl₃, 300MHz) δ 1.14 (d, J = 6.6 Hz, 6H); 2.20 (s, 3H); 2.75-3.80 (br m, 4H); 3.39-3.50 (m, 1H); 3.70, 3.77 (br s, br s, 3H); 4.49-4.75 (br m, 2H); 5.39 (br s, 1H); 6.71(m, 1H); 6.91-7.04 (br m, 1H); 7.25-7.64 (m, 6H); 8.42 (br m, 1H). MS (APCI) (M+H)⁺ at m/z 512. Anal calcd for C₂₆H₂₉N₃S₁O₆: C, 61.04; H, 5.71; N, 8.21. Found: C, 61.40; H, 6.05; N, 7.88.

### Example 82

### (2-Biphenyl)[2-chloro-4-(E-((morpholin-1-yl)carbonyl)ethenyl)phenyl]sulfide

To a stirred solution of bromide from Example 12 (60 mg, 0.14 mmol)in 1 mL of toluene was added 0.5 mL of sat. Na₂CO₃, Pd(PPh₃)₄ (8 mg, 0.007 mmol), phenylboronic acid (17 mg, 0.14 mmol). The mixture was flushed with nitrogen and heated at 100 °C for 3 h. The reaction mixture was then allowed to cool down to room temperature and partitioned between ethyl acetate and brine. The organic layer was then dried with Na₂SO₄, filtered, and concentrated in vacuo. The crude product was purified using Gilson Preparative HPLC as described in Example 38B to give the title compound as colorless oil (40 mg, 67% yield); ¹H NMR (CDCl₃, 300 MHz) δ 3.58-3.86 (m, 8H), 6.77 (d, *J* = 15.6 Hz, 1H), 6.86 (d, *J* = 8.4 Hz, 1H), 7.67 (dd. *J* = 2.1, 8.4 Hz, 1H), 7.29-7.40 (m, 3H), 7.40-7.48 (m, 6H), 7.56 (d, *J* = 15.6 Hz, 1H), 7.65 (d, *J* = 1.8 Hz, 1H). MS (APCI⁺) (M+H)⁺ at *m*/*z* 436, 438.

### Example 83

### (3,4-Dimethylphenyl)[2-nitro-4-(E-((4-acetylpiperazin-1-yl)carbonyl)ethenyl)phenyl]sulfide

To a solution of the compound of Example 32A (40 mg, 0.12 mmole) in 2.5 mL of dimethylformamide was added 3,4-dimethylthiophenol (17 mg, 0.12 mmole), followed by potassium carbonate powder (20 mg, 0.14 mmole). The mixture was heated at 100°C for 20 h. The solvent was removed using N₂ gas flow. Water (5 mL) was then added to the residue, the resulting precipitate was collected through filtration, washed with cold water, and air dried to give the title compound (42 mg, 81%) as light yellow solid. ¹H-NMR (CDCl₃, 400 MHz) δ 2.08 (s, 3H), 2.23 (s, 3H), 2.27 (s, 3H), 3.45 (br, m, 2H), 3.63 (br, m, 6H), 6.79 (s, 1H), 6.82 (d, J = 19 Hz, 1H), 7.18 (d, J = 19 Hz, 1H), 7.24 (dd, J = 4, 19 Hz, 1H), 7.27 (s, 1H), 7.34 (d, J = 21 Hz, 1H), 7.56 (d, J = 39 Hz, 1H), 8.32 (d, J = 4 Hz, 1H). MS (APCI) (M+H)⁺ at m/z 440. FAB High Resolution MS calculated m/z for C₂₃H₂₆N₃O₄S (M+H)⁺: 440.1644. Observed m/z: 440.1646.

### Example 84

### (2-Bromophenyl)[2-trifluoromethyl-4-(E-((4-acetylpiperazin-1-yl)carbonyl)ethenyl)phenyl]sulfide

The title compound was prepared by the procedures described in Example 9 substituting 2,4-dichlorothiophenol with 2-bromothiophenol, and 3,4-dichlorobenzaldehyde with 4-fluoro-3-trifluoromethylbenzaldehyde, to give a white solid. ¹H NMR (d⁶-DMSO, 300 MHz) δ 2.04 (s, 3H), 3.43-3.80 (m, 8H), 7.21 (dd, *J* = 2.1, 8.4 Hz, 1H), 7.24 (d, *J* = 8.4 Hz, 1H), 7.33 (td, *J* = 2.1, 7.65 Hz, 1H), 7.42 (td, *J* = 1.8, 7.65 Hz, 1H), 7.45 (d, *J* = 15.6 Hz, 1H), 7.58 (d, *J* = 15.6 Hz, 1H), 7.78 (dd, *J* = 1.8, 8.4 Hz, 1H), 7.96 (dd, *J* = 1.8, 8.4 Hz, 1H), 8.25 (d, *J* = 1.8 Hz, 1H). MS (APCI⁺) (M+NH₄)⁺ at *m*/*z* 530, 532, 534.

### Example 85

### (5-Indolyl)[2-chloro-4-(E-((4-acetylpiperazin-1-yl)carbonyl)ethenyl)phenyl]sulfide

To a stirred solution of 5-iodoindole (255 mg, 1.05 mmol) in 5.0 mL of anhydrous DMF was added the potassium thiolate (457 mg, 1.26 mmol) from Example 65B, followed by K₂CO₃ (174 mg, 1.26 mmol), and cuprous iodide (20 mg, 0.11 mmol). The resulting mixture was then heated at 120 °C for overnight. The reaction mixture was then allowed to cool to ambient temperature and poured into water. The aqueous mixture was extracted twice with 25 mL of ethyl acetate. The combined organic layer was then washed with water and brine, dried over Na₂SO₄, filtered, concentrated on a rotavap under reduced pressure. The crude product was purified using Gilson Preparative HPLC as described in Example 38B to give the title compound (115 mg, 25 % based on the iodide) as a light-brown solid. ¹H NMR (d⁶-DMSO, 300 MHz) δ 2.03 (s, 3H), 3.40-3.78 (m, 8H), 6.51 (d, J = 8.4 Hz, 1H), 6.53 (s, 1H), 7.23 *(dd, J =* 2.1, 8.4 Hz, 1H), 7.27 (d, *J* = 15.6 Hz, 1H), 7.39 (d, J = 15.6 Hz, 1H), 7.41 (dd, *J* = 1.8, 8.4 Hz, 1H), 7.49 (t, *J* = 2.7 Hz, 1H), 7.56 (d, *J* = 8.4 Hz, 1H), 7.85 (d, *J* = 1.8 Hz, 1H), 7.99 (d, J = 1.8 Hz, 1H). MS (APCI⁻) (M+NH₄)⁻ at *m*/*z* 440, 442. Anal. Calcd for C₂₃H₂₂ClN₃O₂S · 0.53 CH₂Cl₂: C, 58.28; H, 4.79; N, 8.66. Found: C, 58.31; H, 4.93; N, 8.65.

### Example 86

### (5-Benzodioxolyl)[2-chloro-4-(E-((4-acetylpiperazin-1-yl)carbonyl)ethenyl)phenyl]sulfide

The title compound was prepared by the procedures described in Example 85 substituting 5-iodoindole with 1-iodo-3,4-methylenedioxybenzene, providing a white solid. ¹H NMR (CDCl₃, 300 MHz) δ 2.14 (s, 3H), 3.48-3.60 (m, 2H), 3.60-3,84 (m, 6H), 6.05 (s, 2H), 6.75 (d, *J* = 8.4 Hz, 1H), 6.80 (d, *J* = 15.3 Hz, 1H), 6.88 (d. *J* = 8.4 Hz, 1H), 6.98 (d, *J* = 2.1 Hz, 1H), 7.08 (dd, *J* = 2.1, 8.4 Hz, 1H), 7.19 (d, J = 1.8, 8.4 Hz, 1H), 7.52 (d, *J* = 2.1 Hz, 1H), 7.58 (d, *J* = 15.6 Hz, 1H). MS (APCI-) (M+NH₄)⁺ at *m*/*z* 445, 447.

### Example 87

### (2-Isopropylphenyl)[2-nitro-4-(E-((2-carbomethoxypiperazin-1-yl)carbonyl)ethenyl)phenyl]sulfide

Prepared according to the procedures of Example 71, giving a yellow solid. ¹H NMR (DMSO-d₆, 300MHz) δ 1. 14 (d, J = 6.6 Hz, 6H); 2.52-2.91 (br m, 5H); 3.30-3.40 (m, 1H); 3.68, 3.69 (s, s, 3H); 4.10-4.25 (br m, 1H); 5.00-5.21 (br m, 1H); 6.60-6.65(m, 1H); 7.29-7.62 (m, 6H); 7.85-7.95 (m, 1H); 8.64-8.68 (m, 1H). MS (APCI) (M+H)⁺ at m/z 470.

### Example 88

### (2,3-Dimethoxyphenyl)-[2-chloro-4(E-[(morpholin-1-yl)carbonyl]ethenyl)phenyl]sulfide

The title compound was prepared according to the procedures of Example 1, giving a white solid, m.p. 148-150C. ¹H NMR (CDCl₃, 300 MHz) δ 3.60-3.78 (m, 8H), 3.85 (s, 3H), 3.91 (s, 3H), 6.78 (d, J =16Hz, 1H), 6.86-6.98 (m, 3H), 7.20 (dd, J =9Hz, 2Hz. 1H), 7.54 (d, J =2Hz, 1H), 7.58 (d, J =16Hz, 1H). Anal. Calcd. for C₂₁H₂₂ClNO₄S: C, 60.06; H, 5.28; N, 3.33. Found: C, 59.72; H, 5.34; N, 2.97.

### Example 89

### (2-Fluorophenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 2-fluorothiophenol. Yellow solid (40 mg, 78%); ¹H-NMR (CDCl₃, 400 MHz) δ 2.17 (s, 3H), 3.56 (br, m. 2H), 3.77 (br, m, 6H), 6.88 (dd, J =3, 21 Hz, 1H), 6.93 (d, J = 39 Hz, 1H), 7.26 (dd, J = 3, 21 Hz, 1H), 7.33 (dd, J = 3, 19 Hz, 1H), 7.49 (br, d, J = 20 Hz, 1H), 7.58 (m, 1H), 7.66 (m, 2H), 8.46 (d, J = 4 Hz, 1H). MS (APCI) (M+H)⁺ at m/z 430. FAB High Resolution MS calculated m/z for C₂₁H₂₁N₃O₄FS (M+H)⁺: 430.1237. Observed m/z: 430.1246.

### Example 90

### (2-Bromophenyl)[2-trifluoromethyl-4-(E-((4-(tert-butoxycarbonyl)piperazin-1-yl)carbonyl)ethenyl)phenyl]sulfide

The title compound was prepared by the procedures described in Example 1 substituting 2,4-dichlorothiophenol with 2-bromothiophenol, 2-chlorobenzaldehyde with 4-fluoro-3-trifluoromethylbenzadehyde, and 6-amino-1-hexanol with *t*-butyl 1-piperazinecarboxylate, to give a white solid. ¹H NMR (CDCl₃, 300 MHz) d 1.48 (s, 9H), 3.49 (br s, 4H), 3.56-3.78 (m, 4H), 6.89 (d, *J* = 15.6 Hz, 1H), 7.10 (d, *J* = 8.4 Hz, 1H), 7.18-7.35 (m, 3H), 7.49 (d, *J* = 8.4 Hz, 1H), 7.65 (d, *J* = 15.6 Hz, 1H), 7.68 (dd, *J* = 2.1, 8.4 Hz, 1H), 7.85 (br s, 1H). MS (APCI') (M+Cl)⁻ at *m*/*z* 605, 607, 609. Anal. Calcd for C₂₅H₂₆N₂0₃BrF₃S . 0.03 H₂O: C, 52.50; H, 4.59; N, 4.90. Found: C, 52.54; H, 4.71; N, 4.68.

### Example 91

### (2-(Pyrrolidin-1-yl)phenyl)[2-trifluoromethyl-4-(E-((4-(tert-butoxycarbonyl)piperazin-1-yl)carbonyl)ethenyl)phenyl]sulfide

The title compound was prepared by the procedures described in Example 69 substituting the bromide from Example 12 with the bromide from Example 90, to give a white solid. ¹H NMR (CDCl₃, 300 MHz) δ 1.85 (s, 9H), 1.85 (br s, 4H), 3.32-3.55 (m, 8H), 3.55-3.78 (m, 4H), 6.76 (d, *J* = 8.4 Hz, 1H), 6.82 (d, *J* = 15.6 Hz, 1H), 7.23-7.45 (m, 5H), 7.61 (d, *J* = 15.6 Hz, 1H), 7.75 (br s, 1H). MS (APCI⁺) (M+H)⁻ at *m*/*z* 562.

### Example 92

### (3-Carboxamidophenyl)[2-nitro-4-(E-((4-acetylpiperazin-1-yl)carbonyl) ethenyl)phenyl]sulfide

### Example 92A

### (3-Carboxyphenyl)[2-nitro-4-(E-((4-acetylpiperazin-1-yl)carbonyl)ethenyl)phenyl]sulfide

The title compound was prepared by the procedures described in Example 32B substituting 2,4-dichlorothiophenol with 3-mercaptobenzoic acid.

### Example 92B

### (3-Carboxamidophenyl)[2-nitro-4-(E-((4-acetylpiperazin-1-yl)carbonyl)ethenyl)phenyl]sulfide

To a stirred solution of benzoic acid from Example 92A (40 mg, 0.088 mmol) in 1 mL of anhydrous DMF with HOBT (15 mg, 0.097 mmol) was added EDAC (19 mg, 0.097 mmol), followed by ammonium chloride (large excess). The pH of the solution was adjusted to 6 with addition of triethylamine. The resulting mixture was then stirred at ambient temperature for 6 h. Water was added to quenched the reaction. The product precipitated out after stirring for 30 min, which was then isolated by filtration and dried in vacuum oven to give a light yellow solid (25 mg, 63% yield). ¹H NMR (d⁶-DMSO, 300 MHz) δ 2.04 (s, 3H), 3.43-3.82 (m, 8H), 6.84 (d, *J* = 8.7 Hz, 1H), 7.43 (d, *J* = 15.6 Hz, 1H), 7.53 (d, *J* = 15.6 Hz, 1H), 7.56 (d, *J* = 1.8 Hz, 1H), 7.66 (t, *J* = 7.65 Hz, 1H), 8.06 (d, *J* = 7.80 Hz, 1H), 8.12 (s, 2H), 8.67 (d, *J* = 2.1 Hz, 1H). MS (ESI⁺) (M+Na)⁺ at *m*/*z* 477.

### Example 93

### (3-(Hydroxymethyl)phenyl)[2-nitro-4-(E-((4-acetylpiperazin-1-yl)carbonyl)ethenyl)phenyl]sulfide

To a stirred solution of benzoic acid from Example 92A (255 mg, 0.56 mmol) in 5 mL of anhydrous THF at 0 °C was added in turn Et₃N (102 mL, 0.73 mmol) and ethyl chloroformate (70 mL, 0.73 mmol). After 60 min, the reaction mixture was filtered through celite plug into a stirred solution of NaBH₄ in water at 0 °C. The resulting reaction mixture stirred at 0 °C for 2 h before it was extracted with EtOAc (2x20 mL). The combined organic layers was washed with 3N HCl, brine, dried over Na₂SO₄, filtered, concentrated under reduced pressure. The crude product was purified using Gilson Preparative HPLC as described in Example 38B to give the title compound (80 mg, 32% yield) as a light-yellow solid. ¹H NMR (d⁶-DMSO, 300 MHz) δ 2.04 (s, 3H), 3.40-3.79 (m, 8H), 4.56 (s, 2H), 5.38 (br s, 1H), 6.85 (d,*J*= 8.7 Hz, 1H), 7.42 (d, *J* = 15.6 Hz, 1H), 7.52 (br s, 3H), 7.57 (br s, 2H), 7.91 (dd, *J* = 2.1, 8.7 Hz, 1H), 8.66 (d, *J* = 2.1 Hz, 1H). MS (APCI⁺) (M+NH₄)⁺ at *m*/*z* 459.

### Example 94

### Phenyl[2-trifluoromethyl-4-(E-((4-(tert-butoxycarbonyl)piperazin-1-yl)carbonyl)ethenyl)phenyl]sulfide

The title compound was obtained as a reductive side product from the reaction mixture described in Example 91, as a colorless oil. ¹H NMR (CDCl₃, 300 MHz) δ 1.49 (s, 9H), 3.43-3.56 (br s, 4H), 3.56-3.82 (m, 4H), 6.85 (d, *J* = 15.6 Hz, 1H), 7.06 (d, *J* = 8.4 Hz, 1H), 7.37-7.50 (m, 4H), 7.63 (d, *J* = 15.6 Hz, 1H), 7.67 (d, *J* = 8.4 Hz, 1H), 7.76 (d, *J* = 11.7 Hz, 1H), 7.80 (s, 1H). MS (APCI⁻) (M+Cl)- at *m*/*z* 527.

### Example 95

### (2-Isopropylphenyl)[2-trifluoromethyl-4-(E-((2-carbomethoxy-4-(tert-butoxycarbonyl)piperazin-1-yl)carbonyl)ethenyl)phenyl]sulfide

The title compound was prepared according to the procedures of Example 71. ¹H NMR (CDCl₃, 300 MHz) δ 7.79 (s, 1H), 7.62 (d, 1H, J = 15.0 Hz), 7.48 (d, 1 H, J = 7.2 Hz), 7.43 (m, 2H), 7.38 (d, 1 H, J = 8.1 Hz), 7.22 (m, 1H), 6.86 (d, 1H, J = 15.4 Hz), 6.80 (d, 1 H. J = 8.4 Hz), 5.30 (br, 1H), 4.62 (br d, 2H, J = 14.0 Hz), 3.89 (br m, 1H), 3.76 (s, 3H), 3.49 (dq, 1 H, J₁ = J₂ = 6.9 Hz), 3.12 (m, 2H), 2.94 (br, 1H), 1.46 (s, 9H), 1.17 (d, 6H, J = 6.6 Hz). MS (ESI) *m*/*z* -591, -627, -677.

### Example 96

### (2-Isopropylphenyl)[2-nitro-4-(E-((3-(pyridine-4-methylaminocarbonyl)-4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 1.38 (s, 9H); 2.83-3.85 (br m, 5H); 4.09-4.51 (br m, 4H); 4.91-5.09 (br m, 1H); 6.64 (d, J = 8.5 Hz, 1H); 7.12-7.62 (m, 8H); 7.82-7.96 (m, 1H); 8.26-8.48 (m, 2H); 8.63-8.75 (m, 2H). MS (APCI) (M+H)⁺ at m/z 646. Anal calcd for C₃₄H₃₉N₅S₁O₆: C, 63.24; H, 6.09; N, 10.84. Found: C, 63.07; H, 6.43; N, 10.54.

### Example 97

### (2-Ethoxyphenyl)-[2-chloro-4(E-[(morpholin-1-yl)carbonyl]ethenyl)phenyl]sulfide

### Example 97A

### 2-Ethoxybenzenethiol

To 7.82g of ethoxybenzene and 7.41 g of tetramethylethylenediamine in 75 ml ether, cooled in an ice bath, a solution of 25.6 ml of a 2.5 M n-butyllithium solution in hexane, was added dropwise under a nitrogen atmosphere. The mixture was stirred for 1 hour at room temperature and then cooled to -65 degrees. Sulfur (2.28 g) was added in portions. The mixture was stirred for 3 hours at room temperature and then cooled in ice. LiAlH₄ (0.6 g) was added and the mixture was stirred 1 hour at room temperature. The mixture was again cooled in ice while 5 ml water was added dropwise followed by 15% HCl in water until all salts. The aqueous phase was separated and washed with ether. The combined ether layers was washed with HCl, then water. After drying with Na₂SO₄, the ether was evaporated to give 9.66 g of product. NMR analysis showed 70% pure material with 30% of a diaryl sulfide impurity. This mixture was carried forward to the next step.

### Example 97B

### (2-Ethoxyphenyl)-[2-chloro-4(E-[(morpholin-1-yl)carbonyl]ethenyl)phenyl]sulfide

The title compound was prepared according to the procedures of Example 1, substituting the thiol of Example 97A, giving a white solid, m.p. 125-127C. ¹H NMR (CDCl₃, 300 MHz) δ 1.25 (t, J =7Hz, 3H), 3.60-3.78 (m, 8H), 4.05 (q, J =7Hz, 2H), 6.76 (d, J =15Hz, 1H), 6.82 (d, J =9H, 1H), 6.94-7.00 (m, 2H), 7.16 (dd, J =9Hz, 2Hz, 1H), 7.34-7.45 (m, 2H), 7.54 (d, J =2Hz, 1H), 7.58 (d, J =15Hz, 1H). Anal. Calcd. for C₂₁H₂₂ClNO₃S: C, 62.44; H, 5.49; N, 3.47. Found: C, 62.14; H, 5.70; N, 3.22.

### Example 98

### (2-Methoxyphenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 2-methoxythiophenol, giving a yellow solid (40 mg, 77%). 1H-NMR (CDCl3, 400 MHz) δ 2.14 (s, 3H), δ 3.54 (br, m, 2H), δ 3.68 (br, m, 6H), δ 3.79 (s, 3H), δ 6.81 (d, J = 21 Hz, 1H), δ 6.89 (d, J = 39 Hz, 1H), δ 7.03 (d, J = 21 Hz, 1H), δ 7.08 (m, 1H), δ 7.41 (br, d. J = 21 Hz, 1H), δ 7.53 (m, 1H), δ 7.60 (m, 1H), δ 7.65 (br, s, 1H), δ 8.42 (br, s, 1H). MS (APCI) (M+H)+ at m/z 442.

### Example 99

### (2-(Azetidin-1-yl)phenyl)[2-trifluoromethyl-4-(E-((4-(tert-butoxycarbonyl)piperazin-1-yl)carbonyl)ethenyl)phenyl]sulfide

The title compound was prepared by the procedures described in Example 69 substituting pyrrolidine with azetidine hydrochloride, and the bromide from Example 12 with bromide from Example 90, giving a white solid. ¹H NMR (CDCl₃, 300 MHz) δ1.48 (s, 9H), 2.18 (pentet, *J* = 7.43 Hz, 2H), 3.40-3.53 (m, 4H), 3.53-3.77 (m, 4H), 4.02 (t, *J* = 7.43 Hz, 4H), 6.54 (d, *J* = 8.7 Hz, 1H), 6.72 (d, *J* = 8.7 Hz, 1H), 6.78 (tt, *J* = 1.5, 7.35 Hz, 1H), 6.81 (d, *J* = 15.6 Hz, 1H), 7.29-7.42 (m, 3H), 7.61 (d, *J* = 15.6 Hz, 1H), 7.75 (br s, 1H). MS (APCI⁺) (M+H)⁺ at *m*/*z* 548.

### Example 100

### (2-(Piperidin-1-yl)phenyl)[2-trifluoromethyl-4-(E-((4-(tert-butoxycarbonyl)piperazin-1-yl)carbonyl)ethenyl)phenyl]sulfide

The title compound was prepared by the procedures described in Example 69 substituting pyrrolidine with piperidine, and the bromide from Example 12 with bromide from Example 90, and isolated as a white solid. ¹H NMR (CDCl₃, 300 MHz) δ1.48 (s, 9H), 1.54 (br s, 6H), 2.96 (br s, 4H), 3.48 (br s, 4H), 3.55-3.78 (m. 4H), 6.86 (d, *J* = 15.6 Hz, 1H), 6.99 (td, *J* = 1.8, 7.5 Hz, 1H), 7.08 (d, *J* = 8.4 Hz, 1H), 7.19 (dd, *J =* 1.8, 8.1 Hz, 1H), 7.25 (br m, 1H), 7.31 (td, *J* = 1.8, 7.5 Hz, 1H), 7.42 (dd. *J* = 1.8, 8.4 Hz, 1H), 7.65 (d, *J* = 15.6 Hz, 1H), 7.71 (d, *J* = 1.8 Hz, 1H). MS (APCI⁻) (M+H)⁻ at *m*/*z* 576.

### Example 101

### (3-Chloro-2-formylphenyl)[2-chloro-4-(E-((4-acetylpiperazin-1-yl)carbonyl)ethenyl)phenyl]sulfide

The title compound was prepared by the procedures described in Example 65B substituting 2,3-dichlorobenzaldehyde with 2,6-dichlorobenzaldehyde, isolated as a white solid. ¹H NMR (CDCl₃, 300 MHz) δ 2.05 (s, 3H), 3.56 (br s, 2H), 3.61-3.86 (m, 6H), 6.68 (q, *J* = 3.0 Hz, 1H), 6.93 (d, *J* = 15.6 Hz, 1H), 7.23 (d, *J* = 3.0 Hz, 1H), 7.25 (m, 1H), 7.45 (dd, *J* = 2.1, 8.4 Hz, 1H), 7.62 (d, *J* = 8.4 Hz, 1H), 7.67 *(d, J =* 15.6 Hz, 1H), 7.69 (d, *J* = 2.1 Hz, 1H). MS (APCI⁺) (M+H)⁺ at *m*/*z* 463, 465, 467.

### Example 102

### (2-Trifluoromethylphenyl)[2-trifluoromethyl-4-(E-((4-acetylpiperazin-1-yl)carbonyl)ethenyl)phenyl]sulfide

The title compound was prepared according to the procedures of Example 1. ¹H NMR (CDCl₃, 300 MHz) δ 7.84 (s, 1H), 7.80 (m, 1H), 7.66 (d, 1H, J = 15.4 Hz), 7.49 (m, 3H), 7.40 (m, 1H), 7.06 (d, 1H, J = 8.0 Hz), 6.87 (d, 1H, J = 15.4 Hz), 3.62-3.80 (m, 6H), 3.53 (m, 2H). 2.15 (s, 3H). MS (ESI) *m*/*z* 503, 525, 1027.

### Example 103

### (3-Bromophenyl)[2-trifluoromethyl-4-(E-((4-acetylpiperazin-1-yl)carbonyl)ethenyl)phenyl]sulfide

The title compound was prepared according to the procedures of Example 1. ¹H NMR (CDCl₃, 300 MHz) δ 7.83 (s, 1H), 7.66 (d, 1H, J = 15.4 Hz), 7.57 (t, 1H, J = 1.9 Hz), 7.49 (m, 2H), 7.36 (dt, 1H, J = 1.6, 7.8 Hz), 7.24 (m, 1H), 7.18 (d, 1H, J = 8.1 Hz), 6.87 (d, 1 H, J = 15.2 Hz), 3.62-3.82 (m, 6H), 3.54 (m, 2H), 2.15 (s, 3H). MS (ESI) *m*/*z* 514, 515, 535, 537.

### Example 104

### (3.5-Dimethylphenyl)[2-trifluoromethyl-4-(E-((4-acetylpiperazin-1-yl)carbonyl)ethenyl)phenyl]sulfide

The title compound was prepared according to the procedures of Example 1. ¹H NMR (CDCl₃, 300 MHz) δ 7.79 (s, 1H), 7.64 (d, 1H, J = 15.1 Hz), 7.42 (d, 1H, J = 8.8 Hz), 7.49 (m, 2H), 7.13 (s, 2H), 7.04 (s. 2H), 6.84 (d, 1 H, J = 15.2 Hz), 3.62-3.82 (m, 6H), 3.54 (m, 2H), 2.32 (s, 6H), 2.15 (s, 3H). MS (ESI) *m*/*z* 463, 485, 925, 947.

### Example 105

### (2-Isopropylphenyl)[2-nitro-4-(E-((3-dimethylaminocarbonyl-4-(pyridine-4-carbonyl)piperaerazin-1-yl)carbonyl)ethenhyl)phenyl]sulfide

Prepared according to the procedures of Example 71, giving a yellow solid. ¹H NMR (DMSO-d₆, 300MHz) δ 1.14 (d, J = 6.6 Hz, 6H); 2.50-3.83 (br m, 10H); 4.04-4.66 (br m, 3H); 5.32-5.43 (br m, 1H); 6.60-6.69 (m, 1H); 7.15-7.64 (m, 8H); 7.85-7.93 (m, 1H); 8.59-8.72 (m, 3H). MS (APCl) (M+H)- at m/z 588. Anal calcd for C₃₁H₃₃N₅S₁O₅^{·}0.67H₂O: C, 62.07; H, 5.77; N, 11.68. Found: C, 62.13; H, 6.01; N, 11.48.

### Example 106

### (2-Isopropylphenyl)[2-nitro-4-(E-((3-dimethylaminocarbonyl-4-carbomethoxypiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 2.50-3.83 (br m, 14H); 4.16-4.63 (br m, 2H); 4.98 (br s, 1H); 6.60-6.69 (m, 1H); 7.20-7.61 (m, 6H); 7.85-7.93 (m, 1H); 8.59-8.65 (m, 1H). MS (APCI) (M+H)⁺ at m/z 541.

### Example 107

### (2-Isopropylphenyl)[2-nitro-4-(E-((3-dimethylaminocarbonyl-4-acetylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 1.88, 2.04 (s, s, 3H); 2.50-3.83 (br m, 11H); 4.16-4.59 (br m, 2H); 5.04-5.25 (br m, 1H); 6.60-6.69 (m, 1H); 7.21-7.62 (m, 6H); 7.85-7.93 (m, 1H); 8.58-8.65 (m, 1H). MS (APCI) (M+H)⁺ at m/z 525. Anal calcd for C₂₇H₃₂N₄S₁O₅: C, 61.81; H, 6.15; N, 10.68. Found: C, 61.93; H, 6.75; N, 9.67.

### Example 108

### (2-Isopropylphenyl)[2-nitro-4-(E-((3-(1-morpholinocarbonyl)-4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.11-1.16 (br m, 6H); 1.35, 1.40 (br s, br s, 9H); 2.67-5.0(br m, 16H); 6.60-6.69 (m, 1H); 7.28-7.62 (m, 6H); 7.87-7.92 (m, 1H); 8.63-8.67 (br m, 1H). MS (APCI) (M+H)- at m/z 625. Anal calcd for C₃₂H₄₀N₄S₁O₇: C, 61.52; H, 6.45; N, 8.97. Found: C, 61.10; H, 6.65; N, 8.60.

### Example 109

### (2-Isopropylphenyl)[2-nitro-4-(E-((3-(pyridine-4-methylaminocarbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 2.50-4.46 (br m, 10H); 6.63 (d, J = 8.5 Hz, 1H); 7.20-7.64 (m, 8H); 7.85-7.93 (m, 1H); 8.43-8.65 (m, 4H). MS (APCI) (M+H)- at m/z 546. Anal calcd for C₂₉H₃₁N₅S₁O₄^{·}0.46CH₃COOCH₂CH₃: C, 63.20; H. 5.96; N, 11.95. Found: C, 63.29; H, 6.27; N, 11.97.

### Example 110

### (2-Isopropylphenyl)[2-nitro-4-(E-(((3-dimethylaminocarbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 2.50-3.20 (br m, 4H); 2.82 (s, 3H); 3.04 (s, 3H); 3.26-3.49 (m, 1H): 3.52-3.59 (m, 1H); 4.08-4.47 (br m, 2H); 6.63 (d, J = 8.5 Hz, 1H); 7.31-7.62 (m, 6H); 7.86-7.92 (m, 1H); 8.61 (br m, 1H). MS (APCI) (M+H)⁺ at m/z 483. Anal calcd for C₂₅H₃₀N₄S₁O₄·0.39CH₃COOCH₂CH₃: C, 61.71; H, 6.46; N, 10.84. Found: C, 61.96; H, 6.69; N, 10.73.

### Example 111

### (2-Isopropylphenyl)[2-nitro-4-(E-((3-benzylaminocarbonyl)-4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 1.33, 1.42 (br s, br s, 9H); 2.75-4.77 (br m, 10H); 6.60-6.66 (br m, 1H); 7.02-7.94 (br m, 12H); 8.47-8.67 (m, 2H). MS (APCI) (M+H)⁺ at m/z 645.

### Example 112

### (2-Isopropylphenyl)[2-nitro-4-(E-((3-(dimethylaminocarbonyl)-4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 1.35, 1.40 (br s, br s, 9H) 2.50-4.99 (br m, 14H); 6.60-6.69 (m, 1H); 7.21-7.62 (m, 6H); 7.86-7.92 (m, 1H); 8.59-8.63 (br m, 1H). MS (APCI) (M+H)⁺ at m/z 583. Anal calcd for C₃₀H₃₈N₄S₁O₆·0.21C₆H₁₄: C, 62.50; H, 6.87; N, 9.32. Found: C. 62.28; H, 7.15; N, 9.11.

### Example 113

### (2-Bromophenyl)[2-chloro-4-(E-((3-(5S-hydroxymethyl-pyrrolidin-2-on-1-yl)prop-1-lyamino)carbonyl)ethenyl)phenyl]sulfide

(2-Bromophenyl)[2-chloro-4-(2-carboxy-E-ethenyl)phenyl]sulfide was prepared by the procedures described in Example 1 substituting 2,4 dichlorothiophenol with 2-bromothiophenol, 2-chlorobenzaldehyde with 3,4 dichlorobenzaldehyde. 1-(3-aminopropyl)-5-((*S*)-thexyldimethylsilyloxymethyl)-2-pyrrolidinone (0.2818g, 0.8959 mmol) was added to a solution of this cinnamic acid (0.3312g, 0.8959 mmol), 1-[3-(dimethylamino)propyl]-3-ethyl carbodiimide hydrochloride (0.3435g, 1.79 mmol), and 1-hydroxybenzotriazole hydrate (0.1816g, 1.34 mmol) in DMF (4.0 mL). After stirring for 12h the reaction mixture was diluted with EtOAc (250 mL), extracted with sat. NH₄Cl (1x75 mL), extracted with H₂O (2x75 mL), rinsed with brine (75mL), and dried over Na₂SO₄. The resultant thexyldimethylsilyl alcohol was purified by flash chromatography (EtOAc) on silica gel (.4974 g, 83%). Tetrabutylammonium fluoride (.68 mL of 1.0 M solution in THF) was added dropwise to a solution of this protected alcohol (0.4544 g, 0.682 mmol) in THF (1.7 mL). After 2h the reaction was diluted with EtOAc (50 mL) and extracted with sat. NH₄Cl (1x25 mL), extracted with H₂O (2x25 mL), rinsed with brine (25mL), and dried over Na₂SO₄. Flash chromatography (EtOAc → 9:1 CH₂Cl₂:MeOH) on silica gel yielded the title compound (.3144g, 88%). ¹H-NMR (DMSO-d₆, 300MHz) δ 8.14 (t, *J* = 5.5 Hz, 1H), 7.81 (m, 2H), 7.53 (dd, *J* = 8.3, 1.7 Hz, 1H), 7.44 (dt, *J* = 7.7, 1.5, 1H), 7.40 (dt, *J* = 7.7, 1.8, 1H), 7.39 (d, *J* = 15.6 Hz, 1H), 7.28 (dd, *J* = 7.7, 1.8 Hz, 1H), 7.05 (d, *J* = 8.1 Hz, 1H), 6.67 (d, *J* = 15.6 Hz, 1H), 4.84 (t, *J* = 5.1 Hz, 1H), 2.94-3.62 (m, 8H), 1.54-2.29 (m, 6H), MS(APCI) (M+H)⁺ at m/z 523, 525, 527, 529.

### Example 114

### (2-Bromophenyl)[2-chloro-4-(E-((3-(pyrrolidin-2-on-1-yl)prop-1-ylamino)carbonyl) ethenyl)phenyl]sulfide

The title compound was prepared by the procedures described in Example I substituting 2,4 dichlorothiophenol with 2-bromothiophenol, 2-chlorobenzaldehyde with 3,4 dichlorobenzaldehyde, and 6-amino-1-hexanol with 1-(3-aminopropyl)- 2-pyrrolidinone. ¹H-NMR (DMSO-d₆, 300MHz) δ 8.12 (t, *J* = 5.9 Hz, 1H), 7.81 (m, 2H), 7.52 (dd, *J* = 8.1, 2.0 Hz, 1H), 7.44 (dt, *J* = 7.5,1.4, 1H), 7.34 (dt, *J* = 7.5, 2.0, 1H), 7.39 (d, *J* = 15.8 Hz, 1H), 7.28 (dd, *J* = 7.6, 1.9 Hz, 1H), 7.05 (d, *J* = 8.1 Hz, 1H), 6.67 (d, *J* = 15.8 Hz, 1H), 4.02 (d, *J* = .7 Hz. 1H), 3.29-3.35 (m, 2H), 3.11-3.25 (m, 4H). 2.21 (t, *J* = 8.1 Hz, 1H). 1.94 (m, 2H), 1.64 (m, 2H), MS(APCI) (M+H)⁺ at m/z 493, 495, 497, 499.

### Example 115

### (2-Bromophenyl)[2-chloro-4-(E-(N-methyl-N-(3-(pyrrolidin-2-on-1-yl)prop-1-yl)amino)carbonyl)ethenyl)phenyl]sulfide

The title compound was prepared by the procedures described in Example 1 substituting 2,4 dichlorothiophenol with 2-bromothiophenol, 2-chlorobenzaldehyde with 3,4 dichlorobenzaldehyde, and 6-amino-1-hexanol with 1-(3-methylaminopropyl)-2-pyrrolidinone. ¹H-NMR (DMSO-d₆, 300MHz) δ 8.06 (d, *J =* 1.5 Hz, 1H), 7.80 (dd, *J* = 7.7, 1.1 Hz, 1H), 7.64 (dd, *J* = 8.5, 1.7 Hz, 1H), 7.25-7.46 (m, 5H), 7.04 (d, *J* =8. 1, 1.1, 1H), 3.14-5.30 (m, 6H), 3.14 (s, 1H), 2.91 (s, 2H), 2.19 (m, 2H), 1.92 (m, 2H), 1.68 (m, 2H), MS(APCI) (M+H)⁺ at m/z 507, 509, 511, 513.

### Example 116

### (2-[2-Methoxy]ethoxyphenyl)-[2-chloro-4(E-[(morpholin-1-yl)carbonyl]ethenyl)phenyl]sulfide

The title compound was prepared according to the procedures of Example 97, substituting 2-methoxyethoxybenzene, giving a white solid. ¹H NMR (CDCl₃, 300 MHz) δ 3.29 (s, 3H), 3.60 (t, J =7Hz, 2H), 3.60-3.78 (m, 8H), 4.12 (t, J =7Hz, 2H), 6.78 (d, J =15Hz, 1H), 6.82 (d, J =9H, 1H), 6.95-7.03 (m, 2H), 7.18 (dd, J =9Hz. 2Hz, 1H), 7.36-7.45 (m, 2H), 7.52 (d, J =2Hz, 1H), 7.57 (d, J =15Hz, 1H). Anal. Calcd. for C₂₂H₂₄ClNO₄S: C, 60.85; H, 5.57; N, 3.22. Found: C, 60.65; H, 5.59; N. 3.12.

### Example 117

### (2-Isopropylphenyl)[2-nitro-4-(E-((3-(morpholinocarbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 2.50-3.40 (br m, 6H); 3.42-3.64 (br m, 8H); 4.07-4.44 (br m, 2H); 4.08-4.47 (br m, 2H); 6.64 (d, J = 8.5 Hz, 1H); 7.31-7.62 (m, 6H); 7.87-7.92 (m, 1H); 8.61 (br m, 1H). MS (APCI) (M+H)⁻ at m/z 525. Anal calcd for C₂₇H₃₂N₄S₁O₅·1.57H₂O: C, 58.64; H, 6.41; N, 10.13. Found: C, 58.69; H, 6.36; N, 9.78.

### Example 118

### (2-Isopropylphenyl)[2-nitro-4-(E-((4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 1.41 (s, 9H); 3.30-3.40 (m, 1H); 3.50-3.72 (br m, 8H); 6.64 (d, J = 8.5 Hz, 1H); 7.34-7.62 (m, 6H); 7.87-7.92 (dd, J = 8.5, 1.5 Hz, 1H); 8.65 (d, J = 1.5 Hz, 1H). MS (APCI) (M+H)⁺ at m/z 512. Anal calcd for C₂₇H₃₃N₃S₁O₅: C, 63.38; H, 6.50; N, 8.21. Found: C, 63.69; H, 6.62; N, 7.87.

### Example 119

### (2-Isopropylphenyl)[2-nitro-4-(E-((4-methoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 3.62 (s, 3H); 3.30-3.38 (m, 1H); 3.38-3.72 (br m, 8H); 6.64 (d, J = 8.8 Hz, 1H); 7.34-7.62 (m, 6H); 7.87-7.92 (dd, J = 8.8, 2.0 Hz, 1H); 8.64 (d, J = 2.0 Hz, 1H). MS (APCI) (M+H)⁺ at m/z 470. Anal calcd for C₂₄H₂₇N₃S₁O₅·0.34C₆H₁₄: C, 62.77; H, 6.27; N, 8.44. Found: C, 62.70; H, 6.33; N, 8.27.

### Example 120

### (2-Isopropylphenyl)[2-nitro-4-(E-(4-(pyridine-4-carbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 3.30-3.40 (m, 1H); 3.52-3.86 (br m, 8H); 6.61-6.66 (br m, 1H); 7.30-7.62 (m, 8H); 7.83-7.96 (br m, 1H); 8.60-8.71 (m, 3H). MS (APCI) (M+H)⁻ at m/z 517. Anal calcd for C₂₈H₂₈N₄S₁O₄·0.38CH₃COOCH₂CH₃: C, 64.46; H, 5.69; N, 10.19. Found: C, 64.52; H, 5.94; N, 10.21.

### Example 121

### (2-Isopropylphenyl)[2-nitro-4-(E-((3-(pyridine-3-methylaminocarbonyl)-4-tert-butoxycarbonylpiperazin-1-yl)carbonyl)ethenyl)phenyl]sulfide

Yellow solid; ¹H NMR (DMSO-d₆, 300MHz) δ 1.14 (d, J = 6.8 Hz, 6H); 1.31-1.46 (br m, 9H); 3.30-3.41 (m, 1H); 3.15-4.78 (br m, 9H); 6.61-6.67 (br m, 1H); 7.05-7.95 (br m, 9H); 8.20-8.65 (br m, 4H). MS (APCI) (M+H)⁺ at m/z 646. Anal calcd for C₃₄H₃₉N₅S₁O₆·0.13H₂O: C, 62.97; H, 6.49; N, 10.79. Found: C, 62.66; H, 6.26; N, 10.60.

### Example 122

### (2-Isopropylphenyl)[2-nitro-4-(E-((3-(pyridine-2-methylaminocarbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0Hz, 6H); 3.30-3.41 (m, 1H); 2.50-4.46 (br m, 9H); 6.64 (d, J = 8.5 Hz, 1H); 7.21-7.93 (br m, 10H); 8.45-8.65 (br m, 3H). MS (APCI) (M+H)⁺ at m/z 546.

### Example 123

### (2-Isopropylphenyl)[2-nitro-4-(E-((3-(pyridine-3-methylaminocarbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 2.50-4.41 (br m, 10H); 6.61-6.67 (br m, 1H); 7.26-7.70 (br m, 8H); 7.86-7.94 (br m, 1H); 8.40-8.67 (br m, 4H). MS (APCI) (M+H)⁺ at m/z 546.

### Example 124

### (4-Hydroxyphenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 4-hydroxythiophenol. Yellow solid (23 mg, 45%); ¹H-NMR (Pyridine-d₅, 500 MHz) δ 2.08 (s, 3H), 3.42 (br, m, 2H), 3.76 (br, m, 6H), 7.01 (d, J = 17 Hz, 1H), 7.26 (m, 2H), 7.37 (d, J = 31 Hz, 1H), 7.59 (m, 3H), 8.02 (d, J = 31 Hz, 1H), 8.60 (d, J = 4 Hz, 1H). MS (APCI) (M+H)⁺ at m/z 428. FAB High Resolution MS calculated m/z for C₂₁H₂₂N₃O₅S (M+H)⁺: 428.1280. Observed m/z: 428.1296.

### Example 125

### (3,5-Dichlorophenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 3,5-dichlorothiophenol. Yellow solid (12 mg, 21%); ¹H-NMR (CDCl₃, 400 MHz) δ 2.04 (s, 3H), 3.43 (br, m, 2H), 3.62 (br, m, 6H), 6.82 (d, J = 22 Hz, 1H), 6.82 (d, J = 38 Hz, 1H), 7.37 (s, 1H), 7.38 (s, 1H), 7.40 (m, 1H), 7.43 (dd. J = 3, 21 Hz, 1H), 7.55 (d, J = 38 Hz, 1H), 8.29 (d, J = 4 Hz, 1H). MS (APCI) (M+H)⁻ at m/z 480. FAB High Resolution MS calculated m/z for C₂₁H₂₀N₃O₄Cl₂S (M+H)⁺: 480.0552. Observed m/z: 480.0553.

### Example 126

### (2-Bromophenyl)[2-chloro-4-(E-((3-(5S-acetoxymethyl-pyrrolidin-2-on-1-yl)prop-1-ylamino)carbonyl)ethenyl)phenyl]sulfide

To a solution of the compound of Example 113 (0.0466g, 0.0889 mmol) in CH₂Cl₂ (.5 mL) was added triethylamine (0.024 mL, 0.18 mmol) and acetic anhydride (0.0088 mL, 0.0933 mmol). After 12 h the reaction was diluted with MeOH (1.5 mL) and purified by preparative HPLC to provide the title compound (.0458 g. 91%). ¹H-NMR (DMSO-d₆, 300MHz) δ 8.14 (t, *J* = 5.7 Hz, 1H), 7.80 (m, 2H), 7.53 (dd, *J* = 8.5, 1.5 Hz, 1H), 7.45 (dt, *J* = 7.7, 1.5, 1H), 7.35 (dt, *J* = 7.7, 1.8, 1H), 7.39 (d, *J* = 15.6 Hz, 1H), 7.29 (dd, *J* = 7.7, 1.8 Hz, 1H), 7.05 (d, *J* = 8.1 Hz, 1H), 6.67 (d, *J* = 15.6 Hz, 1H), 4.20 (dd, J = 11.8, 3.7 Hz, 1H), 4.03 (dd, J = 11.8, 4.0 Hz, 1H), 3.85 (m, 1H), 3.45 (m, 2H), 3.15 (m, 2H), 2.95 (m, 2H), 2.00-2.48 (m, 2H), 2.02 (s, 3H), 1.51-1.82 (m, 2H), MS(APCI) (M+H)⁺ at m/z 565, 567, 569, 571.

### Example 127

### (2-Bromophenyl)[2-chloro-4-(E-((3-(5S-methoxymethyl-pyrrolidin-2-on-1-yl)prop-1-ylamino)carbonyl)ethenyl)phenyl]sulfide

Sodium hydride (0.0088g, 0.22 mmol, 60% dispersion) was added to a solution of the compound of Example 113 (0.0524g, 0.1 mmol) in DMF (0.5 mL). After 15 min, iodomethane (0.025 mL, 0.4 mmol) was added and the reaction was stirred for 12 h. The reaction was diluted with EtOAc (7 mL) and extracted with sat. NH₄Cl (1x2.5 mL), extracted with H₂O (2x2.5 mL), rinsed with brine (2.5mL), dried over Na₂SO₄, filtered, and concentrated *in vacuo.* The crude products were diluted with MeOH (1.5 mL) and purified by preparative HPLC to provide the title compound (0.0408 g, 74%). ¹H-NMR (DMSO-d₆, 300MHz) δ 8.07 (2, 1H), 7.80 (dd, *J* = 7.9, 1.3 Hz, 1H), 7.64 (dd, *J* = 8.3, 1.6 Hz, 1H), 7.23-7.46 (m, 5H), 7.04 (d, *J* = 8.1, 1H). 3.74 (m, 1H), 4.4-3.52 (m, 6H), 3.27 (s, 1.5H), 3.22 (s, 1.5H), 3.14 (s, 1.5H), 2.91 (s, 1.5H), 1.5-2.3 (m, 6H), MS(APCI) (M+H)⁺ at m/z 551, 553, 555.

### Example 128

### (2-Bromophenyl)[2-chloro-4-(E-((3-(4R-hydroxymethyl-pyrrolidin-2-on-1-yl)prop-1-ylamino)carbonyl)ethenyl)phenyl]sulfide

The title compound was prepared by the procedures described for Example 113 substituting 1-(3-aminopropyl)-5-((*S*)-thexyldimethylsilyloxymethyl)-2-pyrrolidinone with 1-(3-aminopropyl)-4-((*R*)-thexyldimethylsilyloxy)-2-pyrrolidinone. ¹H-NMR (DMSO-d6, 300MHz) δ 8.13 (t, *J* = 5.5 Hz, 1H), 7.80 (m, 2H), 7.53 (dd, *J* = 8.5, 1.7 Hz, 1H), 7.27-7.44 (m, 4H), 7.05 (d, *J* = 8.1 Hz, 1H), 6.67 (d, *J* = 15.8 Hz, 1H), 5.19 (d, J = 3.7 Hz, 1H), 4.28 (br s, 1H), 3.10-3.62 (m, 8H), 2.06 (dd, 1H), 1.63 (m, 1H), MS(APCI) (M+H)⁺ at m/z 509, 511,513.

### Example 129

### Phenyl[2-nitro-4-(E-((4-acetylpiperazin-1-yl)carbonyl)ethenyl)phenyl]sulfide

The title compound was prepared by the procedures described in Example 83 substituting 3,4-dimethylthiophenol with thiophenol. Yellow solid (36 mg, 73%); ¹H-NMR (CDCl₃, 400 MHz) δ 2.20 (s, 3H), 3.59 (br, m, 2H), 3.78 (br, m, 6H), 6.92 (d, J = 21 Hz, 1H), 6.95 (d, J = 39 Hz, 1H), 7.49 (br, d, J = 21 Hz, 1H), 7.56 (m, 3H), 7.65 (m, 2H), 7.69 (d, J = 38 Hz, 1H), 8.46 (d, J = 4 Hz, 1H). MS (APCI) (M+H)⁺ at m/z 412. FAB High Resolution MS calculated m/z for C₂₁H₂₂N₃O₄S (M+H)⁺: 412.1331. Observed m/z: 412.1342.

### Example 130

### (2-Dimethylaminophenyl)[2-nitro-4-(E-((4-acetylpiperazin-1-yl)carbonyl) ethenyl)phenyl]sulfide

To a stirred solution of aniline from Example 47 (21 mg, 0.049 mmol) in 1 mL of ethanol was added Me₂SO₄ (14.0 mL, 0.15 mmol) followed by sat. Na₂CO₃ (25 mL). The mixture was then refluxed for one day. The reaction mixture was allowed to cool down to ambient temperature, partitioned between EtOAc and water. The organic layer was washed with brine, dried over Na₂SO₄, filtered, concentrated under reduced pressure. The residue was then purified on a Gilson Preparative HPLC as described in Example 38B to give the title compound (10 mg, 45% yield), as a light yellow solid. ¹H NMR (CDCl₃, 300 MHz) δ 2.16 (s, 3H), 2.83 (s, 3H), 3.32 (br s, 3H), 3.47-3.85 (m, 8H), 6.75 (d, *J* = 8.4 Hz, 1H), 6.78 (d, *J* = 8.4 Hz, 1H), 6.82 (d, *J* = 8.4 Hz, 1H), 6.89 (d, *J* = 15.6 Hz, 1H), 7.40-7.51 (m, 3H). 7.64 (d, *J* = 15.6 Hz, 1H), 8.45 (d, *J* = 1.8 Hz, 1H). MS (APCI⁺) (M+H)⁺ at *m*/*z* 454.

### Example 131

### (3-((2-Hydroxyethyl)aminocarbonyl)phenyl)[2-nitro-4-(E-((4-acetylpiperazin-1-yl)carbonyl)ethenyl)phenyl]sulfide

The title compound was prepared by the procedures described in Example 92B, substituting ammonium chloride with ethanolamine, to give a light yellow solid. ¹H NMR (d⁶-DMSO, 300 MHz) δ 2.04 (s, 3H), 3.30-3.79 (m, 12H), 4.75 (t, *J*= 5.7 Hz, 1H), 6.85 (d, *J* = 8.7 Hz, 1H), 7.42 (d, *J* = 15.6 Hz, 1H), 7.54 (d, *J* = 15.6 Hz, 1H), 7.66 (t, *J* = 7.8 Hz, 1H), 7.79 (d, *J* = 8.1 Hz, 1H), 7.92 (dd, *J* = 2.1, 8.1 Hz, 1H), 8.04 (d, *J* = 8.4 Hz, 1H), 8.11 (s, 1H), 8.62 (t, *J* = 5.7 Hz, 1H), 8.66 (d, *J* = 2.1 Hz, 1H). MS (APCI⁻) (M+Cl)⁻ at *m*/*z* 533, 535.

### Example 132

### (3-((3-(1-Imidazolyl)propyl)aminocarbonyl)phenyl)[2-nitro-4-(E-((4-acetylpiperazin-1-yl)carbonyl)ethenyl)phenyl]sulfide

The title compound was prepared by the procedures described in Example 92B, substituting ammonium chloride with 3-aminopropyl-1-imidazole, as a light yellow solid. ¹H NMR (d⁶-DMSO, 300 MHz) d 1.96 (quintet, *J* = 6.98 Hz, 2H), 2.04 (s. 3H), 3.24 (q, *J* = 6.98 Hz, 2H), 3.35-3.95 (m, 8H), 4.02 (t, *J* = 6.98 Hz, 2H), 6.87 (d, *J* = 8.4 Hz, 1H), 6.88 (s, 1H), 7.19 (s, 1H), 7.41 (d, *J* = 15.6 Hz, 1H), 7.54 (d, *J* = 15.6 Hz, 1H), 7.64 (s, 1H), 7.68 (d, *J* = 7.8 Hz, 1H), 7.79 (dt, *J* = 1.8, 7.8 Hz, 1H), 7.91 (dd, *J* = 1.8, 8.7 Hz, 1H), 8.03 (d, *J* = 7.8 Hz, 1H), 8.09 (t, *J* = 1.8 Hz, 1H), 8.65 (d, *J* = 1.8 Hz, 1H). MS (APCI⁻) (M+Cl)⁻ at *m*/*z* 597, 599.

### Example 133

### (3-((2-(1-Morpholinyl)ethyl)aminocarbonyl)phenyl)[2-nitro-4-(E-((4-acetylpiperazin-1-yl)carbonyl)ethenyl)phenyl]sulfide

The title compound was prepared by the procedures described in Example 92B, substituting ammonium chloride with 2-aminoethyl-1-morpholine, as a light yellow solid. ¹H NMR (d⁶-DMSO, 300 MHz) δ 2.04 (s, 3H), 2.44 (br s, 4H). 3.20-3.80 (m, 16H), 6.87 (d, *J* = 8.4 Hz, 1H), 7.41 (d, *J* = 15.6 Hz, 1H), 7.54 (d, *J* = 15.6 Hz, 1H), 7.68 (d, *J* = 8.4 Hz, 1H), 7.79 (d, *J* = 8.4 Hz, 1H), 7.91 (dd, *J* = 2.1, 8.4 Hz, 1H), 8.02 (d, *J* = 8.4 Hz, 1H), 8.07 (s, 1H), 8.58 (t, *J*= 6.0 Hz, 1H), 8.65 (d, *J* = 2.1 Hz, 1H). MS (APCI⁺) (M+H)⁺ at *m*/*z* 568.

### Example 134

### (2-Isopropylphenyl)[2-nitro-4-(E-((3-hydroxymethyl-4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 1.41 (s, 9H); 2.62-3.20 (br m, 4H); 3.30-3.40 (m, 1H); 3.72-4.44 (br m, 4H); 4.72-4.98 (br m, 1H); 6.62-6.66 (br m, 1H); 7.25-7.63 (m, 6H); 7.83-7.93 (br m, 1H); 8.57-8.66 (br m, 1H). MS (APCI) (M+H)⁻ at m/z 542. Anal calcd for C₂₈H₃₅N₃S₁O₆·0.21C₆H₁₄: C, 62.78; H, 6.83; N, 7.51. Found: C, 62.65; H, 6.99; N, 7.36.

### Example 135

### (2-Isopropylphenyl)[2-nitro-4-(E-((4-formylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.1 Hz, 6H); 3.30-3.38 (m. 1H); 3.38-3.77 (br m, 8H); 6.64 (d, J = 8.5 Hz, 1H); 7.34-7.62 (m, 6H); 7.88-7.92 (dd, J = 8.5, 1.7 Hz, 1H); 8.08 (s, 1H); 8.65 (d, J = 1.7 Hz, 1H). MS (APCI) (M+H)⁺ at m/z 440. Anal calcd for C₂₃H₂₅N₃S₁O₄: C, 62.85; H, 5.73; N, 9.56. Found: C, 63.05; H, 5.98; N, 9.47.

### Example 136

### (2-Isopropylphenyl)[2-nitro-4-(E-((2-hydroxymethyl-4-tert-butoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.41 (s, 9H); 2.72-3.50 (br m, 4H); 3.30-3.40 (m, 1H); 3.85-4.52 (br m, 4H); 4.74-4.91 (br m, 1H); 6.62-6.66 (br m, 1H); 7.28-7.62 (m, 6H); 7.81-7.91 (br m, 1H); 8.57-8.66 (br m, 1H). MS (APCI) (M+H)⁺ at m/z 542. Anal calcd for C₂₈H₃₅N₃S₁O₆·0.17C₆H₁₄: C, 62.65; H, 6.77; N, 7.55. Found: C, 62.54; H, 6.83; N, 7.33.

### Example 137

### (2-Ethoxyphenyl)-[2-chloro-4(E-[(3-ethoxycarbonylpiperidin-1-yl)carbonyl]ethenyl)phenyl]sulfide

The title compound was prepared according to the procedures of Example 97. ¹H NMR (CDCl₃, 300 MHz) δ 1.25 (t, J = 7 Hz, 6H), broad peaks totaling 9 protons at 1.50-1.62, 1.65-1.92, 2.01-2.15, 2.45-2.55, 2.95-3.05, 3.13-3.30,3.55-3.68, 3.90-4.10, 4.05 (q, J =7Hz, 2H), 4.15 (q, J =7Hz, 2H), 6.84 (d, J =9Hz, 1H), 6.80-6.95 (broad, 1H), 6.94-6.99 (m, 2H), 7.18 (dd, J =9Hz, 2Hz, 1H), 7.34-7.41 (m, 2H), 7.52 (d, J =15Hz, 1H), 7.55 (d, J =2Hz, 1H). Anal. Calcd. for C₂₅H₂₈ClNO₄S: C, 63.35; H, 5.95; N, 2.95. Found: C, 63.17; H, 6.02; N, 26.02; N, 2.81.

### Example 138

### (3- Aminophenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 3-aminothiophenol. Yellow solid (2.9 mg, 5.6%); ¹H-NMR (CDCl₃, 500 MHz) δ 2.20 (s, 3H), 3.60 (br, m, 2H), 3.77 (br, m. 6H), 4.03 (br, s, 2H), 6.85 (dd, J = 4, 16 Hz, 1H), 6.90 (m, 3H), 7.04 (d, J = 17 Hz, 1H), 7.30 (t, J = 16 Hz, 1H), 7.52 (d, J = 17 Hz, 1H), 7.68(d, J = 31 Hz, 1H), 8.44 (d, J = 4 Hz, 1H). MS (APCI) (M+H)⁺ at m/z 427. FAB High Resolution MS calculated m/z for C₂₁H₂₃N₄O₄S (M+H)⁺: 427.1440. Observed m/z: 427.1440.

### Example 139

### (4-Aminophenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 4-aminothiophenol. Yellow solid (2.5 mg, 4.9%); ¹H-NMR (CDCl₃, 500 MHz) δ 2.19 (s, 3H), 3.58 (br, m, 2H), 3.76 (br, m. 6H), 4.03 (br, s, 2H), 6.80 (m, 1H), 6.93 (m, 3H), 7.37 (m, 1H), 7.46 (d, J = 17 Hz, 1H), 7.67 (d, J = 31 Hz, 1H), 8.43 (d, J = 3 Hz, 1H). MS (APCI) (M+H)⁺ at m/z 427. FAB High Resolution MS calculated m/z for C₂₁H₂₃N₄O₄S (M+H)⁺: 427.1440. Observed m/z: 427.1441.

### Example 140

### (2,4-Dimethylphenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 2,4-dimethylthiophenol. Yellow solid (40 mg, 76%); ¹H-NMR (CDCl₃, 400 MHz) δ 1.54 (br, s, 2H), 2.14 (s, 3H), 3.53 (br, m, 2H), 3.71 (br, m, 6H), 6.58 (d, J = 21 Hz, 1H), 6.76 (d, J = 38 Hz, 1H), 7.03 (m, 1H), 7.09 (m, 1H), 7.28 (br, d, J = 19 Hz, 1H), 7.33 (d, J = 20 Hz, 1H), 7.51 (d, J = 38 Hz, 1H), 8.30 (d, J = 5 Hz, 1H). MS (APCI) (M+H)⁺ at m/z 440. FAB High Resolution MS calculated m/z for C₂₃H₂₆N₃O₄S (M+H)⁻: 440.1644. Observed m/z: 440.1656.

### Example 141

### (2,5-Dimethylphenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 2,5-dimethylthiophenol. Yellow solid (34 mg, 64%); ¹H-NMR (CDCl₃, 400 MHz) δ 2.07 (s, 3H), 2.23 (s, 3H), 2.28 (s, 3H), 3.46 (br, m, 2H), 3.64 (br, m, 6H), 6.65 (d, J = 21 Hz, 1H), 6.81 (d, J = 39 Hz, 1H), 7.19 (m, 2H), 7.34 (m, 2H), 7.56 (d, J = 38 Hz, 1H), 8.35 (d, J = 5 Hz, 1H). MS (APCI) (M+H)⁺ at m/z 440. FAB High Resolution MS calculated m/z for C₂₃H₂₆N₃O₄S (M+H)⁺: 440.1644. Observed m/z: 440.1656.

### Example 142

### (4-Methoxyphenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 4-methoxythiophenol. Yellow solid (44 mg, 83%); ¹H-NMR (CDCl₃, 400 MHz) δ 2.09 (s, 3H), 3.48 (br, m, 2H), 3.66 (br, m, 6H), 3.83 (s, 3H), 6.79 (d, J = 22 Hz, 1H), 6.83 (d, J = 40 Hz, 1H), 6.95 (m, 1H), 6.98 (m, 1H), 7.37 (br, d, J = 20 Hz, 1H), 7.43 (m, 1H), 7.46 (m, 1H), 7.58 (d, J = 38 Hz, 1H), 8.35 (d, J = 4 Hz, 1H). MS (APCI) (M+H)⁺ at m/z 442. FAB High Resolution MS calculated m/z for C₂₂H₂₄N₃O₅S (M+H)⁺: 442.1437. Observed m/z: 442.1434.

### Example 143

### (3-Chlorophenyl)[2-nitro-4-(E-((4-acetylpiperazin-1-yl)carbonyl)ethenyl)phenyl]sulfide

The title compound was prepared by the procedures described in Example 83 substituting 3,4-dimethylthiophenol with 3-chlorothiophenol. Yellow solid (43 mg, 80%); ¹H-NMR (CDCl₃, 400 MHz) δ 2.23 (s, 3H), 3.62 (br, m, 2H), 3.80 (br, m, 6H), 6.97 (d, J = 21 Hz, 1H), 6.99 (d, J = 39 Hz, 1H), 7.28 (d, J = 19 Hz, 1H), 7.57 (m, 3H), 7.675 (t, J = 4 Hz, 1H), 7.73 (d, J = 39 Hz, 1H), 8.48 (d, J = 4 Hz, 1H). FAB High Resolution MS calculated m/z for C₂₁H₂₁N₃O₄ClS (M+H)⁺: 446.0941. Observed m/z: 446.0953.

### Example 144

### (2-Chloro, 4,5-diaminophenyl)[2-chloro-4-(E-((4-acetylpiperazin-1-yl)carbonyl)ethenyl)phenyl]sulfide

### Example 144A

### (2-Chloro, 4-nitro. 5-aminophenyl)[2-chloro-4-(E-((4-acetylpiperazin-1-yl)carbonyl)ethenyl)phenyl]sulfide

The title compound was prepared by the procedures described in Example 65B substituting 2,3-dichlorobenzaldehyde with 4,5-dichloro-2-nitroaniline.

### Example 144B

### (2-Chloro, 4.5-diaminophenyl)[2-chloro-4-(E-((4-acetylpiperazin-1-yl)carbonyl)ethenyl)phenyl]sulfide

To a stirred solution of nitrobenzene from Example 144A (170 mg, 0.34 mmol) in 2 mL of EtOH was added SnCl₂ (325 mg, 1.72 mmol). The mixture was then refluxed under nitrogen atmosphere for 2 h. The reaction was allowed to cool down to ambient temperature, quenched with sat. NaHCO₃, extracted with EtOAc(2×20 mL). The combined organic layer was washed with brine, dried over Na₂SO₄, concentrated in vacuo. The residue was then purified on Gilson preparative HPLC as described in Example 38B to give the title compound (70 mg, 44% yield) as a light yellow solid. ¹H NMR (d⁶-DMSO, 300 MHz) δ 2.04 (s, 3H), 3.42-3.80 (m, 8H), 4.84 (s, 2H), 5.32 (s, 2H), 6.51 (d, *J* = 8.4 Hz, 1H), 6.78 (d, *J* = 8.4 Hz, 2H), 7.26 (d, *J* = 15.6 Hz, 1H), 7.41 (d, *J* = 15.6 Hz, 1H), 7.48 (d, *J* = 8.4 Hz, 1H), 7.95 (d, *J* = 1.8 Hz, 1H). MS (APCI⁺) (M+H)⁻ at *m*/*z* 465, 467, 469, 471.

### Example 145

### (3,4-Diaminophenyl)[2-chloro-4-(E-((4-acetylpiperazin-1-yl)carbonyl)ethenyl)phenyl]sulfide

The title compound was prepared by the procedures described in Example 144, substituting 4,5-dichloronitroaniline with 5-chloronitroaniline, resulting in a light brown solid. ¹H NMR (d⁶-DMSO, 300 MHz) δ 2.04 (s, 3H), 3.31-3.80 (m, 8H), 4.75 (s, 2H), 5.01 (s, 2H), 6.61 (t, *J* = 4.2 Hz, 3H), 6.68 (s, 1H), 7.26 (d, *J* = 15.6 Hz, 1H), 7.40 (d, *J* = 15.6 Hz, 1H), 7.46 (d. *J* = 8.4 Hz, 1H), 7.94 (s, 1H), MS (APCI⁺) (M+H)⁺ at *m*/*z* 431, 433.

### Example 146

### (6-Chlorobenzimidazol-2-on-5-yl)[2-chloro-4-(E-((4-acetylpiperazin-1-yl)carbonyl)ethenyl)phenyl]sulfide

A mixture of dianiline from Example 144 (35 mg, 0.075 mmol) and CDI (13 mg, 0.075 mmol) in THF was stirred at ambient temperature for one day. Solvent was then removed under reduced pressure. The crude product then purified on a Gilson preparative HPLC as described in Example 38B to give the title compound (12 mg, 32% yield) as a white solid. ¹H NMR (d⁶-DMSO, 300 MHz) δ 2.04 (s, 3H), 3.40-3.80 (m, 8H), 6.63 (d, *J* = 8.4 Hz, 1H), 7.11 (d, *J* = 2.4 Hz, 1H), 7.12 (s, 1H), 7.23 (s, 1H), 7.32 (d, *J* = 15.6 Hz, 1H), 7.43 (d, *J* = 15.6 Hz, 1H), 7.50 (d, *J* = 8.4 Hz, 1H), 8.03 (br s, 1H). MS (APCI⁻) (M-CO+H)⁺ at *m*/*z* 465, 467.

### Example 147

### (1-Methylindol-7-yl)[2-chloro-4-(E-((4-acetylpiperazin-1-yl)carbonyl)ethenyl)phenyl]sulfide

The title compound was prepared by the procedures described in 85. substituting 5-iodoindole with *N*-methyl-7-bromoindole, giving a light brown solid. ¹H NMR (CDCl₃, 300 MHz) δ 2.14 (s, 3H), 3.47-3.56 (m, 2H), 3.56-3.83 (m, 6H), 3.96 (s, 3H), 6.42 (d, *J*= 8.4 Hz, 1H), 6.55 (d, *J* = 3.6 Hz, 1H), 6.76 (d, *J*= 15.6 Hz, 1H), 6.99 (d, *J* = 3.6 Hz, 1H), 7.09 (dd, *J* = 2.1, 8.4 Hz, 1H), 7.15 (t, *J* = 7.65 Hz, 1H), 7.42 (dd, *J* = 0.9, 7.5 Hz, 1H), 7.53 (d, *J* = 1.8 Hz, 1H), 7.55 (dd, *J* = 15.6 Hz, 1H), 7.77 (dd, *J* = 0.9, 7.5 Hz, 1H). MS (APCI⁺) (M+H)⁺ at *m*/*z* 454, 456.

### Example 148

### (2-Hydroxy, 4-aminophenyl)[2-chloro-4-(E-((4-acetylpiperazin-1-yl)carbonyl)ethenyl)phenyl]sulfide

The title compound was prepared by the procedures described in Example 144, substituting 4,5-dichloronitroaniline with 5-chloronitrophenol, giving a light brown solid. ¹H NMR (d⁶-DMSO, 300 MHz) δ 2.04 (s, 3H), 3.41-3.80 (m,8H), 5.09 (s, 2H), 6.61 (d, *J* = 8.4 Hz, 1H), 6.70 (d, *J* = 7.8 Hz, 1H), 6.79 (s, 1H), 6.80 (dd, *J* = 2.1, 7.8 Hz, 1H), 7.26 (d, *J* = 15.6 Hz, 1H), 7.40 (d, *J* = 15.6 Hz, 1H), 7.46 (d, *J* = 8.4 Hz, 1H), 7.94 (br s, 1H). MS (APCI⁺) (M+H)⁺ at *m*/*z* 432, 434.

### Example 149

### (2-Isopropylphenyl)[2-nitro-4-(E-((4-methylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 2.19 (s, 3H); 2.25-2.36 (br m, 4H); 3.30-3.40 (m, 1H); 3.51-3.72 (br m, 4H); 6.63 (d, J = 8.5 Hz, 1H); 7.24-7.63 (m, 6H); 7.88-7.92 (dd, J = 8.8, 1.8 Hz, 1H); 8.64 (d, J = 1.8 Hz, 1H). MS (APCI) (M+H)⁺ at m/z 426. Anal calcd for C₂₃H₂₇N₃S₁O₃^{·}0.26H₂O: C, 64.19; H, 6.45; N, 9.76. Found: C, 64.21; H, 6.59; N, 9.70.

### Example 150

### (2-Isopropylphenyl)[2-nitro-4-(E-((4-(pyridine-2-carbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H): 3.30-3.40 (m, 1H); 3.51-3.83 (br m, 8H); 6.61-6.66 (br m, 1H); 7.30-7.65 (m, 8H); 7.83-7.97 (m, 2H); 8.57-8.67 (m, 2H). MS (APCI) (M+H)⁺ at m/z 517. Anal calcd for C₂₈H₂₈N₄S₁O₄·0.45H₂O: C, 64.07; H, 5.53; N, 10.67. Found: C, 64.04; H, 5.77; N, 10.97.

### Example 151

### (2-Isopropylphenyl)[2-nitro-4-(E-((4-(pyridine-3-carbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 3.30-3.40 (m, 1H); 3.52-3.87 (br m, 8H); 6.64 (d, J = 8.5 Hz, 1H); 7.30-7.64 (m, 7H); 7.83-7.95 (m, 2H); 8.61-8.70 (m, 3H). MS (APCI) (M+H)⁺ at m/z 517. Anal calcd for C₂₈H₂₈N₄S₁O₄·0.42H₂O: C, 64.16; H, 5.55; N, 10.69. Found: C, 64.18; H, 5.64; N, 10.59.

### Example 152

### (2-Isopropylphenyl)[2-nitro-4-(E-((2-carbomethoxy-4-methoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.1 Hz, 6H); 2.70-3.95 (br m, 4H); 3.30-3.40 (m, 1H); 3.61, 3.61 (s, s, 3H); 3.65, 3.67 (s, s, 3H); 4.16-4.50 (br m, 2H); 5.08-5.39 (br m, 1H); 6.64 (dd, J = 8.5, 5.1 Hz, 1H); 7.30-7.63 (m, 6H); 7.83-7.94 (m, 1H); 8.62-8.67 (m, 1H). MS (APCI) (M+H)⁺ at m/z 528. Anal calcd for C₂₆H₂₉N₃S₁O₇·0.19C₆H₁₄: C, 59.94; H, 5.87; N, 7.72. Found: C, 59.87; H, 5.94; N, 7.59.

### Example 153

### (2-Isopropylphenyl)[2-nitro-4-(E-((2-carboxy-4-methoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 2.70-3.95 (br m, 4H); 3.30-3.40 (m, 1H); 3.61,3.61 (s, s, 3H); 4.16-4.51 (br m, 2H); 5.01-5.28 (br m, 1H); 6.61-6.66 (m, 1H); 7.30-7.63 (m, 6H); 7.83-7.94 (m, 1H); 8.66 (br s, 1H). MS (APCI) (M-H)⁻ at m/z 512.

### Example 154

### (2-Isopropylphenyl)[2-nitro-4-(E-((3-carbomethoxy-4-methylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 2.25, 2.26 (s, s, 3H); 2.20-3.98 (br m, 8H); 3.57, 3.63 (s, s, 3H); 6.63 (d, J = 8.5 Hz, 1H); 7.30-7.63 (m, 6H); 7.91 (dd, J = 8.5, 1.5 Hz, 1H); 8.60-8.68 (br m, 1H). MS (APCI) (M-H)⁺ at m/z 484.

### Example 155

### (2-Ethoxyphenyl)-[2-chloro-4(E-[(3-carboxypiperidin-1-yl)carbonyl]ethenyl)phenyl]sulfide

The compound of Example 137 was hydrolyzed using an excess of aqueous 10% NaOH in methanol, stirring overnight. The reaction mixture was concentrated in vacuo, water was added, and the solution was extracted with ether. The mixture was acidified; the resultant solid was collected by filtration and dried overnight in a vacuum oven, giving a while solid, m.p. 166-171 C. ¹H-NMR (DMSO 300 MHz) δ 1.17 (t, J =7Hz, 3H), broad peaks totaling 9 protons at 1.32-1.48, 1.51-1.78. 1.90-2.04, 2.25-2.50, 2.80-2.90. 2.95-3.17, 3.45-3.51, 3.95-4.19, 4.41-4.51, 4.06 (q, J=7Hz, 1H), 6.80 (d, J = 9Hz, 1H), 7.01 (t, J = 7Hz, 1H), 7.15 (d, J = 8Hz, 1H), 7.26-7.40 (m, 2H), 7.40-7.48 (m, 1H), 7.51 (dd, J = 9Hz, 2Hz, 1H), 7.99 (d, J = 9Hz, 1H). Anal. Calcd. for C₂₃H₂₄ClNO₄S: C, 61.94; H, 5.42; N, 3.14 . Found: C, 61.75; H, 5.65; N, 3.15. The resultant acid (303 mg, 0.631 mmol) was dissolved in 3 ml MeOH. A KOH solution (38 mg, 0.595 mmol, of 87.6% KOH ) in 1 ml MeOH was added. The resulting solution was concentrated in vacuo, and 5 ml. ether was added. The mixture was stirred for one hour to form a powder, which was filtered and dried in the vacuum oven at 60C to yield 307 mg of a solid, water soluble product.

### Example 155

### (2-Ethoxyphenyl)-[2-chloro-4(E-[(3-carboxypiperidin-1-yl)carbonyl]ethenyl)phenyl]sulfide

The compound of Example 137 was hydrolyzed using an excess of aqueous 10% NaOH in methanol, stirring overnight. The reaction mixture was concentrated *in vacuo*, water was added, and the solution was extracted with ether, giving a white solid, m.p. 166-171. ¹H NMR (DMSO, 300 MHz) δ 1.17 (t, J = 7Hz, 3H), broad peaks totaling 9 protons at 1.32-1.48, 1.51-1.78, 1.90-2.04, 2.25-2.50, 2.80-2.90, 2.95-3.17, 3.45-3.51, 3.95-4.19, 4.41-4.51, 4.06 (q, J = 7Hz, 1H), 6.80 (d, J = 9Hz, 1H), 7.01 (t, J = 7Hz, 1H), 7.15 (d, J = 8Hz, 1H), 7.26-7.40 (m, 2H), 7.40-7.48 (m, 1H), 7.51 (dd, J = 9Hz, 2Hz, 1H), 7.99 (d, J = 9Hz, 1H). Anal. Calcd. for C₂₃H₂₄ClNO₄S: C, 61.94; H, 5.42; N, 3.14. Found: C, 61.75; H, 5.65; N, 3.15. The resultant acid (303 mg, 0.631 mmol) was dissolved in 3 ml MeOH. A KOH solution (38 mg, 0.595 mmol, of 87.6% KOH ) in 1 ml MeOH was added. The resulting solution was concentrated in vacuo, and 5 ml. ether was added. The mixture was stirred for one hour to form a powder, which was filtered and dried in the vacuum oven at 60C to yield 307 mg of a solid, water soluble product.

### Example 156

### (2-Ethoxyphenyl)-[2-chloro-4(E-[(2-ethoxycarbonylpiperidin-1-yl)carbonyl]ethenyl)phenyl]sulfide

The title compound was prepared according to the procedures of Example 97. ¹H NMR (CDCl₃, 300 MHz) δ 1.24 (t, J = 7Hz, 3H), 1.28 (t, J = 7Hz, 3H), broad peaks totaling 9 protons at 1.35-1.55, 1.65-1.80, 2.25-2.38, 3.33-3.45, 3.95-4.05, 4.15-4.28, 4.60-4.80, 5.44-5.50, 4.05 (q, J = 7Hz, 2H), 4.20 (q, J = 7Hz, 2H), 6.80-6.98 (m, 4H), 7.12-7.20 (m, 1H)7.35-7.43 (m, 2H), 7.50-7.58 (m, 2H). Anal. Calcd. for C₂₅H₂₈ClNO₄S: C, 63.35; H, 5.95; N, 2.95. Found: C,63.51; H, 6.22; N, 2.61.

### Example 157

### (2-Ethoxyphenyl)[2-trifluoromethyl-4-(E-((1-(tert-butoxycarbonyl)-4-hydroxypyrrolidin-3-ylamino)carbonyl)ethenyl)phenyl]sulfide

The title compound was prepared according to the procedures of Example 1. ¹H NMR (CDCl₃, 300 MHz) δ 7.76 (s, 1H), 7.60 (d, 1H, J = 15.1 Hz), 7.46 (dd, 1H, J = 1.7, 7.5 Hz), 7.38 (m, 2H), 7.01 (d, 1H, J = 15.4 Hz), 6.98 (d, 1H, J = 7.8 Hz), 6.93 (d, 1H, J = 8.3 Hz), 6.42 (d, 1H, J = 15.0 Hz), 4.30 (br, 2H), 3.98 (q, 2H, J = 7.0 Hz), 3.87 (m, 1H), 3.71 (m, 1H), 3.33 (br, 2H), 1.47 (s, 9H), 1.17 (t, 3H, J = 7.0 Hz). MS (ESI) *m*/*z* -551, -1103. Anal. Calcd for C₂₇H₃₁F₃N₂O₅S · 0.61 EtOAc: C, 58.32; H, 5.96; N, 4.62. Found: C, 58.07; H, 5.88; N, 4.76.

### Example 158

### (2-Ethoxyphenyl)-[2-chloro-4(E-(2-carboxypiperidin-1-yl)carbonyl]ethenyl)phenyl]sulfide

The compound of Example 156 was hydrolyzed, and the salt formed, according to the procedures of Example 155. m.p. 170-171C. ¹H-NMR (DMSO 300 MHz) δ 1.16 (t, J = 7Hz, 3H), broad peaks totaling 9 protons at 1.20-1.49, 1.51-1.75, 2.10-2.27, 2.55-2.65, 3.10-3.21, 4.20-4.29, 4.35-4.45, 5.13-5.25, 4.05 (q, J = 7Hz, 2H), 6.80 (d, J = 9Hz, 1H), 6.97-7.07 (m, 1H), 7.15 (d, J = 9Hz, 1H), 7.29-7.57 (m, 5H), 8.02 (s, 1H). Anal. Calcd. for C₂₃H₂₄ClNO₄S: C, 61.94; H, 5.42; N, 3.14. Found: C, 61.91; H, 5.48; N, 2.90.

### Example 159

### (2-Ethoxyphenyl)[2-trifluoromethyl-4-(E-(((pyrrol-3-in-1-yl)carbonyl)ethenyl)phenyl]sulfide

The title compound was prepared according to the procedures of Example 1. ¹H NMR (CDCl₃, 300 MHz) δ 7.81 (s, 1H), 7.68 (d, 1H, J = 15.4 Hz), 7..35-7.47 (m, 3H), 7.04 (d, 1H, J = 8.4 Hz), 6.97 (dd, 1H, J = 1.3, 7.5 Hz), 6.91 (d, 1H, J = 8.5 Hz), 6.70 (d, 1H, J = 15.4 Hz), 5.94 (m, 1H), 5.85 (m, 1H), 4.47 (br, 2H), 4.38 (br, 2H), 3.98 (q, 2H, J = 7.0 Hz), 1.19 (t. 3H, J = 7.0 Hz). MS (ESI) *m*/*z* 420, 839, 861.

### Example 160

### (2-Ethoxyphenyl)[2-trifluoromethyl-4-(E-((3-(pyrrolidin-2-on-1-yl)prop-1-ylamino)carbonyl)ethenyl)phenyl]sulfide

The title compound was prepared according to the procedures of Example 1. ¹H NMR (CDCl₃, 300 MHz) δ 7.78 (s, 1H), 7.54 (d, 1H, J = 15.8 Hz), 7.42 (dd, 1H, J = 1.7, 7.5 Hz), 7.34-7.39 (m, 2H), 7.13 (br, 1H), 7.03 (d, 1H, J = 8.5), 6.97 (dd, 1H, J = 1.1, 7.7 Hz), 6.91 (d, 1H, J = 8.1 Hz), 6.46 (d, 1H, J = 15.8 Hz), 3.98 (q, 2H, J = 7.0 Hz), 3.43 (m, 4H), 3.34 (q, 2H, J = 6.0 Hz), 2.45 (t, 2H, J = 8.1 Hz), 2.08 (m, 2H), 1.75 (m, 2H), 1.18 (t, 3H, J = 7.0 Hz). MS (ESI) *m*/*z* 493, 515, 985, 1007.

### Example 161

### (2-Ethoxyphenyl)[2-trifluoromethyl-4-(E-((4-acetylpiperazin-1-yl)carbonyl)ethenyl)phenyl]sulfide

The title compound was prepared according to the procedures of Example 1. ¹H NMR (CDCl₃, 300 MHz) δ 7.79 (s, 1H), 7.62 (d, 1H, J = 15.6 Hz), 7.44 (dd, 1H, J = 1.7, 7.5 Hz), 7.38 (m, 2H), 7.04 (d, 1H, J = 8.1), 6.97 (dd, 1H, J = 1.4, 7.5 Hz), 6.92 (d, 1H, J = 8.1 Hz), 6.84 (d, 1H, J = 15.6 Hz), 3.98 (q, 2H, J = 7.0 Hz), 3.63-78 (m, 6H), 3.53 (m, 2H), 2.14 (s, 3H), 1.19 (t, 3H, J = 7.0 Hz). MS (ESI) *m*/*z* 479, 501, 957, 979.

### Example 162

### (2-Ethoxyphenyl)[2-trifluoromethyl-4-(E-((4-(ethoxycarbonyl)piperazin-1-yl)carbonyl)ethenyl)phenyl]sulfide

The title compound was prepared according to the procedures of Example 71. ¹H NMR (CDCl₃, 300 MHz) δ 7.79 (d, 1H, J = 1.7 Hz), 7.63 (d, 1H, J = 15.3 Hz), 7.43 (dd, 1H, J = 1.7, 7.7 Hz), 7.38 (m, 2H), 7.04 (d, 1H, J = 8.5), 6.97 (dd, 1H, J = 1.4, 7.5 Hz), 6.92 (d, 1H, J = 8.1 Hz), 6.84 (d, 1H, J = 15.3 Hz), 4.18 (q. 2H. J = 7.1 Hz), 3.98 (q, 2H, J = 6.9 Hz), 3.68 (m, 4H), 3.53 (m, 4H), 1.29 (t, 3H, J = 7.1 Hz) 1.19 (t, 3H, J = 6.9 Hz). MS (ESI) m/z 509, 531, 1017, 1039.

### Example 163

### (2-Ethoxyphenyl)[2-trifluoromethyl-4-(E-((4-(2-furylcarbonyl)piperazin-1-yl)carbonyl)ethenyl)phenyl]sulfide

The title compound was prepared according to the procedures of Example 71. ¹H NMR (CDCl₃, 300 MHz) δ 7.80 (d, 1H, J = 1.5 Hz), 7.66 (d, 1H, J = 15.4 Hz), 7.52 (s, 1H), 7.45 (dd, 1H, J = 1.6, 7.5 Hz), 7.40 (m, 2H), 7.08 (d, 1H, J = 4.0 Hz), 7.04 (d, 1H, J = 8.1), 6.98 (dd, 1H, J = 1.1, 7.3 Hz), 6.93 (d, 1H, J = 8.5 Hz), 6.88 (d, 1H, J = 15.4 Hz), 6.52 (dd, 1H, J = 1.6, 3.5 Hz), 3.98 (q, 2H, J = 7.0 Hz), 3.73-3.90 (m,8H), 1.19 (t, 3H, J = 7.0 Hz). MS (ESI) *m*/*z* 531, 553, 1061, 1083.

### Example 164

### (2-Ethoxyphenyl)-[2-chloro-4(E-[(3-ethoxycarbonylpiperidin-1-yl)carbonyl]ethenyl)phenyl]sulfide

The title compound was prepared according to the procedures of Example 97. ¹H-NMR (CDCl₃) δ 1.25 (t, J = 7Hz, 6H), broad peaks totaling 9 protons at 1.65-1.80, 1.95-2.04, 2.51-2.63, 2.90-3.00, 3.15-3.30, 2.95-4.05, 4.42-4.55, 4.14 (q, J = 7Hz, 2H), 4.15 (q, J = 7Hz, 2H), 6.82 (d, J = 15 Hz, 1H), 6.84 (d, J = 9Hz, 1H), 6.93-6.99 (m, 2H), 7.17 (dd, J = 9Hz, 2Hz, 1H), 7.34-7.41 (m, 2H), 7.52 (d, J = 15Hz, 1H), 7.55 (d, J = 2Hz, 1H). Anal. Calcd. for C₂₅H₂₈ClNO₄S: C, 63.35; H, 5.95; N, 2.95. Found: C, 63.09; H, 6.24; N, 2.77.

### Example 165

### (2-Ethoxyphenyl)-[2-chloro-4(E-[(4-carboxypiperidin-1-yl)carbonyl]ethenyl)phenyl]sulfide

The compound of Example 164 was hydrolyzed, and the salt formed, according to the procedures of Example 155. m.p. 165-166C. ¹H-NMR (DMSO 300 MHz) δ 1.25 (t, J = 7Hz, 3H0, 1.35-1.58 (m. 2H), 1.80-1.95 (m, 2H), 2.50-2.60 (m, 1H), 1.78-1.91 (m, 1H), 3.13-3.24 (m, 1H), 4.05 (q, J = 7Hz, 2H), 4.12-4.35 (m, 2H), 6.80 (d, J = 9Hz, 1H), 6.96-7.05 (t, J = 8 Hz, 1H), 7.15 (d, J = 9Hz, 1H), 7.28-7.48 (m, 4H), 7.51 (dd, J = 9Hz, 2Hz, 1H), 8.00 (d, J = 2Hz).

### Example 166

### (Benzodioxan-6-yl)[2-chloro-4-(E-((4-acetylpiperazin-1-yl)carbonyl)ethenyl)phenyl]sulfide

The title compound was prepared by the procedures described in Example 85, substituting 5-iodoindole with 6-iodobenzenedioxane, giving a white solid. ¹H NMR (CDCl₃, 300 MHz) δ 2.14 (s, 3H), 3.44-3.57 (m, 2H), 3.57-3.86 (m, 6H), 4.25-4.35 (m, 4H), 6.75 (d, *J* = 8.4 Hz, 1H), 6.78 (d, *J* = 15.6 Hz. 1H), 6.93 (d, *J* = 8.4 Hz, 1H), 7.03 (dd, *J* = 2.1, 8.4 Hz, 1H), 7.08 (d, *J* = 2. 1 Hz, 1H). 7.18 (dd, *J* = 2.1, 8.4 Hz, 1H), 7.51 (d, *J* = 2.1 Hz, 1H), 7.57 (d, *J* = 15.6 Hz, 1H). MS (APCI⁺) (M+H)⁻ at *m*/*z* 459, 461.

### Example 167

### (2-Isopropylphenyl)[2-nitro-4-(E-((4-ethoxvcarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 1.19 (t, J = 7.0Hz, 3H); 3.30-3.40 (m, 1H); 3.30-3.73 (br m, 8H); 4.06 (q, J = 7.0 Hz, 2H); 6.64 (d, J = 8.5 Hz, 1H); 7.32-7.63 (m, 6H); 7.90 (dd, J = 8.8, 1.8 Hz, 1H); 8.65 (d, J = 1.8 Hz, 1H). MS (APCI) (M+H)⁺ at m/z 484. Anal calcd for C₂₅H₂₉N₃S₁O₅: C, 62.09; H, 6.04; N, 8.69. Found: C, 61.89; H, 6.13; N, 8.51.

### Example 168

### (2-Isopropylphenyl)[2-nitro-4-(E-((4-isopropoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.20 (d, J = 6.4 Hz, 3H); 3.30-3.40 (m, 1H); 3.32-3.73 (br m, 8H); 4.79 (hept, J = 6.1 Hz, 2H); 6.64 (d, J = 8.5 Hz, 1H); 7.32-7.63 (m, 6H); 7.89 (dd, J = 8.5, 1.7 Hz, 1H); 8.64 (d, J = 1.7 Hz, 1H). MS (APCI) (M+H)⁺ at m/z 498. Anal calcd for C₂₆H₃₁N₃S₁O₅: C, 62.76; H. 6.28; N, 8.44. Found: C, 62.57; H, 6.43; N, 8.33.

### Example 169

### (2-Isopropylphenyl)[2-nitro-4-(E-((4-isobutoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 0.90 (d, J = 6.6 Hz, 6H); 1.14 (d, J = 7.0 Hz, 6H); 1.88 (hept, J = 6.6 Hz, 1H); 3.30-3.40 (m, 1H); 3.30-3.73 (br m, 8H); 3.81 (d, J = 6.3 Hz, 2H); 6.64 (d, J = 8.5 Hz, 1H); 7.32-7.63 (m, 6H); 7.90 (dd, J = 8.5, 1.5 Hz, 1H); 8.65 (d, J = 1.5 Hz, 1H). MS (APCI) (M+H)⁺ at m/z 512. Anal calcd for C₂₇H₃₃N₃S₁O₅: C, 63.38; H, 6.50; N, 8.21. Found: C, 63.15; H, 6.55; N, 8.13.

### Example 170

### (2-Isopropylphenyl)[2-nitro-4-(E-((4-((1-propen-2-oxy)carbonyl)piperazin-1-yl)carbonyl)ethenyl)phenyl)sulfide

Prepared according to the procedures of Example 71, giving a yellow solid. ¹H NMR (DMSO-d₆, 300MHz) δ 1.14 (d, J = 6.8 Hz, 6H); 1.88 (s, 3H); 3.30-3.40 (m, 1H); 3.30-3.78 (br m, 8H); 4.65 (s, 1H); 4.69 (m, 1H); 6.64 (d, J = 8.5 Hz, 1H); 7.32-7.63 (m, 6H); 7.90 (dd, J = 8.5, 1.5 Hz, 1H); 8.65 (d, J = 1.5 Hz, 1H). MS (APCI) (M+NH₄)⁺ at m/z 513. Anal calcd for C₂₆H₂₉N₃S₁O₅: C, 63.01; H, 5.90; N, 8.48. Found: C, 62.98; H, 6.06; N, 8.27.

### Example 171

### (2-Isopropylphenyl)[2-nitro-4-(E-((4-propionylpiperazin-1-yl)carbonyl)ethenyl)phenyl]sulfide

Prepared according to the procedures of Example 71, giving a yellow solid. ¹H NMR (DMSO-d₆, 300MHz) δ 1.00 (t, J = 7.3 Hz, 3H); 1.14 (d, J = 7.0 Hz, 6H); 2.35 (q, J = 7.5 Hz, 2H); 3.30-3.40 (m, 1H); 3.41-3.76 (br m, 8H); 6.64 (d, J = 8.5 Hz, 1H); 7.32-7.63 (m, 6H); 7.90 (dd, J = 8.5, 1.5 Hz, 1H); 8.64 (d, J = 1.5 Hz, 1H). MS (APCI) (M+ NH₄)⁻ at m/z 485. Anal calcd for C₂₅H₂₉N₃S₁O₄: C, 64.22; H, 6.25; N, 8.99. Found: C, 64.04; H, 6.44; N, 8.80.

### Example 172

### (2-Isopropylphenyl)[2-nitro-4-(E-((4-carboxamidopiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 3.30-3.40 (m, 1H); 3.30-3.73 (br m, 8H); 6.10 (s, 2H); 6.64 (d, J = 8.5 Hz, 1H); 7.32-7.63 (m, 6H); 7.91 (dd, J = 8.5, 1.8 Hz, 1H); 8.65 (d, J = 1.8 Hz, 1H). MS (APCI) (M+ NH₂)⁺ at m/z 470. Anal calcd for C₂₃H₂₆N₄S₁O₄^{·}0.26CH₃COOCH₂CH₃: C, 60.48; H, 5.93; N, 11.73. Found: C, 60.10; H, 5.84; N, 11.90.

### Example 173

### (2-Isopropylphenyl)[2-nitro-4-(E-((4-methylaminocarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 2.58 (d, J = 4.4 Hz, 3H); 3.30-3.40 (m, 1H); 3.28-3.70 (br m, 8H); 6.52 (q, J = 4.4 Hz, 1H); 6.64 (d, J = 8.5 Hz, 1H); 7.32-7.62 (m, 6H); 7.90 (dd, J = 8.5, 1.8 Hz, 1H); 8.64 (d, J = 1.8 Hz, 1H). MS (APCI) (M+NH₄)⁺ at m/z 486. Anal calcd for C₂₄H₂₈N₄S₁O₄·0.36CH₃COOCH₂CH₃: C, 61.07; H, 6.22; N, 11.19. Found: C, 61.14; H, 6.41; N, 11.19.

### Example 174

### (2-Isopropylphenyl)[2-nitro-4-(E-((4-(pyrimidin-2-yl)piperazin-1-yl)carbonyl)ethenyl)phenyl]sulfide

Prepared according to the procedures of Example 71, giving a yellow solid. ¹H NMR (DMSO-d₆, 300MHz) δ 1.15 (d, J = 6.6 Hz, 6H); 3.30-3.40 (m, 1H); 3.28-3.85 (br m, 8H); 6.64 (d, J = 8.5 Hz, 1H); 6.68 (d, J = 4.8 Hz, 1H); 7.33-7.63 (m, 6H); 7.92 (dd, J = 8.5, 1.8 Hz, 1H); 8.40 (d, J = 4.8 Hz, 2H); 8.67 (d, J = 1.8 Hz, 1H). MS (APCI) (M+H)⁺ at m/z 490. Anal calcd for C₂₆H₂₇N₅S₁O₃: C, 63.78; H, 5.56; N, 14.30. Found: C, 63.83; H, 5.54; N, 14.11.

### Example 175

### (2-Isopropylphenyl)[2-nitro-4-(E-((4-hydroxyacetylpiperazin-1-yl)carbonyl)ethenyl)phenyl]sulfide

Prepared according to the procedures of Example 71, giving a yellow solid. ¹H NMR (DMSO-d₆, 300MHz) δ 1.15 (d, J = 6.8 Hz, 6H); 3.30-3.40 (m, 1H); 3.28-3.78 (br m, 8H); 4.12 (d, J =5.8 Hz, 2H); 4.61-4.69 (br m, 1H); 6.64 (d, J = 8.5 Hz, 1H); 7.33-7.63 (m, 6H); 7.90 (dd, J = 8.5, 1.8 Hz, 1H); 8.65 (d, J = 1.8 Hz, 1H). MS (APCI) (M+H)⁺ at m/z 470. Anal calcd for C₂₄H₂₇N₃S₁O₅·0.38CH₃COOCH₂CH₃: C, 60.93; H, 6.02; N, 8.35. Found: C, 60.95; H, 6.06; N, 8.35.

### Example 176

### (2-Isopropylphenyl)[2-nitro-4-(E-((4-(pyrazine-2-carbonyl]piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 3.30-3.40 (m, 1H); 3.28-3.88 (br m, 8H); 6.61-6.66 (br m, 1H); 7.31-7.63 (m, 6H); 7.85-7.96 (br m, 1H); 8.61-8.92 (m, 4H). MS (APCI) (M+H)⁻ at m/z 518. Anal calcd for C₂₇H₂₇N₅S₁O₄^{·}0.24CH₃COOCH₂CH₃: C, 62.34; H, 5.41; N, 13.01. Found: C, 62.23; H, 5.50; N, 13.10.

### Example 178

### (2-Isopropylphenyl)[2-nitro-4-(E-((3-hydroxymethyl-4-methylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 2.22 (s, 3H); 1.82-4.63 (br m, 9H); 3.30-3.40 (m, 1H); 6.62-6.66 (br m, 1H); 7.25-7.63 (m, 6H); 7.86-7.92 (br m, 1H); 8.57-8.65 (br m, 1H). MS (APCI) (M+H)⁺ at m/z 456.

### Example 179

### (2-Isopropylphenyl)[2-trifluoromethyl-4-(E-(((2-carboxypyrrol-3-in-1-yl)carbonyl)ethenyl)phenyl]sulfide

The title compound was prepared according to the procedures of Example 1. ¹H NMR (CDCl₃, 300 MHz) δ 7.79 (s, 1H), 7.72 (d, 1H, J = 15.5 Hz), 7.49 (d, 1H, J = 7.4 Hz), 7.36-7.46 (m, 3H), 7.23 (m, 1H), 6.82 (d, 1H, J = 8.5 Hz), 6.74 (d, 1H, J = 15.4 Hz), 6.00 (br, 2H), 4.48 (br, 1H), 4.51 (br, 2H), 3.48 (m, 1H), 1.18 (d. 6H, J = 7.0 Hz). MS (ESI) *m*/*z* -460, -492, -921.

### Example 180

### (2-Isopropylphenyl)[2-trifluoromethyl-4-(E-(((2-hydroxymethylpyrrolidin-1-yl)carbonyl)ethenyl)phenyl]sulfide

The title compound was prepared according to the procedures of Example 1. ¹H NMR (CDCl₃, 300 MHz) δ 7.79 (s, 1H), 7.68 (d, 1H, J = 15.4 Hz), 7.48 (d, 1H, J = 7.4 Hz), 7.45 (m, 2H), 7.38 (d, 1H, J = 8.3 Hz), 7.23 (m, 1H), 6.80 (d, 1H, J = 8.5 Hz), 6.70 (d, 1H, J = 15.4 Hz), 5.82 (m, 1H), 5.70 (m, 1H), 4.92 (m, 1H), 4.18 (br s, 2H), 3.76 (s, 3H), 3.78 (d, 1H, J = 11.5 Hz), 3.50 (m, 2H), 3.01 (t, 2H, J = 7.5 Hz), 2.58 (t, 2H, J = 7.6 Hz), 1.19 (d, 6H. J = 7.1 Hz). MS (ESI) *m*/*z* 450, 472, 921.

### Example 181

### (2-Isopropylphenyl)[2-nitro-4-(E-((3-methylaminocarbonyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 2.60 (d, J = 4.4 Hz, 3H); 2.50-4.45 (br m, 7H); 3.30-3.40 (m, 1H); 6.62-6.66 (br m, 1H); 7.32-7.62 (m, 6H); 7.81-7.92 (m, 2H); 8.59-8.65 (br m, 1H). MS (APCI) (M+H)⁺ at m/z 469.

### Example 182

### (2-Isopropylphenyl)[2-nitro-4-(E-(((3-cyclopropylaminocarbonyl)piperazin-1-yl)carbonyl)ethenyl)phenyl]sulfide

Prepared according to the procedures of Example 71, giving a yellow solid. ¹H NMR (DMSO-d₆, 300MHz) δ 0.40-0.62 (br m, 4H); 1.14 (d, J = 6.8 Hz, 6H); 2.50-4.41 (br m, 8H); 3.30-3.40 (m, 1H); 6.62-6.67 (br m, 1H); 7.32-7.62 (m, 6H); 7.87-7.92 (m, 2H); 8.59-8.64 (br m, 1H). MS (APCI) (M+H)⁺ at m/z 495.

### Example 183

### (2-Isopropylphenyl)[2-nitro-4-(E-((3-carboxamidopiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 2.50-4.42 (br m, 7H); 3.30-3.40 (m, 1H); 6.62-6.67 (br m, 1H); 7.12-7.62 (m, 8H); 7.87-7.92 (m, 1H); 8.60-8.65 (br m, 1H). MS (APCI) (M+H)⁻ at m/z 455.

### Example 184

### (2-Isopropylphenyl)[2-nitro-4-(E-((3-carbomethoxy-4-oxopiperidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 2.32-2.55 (br m, 2H); 3.30-3.40 (m, 1H); 3.64,3.76 (s, s, 3H); 3.68-4.58 (br m, 5H); 6.64 (d, J = 8.5 Hz, 1H); 7.32-7.63 (m, 6H); 7.88-7.96 (m, 1H); 8.60-8.68 (m, 1H). MS (APCI) (M+H)⁺ at m/z 483. Anal calcd for C₂₅H₂₆N₂S₁O₆·0.17C₆H₁₄: C, 62.86; H, 5.75; N, 5.63. Found: C, 62.81; H, 5.83; N, 5.60.

### Example 185

### (2-Isopropylphenyl)[2-nitro-4-(E-((3,5-dimethylpiperazin-1-yl)carbonyl)ethenyl)phenyl]sulfide

Prepared according to the procedures of Example 71, giving a yellow solid. ¹H NMR (DMSO-d₆, 300MHz) δ 0.96-1.06 (m, 6H); 1.14 (d, J = 6.8Hz, 6H); 2.07-4.39 ( br m, 7H); 6.63 (d, J = 8.5 Hz, 1H); 7.30-7.63 (m, 6H); 7.92 (dd, J = 8.5, 1.7 Hz, 1H); 8.60 (d, J = 1.7 Hz, 1H). MS (APCI) (M+H)⁺ at m/z 440. Anal calcd for C₂₆H₂₉N₃S₁O₃: C, 65.58; H, 6.65; N, 9.56. Found: C, 65.36; H, 6.87; N, 9.27.

### Example 186

### (1-Ethylindol-7-yl)[2-chloro-4-(E-((4-acetylpiperazin-1-yl)carbonyl)ethenyl)phenyl]sulfide

The title compound was prepared by the procedures described in Example 85, substituting 5-iodoindole with *N*-ethyl-7-bromoindole. white solid; ¹H NMR (CDCl₃, 300 MHz) δ 1.30 (t, *J* = 7.05 Hz, 3H), 2.14 (s, 3H), 3.52 (br s, 2H), 3.58-3.84 (m, 6H), 4.42 (q, *J* = 7.05 Hz, 2H), 6.42 (d, *J* = 8.4 Hz, 1H), 6.59 (d, *J* = 3.0 Hz, 1H), 6.76 (d, *J* = 15.6 Hz, 1H), 7.08 (d, *J* = 8.4 Hz, 1H), 7.10 (d, *J* = 3.0 Hz, 1H), 7.16 (t, *J* = 7.65 Hz, 1H), 7.42 (dd, *J* = 0.9, 7.5 Hz, 1H),7.53 (d, *J* = 1.8 Hz, 1H), 7.54 (d, *J* = 15.6 Hz, 1H), 7.78 (dd, *J* = 0.9, 7.5 Hz, 1H). MS (APCI⁺) (M+H)⁺ at *m*/*z* 468, 470.

### Example 187

### (3-[2-Methoxy]ethoxyphenyl)-[2-chloro-4(E-[(morpholin-1-yl)carbonyl]ethenyl)phenyl]sulfide

The title compound was prepared according to the procedures of Example 85. ¹H-NMR (CDCl₃ 300 MHz) δ 3.45 (s, 3H), 3.65-3.80 (m, 10H), 4.09-4.13 (m, 2H), 6.82 (broad d, J=15, 1H), 6.88 (d, J=9Hz, 1H), 6.87 (dd, J=9Hz, 2Hz, 1H), 7.03-7.10 (m, 2H), 7.20 (d, J=9Hz, 1H), 7.31 (t, J=8 Hz, 1H), 7.52 (s, 1H), 7.56 (broad d, J=15, 1H).

### Example 188

### (2-Bromophenyl)[2-chloro-4-(E-((4,4'-S-dioxythiomorpholin-1-yl)carbonyl)ethenyl)phenyl]sulfide

4-Methylmorpholine *N*-oxide (0.0935 g, 0.798 mmol) and 4Å molecular sieves (0.0333g) were added to a solution of (2-Bromophenyl)[2-chloro-4-(E-((thiomorpholin-1-yl)carbonyl)ethenyl)phenyl]sulfide (0.1230g, 0.27 mmol; prepared according to the procedures described in Example 1). After 15 min, tetrapropylammonium perruthenate (0.0058g, 0.0166 mmol) was added and after 4h had elapsed the starting material was consumed by TLC and the crude products were passed through a plug of silica with 5:2 hexane:ethyl acetate→ 9:1 CH₂Cl₂: MeOH. The mixture was then purified by preparative HPLC to provide the title compound (0.0138 g, 10%). ¹H-NMR (DMSO-d6, 300MHz) δ 8.12 (d,*J* = 1.47 Hz, 1H), 7.81 (dd, *J* = 7.9, 1.3, 2H), 7.65 (dd, *J* = 8.0, 1.5 Hz, 1H), 7.47 (d, *J* = 9.0 Hz, 1H), 7.27-7.53 (m, 4H), 7.03 (d, *J* = 9.0 Hz, 1H), 4.12 (br s, 2H), 3.98 (br s, 2H), 3.26 (br s, 2H), 3.19 (br s, 2H), 1.54-2.29 (m, 6H), MS(APCI) (M+H)⁺ at m/z 486, 488, 490.

### Example 189

### (2-Bromophenyl)[2-chloro-4-(E-(N-carbomethoxymethyl-N-(3-(pyrrolidin-2-on-1-yl)prop-1-yl)amino)carbonyl)ethenyl)phenyl]sulfide

### Example 189A

### N-Carbomethoxymethyl-N-(3-(pyrrolidin-2-on-1-yl)prop-1-yl)amine

Methyl bromoacetate (1.35 mL, 14.3 mmol) was added dropwise to a solution of 3-aminopropyl-2-pyrrolidinone (2.0 mL, 14.3 mmol) and diisopropylethylamine (2.7 mL) in CH₂Cl₂. The reaction was stirred for 12h and was then concentrated *in vacuo,* and carried forward without further purification.

### Example 189B

### (2-Bromophenyl)[2-chloro-4-(E-(N-carbomethoxymethyl-N-(3-(pyrrolidin-2-on-1-yl)prop-1-yl)amino)carbonyl)ethenyl)phenyl]sulfide

The title compound was prepared by the procedures described for Example 113, substituting 2,4 dichlorothiophenol with 2-bromothiophenol, 2-chlorobenzaldehyde with 3,4 dichlorobenzaldehyde, and 1-(3-aminopropyl)-5-((*S*)-hydroxymethyl)-2-pyrrolidinone with the compound from Example 189A. ¹H-NMR (DMSO-d6, 300MHz) δ 8.07 (dd, *J* = 9.4, 1.7 Hz, 1H), 7.81 (m, 1H), 7.64 (m, 1H), 7.24-7.49 (m, 5H), 7.05 (m, 1H), 4.53 (s, 1H), 4.14 (s, 1H), 3.68 (s, 1H), 3.64 (s, 2H), 3.54 (m, 2H), 3.13-3.43 (m, 4H), 2.39 (m, 2H), 1.91 (m, 2H), 1.72 (m, 2H), MS(APCI) (M+H)⁺ at m/z 565, 567, 569.

### Example 190

### (2-Bromophenyl)[2-chloro-4-(E-((4-S-oxythiomorpholin-1-yl)-2-pyrrolidinone)carbonyl)ethenyl)phenyl]sulfide

The title compound (0.0178g, 14%) was isolated from the same reaction mixture as described in Example 188. ¹H-NMR (DMSO-d6, 300MHz) δ 8.12 (d, *J* = 1.8 Hz, 1H), 7.81 (dd, *J* = 7.9, 1.3 Hz, 1H), 7.65 (dd, *J* = 8.3, 1.7 Hz, 1H), 7.46 (d, *J* = 7.4 Hz, 1H), 7.26-7.48 (m, 4H), 7.04 (d, *J* = 7.4 Hz, 1H), 4.29 (br m, 2H), 3.97 (br m, 1H), 3.61 (br m, 1H), 2.80 (br m, 4H), MS(APCI) (M+H)⁺ at m/z 470, 472, 474.

### Example 191

### (2-Methoxy-5-chlorophenyl)[2-nitro-4-(E-((4-acetylpiperazin-1-yl)carbonyl)ethenyl)phenyl]sulfide

The title compound was prepared according to the procedures of Example 1. ¹H NMR (CDCl₃, 300 MHz) δ 8.44 (s, 1H), 7.66 (d, 1H, J = 15.1 Hz), 7.58 (d, 1H, J = 2.6 Hz), 7.48 (dd, 1H, J = 2.6, 8.8 Hz), 7.44 (m, 1H), 6.97 (d, 1H, J = 8.8 Hz), 6.92 (d, 1H, J = 15.5 Hz), 6.82 (d, 1H, J = 8.5 Hz), 3.78 (s, 3H), 3.70 (m, 6H), 3.54 (m, 2H), 2.15 (s, 3H). MS (ESI) *m*/*z* 476, 498, 951, 973. Anal. Calcd for C₂₂H₂₂ClN₃O_{S}S · 0.48 EtOAc: C, 55.44; H, 5.03; N, 8.11. Found: C, 54.36; H, 4.90; N, 8.50.

### Example 192

### (2-Isopropylphenyl)[2-nitro-4-(E-((3-acetoxymethyl)piperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 2.04 (s, 3H); 3.30-3.40 (m, 1H); 2.50-4.46 (br m, 9H); 6.64 (d, J = 8.8 Hz, 1H); 7.30-7.62 (m, 6H); 7.87-7.93 (m, 1H); 8.58-8.63 (br m, 1H). MS (APCI) (M+H)⁺ at m/z 484. Anal calcd for C₂₅H₂₉N₃S₁O₅·0.2H₂O: C, 61.60; H, 6.09; N. 8.62. Found: C, 61.63; H, 6.21; N, 8.41.

### Example 193

### (2-Isopropylphenyl)[2-nitro-4-(E-((3,5-dimethyl-4acetylpiperazin-1-yl)carbonyl)ethenyl)pheyl]sulfide

Prepared according to the procedures of Example 71, giving a yellow solid. ¹H NMR (DMSO-d₆, 300MHz) δ 1.00-1.20 (br m, 6H); 1.15 (d, J = 6.8Hz, 6H); 2.04 (s, 3H); 2.76-4.58 (br m, 7H); 6.64 (d, J = 8.5 Hz, 1H); 7.32-7.63 (m, 6H); 7.94 (dd, J = 8.5, 1.8 Hz, 1H); 8.66 (d, J = 1.8 Hz, 1H). MS (APCI) (M+H)⁺ at m/z 482. Anal calcd for C₂₆H₃₁N₃S₁O₄·0.3H₂O: C, 64.13; H, 6.54; N, 8.63. Found: C, 64.15; H, 6.61; N, 8.50.

### Example 194

### (1-Methylindol-5-yl)[2-chloro-4-(E-((4-acetypiperazin-1-yl)carbonyl)ethenyl)phenyl]sulfide

The title compound was prepared by the procedures described in Example 85, substituting 5-iodoindole with N-methyl-5-bromoindole, giving a white solid. ¹H NMR (d⁶-DMSO, 300 MHz) δ 2.04 (s, 3H), 3.40-3.80 (m, 8H), 3.86 (s, 3H), 6.49 (d, *J* = 8.4 Hz, 1H), 6.52 (d, *J* = 3.0 Hz, 1H), 7.27 (d, *J* = 15.6 Hz, 1H), 7.31 (dd, *J* = 2.4, 8.4 Hz, 1H), 7.39 (d, *J* = 15.6 Hz, 1H), 7.41 (dd, *J* = 1.8, 8.4 Hz, 1H), 7.48 (d, *J* = 3.0 Hz, 1H), 7.63 (d, *J* = 8.4 Hz, 1H), 7.85 (d, *J* = 1.8 Hz, 1H), 7.99 (br s, 1H). MS (APCI⁺) (M+H)⁺ at *m*/*z* 454, 456.

### Example 195

### (Benzodioxan-6-yl)[2-nitro-4-(E-((4-acetylpiperazin-1-yl)carbonyl)ethenyl)phenyl]sulfide

### Example 195A

### 6-Mercaptobenzodioxane

The title compound was prepared by the procedures described in Example 97A, substituting 2-ethoxybenzene with 6-iodobenzenedioxane.

### Example 195B

### (Benzodioxan-6-yl)[2-nitro-4-(E-((4-acetylpiperazin-1-yl)carbonyl)ethenyl)phenyl]sulfide

The title compound was prepared by the procedures described in Example 32, substituting 2,4-dichlorobenzenethiol with 6-mercaptobenzenedioxane, to give a light-yellow solid; ¹H NMR (d⁶-DMSO, 300 MHz) δ 2.04 (s, 3H), 3.41-3.80 (m, 8H), 4.28-4.38 (m, 4H), 6.86 (d, *J* = 8.4 Hz, 1H), 7.05 (d, *J* = 8.4 Hz, 1H), 7.10 (dd, *J* = 2.1, 8.4 Hz, 1H),7.15 (d, *J* = 2.1 Hz, 1H), 7.40 (d, *J* = 15.6 Hz, 1H), 7.53 (d, *J* = 15.6 Hz, 1H), 7.91 (dd, *J* = 1.8, 8.4 Hz, 1H),8.62 (d, *J* = 1.8 Hz, 1H). MS (APCI-) (M+H)⁺ at *m*/*z* 470. Anal. Calcd for C₂₃H₂₃N₃O₆S · 0.17 H₂O: C, 58.46; H, 4.98; N, 8.89. Found: C, 58.47; H, 4.88; N, 8.78.

### Example 196

### (Benzodioxan-6-yl)[2-nitro-4-(E-((3-(pyrrolidin-2-on-1-yl)prop-1-ylamino)carbonyl) ethenyl)phenyl]sulfide

The title compound was prepared by the procedures described in Example 32, substituting 2,4-dichlorobenzenethiol with 6-mercaptobenzenedioxane, and 1-acetylpiperazine with 3-aminopropyl-1-pyrrolidin-2-one, giving a light-yellow solid. ¹H NMR (d⁶-DMSO, 300 MHz) δ 1.64 (p, *J* = 7.2 Hz, 2H), 1.92 (p, *J* = 7.8 Hz, 2H), 2.21 (t, *J* = 7.8 Hz, 2H), 3.13 (t. *J* = 7.2 Hz, 2H), 3.19 (t, *J* = 7.2 Hz, 2H), 3.38-3.46 (overlapping t, *J* = 7.8 Hz, 2H), 4.27-4.37 (m, 4H), 6.70 (d, *J* = 15.6 Hz, 1H), 6.90 (d, *J* = 8.4 Hz, 1H), 7.05 (d, *J* = 8.4 Hz, 1H), 7.09 (dd, *J* = 2.1, 8.4 Hz, 1H). 7.16 (d, *J* = 2.1 Hz, 1H), 7.46 (d, *J* = 15.6 Hz, 1H), 7.77 (dd, *J* = 2.1, 8.4 Hz, 1H), 8.16 (t, *J* = 6.0 Hz, 1H), 8.41 (d, *J* = 2.1 Hz, 1H). MS (APCl⁻) (M+H)⁺ at *m*/*z* 484. Anal. Calcd for C₂₄H₂₅N₃0₆S · 0.51 CH₂Cl₂ · 0.24 MeOH: C, 55.61; H, 5.09; N, 7.86. Found: C, 55.39; H, 5.48; N. 8.26.

### Example 197

### (Benzodioxan-6-yl)[2-nitro-4-(E-((3-carboethoxypiperidin-1-yl)carbonyl)ethenyl)phenyl]sulfide

The title compound was prepared by the procedures described in Example 196 substituting N-(3'-aminopropyl)-2-pyrrolidinone with ethyl nipecotate, giving a yellow solid, mp 73-75 °C. ¹H NMR (CDCl₃, 300 MHz) δ 1.26 (t, J = 7.0 Hz, 3H), 1.74 (br, 1H), 1.78 (br, 1H), 210 (br, 1H), 2.54 (br, 1H), 2.95-3.70 (br, 2H), 3.90-4.10 (br, 2H), 4.15 (q, J = 7.0 Hz, 2H), 4.30-4.40 (m, 4H), 4.65 (br, 1H), 6.90 (d, J = 8.5 Hz, 1H), 6.98 (d, J = 8.5 Hz, 1H), 7.06 (dd, J = 2.0, 8.0 Hz, 1H), 7.10 (d, J = 2.0 Hz, 1H), 7.40-7.50 (m, 1H), 7.58 (d, J = 15.0 Hz, 1H), 8.40 (d, J = 2.0 Hz, 1H). MS (APCI) m/z 499 (M+H)⁺. Anal. calcd. for C₂₅H₂₆N₂O₇S: C, 60.23; H, 5.26; N, 5.62. Found: C, 60.09; H, 5.43; N, 5.47.

### Example 198

### (Benzodioxan-6-yl)[2-nitro-4-(E-((4-carboethoxypiperidin-1-yl)carbonyl)ethenyl)phenyl]sulfide

The title compound was prepared by the procedure described as in example 196 substituting N-(3'-aminopropyl)-2-pyrrolidinone with ethyl isonipecotate, giving a yellow solid, mp 78-88 °C. ¹H NMR (CDCl₃, 300 MHz) δ 1.27 (t, J = 7.0 Hz, 3H), 1.65 (m, 2H), 2.00 (m, 2H), 2.60 (m, 1H), 2.80-3.50 (br, 2H), 4.15 (br, 1H), 4.16 (q, J = 7.0, 2H), 4.34 (m, 4H), 4.54 (br, 1H), 6.90 (d, J = 8.0 Hz, 1H), 6.98 (d, J = 8.0 Hz, 1H), 7.05 (dd, J = 2.0, 8.0 Hz, 1H), 7.10 (d, J = 2.0 Hz, 1H), 7.12 (br, 1H), 7.44 (d, J = 8.0 Hz, 1H), 7.60 (br, 1H), 8.40 (s, 1H). MS (CI/NH₃) m/z 499 (M+H)⁺. Anal. calcd. for C₂₅H₂₆N₂O₇S 0.03 H₂O: C, 60.16; H, 5.26; N. 5.61. Found: C, 60.15; H, 5.65; N, 5.40.

### Example 199

### (2-Ethoxyphenyl)[2-trifluoromethyl-4-(Z-((4-acetylpiperazin-1-yl)carbonyl)ethenyl)phenyl]sulfide

### Example 199A

### (2-Ethoxyphenyl)[2-trifluoromethyl-4-(Z-((4-carbomethoxyethenyl)phenyl]sulfide

Bis-(2,2,2-trifluoroethyl)(methoxycarbonylmethyl)phosphonate (1.20 g, 3.77 mmole), and 18-crown-6 (3.56 g, 13.48 mmol) were dissolved in 22 ml of dry THF. The mixture was cooled to -78 °C and KN(SiMe₃)₂ (0.5 M in THF, 4.04 mmol) was added and stirred for 30 min. (2-Ethoxyphenyl)[2-trifluoromethyl-4-formyl phenyl]sulfide (1.10 g, 3.77 mmol , prepared according to the procedure of example 1) in 13 ml of THF was added via cannulation. After 1 hr at that temperature, the cooling bath was removed and the mixture allowed to warm to ambient temperature. Saturated NH₄Cl soln. was added and the mixture was extracted with ethyl acetate three times. The combined organics were dried over sodium sulfate, concentrated *in vacuo* and purified by medium pressure chromatography on silica gel to give 772 mg (60% yield) of the cis- isomer (J_{olefinic} = 12.5 Hz ) along with 322 mg (25% yield) of the trans- isomer (J_{olefinic} = 12.5 Hz).

### Example 199B

### (2-Ethoxyphenyl)[2-trifluoromethyl-4-(Z-((4-acetylpiperazin-1-yl)carbonyl)ethenyl)phenyl]sulfide

The compound of Example 199A was converted to the corresponding amide according to the procedures of Example 1. ¹H NMR (CDCl₃, 300 MHz) δ 7.64 (d, 1H, J = 16.9 Hz), 7.32-7.4 (m, 2H), 6.98 (m, 2H), 6.93 (m, 2H), 6.65 (d, 1H, J = 12.1 Hz), 6.08 (d, 1H, J = 12.2 Hz), 3.98 (q, 2H, J = 7.0 Hz), 3.68 (m, 2H), 3.62 (m, 2H), 3.44-3.54 (m, 4H), 2.11 and 2.05 (s, 3H), 1.20 (t, 3H, J = 7.0 Hz). MS (ESI)s *m*/*z* 479, 501.

### Example 200

### (2-Ethoxyphenyl)[2-trifluoromethyl-4-(E-((6-methylpyrid-2-ylamino)carbonyl)ethenyl)phenyl]sulfide

The title compound was prepared according to the procedures of Example 1. ¹H NMR (CDCl₃, 300 MHz) δ 8.12 (d, 1H, J = 8.1 Hz), 7.78 (s, 1H, J = 1.7 Hz), 7.70 (d, 1H, J = 15.6 Hz), 7.63 (t, 1H, J = 7.8 Hz), 7.46 (dd, 1H, J = 1.6, 7.8 Hz), 7.36-7.42 (m, 2H), 7.04 (d, 1H, J = 8.1), 6.99 (dd, 1H, J = 1.2, 7.6 Hz), 6.92 (m, 2H), 6.50 (d, 1H, J = 15.6 Hz), 3.99 (q, 2H, J = 6.9 Hz), 2.47 (s, 3H), 1.19 (t, 3H, J = 7.0 Hz). MS (ESI)s *m*/*z* 459, 481. Anal. Calcd for C₂₄H₂₁F₃N₂O₂S · 1.1 H₂O: C, 60.27; H, 4.89; N, 5.86. Found: C, 60.28; H, 5.05; N, 5.94.

### Example 201

### (2-Methyl-3-chlorophenyl)[2-nitro-4-(E-((4-acetylpiperazin-1-yl)carbonyl)ethenyl)phenyl]sulfide

The title compound was prepared according to the procedures of Example 1. ¹H NMR (CDCl₃, 300 MHz) δ 8.46 (d, 1H, J = 1.5 Hz), 7.64 (d, 1H, J = 15.4 Hz), 7.56 (d, 1H, J = 2.6 Hz), 7.54 (d, 1H, J = 2.2 Hz), 7.47 (d, 1H, J = 8.5 Hz), 7.27 (m, 1H), 6.92 (d, 1H, J = 15.4 Hz), 6.68 (d, 1H, J = 8.5 Hz), 3.63-3.78 (m, 6H), 3.53 (m, 2H), 2.45 (s, 3H), 2.15 (s, 3H). MS (ESI) *m*/*z* 460, 482, 919. Anal. Calcd for C₂₂H₂₂Cl₁N₃O₄S: C, 57.45, H, 4.82, N, 9.14. Found: C, 75.54, 5.08, N, 8.82.

### Example 202

### (Benzodioxan-6-yl)[2-nitro-4-(E-((3-carboxamidopiperidin-1-yl)carbonyl)ethenyl)phenyl]sulfide

The title compound was prepared by the procedures described in Example196, substituting N-(3'-aminopropyl)-2-pyrrolidinone with nipecotamide, giving a llight yellow solid, mp 243-245 °C. ¹H NMR (CDCl₃, 500 MHz) δ 1.38-1.50 (m, 2H), 1.77-2.00 (m, 2H), 2.38 (m, 1H), 2.70 (m, 1H), 3.11 (m, 1H), 4.22 (m, 1H), 4.28-4.30 (m, 2H), 4.32-4.36 (m, 2H), 4.42 (m, 1H), 6.85 (d, J = 8.5 Hz. 1H), 7.04-7.16 (m, 2H), 7.35 (s, 1H), 7.40 (d, J = 13.0 Hz, 1H), 7.48 (d, J = 15.5 Hz, 1H), 7.91 (d, J = 8.5 Hz, 1H), 8.58 (s, 1H). MS (APCI) m/z 470 (M+H)⁺. Anal. calcd. for C₂₃H₂₃N₃O₆S•0.37 H₂O: C, 58.01; H, 5.03; N, 8.82. Found: C, 58.02; H, 5.13; N, 8.61.

### Example 203

### (Benzodioxan-6-yl)[2-nitro-4-(E-((2-carboethoxypiperidin-1-yl)carbonyl)ethenyl)phenyl]sulfide

The title compound was prepared by the procedures described in Example 196, substituting N-(3'-aminopropyl)-2-pyrrolidinone with ethyl pipecolinate, producing a light yellow solid, mp 74-75 °C. ¹H NMR (CDCl₃, 300 MHz) d 1.28 (t, J = 7.0 Hz, 3H), 1.32-1.55 (m, 2H), 1.60-1.82 (m, 3H), 2.33 (m, 1H), 3.40 (m, 1H), 3.98 (m, 1H), 4.23 (q, J = 6.5 Hz, 2H), 4.32 (q, J = 5.0 Hz, 4H), 5.45 (m, 1H), 6.90 (d, J = 8.0 Hz, 1H), 6.97 (d, J = 8.0 Hz, 1H), 7.0-7.10 (m, 3H), 7.44, (d, H=7.5 Hz, 1H), 7.60 (d, J = 15.0 Hz, 1H), 8.38 (m, 1H). MS (APCI) m/z 499 (M+H)⁺. Anal. calcd. for C₂₅H₂₆N₂O₇S•0.11 H₂O: C, 59.99; H, 5.28; N, 5.60. Found: C, 59.98; H, 5.42; N, 5.91.

### Example 204

### (Benzodioxan-6-yl)[2-nitro-4-(E-((4-carboxamidopiperidin-1-yl)carbonyl)ethenyl)phenyl]sulfide

The title compound was prepared by the procedures described in Example 196, substituting N-(3'-aminopropyl)-2-pyrrolidinone with isonipecotamide, giving a light yellow solid, mp >230°C. ¹H NMR (CDCl₃, 500 MHz) δ 1.35 (m, 1H), 1.60 (m, 1H), 1.72 (m, 1H), 1.68 (m, 1H), 2.20 (m, 1H), 2.75 (m, 1H), 3.04 (m, 1H), 3.20 (m, 1H), 4.20 (m, 1H), 4.32 (m, 4H), 6.85 (d, J = 8.5 Hz, 1H), 7.04 (d, J = 8.5 Hz, 1H), 7.09 (dd, J = 2.0, 8.5 Hz, 1H), 7.26 (s, 1H), 7.37 (d, J = 16.0 Hz, 1H), 7.47 (d, J = 16.0 Hz, 1H), 8.58 (d, J = 2.0 Hz, 1H). MS (APCI) m/z 470 (M+H)⁺. Anal. calcd. for C₂₃H₂₃N₃O₆S•0.13 H₂O: C, 58.55; H, 4.97; N, 8.91. Found: C, 58.41; H, 5.14; N, 9.30.

### Example 205

### (Benzodioxan-6-yl)[2-nitro-4-(E-((4-tert-butoxycarbonylpiperazin-1-yl)carbonyl)ethenyl)phenyl]sulfide

The title compound was prepared by the procedures described in Example196, substituting N-(3'-aminopropyl)-2-pyrrolidinone with Boc-piperazine, giving a light yellow solid, mp 165-167 °C. ¹H NMR (CDCl₃, 300 MHz) δ 1.48 (s, 9H), 3.50 (m, 4H), 3.65 (br, m, 4H), 4.32 (m, 4H), 6.89 (d, J = 5.0 Hz, 1H), 6.92 (m, 1H), 6.97 (d, J = 8.0 Hz, 1H), 7.05 (dd, J = 2.0, 8.5 Hz, 1H), 7.10 (d, J = 2.0 Hz, 1H), 7.45 (m, 1H), 7.63 (d, J = 15.5 Hz, 1H), 8.40 (m, 1H). MS (APCI) M/z 528 (M+H)⁺. Anal. calcd. for C₂₆H₂₉N₃O₇S: C, 59.19; H, 5.54; N, 7.96. Found: C, 58.85; H, 5.69; N, 8.20.

### Example 206

### (2-Isopropylphenyl)[2-nitro-4-(E-((syn -3,5-dimethylmorpholin-1-yl)carbonyl)ethenyl)phenyl]sulfide

Prepared according to the procedures of Example 71, giving a yellow solid. ¹H NMR (DMSO-d₆, 300MHz) δ 1.10-1.18 (m, 12H); 2.29-2.39 (m, 1H); 2.67-2.78 (m, 1H); 3.30-3.53 (m, 3H); 4.17-4.38 (m, 2H); 6.63 (d, J = 8.8 Hz, 1H); 7.32-7.63 (m, 6H); 7.92 (dd, J = 8.8, 1.5 Hz, 1H); 8.66 (d, J = 1.8 Hz, 1H). MS (APCI) (M+H)⁺ at m/z 441. Anal calcd for C₂₄H₂₈N₂S₁O₄: C, 65.43; H, 6.41; N, 6.36. Found: C, 65.69; H, 6.70; N, 6.17.

### Example 207

### (2-Isopropylphenyl)[2-nitro-4-(E-((anti-3,5-dimethylmorpholin-1-yl)carbonyl)ethenyl)phenyl]sulfide

Prepared according to the procedures of Example 71, giving a yellow solid. ¹H NMR (DMSO-d₆, 300MHz) δ 1.07-1.12 (m, 6H); 1.15 (d, J = 6.6 Hz, 6H); 3.32-3.48 (m, 3H); 3.60-3.83 (br m, 2H); 3.87-3.98 (m, 2H); 6.63 (d, J = 8.5 Hz, 1H); 7.32-7.63 (m, 6H); 7.93 (dd, J = 8.8, 1.8 Hz, 1H); 8.64 (d, J = 1.8 Hz, 1H). MS (APCI) (M+H)⁺ at m/z 441.

### Example 208

### (2-Isopropylphenyl)[2-nitro-4-(E-((3-carboethoxypiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.08-1.26 (m, 3H); 2.52-3.16 (br m, 4H); 3.25-3.40 (m, 1H); 3.41-4.26 (br m, 5H); 6.61-6.67 (br m, 1H); 7.30-7.62 (m, 6H); 7.87-7.93 (br m, 1H); 8.58-8.64 (br m, 1H). MS (APCI) (M+H)⁺ at m/z 484. Anal calcd for C₂₅H₂₉N₃S₁O₅: C, 62.09; H, 6.04; N, 8.69. Found: C, 61.96; H, 6.28; N, 8.49.

### Example 209

### (2-Isopropylphenyl)[2-nitro-4-(E-((3-isopropoxycarbonylpiperazin-1-yl)carbonyl)ethenyl)phenyl]sulfide

Prepared according to the procedures of Example 71, giving a yellow solid. ¹H NMR (DMSO-d₆, 300MHz) δ 1.07-1.21 (br m, 6H); 1.14 (d, J = 7.0 Hz, 6H); 2.52-3.16 (br m, 4H); 3.30-3.40 (m, 1H); 3.41-4.24 (br m, 3H); 4.81-4.97 (m, 1H); 6.61-6.68 (br m, 1H); 7.32-7.63 (m, 6H); 7.87-7.94 (br m, 1H); 8.60-8.66 (br m, 1H). MS (APCI) (M+H)⁺ at m/z 498. Anal calcd for C₂₆H₃₁N₃S₁O₅: C, 62.76; H, 6.28; N, 8.44. Found: C, 62.51; H, 6.52; N, 8.14.

### Example 210

### (2-Isopropylphenyl)[2-nitro-4-(E-((3-(dimethylaminocarbonyl)-4-methylpiperazin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 2.14 (s, 3H); 2.82, 2.84 (s, s, 3H); 3.12 (s, 3H); 2.12-4.24 (br m, 8H); 6.64 (d, J = 8.5 Hz, 1H); 7.32-7.62 (m, 6H); 7.87-7.94 (br m, 1H); 8.60-8.66 (br m, 1H). MS (APCI) (M+H)⁺ at m/z 497. Anal calcd for C₂₆H₃₂N₄S₁O₄·042H₂O: C, 61.94; H, 6.56; N, 11.11. Found: C, 62.00; H, 6.78; N, 10.89.

### Example 211

### (2-Isopropylphenyl)[2-nitro-4-(E-((3-carbomethoxy-4-hydroxypiperidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 1.59-1.75 (br m, 2H); 2.50-3.14 (br m, 1H); 3.30-3.40 (m, 1H); 3.60, 3.61 (s, s, 3H); 4.01-4.44 (br m, 4H); 5.05-5.10 (br m, 1H); 6.63 (d, J = 8.5 Hz, 1H); 7.34-7.62 (m, 6H); 7.87-7.94 (br m, 1H); 8.60-8.66 (br m, 1H). MS (APCI) (M+H)⁺ at m/z 485.

### Example 212

### (2-Isopropylphenyl)[2-nitro-4-(E-((3-hydroxymethyl-4-hydroxypiperidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.49-1.90 (br m, 2H); 2.75-3.14 (br m, 1H); 3.30-3.40 (m, 1H); 3.40-4.23 (br m, 5H); 4.38-4.52 (m, 1H); 4.60-4.73 (m, 1H); 6.61-6.66 (m, 1H); 7.27-7.61 (m, 6H); 7.84-7.93 (br m, 1H); 8.54-8.63 (br m, 1H). MS (APCI) (M+H)⁺ at m/z 457. Anal calcd for C₂₄H₂₈N₂S₁O₅·047H₂O: C, 61.97; H, 6.27; N, 6.02. Found: C, 62.02; H, 6.49; N, 5.90.

### Example 213

### (2-Ethoxyphenyl)[2-trifluoromethyl-4-(E-((2-carbomethoxy-4-(methoxycarbonyl)piperazin-1-yl)carbonyl)ethenyl)phenyl]sulfide

The title compound was prepared according to the procedures of Example 71. ¹H NMR (CDCl₃, 300 MHz) δ 7.80 (s, 1H), 7.66 (d, 1H, J = 15.4 Hz), 7.45 (dd, 1H, J = 1.6, 7.5 Hz), 7.48 (m, 2H), 7.01 (d, 1H, J = 6.6 Hz), 6.95 (d, 1H, J = 6.8 Hz), 6.90 (m, 2H), 5.34 (br s, 1H). 4.66 (m, 2H), 3.76 (s, 3H), 3.73 (s, 3H), 3.18 (m, 1H), 3.00 (m, 3H). MS (ESI) *m*/*z* 553, 575.

### Example 214

### (2-Ethoxyphenyl)[2-trifluoromethyl-4-(E-((2-carbomethoxy-4-methyl piperazin-1-yl)carbonyl)ethenyl)phenyl]sulfide

The title compound was prepared according to the procedures of Example 71. ¹H NMR (CDCl₃, 300 MHz) δ 7.79 (s, 1H), 7.64 (d, 1H, J = 15.3 Hz), 7.45 (dd, 1H, J = 1.7, 7.8 Hz), 7.4-7.35 (m, 2H), 7.01 (d, 1H, J = 8.1 Hz), 6.97 (dd, 1H, J = 1.2, 7.6 Hz), 6.87-7.91 (m, 2H), 5.36 (br s, 1H), 3.98 (q, 2H, J = 6.9 Hz), 3.90 (m, 1H), 3.78 (s, 3H), 3.65 (m, 1H), 3.42 (m, 1H), 2.85 (m, 1H), 2.32 (s, 3H), 2.24 (m, 1H), 2.19 (m, 1H), 1.18 (t, 3H, J = 6.9 Hz). MS (ESI) *m*/*z* 509, 531.

### Example 215

### (2-Ethoxyphenyl)[2-trifluoromethyl-4-(E-((2-carboxy-4-(methoxycarbonyl)piperazin-1-yl)carbonyl)ethenyl)phenyl]sulfide

The title compound was prepared according to the procedures of Example 71. ¹H NMR (DMSO-d₆, 300 MHz) δ 8.10 (m, 1H), 7.68 (m,1H), 7.42 (m, 2H), 7.30 (m,1H), 7.20 (d, 1H, J = 15.6 Hz), 7.10 (d, 1H, J = 8.1 Hz), 7.04 (d, 1H, J = 8.5 Hz), 6.98 (d, 1H, J = 7.5 Hz), 4.65 (br s, 1H), 4.53 (m, 2H), 4.05 (m, 2H), 4.00 (q. 2H, J = 6.9 Hz), 3.57 (s, 3H), 1.09 (t, 3H, J = 6.9 Hz). MS (ESI) *m*/*z* -537, -569.

### Example 216

### (Indol-6-yl)[2-chloro-4-(E-((4-acetylpiperazin-1-yl)carbonyl)ethenyl)phenyl]sulfide

The title compound was prepared by the procedures described in Example 85, substituting 5-iodoindole with 6-bromoindole, isolated as a white solid. ¹H NMR (d⁶-DMSO, 300 MHz) δ 2.03 (s, 3H), 3.40-3.77 (m, 8H), 6.52-6.55 (m, 1H), 6.60 (d, *J* = 8.4 Hz, 1H), 7.13 (dd, *J* = 1.8, 8.4 Hz, 1H), 7.27 (d, *J* = 15.6 Hz, 1H), 7.40 (d, *J* = 15.6 Hz, 1H), 7.43 (dd, *J* = 1.8, 8.4 Hz, 1H), 7.51 (t, *J* = 3.0 Hz, 1H), 7.64 (m, 1H), 7.70 (d, *J* = 8.4 Hz, 1H), 7.99 (d, *J* = 1.8 Hz, 1H). MS (APCI⁺) (M+H)⁺ at *m*/*z* 440, 442.

### Example 217

### (1-Ethyl,3-(dimethylaminomethyl)indol-7-yl)[2-chloro-4-(E-((4-acetylpiperazin-1-yl)carbonyl)ethenyl)phenyl]sulfide

The title compound was prepared by the procedures described in Example 85, substituting 5-iodoindole with 7-bromo-3-*N,N*-dimethylmethyl-*N*-ethyl indole, and isolated as a light-brown solid. ¹H NMR (CDCl₃, 300 MHz) δ 1.30 (t, *J* = 7.05 Hz, 3H), 2.14 (s, 3H), 2.41 (s, 6H), 2.93-3.05 (m, 2H), 3.47-3.55 (m, 2H), 3.55-3.87 (m, 6H), 6.42 (d, *J* = 8.4 Hz, 1H), 6.85 (d, *J* = 15.6 Hz, 1H), 7.09 (dd, *J* = 2.1, 8.4 Hz, 1H), 7.17 (d, *J* = 8.4 Hz, 1H), 7.23 (d, *J* = 8.4 Hz, 1H), 7.43 (dd, *J* = 0.9, 7.8 Hz, 1H), 7.52 (d, *J* = 2.1 Hz, 1H), 7.54 (d, *J* = 15.6 Hz, 1H), 7.81 (dd, *J* = 0.9, 7.8 Hz, 1H). MS (ESI⁺) (M+H)⁻ at *m*/*z* 525, 527.

### Example 218

### (5-Ethoxybenzodioxan-6-yl)[2-chloro-4-(E-((4-acetylpiperazin-1-yl)carbonyl)ethenyl)phenyl]sulfide

The title compound was prepared by the procedures described in Example 85, substituting 5-iodoindole with 6-bromo-5-ethoxybenzodioxane, as s white solid. ¹H NMR (CDCl₃, 300 MHz) δ 1.28 (t, *J* = 7.2 Hz, 3H), 2.14 (s, 3H), 3.54 (br s. 2H), 3.60-3.88 (m, 6H), 4.06 (q, *J* = 7.2 Hz, 2H), 4.33 (s, 4H), 6.70 (d, *J* = 8.4 Hz, 1H), 6.73 (d, *J* = 8.4 Hz, 1H), 6.78 (d, *J* = 15.6 Hz, 1H0, 6.98 (d, *J* = 8.4 Hz, 1H), 7.17 (dd, *J* = 1.8, 8.4 Hz, 1H), 7.50 (d, *J* = 1.8 Hz, 1H), 7.57 (d, *J* = 15.6 Hz, 1H). MS (APCI⁺) (M+H)⁺ at *m*/*z* 503, 505.

### Example 219

### (2-Ethyl-4-bromophenyl)[2-nitro-4-(E-((4-acetylpiperazin-1-yl)carbonyl)ethenyl)phenyl]sulfide

The title compound was prepared according to the procedures of Example 32. ¹H NMR (CDCl₃, 300 MHz) δ 8.43 (d, 1H, J = 2.0 Hz), 7.64 (d, 1H, J = 15.6 Hz), 7.58 (d, 1H, J = 2.0 Hz), 7.40-7.48 (m, 3H), 6.90 (d, 1H, J = 15.2 Hz), 6.90 (d, 1H, J = 8.5 Hz), 3.63-3.77 (m, 6H), 3.54 (m, 2H), 2.72 (q, 2H, J = 7.5 Hz), 2.15 (s, 3H), 1.18 (t, 3H, J = 7.5 Hz). MS (ESI) *m*/*z* 518, 520,542, 627. Anal. Calcd for C₂₃H₂₄Br₁N₃O₄S : C, 53.08; H, 4.60; N, 7.93. Found: C, 53.29, H, 4.67, N, 8.11.

### Example 220

### (Benzodioxan-6-yl)[2-nitro-4-(E-((2-carboxypiperidin-1-yl)carbonyl)ethenyl)phenyl]sulfide

The title compound was prepared by the hydrolysis of the compound of Example 203 under basic conditions (aq. NaOH/EtOH), producing a light yellow solid:, mp 165 °C(dec.). ¹H NMR (DMSO-d₆, 300 MHz) δ 1.15-1.52 (m, 3H), 1.46-1.62 (m, 2H), 2.32 (m, 1H), 2.80 (m, 1H), 3.45(br, 1/2H), 4.00 (br, 1/2H), 4.44 (br, 1/2H), 4.800 (br, 1/2H), 6.83 (d, J = 8.0 Hz, 1H), 7.03 (d, J = 8.0 Hz, 1H), 7.09 (dd, J = 2.0, 14.0 Hz, 1H), 7.15 (d, J = 2.0 Hz, 1H), 7.20 (d,J = 15.5 Hz. 1H), 7.35 (d, J = 15.5 Hz, 1H), 7.73 (m, 1H), 8.52 (m, 1H). MS (ESI) m/z 469 (M-H)⁺, 471 (M+H)⁺. Anal. calcd. for C₂₃H₂₁N₂O₇SNa•NaOH·2.7 H₂O: C, 47.54; H, 4.75; N, 4.82. Found: C, 47.18; H, 4.36; N, 4.89.

### Example 221

### (Benzodioxan-6-yl)[2-nitro-4-(E-((4-carboxymethylpiperazin-1-yl)carbonyl)ethenyl)phenyl]sulfide

The title compound was prepared by deprotection of the compound 33 with TFA in CH₂Cl₂. The resultant free amine was treated with tert-butyl bromoacetate and TEA in acetonitrile at room temperature, and followed by deprotection with TFA in CH₂Cl₂, giving a light solid, mp 120 °C (dec.). ¹H NMR (DMSO-d₆, 300 MHz) δ 3.20-3.45 (m, 4H), 4.20 (s, 2H), 3.50-3.80 (m, 4H), 4.28-4.46 (m, 4H), 6.86 (d, J = 8.5 Hz, 1H), 7.04 (m, J = 8.0 Hz, 1H), 7.09 (dd, J = 2.0 8.0 Hz, 1H), 7.15 (d, J = 2.0 Hz, 1H), 7.40 (d, J = 15.5 Hz, 1H), 7.56 (d, J = 15.0 Hz, 1H), 7.90 (dd, J = 2.0, 8.5 Hz, 1H), 8.63 (m, 1H). MS (ESI) m/z 484 (M-H)⁺, 486 (M+H)⁺. Calcd. Anal for C₂₃H₂₁N₃O₇S•1.19CF₃COOH•1.34 H₂O: 47.63; H, 4.11; N, 6.89. Found: C, 47.93; H, 4.51; N, 6.49.

### Example 222

### (3-Morpholinophenyl)[2-nitro-4-(E-((4-acetylpiperazin-1-yl)carbonyl)ethenyl)phenyl]sulfide

The title compound was prepared according to the procedures of Example 62, employing the compound of Example 103 as starting material. ¹H NMR (CDCl₃, 300 MHz) δ 7.80 (s, 1H), 7.64 (d, 1H, J = 15.4 Hz), 7.43 (m, 1H), 7.32 (t, 1H, J = 8.1 Hz), 7.08 (m, 2H), 6.99 (m, 2H), 6.84 (d, 1H, J = 15.4 Hz), 3.87 (t, 4H, J = 4.8 Hz), 3.63-3.79 (m, 6H), 3.50-3.55 (m, 2H), 3.18 (t, 4H, J = 4.8 Hz), 2.10 (s, 3H). MS (ESI) *m*/*z* 520, 542, 1061.

### Example 223

### (5-Ethoxybenzodioxan-8-yl)[2-chloro-4-(E-((4-acetylpiperazin-1-yl)carbonyl)ethenyl)phenyl]sulfide

The title compound was prepared by the procedures described in Example 85, substituting 5-iodoindole with 8-bromo-5-ethoxybenzodioxane, giving a white solid. ¹H NMR (CDCl₃, 300 MHz) δ 1.52 (t, *J* = 7.2 Hz, 3H), 2.15 (s, 3H), 3.48-3.59 (m, 2H), 3.59-3.85 (m, 6H), 4.16 (q, *J* = 7.2 Hz, 2H), 4.22-4.30 (m, 2H), 4.30-4.40 (m, 2H), 6.59 (d, *J* = 8.7 Hz, 1H), 6.63 (d, *J* = 8.7 Hz, 1H), 6.78 (d, *J* = 15.6 Hz, 1H), 7.08 (d, *J* = 8.7 Hz, 1H), 7.17 (dd, *J* = 2.1, 8.7 Hz, 1H), 7.51 (d, *J* = 2.1 Hz, 1H), 7.58 (d, *J* = 15.6 Hz, 1H). MS (APCI⁺) (M+H)⁺ at *m*/*z* 503, 505.

### Example 224

### (5-Chloro-8-ethoxyquinolin-7-yl)[2-chloro-4-(E-((4-acetylpiperazin-1-yl)carbonyl)ethenyl)phenyl]sulfide

The title compound was prepared by the procedures described in Example 85, substituting 5-iodoindole with 5-chloro-8-ethoxy-7-iodoquinoline, giving a white solid. ¹H NMR (d⁶-DMSO, 300 MHz) δ 1.37 (t, *J* = 7.2 Hz, 3H), 2.04 (s, 3H), 3.41-3.82 (m, 8H), 4.46 (q, *J =* 7.2 Hz, 2H), 7.29 (s, 1H), 7.37 (d, *J =* 8.4 Hz, 1H), 7.42 (d, *J =* 15.6 Hz, 1H), 7.51 (d, *J* = 15.6 Hz, 1H), 7.68 (dd, *J* = 1.8, 8.4 Hz, 1H), 7.74 (dd, *J* = 3.9, 8.4 Hz, 1H), 8.15 (s, 1H), 8.55 (dd, *J* = 1.8, 8.4 Hz, 1H), 9.05 (dd, *J* = 1.8, 3.9 Hz, 1H). MS (APCI⁺) (M+H)⁺ at *m*/*z* 530, 532, 534.

### Example 225

### (2-Isopropylphenyl)[2-nitro-4-(E-((3-carboethoxypiperidin-1-yl)carbonyl)ethenyl)phenyl]sulfide

### Example 225A

### (2-Isopropylphenyl)[2-nitro-4-(E-(carboxy)ethenyl)phenyl]sulfide

To a stirred mixture of 4-chloro-3-nitrocinnamic acid (500 mg, 2.2 mmol) in 5 mL of anhydrous DMF with K₂CO₃ (911mg, 6.6 mmol) was added 2-isopropylbenzenethiol (372 mL, 2.2 mmol) in 1 mL of DMF dropwise. The resulting mixture was then heated at 70 °C under nitrogen atmosphere over night. Water (25 mL) was then added and the reaction mixture was acidified to pH = 4 with 3N HCl. The cloudy mixture was extracted with EtOAc (2×20 mL). The combined organic layer was washed with brine, dried over Na₂SO₄, concentrated in vacuo to give the title compound as viscous light-yellow oil, which was used for coupling with further purification.

### Example 225B

### (2-Isopropylphenyl)[2-nitro-4-(E-((3-carboethoxypiperidin-1-yl)carbonyl)ethenyl)phenyl]sulfide

The title compound was prepared by the procedures described in Example 92, substituting the benzoic acid with cinnamic acid from 225A, and ammonium chloride with ethyl nipecotate, giving a light-yellow solid. ¹H NMR (CDCl₃, 300 MHz) δ 1.18 (d, *J* = 6.6 Hz, 6H), 1.27 (t, *J* = 7.2 Hz, 3H), 1.69-1.82 (m, 1H), 1.82-1.99 (m, 1H), 1.99-2.20 (m, 1H), 2.45-2.62 (m, 2H), 3.45 (septet, *J =* 6.6 Hz, 1H), 3.56-3.80 (m, 1H), 3.80-4.10 (m, 2H), 4.16 (q, *J* = 7.2 Hz, 2H), 4.65-4.81 (m, 1H), 6.69 (d, *J* = 8.4 Hz, 1H), 7.00 (br s, 1H), 7.31 (dd, *J* = 2.4, 6.9 Hz, 1H), 7.42 (br d, *J* = 8.4 Hz, 1H), 7.51 (d, *J* = 15.6 Hz, 1H), 7.52 (overlapping d, 2H), 7.58 (d, *J* = 15.6 Hz, 1H), 8.43 (s, 1H). MS (APCI⁺) (M+H)⁺ at *m*/*z* 483.

### Example 226

### (2-Isopropylphenyl)[2-nitro-4-(E-((3-carboxypiperidin-1-yl)carbonyl)ethenyl)phenyl]sulfide

The title compound was prepared by the procedures described in Example 155, substituting the ethyl ester from Example 137 with the ethyl ester from Example 225B, and KOH with NaOH, to give a light-yellow solid. ¹H NMR (d⁶-DMSO, 300 MHz) δ 1.15 (d, *J* = 6.9 Hz, 6H), 1.30-1.50 (m, 1H), 1.50-1.80 (m, 2H), 1.88-2.04 (m, 2H), 2.95-3.17 (m, 1H), 3.94-4.06 (m, 1H), 4.06-4.22 (m, 2H), 4.40-4.52 (m, 1H), 6.63 (d, *J* = 8.7 Hz, 1H), 7.33-7.53 (m, 3H), 7.56-7.68 (m, 3H), 7.91 (dd, *J* = 1.8, 8.4 Hz, 1H), 8.63 (d, *J* = 8.4 Hz, 1H). MS (APCI⁺) (M+H)⁺ at *m*/*z* 455.

### Example 227

### (2-Isopropylphenyl)[2-nitro-4-(E-(((3-ethanesulfonylaminocarbonyl)piperidin-1-yl)carbonyl)ethenyl)phenyl]sulfide

To a stirred solution of free acid (50 mg, 0.11 mmol) from Example 226 in 1 mL of methylene chloride was added ethyl sulfonamide (18 mg, 0.17 mmol), EDAC (25 mg, 0.13 mmol), and DAMP (2.7 mg, 0.022 mmol) sequentially. The mixture was stirred at ambient temperature for 16 h. The solvent was then removed on a rotavap under reduced pressure and the residue was purified on an Alltech sep-pak, eluting with 1 % MeOH in EtOAc to give 30 mg (50 % yield) the title compound as a light yellow solid. ¹H NMR (CDCl₃, 300 MHz) 8 1.18 (d, *J* = 6.3 Hz, 6H), 1.34 (t, *J* = 7.5 Hz, 3H), 1.61-1.74 (m, 2H), 1.84-2.04 (m, 1H), 2.13-2.35 (m, 1H), 2.60-2.75 (m, 2H), 3.44 (p, *J* = 7.5 Hz, 2H), 3.53-3.66 (m, 1H), 3.66-3.85 (m, 2H), 4.00-4.18 (m, 1H), 6.71 (d, *J* = 8.7 Hz, 1H), 6.88 (d, *J* = 15.6 Hz, 1H), 7.31 (dd, *J* = 2.4, 8.4 Hz, 1H), 7.41 (d, *J* = 1.8, 8.4 Hz, 1H), 7.51 (d, *J* = 1.8 Hz, 1H), 7.54 (d, *J* = 8.4 Hz, 1H), 7.67 (d, *J* = 15.6 Hz, 1H), 8.43 (s, 1H). MS (ESI⁺) (M+H)⁺ at *m*/*z* 546.

### Example 228

### (2-Isopropylphenyl)[2-nitro-4-(E-(((3-(4-methylpiperazine)sulfonylaminocarbonyl)piperidin-1-yl)carbonyl)ethenyl)phenyl]sulfide

The title compound was prepared by the procedures described in Example 228, substituting ethyl sulfonamide with *N*-methylpiperazine sulfonamide, giving a light yellow solid. ¹H NMR (CDCl₃, 300 MHz) δ 1.18 (d, *J* = 6.5 Hz, 6H), 1.40-2.10 (m, 9H), 2.60 (s, 3H), 2.60-2.76 (m, 4H), 2.90 (br s, 3H), 3.44 (septet, *J* = 6.5 Hz, 1H), 3.52-4.08 (m, 4H), 6.71 (d, *J* = 8.4 Hz, 1H), 6.95 (d, *J* = 15.6 Hz, 1H), 7.31 (d, *J* = 2.1, 8.4 Hz, 1H), 7.43-7.57 (m, 4H), 7.64 (d, *J* = 15.6 Hz, 1H), 8.44 (s, 1H). MS (ESI⁺) (M+H)⁺ at *m*/*z* 616. Anal. Calcd for C₂₉H₃₇N₅O₆S₂ · 1.13 H₂O: C, 54.76; H, 6.22; N, 11.01. Found: C, 54.78; H, 6.11; N, 10.87.

### Example 229

### (2-Isopropylphenyl)[2-nitro-4-(E-(((3-p-toluenesulfonylaminocarbonyl)piperidin-1-yl)carbonyl)ethenyl)phenyl]sulfide

The title compound was prepared by the procedures described in Example 228, substituting ethyl sulfonamide with *p*-toluenesulfonamide, giving a light yellow solid. ¹H NMR (CDCl₃, 300 MHz) δ 1.19 (d, *J* = 6.5 Hz, 6H), 1.75-1.94 (m, 2H), 2.05-2.24 (m, 1H), 2.40 (s, 3H), 2.48-2.60 (m, 2H), 3.45 (septet, *J* = 6.5 Hz, 1H), 3.50-3.85 (m, 3H), 3.85-4.12 (m, 1H), 6.72 (d, *J* = 8.4 Hz, 1H), 6.86 (d, *J* = 15.6 Hz, 1H), 7.27-7.34 (m, 2H), 7.43 (dd, *J* = 2.1, 8.4 Hz, 1H), 7.50 (overlapping d, 1H), 7.53 (d, *J* = 8.4 Hz, 2H), 7.55 (d, *J* = 8.4 Hz, 1H), 7.92 (d, *J* = 8.4 Hz, 2H), 8.44 (s, 1H). MS (ESI⁺) (M+H)⁺ at *m*/*z* 608.

### Example 230

### (2-Isopropylphenyl)[2-nitro-4-(E-((3-methyl-4-acetylpiperazin-1-yl)carbonyl)ethenyl)phenyl]sulfide

Prepared according to the procedures of Example 71, giving a yellow solid. ¹H NMR (DMSO-d₆, 300MHz) δ 0.94-1.18 (m, 3H); 1.14 (d, J = 7.0 Hz, 6H); 1.98-2.08 (br m, 3H); 2.69-3.74 (br m, 4H); 4.02-4.65 (br m, 4H); 6.64 (d, J = 8.5 Hz, 1H); 7.31-7.63 (m, 6H); 7.88-7.96 (br m, 1H); 8.65 (br s, 1H). MS (APCI) (M+H)⁺ at m/z 468. Anal calcd for C₂₅H₂₉N₃S₁O₄^{·}0.1H₂O: C, 63.91; H, 6.70; N, 8.94. Found: C, 63.54; H, 6.41; N, 8.67.

### Example 231

### (2-Hydroxyphenyl)-[2-chloro-4(E-[(morpholin-1-yl)carbonyl]ethenyl)phenyl]sulfide

The title compound was prepared according to the procedures of Example 1, giving a white solid, m.p. 157-158C. ¹H-NMR (CDCl₃ 300 MHz) δ 3.60-3.76 (m, 8H), 6.42 (s, 1H), 6.57 (d, J = 9hz, 1H), 6.76 (d, J = 15Hz, 1H), 6.99-7.04 (m, 1H), 7.10-7.20 (m, 2H), 7.42-7.55 (m, 4H). Anal. Calcd. for C₁₉H₁₈ClNO₃S: C, 60.71; h, 4.83; N, 3.73. Found: C, 60.48; H, 5.05; N, 3.69.

### Example 232

### (1-(Carboxymethyl)indol-5-yl)[2-chloro-4-(E-((4-acetylpiperazin-1-yl)carbonyl)ethenyl)phenyl]sulfide

To a stirred solution of indole compound from Example 85 (35 mg, 0.080 mmol) in 1 mL of anhydrous DMSO was added crushed KOH (18 mg, 0.32 mmol). After 45 min, *t*-butyl bromoacetate (23.5 mL, 0.16 mmol) was added. The resulting mixture was stirred at ambient temperature for 10 h. Water was then added and the reaction mixture was acidified with 3 N HCl to pH = 3. The title compound (25 mg, 63 %) was collected through filtration and dried in vacuum oven, giving a white solid. ¹H NMR (d⁶-DMSO, 300 MHz) δ 2.04 (s, 3H), 3.38-3.80 (m, 8H), 4.59 (s, 2H), 6.45 (d, *J* = 3.0 Hz, 1H), 6.52 (d, *J* = 8.7 Hz, 1H), 7.21 (dd, *J* = 2.1, 8.7 Hz, 1H), 7.25 (d, *J* = 15.6 Hz, 1H), 7.38 (d, *J* = 15.6 Hz, 1H), 7.40 (d, *J* = 3.0 Hz, 1H), 7.47 (d, *J* = 8.4 Hz, 1H), 7.80 (d, *J* = 2.1 Hz, 1H), 7.97 (s, 1H). MS (ESI⁺) (M-H)⁺ at *m*/*z* 496, 498.

### Example 233

### (Benzodioxan-6-yl)[2-trifluoromethyl-4-(E-((4-acetylpiperazin-1-yl)carbonyl)ethenyl)phenyl]sulfide

The title compound was prepared by the procedures described in Example 84, substituting 2-bromothiophenol with 6-mercaptobenzenedioxane. white solid; ¹H NMR (CDCl₃, 300 MHz) δ 2.15 (s, 3H), 3.46-3.89 (m, 8H), 4.30 (dd, *J* = 2.1, 6.0 Hz, 4H), 6.84 (d, *J* = 15.0 Hz, 1H), 6.92 (d, *J* = 8.4 Hz, 1H), 6.97-7.10 (m, 3H), 7.42 (d, *J* = 8.4 Hz, 1H), 7.64 (d, *J* = 15.0 Hz, 1H), 7.77 (s, 1H). MS (ESI⁺)*m*/*z* 493 (M+H)⁺.

### Example 234

### (2-Isopropylphenyl)[2-nitro-4-(E-((3-(1-pyrrolidin-2-onyl)prop-1-ylamino)carbonyl)ethenyl)phenyl]sulfide

Prepared according to the procedures of Example 71, giving a yellow solid. ¹H NMR (DMSO-d₆, 300MHz) δ 1.14 (d, J = 7.1 Hz, 6H); 1.58-1.68 (m, 2H); 1.85-1.97 (m, 2H); 2.18-2.24 (m,2H); 3.10-3.22 (m, 4H); 3.30-3.39 (m, 3H); 6.65-6.72 (m, 2H); 7.32-7.45 (m, 2H); 7.57-7.62 (m, 3H); 7.76 (dd, J = 8.8, 2.0 Hz, 1H); 8.11-8.17 (m, 1H); 8.44 (d, J = 2.0 Hz, 1H). MS (APCI) (M+H)⁺ at m/z 468. Anal calcd for C₂₅H₂₉N₃S₁O₄·0.26CH₃COOCH₂CH₃: C, 63.77; H, 6.39; N, 8.57. Found: C, 63.46; H, 6.37; N, 8.90.

### Example 235

### (3-(2-Morpholinoethylamino)phenyl)[2-trifluoromethyl-4-(E-((4-acetylpiperazin-1-yl)carbonyl)ethenyl)phenyl]sulfide

The title compound was prepared according to the procedures of Example 62, employing the compound of Example 103 as starting material. ¹H NMR (CDCl₃, 300 MHz) δ 7.78 (d, 1H, J = 1.4 Hz), 7.64 (d, 1H, J = 15.4 Hz), 7.42 (d, 1H, J = 8.8 Hz), 7.21 (t, 1H, J = 7.9 Hz), 7.12 (d, 1H, J = 8.5 Hz), 6.84 (d, 1H, J = 15.4 Hz), 6.82 (m, 1H), 6.76 (t, 1H, J = 1.8 Hz), 6.66 (m, 1H), 3.72 (m, 10H), 3.51-3.55 (m, 2H), 3.16 (t, 2H, J = 5.9 Hz), 2.64 (t, 2H, J = 5.9 Hz), 2.50 (m, 4H), 2.15 (s, 3H). MS (ESI) *m*/*z* 563.

### Example 236

### (2-Pyrrolidin-1-ylphenyl)[2-nitro-4-(E-((4-acetylpiperazin-1-yl)carbonyl)ethenyl)phenyl]sulfide

The title compound was prepared according to the procedures of Example 62, employing the compound of Example 103 as starting material. ¹H NMR (CDCl₃, 300 MHz) δ 7.77 (s, 1H), 7.64 (d, 1H, J = 15.4 Hz), 7.40 (m, 1H), 7.22 (d, 1H, J = 7.8 Hz), 7.10 (d, 1H, J = 8.8 Hz), 6.82 (d, 1H, J = 15.3 Hz), 6.76 (d, 1H, J = 7.8 Hz), 6.70 (t, 1H, J = 2.0 Hz), 6.59 (dd, 1H, J = 2.4, 8.1 Hz), 3.61-3.79.(m, 6H), 3.51-3.54 (m, 2H), 3.28 (m, 4H), 2.14 (s, 3H), 2.01 (m, 4H). MS (ESI) *m*/*z* 504.

### Example 239

### (2-(Hydroxymethyl)-benzodioxan-6-yl)[2-chloro-4-(E-((4-acetylpiperazin-1-yl)carbonyl)ethenyl)phenyl]sulfide

The title compound was prepared by the procedures described in Example 85, substituting 5-iodoindole with a mixture of 2-hydroxymethyl-6-bromobenzodioxane and 2-hydroxymethyl-7-bromobenzodioxane, giving a white solid. ¹H NMR (CDCl₃, 300 MHz, mixture of 3:2 regioisomers) δ 2.15 (s, 3H), 3.46-3.83 (m, 8H), 3.83-4.01 (m, 2H), 4.10-4.42 (m, 4H), 6.75 (d, *J* = 8.4 Hz, 1H), 6.79 (d, *J* = 15.9 Hz, 1H), [6.95 (d), 6.98 (d), *J* = 4.8 Hz, 1H in total], [7.04 (t), 7.07 (t), *J* = 1.5 Hz, 1H in total], [7.10 (d), 7.11 (d), *J* = 2.4 Hz, 1H in total], 7.19 (d, *J* = 8.4 Hz, 1H), 7.53 (s, 1H), 7.58 (d, *J* = 15.6 Hz, 1H). MS (APCI⁺) (M+H)⁺ at *m*/*z* 489.

### Example 240

### (Benzodioxan-6-yl)[2-trifluoromethyl-4-(E-((3-(pyrrolidin-2-on-1-yl)prop-1-ylamino)carbonyl)ethenyl)phenyl]sulfide

The title compound was prepared by the procedures described in Example 233, substituting 1-acetylpiperazine with 3-aminopropyl-1-pyrrolidin-2-one, giving a white solid. ¹H NMR (CDCl₃, 300 MHz) δ 1.69-1.80 (m, 2H), 2.08 (p, *J* = 7.5 Hz, 2H), 2.44 (t, *J* = 7.5 Hz, 2H), 3.27-3.48 (m, 6H), 4.24-4.34 (m, 4H), 6.44 (d, *J* = 15.6 Hz, 1H), 6.90 (d, *J* = 8.4 Hz, 1H), 7.00 (d, *J* = 8.4 Hz, 1H), 7.01 (dd, *J* = 2.7, 8.4 Hz, 1H), 7.06 (d, *J* = 2.7 Hz, 1H), 7.08 (s, 1H), 7.40 (dd, *J* = 2.1, 8.4 Hz, 1H), 7.53 (d, *J* = 15.6 Hz, 1H), 7.75 (d, *J*= 2.1 Hz, 1H). MS (ESI⁺) (M+H)⁺ at *m*/*z* 507.

### Example 241

### (3-(Dimethylaminomethyl)indol-5-yl)[2-chloro-4-(E-((4-acetylpiperazin-1-yl)carbonyl)ethenyl)phenyl]sulfide

The title compound was prepared by the procedures described in Example 217, substituting the indole from 186 with the indole from Example 85, resulting in a white solid. ¹H NMR (CDCl₃, 300 MHz) δ 2.15 (s, 3H), 2.54 (s, 6H), 3.47-3.85 (m, 8H), 4.05 (s, 2H), 6.56 (d, *J* = 8.7 Hz, 1H), 6.77 (d, *J* = 15.6 Hz, 1H), 7.09 (d, *J* = 8.7 Hz, 1H), 7.36 (dd, *J* = 1.5, 8.7 Hz, 1H), 7.50 (d, *J* = 8.7 Hz, 1H), 7.52 (s, 2H), 7.56 (d, *J* = 15.6 Hz, 1H),7.88 (s, 1H), 9.27 (s, 1H). MS (ESI⁺) (M+H)⁺ at *m*/*z* 497, 499. Anal. Calcd for C₂₆H₂₉ClN₄O₂S · 0.46 TFA · 1.72 MeOH: C, 56.89; H, 6.06; N, 9.27. Found: C, 56.83; H, 6.15; N, 9.46.

### Example 242

### (2-Isopropylphenyl)[2-nitro-4-(E-((2-carboethoxypiperidin-1-yl)carbonyl)ethenyl)phenyl]sulfide

The title compound was prepared by the procedures described in Example 225, substituting ethyl nipecotate with ethyl pipecolinate, giving a light-yellow solid. ¹H NMR (CDCl₃, 300 MHz) δ 1.18 (d, *J =* 6.9 Hz, 6H), 1.28 (t, *J* = 7.35 Hz, 3H), 1.34-1.62 (m, 2H), 1.62-1.84 (m, 3H), 2.32 (br d, *J* = 13.2 Hz, 1H), 3.33-3.54 (m, 1H), 3.45 (septet, *J* = 6.9 Hz, 1H), 3.99 (br d, *J* = 13.2 Hz, 1H), 4.21 (q, *J* = 7.35 Hz, 2H), 5.46 (br s, 1H), 6.69 (d, *J* = 8.7 Hz, 1H), 7.01 (d, *J* = 15.6 Hz, 1H), 7.25-7.34 (m, 1H), 7.42 (d, J = 8.7 Hz, 1H), 7.46-7.60 (m, 3H), 7.58 (d, J = 15.6 Hz, 1H), 8.44 (s, 1H). MS (ESI⁺) (M+H)⁺ at *m*/*z* 483.

### Example 243

### (2-Isopropylphenyl)[2-nitro-4-(E-((2-carboxypiperidin-1-yl)carbonyl)ethenyl)phenyl]sulfide

The title compound was prepared by the procedures described in Example 226, substituting the ethyl ester from Example 225 with the ethyl ester from Example 242, giving a light-yellow solid. ¹H NMR (CDCl₃, 300 MHz) δ 1.18 (d, *J* = 6.9 Hz, 6H), 1.40-1.89 (m, 5H), 2.34 (br d, *J* = 11.7 Hz, 1H), 3.31-3.51 (m, 1H), 3.44 (septet, *J* = 6.9 Hz, 1H), 4.01 (d, J = 11.7 Hz, 1H), 5.42 (br s, 1H), 6.70 (d, J = 7.8 Hz, 1H), 6.99 (br d, *J* = 15.6 Hz, 1H), 7.29 (td, *J* = 2.7, 6.9 Hz, 1H), 7.41 (d, *J* = 7.8 Hz, 1H), 7.45-7.58 (m, 3H), 7.64 (d, *J* = 15.6 Hz, 1H), 8.43 (s, 1H). MS (ESI⁺) (M+H)⁺ at *m*/*z* 455. Anal. Calcd for C₂₄H₂₆N₂O₅S · 0.08 H₂O: C, 63.22; H, 5.78; N, 6.14. Found: C, 63.21; H, 5.65; N, 6.00.

### Example 244

### (2-Isopropylphenyl)[2-nitro-4-(E-((4-carboethoxypiperidin-1-yl)carbonyl)ethenyl)phenyl]sulfide

The title compound was prepared by the procedures described in Example 225, substituting ethyl nipecotate with ethyl isonipecotate, to give a light-yellow solid. ¹H NMR (CDCl₃, 300 MHz) δ 1.18 (d, *J =* 6.9 Hz, 6H), 1.27 (t, *J =* 7.5 Hz, 3H), 1.64-1.86 (m, 2H), 1.94-2.09 (m, 2H), 2.90-3.15 (m, 1H), 3.15-3.39 (m, 1H), 3.44 (septet, *J* = 6.9 Hz, 1H), 3.95-4.14 (m, 1H), 4.16 (q, *J* = 7.5 Hz, 2H), 4.40-4.63 (m, 1H), 6.69 (d, *J* = 8.7 Hz, 1H), 6.98 (d, *J* = 15.6 Hz, 1H), 7.29 (td, *J* = 2.7, 6.9 Hz, 1H), 7.41 (d, *J* = 8.4 Hz, 1H), 7.46-7.60 (m, 3H), 7.58 (d, *J* = 15.6 Hz, 1H), 8.43 (s, 1H). MS (ESI⁺) (M+H)⁺ at *m*/*z* 483.

### Example 245

### (2-Isopropylphenyl)[2-nitro-4-(E-((4-carboxypiperidin-1-yl)carbonyl)ethenyl)phenyl]sulfide

The title compound was prepared by the procedures described in Example 226, substituting the ethyl ester from Example 225 with the ethyl ester from Example 244, producing a light-yellow solid. ¹H NMR (CDCl₃, 300 MHz) δ 1.18 (d, *J* = 6.9 Hz, 6H), 1.65-1.89 (m, 2H), 1.97-2.14 (m, 2H), 2.59-2.74 (m, 1H), 2.93-3.20 (m, 1H), 3.20-3.42 (m, 1H), 3.44 (septet, *J* = 6.9 Hz, 1H), 3.97-4.18 (m, 1H), 4.40-4.65 (m, 1H), 6.70 (d, *J* = 8.7 Hz, 1H), 6.97 (d, *J* = 15.6 Hz, 1H), 7.30 (td, *J* = 2.7, 6.9 Hz, 1H), 7.41 (d, *J* = 8.7 Hz, 1H), 7.46-7.65 (m, 3H), 7.60 (d, *J* = 15.6 Hz, 1H), 8.43 (s, 1H). MS (ESI⁺) (M+H)⁺ at *m*/*z* 455.

### Example 246

### (2-Isopropylphenyl)[2-nitro-4-(E-(((4-p-toluenesulfonylaminocarbonyl)piperidin-1-yl)carbonyl)ethenyl)phenyl]sulfide

The title compound was prepared by the procedures described in Example 229, substituting the acid from Example 226 with the acid from Example 245. light-yellow solid; ¹H NMR (d⁶-DMSO, 300 MHz) δ 1.14 (d, *J* = 6.9 Hz, 6H), 1.18-1.39 (m, 2H), 1.67-1.79 (m, 2H), 2.39 (s, 3H), 2.60-2.75 (m, 1H), 2.96-3.14 (m, 1H), 3.26-3.42 (m, 1H), 3.34 (septet, *J* = 6.9 Hz, 1H), 4.10-4.42 (m, 2H), 6.62 (d, *J* = 8.4 Hz, 1H), 7.32-7.43 (m, 4H), 7.45 (d, *J* = 15.6 Hz, 1H), 7.58 (d, *J* = 8.4 Hz, 2H), 7.60 (d, *J* = 3.6 Hz, 1H), 7.78 *(d, J =* 8.4 Hz, 2H), 7.87 *(dd, J =* 2.7, 8.4 Hz, 1H), 8.60 (d, *J* = 2.7 Hz, 1H). MS (ESI⁺) (M+H)⁺ at *m*/*z* 606. Anal. Calcd for C₃₁H₃₃N₃O₆S₂ · 0.26 H₂O: C, 60.80; H, 5.52; N, 6.86. Found: C, 60.85; H, 5.84; N, 6.61.

### Example 247

### (2-Isopropylphenyl)[2-nitro-4-(E-((3-carboxy-4-hydroxypiperidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.53-1.70 (br m, 2H); 2.92-3.52 (br m, 1H); 3.30-3.40 (m, 1H); 3.98-4.44 (br m, 4H); 4.90-5.20 (br m, 1H); 6.63 (d, J = 8.5 Hz, 1H); 7.34-7.62 (m, 6H); 7.87-7.94 (br m, 1H); 8.58-8.64 (br m, 1H). MS (APCI) (M+H)⁺ at m/z 471. Anal calcd for C₂₄H₂₆N₂S₁O₆: C, 61.26; H, 5.57; N, 5.95. Found: C, 61.05; H, 5.85; N, 5.73.

### Example 248

### (Benzodioxan-6-yl)[2-trifluoromethyl-4-(E-((3-carboethoxypiperidin-1-yl)carbonyl)ethenyl)phenyl]sulfide

The title compound was prepared by the procedures described in Example 240 substituting N-(3'-aminopropyl)-2-pyrrolidinone with ethyl nipecotate, giving a white hygroscopic solid. ¹H NMR (CDCI₃, 300 MHz) δ 1.26 (t, J = 7.0 Hz, 3H), 1.54 (m, 1H), 1.65-1.80 (m, 2H), 2.10 (m, 1H), 2.54 (m, 1H), 2.92-3.40 (m, 2H), 3.60-4.10 (m, 2H), 4.14 (q, J = 7.0 Hz, 2H), 4.25-4.32 (m, 4H), 6.91 (d, J = 7.5 Hz, 1H), 7.00 (dd, J = 2.0, 15.0 Hz, 3H), 7.05 (d, J = 2.0 Hz, 1H), 7.40 (d, J = 8.0, 1H), 7.56 (d, J = 15.0 Hz, 1H), 7.76 (s, 1H). MS (CI/NH₃) m/z 522 (M+H)⁺. Anal. calcd. for C₂₆ H₂₆F₃NO₅S: C, 59.88; H, 5.02; N, 2.69. Found: C; 59.92; H, 5.39; N, 2.56.

### Example 249

### (Benzodioxan-6-yl)[2-trifluoromethyl-4-(E-((2-carboethoxypiperidin-1-yl)carbonyl)ethenyl)phenyl]sulfide

The title compound was prepared by the procedures described in Example 240 substituting N-(3'-aminopropyl)-2-pyrrolidinone with ethyl pipecolinate. ¹H NMR (CDCl₃, 300 MHz) δ 1.28 (t, J = 7.0 Hz, 3H), 1.35-1.54 (m, 2H), 1.64-1.82 (m, 3H), 2.30 (m, 1H), 3.40 (m, 1H), 4.00 (m, 1H), 4.22 (q, J = 7.0 Hz, 2H), 4.26-4.34 (m, 4H), 5.48 (m, 1H), 6.91 (d, J = 8.5 Hz, 1H), 6.98 (m, 1H), 7.02 (dd, J = 2.0, 8.0 Hz, 2H), 7.06 (d, J = 2.0 Hz, 1H), 7.41 (d, J = 8.0 Hz, 1H), 7.57 (d, J = 15.0 Hz, 1H), 7.77 (s, 1H). MS (CI/NH₃) m/z 522 (M+H)⁺. Anal. calcd for C₂₆H₂₆F₃NO₅S: C; 59.88; H, 5.02; N, 2.69. Found: C, 60.25; H, 5.12; N, 2.55.

### Example 250

### (Benzodioxan-6-yl)[2-nitro-4-(E-((4-carboxypiperidin-1-yl)carbonyl)ethenyl)phenyl]sulfide

The title compound was prepared by the hydrolysis of compound 198 under basic condition (aq. NaOH/EtOH), and purified by reversed-phase HPLC. ¹H NMR (DMSO-d₆, 300 MHz) δ 1.44 (m, 2H), 1.78 (m, 2H), 2.04 (m, 2H), 2.82 (m, 1H), 4.02-4.20 (m, 2H), 4.4.20-4.35 (m, 4H), 6.90 (d, J = 8.0 Hz,1H), 6.97 (d, J = 8.0 Hz, 1H), 7.05(dd, J = 2.0, 8.0 Hz, 1H), 7.10(d, J = 2.0 Hz, 1H), 7.15 (br, 1H), 7,44 (m 1H), 7.60 (br, 1H), 8.40 (s, 1H). MS (ESI) m/z 469 (M-1)⁻.

### Example 251

### (Benzodioxan-6-yl)[2-trifluoromethyl-4-(E-((3-carboxypyrrolidin-1-yl)carbonyl)ethenyl)phenyl]sulfide

The title compound was prepared according to the procedures of Example 1. ¹H NMR (CDCl₃, 300 MHz) δ 7.75 (s, 1H), 7.60 (d, 1H, J = 15.0 Hz) 7.40 (br, 1H), 7.06 (d, 1H, J = 2.2 Hz), 6.96-7.02 (m, 3H), 6.90 (d, 1H, J = 8.5 Hz), 4.30 (m, 5H), 3.99 (br, 2H), 3.29 (br, 2H), 2.60 (br, 2H), 1.85 (br, 2H). MS (ESI) *m*/*z* -492.

### Example 252

### (Benzodioxan-6-yl)[2-trifluoromethyl-4-(E-((4-carboethoxypiperidin-1-yl)carbonyl)ethenyl)phenyl]sulfide

The title compound was prepared by the procedures described in Example 240 substituting N-(3'-aminopropyl)-2-pyrrolidinone with ethyl isonipecotate, giving a white sticky solid. ¹H NMR (CDCl₃, 300 MHz) δ 1.26 (t, J = 7.0 Hz, 3H), 1.68-1.80 (m, 2H), 1.98-2.10 (, 2H), 2.54-2.70 (m, 2H), 3.00-3.30 (br, 2H), 4.15 (m, 3H), 4.26-4.34 (m, 4H), 6.90 (d, J = 8.0 Hz, 2H), 7.00 (dd, J = 2.0, 8.0 Hz, 2H), 7.06 (d, J = 2.0 Hz, 1H), 7.41 (m, 1H), 7.50 (br, 1H), 7.75 (s, 1H). MS (CI/NH3) m/z 522 (M+H)⁺. Anal. calcd. for C₂₄H₂₂F₃NO₅S• 0.1 H₂O: C, 58.20; H, 4.52; N, 2.83. Found: C, 58.14; H, 4.69; N, 2.76.

### Example 253

### (Benzodioxan-6-yl)[2-trifluoromethyl-4-(E-((2-carbomethoxy-4-tert-butoxycarbonylpiperazin-1-yl)carbonyl)ethenyl)phenyl]sulfide

The title compound was prepared by the procedures described in Example 240 substituting N-(3'-aminopropyl)-2-pyrrolidinone with 1-Boc-3-carbomethoxypiperazine, giving a white solid, mp 85-87 °C. ¹H NMR (CDCl₃, 300 MHz) δ 1.46 (s, 9H), 2.90-3.00 (m, 2H), 3.08-3.20 (m, 2H), 3.76 (s, 3H), 3.90 (m, 1H), 4.25-4.34 (m, 4H), 4.58-4.66 (m, 2H), 6.92 (d, J = 8.0 Hz, 1H), 6.98 (m, 1H), 7.02 (dd, J = 2.0, 8.0 Hz, 2H), 7.06 (d, J = 2.0 Hz, 1H), 7.40 (m, 1H), 7.62 (br, 1H), 7.76 (s, 1H). MS (APCI) m/z 609 (M+H)⁺. Anal. calcd. for C₂₉H₃₁F₃N₂O₇S: C, 57.23; H, 5.13; N, 4.60. Found: C, 57.09; H, 5.25; N, 4.37.

### Example 254

### (Benzodioxan-6-yl)[2-trifluoromethyl-4-(E-((2-carbomethoxy-4-methoxycarbonylpiperazin-1yl)carbonyl)ethenyl)phenyl]sulfide

The title compound was prepared by treating the compound of Example 255 with methyl chloroformate and pyridine in CH₂Cl₂ at room temperature, producing a white foam. ¹H NMR (CDCl₃, 300 MHz) δ 3.00 (m, 1H), 3.18 (m, 1H), 3.60 (m, 1H), 3.72 (s, 3H), 3.76 (s, 3H), 3.90 (m, 1H), 4.10 (br, 1H), 4.28-4.34 (m, 4H), 4.64 (m, 1H), 5.32 (m, 1H), 6.85 (d, J = 15.5 Hz, 1H), 6.92 (d, J = 8.0 Hz, 1H), 6.98 (m, 1H), 7.02 (dd, J = 2.0, 8.0 Hz, 1H), 7.08 (d, J = 2.0 Hz, 1H), 7.40 (d, J = 8.0 Hz, 1H), 7.64 (d, J = 15.0 Hz, 1H), 7.77 (s, 1H). MS (CI/NH₃) m/z 567 (M+H)⁺. Anal. calcd. for C₂₆H₂₅F₃N₂O₇S: C, 55.12; H, 4.45; N, 4.94. Found: C, 55.18; H, 4.70; N, 4.68.

### Example 255

### (Benzodioxan-6-yl)[2-trifluoromethyl-4-( E-((2-carbomethoxypiperazin-1-yl)carbonyl)ethenyl)phenyl]sulfide

The title compound was prepared by deprotection of compound 253 with TFA in CH₂Cl₂, resulting in a light yellow solid, mp 70-72 °C. ¹H NMR (CDCl₃, 300 MHz) δ 2.90 (m, 1H), 3.05 (m, 1H), 3.35 (m, 1H), 3.68 (m, 1H), 3.80 (s, 3H), 4 00 (m, 1H), 4.25-4.34 (m, 4H), 4,70 (br, 1H), 5.46 (m, 1H), 6.84 (d, J = 15.5 Hz, 1H), 6.90 (d, J = 8.0 Hz, 1H), 6.96-7.04 (m, 2H), 7.06 (m, 1H), 7.40 (d, J = 8.0 Hz, 1H), 7.65 (d, J = 15.5 Hz, 1H), 7.77 (s, 1H). MS (CI/NH₃) m/z 509 (M+H)⁺. Anal. calcd. for C₂₄H₂₃F₃N₂O₅S•1.55 H₂O: C, 53.74; H, 4.90; N, 5.22. Found: C, 54.04; H, 4.59; N, 4.82.

### Example 256

### (2-Methyl-3-(carboethoxymethyl)indol-5-yl)[2-trifluoromethyl-4-(E-((morpholin-1-yl)carbonyl)ethenyl)phenyl]sulfide

### Example 256A

### (4-Bromophenyl)[2-trifluoromethyl-4-(E-((morpholin-1-yl)carbonyl)ethenyl)phenyl]sulfide

The bromide was prepared by the procedure described in Example 12, substituting 2-bromothiophenol with 4-bromothiophenol, and 3,4-dichlorobenzaldehyde with 4-fluoro-3-trifluoromethylbenzaldehyde.

### Example 256B

### (4-Hydrazinophenyl)[2-trifluoromethyl-4-(E-((morpholin-1-yl)carbonyl)ethenyl)phenyl]sulfide, benzophenone hydrazone

To a stirred solution of above-described bromide (1.0 g, 2.12 mmol) in 10 mL of toluene with Pd(OAc)₂ (9.5 mg, 0.04 mmol), BINAP (40 mg, 0.06 mmol), and benzophenone hydrazone (437 mg, 2.12 mmol) was added NaOt-Bu (285 mg, 2.97 mmol). The reaction mixture was bubbled with N₂ for 2 min before it was heated at 80 °C for 4 h. The reaction mixture was then allowed to cool down to ambient temperature. Ether was then added and the mixture was filtered through celite, washed with diethyl ether. The filtrate was concentrate in vacuo and the residue was purified on a SiO₂ flash column chromatography eluting with 10-30% EtOAc/hexanes to give 170 mg (13%) of the title compound as light brown foamy solid.

### Example 256C

### (2-Methyl-3-(carboethoxymethyl)indol-5-yl)[2-trifluoromethyl-4-(E-((morpholin-1-yl)carbonyl)ethenyl)phenyl]sulfide

To a stirred solution of hydrazone (90 mg, 0.15 mmol) in 2 mL of ethanol was added levunilic acid (24 mL, 23 mmol) and *p*-TsOH (146 mg, 0.75 mmol). The mixture was then refluxed for 2 days. After cooled down to ambient temperature, the reaction mixture was partitioned between EtOAc and sat. NaHCO₃. The organic layer was then washed with brine, dried over Na₂SO₄, concentrated in vacuo. The residue was then purified on Gilson preparative HPLC as described in Example 38B to give 6.0 mg (7%) of the title compound. light-brown solid. ¹H NMR (CDCl₃, 300 MHz) δ 1.20 (t, *J* = 7.4 Hz, 3H), 2.46 (s, 3H), 3.55-3.83 (br m, 8H), 3.67 (s, 2H), 4.12 (q, *J* = 7.4 Hz, 2H), 6.79 (d, *J* = 15.3 Hz, 1H), 6.84 (d, *J* = 8.4 Hz, 1H), 7.23-7.31 (m, 2H), 7.34 (d, *J* = 8.4 Hz, 1H), 7.60 (d, *J* = 15.3 Hz, 1H), 7.76 (s, 1H), 7.80 (s, 1H), 8.04 (s, 1H). MS (ESI⁺) (M+H)⁺ at *m*/*z* 533.

### Example 257

### (1-(2-Methoxyethyl)indol-5-yl)[2-chloro-4-(E-((4-acetylpiperazin-1-yl)carbonyl)ethenyl)phenyl]sulfide

The title compound was prepared by the procedures described in Example 232, substituting *t*-butyl bromoacetate with bromoethylmethyl ether. white solid; ¹H NMR (CDCl₃, 300 MHz) δ 2.14 (s, 2H), 3.35 (s, 3H), 3.46-3.56 (m, 2H), 3.56-3.80 (m, 6H), 3.75 (t, *J* = 5.6 Hz, 2H), 4.33 (t, *J* = 5.6 Hz, 2H), 6.54 (d, *J* = 3.3 Hz, 1H), 6.61 (d, *J* = 8.7 Hz, 1H), 6.75 (d, *J* = 15.3 Hz, 1H), 7.09 (dd, *J* = 2.1, 11.7 Hz, 1H), 7.26 (overlapping d, 1H), 7.36 (dd, *J* = 2.1, 8.7 Hz, 1H), 7.44 (d, *J* = 8.7 Hz, 1H), 7.51 (d, *J* = 2.1 Hz, 1H), 7.56 (d, *J* = 15.3 Hz, 1H), 7.88 (d, *J* = 1.5 Hz, 1H). MS (ESI⁻) (M+H)⁺ at *m*/*z* 498, 500.

### Example 258

### (2-Isopropylphenyl)[2-nitro-4-(E-((3-acetoxymethyl-4-hydroxypiperidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 1.51-1.90 (br m, 2H); 1.92-2.06 (m, 3H); 2.50-3.21 (br m, 2H); 3.30-3.40 (m, 1H); 3.40-4.44 (br m, 5H); 4.88-4.97 (br m, 1H); 6.63 (d, J = 8.5 Hz, 1H); 7.31-7.62 (m, 6H); 7.87-7.94 (br m, 1H); 8.58-8.64 (br m, 1H). MS (APCI) (M+H)⁺ at m/z 499. Anal calcd for C₂₆H₃₀N₂S₁O₆·0.29H₂O: C, 61.98; H, 6.12; N, 5.56. Found: C, 62.00; H, 6.35; N, 5.55.

### Example 259

### (2-Isopropylphenyl)[2-nitro-4-(E-((3-(dimethylaminocarbonyl)-4-hydroxypiperidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.54-1.75 (br m, 2H); 2.81, 2.82 (br s, br s, 3H); 3.00, 3.04 (br s, br s, 3H); 2.75-3.60 (br m, 3H); 3.30-3.40 (m, 1H); 3.90-4.28 (br m, 2H); 4.95-5.28 (br m, 1H); 6.61-6.66 (m, 1H); 7.34-7.62 (m, 6H); 7.87-7.94 (br m, 1H); 8.58-8.63 (br m, 1H). MS (ESI) (M+H)⁺ at m/z 498. Anal calcd for C₂₆H₃₁N₃S₁O₅·0.34H₂O: C, 61.99; H, 6.34; N, 8.34. Found: C, 61.96; H, 6.37; N, 8.56.

### Example 260

### (2-Isopropylphenyl)[2-nitro-4-(E-((3-cyanomorpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 3.30-3.40 (m, 1H); 3.30-4.16 (br m, 5H); 4.20-4.29 (br m, 1H); 5.07 (t, J = 3.5 Hz, 1H); 6.65(d, J = 8.8 Hz, 1H); 7.32-7.44 (m, 2H); 7.54-7.62 (m, 4H); 7.91 (dd, J = 8.8, 2.0 Hz, 1H); 8.67 (d, J = 2.0 Hz, 1H). MS (APCI) (M+H)⁺ at m/z 438. Anal calcd for C₂₃H₂₃N₃S₁O₄·0.25C₆H₁₄: C, 64.11; H, 5.82; N, 9.15. Found: C, 63.99; H, 6.00; N, 9.12.

### Example 261

### (2-Isopropylphenyl)[2-nitro-4-(E-((3-carboethoxymorpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 1.12-1.27 (m, 3H); 3.30-3.40 (m, 1H); 3.15-4.33 (br m, 9H); 6.64 (d, J = 8.5 Hz, 1H); 7.32-7.42 (m, 2H); 7.50-7.62 (m, 4H); 7.88-7.96 (br m, 1H); 8.65 (br s, 1H). MS (APCI) (M+H)⁺ at.m/z 485. Anal calcd for C₂₅H₂₈N₂S₁O₆: C, 61.97; H, 5.82; N, 5.78. Found: C, 61.83; H, 6.07; N, 5.74.

### Example 262

### (2-Isopropylphenyl)[2-nitro-4-(E-((3-(tetrazol-5-yl)morpholin-1-yl)carbonyl)ethenyl)phenyl]sulfide

The compound of Example 260 (160 mg, 0.336), sodium azide (56.6 mg, 0.872 mmol), n-Bu₃SnCl and THF were mixed in a reaction tube, flushed with nitrogen and heated to reflux overnight. The mixture was then cooled to ambient temperature and 1N HCl soln. was added. The mixture was extracted with ethyl acetate three times and the combined organics were dried over MgSO₄. The mixture was filtered through a short silica gel plug to give 96 mg (56% yield) of the desired material. ¹H NMR (DMSO-d₆, 300MHz) δ 1.14 (d, J = 6.8 Hz, 6H); 2.96-4.62 (br m, 7H); 4.77 (dd, J = 10.5, 2.7 Hz, 1H); 6.58-6.67 (m, 1H); 7.32-7.62 (m, 6H); 7.92 (dd, J = 8.8, 2.0 Hz, 1H); 8.62-8.67 (br m, 1H). MS (APCI) (M+H)⁺ at m/z 481. Anal calcd for C₂₃H₂₄N₆S₁O₄^{·}1.2H₂O: C, 54.93; H, 5.31; N, 16.71. Found: C, 54.97; H, 5.12; N, 16.50.

### Example 263

### (Benzodioxan-6-yl)[2-trifluoromethyl-4-(E-((4-carboxypiperidin-1-yl)carbonyl)ethenyl)phenyl]sulfide

The title compound was prepared by hydrolysis of the compound of Example 252 under basic conditions (aq. NaOH/EtOH), giving a white solid, mp 88 °C (dec.). ¹H NMR (DMSO-d₆, 300 MHz) δ 1.40 (m, 2H), 1.98 (m, 2H), 2.95 (m, 1H), 3.15 (m, 1H), 3.45 (m, 1H), 4.20 (m, 2H), 4.35 (m, 4H), 7.00 (m, 4H), 7.20 (m, 2H), 7.90 (m, 1H), 8.20 (m, 1H), 12.30 (s, 1H). MS (APCI) m/z 494 (M+H)⁺. Anal. calcd. for C₂₄H₂₂F₃NO₅S•0.1 H₂O: C, 58.20; H, 4.52; N, 2.83. Found: C, 58.14; H, 4.69; N, 2.76.

### Example 264

### (Benzodioxan-6-yl)[2-trifluoromethyl-4-(E-((2-carboxypiperidin-1-yl)carbonyl)ethenyl)phenyl]sulfide

The title compound was prepared by hydrolysis of the compound of Example 249 under basic conditions (aq. NaOH/EtOH), resulting in a white solid, mp 90 °C (dec.). ¹H NMR (DMSO-d₆, 300 MHz) δ 1.15-1.50 (m, 2H), 1.50-1.70 (m, 2H), 2.16 (m, 1H), 2.56 (m, 1H), 3.15 (m, 1H), 4.30 (s, 4H), 4.32 (m, 1H), 5.20 (m, 1H), 7.02 (m, 4H), 7.30-7.52 (m, 2H), 7.84 (m, 1H), 8.15 (s, 1H). MS (APCI) m/z 494 (M+H)⁺. Anal. calcd. for C₂₄H₂₂F₃NO₅•0.3 H₂O: C, 57.78; H, 4.57; N, 2.81. Found: C, 57.87; H, 4.5
7; N, 2.76.

### Example 265

### (Benzodioxan-6-yl)[2-trifluoromethyl-4-(E-((4-carbomethoxypiperazin-1-yl)carbonyl)ethenyl)phenyl]sulfide

The title compound was prepared according to the procedures of Example 1. ¹H NMR (CDCl₃, 300 MHz) δ 7.76 (s, 1H), 7.62 (d, 1H, J = 15.0 Hz), 7.40 (d, 1H, J = 8.6 Hz) 7.06 (d, 1H, J = 2.1 Hz), 6.98-7.04 (m, 2H), 6.91 (d, 1H, J = 8.4 Hz), 6.84 (d, 1H, J = 15.6 Hz), 4.31 (m, 4H), 4.18 (q, 2H, J = 7.1 Hz), 3.68 (br, 4H), 3. 54 (br s, 4H), 1.29 (t, 3H, J = 7.2 Hz). MS (ESI) *m*/*z* 523, 545, 1045, 1067.

### Example 266

### (Benzodioxan-6-yl)[2-trifluoromethyl-4-(E-((3-aza-6,9-diooxaspiro[5,4]decan-1-yl)carbonyl)ethenyl)phenyl]sulfide

The title compound was prepared according to the procedures of Example 1. ¹H NMR (DMSO-d₆, 300 MHz) δ 8.13 (s, 1H), 7.84 (d, 1H, J = 9.0 Hz), 7.48 (d, 1H, J = 15.4 Hz) 7.38 (d, 1H, J = 15.4 Hz), 6.98-7.06 (m, 4H), 4.30 (m, 4H), 3.92 (s, 4H), 3.74 (br, 2H), 2.62 (br, 2H), 1.63 (br, 4H). MS (ESI) *m*/*z* 508, 1015.

### Example 267

### (Benzodioxan-6-yl)[2-trifluoro-4-(E-((4-(benzimidazolon-1-yl)piperidin-1-yl)carbonyl)ethenyl)phenyl]sulfide

The title compound was prepared according to the procedures of Example 1. ¹H NMR (CDCl₃, 300 MHz) δ 8.32 (s, 1H), 7.79 (s, 1H), 7.66 (d, 1H, J = 15.4 Hz), 7.44 (d, 1H, J = 8.5 Hz), 7.0-7.12 (m, 6H), 6.94 (d, 1H, J = 9.9 Hz), 6.90 (d, 1H, J = 2.6 Hz), 4.98 (m, 1H), 4.59 (m, 1H), 4.20 (m, 5H), 3.31 (br, 1H), 2.83 (br, 1H), 2.40 (m, 2H), 1.98 (m, 2H). MS (ESI) *m*/*z* 582, 604, 1163, 1185.

### Example 268

### (Benzodioxan-6-yl)[2-trifluoromethyl-4-(E-((4-(methylaminocarbonyl)piperidin-1-yl)carbonyl)ethenyl)phenyl]sulfide

The title compound was prepared according to the procedures of Example 1. ¹H NMR (CDCl₃, 300 MHz) δ 7.75 (s, 1H), 7.67 (d, 1H, J = 15.4 Hz) 7.40 (d, 1H, J = 8.1 Hz), 7.06 (d, 1H, J = 2.4 Hz), 6.96-7.02 (m, 2H), 6.90 (d, 1H, J = 8.2 Hz), 4.28 (m, 4H), 3.95 (br, 2H), 3.50 (m, 1H),2.82 (s, 3H), 2.40 (m, 1H), 2.15 (br, 1H), 1.88 (br, 1H), 1.73 (br, 2H). MS (ESI) *m*/*z* 507, 529, 1035.

### Example 269

### (Benzodioxan-6-yl)[2-trifluoromethyl-4-(E-((3-carbomethoxy-4-methoxycarbonylpiperazin-1-yl)carbonyl)ethenyl)phenyl]sulfide

The title compound was prepared by the procedures described in Example 240 substituting N-(3'-aminopropyl)-2-pyrrolidinone with 2-carbomethoxy-1-methoxycarbonylpiperazine, producing a light yellow solid, mp 56 °C (dec.). ¹H NMR (CDCl₃, 300 MHz) δ 2.70-3.50 (br, 4H), 3.70 (s, 3H), 3.76 (d, J = 9.0 Hz, 3H), 4.00(m, 1H), 4.20 (m, 4H), 4.50-5.00 (br, 2H), 6.91 (d, J = 8.5 Hz, 1H), 6.92-7.02 (m, 2H), 7.07 (d, J = 2.0 Hz, 1H), 7.25 (m, 1H), 7.40 (m, 1H), 7.60 (m, 1H), 7.72 (s, 1H). MS (APCI) m/z 567 (M+H)⁺. Anal. calcd. for C₂₆H₂₅F₃N₂O₇S: C, 55.12; H, 4.45; N, 4.94. Found: C, 55.33; H, 4.74; N, 4.76.

### Example 270

### (2-Isopropylphenyl)[2-nitro-4-(E-((3-carboxymorpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 3.08-4.33 (br m, 7H); 3.30-3.40 (m, 1H); 6.58-6.68 (m, 1H); 7.32-7.66 (m, 6H); 7.87-7.94 (m, 1H); 8.53-8.65 (m, 1H). MS (APCI) (M+H)⁺ at m/z 457. Anal calcd for C₂₃H₂₄N₂S₁O₆: C, 60.51; H, 5.30; N, 6.14. Found: C, 60.33; H, 5.54; N, 5.80.

### Example 271

### (Benzodioxan-6-yl)[2-trifluoromethyl-4-(E-((2-carboxy-4-methoxycarbonylipiperazin-1-yl)carbonyl)ethenyl)phenyl]sulfide

The title compound was prepared by treating the compound of Example 255 with methyl chloroformate and pyridine in CH₂Cl₂ at room temperature, and followed by hydrolysis under basic conditions (aq. NaOH/EtOH), producing a white solid, mp 102°C (dec.). ¹H NMR (DMSO-d₆, 300 MHz) δ 2.85 (m, 1H), 3.02 (m, 1H), 3.20 (m, 1H), 3.40 (m, 1H), 3.62 (s, 3H), 3.88 (m, 1H), 4.29 (s, 4H), 4.35 (m, 1H), 5.15 (m, 1H), 6.90-7.10 (m, 3H), 7.30 (d, J = I5.0 Hz, 1H), 7.40 (d, J = 15.0 Hz, 1H), 7.54 (d, J = 15.0 Hz, 1H), 7.82 (m, 1H), 8.15 (m, 1H). MS (ESI) m/z 553 (M+H)⁺. Anal. calcd. for C₂₅H₂₃F₃N₂O₇S•0.25 H₂O: C, 53.91; H, 4.25; N, 5.03. Found: 53.91; H, 4.35; N, 5.05.

### Example 272

### (Benzodioxan-6-yl)[2-trifluoromethyl-4-(E-((morpholin-1-yl)carbonyl)ethenyl)phenyl]sulfide

The title compound was prepared according to the procedures of Example 1. ¹H NMR (CDCl₃, 300 MHz) δ 7.76 (s, 1H), 7.62 (d, 1H, J = 15.6 Hz), 7.40 (dd, 1H, J = 1.8, 8.2 Hz), 7.04 (d, 1H, J = 2.1 Hz), 6.98-7.03 (m, 2H), 6.91 (d, 1H, J = 8.1 Hz), 6.81 (d, 1H, J = 15.3 Hz), 4.30 (m, 4H), 3.65-3.74 (br m, 8H). MS (ESI) *m*/*z* 452, 474, 925.

### Example 273

### (Benzodioxan-6-yl)[2-trifluoromethyl-4-(E-((4-(pyrrolidin-1-yl)piperidin-1-yl)carbonyl)ethenyl)phenyl]sulfide

The title compound was prepared according to the procedures of Example 1. ¹H NMR (CDCl₃, 300 MHz) δ 7.75 (s, 1H), 7.65 (d, 1H, J = 15.3 Hz), 7.40 (dd, 1H, J = 1.4, 8.3 Hz), 7.06 (d, 1H, J = 2.4 Hz), 6.98-7.02 (m, 2H), 6.90 (d, 1H, J = 8.1 Hz), 6.85 (d, 1H, J = 15.3 Hz), 4.68 (m,1H), 4.20 (m, 4H), 3.10 (m,1H), 3.14 (m,1H), 2.81 (s, 4H), 2.58 (br, 1H), 2.02 (s, 4H), 1.88 (s, 4H), 1.64 (m,1H). MS (ESI) *m*/*z* 519, 1037.

### Example 274

### (2-Isopropylphenyl)[2-nitro-4-(E-((3-aza-6,9-diooxaspiro[5.4]decan-1-yl)carbonyl)ethenyl)phenyl]sulfide

The title compound was prepared according to the procedures of Example 1. ¹H NMR (CDCl₃, 300 MHz) δ 8.44 (s, 1H), 7.50-7.62 (m, 4H), 7.41 (d, 1H, J = 8.0 Hz), 7.30 (m, 1H), 6.96 (br d, 1H, J = 15.6 Hz), 6.69 (d, 1H, J = 9.4 Hz), 4.00 (s, 4H), 3.75 (br m, 4H), 3.44 (m, 1H), 1.75 (br s, 4H), 1.18 (d, 6H, J = 7.0 Hz). MS (ESI) *m*/*z* 439,937.

### Example 275

### (2-Isopropylphenyl)[2-nitro-4-(E-((2-(dimethylaminomethyl)piperidin-1-yl)carbonyl)ethenyl)phenyl]sulfide

The title compound was prepared according to the procedures of Example 1. ¹H NMR (CDCl₃, 300 MHz) δ 8.40 (d, 1H, J = 1.8 Hz), 7.50-7.58 (m,4H), 7.42 (d, 1H, J = 8.1 Hz), 7.30 (dd, 1H, J = 1.9, 7.0 Hz), 7.00 (d, 1H, J = 15.4 Hz), 6.68 (d, 1H, J = 8.5 Hz), 5.10 (br, 1H), 3.92 (br, 1H), 3.44 (quintet, 1H, J = 6.9 Hz), 3.20 (m,1H), 2.26-2.50 (m, 7H), 1.62-1.85 (m, 7H), 1.48 (m,1H), 1.18 (d, 6H, J = 7.0 Hz). MS (ESI) *m*/*z* 468.

### Example 276

### (2-Isopropylphenyl)[2-nitro-4-(E-((piperidin-1-ylamino)carbonyl)ethenyl)phenyl]sulfide

The title compound was prepared according to the procedures of Example 1. ¹H NMR (CDCl₃, 300 MHz) δ 8.44 (d, 1H, J = 1.8 Hz), 7.66 (d, 1H, J = 16.2 Hz), 7.55 (d, 1H, J = 7.4 Hz), 7.47-7.51 (m, 3H), 7.30 (m,2H), 6.72 (d, 1H, J = 8.5 Hz), 6.37 (s, 1H), 3.48 (m, 2H), 3.10 (m,2H), 2.63 (m,1H), 1.81-1.89 (m,2H), 1.62-1.77 (m,4H), 1.19 (d, 6H, J = 7.0 Hz). MS (ESI) *m*/*z* 426, 851.

### Example 277

### (Benzodioxan-6-yl)[2-trifluoromethyl-4-(E-((3-carboxy-4-methoxycarbonylpiperazin-1-yl)carbonyl)ethenyl)phenyl]sulfide

The title compound was prepared by hydrolysis of the compound of Example 269 under basic conditions (aq. NaOH/EtOH). ¹H NMR (DMSO-d₆, 300 MHz) δ 2.60-3.30 (m, 3H), 3.40-3.50 (m, 1H), 3.62 (d, J=12.0 Hz, 1H),3.80 (m, 1H), 4.25-4.35 (m, 4H), 4.55 (m, 1H), 7.00 (s, 2H), 7.00-7.06 (m, 1H), 7.25 (m,2H), 7.5 (m, 1H), 7.80 (m, 1H), 8.10 (m, 1H). MS (APCI) m/z 553 (M+H)⁺. Calcd. Anal. C₂₄H₂₃F₃N₂O₅•1.55 H₂O: C, 54.35; H, 4.20; N, 5.07. Found: C, 54.16; H, 4.19; N, 4.96.

### Example 278

### (2-(Dimethylaminocarbonyl)-benzodioxan-6-yl)[2-chloro-4-(E-((4-acetylpiperazin-1-yl)carbonyl)ethenyl)phenyl]sulfide

The title compound was prepared by the procedures described in Example 85, substituting 5-iodoindole with 2-*N,N*-dimethylcarboxamide-6-bromobenzenedioxane and 3-*N,N*-dimethylcarboxamide-6-bromobenzenedioxane, giving a white solid. ¹H NMR (CDCl₃, 300 MHz, mixture of regioisomers) δ 1.93 (s, 3H), 2.15 (s, 6H), 3.53 (br s, 2H), 3.59-3.90 (br m, 8H), 4.86-5.01 (m, 1H), 6.74-6.81 (m, 1H), 6.80 (d, *J* = 15.3 Hz, 1H), 6.93 (d, *J* = 8.7 Hz, 1H), 7.02 (d, CDCl₃1.8 Hz, 1H), 7.13 (dd, *J* = 1.8, 8.4 Hz, 1H), 7.16-7.25 (m, 1H), 7.54 (s, 1H), 7.58 (d, *J* = 15.6 Hz, 1H). MS (ESI⁺) (M+Na)⁺ at *m*/*z* 552, 554.

### Example 279

### (2-Isopropylphenyl)[2-nitro-4-(E-((3-(2-(methoxymethyl)tetrazol-5-yl)piperidin-1-yl)carbonyl)ethenyl)phenyl]sulfide

The title compound was prepared by the procedures described in Example 225, substituting ethyl nipecotate with 3-*N*-methoxymethyltetrazolylpiperidine, to give a light-yellow solid. ¹H NMR (CDCl₃, 300 MHz) δ 1.19 (d, *J* = 6.9 Hz, 6H), 1.62-1.80 (br m, 2H), 1.80-2.20 (br m, 2H), 2.20-2.39 (br m, 2H), 3.12-3.38 (br m, 2H), 3.46 (s, 3H), 4.11 (septet, *J* = 6.9 Hz, 1H), 4.17-4.34 (br m, 1H), 5.79 (s, 2H), 6.70 (br s, 1H), 7.05 (d, *J* = 15.3 Hz, 1H), 7.31 (d, *J* = 7.8 Hz, 1H), 7.35-7.68 (m, 5H), 8.42 (br s, 1H). MS (ESI⁺) (M+H)⁺ at *m*/*z* 523.

### Example 280

### (2-Isopropylphenyl)[2-nitro-4-(E-((3-(1-(methoxymethyl)tetrazol-5-yl)piperidin-1-yl)carbonyl)ethenyl)phenyl]sulfide

The title compound was prepared by the procedures described in Example 279 and separated from the same reaction mixture via SiO₂ flash column chromatography, to give a light-yellow solid. ¹H NMR (CDCl₃, 300 MHz) δ 1.19 (d, *J* = 6.9 Hz, 6H), 1.62-1.80 (br m, 2H), 1.80-2.20 (br m, 2H), 2.20-2.39 (br m, 2H), 3.12-3.38 (br m, 2H), 3.46 (s, 3H), 4.11 (septet, *J* = 6.9 Hz, 1H), 4.17-4.34 (br m, 1H), 5.79 (s, 2H), 6.70 (br s, 1H), 7.05 (d, *J* = 15.3 Hz, 1H), 7.31 (d, *J*= 7.8 Hz, 1H), 7.35-7.68 (m, 5H), 8.42 (br s, 1H). MS (ESI⁺) (M+H)⁺ at *m*/*z* 523.

### Example 281

### (1-Methylindol-5-yl)[2-chloro-4-(E-((3-(1-pyrrolidin-2-onyl)propylamino)carbonyl)ethenyl)phenyl]sulfide

### Example 281A

### Triisopropylsilyl(1-methylindol-5-yl)sulfide

To a stirred solution of 5-bromo-*N*-methyl indole (300 mg, 1.43 mmol) in 5 mL of benzene in a sealed tube was charged with Pd(PPh₃)₄ (82 mg, 0.072 mmol), followed by KSTIPS (326 mg, 1.43 mmol). The mixture was flushed with N₂, the tube was capped, and the reaction mixture refluxed for 2 h. The reaction mixture was then allowed to cool down, partitioned between Et₂O and water. The organic layer was washed with brine, dried over Na₂SO₄, concentrated in vacuo. The residue was purified on a SiO₂ flash column chromatography eluting with 5% EtOAc/hexanes to give 400 mg (88 %) of the title compound as colorless oil.

### Example 281B

### 3-Chloro-4-((1-methylindol-5-yl)thio) benzaldehyde

To a stirred solution of thiolsilyl ether (1.0 g, 3.13 mmol) in 5 mL of DMF with 3-chloro-4-flurobenzaldehyde (500 mg, 3.13 mmol) at ambient temperature was added CsF (5.7 mg, 0.38 mmol). The mixture was stirred over night before it was poured in water and extracted with Et₂O (2×25 mL). The combined organic layer was washed with water and brine, dried over Na₂SO₄, concentrated in vacuo. The residue was purified on a SiO₂ flash column chromatography eluting with 5-10 % EtOAc/hexanes to give 650 mg (71 %) of the title compound as white solid.

### Example 281C

### (1-Methylindol-5-yl)[2-chloro-4-(E-((3-(1-pyrrolidin-2-onyl)propylamino)carbonyl)ethenyl)phenyl]sulfide

The title compound was prepared by the procedures described in Example 92, substituting the benzoic acid with cinnamic acid prepared from the above-described aldehyde, and ammonium with 3-aminopropyl-1-pyrrolidin-2-one, to give a white solid. ¹H NMR (CDCl₃, 300 MHz) δ 1.74 (br m, 2H), 2.07 (br m, 2H), 2.44 (br m, 2H), 3.32 (br m, 2H), 3.40 (br m, 4H), 3.85 (s, 3H), 6.36 (d, *J* = 15.3 Hz, 1H), 7.14 (d, *J* = 3.0 Hz, 1H), 7.36 (dd, *J* = 1.5, 9.0 Hz, 1H), 7.41 (d, *J* = 9.0 Hz, 1H), 7.50 (s, 1H), 7.89 (d, *J* = 1.5 Hz, 1H). MS (ESI⁺) (M+H)⁺ at *m*/*z* 468, 470. Anal. Calcd for C₂₅H₂₆ClN₃O₂S · 1.37 H₂O: C, 60.95; H, 5.88; N, 8.53. Found: C, 60.97; H, 5.98; N, 8.46.

### Example 282

### (2-Isopropylphenyl)[2-nitro-4-(E-((3-(tetrazol-5-yl)piperidin-1-yl)carbonyl)ethenyl)phenyl]sulfide

The compound from Example 279 (75 mg, 0.14 mmol) was dissolved in I mL of neat TFA and left at ambient temperature for overnight. The reagent was then removed in vacuo and the residue was evaporated twice with benzene. The crude product was purified using Gilson Preparative HPLC as described in Example 38B to give the title compound as a light-yellow solid (50 mg, 72 %); ¹H NMR (CDCl₃, 300 MHz) δ 1.17 (d, *J* = 6.5 Hz, 6H), 1.25-1.39 (m, 1H), 1.69-1.81 (m, 1H), 2.09 (br s, 1H), 2.14-2.30 (m, 1H), 2.57-2.71 (m, 1H), 3.35-3.66 (m, 3H), 3.90-4.03 (m, 1H), 4.66-4.78 (m, 1H), 6.73 (d, *J* = 8.7 Hz, 1H), 6.86 (d, *J* = 15.3 Hz, 1H), 7.32 (dd, *J* = 2.1, 6.9 Hz, 1H), 7.42 (dd, *J* = 2.1, 8.7 Hz, 1H), 7.47-7.57 (m, 3H), 7.76 (d, *J* = 15.3 Hz, 1H), 8.46 (d, *J* = 2.1 Hz, 1H). MS (ESI⁺) (M+H)⁺ at *m*/*z* 479. Anal. Calcd for C₂₄H₂₆N₆O₃S · 0.28 H₂O: C, 59.61; H, 5.54; N, 17.38. Found: C, 59.71; H, 5.44; N, 16.99.

### Example 283

### (1-Methylindol-5-yl)[2-chloro-4-(E-((3-carboethoxypiperidin-1-yl)carbonyl)ethenyl)phenyl]sulfide

The title compound was prepared by the procedures described in Example 281 C, substituting aminopropyl pyrrolidinone with ethyl nipecotate, giving a white solid. ¹H NMR (CDCl₃, 300 MHz) δ 1.26 (t, *J* = 7.5 Hz, 3H), 1.65-1.96 (m, 2H), 2.00-2.20 (m, 1H), 2.04 (s, 1H), 2.54 (br m, 1H), 3.12-3.34 (m, 1H), 3.85 (s, 3H), 3.92-4.07 (m, 1H), 4.07-4.20 (m, 1H), 4.15 (q, *J* = 7.5 Hz, 2H), 4.65-4.90 (m, 1H), 6.53 (d, *J =* 3.0 Hz, 1H), 6.57(d, *J* = 8.1 Hz, 1H), 6.85 (d, J = 15.3 Hz, 1H), 7.08 (d, *J* = 8.7 Hz, 1H), 7.14 (d, *J* = 3.0 Hz, 1H), 7.37 (dd, *J* = 1.5, 8.7 Hz, 1H), 7.42 (d, *J* = 8.7 Hz, 1H), 7.51 (s, 1H), 7.51 (d, *J* = 15.3 Hz, 1H), 7.89 (d, *J* = 1.5 Hz, 1H). MS (ESI⁺) (M+H)⁺ at *m*/*z* 483, 485.

### Example 284

### (1-Methylindol-5-yl)[2-chloro-4-(E-((3-carboxypiperidin-1-yl)carbonyl)ethenyl)phenyl]sulfide

The title compound was prepared by the procedures described in Example 155, substituting the ethyl ester from Example 137 with ethyl ester from Example 283, and KOH with NaOH, to provide a white solid. ¹H NMR (CDCl₃, 300 MHz) δ 1.45-1.69 (m, 1H), 1.69-1.98 (m, 2H), 1.98-2.22 (m, 1H), 2.51-2.70 (m, 1H), 3.05-3.47 (m, 1H), 3.80-4.20 (m, 2H), 3.85 (s, 3H), 4.47-4.68 (m, 1H), 6.53 (d, *J* = 3.0 Hz, 1H), 6.57 (d, *J* = 8.1 Hz, 1H), 6.87 (d, *J* = 15.3 Hz, 1H), 7.08 (d, *J* = 8.1 Hz, 1H), 7.14 (d, *J* = 3.0 Hz, 1H), 7.37 (d, *J* = 9.0 Hz, 1H), 7.42 (d, *J* = 9.0 Hz, 1H), 7.51 (s, 1H), 7.52 (d, *J* = 15.3 Hz, 1H), 7.89 (br s, 1H). MS (ESI⁺) (M-H+H)⁺ at *m*/*z* 453, 455.

### Example 285

### (1-Methylindol-5-yl)[2-chloro-4-(E-((4-carboethoxypiperidin-1-yl)carbonyl)ethenyl)phenyl]sulfide

The title compound was prepared by the procedures described in Example 281 C, substituting aminopropyl pyrrolidinone with ethyl isonipecotate, giving a white solid. ¹H NMR (CDCl₃, 300 MHz) d 1.26 (t, *J* = 7.5 Hz, 3H), 1.64-1.83 (m, 2H), 1.88-2.08 (m, 2H), 2.48-2.67 (m, 1H), 2.86-3.40 (m, 2H), 3.85 (s, 3H), 3.89-4.24 (m, 1H), 4.15 (q, *J* = 7.5 Hz, 2H), 4.24-4.65 (m, 1H), 6.53 (d, *J* = 3.0 Hz, 1H), 6.58 (d, *J* = 8.1 Hz, 1H), 6.81 (d, *J =* 15.3 Hz, 1H), 7.07 (d, *J =* 8.1 Hz, 1H), 7.14 (d, *J* = 3.0 Hz, 1H), 7.37 (dd, *J* = 1.5,9.0 Hz, 1H), 7.50 (d, *J* = 9.0 Hz, 1H), 7.50 (d, *J* = 15.3 Hz, 1H), 7.88 (d, *J* = 1.5 Hz, 1H). MS (ESI⁺) (M+H)⁺ at *m*/*z* 483, 485.

### Example 286

### (1-Methylindol-5-yl)[2-chloro-4-(E-((3-carboxypiperidin-1-yl)carbonyl)ethenyl)phenyl]sulfide

The title compound was prepared by the procedures described in Example 155, substituting the ethyl ester from Example 137 with ethyl ester from Example 285, and KOH with NaOH, giving a white solid. ¹H NMR (CDCl₃, 300 MHz) δ 1.60-1.90 (m, 2H), 1.90-2.10 (m, 2H), 2.57-2.72 (m, 1H), 2.80-3.40 (m, 2H), 3.85 (s, 3H), 3.91-4.20 (m, 1H), 4.30-4.68 (m, 1H), 6.53 (d, *J* = 3.0 Hz, 1H), 6.57 (d, *J* = 8.1 Hz, 1H), 6.80 (d, *J* = 15.3 Hz, 1H), 7.07 (d, *J* = 8.1 Hz, 1H), 7.15 (d, *J* = 3.0 Hz, 1H), 7.37 (dd, *J* = 1.5, 9.0 Hz, 1H), 7.51 (d, *J* = 9.0 Hz, 1H), 7.51 (s, 1H), 7.51 (d, *J* = 15.3 Hz, 1H), 7.89 (br s, 1H). MS (ESI⁺) (M+H)⁺ at *m*/*z* 455, 457. Anal. Calcd for C₂₄H₂₃ClN₂O₃S · 0.42 H₂O: C, 62.32; H, 5.20; N, 6.06. Found: C, 62.35; H, 5.30; N, 5.87.

### Example 287

### (2-Isopropylphenyl)[2-nitro-4-(E-((2-(1-methylpyrrolidin-2-yl)ethylamino)carbonyl)ethenyl)phenyl]sulfide

The title compound was prepared according to the procedures of Example 1. ¹H NMR (CDCl₃, 300 MHz) δ 8.44 (d, 1H, J = 1.8 Hz), 7.56 (d, 1H, J = 3.7 Hz), 7.50-7.58 (m, 3H), 7.43 (DD, 1H, J = 1.84, 8.4 Hz), 7.30 (dd, 1H, J = 2.2, 6.8 Hz), 6.78 (d, 1H, J = 8.5 Hz), 6.52 (d, 1H, J = 15.8 Hz), 3.63 (m,2H), 3.42 (m, 3H), 3.00 (m,1H), 3.78 (m,1H), 2.59 (s, 3H), 2.05 (m,1H), 2.00 (m, 5H), 1.18 (d, 6H, J = 7.0 Hz). MS (ESI) *m*/*z* 454, 490.

### Example 288

### (2-Isopropylphenyl)[2-nitro-4-(E-((4-(pyrrolidin-1-yl)piperidin-1-yl)carbonyl)ethenyl)phenyl]sulfide

The title compound was prepared according to the procedures of Example 1. ¹H NMR (CDCl₃, 300 MHz) δ 8.43 (d, 1H, J = 1.8 Hz), 7.57 (d, 1H, J = 8.5 Hz), 7.51-7.55 (m, 3H), 7.41 (dd, 1H, J = 1.84, 8.8 Hz),7.31 (dd, 1H, J = 2.4, 7.5 Hz),6.92 (d, 1H, J = 15.4 Hz), 6.70 (d. 1H, J = 8.5 Hz), 4.70 (m, 1H), 4.10 (m,1H), 3.44 (pent, 1 H, J = 6.8 Hz), 3.16 (m, 1H), 2.80 (br, 4H), 2.55 (br, 1H), 2.03 (m,4H), 1.90 (m, 4H), 1.65 (m, 1H), 1.18 (d, 6H, J = 7.0 Hz). MS (ESI) *m*/*z* 480, 959.

### Example 289

### (2-Isopropylphenyl)[2-nitro-4-(E-((4-sulfopiperidin-1-yl)carbonyl)ethenyl)phenyl]sulfide

The title compound was prepared according to the procedures of Example 1. ¹H NMR (DMSO-d₆, 300 MHz) δ 8.63 (d, 1H, J = 1.8 Hz), 7.92 (dd, 1H, J = 1.8,8.8 Hz), 7.60 (m, 3H), 7.47 (d, 1H, J = 14.2 Hz), 7.42 (d, 1H, J = 14.2 Hz), 6.62 (d, 1H, J = 8.5 Hz), 4.45 (m, 2H), 4.38 (m, 2H), 3.34 (m, 1H), 3.00 (m, 2H), 2.70 (m, 1H), 2.60 (m,2H), 1.14 (d, 6H, J = 6.9 Hz). MS (ESI) *m*/*z* 491,981.

### Example 290

### (2-Isopropylphenyl)[2-nitro-4-(E-((3-hydroxypiperidin-1-yl)carbonyl)ethenyl)phenyl]sulfide

The title compound was prepared according to the procedures of Example 1. ¹H NMR (CDCl₃, 300 MHz) δ 8.43 (s, 1H), 7.50-7.62 (m, 4H), 7.41 (d, 1H, J = 8.1 Hz), 6.97 (m, 1H), 6.69 (d, 1H, J = 8.1 Hz), 3.85 (m, 2H), 3.65 (m, 1H), 3.50 (m,3H), 1.93 (m,2H), 1.65 (m, 2H), 1.18 (d, 6H, J = 6.6 Hz). MS (ESI) *m*/*z* 427, 449, 853, 875.

### Example 291

### (Benzodioxan-6-yl)[2-trifluoromethyl-4-(E-((3-((ethanesulfonylamino)carbonyl)piperidin-1-yl)carbonyl)ethenyl)phenyl]sulfide

The title compound was prepared by the procedures described in Example 227. The product was purified by reversed-phase HPLC. ¹H NMR (CDCl₃, 300 MHz) δ 1.34 (t, J = 7.0 Hz, 2H), 1.44 (t, J=7.0 Hz, 3H), 1.95 (br, 1/2H), 2.20 (br, 1/2H), 2.68 (br, 1H), 3.14 (q, J=7.0 Hz, 2H), 3.45 (m, 1H), 3.65 (m, 1H), 3.93 (m, 1H), 4.30 (m, 4H), 4.50-4.60 (br, 2H), 6.92 (d, J=8.0 Hz, 1H), 6.98-7.04 (m, 3H), 7.06 (m, 1H), 7.40 (d, J=8.0 Hz, 1H), 7.65 (m, 1H), 7.75 (s, 1H). MS (APCI) m/z 585 (M+H)⁺.

### Example 292

### (Benzodioxan-6-yl)[2-trifluoromethyl-4-(E-((3-((p-toluenesulfonylamino)carbonyl)piperidin-1-yl)carbonyl)ethenyl)phenyl]sulfide

The title compound was prepared by the same procedure described in Example 229. ¹H NMR (CDCl₃, 300 MHz) δ 1.25 (m, 2H), 1.55 (m, 1H), 1.70-2.25 (br, 1H), 2.41 (d, J = 13.0 Hz, 3H), 2.55 (br, 1H), 3.50-3.80 (br, 2H), 4.20-4.35 (m, 4H), 4.68-4.75 (m, 2H), 6.90 (d, J = 8.0 Hz, 1H), 7.00-7.10 (m, 2H), 7.30 (d, J = 8.0 Hz, 1H), 7.81 (d, J = 8.0 Hz, 1H), 7.91 (m, 1H). MS (CI/NH₃) m/z 647 (M+H)⁺. Anal. calcd. for C₃₁H₂₉F₃N₂O₆S₂ •0.5 H₂O: C, 56.78; H, 4.61; N, 4.27. Found: C, 56.86; H, 4.69; N, 4.35.

### Example 293

### (Benzodioxan-6-yl)[2-trifluoromethyl-4-(E-((4-((ethanesulfonylamino)carbonyl)piperidin-1-yl)carbonyl)ethenyl)phenyl]sulfide

The title compound was prepared by the procedures described in Example 227, giving a white foam. ¹H NMR (CDCl₃, 300 MHz) δ 1.35-1.40 (m, 2H), 1.44 (t, J=7.0 Hz, 3H), 1.76 (m, 1H), 2.0 (m, 1H), 2.50-3.20 (br, 1H), 3.15 (q, J=7.0 Hz, 2H), 3.40-3.55 (m, 2H), 4.25-4.32 (m, 4H), 4.52 (br, 2H), 6.90 (d, J=8.0 Hz, 1H), 6.98-7.05 (dd, J=2.0, 8.0 Hz, 2H), 7.06 (d, J=2.0 Hz, 1H), 7.40 (m, 1H), 7.60 (m, 1H), 7.75 (s, 1H), 8.22 (br, 1H). MS (APCI) m/z 585 (M+H)⁺. Anal. calcd. for C₂₆H₂₇F₃N₂O₆S₂•0.8 H₂O: C, 52.13; H, 4.81; N, 4.68. Found: C; 52.14; H, 4.80; N, 4.66.

### Example 294

### (Benzodioxan-6-yl)[2-trifluoromethyl-4-(E-((2(tetrazol-5-yl)morpholin-1-yl)carbonyl)ethenyl)phenyl]sulfide

The corresponding nitrile (160 mg, 0.336 mmol, prepared via the procedures of Example 1), sodium azide (56.6 mg, 0.872 mmol), n-Bu₃SnCl and THF were mixed in a reaction tube, flushed with nitrogen and heated to reflux overnight. The mixture was then cooled to ambient temperature, and 1N HCl soln. was added. The mixture was extracted with ethyl acetate three times and the combined organics were dried over MgSO₄. The mixture was filtered through a short silica gel plug to give 96 mg (56% yield) of the desired material. ¹H NMR (DMSO-d₆, 500 MHz, 100 °C) δ 7.99 (d, 1H, J = 1.7 Hz), 7.79 (dd, 1H, J = 2.0, 8.6 Hz), 7.50 (d, 1H, J = 15.3 Hz), 7.24 (d, 1H, J = 15.6 Hz), 7.14 (d, 1H, J = 8.2 Hz), 6.96 (m, 1H), 6.94 (d, 1H, J = 2.1 Hz), 6.92 (m, 1H), 4.60 (dd, 1H, J = 3.0, 9.8 Hz), 4.50 (br d, 1H, J = 12.2 Hz), 4.26 (m, 5H), 4.17 (m, 1H), 4.00 (dt, 1H, J = 3.2, 11.6 Hz), 3.72 (td, 1H, J = 3.0, 11.0 Hz), 3.43 (br m, 1H), 3.29 (br m, 1H). MS (ESI) *m*/*z* -518. Anal. Calcd for C₂₃H₂₀F₃N₅O₄S · 1.83 HOAc: C, 50.88; H, 4.38; N, 11.13. Found: C, 50.61; H, 4.46; N, 11.4.

### Example 295

### (2-Isopropylphenyl)[2-nitro-4-(E-((2-butyl, 5-(tetrazol-5-yl)morpholin-1-yl)carbonyl)ethenyl)phenyl]sulfide

### Example 295A

### 2-Butyl-5-cyanomorpholine

The title compound was prepared by the procedures described in Example 260A, substituting ethanolamine with 2-aminohexanol.

### Example 295B

### (2-Isopropylphenyl)[2-nitro-4-(E-((2-butyl-5-cyanomorpholin-1-yl)carbonyl)ethenyl)phenyl]sulfide

The title compound was prepared by the procedures described in Example 260B, substituting the morpholine from Example 260A with the compound of Example 295A.

### Example 295C

### (2-Isopropylphenyl)[2-nitro-4-(E-((2-butyl-5-(tetrazol-5-yl)morpholin-1-yl)carbonyl)ethenyl)phenyl]sulfide

The title compound was prepared by the procedures described in Example 262, substituting the nitrile compound from Example 260 with the compound of Example 295B, giving a light-yellow solid. ¹H NMR (CDCl₃, 300 MHz, 3:2 mixture of diastereomers) δ 0.89 (t, *J* = 7.5 Hz, 1H), 1.01 (br m, 1H), 1.19 (d, *J* = 6.5 Hz, 6H), 1.23-1.43 (m, 4H), 1.68-1.84 (m, 1H), 3.10-3.61 (m, 2H), 3.83-4.17 (m, 2H), 4.40-5.26 (m, 2H), 6.67-6.77 (m, 1H), [6.91 (d), 7.02 (d), *J* = 15.3 Hz, 1H in total], 7.25-7.37 (m, 2H), 7.44-7.60 (m, 3H), [7.67 (d), 7.79 (d), *J =* 15.3 Hz, 1H in total], 8.43-8.50 (m, 1H). MS (ESI⁺) (M-H)⁺ at *m*/*z* 535.

### Example 296

### (2-(and 3-)(Hydroxymethyl)-benzodioxan-6-yl)[2-nitro-4-(E-((4-acetylpiperazin-1-yl)carbonyl)ethenyl)phenyl]sulfide

### Example 296A

### Triisopropylsilyl (2-(and 3-)hydroxymethylbenzodioxan-6-yl)sulfide

The title compound was prepared by the procedures described in Example 281A, substituting 5-bromo-*N*-methyl indole with a mixture of 6-bromo-2-hydroxymethylbenzenedioxane and 6-bromo-3-hydroxymethylbenzenedioxane.

### Example 296B

### (2-(and 3-)(Hydroxymethyl)-benzodioxan-6-yl)[2-nitro-4-(E-((4-acetylpiperazin-1-yl)carbonyl)ethenyl)phenyl]sulfide

The title compound was prepared by the procedures described in Example 281B, substituting 3-chloro-4-flurobenzaldehyde with 4-chloro-3-nitrocinnamide, giving a light yellow solid. ¹H NMR (CDCl₃, 300 MHz, 3:2 mixture of diastereomers) δ [2.11 (s), 2.15 (s), 3H in total], 3.48-3.83 (m, 8H), 3.83-4.04 (m, 2H), 4.20 (dd, *J* = 8.4, 11.4 Hz, 1H), 4.26-4.44 (m, 2H), 6.89 (d, *J* = 5.7 Hz, 1H), 6.92 (s, 1H), 6.97-7.11 (m, 1H), 7.04 (d, *J* = 15.0 Hz, 1H), 7.14 (d, *J* = 2.1 Hz, 1H), 7.46 (br d, *J* = 9.0 Hz, 1H), 7.65 (d, *J* = 15.0 Hz, 1H), 8.41 (d, *J* = 2.1 Hz, 1H). MS (ESI⁺) (M+H)⁺ at *m*/*z* 500.

### Example 297

### (2-(and 3-)(Hydroxymethyl)-benzodioxan-6-yl)[2-nitro-4-(E-((3-(pyrrolidin-2-on-1-yl)prop-1-ylamino)carbonyl)ethenyl)phenyl]sulfide

The title compound was prepared by the procedures described in Example 296B, substituting the acetylpiperazine 4-chloro-3-nitrocinnamide with 3-aminopropyl-1-pyrrolidin-3-one 4-chloro-3-nitrocinnamide, giving a light-yellow solid. ¹H NMR (CDCl₃, 300 MHz, 3:2 mixture of diastereomers) δ 1.75 (br m, 2H), 2.08 (p; *J =* 7.5 Hz, 2H), 2.45 (t, *J =* 7.5 Hz, 2H), 3.27-3.48 (m, 6H), 3.82-4.03 (m, 2H), 4.13-4.44 (m, 3H), 6.49 (d, *J* = 15.0 Hz, 1H), 6.88 (d, *J* = 8.4 Hz, 1H), [6.99 (d), 7.01 (d), *J =* 8.4 Hz, 1H in total], [7.06 (dd), 7.08 (dd), *J =* 1.5, 2.4 Hz, 1H in total], [7.13 (d), 7.14 (d), *J* = 2.4 Hz, 1H in total], 7.17 (br s, 1H), 7.46 (d, *J* = 8.4 Hz, 1H), 7.54 (d, *J* = 15.0 Hz, 1H), 8.36 (d, *J* = 1.5 Hz, 1H). MS (ESI⁺) (M+H)⁺ at *m*/*z* 514.

### Example 298

### (2-(and 3-)(Hydroxymethyl)-benzodioxan-6-yl)[2-trifluoromethyl-4-(E-((3-(pyrrolidin-2-on-1-yl)prop-1-ylamino)carbonyl)ethenyl)phenyl]sulfide

The title compound was prepared by the procedures described in Example 281, substituting 6-thiolsilyl indole with the thiolsilyl ether described in Example 296A, and 3-chloro-4-fluorobenzaldehyde with 4-fluoro-3-trifluoromethylbenzaldehyde, producing a white solid. ¹H NMR (CDCl₃, 300 MHz, 3:2 mixture of diastereomers) δ 1.75 (br m, 2H), 2.09 (br m, 2H), 2.45 (br m, 2H), 3.25-3.60 (m, 6H), 3.80-4.43 (m, 5H), 6.46 (d, *J* = 15.3 Hz, 1H), [6.92 (d), 6.95 (d), *J* = 6.8 Hz, 1H in total], [7.03 (d), 7.04 (d), *J* = 8.1 Hz, 1H in total], 7.06-7.10 (m, 1H), 7.13 (br s, 1H), 7.42 (d, *J* = 8.1 Hz, 1H), 7.54 (d, *J* = 15.3 Hz, 1H), 7.77 (s, 1H). MS (ESI⁺) (M+H)⁺ at *m*/*z* 537.

### Example 299

### (3-Hydroxymethyl)-benzodioxan-6-yl)[2-nitro-4-(E-((3-(pyrrolidin-2-on-1-yl)prop-1-ylamino)carbonyl)ethenyl)phenyl]sulfide

### Example 299A

### 3-(Hydroxymethyl)-6-bromo-benzodioxane

To a stirred solution of 5-bromosalicylaldehyde (5.0 g, 24.9 mmol), and epichlorohydrin (5.6 mL, 72.1 mmol) in 20 mL of DMF at 80 °C was added K₂CO₃ slowly in portions. The resulting mixture was then heated at 90 °C for 3 h. Reaction was then stopped, water was added, extracted with diethyl ether. The organic extracts were washed with water, brine, dried over Na₂SO₄, concentrated in vacuo. The residue was purified on a SiO₂ flash column chromatography eluting with 15-30 % EtOAc/hexanes to give 2.82 g (44 %) of the title compound as colorless oil.

To a stirred solution of the aldehyde (2.82 g, 11 mmol) in 35 mL of CHCl₃ was added mCPBA (2.27 g, 13 mmol). The mixture was stirred at ambient temperature for 30 min and then heated at 50 °C for 2 h. The reaction was then quenched with aq. Na₂S₂O₅, extracted with Et₂O (2×50 mL). The combined organic layer was washed with aq. NaHCO₃, brine, dried over Na₂SO₄, concentrated in vacuo to give 2.92 g of crude product which was proceeded to the next step without purification.

To a stirred solution of the above-described crude formate (2.92 g) in 5 mL of THF was added 3N aq. NaOH (3.9 mL, 11.7 mmol). The reaction mixture was then heated at 70 °C for 4 h. The reaction mixture was then partitioned between EtOAc and water. The organic layer was then washed with brine, dried over Na₂SO₄, concentrated in vacuo to give 2.50 g (93% over two steps) of the title compound.

### Example 299B

### Triisopropyl (3-(hydroxymethyl)-benzodioxan-6-yl)sulfide

The title compound was prepared by the procedures described in Example 281 A, substituting 5-bromo-*N*-methyl indole with the bromide from Example 299A.

### Example 299C

### (3-Hydroxymethyl)-benzodioxan-6-yl)[2-nitro-4-(E-((3-(pyrrolidin-2-on-1-yl)prop-1-ylamino)carbonyl)ethenyl)phenyl]sulfide

The title compound was prepared by the procedures described in Example 297, substituting the mixture of thiolsilyl ethers from Example 296A with the compound of Example 299B, giving a white solid. ¹H NMR (CDCl₃, 300 MHz) δ 1.74 (br m, 2H), 2.08 (t, *J* = 7.5 Hz, 2H), 2.44 (t, *J* = 7.5 Hz, 2H), 3.25-3.53 (m, 6H), 3.88 (dd, *J* = 4.8, 16.8 Hz, 1H), 3.97 (dd, *J* = 4.8, 16.8 Hz, 1H), 4.21 (dd, *J* = 3.1, 12.9 Hz, 1H), 4.26-4.36 (m, 1H), 4.40 (dd, *J* = 2.4, 12.9 Hz, 1H), 6.49 (d, *J* = 15.3 Hz, 1H), 6.88 (d, *J* = 8.7 Hz, 1H), 7.00 (d, *J* = 8.7 Hz, 1H), 7.07 (dd, *J* = 2.4, 8.7 Hz, 1H), 7.14 (d, *J* = 2.4 Hz, 1H), 7.20 (br s, 1H), 7.46 (dd, *J* = 0.9, 8.7 Hz, 1H), 7.54 (d, *J* = 15.3 Hz, 1H), 8.36 (s, 1H). MS (ESI⁺) (M+H)⁺ at *m*/*z* 514. Anal. Calcd for C₂₅H₂₇N₃O₇S · 0.82 H₂O: C, 56.83; H, 5.46; N, 7.95. Found: C, 56.84; H, 5.18; N, 7.74.

### Example 300

### (Benzodioxan-6-yl)[2-chloro-4-(E-((3-carboxypiperidin-1-yl)carbonyl)ethenyl)phenyl]sulfide

The title compound was prepared by the procedures described in Example 263, substituting 4-fluoro-3-trifluoromethylbenzaldehyde with 3-chloro-4-fluorobenzaldehyde, giving a white solid. ¹H NMR (CDCl₃, 300 MHz) δ 1.64-1.88 (br m, 2H), 1.95-2.09 (br m, 2H), 2.57-2.73 (m, 1H), 2.90-3.17 (m, 1H), 3.17-3.50 (m, 1H), 3.90-4.19 (m, 1H), 4.25-4.36 (m, 4H), 4.39-4.66 (m, 1H), 6.75 (d, *J* = 8.4 Hz, 1H), 6.84 (d, *J* = 15.3 Hz, 1H), 6.93 (d, *J* = 8.7 Hz, 1H), 7.03 (dd, *J* = 2.4, 8.7 Hz, 1H), 7.08 (d, *J* = 2.4 Hz, 1H), 7.18 (d, *J* = 8.4 Hz, 1H), 7.51 (s, 1H), 7.54 (d, *J* = 15.3 Hz, 1H). MS (ESI⁺) (M+H)⁺ at m/z 460, 462.

### Example 301

### (2-(and 3-)(Aminomethyl)-benzodioxan-6-yl)[2-trifluoromethy]-4-(E-((3-(pyrrolidin-2-on-1-yl)prop-1-ylamino)carbonyl)ethenyl)phenyl]sulfide

### Example 301A

### (2-(and 3-)(Mesyloxymethyl)-benzodioxan-6-yl)[2-trifluoromethyl-4-(E-((3-(pyrrolidin-2-on-1-yl)prop-1-ylamino)carbonyl)ethenyl)phenyl]sulfide

To a stirred solution of alcohol from Example 298 (200 mg, 0.37 mmol)) in 2 mL of methylene chloride with Et₃N(104 mL, 0.74 mmol)) was added methanesulfonyl chloride (35 mL, 0.56 mmol) dropwise. The mixture was then stirred at ambient temperature for one hour. The reaction mixture was then poured into 3N HCl, extracted with EtOAc (2×10 mL). The combined organic layer was washed with aq. NaHCO₃, brine, dried over Na₂SO₄, concentrated in vacuo to give 275 mg of crude product which was proceeded to the next step without purification.

### Example 301B

### (2-(and 3-)(Azidomethyl)-benzodioxan-6-yl)[2-trifluoromethyl-4-(E-((3-(pyrrolidin-2-on-1-yl)prop-1-ylamino)carbonyl)ethenyl)phenyl]sulfide

To a stirred solution suspension of NaN₃ (44 mg, 0.68 mmol) in 1 mL of DMSO was added mesylate (275 mg) in 0.5 mL of DMSO solution. The reaction mixture was then heated at 70 °C for 2 h, then cooled down to room temperature, water was added, extracted with EtOAc (2×10 mL). The combined organic layer was washed with water, brine, dried over Na₂SO₄, concentrated in vacuo. The residue was purified on a SiO₂ flash column chromatography eluting with 5-10% MeOH/EtOAc to give 35 (17%, two steps) mg of the title compound as light brown oil.

### Example 301C

### (2-(and 3-)(Aminomethyl)-benzodioxan-6-yl)[2-trifluoromethyl-4-(E-((3-(pyrrolidin-2-on-1-yl)pron-1-ylamino)carbonyl)ethenyl)phenyl]sulfide

To a stirred solution of azide (230 mg, 0.41 mmol) in 1 mL of THF was added PPh₃ (118 mg, 0.45 mmol), followed by one drop of water. The mixture was then stirred at room temperature for one hour. The volatile solvent was then removed in vacuo and the crude product was purified using Gilson Preparative HPLC as described in Example 38B to give 25 mg (11%) of the title compound. Light brown oil; ¹H NMR (CDCl₃, 300 MHz, 3:2 mixture of diastereomers) δ 1.74 (br m, 2H), 1.96-2.16 (m, 2H), 2.35-2.50 (m, 2H), 3.23-3.47 (m, 6H), 3.92-4.63 (m, 5H), 6.41-6.55 (m, 1H), 6.83-7.10 (m, 3H), 7.36-7.58 (m, 3H), 7.67-7.67 (m, 2H). MS (ESI⁺) (M+H)⁺ at *m*/*z* 536. Anal. Calcd for C₂₆H₂₈F₃N₃O₄S · 0 H2O: C, 58.31; H, 5.27; N, 7.85. Found: C, 58.34; H, 5.48; N, 7.78.

### Example 302

### (2-Isopropylphenyl)[2-nitro-4-(E-((3-(methylaminocarbonyl)morpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.6 Hz, 6H); 2.61 (d, J = 4.8 Hz, 3H); 3.14-4.62 (br m, 7H); 3.30-3.40 (m, 1H); 6.63 (d, J = 8.8 Hz, 1H); 7.32-7.62 (m, 6H); 7.80-7.97 (m, 2H); 8.66(d, J = 1.5 Hz, 1H). MS (APCI) (M+H)⁺ at m/z 470. Anal calcd for C₂₄H₂₇N₃S₁O₅^{·}0.8H₂O: C, 59.58; H, 5.96; N, 8.68. Found: C, 59.57; H, 5.94; N, 8.72.

### Example 303

### (2-Isopropylphenyl)[2-nitro-4-(E-((3-(hydroxymethyl)molpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 2.70-3.51 (br m, 5H); 3.30-3.40 (m, 1H); 3.83-3.93 (m, 1H); 4.03-4.47 (br m, 2H); 4.74-4.82 (m, 1H); 6.64 (d, J = 8.5 Hz, 1H); 7.30-7.62 (m, 6H); 7.86-7.94 (m, 1H); 8.59-8.65 (m, 1H). MS (APCI) (M+H)⁺ at m/z 443. Anal calcd for C₂₃H₂₆N₂S₁O₅: C, 62.43; H, 5.92; N, 6.33. Found: C, 62.12; H, 6.20; N, 6.06.

### Example 304

### (2-Isopropylphenyl)[2-nitro-4-(E-((3-(acetoxymethyl)morpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.1 Hz, 6H); 2.04 (s, 3H); 3.30-3.40 (m, 1H); 2.58-4.41 (br m, 9H); 6.64 (d, J = 8.5 Hz, 1H); 7.30-7.62 (m, 6H); 7.90 (dd, J = 8.5, 1.8 Hz, 1H); 8.59-8.65 (m, 1H). MS (APCI) (M+H)⁺ at m/z 485. Anal calcd for C₂₅H₂₈N₂S₁O₆: C, 61.97; H, 5.82; N, 5.78. Found: C, 61.85; H. 5.84; N, 5.68.

### Example 305

### (2-Isopropylphenyl)[2-nitro-4-(E-((3-(aminomethyl)morpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 2.61 (d, J = 5.5 Hz, 2H); 2.49-3.60 (br m, 5H); 3.82-3.93 (m, 1H); 4.13-4.45 (m, 2H); 6.64 (d, J = 8.5 Hz, 1H); 7.32-7.62 (m, 6H); 7.88-7.95 (m, 1H); 8.59-8.67(m, 1H). MS (APCI) (M+H)⁺ at m/z 442. Anal calcd for C₂₃H₂₇N₃S₁O₄·0.4H₂O: C, 61.55; H, 6.25; N, 9.36. Found: C, 61.60; H, 6.25; N, 9.00.

### Example 306

### (2-Isopropylphenyl)[2-nitro-4-(E-((3-(acetamidomethyl)morpholin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.82 (s, 3H); 2.70-3.50 (br m, 7H); 3.85-3.94 (m, 1H); 4.13-4.40 (m, 2H); 6.64 (d, J = 8.5 Hz, 1H); 7.32-7.62 (m, 6H); 7.88-8.06 (m, 1H); 8.59-8.67(m, 1H). MS (APCI) (M+H)⁺ at m/z 484. Anal calcd for C₂₅H₂₉N₃S₁O₅·0.27H₂O: C, 61.47; H, 6.10; N, 8.60. Found: C, 61.50; H, 6.34; N, 8.53.

### Example 307

### (Benzodioxan-6-yl)[2-chloro-4-(E-((3-(pyrrolidin-2-on-1-yl)prop-1-ylamino)carbonyl)ethenyl)phenyl]sulfide

The title compound was prepared by the procedures described in Example 300 substituting ethyl isonipecotate with N-(3'-aminopropyl)-2-pyrrolidinone. ¹H NMR (CDCl₃, 300 MHz) δ 1.75 (br s, 2H), 2.02-2.34 (m, 2H), 2.40-2.50 (m, 2H), 3.30-3.50 (m, 6H), 4.28-4.33 (m, 4H), 6.40 (br, 1H), 6.75 (d, J = 8.0 Hz, 1H), 6.93 (d, J = 8.5 Hz, 1H), 7.02 (dd, J = 2.0, 8.0 Hz, 1H), 7.08 (d, J = 2.0 Hz, 1H), 7.18 (d, J = 8.5 Hz, 1H), 7.45 (m, 1H), 7.50 (s, 1H). MS (ESI) m/z 473 (M+H)⁺. Anal. calcd. for C₂₄H₂₅ClN₂O₄S•0.5 H2O: C, 59.81; H, 5.44; N, 5.81. Found: C, 59.76; H, 5.80; N, 5.43.

### Example 308

### (Benzodioxan-6-yl)[2-chloro-4-(E-((3-carboethoxypiperidin-1-yl)carbonyl)ethenyl)phenyl]sulfide

The title compound was prepared by the procedures described in Example 300 substituting ethyl isonipecotate with ethyl nipecotate. ¹H NMR (CDCl₃, 300 MHz) δ1.25 (t, J = 7.0 Hz, 3H), 1.60-1.90 (br, 2H), 2.10 (br, 1H), 2.52 (br, 1H), 3.00-3.50 (br, 2H), 3.80 (br, 1H), 4.10-4.20 (m, 4H), 4.28-4.35 (m, 4H), 6.74 (d, J = 8.0 Hz, 1H), 6.92 (d, J = 8.0 Hz, 1H), 7.02 (dd, J = 2.0, 8.0 Hz, 1H), 7.08 (d, J = 2.0 Hz, 1H), 7.18 (m, 1H), 7.50-7.03 (m, 3H). MS (ESI) m/z 488 (M+H)⁺. Anal. calcd. for C₂₅H₂₆ClNO₅SNa·0.5 H₂O: C, 60.42; H, 5.48; N, 2.82. Found: C, 60.61; H, 5.51; N, 2.42.

### Example 309

### (Benzodioxan-6-yl)[2-chloro-4-(E-((2-carboethoxypiperidin-1-yl)carbonyl)ethenyl)phenyl]sulfide

The title compound was prepared by the same procedure described in Example 300 substituting ethyl isonipecotate with ethyl pipecolinate. ¹H NMR (CDCl₃, 300 MHz) δ 1.30 (t, J = 7.0 Hz, 3H), 1.30-1.50 (br,3H), 1.55-1.85 (br, 3H), 2.30 (m, 1H), 4.00 (m, 1H), 4.20 (m, 2H), 4.30 (m, 4H), 5.44 (br, 1H), 6.85 (d, J = 8.0 Hz, 1H), 6.90 (d, J = 8.0 Hz, 1H), 7.00 (dd, J = 2.0, 8.0 Hz, 1H), 7.07 (d, J = 2.0 Hz, 1H), 7.10-7.20 (m, 2H), 7.22 (m, 1H), 7.50 (s, 1H). MS (ESI) m/z 488 (M+H)⁺. Anal. calcd. for C₂₅H₂₆ClNO₅S: C, 61.53; H, 5.37; N, 2.87. Found: C, 61.86; H, 5.63; N, 2.56.

### Example 310

### (2-Methoxyphenyl)-[2,3-dichloro-4(E-[(morpholin-1-yl)carbonyl]ethenyl)phenyl]sulfide

### Example 310A

### 2,3-Dichloro-4-trifluoromethanesulfonyloxy-benzaldehyde

2,3-Dichloro-4-hydroxy-benzaldehyde (9.10 g. , J. Med. Chem. 19 (4), 534, 1994) was dissolved in 45 ml. pyridine at room temperature. The solution was placed in an ice bath and immediately, 15.63 g. of trifluoromethanesulfonic anhydride was added slowly. [Note: If the pyridine solution is cooled to zero before addition of triflic anhydride the aldehyde crystallizes out and the mixture cannot be stirred.] After the addition is complete the dark mixture was stirred for 1 hour at room temperature. It was then poured into a stirred mixture of ice water, 100 ml. of concentrated HCl and ether. [Note: Not everything is soluble in this mixture] The ether layer was separated, dried over sodium sulfate, and the solvent removed. Warm heptane was added to this residue, and any insoluble material was filtered. The solution was concentrated to give 8.74 g. (57% yield) of product as an orange oil which solidified in the refrigerator.

### Example 310B

### 2,3-Dichloro-4-(2-methoxyphenylthio)-benzaldehyde

2,3-Dichloro-4-trifluoromethanesufonyloxy-benzaldehyde (2.50 g.) was dissolved in 6 ml. acetonitile. 2-Methoxybenzenethiol (2.55 g. of 70% pure material, 50% excess) was added. With cooling 2.50 g. diisopropylethylamine was added slowly. The solution was removed from the ice bath, whereon a solid formed.. The solution was warmed in a 50C waterbath for 5 minutes. More acetonitrile (5 ml.) was added and the mixture was cooled in ice, and then filtered to get 2.047 g. of product, m.p. 137-139C.

### Example 310C

### 2,3-Dichloro-4-(2-methoxyphenythio)-cinnamic acid

A mixture of 2,3-dichloro-4-(2-methoxyphenylthio)-benzaldehyde (2.03 g.), 1.44 g. malonic acid, 5 ml. pyridine, and 0.100 g piperidine was heated to 115 degrees for 1.5 hours. The mixture was cooled, and ice and HCl were added. The resulting solid was filtered, washed with water and dissolved in tetrahydrofuran. This solution was dried over sodium sulfate, the solvent removed and ether added to give 1.733 g of product, m.p. 187-188C.

### Example 310D

### (2-Methoxyphenyl)-[2,3-dichloro-4(E-[(morpholin-1-yl)carbonyl]ethenyl)phenyl]sulfide

The title compound was prepared according to the procedure of Example 1, substituting the cinnamic acid of Example 310C, giving a white solid, m.p. 161-162C. ¹H-NMR (CDCl₃ 300 MHz) δ 3.83 (s, 3H), 6.55 (d, J = 9Hz, 1H), 6.70 (broad d, J = 15 Hz, 1H), 6.99-7.05 (m, 2H), 7.26 (d, J = 9 Hz, 1H), 7.43-7.50 (m, 2H), 8.07 (broad d, J = 15 Hz, 1H) Anal. Calcd. for C₂₀H₁₉Cl₂NO₃S: C, 56.61; H, 4.51; N, 3.30. Found: C, 56.75; H, 4.57; N, 2.61.

### Example 311

### (2-Methoxyphenyl)-[2,3-dimethyl-4(E-[(morpholin-1-yl)carbonyl]ethenyl)phenyl]sulfide

The title compound was prepared according to the procedures of Example 310. ¹H-NMR (CDCl₃ 300 MHz) δ 2.39 (s, 3H), 2.42 (s, 3H), 3.60-3.80 (m, 8H), 3.90 (s, 3H), 6.69 (d, J = 15 Hz, 1H), 6.82-6.94 (m, 3H), 7.05 (d, J = 9Hz, 1H), 7.20-7.30 (m, 2H), 8.06 (d, J = 15 Hz, 1H). Anal. Calcd. for C₂₂H₂₅NO₃S: C, 68.91; H, 6.57; N, 3.65. Found: C, 68.75; H, 6.67; N, 3.24.

### Example 312

### (2-Isopropylphenyl)[2-nitro-4-(E-((indol-5-ylamino)carbonyl)ethenyl)phenyl]sulfide

The title compound was prepared according to the procedures of Example 1. ¹H NMR (DMSO-d₆, 300 MHz) δ 11.04 (s, 1H), 10.10 (s, 1H), 8.52 (d, 1H, J = 1.5 Hz), 8.02 (s, 1H), 7.81 (dd, 1H, J = 1.8, 8.5 Hz), 7.53-6.63 (m, 4H7.39 (m, 1H), 7.25-7.35 (m, 3H), 6.94 (d, 1H, J = 15.8 Hz), 7.72 (d, 1H, J = 8.5 Hz), 6.40 (m, 1H), 3.33 (m, 1H), 1.16 (d, 6H, J = 6.6 Hz). MS (ESI) *m*/*z* 458, 480, 915. Anal. Calcd for C₂₆H₂₃N₃O₃S · 0.22 H₂O: C, 67.67; H, 5.12; N, 9.10. Found: C, 67.68; H, 5.19; N, 9.08.

### Example 313

### (Benzodioxan-6-yl)[2-chloro-4-(E-((3-carboxypiperidin-1-yl)carbonyl)ethenyl)phenyl]sulfide

The title compound was prepared by hydrolysis of the compound of Example 308 under basic condition (aq. NaOH/EtOH). ¹H NMR (DMSO-d₆, 300 MHz) δ 1.10-1.40 (m, 2H), 1.60 (m, 1H), 1.76-1.96 (m, 3H), 2.88 (m, 1H), 3.98 (m, 1H), 3.98 (m, 1H), 4.30 (m, 4H), 6.72 (d, J = 8.0 Hz, 1H), 7.02 (m, 3H), 7.30 (m, 2H), 7.48 (m, 1H), 7.92 (m, 1H). MS (ESI) m/z 458 (M+H)⁺. Anal. calcd. for C₂₃H₂₁ClNO₅SNa: C, 55.76; H, 4.58; N, 2.83. Found: C, 55.76; H, 4.78; N, 2.63.

### Example 314

### (Benzodioxan-6-yl)[2-chloro-4-(E-((3-(tetrazol-5-yl)piperidin-1-yl)carbonyl)ethenyl)phenyl]sulfide

The title compound was prepared by the procedures described in Example 282, producing a white solid. ¹H NMR (CDCl₃, 300 MHz) δ 1.66-1.80 (m, 2H), 2. 10-2.30 (m, 2H), 2.64 (m, 1H), 3.55 (m, 2H), 3.98 (m, 1H), 4.25 (m, 1H), 4.30-4.36 (m, 4H), 6.72 (dd, J = 3.0,12.0 Hz, 2H), 6.93 (d, J = 8.0 Hz, 1H), 7.03 (dd, d=2.0, 8.0 Hz, 1H), 7.09 (d, J = 2.0 Hz, 1H), 7.20 (d, J = 8.5 Hz, 1H), 7.52 (s, 1H), 7.70 (d, J = 15.0 Hz, 1H). MS (ESI) m/z 484 (M+H)⁺. Anal. calcd. for C₂₃H₂₂ClN₅O₃S•0.38 H₂O: C, 56.28; H, 4.67; N, 14.27. Found: C, 56.46; H, 4.58; N, 13.94.

### Example 315

### (Benzodioxan-6-yl)[2-chloro-4-(E-((4-(tert-butoxycarbonyl)piperazin-1-yl) carbonyl]ethenyl)phenyl]sulfide

The title compound was prepared by the procedures described in Example 300 substituting ethyl isonipecotate with 1-Boc-piperazine. ¹H NMR (CDCl₃, 300 MHz) δ 1.50 (s, 9H), 3.50 (br, s 4H), 3.70 (br, 4H), 4.28-4.35 (m, 4H), 6.74 (d, J = 8.0 Hz, 1H), 6.82 (m, 1H), 6.92 (d, J = 8.0 Hz, 1H), 7.02 (dd, J = 2.0, 8.0 Hz, 1H), 7.17 (d, J = 2.0 Hz, 1H), 7.28 (d, J = 8.0 Hz, 1H), 7.50 (s, 2H), 7.58 (m, 1H). MS (ESI) m/z 517 (M+H)⁺. Anal. calcd. for C₂₆H₂₉ClN₂O₅S•0.1 H₂O: C, 60.19; H, 5.67; N, 5.40. Found: C, 60.20; H, 5.97; N, 5.11.

### Example 316

### (Benzodioxan-6-yl)[2-chloro-4-(E-((2-carboxypiperidin-1-yl)carbonyl)ethenyl)phenyl]sulfide

The title compound was prepared by hydrolysis of the compound of Example 309 under basic conditions (aq. NaOH/EtOH). ¹H NMR (DMSO-d₆, 300 MHz) δ 1.10-1.40 (m, 3H), 1.45-1.60 (m, 2H), 2.25-2.45 (m, 2H), 2.55-2.80 (m, 1H), 4.30 (m, 4H), 4.50 (m, 1H), 6.70 (d, J = 8.0 Hz, 1H), 7.00 (m, 3H),7.10 (m, 1H), 7.25 (d, J = 16.0 Hz, 1H), 7.48 (d, J = 8.0 15.5 Hz, 1H), 7.90 (d, J = 15.5 Hz, 1H). MS (ESI) m/z 458 (M+H)⁺. Anal. calcd. for C₂₃H₂₁ClNO₅SNa•1.3 H₂O: C, 54.69; H, 4.73; N, 2.45. Found: C, 54.67; H, 4.71; N, 2.77.

### Example 317

### (Benzodioxan-6-yl)[2-chloro-4-(E-((3-(tetrazol-5-yl)morpholin-1-yl) carbonyl)ethenyl)phenyl]sulfide

The title compound was prepared by the procedures described in Example 262. ¹H NMR (CDCl₃, 300 MHz) δ 1.50-1.70 (m, 2H), 3.15 (br, 1H), 3.70-3.90 (m, 2H), 4.25-4.35 (m, 4H), 4.55 (m, 1H), 5.04 (br, 1H), 6.72 (d, J = 8.0 Hz, 1H), 6.93 (d, J = 8.0 Hz, 1H), 7.03 (dd, J = 2.0, 8.0 Hz, 1H), 7.07 (d, J = 2.0 Hz, 1H), 7.20-7.30 (m, 2H), 7.50 (m, 1H), 7.65 (m, 1H). MS (ESI) m/z 486 (M+H)⁺. Anal. calcd. for C₂₂H₂₀ClN₅O₄S •H₂O: C, 52.43; H, 4.40; N, 13.90. Found: C, 52.34; H, 4.35; 13.62.

### Example 318

### (Benzodioxan-6-yl)[2-chloro-4-(E-((4-(methylaminocarbonyl)piperazin-1-yl) carbonyl)ethenyl)phenyl]sulfide

The title compound was prepared by deprotection of the of Example 315 compound using anhydrous TFA in dichloromethane, followed by treatment with methyl isocyanate. ¹H NMR (CDCl₃, 300 MHz) δ 2.88 (s, 3H), 3.50 (br, 4H), 3.72 (br, 4H), 4.30 (m, 4H), 6.74 (d, J = 8.0 Hz, 1H), 6.82 (d, J = 15.0 Hz, 1H), 6.92 (d, J = 8.0 Hz, 1H), 7.03 (dd, J = 2.0, 8.0 Hz, 1H), 7.08 (d, J = 2.0 Hz, 1H), 7.20 (d, J = 8.0 Hz, 1H), 7.50 (s, 1H), 7.60 (m, 1H). MS (ESI) m/z 474 (M+H)⁺. Anal. calcd. for C₂₃H₂₄ClN₃O₄S: C, 57.63; H, 5.17; N, 8.77. Found: C, 57.53; H, 5.02; N, 8.58.

### Example 319

### (2-Methoxyphenyl)-[2,3-dichloro-4(E-[(4-carboxypiperidin-1-yl)carbonyl]ethenyl)phenyl]sulfide

The title compound was prepared according to the procedures of Example 310. ¹H-NMR (CDCl₃ 300 MHz) δ 1.66-1.83 (m, 2H), 1.95-2.09 (m, 2H), 2.57-2.69 (m, 1H), 2.94-3.08 (m, 1), 3.15-3.31 (m, 1H), 3.72 (s, 3H), 3.90-4.05 (m, 1H), 4.41-4.55 (m, 1H), 6.55 (d, J = 9Hz, 1H), 6.73 (d, J = 15Hz, 1H), 7.00-7.05 (m, 2H), 7.27 (d, J = 8Hz, 1H), 7.44-7.50 (m, 2H), 7.92 (d, J = 15Hz, 1H). Anal. Calcd. for C₂₂H₂₁Cl₂NO₄S: C, 56.66; H, 4.54; N, 3.00. Found: C, 56,89; H, 4.84; N, 2.64.

### Example 320

### (Benzodioxan-6-yl)[2-chloro-4-(E-((4-(tetrazol-5-yl)piperidin-1-yl)carbonyl)ethenyl)phenyl]sulfide

The title compound was prepared by the procedures described in Example 314 substituting 3-(tetrazol-5-yl)piperidine with 4-(tetrazol-5-yl)piperidine. The crude reaction product was purified by reversed-phase HPLC. ¹H NMR (DMSO-d₆, 300 MHz) δ 1.22 (m, 1H), 1.55-1.75 (m, 2H), 2.06 (m, 1H), 2.45 (m, 1H), 4.22 (m, 4H), 4.30 (m, 4H), 6.70 (m, 1H), 7.00 (dd, J = 2.0, 8.0 Hz, 2H), 7.25-7.40 (m, 4H), 7.50 (m, 1H). MS (ESI) m/z 484 (M+H)⁺.

### Example 321

### (2-Methoxyphenyl)-[3-chloro-4(E-[(morpholin-1-yl)carbonyl]ethenyl)phenyl]sulfide

The title compound was prepared according to the procedures of Example I, giving a white solid, m.p. 124-125C. ¹H-NMR (CDCl₃ 300 MHz) δ 3.60-3.80 (m, 8H), 3.85 (s, 3H), 6.80 (d, J = 15 Hz, 1H), 6.95-7.01 (m, 2H), 7.05 (dd, J = 9Hz, 2 Hz, 1H), 7.15 (d, J = 2Hz, 1H), 7.35-7.48 (m, 3H), 7.75 (d, J = 15 Hz, 1H). Anal. Calcd. for C₂₀H₂₀ClNO₃S: C, 61.61; H, 5.17; N, 3.59. Found: C, 61.43; H, 5.30; N, 3.73.

### Example 322

### (2-Isopropylphenyl)[2-nitro-4-(E-((4-oxopiperidin-1-yl)carbonyl)ethenyl)phenyl]sulfide

The title compound was prepared according to the procedures of Example 1. ¹H NMR (CDCl₃, 300 MHz) δ 8.45 (s, 1H), 7.50-7.57 (m, 3H), 7.42(br d, 1H, J = 8.1 Hz), 7.30 (m, 1H), 7.02 (br, 1H), 6.72 (d, 1 H, J = 8.4 Hz), 4.01 (br s, 4H), 3.44 (quintet, 1H, J = 6.8 Hz), 2.56 (br m, 4H), 1.18 (d, 6H, J = 7.1 Hz). MS (ESI) *m*/*z* 425, 457. Anal. Calcd for C₂₃H₂₄N₂O₄S : C, 65.07; H, 5.70; N, 6.60. Found: C, 64.92; H, 5.67; N, 6.62.

### Example 323

### (Benzodioxan-6-yl)[2-trifluoromethyl-4-(E-((3-R-carboethoxypiperidin-1-yl)carbonyl)ethenyl)phenyl]sulfide

The title compound was prepared by the procedures described in Example 248, substituting ethyl (±)nipecotate with ethyl nipecotate tartrate, giving a white solid. ¹H NMR (CDCl₃, 300 MHz) δ 1.26 (t, *J* = 7.4 Hz, 3H), 1.46-1.67 (m, 1H), 1.67-1.98 (m, 2H), 1.98-2.23 (m, 1H), 2.46-2.63 (m, 1H), 3.10-3.42 (m, 1H), 3.53-4.13 (m, 2H), 4.16 (q, *J* = 7.4 Hz, 2H), 4.25-4.40 (m, 4H), 4.60-4.88 (m, 1H), 6.91 (d, *J* = 8.4 Hz, 1H), 6.93 (d, *J* = 15.3 Hz, 1H), 6.97-7.05 (m, 2H), 7.07 (d, *J* = 2.7 Hz, 1H), 7.42 (d, *J* = 8.4 Hz, 1H), 7.59 (d, *J* = 15.3 Hz, 1H), 7.77 (s, 1H). MS (ESI⁺) (M+H)⁺ at *m*/*z* 522.

### Example 324

### (Benzodioxan-6-yl)[2-trifluoromethyl-4-(E-((3-R-carboxypiperidin-1-yl)carbonyl)ethenyl)phenyl]sulfide

The title compound was prepared by the procedures described in Example 251, substituting the ethyl ester from Example 248 with ethyl ester from Example 323, giving a white solid. ¹H NMR (CDCl₃, 300 MHz) δ 1.48-1.71 (m, 1H), 1.71-2.01 (m, 2H), 2.01-2.20 (m, 1H), 2.53-2.70 (m, 1H), 3.18-3.54 (m, 1H), 3.86-4.20 (m, 2H), 4.20-4.33 (m, 4H), 4.45-4.75 (m, 1H), 6.90 (d,*J*= 8.7 Hz, 1H), 6.95-7.04 (m, 3H), 7.06 (d, *J* = 2.4 Hz, 1H), 7.35-7.45 (br m, 1H), 7.60 (d, *J* = 15.3 Hz, 1H), 7.75 (s, 1H). MS (ESI⁺) (M+H)⁺ at *m*/*z* 494.

### Example 325

### (Benzodioxan-6-yl)[2,3-dichloro-4-(E-((3-(pyrrolidin-2-on-1-yl)prop-1-ylamino)carbonyl)ethenyl)phenyl]sulfide

The title compound was prepared by the procedures described in Example 240, substituting 4-fluoro-3-trifluoromethylbenzaldehyde with 2,3-dichloro-4-trifluoromethanesulfoxybenzaldehyde, giving a white solid. ¹H NMR (CDCl₃, 300 MHz) δ 1.71-1.82 (m, 2H), 2.08 (p, *J =* 7.5 Hz, 2H), 2.46 (t, *J*= 7.5 Hz, 2H), 3.2603.50 (m, 6H), 4.23-4.36 (m, 4H), 6.36 (t, *J* = 15.6 Hz, 1H), 6.60 (d, *J*= 8.7 Hz, 1H), 6.44 (d, *J* = 8.7 Hz, 1H), 7.03 (dd, *J* = 2.4, 8.7 Hz, 1H), 7.09 (d, *J* = 2.4 Hz, 1H), 7.31 (d, *J* = 8.7 Hz, 1H), 7.94 (d, *J* = 15.6 Hz, 1H). MS (ESI⁺) (M+H)⁺ at *m*/*z* 507, 509, 511. Anal. Calcd for C₂₄H₂₄Cl₂N₂O₄S · 1.87 H₂O: C, 53.27; H, 5.17; N, 5.18. Found: C, 53.30; H, 5.17; N, 4.83.

### Example 326

### (Benzodioxan-6-yl)[2,3-dichloro-4-(E-((4-acetylpiperazin-1-yl)carbonyl)ethenyl)phenyl]sulfide

The title compound was prepared by the procedures described in Example 325, substituting aminopropyl pyrrolidinone with 1-acetylpiperazine. white solid; ¹H NMR (CDCl₃, 300 MHz) δ 2.17 (s, 3H), 3.50-3.94 (m, 8H), 4.26-4.40 (m, 4H), 6.61 (d, *J =* 8.7 Hz, 1H), 6.71 (d, *J* = 15.6 Hz, 1H), 6.95 (d, *J* = 8.4 Hz, 1H), 7.04 (dd, *J* = 2.4, 8.4 Hz, 1H), 7.09 (d, *J* = 2.4 Hz, 1H), 7.30 (d, *J* = 8.7 Hz, 1H), 7.99 (d, *J* = 15.6 Hz, 1H). MS (ESI⁺) (M+Na)⁺ at *m*/*z* 515, 517, 519. Anal. Calcd for C₂₃H₂₂Cl₂N₂O₄S · 0.52 CH₂Cl₂: C, 52.55; H, 4.32; N, 5.21. Found: C, 52.63; H, 4.16; N, 4.82.

### Example 327

### (Benzodioxan-6-yl)[2,3-dichloro-4-(E-((3-carboethoxypiperidin-1-yl)carbonyl)ethenyl)phenyl]sulfide

The title compound was prepared by the procedures described in Example 325, substituting aminopropyl pyrrolidinone with ethyl nipecotate, giving a white solid. ¹H NMR (CDCl₃, 300 MHz) δ 1.26 (t, *J* = 7.0 Hz, 3H), 1.66-1.96 (m, 2H), 1.96-2.21 (m, 1H), 2.44-2.60 (m, 1H), 2.85-3.40 (m, 2H), 3.50-3.70 (m, 1H), 3.80-4.10 (m, 2H), 4.15 (q, *J*= 7.0 Hz, 2H), 4.26-4.40 (m, 4H), 6.66 (d, *J* = 8.7 Hz, 1H), 6.74 (d, *J* = 15.3 Hz, 1H), 6.95 (d, *J =* 8.4 Hz, 1H), 7.03 *(dd, J =* 2.4, 8.4 Hz, 1H), 7.09 (d, *J* = 2.4 Hz, 1H), 7.25-7.38 (m, 1H), 7.93 (d, *J =* 15.3 Hz, 1H). MS (ESI⁺) (M+Na)⁺ at *m*/*z* 544, 546, 548.

### Example 328

### (Benzodioxan-6-yl)[2,3-dichloro-4-(E-((4-carboethoxypiperidin-1-yl)carbonyl)ethenyl)phenyl]sulfide

The title compound was prepared by the procedures described in Example 325, substituting aminopropyl pyrrolidinone with ethyl isonipecotate, giving a white solid. ¹H NMR (CDCl₃, 300 MHz) δ 1.26 (t, *J* = 7.2 Hz, 3H), 1.69 (td, *J* = 3.9, 10.8 Hz, 1H), 1.74 (td, *J* = 3.9, 10.8 Hz, 1H), 1.82-2.05 (m, 2H), 2.50-2.63 (m, 1H), 2.84-3.31 (m, 2H), 3.81-4.06 (m, 1H), 4.15 (q, *J* = 7.2 Hz, 2H), 4.24-4.34 (m, 4H), 4.34-4.59 (m, 1H), 6.61 (d, *J* = 8.7 Hz, 1H), 6.74 (d, *J* = 15.6 Hz, 1H), 6.94 (d, *J* = 8.7 Hz, 1H), 7.03 (dd, *J* = 2.7, 8.7 Hz, 1H), 7.08 (d, *J* = 2.7 Hz, 1H), 7.29 (d, *J* = 8.7 Hz, 1H), 7.90 (d, *J* = 15.6 Hz, 1H). MS (ESI⁺) (M+H)⁺ at *m*/*z* 522, 524, 526. Anal. Calcd for C₂₅H₂₅Cl₂NO₅S: C, 57.48; H, 4.82; N, 2.68. Found: C, 57.82; H, 4.96; N, 2.28.

### Example 329

### (Benzodioxan-6-yl)[2,3-dichloro-4-(E-((3-carboxypiperidin-1-yl)carbonyl)ethenyl)phenyl]sulfide

The title compound was prepared by the procedures described in Example 155, substituting the ethyl ester from Example 137 with the ethyl ester from Example 327, and KOH with NaOH, providing a white solid. ¹H NMR (CDCl₃, 300 MHz) δ 1.70-2.0 (m, 2H), 2.0-2.20 (m, 1H), 2.54-2.68 (m, 1H), 3.03-3.46 (m, 2H), 3.80-4.11 (m, 2H), 4.27-4.40 (m, 4H), 4.50-4.70 (m, 1H), 6.60 (d, *J* = 8.9 Hz, 1H), 6.79 (d, *J* = 15.3 Hz, 1H), 6.94 (d, *J* = 8.5 Hz, 1H), 7.03 (dd, *J* = 2.1, 8.5 Hz, 1H), 7.08 (d, *J* = 2.1 Hz, 1H), 7.30 (d, *J* = 8.9 Hz, 1H), 7.93 (d, *J* = 15.3 Hz, 1H). MS (ESI⁺) (M-2H)⁻ at *m*/*z* 492,494,496. Anal. Calcd for C₂₃H₂₁Cl₂NO₅S · 0.73 H₂O: C, 54.43; H, 4.46; N, 2.76. Found: C, 54.43; H, 4.39; N, 2.49.

### Example 330

### (Benzodioxan-6-yl)[2,3-dichloro-4-(E-((4-carboxypiperidin-1-yl)carbonyl)ethenyl)phenyl]sulfide

The title compound was prepared by the procedures described in Example 155, substituting the ethyl ester from Example 137 with the ethyl ester from Example 328, and KOH with NaOH, to produce a white solid. ¹H NMR (d⁶-DMSO, 300 MHz) δ 1.33-1.55 (m, 2H), 1.62-1.78 (m, 2H), 1.93-2.07 (m, 1H), 2.90 (brt,*J*= 10.5 Hz, 1H), 3.16 (brt, *J* = 10.5 Hz, 1H), 3.96 (br d, *J* = 13.5 Hz, 1H), 4.09 (br d, *J* = 13.5 Hz, 1H), 4.26-4.42 (m, 4H), 6.60 (d, *J* = 9.0 Hz, 1H), 7.04-7.08 (m, 2H), 7.13 (d,*J* = 1.5 Hz, 1H), 7.22 (d, *J* = 15.3 Hz, 1H), 7.70 (d, *J* = 15.3 Hz, 1H), 7.86 (d, *J* = 9.0 Hz, 1H). MS (ESI⁺) (M+H)⁺ at *m*/*z* 516, 518, 520. Anal. Calcd for C₂₃H₂₀Cl₂N₁NaO₅S · 0.36 Et₂O: C, 54.06; H, 4.38; N, 2.58. Found: C, 53.99; H, 4.37; N, 2.22.

### Example 331

### (2-Isopropylphenyl)[2,3-dichloro-4-(E-((3-(1-pyrrolidin-2-onyl)propylamino)carbonyl)ethenyl)phenyl]sulfide

The title compound was prepared by the procedures described in Example 325, substituting 6-mercaptobenzodioxane with 2-isopropylbenzenethiol, to give a white solid. ¹H NMR (CDCl₃, 300 MHz) δ 1.19 (d, *J* = 7.2 Hz, 6H), 1.76 (p, *J* = 5.8 Hz, 2H), 2.08 (p, *J* = 7.65 Hz, 2H), 2.46 (t, *J* = 7.65 Hz, 2H), 3.32 (q, *J* = 5.8 Hz, 2H), 3.36-3.51 (m, 5H), 6.35 (d, *J* = 15.3 Hz, 1H), 6.40 (d, *J* = 8.7 Hz, 1H), 7.10 (brt, *J* = 7.5 Hz, 1H), 7.20-7.30 (m, 2H), 7.42-7.53 (m, 2H), 7.94 (d, *J* = 15.3 Hz, 1H). MS (ESI⁺) (M+H)⁺ at *m*/*z* 491, 493, 495. Anal. Calcd for C₂₅H₂₈Cl₂N₂O₂S · 0.7 CH₂Cl₂: C, 56.03; H, 5.38; N, 5.08. Found: C, 56.06; H, 5.22; N, 5.01.

### Example 332

### (2-Isopropylphenyl)[2,3-dichloro-4-(E-((4-acetylpiperazin-1-yl)carbonyl)ethenyl)phenyl]sulfide

The title compound was prepared by the procedures described in Example 326, substituting 6-mercaptobenzodioxane with 2-isopropylbenzenethiol, providing a white solid. ¹H NMR (CDCl₃, 300 MHz) δ 1.19 (d, *J* = 7.2 Hz, 6H), 2.17 (s, 3H), 3.46 (septet, *J* = 7.2 Hz, 1H), 3.50-3.90 (m, 8H), 6.41 (d, *J* = 8.7 Hz, 1H), 6.71 (d, *J* = 15.3 Hz, 1H), 7.21-7.35 (m, 2H), 7.44-7.57 (m, 3H), 7.99 (d, *J* = 15.3 Hz, 1H). MS (ESI⁺) (M+H)⁺ at *m*/*z* 477, 479, 481. Anal. Calcd for C₂₄H₂₆Cl₂N₂O₂S · 0.32 CH₂Cl₂: C, 57.89; H, 5.32; N, 5.55. Found: C, 57.85; H, 5.25; N, 5.74.

### Example 333

### (2-Isopropylphenyl)[2,3-dichloro-4-(E-((3-carboethoxypiperidin-1-yl)carbonyl)ethenyl)phenyl]sulfide

The title compound was prepared by the procedures described in Example 327, substituting 6-mercaptobenzodioxane with 2-isopropylbenzenethiol, giving a white solid. ¹H NMR (CDCl₃, 300 MHz) δ 1.20 (d, *J* = 7.2 Hz, 6H), 1.20-1.35 (m, 5H), 1.65-1.93 (m, 1H), 1.93-2.16 (m, 1H), 2.43-2.58 (m, 1H), 3.06-3.35 (m, 1H), 3.47 (septet, *J* = 7.2 Hz, 1H), 3.77-4.23 (m, 4H), 4.50-4.77 (m, 1H), 6.41 (d, *J* = 8.4 Hz, 1H), 6.80 (d, *J* = 15.3 Hz, 1H), 7.18-7.32 (m, 2H), 7.40-7.55 (m, 2H), 7.93 (d, *J* = 15.3 Hz, 1H). MS (ESI⁺) (M+H)⁺ at *m*/*z* 506, 508, 510.

### Example 334

### (2-Isopropylphenyl)[2,3-dichloro-4-(E-((4-carboethoxypiperidin-1-yl)carbonyl)ethenyl)phenyl]sulfide

The title compound was prepared by the procedures described in Example 328, substituting 6-mercaptobenzodioxane with 2-isopropylbenzenethiol, to give a white solid. ¹H NMR (CDCl₃, 300 MHz) δ 1.19 (d, *J* = 7.2 Hz, 6H), 1.26 (t, *J* = 7.05 Hz, 3H), 1.69 (td, *J* = 3.9, 10.8 Hz, 1H), 1.74 (td, *J* = 3.9, 10.8 Hz, 1H), 1.88-2.06 (m, 2H), 2.50-2.63 (m, 1H), 2.84-3.08 (m, 1H), 3.08-3.32 (m, 1H), 3.47 (septet, *J* = 7.2 Hz, 1H), 3.86-4.06 (m, 1H), 4.15 (q, *J* = 7.05 Hz, 2H), 4.37-4.61 (m, 1H), 6.40 (d, *J* = 8.7 Hz, 1H), 6.73 (d, *J* = 15.6 Hz, 1H), 7.22-7.35 (m, 2H), 7.44-7.57 (m, 3H), 7.92 (d, *J* = 15.6 Hz, 1H). MS (ESI⁺) (M+H)⁺ at *m*/*z* 506, 508, 510. Anal. Calcd for C₂₆H₂₉Cl₂NO₃S · 0.01 H₂O: C, 61.64; H, 5.77; N, 2.76. Found: C, 61.64; H, 5.90; N, 2.70.

### Example 335

### (2-Isopropylphenyl)[2,3-dichloro-4-(E-((3-carboxypiperidin-1-yl)carbonyl)ethenyl)phenyl]sulfide

The title compound was prepared by the procedures described in Example 329, substituting 6-mercaptobenzodioxane with 2-isopropylbenzenethiol, giving a white solid. ¹H NMR (CDCl₃, 300 MHz) δ 1.19 (d, *J* = 7.2 Hz, 1H), 1.43-1.67 (m, 1H), 1.67-1.97 (m, 2H), 1.97-2.19 (m, 1H), 2.52-2.64 (m, 1H), 3.04-3.38 (m, 1H), 3.47 (septet, *J* = 7.2 Hz, 1H), 3.75-4.10 (m, 2H), 4.44-4.70 (m, 1H), 6.40 (d, *J*= 8.4 Hz, 1H), 6.79 (d, *J* = 15.3 Hz, 1H), 7.18-7.29 (m, 2H), 7.41-7.53 (m, 3H), 7.93 (d, *J* = 15.3 Hz, 1H). MS (ESI⁺) (M+H)⁺ at *m*/*z* 478, 480, 482. Anal. Calcd for C₂₄H₂₅Cl₂NO₃S · 0.05 H₂O · 0.01 EtOH: C, 60.13; H, 5.29; N, 2.92. Found: C, 60.14; H, 5.11; N, 2.52.

### Example 336

### (2-Isopropylphenyl)[2,3-dichloro-4-(E-((4-carboxypiperidin-1-yl)carbonyl)ethenyl)phenyl]sulfide

The title compound was prepared by the procedures described in Example 330, substituting 6-mercaptobenzodioxane with 2-isopropylbenzenethiol, giving a white solid. ¹H NMR (d⁶-DMSO, 300 MHz) δ 1.16 (d, *J* = 7.2 Hz, 6H), 1.33-1.53 (m, 2H), 1.64-1.78 (m, 2H), 1.97-2.10 (m, 1H), 2.88 (brt, *J* = 10.5 Hz, 1H), 3.15 (brt, *J* = 10.5 Hz, 1H), 3.97 (br d, *J* = 13.2 Hz, 1H), 4.11 (br d, *J* = 13.2 Hz, 1H), 6.41 (d, *J* = 9.0 Hz, 1H), 7.22 (d, *J* = 15.6 Hz, 1H), 7.31-7.42 (m, 1H), 7.53 (d, *J* = 7.8 Hz, 1H), 7.56-7.64 (m, 2H), 7.71 (d, *J* = 15.6 Hz, 1H), 7.85 (d, *J* = 9.0 Hz, 1H). MS (ESI⁺) (M+H)⁺ at *m*/*z* 478, 480, 482. Anal. Calcd for C₂₄H₂₄Cl₂NNaO₃S · 0.95 H₂O: C, 55.70; H, 5.04; N, 2.71. Found: C, 55.69; H, 4.90; N, 2.57.

### Example 337

### (1-Methylindol-5-yl)[2,3-dichloro-4-(E-((3-carboethoxypiperidin-1-yl)carbonyl)ethenyl)phenyl]sulfide

The title compound was prepared by the procedures described in Example 283, substituting 4-fluoro-3-chlorobenzaldehyde with 2,3-dichloro-4-trifluoromethanesulfoxybenzaldehyde, giving a white solid. ¹H NMR (CDCl₃, 300 MHz) δ 1.23 (t, J = 7.5 Hz, 3H), 1.46-1.67 (m, 1H), 1.67-1.95 (m, 2H), 1.95-2.17 (m, 1H), 2.43-2.60 (m, 1H), 3.02-3.42 (m, 1H), 3.67-3.92 (m, 2H), 3.86 (s, 3H), 4.13 (q, *J* = 7.5 Hz, 2H), 4.59-4.80 (m, 1H), 6.46 (d, *J* = 8.7 Hz, 1H), 6.54 (d, *J* = 3.0 Hz, 1H), 6.77 *(d, J =* 15.3 Hz, 1H), 7.15 *(d, J =* 3.0 Hz, 1H), 7.19 (d, *J* = 8.7 Hz, 1H), 7.37 (d, *J* = 8.7 Hz, 1H), 7.42 (d, *J* = 8.7 Hz, 1H), 7.89 (s, 1H), 7.92 (d, *J* = 15.3 Hz, 1H). MS (ESI⁺) (M+H)⁺ at *m*/*z* 517, 519, 521.

### Example 338

### (1-Methylindol-5-yl)[2,3-dichloro-4-(E-((3-carboxypiperidin-1-yl)carbonyl)ethenyl)phenyl]sulfide

The title compound was prepared by the procedures described in Example 155, substituting the ethyl ester from Example 137 with ethyl ester from Example 337, and KOH with NaOH, to give a white solid. ¹H NMR (d⁶-DMSO, 300 MHz) δ 1.29-1.45 (m, 1H), 1.45-1.78 (m, 2H), 1.78-2.02 (m, 1H), 2.20-2.40 (m, 1H), 2.82 (brt, *J* = 10.5 Hz, 1H), 3.08 (brt, *J* = 10.5 Hz, 1H), 3.80-4.07 (m, 2H), 3.86 (s, 3H), 4.38-4.50 (m, 1H), 6.42 (d, *J* = 8.4 Hz, 1H), 6.54 (d, *J* = 3.0 Hz, 1H), 7.19 (d, *J* = 15.3 Hz, 1H), 7.32 (dd, *J* = 1.8, 8.7 Hz, 1H), 7.48 (d, *J =* 3.0 Hz, 1H), 7.64 (d, *J* = 8.7 Hz, 1H), 7.67-7.77 (m, 2H), 7.87 (d, *J* = 1.8 Hz, 1H). MS (ESI⁺) (M+H)⁺ at *m*/*z* 489, 491, 493. Anal. Calcd for C₂₄H₂₂Cl₂N₂O₃S · 0.56 CH₂Cl₂: C, 54.94; H, 4.34; N, 5.22. Found: C, 54.89; H, 4.44; N, 5.32.

### Example 339

### (1-Methylindol-5-yl)[2,3-dichloro-4-(E-((4-carboethoxypiperidin-1-yl)carbonyl)ethenyl)phenyl]sulfide

The title compound was prepared by the procedures described in Example 285, substituting 4-fluoro-3-chlorobenzaldehyde with 2,3-dichloro-4-trifluoromethanesulfoxybenzaldehyde, providing a white solid. ¹H NMR (CDCl₃, 300 MHz) δ 1.25 *(t, J =* 7.2 Hz, 3H), 1.62-1.79 (m, 2H), 1.87-2.04 (m, 2H), 2.41-2.63 (m, 1H), 2.85-3.41 (m, 2H), 3.85 (s, 3H), 3.87-4.10 (m, 1H), 4.15 (q, *J* = 7.2 Hz, 2H), 4.32-4.60 (m, 1H), 6.46 (d, *J* = 8.7 Hz, 1H), 6.54 (d, *J* = 3.0 Hz, 1H), 6.71 (d, *J* = 15.3 Hz, 1H), 7.15 (d, *J* = 3.0 Hz, 1H), 7.17 (d, *J* = 8.7 Hz, 1H), 7.36 (dd, *J* = 2.4, 8.4 Hz, 1H), 7.42 (d, *J* = 8.4 Hz, 1H), 7.88 (d, *J* = 2.4 Hz, 1H), 7.90 (d, *J* = 15.3 Hz, 1H). MS (ESI⁺) (M+H)⁺ at *m*/*z* 517, 519, 521. Anal. Calcd for C₂₆H₂₆Cl₂N₂O₃S · 0.12 H₂O: C, 60.10; H, 5.09; N, 5.39. Found: C, 60.09; H, 5.21; N, 5.54.

### Example 340

### (1-Methylindol-5-yl)[2,3-dichloro4-(E-((4-carboxypiperidin-1-yl)carbonyl)ethenyl)phenyl]sulfide

The title compound was prepared by the procedures described in Example 155, substituting the ethyl ester from Example 137 with ethyl ester from Example 339, and KOH with NaOH, to give a white solid. ¹H NMR (d⁶-DMSO, 300 MHz) δ 1.31-1.53 (m, 2H), 1.62-1.76 (m, 2H), 1.94-2.09 (m, 1H), 2.88 (brt, *J*= 10.5 Hz, 1H), 3.13 (brt, *J* = 10.5 Hz, 1H), 3.86 (s, 3H), 3.93 (br d, *J* = 13.2 Hz, 1H), 4.09 (br d, *J* = 13.2 Hz, 1H), 6.41 (d, *J* = 8.7 Hz, 1H), 6.53 (dd, *J* = 0.9, 3.0 Hz, 1H), 7.04 (d, *J* = 15.3 Hz, 1H), 7.32 (dd, *J* = 2.1, 8.7 Hz, 1H), 7.48 (d, *J* = 3.0 Hz, 1H), 7.64 (d, *J* = 8.7 Hz, 1H), 7.69 (d, *J* = 15.3 Hz, 1H), 7.73 (d, *J* = 8.7 Hz, 1H), 7.88 (d, *J* = 2.1 Hz, 1H). MS (ESI⁺) (M+H)⁺ at *m*/*z* 489, 491, 493. Anal. Calcd for C₂₄H₂₁Cl₂N₂NaO₃ S · 0 H2O: C, 56.37; H, 4.14; N, 5.48. Found: C, 56.44; H, 4.38; N, 5.20.

An alternative method for preparing Example 340 is given below.

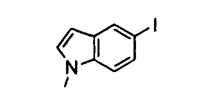

### Example 340A

### 1-Methyl-5-iodoindole

To a solution of 5-iodoindole (75 g, 0.31 mol) in dry THF (750mL), at -78°C was added sodium hydride (60% in mineral oil, 14.85 g, 0.37 mol) in one portion. The suspension was stirred at -78°C for 1 hour after which iodomethane (28.8 mL, 0.46 mol) was added. The reaction mixture was stirred overnight with a slow elevation on temperature to room temperature(no more dry ice was added). Ether (600mL) and hexane (1.2L) were added and the mixture was washed with brine (1.6L) and water (1.5L), dried over Na₂SO₄ and filtered. The solution was concentrated and the residual brown solid was recrystallized from hexane to give the title compound (66 g). The impure fraction from the mother liquor was flash chromatographed (8% EtOAc in hexane) to give an additional quantity of desired product (12.5 g, combined yield of 99%). MS (DCI/NH₃) m/e 258 (M+H)⁺.

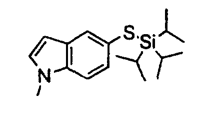

### Example 340B

### 1-Methyl-S-triisopropylsilyl-5-indolethiol

Potassium hydride (35% in mineral oil, 12.03 g, 0.105 mol) was charged to a 250 mL RBF and was washed with dry THF (2x50mL). The resultant KH powder was then suspended in dry THF (75 mL), and cooled to 5 °C. Triisopropylsilylthiol (20.0 g, 0.105 mol) was slowly added via syringe over a period of 15 minutes. Vigorous escape of hydrogen gas was observed with addition of the thiol. The suspension was stirred at 5°C for 1 our and became homogenous. After another hour stirring at room temperature, this solution was cannulated to a THF solution (100mL) containing Example 340A (24.5 g, 95.5 mmol) and tetrakis(triphenylphosphine)palladium(0) (2.2 g, 1.91 mmol). The yellow suspension was stirred at 70°C for 1 hour. After cooled, ether and hexane were added, and the mixture was washed with brine, dried (Na₂SO₄) and concentrated. The residual oil was purified by flash chromatography (silica gel, 3% EtOAc in hexane) to give the title compound (26.7 g, 88%). MS (DCI/NH₃) m/e 320 (M+H)⁺.

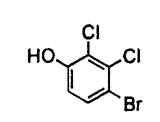

### Example 340C

### 4-Bromo-2,3-dichlorophenol

To a solution of 2,3-dichlorophenol (200 g, 1.227 mol) in dichloromethane (800 mL), at 0 °C was added dropwise bromine (196.1g, 1.227 mol) from a dropping funnel within 1 hour. The red solution was stirred overnight (0°C - rt), and washed with 10% NaHSO₃. The organic phase was dried over Na₂SO₄, and concentrated. The residual white solid was recrystallized from hexane to give example 340C as white needles (207g, 70%). MS (DCI/NH₃) *m*/*e* 241 (M+H)⁺.

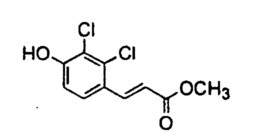

### Example 340D

### Methyl 2,3-dichloro-4-hydroxyphenylacrylate

A 1 L RBF was charged with Example 340C (48.4 g, 0.2 mol), Pd₂(dba)₃ (4.6 g, 5 mmol), (Tol)₃P (4.66 g, 15.2 mmol), and purged with nitrogen. Dry DMF (300 mL), methyl acrylate (51.66 g, 0.6 mol) and triethylamine (84 mL, 0.6 mol) were then added. The reaction mixture was purged with nitrogen and stirred at 100°C (oil bath) for 16 hours. After cooled to room temperature, a lot of white crystalline material formed. Ethyl acetate (500 mL) and brine (not saturated, 800 mL) were added, and stirred. The white crystalline material dissolved. A little insoluble black solid (Pd) was filtered off. To the solution was then added, with stirring, saturated NaCl solution (2 L) and hexane (500 mL). The mixture was stirred for 1 hour. The formed yellowish solid was collected by filtration, washed with water (400 mL), acetonitrile (50 mL) and 1:1 ethyl acetate/hexane (500 mL), and dried to give pure desired compound (44.99g, 91 %). MS (DCI/NH₃) *m*/*e* 247 (M+H)⁺.

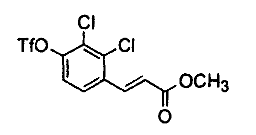

### Example 340E

### Methyl 2,3-dichloro-4-trifluoromethane sulfonyloxyphenylacrylate

To a suspension of Example 340D (18.62 g, 75.4 mmol) in pyridine (150 mL) at 5°C was added trifluoromethylsulfonyl anhydride (25.53 g, 90 mmol) very slowly. The suspension was stirred at 5 °C for 1 hour and became homogeneous. The solution was kept at 5 °C for 2 hours and at room temperature for 20 minutes. Ether (700mL) was added and the mixture was washed 10%HCl (700 mL)/brine (300 mL), 10%HCl (100 mL)/brine (900 mL), and brine (500 mL). The organic phase was dried (Na₂SO₄) and concentrated to give the title compound (24.86 g, 87%). MS (DCI/NH₃) *m*/*e* 379 (M+H)⁺.

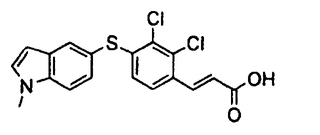

### Example 340F

### (1-Methylindol-5-yl)[2,3-dichloro-4-(E-(carboxyethenyl)phenyl]sulfide

To a solution of Example 340B (38.5 g, 0.12 mol) and Example 340E (30.3 g, 0.08 mol) in dry N-methylpyrrolidinone (300 mL) was added CsF (18.2 g, 0.12 mol) at 5°C under nitrogen atmosphere. After 1 hour stirring at the same temperature, the cooling bath was removed, and the mixture was stirred at room temperature for 0.5 hour. Ethyl acetate (800 mL) was added, and the mixture was washed with brine and water, and concentrated. The residual oil was separated by flash chromatography (20% EtOAc/hexane) to give a yellow solid (30 g).

This yellow solid was dissolved in THF (150 mL), and was added a solution of LiOH (4.0 g, 0.16 mol) in H₂O (50 mL). The mixture was stirred at room temperature for 1 hour and more water (100 mL) was added to form a transparent solution. After overnight stirring the solution was acidified with 10 % aq. HCl. The mixture was concentrated under reduced pressure to about 100 mL. The formed solid material was collected by filtration, washed with water (200 mL), acetonitrile (30 mL), 1:1 ether/hexane, and dried to give the title compound (22.3 g, overall 74%). MS (DCI/NH₃) *m*/*e* 378 (M+H)⁺.

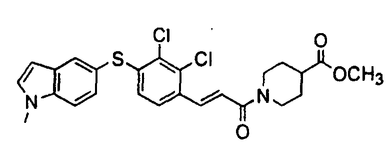

### Example 340G

### (1-Methylindol-5-yl)[2,3-dichloro-4-(E-((4-carbomethoxypiperidin-1-yl)carbonyl)ethenyl)phenyl]sulfide

To a solution of Example 340F (9.5 g, 25.1 mmol) and methyl isonipecotate (7.19 g, 50.2 mmol) in DMF (70 mL) was added EDC (9.64 g, 50.2 mmol), HOBt (6.78 g, 50.2 mmol) and triethylamine (7.0 mL, 50.2 mmol). The reaction mixture was stirred at room temperature for 15 hours. Ethyl acetate (800 mL) was added, and the mixture was washed with brine, and concentrated. The residue was purified by flash chromatography (60% EtOAc in hexane) to give example 340G as white powder (10.86 g, 94%). MS (ESI⁺) *m*/*z* 503 (M+H)⁺.

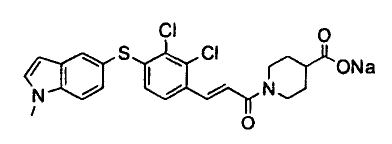

### Example 340

### (1-Methylindol-5-yl)[2,3-dichloro-4-(E-((4-carboxypiperidin-1-yl)carbonyl)ethenyl)phenyl]sulfide, sodium salt

To a suspension of Example 340G (11.8 g, 23.6 mmol) in THF (150 mL) was added a solution of lithium hydroxide monohydrate (1.98 g, 47.2 mmol) in H₂O (30 mL). The mixture was stirred at room temperature overnight. Water (120 mL) was added and formed transparent solution was stirred for another hour before 10% HCl (30 mL) was added. The mixture was concentrated under reduced pressure to about 120 mL. The formed solid material was collected by filtration, washed with water, acetonitrile, and dried to give a white solid (11.0 g).

10.50 grams of the solid was suspended in methanol (60 mL), and was treated with a solution NaOH (0.859g) in methanol (20 mL). After all of the solid material went into solution, the solvent was removed under reduced pressure. The residual yellow oil was triturated with ether, and dried to give the title compound as yellow powder (11.33 g, 95%).

### Example 341

### (2-Ethoxyphenyl)-[2,3-dichloro-4(E-[(4-carboxypiperidin-1-yl)carbonyl]ethenyl)phenyl]sulfide

The title compound was prepared according to the procedures of Example 310, substituting 2-ethoxybenzenethiol prepared according to the procedures of Example 97A. ¹H-NMR (CD₃OD, 300 MHz) Potassium salt δ 1.20 (t, J=7Hz, 3H), 1.55-1.72 (m, 2H), 1.88-1.98 (m, 2H), 2.32 (m, 1H), 2.88 (t, J=12Hz, 1H), 3.20 (t, J=12 Hz, 1H), 4.05 (q, J=7Hz, 2H), 4.14 (d, J=12 Hz, 1H), 4.48, (d, J = 12 Hz, 1H), 6.64 9d, J=9Hz, 1H), 7.00-7.15 (m, 3H), 7.44-7.50 (m, 2H), 7.56 (d, J=9Hz, 1H), 7.90 (d, J=15 Hz, 1H) Anal. Calcd. for C₂₃H₂₂KCl₂NO₄S 0.5 H₂O: C, 52.37, H, 4.39, N, 2.66. Found: C, 52,23; H, 4.56; N, 2.49.

### Example 342

### (2-Ethoxyphenyl)-[2,3-dichloro-4(E-[(morpholin-1-yl)carbonyl]ethenyl)phenyl]sulfide

The title compound was prepared according to the procedures of Example 310, substituting 2-ethoxybenzenethiol prepared according to the procedures of Example 97A. ¹H-NMR (CDCl₃ 300 MHz) δ 1.25 (t, J=7Hz, 3H), 3.55-3.80 (m, 8H), 4.05 (q, J=7Hz, 2H), 6.63 (d, J=9Hz, 1H), 6.71 (d, J=15 Hz, 1H), 6.95-7.03 (m, 2H), 7.26 (d, J=9Hz, 1H), 7.39-7.50 (m, 2H), 7.99 (d, J=15 Hz, 1H) Anal. Calcd. for C₂₁H₂₁Cl₂NO₃S: C, 57.54; H, 4.82; N, 3.20. Found: C, 57.55; H, 4.77; N, 3.14.

### Example 343

### (2-Ethoxyphenyl)-[2,3-dichloro-4(E-[(3-carboxypiperidin-1-yl)carbonyl]ethenyl)phenyl]sulfide

The title compound was prepared according to the procedures of Example 310, substituting 2-ethoxybenzenethiol prepared according to the procedures of Example 97A. ¹H-NMR (CD₃OD 300MHz) δ 1.20 (t, J=7Hz, 3H), broad peaks totaling 9 protons at 1.4-1.95, 2.0-2.14, 2.22-2.35, 2.75-3.134.10-4.34, 4.69-4.76, 4.05 (q, J=7Hz, 2H), 6.64 (d, J=9Hz, 1H), 7.03 (t, J=8Hz, 1H), 7.10 (d, J=9Hz, 1H), 7.22 (d, J=15 Hz, 1H), 7.45-7.50 (m, 2H), 7.62 (d, J=9Hz, 1H), 7.80 (d, J=15 Hz, 1H). The acid (303 mg, 0.63 mmol) was dissolved in 3 mL of methanol. A solution of KOH (0.60 mmol) in 1 mL of methanol was added. The resultant solution was stirred for 5 min and concentrated in vacuo. Ether (5 mL) was added, and the mixture was stirred for 1 hr. The resultant powder was collected by filtration and dried under vacuum at 60C to give 307 mg of a solid, water-soluble product. Anal. Calcd. for C₂₃H₂₂KC₁₂NO₄S 0.5 H₂O; C, 52.37; H, 4.39; N, 2.66 . Found: C, 52.20; H, 4.65, N, 3.04.

### Example 344

### (2-Isopropylphenyl)[2-nitro-4-(E-((3-carboethoxypyrrolidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 7.0 Hz, 6H); 1.20 (t, J = 7.0 Hz, 3H); 1.92-2.30 (m, 2H); 3.10-4.01 (m, 6H); 4.06-4.17 (m, 2H); 6.64 (d, J = 8.5 Hz, 1H); 7.06-7.17 (m, 1H), 7.34-7.62 (m, 5H); 7.88-7.96 (m, 1H); 8.62 (dd, J = 1.5, 8.5 Hz, 1H). MS (APCI) (M+H)⁺ at m/z 469. Anal calcd for C₂₅H₂₈N₂S₁O₅: C, 64.08; H, 6.02; N, 5.98. Found: C, 64.12; H, 5.98; N, 5.89.

### Example 345

### (2-Isopropylphenyl)[2-nitro-4-(E-((3-carboxypyrrolidin-1-yl)carbonyl)ethenyl)phenyl]sulfide

Prepared according to the procedures of Example 71, giving a yellow solid. ¹H NMR (DMSO-d₆, 300MHz) δ 1.14 (d, J = 6.8 Hz, 6H); 1.92-2.24 (m, 2H); 3.01-3.92 (m, 6H); 6.64 (dd, J = 1.7, 8.5 Hz, 1H); 7.04-7.16 (m, 1H), 7.33-7.61 (m, 5H); 7.87-7.95 (m, 1H); 8.61 (dd, J = 1.7, 8.5 Hz, 1H). MS (APCI) (M+H)⁺ at m/z 441. Anal calcd for C₂₃H₂₄N₂S₁O₅: C, 62.71; H, 5.49; N, 6.36. Found: C, 62.47; H, 5.39; N, 6.09.

### Example 346

### (2-Isopropylphenyl)[2,3-difluoro-4-(E-((3-carboethoxypiperidin-1-yl)carbonyl)ethenyl)phenyl]sulfide

Prepared according to the procedures of Example 71, giving a yellow solid. ¹H NMR (DMSO-d₆, 300MHz) δ 1.18 (d, J = 7.0 Hz, 6H); 1.10-1.22 (m, 3H); 1.30-2.07(br m, 4H); 2.50-3.45 (br m, 3H); 3.55-4.47 (br m, 5H); 6.62-6.72 (m, 1H); 7.23-7.73 (m, 7H). MS (APCI) (M+H)⁺ at m/z 474.

### Example 347

### (2-Isopropylphenyl)[2,3-difluoro-4-(E-((3-carboxypiperidin-1-yl)carbonyl)ethenyl)phenyl]sulfide

Prepared according to the procedures of Example 71, giving a yellow solid. ¹H NMR (DMSO-d₆, 300MHz) δ 1.18 (d, J = 7.0 Hz, 6H); 1.30-2.03 (br m, 4H); 2.25-3.50 (br m, 4H); 3.87-4.51 (br m, 2H); 6.62-6.72 (m, 1H); 7.23-7.73 (m, 7H). MS (APCI) (M+H)⁺ at m/z 446.

### Example 348

### (2-Isopropylphenyl)[2,3-difluoro-4-(E-((4-carboxypiperidin-1-yl)carbonyl)ethenyl)phenyl]sulfide

Prepared according to the procedures of Example 71, giving a yellow solid. ¹H NMR (DMSO-d₆, 300MHz) δ 1.18 (d, J = 6.8Hz, 6H); 1.30-1.91 (br m, 4H); 2.50-3.50 (br m, 4H); 4.02-4.34 (br m, 2H); 6.62-6.72 (m, 1H); 7.23-7.73 (m, 7H). MS (APCI) (M+H)⁺ at m/z 446.

### Example 349

### (Benzodioxan-6-yl)[2-trifluoromethyl-4-(E-((3-ethoxycarbonylpyrrolidin-1-yl)carbonyl)ethenyl)phenyl]sulfide

The title compound was prepared according to the procedures of Example 1. ¹H NMR (CDCl₃, 300 MHz) δ 7.77 (s, 1H), 7.62 (d, 1H, J = 15.4 Hz) 7.42 (d, 1H, J = 8.5 Hz), 7.06 (d, 1H, J = 2.1 Hz), 6.98-7.04 (m, 2H), 6.91 (d, 1H, J = 8.1 Hz), 6.68 (dd, 1H, J = 3.3, 15.3 Hz), 4.30 (m, 4H), 4.19 (q, 2H, J = 7.0 Hz), 3.56-3.92 (m, 4H), 3.06-3.24 (m, 1H),2.10-2.35 (m, 2H), 1.28 and 1.29 (two t, 3H, J = 7.2 Hz). MS (ESI) *m*/*z* 508, 1015.

### Example 350

### (Benzodioxan-6-yl)[2-trifluoromethyl-4-(E-((3-carboxypyrrolidin-1-yl)carbonyl)ethenyl)phenyl]sulfide

The title compound was prepared by hydrolysis of the compound of Example 349 according to standard procedures. ¹H NMR (DMSO-d₆, 300 MHz) δ 8.10 (d, 1H, J = 9.9 Hz), 7.84 (t, 1H, J = 7.8 Hz), 7.46 (d, 1H, J = 15.3 Hz), 7.10 (d, 1H, J = 15.3 Hz), 6.97-7.06 (m, 4H), 4.30 (m, 4H), 3.50 (br, overlapped with water residue peak), 3.00 (m, 1H), 2.10 (m, 1H), 2.00 (m, 1H). MS (ESI) *m*/*z* -478, -957.

### Example 351

### (2-Methoxyphenyl)[2-chloro-3-trifluoromethyl-4-(E-((4-carboethoxypiperidin-1-yl)carbonyl)ethenyl)phenyl]sulfide

### Example 351A

### 3-Chloro-4-hydroxy-2-(trifluoromethyl)benzaldehyde

Chloroform (6.7g, 2.0 eq.) was added dropwise to a stirred mixture of Ca(OH)₂ (8.95g, 120 mmol.), K₂CO₃ (13.5g, 98 mmol.), 2-chloro-3-(trifluoromethyl)phenol (5.0g, 22 mmol.), and H₂O (50 mL) at 60°-70° over 2 h. The reaction mixture was cooled, and acidified with cone. HCl. The product was extracted into EtOAc and dried over Na₂SO₄. Solvent was evaporated, the crude product was separated and purified through a silica column, eluting with hexane and EtOAc (3:2) to give 580 mg (10%) of the title compound.

### Example 351B

### (2-Methoxyphenyl)[2-chloro-3-trifluoromethyl-4-(E-carboxyethenyl)phenyl]sulfide

The title compound was prepared according to the procedures described in Example 310, substituting the compound of Example 351A for 4-hydroxy-2,3-dichlorobenzaldehyde.

### Example 351C

### (2-Methoxyphenyl)[2-chloro-3-trifluoromethyl-4-(E-((4-carboethoxypiperidin-1-yl)carbonyl)ethenyl)phenyl]sulfide

To the acyl chloride (37 mg, 0.1 mmol) prepared from the compound of Example 351B, as a solution in CH₂Cl₂ was added 1.2 eq.. of ethyl isonipecotate and 1.2 eq. of Hunig's base. The mixture was stirred at room temperature for 20 min., ∼90% of the solvent was removed in vacuo, and the resultant solution was loaded on a silica column to elute with hexane and EtOAc (3:2) to give 51mg (98%) of the title compound. ¹H-NMR (CDCl3, 300MHz) δ 1.25 (t, J=7.5Hz, 3H), 1.65-1.78 (m, 2H), 1.92-2.02 (br, 2H), 2.51-2.60 (m, 1H), 2.93-3.24 (br, 2H), 3.82 (s, 3H), 3.88-3.96 (m,1H), 4.15 (q,J=7.5Hz, 2H), 4.40-4.50 (br, 1H), 6.48 (d, J=15Hz, 1H), 6.72 (d, J=9Hz, 1H), 7.02 (d, J=7.5Hz, 2H), 7.12 (d, J=9Hz, 1H), 7.49 (t, J=9Hz, 2H), 7.86 (qq, J=4.5Hz, 1H). MS (DCI/NH₃) m/e 528 (M+H)⁺.

### Example 352

### (2-Methoxyphenyl)[2-chloro-3-trifluoromethyl-4-( E-((4-carboethoxypiperidin-1-yl)carbonyl)ethenyl)phenyl]sulfide

The compound of Example 351 was hydrolyzed by aq. NaOH in EtOH at rt. to give 90% yield of the title compound. ¹H NMR(DMSO, 300MHz) δ 1.37-1.52 (br. 2H), 1.78-1.86 (br. 2H), 2.45-2.55 (m, 1H), 2.83 (t, J=12Hz, 1H), 3.17 (t, J=13.5 Hz, 1H), 3.80 (s, 3H), 4.07 (d, J=12Hz, 1H), 4.26 (d, J=13.5Hz, 1H), 6.75 (d, J=9Hz, 1H), 6.98 (d, J=15Hz, 1H), 7.11 (t, J=9Hz, 1H), 7.26 (d, J=9Hz, 1H), 7.53 (d, J=7.5Hz, 1H), 7.62 (d, J=9Hz, 2H), 7.70 (qq, J=4.5Hz, 1H). MS (DCI/NH₃) m/e 500(M+H)⁺.

### Example 353

### (2-Methoxyphenyl)[2-chloro-3-trifluoromethyl-4-(E-((morpholin-1-yl)carbonyl)ethenyl)phenyl]sulfide

Prepared according to the procedures of Example 351, giving 50 mg (91%) of the title compound. ¹H-NMR (CDCl₃, 300MHz) δ 3.56-3.62 (br m, 2H), 3.67-3.77 (br m, 6H), 3.85 (s, 3H), 6.45 (d, J=15Hz, 1H), 6.73 (d, J=9Hz, 1H), 7.03 (d, J=9Hz, 2H), 7.09 (t, J=9Hz, 1H), 7.52 (d, J=9Hz, 2H), 2.93 (qq, J=6Hz, 1H). MS (DCI/NH₃) *m*/*z* 458 (M+H)⁺.

### Example 354

### (Benzodioxan-6-yl) [ 4-(E-((4-carboxypiperidin-1-yl)carbonyl)ethenyl)naphthyl]sulfide

The methods of Example 310 and 311 were used to convert 4-hydroxy-2-naphthaldehyde and 6-benzodioxanethiol to the desired product as a yellow solid. ¹H NMR (DMS-d₆, 300MHz) δ 1.50 (br s, 2H), 1.83-1.92 (m, 2H), 2.5-2.6 (m, 1H), 2.85-2.95 (m, 1H), 3.18-3.29 (m, 1H), 4.22 (br s, 5H), 4.30-4.38 (m, 1H), 6.87-6.92 (m, 3H), 7.38 (d, J=15Hz, 1H), 7.45 (d, J=7.5Hz, 1H), 7.64-7.70 (m, 2H), 7.93 (d, J=7.5Hz, 1H), 8.20-8.45 (m, J=3H). MS(ESI⁺ ) *m*/*z* 476 (M+H)⁺. Anal calcd for C₂₇H₂₅NO₅S^{·}0.67H₂O: C, 66.50; H, 5.44; N, 2.87. Found: C, 66.56; H, 5.81; N, 2.49.

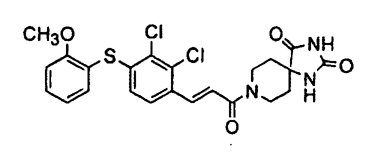

### Example 355

### (2-Methoxyphenyl)[2,3-dichloro-4-(E-((4-(spiro-hydantoin-5-yl)-piperidin-1-yl)carbonyl)ethenyl)phenyl]sulfide

The title compound was prepared from Example 310C, using the procedures described in Example 340 and substituting methyl isonipecotate with piperadine-4-spiro-5'-hydantoin, which was prepared according to a literature method (Wysong, C., et al, *J. Org. Chem*. **1996,** 7650). ¹H NMR (300 MHz, DMSO-d₆) δ 1.65 (m, 2H), 1.75 (m, 2H), 3.05 (m, 1H), 3.50 (m, 1H), 4,12 (m, 1H), 4.20 (m, 1H), 6.56 (d, J=6.5Hz, 1H), 7.10 (t, J=8.0Hz, 1H), 7.22 (d, J=8.0 Hz, 1H), 7.28 (d, J=15.6 Hz, 1H), 7.49 (dd, J=8.0, 1.7Hz, 1H), 7.56 (t, J=8.2Hz, 1H), 7.76 (d, J=15.6Hz, 1H), 7.84(d, J=8.6Hz, 1H), 8.58 (s, 1H), 10.73(s, 1H). MS (ESI⁻) *m*/*z* 504 (M-H)-.

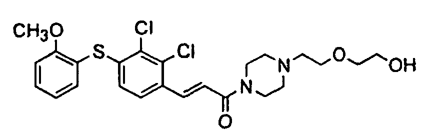

### Example 356

### (2-Methoxyphenyl)[2,3-dichloro-4-(E-(4-(2-(2-hydroxyethoxy)ethyl)piperazin-1-yl)carbonyl)ethenyl)phenyl]sulfide

The title compound was prepared from Example 310C by the procedures described in Example 340 and substituting methyl isonipecotate with N-[2-(2-hydroxyethoxy)ethyl]piperazine. ¹H NMR (300 MHz, DMSO-d₆) δ 3.10 (m, 2H), 3.50 (m, 4H), 4.50 (m, 2H), 4.70 (s, 1H), 6.57 (d, J=8.5Hz, 1H), 7.09 (t, J=8.0Hz, 1H), 7.23 (d, J=8.0Hz, 1H), 7.26 (d, J=15.5Hz, 1H), 7.49 (dd, J=7.8, 1.7Hz, 1H), 7.57 (t, J=8.2Hz, 1H), 7.78 (d, J=15.6Hz, 1H), 7.80 (d, J=7.8Hz, 1H). MS (ESI⁻) *m*/*z* 545 (M-H)-.

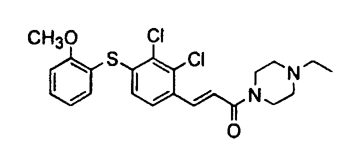

### Example 357

### (2-Methoxyphenyl)[2,3-dichloro-4-(E-((4-ethylpiperazin-1-yl)carbonyl)ethenyl)phenyl]sulfide

The title compound was prepared from Example 310C by the procedures described in Example 340 and substituting methyl isonipecotate with 1-ethylpiperazine. ¹H NMR (300 MHz, CDCl₃) δ 1.09 (t, J=7.1 Hz, 3H), 2.42 (q, J=7.1Hz, 2H), 2.47 (m, 4H), 3.60 (m, 2H), 3.75 (m, 2H), 3.82 (s, 3H), 6.56 (d, J=8.5Hz, 1H), 6.74(d, J=15.3Hz, 1H), 7.02 (m, 2H), 7.26 (d, J=8.5Hz, 1H), 7.46 (m, 2H), 7.94 (d, J=15.5Hz, 1H). MS (ESI⁺) *m*/*z* 451 (M+H)⁺.

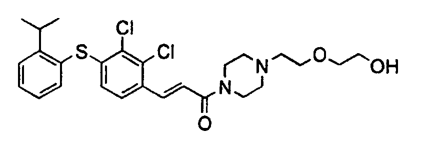

### Example 358

### (2-Isopropylphenyl)[2,3-dichloro-4-(E-((4-(2-(2-hydroxyethoxy)ethyl)piperazin-1-yl)carbonyl)ethenyl)phenyl]sulfide

The title compound was prepared from the cinnamide acid of Example 331, using the procedures described in Example 340 and substituting methyl isonipecotate with N-[2-(2-hydroxyethoxy)ethyl]piperazine. ¹H NMR (300 MHz, DMSO-d₆) δ 1.18 (d, 6H), 3.0 (m, 3H), 3.30 (m, 2H), 3.50 (m, 10H), 3.80 (m, 2H), 4.50 (t, 1H), 6.45 (d, 1H), 7.30 (d, 1H), 7.35 (dd, 1H), 7.55 (d, 1H), 7.60 (m, 2H), 7.75 (d, 1H), 7.80 (d, 1H). MS (ESI⁻) *m*/*z* 523 (M+H)⁺.

### Example 359

### (Benzodioxan-6yl)[2,3-bis(trifluoromethyl)-4-(E-((4-carboxypiperidin-1-yl)carbonyl)ethenyl)phenyl]sulfide

### Example 359A

### 1-Methyl-2,3-bis(trifluoromethyl)-7-oxabicyclo[2.2.1]hepta-2,5-diene

Hexafluoro-2-butyne (21.0 g, 0.13 mol) was transferred into a reaction bottle and added 2-methylfuran (12.86 g, 0.157 mol). This resulting mixture bottle was sealed and heated for 15 hr. at 120 °C. After cooling, the excess 2-methylfuran was rotoevaporated in vacuo at rt, to give crude title product (29 g, 92%), which was used directly.

### Example 359B

### 4-Methyl-2,3-bis(trifluoromethyl)phenol

A mixture of Example 359A (12.0 g, 0.05 mol) and boron trfluoride-diethyl ether complex (150 ml) was stirred at room temp overnight, then neutralized carefully with 20% aqueous potassium carbonate, then the mixture was extracted with ether. The ether layer was dried over MgSO₄ and evaporated under reduced pressure to afford 10.4g (85%) of the title compound.

### Example 359C

### 4-[4-Bromobenzene sufonyloxy-2,3-bis(trifluoromethyl)]benzylbromide

The phenol compound of Example 359B (10 g, 0.04 mol) was treated with 4-bromobenzenesulfonyl chloride (11.0 g, 0.043 mol) and Hunig's base (5.56 g, 0.043 mol) in CH₂Cl₂ (150 ml). The solution was washed with water, brine and dried over MgSO₄. After evaporating the solvent, N-bromosuccinimide (7.3 g, 0.04 mol) and benzoyl peroxide (200 mg) were added and the mixture was suspended in CCl₄ (100ml). The resulting mixture was refluxed for 13 hr. When the reaction was cooled, the white solid was filtered and washed with CCl₄ to afford the crude title compound. This crude product was used for next step without further purification.

### Example 359D

### 4-Hydroxy-2,3-bis(trfluoromethyl)benzaldehyde

The crude product of Example 359C was dissolved in 60 ml of DMSO and 20 ml of CH₂Cl₂, and 12 g of trimethylamine N-oxide added. The resulting mixture was stirred at rt for 2.5 hr. The reaction mixture was poured into an ice cold 50% saturated aqueous NaCl solution (200 ml) and extracted with ether (3X100 ml). The combined organic layer was washed with brine and dried over Na₂SO₄. After evaporation of solvent, the product was purified by column chromatography, eluted with hexane:EtOAc (3:2) to provide 3.0 g of the title compound, plus 4.0 g of recovered 4-[4-bromobenzenesulfonyloxy-2,3-bis(trfluoromethyl)]toluene.

### Example 359E

### (Benzodioxan-6-yl)-[2,3-bis(trfluoromethyl)-4-(E-carboethenyl)phenyl]sulfide

The title compound was prepared according to the procedures described in Example 330, substituting the compound of Example 359D for 4-hydrox-2,3-dichlorobenzaldehyde.

### Example 359F

### (Benzodioxan-6yl)[2,3-bis(trifluoromethyl)-4-(E-((4-carboxypiperidin-1-yl)carbonyl)ethenyl)phenyl]sulfide

The title compound was prepared from Example 359E by the procedures described in Example 330, giving a white solid. ¹H NMR (CD₃OD, 300MHz) δ 1.65(br s, 2H),1.93-2.04 (m, 2H), 2.57-2.65 (m, 1H), 2.95-3.05 (m, 1H), 3.25 (m, 1H), 4.12 (m, 1H), 4.28 (m, 4H), 4.41 (m, 1H), 6.92-7.03 (m, 4H), 7.25 (d, J=9Hz, 1H), 7.72 (d, J=9Hz, 1H), 7.72-7.81 (m, 1H). MS (ESI) *m*/*e* 562 (M+H)⁺. Anal calcd for C₂₅H₂₁NO₅F₆S: C, 53.48; H, 3.77; N, 2.49. Found: C, 53.42; H, 3.69; N, 2.25.

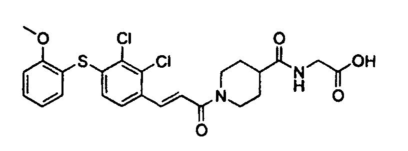

### Example 360

### (2-Methoxyphenyl) [2,3-dichloro-4-(E-((4-(carboxymethylamino)carbonyl-piperidin-1-yl)carbonyl)ethenyl)phenyl]sulfide

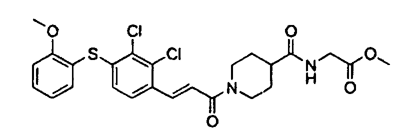

### Example 360A

### (2-Methoxyphenyl)[2,3-dichloro-4-(E-((4-(methylaminomethylcarboxylate)carbonylpiperidin-1-yl)carbonyl)ethenyl)phenyl]sulfide

The title compound was prepared by the procedure described in Example 363 using glycine methyl ester as the coupling substrate. HPLC (Supelco C-18 column, water:acetonitrile 50:90- 90:50, 9 minute elution, flow rate 1.5 mL/min, rt = 6.11 min. MS (APCI) m/e 537 (M+H)⁺;
¹H NMR (300 MHz, DMSO-d₆) δ 1.46(m, 3H), 1.78(br d, 2H), 2.79(m, 1H), 3.15(m, 1H), 3.62(s, 3H), 3.80(s, 3H), 3.83(d, 2H), 4.20(m, 1H), 4.40(m, 1H), 6.58(d, 1H), 7.09(t, 1H), 7.22(d, 1H), 7.25(dd, 1H), 7.48(d, 1H), 7.56(t, 1H), 7.72(d, 1H), 7.81(d, 1H), 8.28(t, 1H). Anal calcd for C₂₅H₂₆Cl₂N₂O₅S·1.3 H₂O: C, 53.54; H, 5.14; N, 4.99. Found: C, 53.49; H, 4.88; N, 4.75.

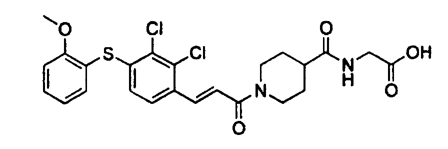

### Example 360B

### (2-Methoxyphenyl)[2,3-dichloro-4-(E-((4-(carboxymethylamino)carbonyl-piperidin-1-yl)carbonyl)ethenyl)phenyl]sulfide

The title compound was hydrolyzed as described in Example 340H. HPLC (Supelco C-18 column, water:acetonitrile 90:0- 0:90, 30 minute elution, flow rate 0.8 mL/min) rt 26.14 min. ¹H NMR (300 MHz, DMSO-d₆) δ 1.46 (m, 2H), 1.75 (m, 2H), 2.73 (m, 1H), 3.12 (m, 1H), 3.70 (m, 2H), 3.79 (s, 3H), 4.02 (m, 1H), 4.20 (m, 1H), 4.41 (m, 1H), 6.65 (d, 1H), 7.09 (dt, 1H), 7.22 (d, 1H), 7.25 (dd, 1H), 7.48 (dd, 1H), 7.58 (m, 1H), 7.72 (d, 1H), 7.82 (d, 1H), 8.11 (m, 1H). MS (APCI) *m*/*e* 523 (M+H)⁺.

### Example 361

### (2-Methoxyphenyl)[2,3-bis(trifluoromethyl)-4-(E-((4-carboxymethylpiperazin-1-yl)carbonyl)ethenyl)phenyl]sulfide

The title compound was prepared according to the procedures of Example 22, employing the compound of Example 359D as starting material, to give a white solid. ¹H NMR (CD₃OD, 300 MHz) δ 3.07-3.12 (m, 4H), 3.48 (s, 2H), 3.74 (s, 3H), 3.89 (br s, 4H), 6.99-7.18 (m, 4H), 7.53 (d, J=9Hz, 2H), 7.72 (d, J=9Hz,1H), 7.78-7.88 (m, 1H). MS (ESI) *m*/*z* 549 (M+H)⁺. Anal calcd for C₂₆H₂₆F₆N₂O₄S·0.9HAc: C, 51.43, H, 4.28, N, 4.65. Found: C, 51.48, H, 4.12, N,4.45.

### Example 362

### (2-Methoxyphenyl)[2,3-bis(trifluoromethyl)-4-(E-((4-N-(2-hydroxyethyl)piperazin-1-yl)carbonyl)ethenyl)phenyl]sulfide

The title compound was prepared by the procedures described in Example 356, employing the compound of Example 359D as starting material to give an oil. ¹H NMR (CDCl₃, 300 MHz) δ 2.68 (br s, 6H), 3.71 (br s, 4H), 3..80 (br s, 5H), 6.55 (d, J=15Hz, 1H), 6.93-7.02 (m, 2H), 7.10 (d, J=9Hz, 1H), 7.35 (d, J=9Hz, 1H), 7.41-7.50 (m, 2H), 7.82 (qq, J=15Hz, 1H). MS (ESI) *m*/*z* 535 (M+H)⁺. Anal calcd for C₂₄H₂₄F₆N₂O₃S·HCl: C,50.49; H, 4.41; N, 4.91. Found: C, 50.72; H, 4.70; N, 4.55.

### Example 363

### (1-Methylindol-5-yl)[2,3-dichloro-4-(E-((4-(carbo-2,3-dihydroxypropylamino)piperidin-1-yl)carbonyl)ethenyl)phenyl]sulfide

To a solution of Example 340H (100mg, 0.2 mmol) and 3-amino-1,2-propanediol (37.4 mg, 0.41 mmol) in DMF (3 mL) was added EDC (78 mg, 0.41 mmol), HOBt (55 mg, 0.41 mmol) and triethylamine (0.057 mL, 0.41 mmol). The reaction mixture was stirred at room temperature for 15 hours. Ethyl acetate (60 mL) was added, and the mixture was washed with brine. The aqueous phase was extracted with 10% MeOH in methylene chloride. The combined organic phases were concentrated to dry. The residual material was triturated with water, filtered, washed with water, acetonitrile and ethyl acetate, and dried to give example 363 (92 mg, 80%). ¹H NMR (300 MHz, DMSO-d₆) δ 1.44 (m, 1H), 1.72 (m, 1H), 2.41 (m, 1H), 2.70 (t, 1H), 3.00 (m, 2H), 3.20 (m, 2H), 3.27 (m, 2H), 3.50 (m, 2H), 3.90 (s, 3H), 4.18 (br d, 1H), 4.40 (br d, 1H), 4.50 (t, 1H), 4.77 (d, 1H), 6.40 (d, 1H), 6.58 (d, 1H), 7.19 (d, 1H), 7.35 (d, 1H), 7.5 0(d, 1H), 7.66 (d, 1H), 7.70 (m, 2H), 7.80 (t, 1H), 7.88 (s, 1H). MS (ESI⁺) *m*/*z* 562 (M+H)⁺. Anal. calcd for C₂₇H₂₉Cl₂N₃SO₄·0.25H₂O: C, 57.19; H, 5.24; N, 7.41. Found: C, 57.07; H, 5.22; N, 7.13.

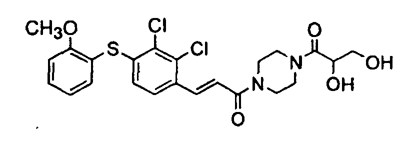

### Example 364

### (2-Methoxyphenyl) [2,3-dichloro-4-(E-(4-(2,3-dihydroxypropionyl)piperazin-1-yl)carbonyl)ethenyl)phenyl]sulfide

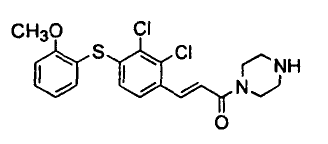

### Example 364A

### (2-Methoxyphenyl)[2,3-dichloro-4-(E-((piperazin-1-yl)carbonyl)ethenyl)phenyl]sulfide

The title compound was prepared by the procedures described in Example 340G substituting methyl isonipecotate with piperazine. MS (DCI/NH₃) *m*/*e* 423 (M+H)⁺.

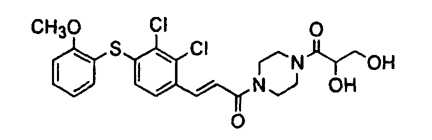

### Example 364B

### (2-Methoxyphenyl)[2,3-dichloro-4-(E-(4-(2,3-dihydroxypropionyl)piperazin-1-yl)carbonyl)ethenyl)phenyl]sulfide

The title compound was prepared by the procedures described in Example 340, substituting methyl isonipecotate with Example 364A and substituting Example 340G with DL-glyceric acid Ca salt. ¹H NMR (300 MHz, DMSO-d₆) δ 3.2-3.8 (m, 12H), 4.38 (t, 1H), 6.58 (d, 1H), 7.10 (t, 1H), 7.27 (d, 1H), 7.28 (d, 1H), 7.50 (d, 1H), 7.60 (t, 1H), 7.79 (d, 1H), 7.83 (d, 1H). MS (ESI⁺) *m*/*z* 511 (M+H)⁺.

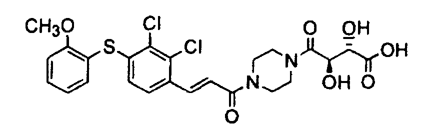

### Example 365

### (2-Methoxyphenyl)[2,3-dichloro-4-(E-(4-(2,3-dihydroxy-3-carboxypropionyl)piperazin-1-yl)carbonyl)ethenyl)phenyl]sulfide

The title compound was prepared by the procedures described in Example 340 substituting methyl isonipecotate with Example 364A and substituting Example 340G with meso-tartaric acid. ¹H NMR (300 MHz, CDCl₃) δ 3.70 (m, 8H), 4.33 (br s, 1H), 4.72 (br s, 1H), 6.58 (d, 1H), 6.77 (d, 1H), 7.03 (m, 2H), 7.25(d, 1H), 7.50 (d, 1H), 7.52 (d, 1H), 8.00 (d, 1H). MS (ESI⁺) *m*/*z* 555 (M+H)⁺.

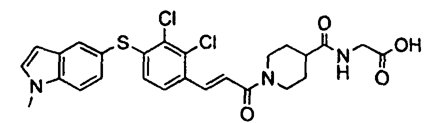

### Example 366

### (1-Methylindol-5-yl)[2,3-dichloro-4-(E-((4-(carboxymethylamino)carbonylpiperidin-1-yl)carbonyl)ethenyl)phenyl]sulfide

The title compound was prepared by the procedures described in Example 363 substituting 3-amino-1,2-propanediol with glycine methyl ester hydrochloride followed by hydrolysis. ¹H NMR (300 MHz, DMSO-d₆) δ 1.42 (m, 2H), 1.75 (m, 2H), 2.45 (m, 1H), 2.78 (m, 1H), 3.10 (m, 1H), 3.72 (d, 2H), 3.90 (s, 3H), 4.18 (br d, 1H), 4.40 (br d, 1H), 6.42 (d, 1H), 6.57 (d, 1H), 7.18 (d, 1H), 7.32 (d, 1H), 7.50 (d, 1H), 7.65 (d, 1H), 7.67 (d, 1H), 7.70 (m, 1H), 7.88 (s, 1H), 8.18 (t, 1H). MS (ESI⁺) *m*/*z* 546 (M+H)⁺. Anal. calcd for C₂₆H₂₅N₃Cl₂SO₄: C, 57.15; H, 4.61; N, 7.69. Found: C, 57.17; H, 4.64; N, 7.39.

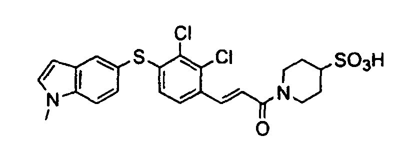

### Example 367

### (1-Methylindol-5-yl)[2,3-dichloro-4-(E-((4-sulfopiperidin-1-yl)carbonyl)ethenyl)phenyl]sulfide

The title compound was prepared from Example 340F, by the procedures described in Example 340G, substituting methyl isonipecotate with piperadine-4-sulfonic acid. ¹H NMR (300 MHz, DMSO-d₆) δ 1.40 (m, 2H), 1.90 (m, 2H), 3.03 (m, 1H), 4.10 (m, 3H), 4.42 (br d, 1H), 6.40 (d, J=8.8Hz, 1H), 6.53 (d, J=3.1Hz, 1H), 7.15 (d, J=15.3Hz, 1H), 7.33 (dd, J=8.5, 1.7Hz, 1H), 7.48 (d, J=3.1Hz, 1H), 7.65 (d, J=8.5Hz, 1H), 7.67 (d, J=15.2Hz, 1H), 7.74 (d, J=8.8Hz, 1H), 7.87 (d, J=1.5Hz). MS (ESI⁺) *m*/*z* 525 (M+H)⁺. Anal. calcd for C₂₃H₂₂N₂Cl₂S₂O₄·0.8 TFA: C, 47.91; H, 3.73; N, 4.54. Found: C, 47.71; H, 3.84; N, 4.73.

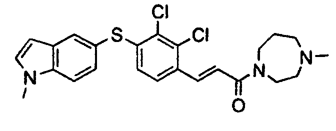

### Example 368

### (1-Methylindol-5-yl)[2,3-dichloro-4-(E-(4-methylhomopiperazin-1-ylcarbonyl)ethenyl)phenyl]sulfide

The title compound was prepared by the procedures described in Example 340G substituting methyl isonipecotate with N-methyl homopiperazine. ¹H NMR (300 MHz, DMSO-d₆) δ 2.06 (m, 2H), 2.81 (m, 2H), 3.17 (m, 2H), 3.55 (m 3H), 3.70 (s, 3H), 3.86 (s, 3H), 4.05 (m, 1H), 6.42 (dd, J=8.4,3.3Hz, 1H), 6.54 (d, J=3.0Hz, 1H), 7.08 (dd, J=15.4,7.5Hz, 1H), 7.35 (dd, J=8.8,2.0Hz, 1H), 7.49 (d, J=3.0Hz, 1H), 7.65 (d, J=8.5Hz, 1H), 7.73 (d, J=8.8Hz, 1H), 7.80 (d, J=15.2Hz, 1H), 7.88 (d, J=2.0Hz, 1H). MS (ESI⁺) *m*/*z* 474 (M+H)⁺. Anal. calcd for C₂₆H₂₆N₃Cl₂SF₃O₃·0.75 TFA: C, 49.01; H, 4.00; N, 6.23. Found: C, 48.71; H, 4.09; N, 6.13.

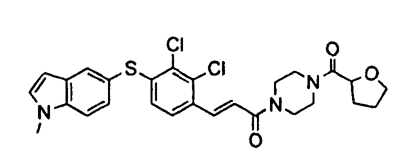

### Example 369

### (1-Methylindol-5-yl) [2,3-dichloro-4-(E-(4-tetrohydrofuroylpiperazin-1-yl)carbonyl)ethenyl)phenyl]sulfide

The title compound was prepared by the procedures described in Example 340G substituting methyl isonipecotate with 1-tetrahydrofuroylpiperazine. ¹H NMR (300 MHz, DMSO-d₆) δ 1.80 (m, 2H), 2.00 (m, 2H), 3.50 (m, 8H), 3.75 (m, 2H), 3.88 (s, 3H), 4.68 (t, 1H), 6.42 (d, 1H), 6.57 (d, 1H), 7.19 (d, 1H), 7.32 (d, 1H), 7.48 (d, 1H), 7.65 (d, 1H), 7.70 (d, 1H), 7.75 (d, 1H), 7.87 (s, 1H). MS (ESI⁺) *m*/*z* 544 (M+H)⁺. Anal calcd for C₂₇H₂₇N₃Cl₂SO₃: C, 59.56; H, 4.99; N, 7.71. Found: C, 59.40; H, 4.94; N, 7.61.

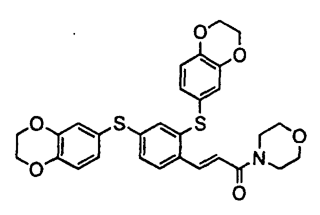

### Example 370

### (Benzodioxan-6-yl)[2-(benzodioxan-6-thioxy)-4-(E-((4-morpholino)carbonyl)ethenyl)phenyl]sulfide

### Example 370A

### (E)-Morpholino 2,4-difluorocinnamide

The title compound was processed as reported in Example 1 C substituting morpholine (1.04 mL, 11.9 mmol) for the amine and *trans*-2,4-difluorocinnamic acid (1.00 g, 5.4 mmol) for the carboxylic acid. The title compound was obtained as an off-white foam (1.4 g, 100%). ¹H NMR (DMSO-d₆, 300 MHz) d 8.04 (dd, J=15.26, 8.82 Hz, 1H), 7.53 (d, J=14.91 Hz, 1H), 7.38-7.30 (m, 1H), 3.61-3.48 (m, 8H). MS (APCI) *m*/*z* 254 (M+H)⁺.

### Example 370B

### Morpholinyl-(E)-2,4-bis (1,4-benzodioxane-6-mercaptan)cinnamic amide

Example 370A (233 mg, 1.00 mmol) was combined with cesium carbonate (652 mg, 2.00 mmol), 1,4-benzodioxane-6-thiol (370 mg, 2.20 mmol), and DMF (5 mL). The mixture was processed as reported in Example 1 A to provide the title compound (220 mg, 40%) as a white foam. ¹H NMR (DMSO-d₆, 300 MHz) δ 7.83 (d, J=15.20 Hz, 1H), 7.80 (d, J=8.20 Hz, 1H), 7.17 (d, J=15.3 Hz, 1H), 7.02 (dd, J=8.5, 2.0 Hz, 1H), 6.87-6.75 (m, 6H), 6.48 (s, 1H), 4.33-4.25 (m, 8H), 3.61-3.48 (m, 8H). MS (APCI) *m*/*z* 550 (M+H)⁺.

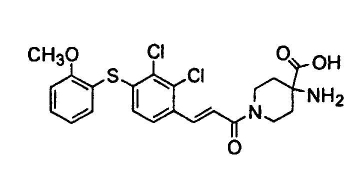

### Example 371

### (2-Methoxyphenyl)[2,3-dichloro-4-(E-((4-amino-4-carboxypiperidin-1-yl)carbonyl)ethenyl)phenyl]sulfide

To a suspension of Example 355 (700 mg, 1.4 mmol) in DME (10 mL) was added a solution of (BOC)₂O (1.51 g, 6.9 mmol) in DME (5 mL), triethylamine (0.23 mL, 1.7 mmol) and DMAP (9 mg, 0.07 mmol). The reaction mixture was stirred at room temperature overnight. Additional triethylamine (0.23 mL) and DMAP (30 mg) were added, and the mixture was heated at 60 °C for 6 hours. After aqueous work up, the crude product was suspended in DME (5 mL) and water (5 mL) containing 200 mg of NaOH. The suspension was stirred for 5 hours at room temperature, and separated by HPLC to give the title compound (300 mg, 45%). ¹H NMR (300 MHz, DMSO-d₆) δ 1.78 (m, 2H), 2.10 (m, 2H), 3.60 (m, 2H), 3.80 (s, 3H), 3.86 (m, 2H), 6.58 (d, 1H), 7.10 (d, 1H), 7.25 (d, 1H), 7.28 (d, 1H), 7.50 (d, 1H), 7.58 (t, 1H), 7.77 (d, 1H), 7.80 (d, 1H), 8.50 (br s, 2H). MS (ESI⁺) *m*/*z* 481 (M+H)⁺. Anal calcd for C₂₂H₂₂N₂Cl₂SO₄·0.75 H₂O: C, 47.34; H, 4.06; N, 4.60. Found: C, 47.31; H, 4.05; N, 4.43.

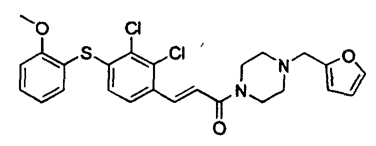

### Example 372

### (2-Methoxyphenyl)[2,3-dichloro-4-((4-furoylpiperazin-1-yl)carbonyl)ethenyl)phenyl]sulfide

To a solution of Example 364A (100mg, 0.24 mmol) and 2-furfural (30mg, 0.24 mmol) in dichloroethane (2 mL) was added NaBH(OAc)₃ (142 mg, 0.67 mmol) under nitrogen atmosphere. The mixture was stirred for 16 hours at room temperature. Dichloromethane (20 mL) was added and the mixture was washed with 5% NaHCO₃, then with brine, and the organic phase was separated and concentrated. The residual solid was chromatographed by flash chromatography (5% MeOH/CH₂CL₂) and desired fractions were combined, concentrated and dried to afford the title compound as an off-white solid (84 mg, 69%). HPLC (Supelco C-18 column, water:acetonitrile 100:0- 0:100, 15 minute elution, flow rate 1.5 mL/min) rt 11.90 min. ¹H NMR (300 MHz, DMSO-d₆) δ 2.39 (m, 4H), 3.52 (s, 2H), 3.55 (m, 2H), 3.63 (m, 2H), 3.79 (s, 3H), 6.29 (d, 1H), 6.40 (m, 1H), 6.57 (d, 1H), 7.08 (dt, 1H), 7.21 (d, 1H), 7.23 (dd, 1H), 7.48 (dd, 1H), 7.57 (m, 2H), 7.72 (d, 1H), 7.80 (d, 1H). MS (ESI) *m*/*e* 503 (M+H)⁺.

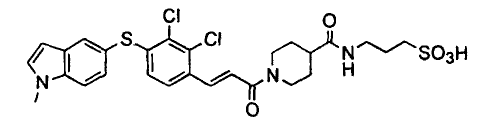

### Example 373

### (1-Methylindol-5-yl)[2,3-dichloro-4-(E-(4-(carbo-3-sulfopropylamino)piperadin-1-yl)carbonyl)ethenyl)phenyl]sulfide

The title compound was prepared from Example 340H by the procedures described in Example 363 substituting 3-amino-1,2-propanediol with 3-amino-1-propanesulfonic acid. ¹H NMR (300 MHz, DMSO-d₆) δ 1.40 (m, 2H), 1.70 (m, 4H), 2.38 (m, 1H), 2.42 (m, 2H), 2.70 (m, 1H), 3.05 (m, 3H), 3.86 (s, 3H), 4.18 (br d, 1H), 4.40 (br d, 1H), 6.40 (d, 1H), 6.55 (d, 1H), 7.20 (d, 1H), 7.35 (d, 1H), 7.50 (d, 1H), 7.65 (d, 1H), 7.70 (d, 1H), 7.77 (d, 1H), 7.87 (d, 1H). MS (ESI⁺) *m*/*z* 610 (M+H)⁺. Anal calcd for C₂₇H₂₉N₃Cl₂S₂O₅·1.5 TFA: C, 46.10; H, 3.93; N, 5.38. Found: C, 46.52; H, 4.03; N, 5.66.

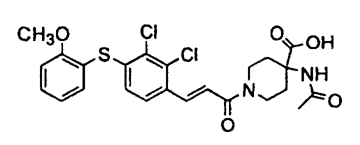

### Example 374

### (2-Methoxyphenyl)[2,3-dichloro-4-(E-(4-acetylamino-4-carboxypiperidin-1-ylcarbonyl)ethenyl)phenyl]sulfide

To a suspension of Example 371 (90 mg, 0.187 mmol) and triethylamine (0.08 mL, 0.57 mmol) in DMF (3 mL) was added acetyl chloride (0.1 mL) at room temperature. The mixture was stirred for 3 hours. Ethyl acetate (60 mL) was added, and the mixture was washed with brine. The organic phase was dried, filtered and concentrated. The residue was separated by HPLC (C-18, CH₃CN/H₂O) to give example 374 (56 mg, 57%).
¹H NMR (300 MHz, DMSO-d₆) δ 1.78 (m, 2H), 1.82 (s, 3H), 1.98 (m, 2H), 3.05 (t, 1H), 3.38 (t, 1H), 3.80 (s, 3H), 4.00 (br d, 1H), 4.12 (br d, 1H), 6.58 (d, 1H), 7.08 (t, 1H), 7.23 (d, 1H), 7.25 (d, 1H), 7.50 (d, 1H), 7.58 (t, 1H), 7.78 (d, 1H), 7.80 (d, 1H), 8.18 (s, 1H). MS (ESI⁺) *m*/*z* 523 (M+H)⁺. Anal calcd for C₂₄H₂₄N₂Cl₂SO₅·0.35TFA: C, 52.80; H, 4.40; N, 5.05. Found: C, 52.74; H, 4.42; N, 5.11.

### Example 375

### (2-Methoxyphenyl)[2,3-bis(trifluoromethyl)-4-(E-((4-carboxypiperidin-1-yl)carbonyl)ethenyl)phenyl]sulfide

The title compound was prepared by the procedures described in Example 352, employing the compound of Example 359D to give a white solid. ¹H NMR(CD₃OD, 300 MHz) δ 1.65 (br s, 2H), 1.94-2.03 (m, 2H), 2.57-2.67 (m, 1H), 2.95-3.05 (m, 1H), 3.23-3.32 (m, 1H), 3.75 (s, 3H), 4.12 (br s, 1H), 4.40 (br s, 1H), 7.00 (d, J=15Hz, 1H), 7.03-7.20 (m, 3H), 7.47-7.53 (m, 2H), 7.68 (d, J=9Hz, 1H), 7.77 (qq, J=15Hz, 1H). MS (ESI) *m*/*z* 534 (M+H)+. Anal calcd for C₂₄H₂₁NF₆O₄S: C, 54.03; H, 3.97; N, 2.63. Found: C, 54.11; H, 4.04; N, 1.76.

### Example 376

### (2-Methoxyphenyl) 5-[8-(E-((4-(aminocarbonyl)piperidin-1-yl)carbonyl)ethenyl)quinolinyl]sulfide

### Example 376A

### 5-Chloro-8-(trifluoromethanesulfonyloxy)quinoline

5-Chloro-8-hydroxyquinoline was treated as described in Example 340E to provide the title compound. ¹H NMR (DMSO-d₆, 300 MHz) δ 7.59 (7.5Hz, 1H), 7.65-7.69 (m,2H), 8.63 (dd, J₁=9Hz, J₂=1.5Hz, 1H), 9.21 (dd, J₁=6Hz, J₂=1.5Hz, 1H). MS (APCI-NH₃) *mle* 312, 314 (M+H)⁺.

### Example 376B

### 5-Chloro-8-[E-(methoxycarbony)ethenyl]quinoline

The method of Example 340D was used, substituting the product from Example 376A for Example 340C. Thus, Example 376A (6.23 g, 20.0 mmol) was converted to the title compound (2.22 g, 45%). ¹H NMR (DMSO-d₆, 300 MHz) δ 3.78 (s, 3H), 6.98 (d, J=16.5Hz, 1H), 7.78-7.83 (m, 1H), 7.88 (d, J=9Hz, 1H), 8.32 (d, J=9Hz, 1H), 8.65 (dd, J₁=9Hz, J₂=1.5Hz, 1H), 8.85 (d, J=16.5 Hz, 1H), 9.12 (dd, J₁=4.5Hz, J₂=1.5Hz, 1H). MS (APCI-NH₃) *m*/*e* 248, 250 (M+H)⁺.

### Example 376C

### (2-Methoxyphenyl)5-[8-(E-(methoxycarbonyl)ethenyl)quinolinyl]sulfide

The method of Example 340F was used, substituting the product from Example 376B for Example 340E. Thus, Example 376B (2.19 g, 8.84 mmol) was converted to the title compound (1.07 g, 36%). ¹H NMR (DMSO-d₆, 300 MHz) δ 3.83 (s, 3H), 6.80 (d, J=16.5Hz, 1H), 6.86-6.99 (m, 2H), 7.16 (d, J=6Hz, 1H), 7.33-7.38 (m, 1H), 7.44 (d, J=7.5Hz, 1H), 7.67-7.72 (m,1H), 8.22 (d, J=7.5Hz, 1H), 8.63 (dd, J₁=9Hz, J₂=1.5Hz, 1H), 8.82 (d, J=16.5Hz, 1H), 9.07 (dd, J₁=6Hz, J₂=1.5Hz), 12.48 (s, 1H). MS (APCI-NH₃) *mle* 338 (M+H)⁺.

### Example 376C

### (2-Methoxyphenyl) 5-[8-(E-((4-(aminocarbonyl)piperidin-1-yl)carbonyl)ethenyl)quinolinyl]sulfide

The method of Example 340G was used, substituting the product from Example 376B for Example 340F, and substituting 4-piperidinecarboxamide for methyl isonipecotate. ¹H NMR (DMSO-d₆, 300 MHz) δ 1.71-2.82 (m, 2H), 2.96-2.03 (m, 2H), 2.44-2.52 (m, 1H), 2.81-2.94 and 3.16-3.30 (m, 1H), 3.37-3.54 (m, 2H), 3.88 (s, 3H), 4.17-4.34 and 4.60-4.80 (m, 1H), 5.72 (s, 2H), 6.82 (t, 4.5Hz, 1H), 6.90 (dd, J₁=4.5Hz, J₂=0.75Hz, 1H), 6.93 (d, 6Hz, 1H), 7.23-7.28 (m, 1H), 7.40 (d, J=9Hz, 1H), 7.47-7.50 (m, 1H), 7.51 (d, J=6Hz, 1H), 7.82 (d, J=4.5 Hz, 1H), 8.57 (d, J=9Hz, 1H), 8.74 (dd, J₁=4.5Hz, J₂=0.75Hz, 1H), 9.00 (m, 1H).

### Example 377

### (2-Methoxyphenyl) [2-trifluoromethyl-4-(E-((4-carboxypiperidin-1-yl)carbonyl)ethenyl)phenyl]sulfide

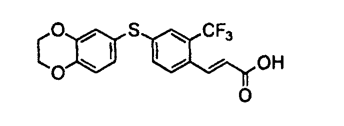

### Example 377A

### 2-Trifluoromethyl-4-(thiobenzodioxan-6-yl)cinnamic acid

A solution of commercially available 4-fluoro-2-(trifluoromethyl)cinnamic acid (5 g, 21.4 mmol) in ethyl acetate (200 mL) under nitrogen at ambient temperature was treated with a solution of diazomethane in diethyl ether to a persistent yellow color, stirred an additional ten minutes, then quenched by dropwise addition of glacial acetic acid. The resultant clear solution was washed with saturated NaHCO₃, brine, dried (MgSO₄), filtered through a plug of silica, rinsed with ethyl acetate and concentrated to give 5.4 grams of a yellow oil. A solution of this methyl ester (2.5 g,10 mmol) and 6-mercaptobenzodioxane (1.9 g, 11mmol) in 40 mL of dimethylformamide was treated with cesium carbonate (3.9 g, 12 mmol), and stirred at room temperature for 20 hours. The resultant orange heterogeneous solution was diluted with diethyl ether and water, washed with 1 M NaOH, distilled water, brine, dried (MgSO₄), filtered through a plug of silica, concentrated and then flash chromatographed with 20% ethyl acetate/hexane followed by 33% ethyl acetate/hexane to give 2.8 g of a light yellow syrup. A solution of this diaryl sulfide ester (2.8 g, 7.1 mmol) in THF (21 mL) and distilled water (7 mL) was treated with lithium hydroxide hydrate (450 mg, 10.7 mmol) and stirred 67 hours at ambient temperature. The resultant solution was diluted with distilled water, washed with diethyl ether, acidified to pH 1-2 with 3 M H₂SO₄, extracted with diethyl ether, washed with brine, dried (MgSO₄) and concentrated to give 2.7 g (7.1 mmol) of the title compound as an off-white powder (71%). ¹H NMR (300 MHz, d6-DMSO) δ 7.97 (d, 1H), 7.72 (dq, 1H), 7.47 (d, 1H), 7.31 (dd, 1H), 7.05 (m, 3H), 6.58 (d, 1H), 4.3 (m, 4H). MS (APCI-NH₃) *m*/*e* 383 (M+H)⁺, 400 (M+NH₄)⁺.

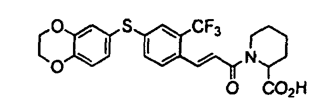

### Example 377B

### (Benzodioxan-6-yl)[3-trifluoromethyl-4-(E-((2-carboxypiperidin-1-yl)carbonyl)ethenyl)phenyl]sulfide

Example 377A (382 mg, 1 mmol) was coupled with (d,l)-ethyl pipicolinate according to the procedure of Example 340G. The derived ethyl ester was hydrolyzed using the method of Example 340H to give 280 mg of the title compound as a light yellow foam (84%). Analytical HPLC: 4.6X250 mm C18 column, 0.8 mL/min, 254 nm, CH₃CN:H₂O with 0.1% TFA, 0:100 (0 min), ramp to 90:10 (0-10 min), 90:10 (10-18 min), ramp to 0:100 (18-20 min), rt 11.29 min (98.2 area%). ¹H NMR (300 MHz, d6-DMSO) δ 8.07 (t, 1H), 7.65 (dq, 1H), 7.38 (m, 3H), 7.03 (m, 3H), 5.15 (m, 1H), 4.4 (m, 1H), 4.29 (m, 4H), 4.1 (m, 1H), 3.2 (m, 1H), 2.2 (m, 1H), 1.68 (m, 2H), 1.3 (m, 2H). MS (APCI-NH₃) *m*/*e* 494 (M+H)⁺, 511 (M+NH₄)⁺.

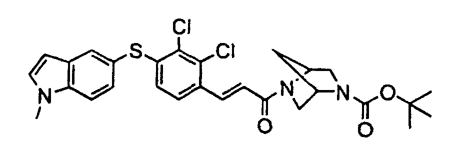

### Example 378

### (1-Methylindol-5-yl)[2,3-dichloro-4-(E-(((1S,4S)-5-tert-butyloxycarbonyl-2,5-diazabycyclo(2,2,1)heptan-2-yl)carbonyl)ethenyl)phenyl]sulfide

The title compound was prepared by the procedures described in Example 340 substituting methyl isonipecotate with t-butyl (1S,4S)-(-)2,5-diazabicyclo(2,2,1)heptane-2-carboxylate. ¹H NMR (300 MHz, DMSO-d₆) δ 1.40 (s, 9H), 1.82 (m, 2H), 3.17 (m, 1H), 3.30 (m, 2H), 3.58 (m, 1H), 3.82 (s, 3H), 4.05 (m, 1H), 4.40 (m, 1H), 4.75 (br s, 1H), 4.92 (br s, 1H), 6.42 (dd, 1H), 6.58 (d, 1H), 6.75 (d, 1H), 7.05 (d, 1H), 7.35 (d, 1H), 7.50 (d, 1H), 7.65 (d, 1H), 7.68 (d, 1H), 7.78 (t, 1H), 7.77 (s, 1H). MS (ESI⁺) *m*/*z* 558 (M+H)⁺. Anal calcd for C₂₈H₂₉N₃Cl₂SO₃: C, 60.21; H, 5.23; N, 7.52. Found: C, 60.23; H, 5.36; N, 7.41.

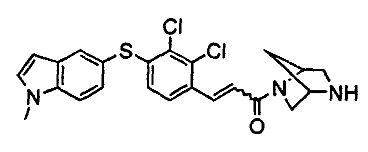

### Example 379

### (1-Methylindol-5-yl)[2,3-dichloro-4-(E/Z-((1S,4S)-2,5-diazabycyclo(2,2,1)heptan-2-ylcarbonyl)ethenyl)-2,3-dichlorophenyl]sulfide

To a solution of Example 378 (820 mg, 1.47 mmol) in CH₂Cl₂ (20 mL) was added trifluoroacetic acid (2mL) at 0°C. The yellow solution was stirred at the same temperature for 2 hours. More CH₂Cl₂ (50 mL) was added and the solution was poured into water (100 mL) containing NaHCO₃ (4.5 g). The insoluble material was collected by filtration, washed with water and methanol. The CH₂Cl₂ solution was concentrated, and the residual solid was filtered, washed with water, methanol and CH₂Cl₂. The combined solid was dried to give the title compound (650 mg, 95%). ¹H NMR (300 MHz, DMSO-d₆) δ 1.70 (m, 2H), 2.90 (m, 1H), 3.50 (m, 4H), 3.88 (s, 3H), 4.85 (m, 1H), 6.45 (d, 1H), 6.60 (dd, 1H), 6.77 (d, 1H), 7.05 (dd, 1H), 7.25 (s, 1H), 7.35 (dd, 1H), 7.65 (d, 1H), 7.70 (d, 1H), 7.80 (d, 1H). MS (ESI⁺) *m*/*z* 458(M+H)⁺.

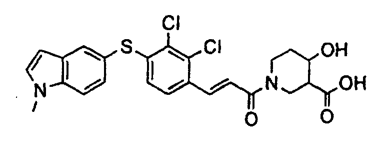

### Example 380

### (1-Methylindol-5-yl)[2,3-dichloro-4-(E-(4-hydroxy-3-carboxypiperadin-1-ylcarbonyl)ethenyl)phenyl]sulfide

To a suspension of Example 340G (300 mg, 0.794 mmol) and methyl 4-oxo-3-piperadine carboxylate hydrochloride (307 mg, 1.59 mmol) in DMF (10 mL) was added EDC (305 mg, 1.59 mmol), HOBt (215 mg, 1.59 mmol) and triethylamine (0.443 mL, 1.59 mmol). The suspension was stirred at room temperature overnight. Ethyl acetate (100 mL) was added and the mixture was washed with brine, water and was concentrated. The residual oil was separated by flash chromatography (60% EtOAc in hexane) to give a white solid (220 mg).

180 mg of this solid was dissolved in THF (10 mL). A solution of lithium hydroxide monohydrate (29 mg, 0.68 mmol) in water (10 mL) was added. The mixture was stirred at room temperature 2 hours, NaBH₄ (50 mg) was then added. After 4 hours stirring, the solution was acidified and concentrated to 5 mL. The formed white solid was collected by filtration, washed with water, acetonitrile, and dried to give the title compound (92 mg). ¹H NMR (300 MHz, DMSO-d₆) δ 1.60 (m, 2H), 3.00 (m, 1H), 3.40 (m, 1H), 3.85(1 H, 4.05 (m, 1H), 4.20 (m, 1H), 4.35 (m, 1H), 5.00 (m, 1H), 6.42 (d, 1H), 6.58 (d, 1H), 7.20 (dd, 1H), 7.35 (d, 1H), 7.50 (d, 1H), 7.6-7.8 (m, 3H), 7.90 (s, 1H). MS (ESI⁺) *m*/*z* 505 (M+H)⁺. Anal calcd for C₂₄H₂₂N₂Cl₂SO₄: C, 57.03; H, 4.38; N, 5.54. Found: C, 56.77; H, 4.17; N, 5.34.

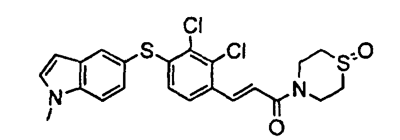

### Example 381

### (1-Methylindol-5-yl)[2,3-dichloro-4-(E-(S-oxothiomorpholin-1-ylcarbonyl)ethenyl)phenyl]sulfide

The title compound was prepared by the procedures described in Example 340 substituting methyl isonipecotate with thiomorpholine S-oxide. ¹H NMR (300 MHz, CDCl₃) δ 2.70 (m, 2H), 2.85 (m, 2H), 3.85 (s, 3H), 3.90 (m, 2H), 4.20 (m, 1H), 4.60 (m, 1H), 6.45 (d, 1H), 6.55 (d, 1H), 6.70 (d, 1H), 7.18 (d, 1H), 7.20 (d, 1H), 7.38 (d, 1H), 7.41 (d, 1H), 7.77 (s, 1H), 7.98 (d, 1H). MS (ESI⁺) *m*/*z* 479 (M+H)⁺.

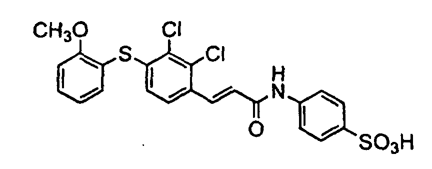

### Example 382

### (2-Methoxyphenyl)[2,3-dichloro-4-(E-((4-sulfophenylamino)carbonyl)ethenyl)phenyl]sulfide

The title compound was prepared by the procedures described in Example 1C substituting Example 1B with (2-methoxy) [2,3-dichloro-4-(*E*-(2-carboxyethenyl)phenyl]sulfide and substituting 6-amino-1-hexanol with sulfanilic acid. ¹H NMR (300 MHz, DMSO-d₆) δ 3.82 (s, 3H), 6.65 (d, 1H), 6.82 (d, 1H), 7.12 (t, 1H), 7.25 (d, 1H), 7.5-7.7 (m, 7H), 7.85 (d, 1H), 10.40 (s, 1H). MS (ESI⁺) *m*/*z* 510 (M+H)⁺. Anal calcd for C₂₂H₁₇Cl₂NS₂O₅.0.65TFA: C, 50.80; H, 3.25; N, 2.55. Found: C, 50.75; H, 3.43; N, 2.65.

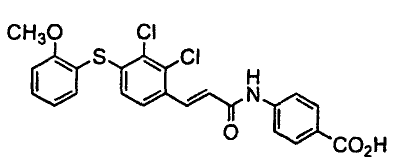

### Example 383

### (2-Methoxyphenyl) [2.3-dichloro-4-(E-((4-carboxyphenylamino)carbonyl)ethenyl)phenyl]sulfide

The title compound was prepared by the procedures described in Example I C substituting Example 1B with (2-methoxy) [2,3-dichloro-4-(*E*-(2-carboxyethenyl)phenyl]sulfide and substituting 6-amino-1-hexanol with 4-aminobenzoic acid. ¹H NMR (300 MHz, DMSO-d₆) δ 3.82 (s, 3H), 6.65 (d, 1H), 6.82 (d, 1H), 7.10 (t, 1H), 7.30 (d, 1H), 7.60 (m, 3H), 7.82 (t, 3H), 7.90 (d, 1H), 7.92 (d, 1H), 10.65 (s, 1H), 12.75 (s, 1H). MS (ESI⁺) *m*/*z* 474 (M+H)⁺.

### Example 384

### [3-(4-Morpholino)phenyl][2,3-dichloro-4-(E-((4-carboxypiperidin-1-yl)carbonyl]ethenyl)phenyl]sulfide

### Example 384A

### (3-Bromophenyl)[2,3-dichloro-4-(E-[methoxycarbonyl]ethenyl)phenyl]sulfide

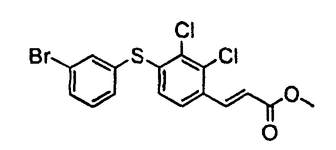

### Example 384A

To a solution of the resultant compound from Example 340E (12.0 g, 31.7 mmol) in N-methylpiperidinone (63 mL) at 0 °C (under dry N₂) was added 3-bromothiophenol (4.0 mL, 7.3 g, 38.8 mmol) and a solution of lithium tert-butoxide (3.1 g, 38.8 mmol), and the resulting solution was stirred for 3 hr at 0 °C. The reaction was diluted with 500 mL EtOAc and extracted sequentially with 100 mL water, 3 x 60 mL of 1 N aq. NaOH, then 2 x 100 mL brine. The organic phase was dried over Na2SO4, filtered, and concentrated in vacuo to produce the crude title compound (9.2 g). ¹H NMR (DMSO-d₆, 300 MHz) δ 3.75 (s, 3H), 6.67 (d, 15Hz, 1H), 6.83 (d, J-9Hz, 1H), 7.46-7.59 (m, 2H), 7.72-7.76 (m, 2H), 7.80 (t, J=2.5Hz, 1H), 7.85 (d, J=9Hz, 1H), 7.88 (d, J=15Hz, 1H). MS (APCI) m/e 419 (M+H)⁺.

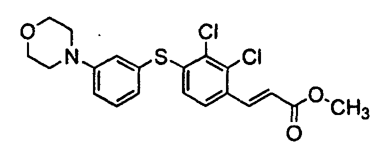

### Example 384B

### [3-(4-Morpholino)phenyl][2,3-dichloro-4-(E-[methoxycarbonyl]ethenyl)phenyl]sulfide

The procedure of Old, D. W.; Wolfe, J. P.; Buchwald, S. L. *J. Am. Chem. Soc.* **1998**, *120*, 9722-9723, was adapted. To a stirred solution of Example 384A (200 mg, 0.479 mmol) in ethylene glycol dimethyl ether (1 mL) with 1-(N,N-dimethylamino)-1'-(dicyclohexylphophino)biphenyl (14 mg, 7.5 mol%), Pd₂(dba)₃ (11 mg, 2.5 mol%), and morpholine (0.05 ml, 0.574 mmol) was added powdered K₃PO₄ (142 mg, 0.67 mmol). The reaction mixture was bubbled with N₂ for 5 minutes and heated at 90 °C in sealed tube for 18 hours. This was allowed to cool to ambient temperature, diluted with ethyl acetate (5 mL) and washed with brine (2x3 mL). The dried (Na₂SO₄) organic layer was evaporated under reduced pressure to obtain the crude product (260 mg). The title compound (80 mg, 39%) was isolated by flash chromatography on silica gel eluting with 7.5 %acetone hexane. ¹H NMR (DMSO-d₆, 300 MHz) δ 3.04 - 3.07 (m, 2H), 3.13-3.14 (m, 2H), 3.69-3.78, m, 7H), 6.60-6.70 (m, 2H), 6.96-7.01 (m, 1H), 7.11-7.21 (m, 2H), 7.36-7.44 (m, 1H), 7.78-7-94 (m, 2H). MS (ESI) *m*/*e* 424, 426 (M+H)⁺.

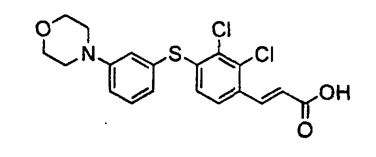

### Example 384C

### [3-(4-Morpholino)phenyl] [2,3-dichloro-4-(E-[carboxy]ethenyl)phenyl]sulfide

The title compound (42 mg, 55%) was prepared by treatment of Example 384B (80 mg, 0.189 mmol) with LiOH (27 mg, 0.566 mmol) as described for Example 340H. ¹H NMR (DMSO-d₆, 300 MHz) δ 3.04-3.08 (m, 2H), 3.13-3.19 (m,2H), 3.70-3.78 (m, 4H), 6.53 (d, J=15Hz, 1H), 6.55 (d, J=15.75Hz, 1H), 6.67 (d, J=8.25Hz, 1H), 7.10-7.20 (m, 2H), 7.77-7.91 (m, 3H). MS (ESI) m/e 410,412 (M+H)⁺.

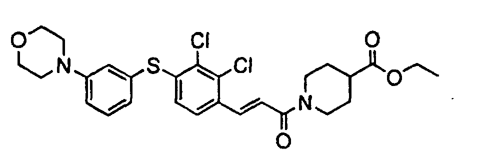

### Example 384D

### [3-(4-Morpholino)phenyl] [2,3-dichloro-4-(E-[(4-ethoxycarbonylpiperidin-1-yl)carbonyl]ethenyl)phenyl]sulfide

To stirred a solution of Example 384C (40 mg, 0.098 mmol) in N,N-dimethylformamide (1 mL) containing HOBt^{·}H₂O (23 mg, 0.146 mmol), N-methylmorpholine (0.032 mL, 0.293 mmol) and ethyl isonipecotate (0.018 mL, 0.117 mmol) was added EDCI (28 mg, 0.146 mmol) at 0 °C and stirred at ambient temperature for 12 hours. The reaction mixture was diluted with ethyl acetate (15 mL), washed with brine (2x6 mL), dried (Na₂SO₄) and evaporated to dryness under reduced pressure. The title compound (40 mg, 78%) was obtained by flash chromatography on silica gel eluting with 20 %acetone hexane. ¹H NMR (DMSO-d₆, 500 MHz) δ 1.18 (m, 3H), 1.40-1.53 (m, 2H), 1.82-1.93 (m, 2H), 2.60-2.68 (m, 1H), 2.82-2.91 (2H), 3.05 (t, J=5Hz, 2H), 3.15 (t, J=5Hz, 2H), 3.73 (t, J=5Hz, 2H), 3.78 (t, J=5Hz, 2H), 4.02-4.10 (m,, 2H), 4.12-4.35 (m, 2H), 6.72 (d, J=10Hz, 1H), 6.97 (m, 1H), 7.10-7.27 (m, 2H), 7.38 (m, 1H), 7.73-7.80 (m, 1H), 7.85 (d, J=8.75 Hz, 1H), 7.96 (d, J-10Hz, 1H).

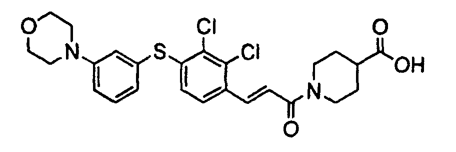

### Example 384E

### [3-(4-Morpholino)phenyl][2,3-dichloro-4-(E-[(4-carboxypiperidin-1-yl)carbonyl]ethenyl)phenyl]sulfide

Using the procedure of Example 340H, Example 384D is hydrolyzed to the title compound.

Compounds that antagonize the interaction between ICAM-1 and LFA-1 can be identified, and their activities quantitated, using both biochemical and cell-based adhesion assays. A primary biochemical assay measures the ability of the compound in question to block the interaction between the integrin LFA-1 and its adhesion partner ICAM-1, as described below:

### ICAM-1 / LFA-1 Biochemical Interaction Assay

In the biochemical assay, 100 µL of anti-LFA-1 antibody (ICOS Corporation) at a concentration of 5 µg/ml in Dulbecco's phosphate-buffered saline (D-PBS) is used to coat wells of a 96-well microtiter plate overnight at 4°C. The wells are then washed twice with wash buffer (D-PBS w/o Ca⁺⁺ or Mg⁺⁺, 0.05% Tween 20) and blocked by addition of 200 µL of D-PBS, 5% fish skin gelatin. Recombinant LFA-1 (100 µL of 0.7 µg/ml, ICOS Corporation) in D-PBS is then added to each well. Incubation continues for 1 hour at room temperature and the wells are washed twice with wash buffer. Serial dilutions of compounds being assayed as ICAM-1/LFA-1 antagonists, prepared as 10 mM stock solutions in dimethyl sulfoxide (DMSO), are diluted in D-PBS, 2mM MgCl₂, 1% fish skin gelatin and 50 µL of each dilution added to duplicate wells. This is followed by addition of 50 µL of 0.8 µg/ml biotinylated recombinant ICAM-1/Ig (ICOS Corporation) to the wells and the plates are incubated at room temperature for 1 hour. The wells are then washed twice with wash buffer and 100 µL of Europium-labeled Streptavidin (Wallac Oy) diluted 1:100 in Delfia assay buffer (Wallac Oy) are added to the wells. Incubation proceeds for 1 hour at room temperature. The wells are washed eight times with wash buffer and 100 µL of enhancement solution (Wallac Oy, cat. No. 1244-105) are added to each well. Incubation proceeds for 5 minutes with constant mixing. Time-resolved fluorimetry measurements are made using the Victor 1420 Multilabel Counter (Wallac Oy) and the percent inhibition of each candidate compound is calculated using the following equation: 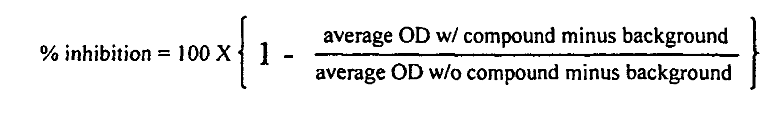 where "background" refers to wells that are not coated with anti-LFA-1 antibody.

Compounds of the present invention exhibit inhibitory activity in the above assay as follows:

| Compound of Example | % inhibition @ 4µM |
|---|---|
| 1 | 75 |
| 2 | 73 |
| 3 | 75 |
| 4 | 72 |
| 5 | 73 |
| 6 | 85 |
| 7 | 87 |
| 8 | 74 |
| 9 | 93 |
| 10 | 79 |
| 11 | 87 |
| 12 | 90 |
| 13 | 79 |
| 14 | 82 |
| 15 | 88 |
| 16 | 86 |
| 17 | 84 |
| 18 | 86 |
| 19 | 93 |
| 20 | 82 |
| 21 | 80 |
| 22 | 90 |
| 23 | 90 |
| 24 | 80 |
| 25 | 82 |
| 26 | 94 |
| 27 | 94 |
| 28 | 87 |
| 29 | 84 |
| 30 | 93 |
| 31 | 92 |
| 32 | 92 |
| 33 | 91 |
| 34 | 91 |
| 35 | 89 |
| 36 | 90 |
| 37 | 91 |
| 38 | 91 |
| 39 | 86 |
| 40 | 90 |
| 41 | 83 |
| 42 | 56 |
| 43 | 82 |
| 44 | 78 |
| 45 | 88 |
| 46 | 87 |
| 47 | 82 |
| 48 | 89 |
| 49 | 93 |
| 50 | 94 |
| 51 | 84 |
| 52 | 86 |
| 53 | 87 |
| 54 | 86 |
| 55 | 82 |
| 56 | 83 |
| 57 | 90 |
| 58 | 80 |
| 59 | 92 |
| 60 | 95 |
| 61 | 88 |
| 62 | 92 |
| 63 | 82 |
| 64 | 81 |
| 65 | 86 |
| 66 | 82 |
| 67 | 84 |
| 68 | 92 |
| 69 | 92 |
| 70 | 92 |
| 71 | 95 |
| 72 | 88 |
| 73 | 89 |
| 74 | 92 |
| 75 | 91 |
| 76 | 92 |
| 77 | 92 |
| 78 | 92 |
| 79 | 90 |
| 80 | 90 |
| 81 | 92 |
| 82 | 86 |
| 83 | 92 |
| 84 | 92 |
| 85 | 65 |
| 86 | 92 |
| 87 | |
| 88 | 86 |
| 89 | 90 |
| 90 | 90 |
| 91 | 90 |
| 92 | 82 |
| 93 | 82 |
| 94 | 90 |
| 95 | 88 |
| 96 | 90 |
| 97 | 94 |
| 98 | 90 |
| 99 | 95 |
| 100 | 92 |
| 101 | 86 |
| 102 | 92 |
| 103 | 93 |
| 104 | 92 |
| 105 | 88 |
| 106 | 86 |
| 107 | 96 |
| 108 | 29 |
| 109 | 90 |
| 110 | 94 |
| 111 | 84 |
| 112 | 93 |
| 113 | 88 |
| 114 | 89 |
| 115 | 86 |
| 116 | 92 |
| 117 | 94 |
| 118 | 94 |
| 119 | 95 |
| 120 | 94 |
| 121 | 94 |
| 122 | 94 |
| 123 | 94 |
| 124 | 91 |
| 125 | 94 |
| 126 | 90 |
| 127 | 89 |
| 128 | 84 |
| 129 | 92 |
| 130 | 91 |
| 131 | 84 |
| 132 | 81 |
| 133 | 83 |
| 134 | 94 |
| 135 | 95 |
| 136 | 94 |
| 137 | 94 |
| 138 | 88 |
| 139 | 92 |
| 140 | 94 |
| 141 | 93 |
| 142 | 94 |
| 143 | 92 |
| 144 | 92 |
| 145 | 92 |
| 146 | 81 |
| 147 | 94 |
| 148 | 92 |
| 149 | 93 |
| 150 | 94 |
| 151 | 92 |
| 152 | 94 |
| 153 | 92 |
| 154 | 94 |
| 155 | 93 |
| 156 | 94 |
| 157 | 90 |
| 158 | 92 |
| 159 | 95 |
| 160 | 94 |
| 161 | 94 |
| 162 | 95 |
| 163 | 94 |
| 164 | 92 |
| 165 | 92 |
| 166 | 95 |
| 167 | 94 |
| 168 | 93 |
| 169 | 92 |
| 170 | 93 |
| 171 | 94 |
| 172 | 94 |
| 173 | 94 |
| 174 | 92 |
| 175 | 94 |
| 176 | 94 |
| 177 | 90 |
| 178 | 93 |
| 179 | 93 |
| 180 | 88 |
| 181 | 93 |
| 182 | 92 |
| 183 | 92 |
| 184 | 94 |
| 185 | 93 |
| 186 | 83 |
| 187 | 86 |
| 188 | 81 |
| 189 | 76 |
| 190 | 86 |
| 191 | 93 |
| 192 | 95 |
| 193 | 92 |
| 194 | 86 |
| 195 | 90 |
| 196 | 92 |
| 197 | 94 |
| 198 | 93 |
| 199 | 87 |
| 200 | 83 |
| 201 | 92 |
| 202 | 90 |
| 203 | 92 |
| 204 | 92 |
| 205 | 94 |
| 206 | 94 |
| 207 | 94 |
| 208 | 93 |
| 209 | 92 |
| 210 | 93 |
| 211 | 94 |
| 212 | 94 |
| 213 | 94 |
| 214 | 92 |
| 215 | 98 |
| 216 | 86 |
| 217 | 94 |
| 218 | 94 |
| 219 | 98 |
| 220 | 91 |
| 221 | 90 |
| 222 | 98 |
| 223 | 96 |
| 224 | 86 |
| 225 | 98 |
| 226 | 96 |
| 227 | 96 |
| 228 | 96 |
| 229 | 96 |
| 230 | 92 |
| 231 | 88 |
| 232 | 90 |
| 233 | 93 |
| 234 | 98 |
| 235 | 92 |
| 236 | 90 |
| 237 | 92 |
| 238 | 97 |
| 239 | 98 |
| 240 | 97 |
| 241 | 91 |
| 242 | 58 |
| 243 | 95 |
| 244 | 96 |
| 245 | 96 |
| 246 | 97 |
| 247 | 93 |
| 248 | 96 |
| 249 | 96 |
| 250 | 92 |
| 251 | 98 |
| 252 | 97 |
| 253 | 96 |
| 254 | 98 |
| 255 | 97 |
| 256 | 94 |
| 257 | 94 |
| 258 | 96 |
| 259 | 96 |
| 260 | 92 |
| 261 | 96 |
| 262 | 96 |
| 263 | 94 |
| 264 | 94 |
| 265 | 96 |
| 266 | 86 |
| 267 | 94 |
| 268 | 96 |
| 269 | 94 |
| 270 | 95 |
| 271 | 95 |
| 272 | 94 |
| 273 | 93 |
| 274 | 96 |
| 275 | 94 |
| 276 | 86 |
| 277 | 94 |
| 278 | 88 |
| 279 | 94 |
| 280 | 94 |
| 281 | 96 |
| 282 | 96 |
| 283 | 95 |
| 284 | 94 |
| 285 | 94 |
| 286 | 96 |
| 287 | 92 |
| 288 | 92 |
| 289 | 95 |
| 290 | 90 |
| 291 | 96 |
| 292 | 96 |
| 293 | 96 |
| 294 | 96 |
| 295 | 94 |
| 296 | 94 |
| 297 | 94 |
| 298 | 94 |
| 299 | 92 |
| 300 | 92 |
| 301 | 91 |
| 302 | 92 |
| 303 | 94 |
| 304 | 94 |
| 305 | 92 |
| 306 | 93 |
| 307 | 93 |
| 308 | 94 |
| 309 | 94 |
| 310 | 92 |
| 311 | 92 |
| 312 | 86 |
| 313 | 90 |
| 314 | 96 |
| 315 | 96 |
| 316 | 94 |
| 317 | 92 |
| 318 | 98 |
| 319 | 98 |
| 320 | 89 |
| 321 | 94 |
| 322 | 96 |
| 323 | 98 |
| 324 | 96 |
| 325 | 98 |
| 326 | 98 |
| 327 | 98 |
| 328 | 98 |
| 329 | 98 |
| 330 | 98 |
| 331 | 97 |
| 332 | 98 |
| 333 | 98 |
| 334 | 94 |
| 335 | 98 |
| 336 | 98 |
| 337 | 93 |
| 338 | 93 |
| 339 | 92 |
| 340 | 93 |
| 341 | 94 |
| 342 | 94 |
| 343 | 94 |
| 344 | 93 |
| 345 | 92 |
| 346 | 90 |
| 347 | 92 |
| 348 | 90 |
| 349 | 92 |
| 350 | 91 |
| 351 | 94 |
| 352 | 94 |
| 353 | 92 |
| 354 | 91 |
| 355 | 96 |
| 356 | 96 |
| 357 | 97 |
| 358 | 97 |
| 359 | 96 |
| 360 | 98 |
| 361 | 98 |
| 362 | 98 |
| 363 | 96 |
| 364 | 96 |
| 365 | 96 |
| 366 | 96 |
| 367 | 97 |
| 368 | 93 |
| 369 | 96 |
| 370 | 73 |
| 371 | 93 |
| 372 | 93 |
| 373 | 97 |
| 374 | 96 |
| 376 | 40 |
| 377 | 96 |
| 378 | 97 |
| 379 | 95 |
| 380 | 97 |
| 381 | 99 |
| 382 | 97 |
| 383 | 97 |

Biologically relevant activity of the compounds in this invention is confirmed using a cell-based adhesion assay, which measures their ability to block the adherence of JY-8 cells (a human EBV-transformed B cell line expressing LFA-1 on its surface) to immobilized ICAM-1, as follows:

### ICAM-1 / JY-8 cell adhesion assay

For measurement of inhibitory activity in the cell-based adhesion assay, 96-well microtiter plates are coated with 70 µL of recombinant ICAM-1/Ig (ICOS Corporation) at a concentration of 5 µg/mL in D-PBS w/o Ca⁺⁺ or Mg⁺⁺ overnight at 4°C. The wells are then washed twice with D-PBS and blocked by addition of 200 µL of D-PBS, 5% fish skin gelatin by incubation for 1 hour at room temperature. Fluorescent tagged JY-8 cells (a human EBV-transformed B cell line expressing LFA-1 on its surface; 50 µL at 2 x 10⁶ cells/ml in RPMI 1640/1% fetal bovine serum) are added to the wells. For fluorescent labeling of JY-8 cells, 5 x 10⁶ cells washed once in RPMI 1640 are resuspended in 1 mL of RPMI 1640 containing 2 µM Calceiun AM (Molecular Probes), are incubated at 37°C for 30 minutes and washed once with RPMI-1640/ 1% fetal bovine serum. Dilutions of compounds to be assayed for ICAM-1/LFA-1 antagonistic activity are prepared in RPMI-1640/ 1% fetal bovine serum from 10mM stock solutions in DMSO and 50 µL are added to duplicate wells. Microtiter plates are incubated for 45 minutes at room temperature and the wells are washed gently once with RPMI-1640/ 1% fetal bovine serum. Fluorescent intensity is measured in a fluorescent plate reader with an excitation wavelength at 485 nM and an emission wavelength at 530 nM. The percent inhibition of a candidate compound at a given concentration is calculated using the following equation: 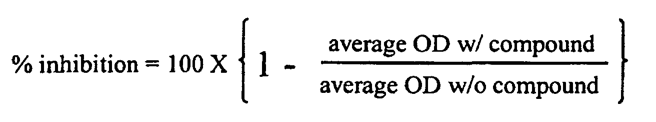 and these concentration/inhibition data are used to generate dose response curves, from which IC₅₀ values are derived.

Compounds of the present invention exhibit blocking activity in the above assay as follows:

| Compound of Example | % inhibition @ 4µM |
|---|---|
| 1 | 17 |
| 2 | 49 |
| 3 | 67 |
| 4 | 69 |
| 5 | 54 |
| 6 | 77 |
| 7 | 69 |
| 8 | 62 |
| 9 | 72 |
| 10 | 60 |
| 11 | 75 |
| 12 | 72 |
| 13 | 63 |
| 14 | 67 |
| 15 | 72 |
| 16 | 67 |
| 17 | 72 |
| 18 | 62 |
| 19 | 84 |
| 20 | 61 |
| 21 | 55 |
| 22 | 78 |
| 23 | 70 |
| 24 | 38 |
| 25 | 45 |
| 26 | 80 |
| 27 | 80 |
| 28 | 75 |
| 29 | 64 |
| 30 | 80 |
| 31 | 82 |
| 32 | 80 |
| 33 | 67 |
| 34 | 76 |
| 35 | 71 |
| 36 | 72 |
| 37 | 78 |
| 38 | 73 |
| 39 | 82 |
| 40 | 87 |
| 41 | 79 |
| 42 | 80 |
| 43 | 66 |
| 44 | 69 |
| 45 | 62 |
| 46 | 61 |
| 47 | 57 |
| 48 | 78 |
| 49 | 80 |
| 50 | 84 |
| 51 | 80 |
| 52 | 70 |
| 53 | 74 |
| 54 | 76 |
| 55 | 73 |
| 56 | 70 |
| 57 | 84 |
| 58 | 64 |
| 59 | 65 |
| 60 | 77 |
| 61 | 82 |
| 62 | 74 |
| 63 | 69 |
| 65 | 84 |
| 66 | 65 |
| 67 | 71 |
| 68 | 66 |
| 70 | 67 |
| 71 | 70 |
| 74 | 78 |
| 75 | 80 |
| 76 | 75 |
| 77 | 83 |
| 78 | 81 |
| 79 | 75 |
| 80 | 85 |
| 81 | 60 |
| 82 | 67 |
| 84 | 81 |
| 86 | 71 |
| 88 | 69 |
| 89 | 70 |
| 90 | 71 |
| 91 | 72 |
| 92 | 73 |
| 93 | 69 |
| 94 | 73 |
| 95 | 71 |
| 96 | 82 |
| 97 | 60 |
| 98 | 68 |
| 99 | 60 |
| 100 | 62 |
| 101 | 66 |
| 102 | 77 |
| 103 | 71 |
| 104 | 74 |
| 105 | 63 |
| 106 | 64 |
| 107 | 62 |
| 108 | 59 |
| 109 | 75 |
| 110 | 72 |
| 111 | 64 |
| 112 | 77 |
| 116 | 65 |
| 117 | 36 |
| 118 | 71 |
| 119 | 82 |
| 120 | 72 |
| 121 | 74 |
| 122 | 79 |
| 123 | 54 |
| 134 | 83 |
| 135 | 74 |
| 136 | 85 |
| 137 | 75 |
| 140 | 65 |
| 142 | 76 |
| 144 | 63 |
| 147 | 77 |
| 150 | 70 |
| 151 | 76 |
| 152 | 74 |
| 154 | 68 |
| 155 | 69 |
| 158 | 66 |
| 159 | 76 |
| 161 | 84 |
| 162 | 82 |
| 163 | 83 |
| 165 | 74 |
| 166 | 72 |
| 167 | 78 |
| 168 | 75 |
| 170 | 78 |
| 171 | 72 |
| 172 | 66 |
| 173 | 68 |
| 174 | 67 |
| 175 | 63 |
| 176 | 66 |
| 184 | 74 |
| 191 | 65 |
| 192 | 73 |
| 193 | 74 |
| 194 | 76 |
| 197 | 76 |
| 204 | 74 |
| 205 | 74 |
| 206 | 60 |
| 207 | 60 |
| 208 | 65 |
| 209 | 64 |
| 210 | 55 |
| 211 | 62 |
| 212 | 60 |
| 215 | 58 |
| 218 | 74 |
| 219 | 68 |
| 222 | 60 |
| 225 | 74 |
| 226 | 54 |
| 227 | 68 |
| 228 | 67 |
| 229 | 73 |
| 230 | 78 |
| 233 | 68 |
| 234 | 80 |
| 235 | 74 |
| 238 | 74 |
| 239 | 78 |
| 240 | 69 |
| 243 | 76 |
| 244 | 78 |
| 245 | 70 |
| 247 | 65 |
| 248 | 75 |
| 251 | 72 |
| 252 | 66 |
| 253 | 76 |
| 254 | 75 |
| 255 | 72 |
| 257 | 75 |
| 258 | 81 |
| 259 | 74 |
| 261 | 74 |
| 262 | 74 |
| 263 | 61 |
| 264 | 65 |
| 265 | 72 |
| 266 | 69 |
| 268 | 63 |
| 270 | 64 |
| 271 | 66 |
| 272 | 68 |
| 274 | 55 |
| 279 | 51 |
| 281 | 58 |
| 284 | 54 |
| 286 | 41 |
| 289 | 8 |
| 290 | 77 |
| 319 | 62 |
| 325 | 78 |
| 326 | 61 |
| 327 | 73 |
| 329 | 75 |
| 330 | 79 |
| 332 | 82 |
| 333 | 70 |
| 334 | 81 |
| 335 | 66 |
| 336 | 77 |
| 340 | 83 |
| 341 | 88 |
| 352 | 81 |

Compounds of the present invention have been demonstrated to act via interaction with the integrin LFA-1, specifically by binding to the interaction domain (I-domain), which is known to be critical for the adhesion of LFA-1 to a variety of cell adhesion molecules. As such, it is expected that these compounds should block the interaction of LFA-1 with other CAM'_{S}. This has in fact been demonstrated for the case of ICAM-3. Compounds of the present invention may be evaluated for their ability to block the adhesion ofJY-8 cells (a human EBV-transformed B cell line expressing LFA-1 on its surface) to immobilized ICAM-3, as follows:

### ICAM-3 / JY-8 cell adhesion assay

For measurement of inhibitory activity in the cell-based adhesion assay, 96-well microtiter plates are coated with 50 µL of recombinant ICAM-3/Ig (ICOS Corporation) at a concentration of 10 µg/mL in D-PBS w/o Ca⁺⁺ or Mg⁺⁺ overnight at 4°C. The wells are then washed twice with D-PBS, blocked by addition of 100 µL of D-PBS, 1% bovine serum albumin (BSA) by incubation for 1 hour at room temperature, and washed once with RPMI-1640 / 5% heat-inactivated fetal bovine serum (adhesion buffer). Dilutions of compounds to be assayed for ICAM-3/LFA-1 antagonistic activity are prepared in adhesion buffer from 10 mM stock solutions in DMSO and 100 µL are added to duplicate wells. JY-8 cells (a human EBV-transformed B cell line expressing LFA-1 on its surface; 100 µL at 0.75 x 10⁶ cells/ml in adhesion buffer) are then added to the wells. Microtiter plates are incubated for 30 minutes at room temperature; the adherent cells are then fixed with 50 µL of 14% glutaraldehyde/D-PBS and incubated for an additional 90 minutes. The wells are washed gently with dH₂O; 50 µL of dH₂O is added, followed by 50 µL of 1% crystal violet. After 5 minutes the plates are washed 3X with dH₂O; 75 µL of dH₂O and 225 µL of 95% EtOH are added to each well to extract the crystal violet from the cells. Absorbance is measured at 570 nM in an ELISA plate reader. The percent inhibition of a candidate compound is calculated using the following equation:

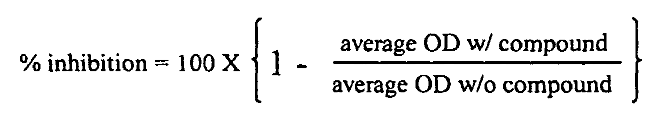

Compounds of the present invention exhibit blocking activity in the above assay as follows:

| Compound Of Example | % inhibition @ 0.6µM |
|---|---|
| 9 | 100 |
| 12 | 100 |
| 15 | 100 |
| 16 | 100 |
| 17 | 100 |
| 18 | 100 |
| 26 | 100 |
| 27 | 100 |
| 30 | 100 |
| 32 | 100 |
| 34 | 100 |
| 35 | 100 |
| 41 | 100 |
| 45 | 100 |
| 46 | 100 |
| 49 | 100 |
| 50 | 100 |
| 54 | 100 |
| 59 | 100 |
| 60 | 100 |
| 62 | 100 |

The ability of the compounds of this invention to treat arthritis can be demonstrated in a murine collagen-induced arthritis model according to the method of Kakimoto, et al., *Cell Immunol* 142: 326-337, 1992, in a rat collagen-induced arthritis model according to the method of Knoerzer, et al., *Toxicol Pathol* 25:13-19, 1997, in a rat adjuvant arthritis model according to the method of Halloran, et al., *Arthitis Rheum* 39: 810-819, 1996, in a rat streptococcal cell wall-induced arthritis model according to the method of Schimmer, et al., *J Immunol* 160: 1466-1477, 1998, or in a SCID-mouse human rheumatoid arthritis model according to the method of Oppenheimer-Marks et al., *J Clin Invest* 101: 1261-1272, 1998.

The ability of the compounds of this invention to treat Lyme arthritis can be demonstrated according to the method of Gross et al., *Science* 281, 703-706, 1998.

The ability of compounds of this invention to treat asthma can be demonstrated in a murine allergic asthma model according to the method of Wegner et al., *Science* 247:456-459, 1990, or in a murine non-allergic asthma model according to the method of Bloemen et al., *Am J Respir Crit Care Med* 153:521-529, 1996.

The ability of compounds of this invention to treat inflammatory lung injury can be demonstrated in a murine oxygen-induced lung injury model according to the method of Wegner et al., *Lung* 170:267-279, 1992, in a murine immune complex-induced lung injury model according to the method of Mulligan et al., *J Immunol* 154:1350-1363, 1995, or in a murine acid-induced lung injury model according to the method of Nagase, et al., *Am J Respir Crit Care Med* 154:504-510, 1996.

The ability of compounds of this invention to treat inflammatory bowel disease can be demonstrated in a rabbit chemical-induced colitis model according to the method of Bennet et al., *J Pharmacol Exp Ther* 280:988-1000, 1997.

The ability of compounds of this invention to treat autoimmune diabetes can be demonstrated in an NOD mouse model according to the method of Hasagawa et al., *Int Immunol* 6:831-838, 1994, or in a murine streptozotocin-induced diabetes model according to the method of Herrold et al., *Cell Immunol* 157:489-500, 1994.

The ability of compounds of this invention to treat inflammatory liver injury can de demonstrated in a murine liver injury model according to the method of Tanaka et al., *J Immunol* 151:5088-5095, 1993.

The ability of compounds of this invention to treat inflammatory glomerular injury can be demonstrated in a rat nephrotoxic serum nephritis model according to the method of Kawasaki, et al., *J Immunol* 150:1074-1083, 1993.

The ability of compounds of this invention to treat radiation-induced enteritis can be demonstrated in a rat abdominal irradiation model according to the method of Panes et al., *Gastroenterology* 108:1761-1769, 1995.

The ability of compounds of this invention to treat radiation pneumonitis can be demonstrated in a murine pulmonary irradiation model according to the method of Hallahan et al., *Proc Natl Acad Sci USA* 94:6432-6437, 1997.

The ability of compounds of this invention to treat reperfusion injury can be demonstrated in the isolated rat heart according to the method of Tamiya et al., *Immunopharmacology* 29(1): 53-63, 1995, or in the anesthetized dog according to the model of Hartman et al., *Cardiovasc Res* 30(1): 47-54, 1995.

The ability of compounds of this invention to treat pulmonary reperfusion injury can be demonstrated in a rat lung allograft reperfusion injury model according to the method of DeMeester et al., *Transplantation* 62(10): 1477-1485, 1996, or in a rabbit pulmonary edema model according to the method of Horgan et al., *Am J Physiol* 261(5): H1578-H1584, 1991.

The ability of compounds of this invention to treat stroke can be demonstrated in a rabbit cerebral embolism stroke model according the method of Bowes et al., *Exp Neurol* 119(2): 215-219, 1993, in a rat middle cerebral artery ischemia-reperfusion model according to the method of Chopp et al., *Stroke* 25(4): 869-875, 1994, or in a rabbit reversible spinal cord ischemia model according to the method of Clark et al., *Neurosurg* 75(4): 623-627, 1991.

The ability of compounds of this invention to treat peripheral artery occlusion can be demonstrated in a rat skeletal muscle ischemia / reperfusion model according to the method of Gute et al., *Mol Cell Biochem* 179: 169-187, 1998.

The ability of compounds of this invention to treat graft rejection can be demonstrated in a murine cardiac allograft rejection model according to the method of Isobe et al., *Science* 255: 1125-1127, 1992, in a murine thyroid gland kidney capsule model according to the method of Talento et al., *Transplantation* 55: 418-422, 1993, in a cynomolgus monkey renal allograft model according to the method of Cosimi et al., *J Immunol* 144: 4604-4612, 1990, in a rat nerve allograft model according to the method of Nakao et al., *Muscle Nerve* 18: 93-102, 1995, in a murine skin allograft model according to the method of Gorczynski and Wojcik, *J Immunol* 152 : 2011-2019, 1994, in a murine corneal allograft model according to the method of He et al., *Opthalmol Vis Sci* 35: 3218-3225, 1994, or in a xenogeneic pancreatic islet cell transplantation model according to the method of Zeng et al., *Transplantation* 58:681-689, 1994.

The ability of compounds of this invention to treat graft-vs.-host disease (GVHD) can be demonstrated in a murine lethal GVHD model according to the method of Harning et al., *Transplantation* 52:842-845, 1991.

The ability of compounds of this invention to treat cancers can be demonstrated in a human lymphoma metastasis model (in mice) according to the method of Aoudjit et al., *J Immunol* 161:2333-2338, 1998.

## Claims

1. A compound of formula I 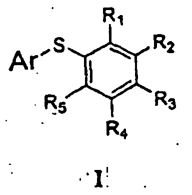 wherein R₁, R₂, R₃, R₄ and R₅ are independently selected from
a. hydrogen,
b. halogen,
c. alkyl,
d. haloalkyl,
e. alkoxy,
f. cyano,
g. nitro,
h. carboxaldehyde, and
with the proviso that at least one of R₁ to R₃ is a "*cis*-cinnamide" or a *"trans*-cinnamide", defined as 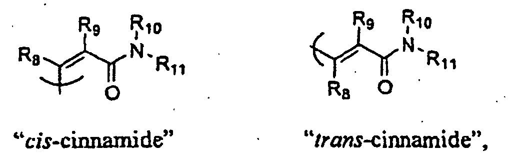 wherein R₈ and R₉ are independently selected from
a. hydrogen,
b. alkyl,
c. carboxy alkyl,
d. alkylaminocarbonyl alkyl, and
e. dialkylaminocarbonyl alkyl,
and R₁₀ and R₁₁ are independently selected from
a. hydrogen,
b. alkyl,
c. cycloalkyl,
d. alkoxycarbonylalkyl,
e. hydroxyalkyl,
f. heterocyclyl representing a 4-, 5-, 6- or 7-membered ring which contains one, two or three heteroatoms independently selected from nitrogen, oxygen and sulfur, which can be fused to one or two rings independently selected from an aryl ring as defined below, a cyclohexane ring, a cyclohexene ring, a cyclopentane ring, a cyclopentene ring or another monocyclic heterocyclic ring,
g. heterocyclylalkyl, where the heterocyclyl is as defined above,
h. heterocyclylamino, where the heterocyclyl is as defined above,
i. a heterocyclyl group as defined in f. above having substituents independently selected from alkyl, halogen, hydroxy and alkoxy;
j. a heterocyclylalkyl group as defined in g. above having substituents independently selected from alkyl, halogen, hydroxy and alkyoxy;
or where NR₁₀R₁₁ is a heterocyclyl or substituted heterocyclyl group where the heterocyclyl group is as defined in f. above and the substituents are independently selected from
1) alkyl,
2) alkoxy,
3) alkoxyalkyl,
4) cycloalkyl,
5) an aryl group being a mono- or bicyclic carbocyclic ring system having one or two aromatic rings which can be fused to a cyclohexane, cyclohexene, cyclopentane or cyclopentene ring,
6) a heterocyclyl group as defined above,
7) heterocyclylcarbonyl, where the heterocyclyl is as defined above,
8) heterocyclylalkylaminocarbonyl, where the heterocyclyl is as defined above,
9) hydroxy,
10) hydroxyalkyl,
11) hydroxyalkoxyalkyl,
12) carboxy,
13) carboxyalkyl,
14) carboxycarbonyl,
15) carboxaldehyde,
16) alkoxycarbonyl,
17) arylalkoxycarbonyl, where the aryl is as defined above,
18) aminoalkyl,
19) aminoalkanoyl,
20) carboxamido,
21) alkoxycarbonylalkyl,
22) carboxamidoalkyl,
23) cyano,
24) tetrazolyl,
25) substituted tetrazolyl,
26) alkanoyl,
27) hydroxyalkanoyl,
28) alkanoyloxy,
29) alkanoylamino,
30) alkanoyloxyalkyl,
31) alkanoylaminoalkyl,
32) sulfonate,
33) alkylsulfonyl,
34) alkysulfonylaminocarbonyl,
35) arylsutfonylaminocarbonyl, where the aryl is as defined above, and
36) heterocyclylsulfonylaminocarbonyl, where the heterocyclyl group is as defined above,
and wherein Ar is a substituted aryl or substituted heteroaryl group, which has one or two aromatic rings and which can be fused to a cyclohexane, cyclohexene, cyclopentane or cyclopentene ring, where substitutions are independently selected from
a. hydrogen,
b. halogen,
c. alkyl,
d. an aryl group as defined above,
e. haloalkyl,
f. hydroxy,
g. alkoxy,
h. alkoxyalkyl,
i. alkoxycarbonyl,
j. alkoxyalkoxy,
k. hydroxyalkyl,
l. aminoalkyl,
m. aminocarbonyl,
n. alkyl(alkoxycarbonylalkyl)aminoalkyl,
o. a.heterocyclyl group as defined above,
p. a heterocyclylalkyl group as defined above,
q. a heterocyclylalkyl group as defined above having substituents independently selected from alkyl, halogen, hydroxy and alkoxy;
r. carboxaldehyde,
s. carboxaldehyde hydrazone,
t. carboxamide,
u. alkoxycarbonylalkyl,
v. carboxy,
w. carboxyalkyl,
x. hydroxycarbonylalkyl(carboxyalkyl),
y. hydroxyalkylaminocarbonyl,
z. cyano,
aa. amino,
bb. heterocyclylalkylamino, where the heterocyclyl is as defined above,
cc. heterocyclylaminocarbonyl, where the heterocyclyl is as defined above, and
dd. "*trans*-cinnamide",
provided that
(i) if Ar is phenyl and R₁, R₂, R₄, and R₅ are simultaneously hydrogen, then R₃ is not a *cis*-cinnamide or *trans*-cinnamide where R₈ is methyl, R₉ is hydrogen and R₁₀ and R₁₁ are simultaneously -C₂H₅, and
(ii) if Ar is pyridyl or C₁₋₃ alkyl substituted pyridyl, and R₁, R₂, R₄, and R₅ are simultaneously hydrogen, then R₃ is not a cis-cinnamide or *trans*-cinnamide where R₈ is hydrogen, R₉ is hydrogen or C₁₋₃ alkyl, and R₁₀ and R₁₁ are independently hydrogen, C₁₋₄ alkyl, or C₃₋₆ cycloalkyl,
or a pharmaceutically acceptable salt thereof.

2. A compound according to Claim 1 wherein R₁ is a "*cis*-cinnamide" or a "*trans*-cinnamide", and R₃ is hydrogen.

3. A compound according to Claim 1 wherein R₃ is a "*cis*-cinnamide" or a "*trans*-cinnamide", and R₁ is hydrogen.

4. A compound according to Claim 1 wherein R₃ is a "*cis-*cinnamide" or a "*trans*-cinnamide", and R₁, R₈, and R₉ are hydrogen.

5. A compound according to Claim 4 wherein R₃ is a "*cis*-cinnamide".

6. A compound according to Claim 4 wherein R₃ is a "*trans*-cinnamide".

7. A compound according to Claim 1 wherein R₃ is a "*cis*-cinnamide" or a "*trans*-cinnamide", R₁, R₂, and R₄ are each independently hydrogen or alkyl; and R₅ is selected from halogen, haloalkyl, and nitro.

8. A compound according to Claim 4 wherein Ar is aryl, substituted aryl, heteroaryl, or substituted heteroaryl.

9. A compound according to Claim 4 wherein R₁₀ and R₁₁ are each independently selected from hydrogen, alkyl, cycloalkyl, alkoxycarbonylalkyl, hydroxyalkyl, and heterocyclylalkyl.

10. A compound according to Claim 4 wherein NR₁₀R₁₁ is heterocyclyl or substituted heterocyclyl.

11. A compound according to Claim 8 wherein Ar is selected from substituted phenyl, 1,3-benzimidazol-2-one, 1,4-benzodioxane, 1,3-benzodioxole, 1-benzopyr-2-en-4-one, indole, isatin, 1,3-quinazolin-4-one, and quinoline.

12. A compound according to Claim 1 selected from :
(2,4-Dichlorophenyl)[2-(*E*-((6-hydroxyhexylamino)carbonyl)ethenyl)phenyl]sulfide;
(2,4-Dichlorophenyl)[2-(*E*-((3-(1-imidazolyl)propylamino)carbonyl)ethenyl)phenyl]sulfide;
(2,4-Dichlorophenyl)[2-chloro-4-(*E*-((2 hydroxyethylamino)carbonyl)ethenyl)phenyl]sulfide;
(2,4-Dichlorophenyl)[2-chloro-4-(*E*-((6 ydroxyhexylamino)carbonyl)ethenyl)phenyl]sulfide;
(2,4-Dichlorophenyl)[2-chloro-4-(*E*-((bis-(2-hydroxyethyl)amino)carbonyl)ethenyl)phenyl]sulfide;
(2,4-Dichlorophenyl)[2-chloro-4-(*E*-((3-(1-pyrrolidin-2-only)propylamino)carbonyl)ethenyl)phenyl]sulfide;
(2,4-Dichlorophenyl)[2-chloro-4-(*E*-((1-morpholinyl)carbonyl)ethenyl)phenyl]sulfide;
(2,4-Dichlorophenyl)[2-chloro-4-(*E*-((4-methylpiperazin-1-yl)carbonyl)ethenyl)phenyl]sulfide;
(2,4-Dichlorophenyl)[2-chloro-4-(*E*-((4-acetylpiperazin-1-yl)carbonyl)ethenyl)phenyl]sulfide;
(2,4-Dichlorophenyl)[2-chloro-4-(*E*-((4-(2-pyridyl)piperazin-1-yl)carbonyl)ethenyl)phenyl]sulfide;
(2-(Hydroxymethyl)phenyl)[2-chloro-4-(*E*-((1-morpholinyl)carbonyl)ethenyl)phenyl]sulfide;
(2-Bromophenyl)[2-chloro-4-(*E*-((1-morpholinyl)carbonyl)ethenyl)phenyl]sulfide;
(2,4-Dichlorophenyl)[2-chloro-4-(*E*-((4-(2-hydroxyethyl)piperazin-1-yl)carbonyl)ethenyl)phenyl]sulfide;
(2,4-Dichlorophenyl)[2-chloro-4-(*E*-((4-(2-hydroxyethoxyethyl)piperazin-1-yl)carbonyl)ethenyl)phenyl]sulfide;
(2-Bromophenyl)[2-chloro-4-(*E*-((3-(hydroxymethyl)piperidin-1-yl)carbonyl)ethenyl)phenyl]sulfide;
(2-Bromophenyl)[2-chloro-4-(*E*-((2-(hydroxymethyl)piperidin-1-yl)carbonyl)ethenyl)phenyl]sulfide;
(2-Bromophenyl)[2-chloro-4-(*E*-((3-acetamidopyrrolidin-1-yl)carbonyl)ethenyl)phenyl]sulfide;
(2-Bromophenyl)[2-chloro-4-(*E*-((4-hydroxypiperidin-1-yl)carbonyl)ethenyl)phenyl]sulfide;
(2-Bromophenyl)[2-chloro-4-(*E*-((piperidin-1-yl)carbonyl)ethenyl)phenyl]sulfide;
(2,4-Dichlorophenyl)[2-chloro-4-(*E*-((3-carboxypiperidin-1-yl)carbonyl)ethenyl)phenyl]sulfide;
(2,4-Dichlorophenyl)[2-chloro-4-(*E*-((4-carboxypiperidin-1-yl)carbonyl)ethenyl)phenyl]sulfide;
(2-Bromophenyl)[2-chloro-4-(*E*-((4-acetylhomopiperazin-1-yl)carbonyl)ethenyl)phenyl]sulfide;
(2-Bromophenyl)[2-chloro-4-(*E*-((thiomorpholin-1-yl)carbonyl)ethenyl)phenyl]sulfide;
(2-Bromophenyl)[2-chloro-4-(*E*-((4-(1-benzimidazol-2-only)piperidin-1-yl)carbonyl)ethenyl)phenyl]sulfide;
(2-Bromophenyl)[2-chloro-4-(*E*-((2-etrahydroisoquinolinyl)carbonyl)ethenyl)phenyl]sulfide;
(2-Methylphenyl)[2-trifluoromethyl-4-(*E*-((4-acetylpiperazin-1-yl)carbonyl)ethenyl)phenyl]sulfide;
(2-Methylphenyl)[2-trifluoromethyl-4-(*E*-((1-morpholinyl)carbonyl)ethenyl)phenyl]sulfide;
(2-Methylphenyl)[2-trifluoromethyl-4-(*E*-((2-(1-morpholinyl)ethylamino)carbonyl)ethenyl)phenyl]sulfide;
(2-Methylphenyl)[2-trifluoromethyl-4-(*E*-((4-phenylpiperazin-1-yl)carbonyl)ethenyl)phenyl]sulfide;
(2-Methylphenyl)[2-trifluoromethyl-4-(*E*-((3-(1-pyrrolidin-2-onyl)propylamino) carbonyl)ethenyl)phenyl]sulfide;
(2-Methylphenyl)[2-trifluoromethyl-4-(*E*-((cyclopropylamino)carbonyl)ethenyl)phenyl]sulfide;
(2,4-Dichlorophenyl)[2-nitro-4-(*E*-((4-acetylpiperazin-1-yl)carbonyl)ethenyl)phenyl]sulfide;
(2,4-Dichlorophenyl)[2-nitro-4-(*E*-((3-(1-pyrrolidin-2-only)propylamino)carbonyl)ethenyl)phenyl]sulfide;
(2,3-Dichlorophenyl)[2-nitro-4-(*E*-((4-acetylpiperazin-1-yl)carbonyl)ethenyl)phenyl]sulfide;
(4-Bromophenyl)[2-nitro-4-(*E*-((4-acetylpiperazin-1-yl)carbonyl)ethenyl)phenyl]sulfide;
(4-Methylphenyl)[2-nitro-4-(*E*-((4-acetylpiperazin-1-yl)carbonyl)ethenyl)phenyl]sulfide;
(2,4-Dichlorophenyl)[2-nitro-4-(*E*-((4-(*tert*-butoxycarbonyl)piperazin-1-yl)carbonyl)ethenyl)phenyl]sulfide;
(2,4-Dichlorophenyl)[2-nitro-4-(*E*-((4-(2-furoylcarbonyl)piperazin-1-yl)carbonyl)ethenyl)phenyl]sulfide;
(2,4-Dichlorophenyl)[2-nitro-4-(*E*-((4-(methanesulfonyl)piperazin-1-yl)carbonyl)ethenyl)phenyl]sulfide;
(2,4-Dichlorophenyl)[2-nitro-4-(*E*-((4-(diethylaminocarbonylmethyl)piperazin-1-yl)carbonyl)ethenyl)phenyl]sulfide;
(2,4-Dichlorophenyl)[2-nitro-4-(*E*-((4-(diethylaminocarbonyl)piperazin-1-yl)carbonyl)ethenyl)phenyl]sulfide;
(2,4-Dichlorophenyl)[2-nitro-4-(*E*-((4-(*tert*-butoxycarbonylmethyl)piperazin-1-yl)carbonyl)ethenyl)phenyl]sulfide;
(2,4-Dichlorophenyl)[2-nitro-4-(*E*-((4-(carboxycarbonyl)piperazin-1-yl)carbonyl)ethenyl)phenyl]sulfide;
(2,4-Dichlorophenyl)[2-nitro-4-(*E*-((4-(carboxymethyl)piperazin-1-yl)carbonyl)ethenyl)phenyl]sulfide;
(2-Methylphenyl)[2-nitro-4-(*E*-((4-acetylpiperazin-1-yl)carbonyl)ethenyl)phenyl]sulfide;
(2-Chlorophenyl)[2-nitro-4-(*E*-((4-acetylpiperazin-1-yl)carbonyl)ethenyl)phenyl]sulfide;
(2-Aminophenyl)[2-nitro-4-(*E*-((4-acetylpiperazin-1-yl)carbonyl)ethenyl)phenyl]sulfide;
(2-Hydroxymethylphenyl)[2-nitro-4-(*E*-((4-acetylpiperazin-1-yl)carbonyl)ethenyl)phenyl]sulfide;
(2-Ethylphenyl)[2-nitro-4-(*E*-((4-acetylpiperazin-1-yl)carbonyl)ethenyl)phenyl]sulfide;
(2-*iso*-Propylphenyl)[2-nitro-4-(*E*-((4-acetylpiperazin-1-yl)carbonyl)ethenyl)phenyl]sulfide;
(2-*tert*-Butylphenyl)[2-nitro-4-(*E*-((4-acetylpiperazin-1-yl)carbonyl)ethenyl)phenyl]sulfide;
(2-Chlorophenyl)[2-chloro-4-(*E*-((4-acetylpiperazin-1-yl)carbonyl))2-propenyl)phenyl]sulfide;
(2-(1-Morpholinylmethyl)phenyl)[2-chloro-4-(*E*-((1-morpholinyl)carbonyl)ethenyl)phenyl]sulfide;
(2-(4-(1,3-Benzodioxolyl-5-methyl)piperazin-1-ylmethyl)phenyl)[2-chloro-4-(*E*-((1-cnorpholinyl)carbonyl)ethenyl)phenyl]sulfide;
(2-(4-(*iso*-Propylaminocarbonylmethyl)piperazin-1-ylmethyl)phenyl)[2-chloro-4-(*E*-((1-morpholinyl)carbonyl)ethenyl)phenyl]sulfide;
(2-((*N*-Ethoxycarbonylmethyl-*N*-methyl)aminomethyl)phenyl)[2-chloro-4-(*E-*((1-morpholinyl)carbonyl)ethenyl)phenyl]sulfide;
(2-Formylphenyl)[2-chloro-4-(*E*-((1-morpholinyl)carbonyl)ethenyl)phenyl]sulfide;
(2-(4-Formylpiperazin-1-ylmethyl)phenyl)[2-chloro-4-(*E*-((1-morpholinyl)carbonyl)ethenyl)phenyl]sulfide;
(2-(*E*-((1-Morpholinyl)carbonyl)ethenyl)phenyl)[2-chloro-4-(*E*-((1-morpholinyl)carbonyl)ethenyl)phenyl]sulfide;
(2-Formylphenyl)[2-nitro-4-(*E*-((4-acetylpiperazin-1-yl)carbonyl)ethenyl)phenyl]sulfide;
(2-Formylphenyl)[2-chloro-4-(*E*-((1-morpholinyl)carbonyl)ethenyl)phenyl]sulfide, N,N-dimethyl hydrazone;
(2-((3-(1-Morpholinyl)propyl)-1-amino)phenyl)[2-chloro-4-(*E*-((1-morpholinyl)carbonyl)ethenyl)phenyl]sulfide;
(2,4-Dichlorophenyl)[2-bromo-4-(*E*-((3-(1-pyrrolidin-2-only)propylamino)carbonyl)ethenyl)phenyl]sulfide;
(2,4-Dichlorophenyl)[2-formyl-4-(*E*-((1-morpholinyl)carbonyl)ethenyl)phenyl]sulfide;
(2-Chloro-6-formylphenyl)[2-chloro-4-(*E*-((4-acetylpiperazin-1-yl)carbonyl)ethenyl)phenyl]sulfide;
(2-Cyanophenyl)[2-chloro-4-(*E*-((4-acetylpiperazin-1-yl)carbonyl)ethenyl)phenyl]sulfide;
(2-Isopropylphenyl)[2-cyano-4-(*E*-((morpholin-1-yl)carbonyl)ethenyl)phenyl]sulfide;
(2-Bromophenyl)[2-nitro-4-(*E*-((4-acetylpiperazin-1-yl)carbonyl)ethenyl)phenyl)sulfide;
(2-(Pyrrolidin-1-yl)phenyl)[2-chloro-4-(*E*-((morpholin-1-yl)carbonyl)ethenyl)phenyl]sulfide;
(2-Methoxyphenyl)-[2-chloro-4(*E*-[(morpholin-1-yl)carbonyl]ethenyl)phenyl]sulfide;
(2-Isopropylphenyl)[2-nitro-4-(*E*-((3-carbomethoxypiperazin-1-yl)carbonyl)ethenyl)phenyl]sulfide;
(2-Methylphenyl)[2-nitro-4-(*E*-((3-carboxamido-4-carbobenzoxypiperazin-1-yl)carbonyl)ethenyl)phenyl]sulfide;
(2-Isopropylphenyl)[2-nitro-4-(*E*-((2-carbomethoxy-4-*tert*-butoxycarbonylpiperazin-1-yl)carbonyl)ethenyl)phenyl]sulfide;
(2-Isopropylphenyl)[2-nitro-4-(*E*-((2-carboxy-4-*tert*-butoxycarbonylpiperazin-1-yl)carbonyl)ethenyl)phenyl]sulfide;
(2-Isopropylphenyl)[2-trifluoromethyl-4-(*E*-((4-acetylpiperazin-1-yl)carbonyl)ethenyl)phenyl]sulfide;
(2-isopropylphenyl)(2-trifluoromethyl-4-(*E*-((morpholin-1-yl)carbonyl)ethenyl)phenyl]sulfide;
(2-Isopropylphenyl)[2-trifluoromethyl-4-(*E*-((3-(pyrrolidin-2-on-1-yl)prop-1-ylamino)carbonyl)ethenyl)phenyl]sulfide;
(2-Isopropylphenyl)[2-trifluoromethyl-4-(*E*-((cyclobutylamino)carbonyl)ethenyl)phenyl]sulfide;
(2-Isopropylphenyl)(2-trifluoromethyl-4-(*E*-((cyclopentylamino)carbonyl)ethenyl)phenyl]sulfide;
(2-Isopropylphenyl)[2-trifluoromethyl-4-(*E*-((5-hydroxypent-1-ylamino)carbonyl)ethenyl)phenyl]sulfide;
(2-Isopropylphenyl)[2-nitro-4-(*E*-((3-carbomethoxy-4-acetylpiperazin-1-yl)carbonyl)ethenyl)phenyl]sulfide;
(2-Biphenyl)[2-chloro-4-(*E*-((morpholin-1-yl)carbonyl)ethenyl)phenyl]sulfide;
(3,4-Dimethylphenyl)[2-nitro-4-(*E*-((4-acetylpiperazin-1-yl)carbonyl)ethenyl)phenyl]sulfide;
(2-Bromophenyl)[2-trifluoromethyl-4-(*E*-((4-acetylpiperazin-1-yl)carbonyl)ethenyl)phenyl]sulfide;
(5-Indolyl)[2-chloro-4-(*E*-((4-acetylpiperazin-1-yl)carbonyl)ethenyl)phenyl]sulfide;
(5-Benzodioxolyl)[2-chloro-4-(*E*-((4-acetylpiperazin-1-yl)carbonyl)-t ethenyl)phenyl]sulfide;
(2-Isopropylphenyl)(2-nitro-4-(*E*-((2-carbomethoxypiperazin-1-yl)carbonyl)ethenyl)phenyl]sulfide;
(2,3-Dimethoxyphenyl)-[2-chloro-4(*E*-[(morpholin-1-yl)carbonyl]ethenyl)phenyl]sulfide;
(2-Fluorophenyl)[2-nitro-4-(*E*-((4-acetylpiperazin-1-yl)carbonyl)ethenyl)phenyl]sulfide;
(2-Bromophenyl)[2-trifluoromethyl-4-(*E*-((4-(*tert*-butoxycarbonyl)piperazin-1-yl)carbonyl)ethenyl)phenyl]sulfide;
(2-(Pyrrolidin-1-yl)phenyl)[2-trifluoromethyl-4-(*E-*((4-(*tert*-butoxycarbonyl)piperazin-1-yl)carbonyl)ethenyl)phenyl]sulfide;
(3-Carboxamidophenyt)[2-nitro-4-(*E*-((4-acetylpiperazin-1-yl)carbonyl)ethenyl)phenyl]sulfide;
(3-(Hydroxymethyl)phenyl)[2-nitro-4-(*E*-((4-acetylpiperazin-1-yl)carbonyl)ethenyl)phenyl]sulfide;
Phenyl[2-trifluoromethyl-4-(*E*-((4-(*tert*-butoxycarbonyl)piperazin-1-yl)carbonyl)ethenyl)phenyl]sulfide;
(2-Isopropylphenyl)[2-trifluoromethyl -4-(*E*-((2-carbomethoxy-4-(*tert*-butoxycarbonyl)piperazin-1-yl)earbonyl)ethenyl)phenyl]sulfide;
(2-Isopropylphenyl)[2-nitro-4-(*E*-((3-(pyridine-4-methylaminocarbonyl)-4-*tert*-butoxycarbonylpiperazin-1-yl)carbonyl)ethenyl)phenyl]sulfide;
(2-Ethoxyphenyl)-[2-chloro-4(*E*-[(morpholin-1-yl)carbonyl]ethenyl)phenyl]sulfide;
(2-Methoxyphenyl)[2-nitro-4-(*E*-((4-acetylpiperazin-1-yl)carbonyl)ethenyl)phenyl]sulfide;
(2-(Azetidin-1-yl)phenyl)[2-trifluoromethyl-4-(*E*-((4-(tert-butoxycarbonyl)piperazin-1-yl)carbonyl)ethenyl)phenyl]sulfide;
(2-(Piperidin-1-yl)phenyl)[2-trifluoromethyl-4-(*E*-((4-(*tert*-butoxycarbonyl)piperazin-1-yl)carbonyl)ethenyl)phenyl]sulfide;
(3-Chloro-2-formylphenyl)[2-chloro-4-(*E*-((4-acetylpiperazin-1-yl)carbonyl)ethenyl)phenyl]sulfide;
(2-Trifluoromethylphenyl)[2-trifluoromethyl-4-(*E*-((4-acetylpiperazin-1-yl)carbonyl)ethenyl)phenyl]sulfide;
(3-Bromophenyl)[2-trifluoromethyl-4-(*E*-((4-acetylpiperazin-1-yl)carbonyl)ethenyl)phenyl]sulfide;
(3,5-Dimethylphenyl)[2-trifluoromethyl-4-(*E*-((4-acetylpiperazin-1-yl)carbonyl)ethenyl)phenyl]sulfide;
(2-Isopropylphenyl)[2-nitro-4-(*E*-((3-dimethylaminocarbonyl-4-(pyridine-4-carbonyl)piperazin-1-yl)carbonyl)ethenyl)phenyl]sulfide;
(2-Isopropylphenyl)[2-nitro-4-(*E*-((3-dimethylaminocarbonyl-4-carbomethoxypiperazin-1-yl)carbonyl)ethenyl)phenyl]sulfide;
(2-Isopropylphenyl)[2-nitro-4-(*E*-((3-dimethylaminocarbonyl-4-acetylpiperazin-1-yl)carbonyl)ethenyl)phenyl]sulfide;
(2-Isopropylphenyl)[2-nitro-4-(*E*-((3-(1-morpholinocarbonyl)-4-*tert*-butoxycarbonylpiperazin-1-yl)carbonyl)ethenyl)phenyl]sulfide;
(2-Isopropylphenyl)[2-nitro-4-(*E*-((3-(pyridine-4-methylaminocarbonyl)piperazin-1-yl)carbonyl)ethenyl)phenyl]sulfide;
(2-Isopropylphenyl)(2-nitro-4-(*E*-(((3-dimethylaminocarbonyl)piperazin-1-yl)earbonyl)ethenyl)phenyl]sulfide;
(2-Isopropylphenyl)[2-nitro-4-(*E*-((3-(benzylaminoearbonyl)-4-*tert*-butoxycarbonylpiperazin-1-yl)carbonyl)ethenyl)phenyl]sulfide;
(2-Isopropylphenyl)[2-nitro-4-(*E*-((3-(dimethylaminocarbonyl)-4-*tert*-butoxycarbonylpiperazin-1-yl)carbonyl)ethenyl)phenyl]sulfide;
(2-Bromophenyl)[2-chloro-4-(*E*-((3-(5*S*-hydroxymethyl-pyrrolidin-2-on-1-yl)prop-1-ylamino)carbonyl)ethenyl)phenyl]sulfide;
(2-Bromophenyl)[2-chloro-4-(*E*-((3-(pyrrolidin-2-on-1-yl)prop-1-ylamino)carbonyl)ethenyl)phenyl]sulfide;
(2-Bromophenyl)[2-chloro-4-(*E*-(N-methyl-N-(3-(pyrrolidin-2-on-1-yl)prop-1-yl)amino)carbonyl)ethenyl)phenyl]sulfide;
(2-[2-Methoxy]ethoxyphenyl)-[2-chloro-4(*E*-[(morpholin-1-yl)carbonyl]ethenyl)phenyl]sulfide;
(2-Isopropylphenyl)[2-nitro-4-(*E*-((3-(morpholinocarbonyl)piperazin-1-yl)carbonyl)ethenyl)phenyl]sulfide;
(2-Isopropylphenyl)(2-nitro-4-(*E*-((4-*tert*-butoxycarbonylpiperazin-1-yl)carbonyl)ethenyl)phenyl]sulfide;
(2-Isopropylphenyl)[2-nitro-4-(*E*-((4-methoxycarbonylpiperazin-1-yl)carbonyl)ethenyl)phenyl]sulfide;
(2-Isopropylphenyl)[2-nitro-4-(*E*-(4-(pyridine-4-carbonyl)piperazin-1-yl)carbonyl)ethenyl)phenyl]sulfide;
(2-Isopropylphenyl)[2-nitro-4-(*E*-((3-(pyridine-3-methylaminocarbonyl)-4-*tert*-butoxycarbonylpiperazin-1-yl)carbonyl)ethenyl)phenyl]sulfide;
(2-Isopropylphenyl)[2-nitro-4-(*E*-((3-(pyridine-2-methylaminocarbonyl)piperazin-1-yl)carbonyl)ethenyl)phenyl]sulfide;
(2-Isopropylphenyl)[2-nitro-4-(*E*-((3-(pyridine-3-methylaminocarbonyl)piperazin-1-yl)carbonyl)ethenyl)phenyl]sulfide;
(4-Hydroxyphenyl)[2-nitro-4-(*E*-((4-acetylpiperazin-1-yl)carbonyl)ethenyl)phenyl]sulfide;
(3,5-Dichlorophenyl)[2-nitro-4-(*E*-((4-acetylpiperazin-1-yl)carbonyl)ethenyl)phenyl]sulfide;
(2-Bromophenyl)[2-chloro-4-(*E*-((3-(5*S*-acetoxymethyl-pyrrolidin-2-on-1-yl)prop-1-ylamino)carbonyl)ethenyl)phenyl]sulfide;
(2-Bromophenyl)[2-chloro-4-(*E*-((3-(5*S*-methoxymethyl-pyrrolidin-2-on-1-yl)prop-1-ylamino)carbonyl)ethenyl)phenyl]sulfide;
(2-Bromophenyl)[2-chloro-4-(*E*-((3-(4*R*-hydroxymethyl-pyrrolidin-2-on-1-yl)prop-1-ylamino)carbonyl)ethenyl)phenyl]sulfide;
Phenyl[2-nitro-4-(*E*-((4-acetylpiperazin-1-yl)carbonyl)ethenyl)phenyl]sulfide;
(2-Dimethylaminophenyl)[2-nitro-4-(*E*-((4-acetylpiperazin-1-yl)carbonyl)ethenyl)phenyl]sulfide;
(3-((2-Hydroxyethyl)aminocarbonyl)phenyl)[2-nitro-4-(*E*-((4-acetylpiperazin-1-yl)carbonyl)ethenyl)phenyl]sulfide;
(3-((3-(1-Imidazolyl)propyl)aminocarbonyl)phenyl)[2-nitro-4-(*E*-((4-acetylpiperazin-1-yl)carbonyl)ethenyl)phenyl]sulfide;
(3-((2-(1-Morpholinyl)ethyl)aminocarbonyl)phenyl)(2-nitro-4-(*E*-((4-acetylpiperazin-1-yl)carbonyl)ethenyl)phenyl]sulfide;
(2-Isopropylphenyl)[2-nitro-4-(*E*-((3-hydroxymethyl-4-*tert*-butoxycarbonylpiperazin-1-yl)carbonyl)ethenyl)phenyl]sulfide;
(2-Isopropylphenyl)[2-nitro-4-(*E*-((4-formylpiperazin-1-yl)carbonyl)ethenyl)phenyl]sulfide;
(2-Isopropylphenyl)[2-nitro-4-(*E*-((2-hydroxymethyl-4-*tert*-butoxycarbonylpiperazin-1-yl)carbonyl)ethenyl)phenyl]sulfide;
(2-Ethoxyphenyl)-[2-chloro-4(*E*-[(3-ethoxycarbonylpiperidin-1-yl)carbonyl]ethenyl)phenyl]sulfide;
(3-Aminophenyl)(2-nitro-4-(*E*-((4-acetylpiperazin-1-yl)carbonyl)ethenyl)phenyl]sulfide;
(4-Aminophenyl)[2-nitro-4-(*E*-((4-acetylpiperazin-1-yl)carbonyl)ethenyl)phenyl]sulfide;
(2,4-Dimethylphenyl)[2-nitro-4-(*E*-((4-acetylpiperazin-1-yl)carbonyl)ethenyl)phenyl]sulfide;
(2,5-Dimethylphenyl)[2-nitro-4-(*E*-((4-acetylpiperazin-1-yl)carbonyl)ethenyl)phenyl]sulfide;
(4-Methoxyphenyl)[2-nitro-4-(*E*-((4-acetylpiperazin-1-yl)carbonyl)ethenyl)phenyl]sulfide;
(3-Chlorophenyl)[2-nitro-4-(*E*-((4-acetylpiperazin-1-yl)carbonyl)ethenyl)phenyl]sulfide;
(2-Chloro, 4,5-diaminophenyl)[2-chloro-4-(*E*-((4-acetylpiperazin-1-yl)carbonyl)ethenyl)phenyl]sulfide;
(3,4-Diaminophenyl)[2-chloro-4-(*E*-((4-acetylpiperazin-1-yl)carbonyl)ethenyl)phenyl]
(6-Chlorobenzimidazol-2-on-5-yl)[2-chloro-4-(*E*-((4-acetylpiperazin-1-yl)carbonyl)ethenyl)phenyl]sulfide;
(1-Methylindol-7-yl)[2-chloro-4-(*E*-((4-acetylpiperazin-1-yl)carbonyl)ethenyl)phenyl]sulfide;
(2-Hydroxy, 4-aminophenyl)[2-chloro-4-(*E*-((4-acetylpiperazin-1-yl)carbonyl)ethenyl)phenyl]sulfide;
(2-Isopropylphenyl)[2-nitro-4-(*E*-((4-mithylpiperazin-1-yl)carbonyl)ethenyl)phenyl]sulfide;
(2-Isopropylphenyl)[2-nitro-4-(*E*-((4-(pyridine-2-carbonyl)piperazin-1-yl)carbonyl)ethenyl)phenyl]sulfide;
(2-Isopropylphenyl)[2-nitro-4-(*E*-((4-(pyridine-3-carbonyl)piperazin-1-yl)carbonyl)ethenyl)phenyl]sulfide;
(2-Isopropylphenyl)[2-nitro-4-(*E*-((2-carbomethoxy-4-methoxycarbonylpiperazin-1-yl)carbonyl)ethenyl)phenyl]sulfide;
(2-Isopropylphenyl)[2-nitro-4-(*E*-((2-carboxy-4-methoxycarbonylpiperazin-1-yl)carbonyl)ethenyl)phenyl]sulfide;
(2-Isopropylphenyl)[2-nitro-4-(*E*-((3-carbomethoxy-4-methylpiperazin-1-yl)carbonyl)ethenyl)phenyl]sulfide;
(2-Ethoxyphenyl)-[2-chloro-4(*E*-[(3-carboxypiperidin-1-yl)carbonyl)ethenyl)phenyl]sulfide;
(2-Ethoxyphenyl)-[2-chloro-4(*E*-[(3-carboxypiperidin-1-yl)carbonyl]ethenyl)phenyl]sulfide;
(2-Ethoxyphenyl)-[2-chloro-4(*E*-[(2-ethoxycarbonylpiperidin-1-yl)carbonyl]ethenyl)phenyl]sulfide;
(2-Ethoxyphenyl)(2-trifluoromethyl-4-(*E*-((1-(*tert*-butoxycarbonyl)-4-hydroxypyrrolidin-3-ylamino)carbonyl)ethenyl)phenyl]sulfide;
(2-Ethoxyphenyl)-[2-chloro-4(*E*-[(2-carboxypiperidin-1-yl)carbonyl]ethenyl)phenyl]sulfide;
(2-Ethoxyphenyl)[2-trifluoromethyl-4-(*E*-(((pyrrol-3-in-1-yl)carbonyl)ethenyl)phenyl]sulfide;
(2-Ethoxyphenyl)[2-trifluoromethyl-4-(*E*-((3-(pyrrolidin-2-on-1-yl)prop-1-ylamino)carbonyl)ethenyl)phenyl]sulfide;
(2-Ethoxyphenyl)[2-trifluoromethyl-4-(*E*-((4-acetylpiperazin-1-yl)carbonyl)ethenyl)phenyl]sulfide;
(2-Ethoxyphenyl)[2-trifluoromethyl-4-(*E*-((4-(ethoxycarbonyl)piperazin-1-yl)carbonyl)ethenyl)phenyl]sulfide;
(2-Ethoxyphenyl)(2-trifluoromethyl-4-(*E*-((4-(2-furylcarbonyl)piperazin-1-yl)carbonyl)ethenyl)phenyl]sulfide;
(2-Ethoxyphenyl)-[2-chloro-4(*E*-[(3-ethoxycarbonylpiperidin-1-yl)carbonyl]ethenyl)phenyl]sulfide;
(2-Ethoxyphenyl)-[2-chloro-4(*E*-[(4-carboxypiperidin-1-yl)carbonyl]ethenyl)phenyl]sulfide;
(Benzodioxan-6-yl)[2-chloro-4-(*E*-((4-acetylpiperazin-1-yl)carbonyl)ethenyl)phenyl]sulfide;
(2-Isopropylphenyl)[2-nitro-4-(*E*-((4-ethoxycarbonylpiperazin-1-yl)carbonyl)ethenyl)phenyl]sulfide;
(2-Isopropylphenyl)[2-nitro-4-(*E*-((4-isopropoxycarbonylpiperazin-1-yl)carbonyl)ethenyl)phenyl]sulfide;
(2-Isopropylphenyl)[2-nitro-4-(*E*-((4-isobutoxycarbonylpiperazin-1-yl)carbonyl)ethenyl)phenyl]sulfide;
(2-Isopropylphenyl)[2-nitro-4-(*E*-((4-((1-propen-2-oxy)carbonyl)piperazin-1-yl)carbonyl)ethenyl)phenyl]sulfide;
(2-Isopropylphenyl)[2-nitro-4-(*E*-((4-propionylpiperazin-1-yl)carbonyl)ethenyl)phenyl]sulfide;
(2-Isopropylphenyl)[2-nitro-4-(*E*-((4-carboxamidopiperazin-1-yl)carbonyl)ethenyl)phenyl]sulfide;
(2-Isopropylphenyl)[2-nitro-4-(*E*-((4-methylaminocarbonylpiperazin-1-yl)carbonyl)ethenyl)phenyl]sulfide;
(2-Isopropylphenyl)[2-nitro-4-(*E*-((4-(pyrimidin-2-yl)piperazin-1-yl)carbonyl)ethenyl)phenyl]sulfide;
(2-Isopropylphenyl)[2-nitro-4-(*E*-((4-hydroxyacetylpiperazin-1-yl)carbonyl)ethenyl)phenyl]sulfide;
(2-Isopropylphenyl)[2-nitro-4-(*E*-((4-(pyrazine-2-carbonyl)piperazin-1-yl)carbonyl)ethenyl)phenyl]sulfide;
(2-Isopropylphenyl)[2-trifluoromethyl-4-(*E*-(((2-carboxypyrrol-3-in-1-yl)carbonyl)ethenyl)phenyl]sulfide;
(2-Isopropylphenyl)[2-nitro-4-(*E*-((3-hydroxymethyl-4-methylpiperazin-1-yl)carbonyl)ethenyl)phenyl]sulfide;
(2-Isopropylphenyl)[2-trifluoromethyl-4-(*E*-(((2-carboxypyrrol-3-in-1-yl)carbonyl)ethenyl)phenyl]sulfide;
(2-Isopropylphenyl)[2-trifluoromethyl-4-(*E*-(((2-hydroxymethylpyrrolidin-1-yl)carbonyl)ethenyl)phenyl]sulfide;
(2-Isopropylphenyl)[2-nitro-4-(*E*-((3-methylaminocarbonyl)piperazin-1-yl)carbonyl)ethenyl)phenyl]sulfide;
(2-Isopropylphenyl)(2-nitro-4-(*E*-(((3-cyclopropylaminocarbonyl)piperazin-1-yl)carbonyl)ethenyl)phenyl]sulfide;
(2-Isopropylphenyl)[2-nitro-4-(*E*-((3-carboxamidopiperazin-1-yl)carbonyl)ethenyl)phenyl]sulfide;
(2-Isopropylphenyl)[2-nitro-4-(*E*-((3-carbomethoxy-4-oxopiperidin-1-yl)carbonyl)ethenyl)phenyl]sulfide;
(2-Isopropylphenyl)[2-nitro-4-(*E*-((3,5-dimethylpiperazin-1-yl)carbonyl)ethenyl)phenyl]sulfide;
(1-Ethylindol-7-yl)[2-chloro-4-(*E*-((4-acetylpiperazin-1-yl)carbonyl)ethenyl)phenyl]sulfide;
(3-[2-Methoxy]ethoxyphenyl)-[2-chloro-4(*E*-[(morpholin-1-yl)carbonyl]ethenyl)phenyl]sulfide;
(2-Bromophenyl)[2-chloro-4-(*E*-((4,4'-*S*-dioxythiomorpholin-1-yl)carbonyl)ethenyl)phenyl]sulfide;
(2-Bromophenyl)[2-chloro-4-(*E*-(N-carbomethoxymethyl-N-(3-(pyrrolidin-2-on-1-yl)prop-1-yl)amino)carbonyl)ethenyl)phenyl]sulfide;
(2-Bromophenyl)[2-chloro-4-(*E*-((4-*S*-oxythiomorpholin-1-yl)-2-pyrrolidinone)carbonyl)ethenyl)phenyl]sulfide;
(2-Methoxy-5-chlorophenyl)[2-nitro-4-(*E*-((4-acetylpiperazin-1-yl)carbonyl)ethenyl)phenyl]sulfide;
(2-Isopropylphenyl)[2-nitro-4-(*E*-((3-acetoxymethyl)piperazin-1-yl)carbonyl)ethenyl)phenyl]sulfide;
(2-Isopropylphenyl)[2-nitro-4-(*E*-((3,5-dimethyl-4acetylpiperazin-1-yl)carbonyl)ethenyl)phenyl]sulfide;
(1-Methylindol-5-yl)[2-chloro-4-(*E*-((4-acetylpiperazin-1-yl)carbonyl)ethenyl)phenyl]sulfide;
(Benzodioxan-6-yl)[2-nitro-4-(*E*-((4-acetylpiperazin-1-yl)carbonyl)ethenyl)phenyl]sulfide;
(Benzodioxan-6-yl)[2-nitro-4-(*E*-((3-(pyrrolidin-2-on-1-yl)prop-1-ylamino)carbonyl)ethenyl)phenyl]sulfide;
(Benzodioxan-6-yl)[2-nitro-4-(*E*-((3-carboethoxypiperidin-1-yl)carbonyl)ethenyl)phenyl]sulfide;
(Benzodioxan-6-yl)[2-nitro-4-(*E*-((4-carboethoxypiperidin-1-yl)carbonyl)ethenyl)phenyl]sulfide;
(2-Ethoxyphenyl)[2-trifluoromethyl-4-(*Z*-((4-acetylpiperazin-1-yl)carbonyl)ethenyl)phenyl]sulfide;
(2-Ethoxyphenyl)[2-trifluoromethyl-4-(*E*-((6-methylpyrid-2-ylamino)carbonyl)ethenyl)phenyl]sulfide;
(2-Methyl-3-chlorophenyl)[2-nitro-4-(*E*-((4-acetylpiperazin-1-yl)carbonyl)ethenyl)phenyl]sulfide;
(Benzodioxan-6-yl)[2-nitro-4-(*E*-((3-carboxamidopiperidin-1-yl)carbonyl)ethenyl)phenyl]sulfide;
(Benzodioxan-6-yl)[2-nitro-4-(*E*-((2-carboethoxypiperidin-1-yl)carbonyl)ethenyl)phenyl]sulfide;
(Benzodioxan-6-yl)[2-nitro-4-(*E*-((4-carboxamidopiperidin-1-yl)carbonyl)ethenyl)phenyl]sulfide;
(Benzodioxan-6-yl)[2-nitro-4-(*E*-((4-*tert*-butoxycarbonylpiperazin-1-yl)carbonyl)ethenyl)phenyl]sulfide;
(2-Isopropylphenyl)(2-nitro-4-(*E*-((*syn*-3,5-dimethylmorpholin-1-yl)carbonyl)ethenyl)phenyl]sulfide;
(2-Isopropylphenyl)(2-nitro-4-(*E*-((*anti*-3,5-dimethylmorpholin-1-yl)carbonyl)ethenyl)phenyl]sulfide;
(2-Isopropylphenyl)[2-nitro-4-(*E*-((3-carboethoxypiperazin-1-yl)carbonyl)ethenyl)phenyl]sulfide;
(2-Isopropylphenyl)[2-nitro-4-(*E*-((3-isopropoxycarbonylpiperazin-1-yl)carbonyl)ethenyl)phenyl]sulfide;
(2-Isopropylphenyl)[2-nitro-4-(*E*-((3-(dimethylaminocarbonyl)-4-methylpiperazin-1-yl)carbonyl)ethenyl)phenyl]sulfide;
(2-Isopropylphenyl)[2-nitro-4-(*E*-((3-carbomethoxy-4-hydroxypiperidin-1-yl)carbonyl)ethenyl)phenyl]sulfide;
(2-Isopropylphenyl)[2-nitro-4-(*E*-((3-hydroxymethyl-4-hydroxypiperidin-1-yl)carbonyl)ethenyl)phenyl]sulfide;
(2-Ethoxyphenyl)[2-trifluoromethyl-4-(*E*-((2-carbomethoxy-4-(methoxycarbonyl)piperazin-1-yl)carbonyl)ethenyl)phenyl]sulfide;
(2-Ethoxyphenyl)(2-trifluoromethyl-4-(*E*-((2-carbomethoxy-4-methyl piperazin-1-yl)carbonyl)ethenyl)phenyl]sulfide;
(2-Ethoxyphenyl)[2-trifluoromethyl-4-(*E*-((2-carboxy-4-(methoxycarbonyl)piperazin-1-yl)carbonyl)ethenyl)phenyl]sulfide;
(Indol-6-yl)[2-chloro-4-(*E*-((4-acetylpiperazin-1-yl)carbonyl)ethenyl)phenyl]sulfide;
(1-Ethyl,3-(dimethylaminomethyl)indol-7-yl)[2-chloro-4-(*E*-((4-acetylpiperazin-1-yl)carbonyl)ethenyl)phenyl]sulfide;
(5-Ethoxybenzodioxan-6-yl)[2-chloro-4-(*E*-((4-acetylpiperazin-1-yl)carbonyl)ethenyl)phenyl]sulfide;
(2-Ethyl-4-bromophenyl)[2-nitro-4-(*E*-((4-acetylpiperazin-1-yl)carbonyl)ethenyl)phenyl]sulfide;
(Benzodioxan-6-yl)[2-nitro-4-(*E*-((2-carboxypiperidin-1-yl)carbonyl)ethenyl)phenyl]sulfide;
(Benzodioxan-6-yl)[2-nitro-4-(*E*-((4-carboxymethylpiperazin-1-yl)carbonyl)ethenyl)phenyl]sulfide;
(3-Morpholinophenyl)[2-nitro-4-(*E*-((4-acetylpiperazin-1-yl)carbonyl)ethenyl)phenyl]sulfide;
(5-Ethoxybenzodioxan-8-yl)[2-chloro-4-(*E*-((4-acetylpiperazin-1-yl)carbonyl)ethenyl)phenyl]sulfide;
(5-Chloro-8-ethoxyquinolin-7-yl)[2-chloro-4-(*E*-((4-acetylpiperazin-1-yl)carbonyl)ethenyl)phenyl]sulfide;
(2-Isopropylphenyl)[2-nitro-4-(*E*-((3-carboethoxypiperidin-1-yl)carbonyl)ethenyl)phenyl]sulfide;
(2-Isopropylphenyl)[2-nitro-4-(*E*-((3-carboxypiperidin-1-yl)carbonyl)ethenyl)phenyl]sulfide;
(2-Isopropylphenyl)[2-nitro-4-(*E*-(((3-ethanesulfonylaminocarbonyl)piperidin-1-yl)carbonyl)ethenyl)phenyl]sulfide;
(2-Isopropylphenyl)[2-nitro-4-(*E*-(((3-(4-methylpiperazine) sulfonylaminocarbonyl)piperidin-1-yl)carbonyl)ethenyl)phenyl]sulfide;
(2-Isopropylphenyl)[2-nitro-4-(*E*-(((3-*p*-toluenesulfonylaminocarbonyl)piperidin-1-yl)carbonyl)ethenyl)phenyl]sulfide;
(2-Isopropylphenyl)[2-nitro-4-(*E*-((3-methyl-4-acetylpiperazin-1-yl)carbonyl)ethenyl)phenyl]sulfide;
(2-Hydroxyphenyl)-[2-chloro-4(*E*-[(morpholin-1-yl)carbonyl]ethenyl)phenyl]sulfide (1-(Carboxymethyl)indol-5-yl)[2-chloro-4-(*E*-((4-acetylpiperazin-1-yl)carbonyl)ethenyl)phenyl]sulfide;
(Benzodioxan-6-yl)[2-trifluoromethyl-4-(*E*-((4-acetylpiperazin-1-yl)carbonyl)ethenyl)phenyl]sulfide;
(2-Isopropylphenyl)[2-nitro-4-(*E*-((3-(1-pyrrolidin-2-onyl)prop-1-ylamino) carbonyl)ethenyl)phenyl]sulfide;
(3-(2-Morpholinoethylamino)phenyl)[2-trifluoromethyl-4-(*E*-((4-acetylpiperazin-1-yl)carbonyl)ethenyl)phenyl]sulfide;
(2-Pyrrolidin-1-ylphenyl)[2-nitro-4-(*E*-((4-acetylpiperazin-1-yl)carbonyl)ethenyl)phenyl]sulfide;
(3-Bromophenyl)[2-nitro-4-(*E*-((3-carboethoxypyrrolidin-1-yl)carbonyl)ethenyl)phenyl]sulfide;
(3-Bromophenyl)[2-nitro-4-(*E*-((4-carboethoxypyrrolidin-1-yl)carbonyl)ethenyl)phenyl]sulfide;
(2-(Hydroxymethyl)-benzadioxan-6-yl)[2-chloro-4-(*E*-((4-acetylpiperazin-1-yl)carbonyl)ethenyl)phenyl]sulfide;
(Benzodioxan-6-yl)[2-trifluoromethyl-4-(*E*-((3-(pyrrolidin-2-on-1-yl)prop-1-ylamino)carbonyl)ethenyl)phenyl]sulfide;
(3-(Dimethylaminomethyl)indol-5-yl)[2-chloro-4-(*E*-((4-acetylpiperazin-1-yl)carbonyl)ethenyl)phenyl]sulfide;
(2-Isopropylphenyl)[2-nitro-4-(*E*-((2-carboethoxypiperidin-1-yl)carbonyl)ethenyl)phenyl]sulfide;
(2-Isopropylphenyl)[2-nitro-4-(*E*-((2-carboxypiperidin-1-yl)carbonyl)ethenyl)phenyl]sulfide;
(2-Isopropylphenyl)[2-nitro-4-(*E*-((4-carboethoxypiperidin-1-yl)carbonyl)ethenyl)phenyl]sulfide;
(2-Isopropylphenyl)[2-nitro-4-(*E*-((4-carboxypiperidin-1-yl)carbonyl)ethenyl)phenyl]sulfide;
(2-Isopropylphenyl)[2-nitro-4-(*E*-(((4-*p*-toluenesulfonylaminocarbonyl)piperidin-1-yl)carbonyl)ethenyl)phenyl]sulfide;
(2-Isopropylphenyl)[2-nitro-4-(*E*-((3-carboxy-4-hydroxypiperidin-1-yl)carbonyl)ethenyl)phenyl]sulfide;
(Benzodioxan-6-yl)[2-trifluoromethyl-4-(*E*-((3-carboethoxypiperidin-1-yl)carbonyl)ethenyl)phenyl]sulfide;
(Benzodioxan-6-yl)[2-trifluoromethyl-4-(*E*-((2-carboethoxypiperidin-1-yl)carbonyl)ethenyl)phenyl]sulfide;
(Benzodioxan-6-yl)[2-nitro-4-(*E*-((4-carboxypiperidin-1-yl)carbonyl)ethenyl)phenyl]sulfide;
(Benzodioxan-6-yl)[2-trifluoromethyl-4-(*E*-((3-carboxypyrrolidin-1-yl)carbonyl)ethenyl)phenyl]sulfide;
(Benzodioxan-6-yl)[2-trifluoromethyl-4-(*E*-((4-carboethoxypiperidin-1-yl)carbonyl)ethenyl)phenyl]sulfide;
(Benzodioxan-6-yl)[2-trifluoromethyl-4-(*E*-((2-carbomethoxy-4-*tert*-butoxycarbonylpiperazin-1-yl)carbonyl)ethenyl)phenyl]sulfide;
(Benzodioxan-6-yl)[2-trifluoromethyl-4-(*E*-((2-carbomethoxy-4-methoxycarbonylpiperazin-1-yl)carbonyl)ethenyl)phenyl]sulfide;
(Benzodioxan-6-yl)[2-trifluoromethyl-4-(*E*-((2-carbomethoxypiperazin-1-yl)carbonyl)ethenyl)phenyl]sulfide;
(2-Methyl-3-(carboethoxymethyl)indol-5-yl)[2-trifluoromethyl-4-(*E*-((morpholin-1-yl)carbonyl)ethenyl)phenyl]sulfide;
(1-(2-Methoxyethyl)indol-5-yl)[2-chloro-4-(*E*-((4-acetylpiperazin-1-yl)carbonyl)ethenyl)phenyl]sulfide;
(2-Isopropylphenyl)[2-nitro-4-(*E*-((3-acetoxymethyl-4-hydroxypiperidin-1-yl)carbonyl)ethenyl)phenyl]sulfide;
(2-Isopropylphenyl)[2-nitro-4-(*E*-((3-(dimethylaminocarbonyl)-4-hydroxypiperidin-1-yl)carbonyl)ethenyl)phenyl]sulfide;
(2-Isopropylphenyl)[2-nitro-4-(*E*-((3-cyanomorpholin-1-yl)carbonyl)ethenyl)phenyl]sulfide;
(2-Isopropylphenyl)[2-nitro-4-(*E*-((3-carboethoxymorpholin-1-yl)carbonyl)ethenyl)phenyl]sulfide;
(2-Isopropylphenyl)(2-nitro-4-(*E*-((3-(tetrazol-5-yl)morpholin-1-yl)carbonyl)ethenyl)phenyl]sulfide;
(Benzodioxan-6-yl)[2-trifluoromethyl-4-(*E*-((4-carboxypiperidin-1-yl)carbonyl)ethenyl)phenyl]sulfide;
(Benzodioxan-6-yl)[2-trifluoromethyl-4-(*E*-((2-carboxypiperidin-1-yl)carbonyl)ethenyl)phenyl]sulfide;
(Benzodioxan-6-yl)[2-trifluoromethyl-4-(*E*-((4-carbomethoxypiperazin-1-yl)carbonyl)ethenyl)phenyl]sulfide;
(Benzodioxan-6-yl)[2-trifluoromethyl-4-(*E*-((3-aza-6,9-diooxaspiro[5,4]decan-1-yl)carbonyl)ethenyl)phenyl]sulfide;
(Benzodioxan-6-yl)[2-trifluoro-4-(*E*-((4-(benzimidazolon-1-yl)piperidin-1-yl)carbonyl)ethenyl)phenyl]sulfide;
(Benzodioxan-6-yl)[2-trifluoromethyl-4-(*E*-((4-(methylaminocarbonyl)piperidin-1-yl)carbonyl)ethenyl)phenyl]sulfide;
(Benzodioxan-6-yl)[2-trifluoromethyl-4-(*E*-((3-carbomethoxy-4-methoxycarbonylpiperazin-1-yl)carbonyl)ethenyl)phenyl]sulfide;
(2-Isopropylphenyl)[2-nitro-4-(*E*-((3-carboxymorpholin-1-yl)carbonyl)ethenyl)phenyl]sulfide;
(Benzodioxan-6-yl)[2-trifluoromethyl-4-(*E*-((2-carboxy-4-methoxycarbonylpiperazin-1-yl)carbonyl)ethenyl)phenyl]sulfide;
(Benzodioxan-6-yl)[2-trifluoromethyl-4-(*E*-((morpholin-1-yl)carbonyl)ethenyl)phenyl]sulfide;
(Benzodioxan-6-yl)[2-trifluoromethyl-4-(*E*-((4-(pyrrolidin-1-yl)piperidin-1-yl)carbonyl)ethenyl)phenyl]sulfide;
(2-Isopropylphenyl)[2-nitro-4-(*E*-((3-aza-6,9-diooxaspiro[5.4]decan-1-yl)carbonyl)ethenyl)phenyl]sulfide;
(2-Isopropylphenyl)[2-nitro-4-(*E*-((2-(dimethylaminomethyl)piperidin-1-yl)carbonyl)ethenyl)phenyl]sulfide;
(2-Isopropylphenyl)[2-nitro-4-(*E*-((piperidin-1-ylamino)carbonyl)ethenyl)phenyl]sulfide;
(Benzodioxan-6-yl)[2-trifluoromethyl-4-(*E*-((3-carboxy-4-methoxycarbonylpiperazin-1-yl)carbonyl)ethenyl)phenyl]sulfide;
(2-(Dimethylaminocarbonyl)-benzodioxan-6-yl)[2-chloro-4-(*E*-((4-acetylpiperazin-1-yl)carbonyl)ethenyl)phenyl]sulfide;;
(2-Isopropylphenyl)[2-nitro-4-(*E*-((3-(2-(methoxymethyl)tetrazol-5-yl)piperidin-1-yl)carbonyl)ethenyl)phenyl]sulfide
(2-Isopropylphenyl)[2-nitro-4-(*E*-((3-(1-(methoxymethyl)tetrazol-5-yl)piperidin-1-yl)carbonyl)ethenyl)phenyl]sulfide;
(1-Methylindol-5-yl)[2-chloro-4-(*E*-((3-(1-pyrrolidin-2-onyl)propylamino) carbonyl)ethenyl)phenyl]sulfide;
(2-Isopropylphenyl)[2-nitro-4-(*E*-((3-(tetrazol-5-yl)piperidin-1-yl)carbonyl)ethenyl)phenyl]sulfide;
(1-Methylindol-5-yl)[2-chloro-4-(*E*-((3-carboethoxypiperidin-1-yl)carbonyl)ethenyl)phenyl]sulfide
(1-Methylindol-5-yl)[2-chloro-4-(*E*-((3-carboxypiperidin-1-yl)carbonyl)ethenyl)phenyl]sulfide;
(1-Methylindol-5-yl)[2-chloro-4-(*E*-((4-carboethoxypiperidin-1-yl)carbonyl)ethenyl)phenyl]sulfide;
(1-Methylindol-5-yl)[2-chloro-4-(*E*-((3-carboxypiperidin-1-yl)carbonyl)ethenyl)phenyl]sulfide;
(2-Isopropylphenyl)[2-nitro-4-(*E*-((2-(1-methylpyrrolidin-2-yl)ethylamino)carlionyl)ethenyl)phenyl]sulfide;
(2-Isopropylphenyl)[2-nitro-4-(*E*-((4-(pyrrolidin-1-yl)piperidin-1-yl)carbonyl)ethenyl)phenyl]sulfide;
(2-Isopropylphenyl)[2-nitro-4-(*E*-((4-sulfopiperidin-1-yl)carbonyl)ethenyl)phenyl]sulfide;
(2-Isopropylphenyl)[2-nitro-4-(*E*-((3-hydroxypiperidin-1-yl)carbonyl)ethenyl)phenyl]sulfide;
(Benzodioxan-6-yl)[2-trifluoromethyl-4-(*E*-((3-((ethanesulfonylamino)carbonyl)piperidin-1-yl)carbonyl)ethenyl)phenyl]sulfide;
(Benzodioxan-6-yl)[2-trifluoromethyl-4-(*E*-((3-((*p*-toluenesulfonylamino)carbonyl)piperidin-1-yl)carbonyl)ethenyl)phenyl]sulfide;
(Benzodioxan-6-yl)[2-trifluoromethyl-4-(*E*-((4-((ethanesulfonylamino)carbonyl)piperidin-1-yl)carbonyl)ethenyl)phenyl]sulfide;
(Benzodioxan-6-yl)[2-trifluoromethyl-4-(*E*-((2(tetrazol-5-yl)morpholin-1-yl)carbonyl)ethenyl)phenyl]sulfide;
(2-Isopropylphenyl)[2-nitro-4-(*E*-((2-butyl, 5-(tetrazol-5-yl)morpholin-1-yl)carbonyl)ethenyl)phenyl]sulfide;
(2-(and 3-)(Hydroxymethyl)-benzodioxan-6-yl)[2-nitro-4-(*E*-((4-acetylpiperazin-1-yl)carbonyl)ethenyl)phenyl]sulfide;
(2-(and 3-)(Hydroxymethyl)-benzodioxan-6-yl)[2-nitro-4-(*E*-((3-(pyrrolidin-2-on-1-yl)prop-1-ylamino)carbonyl)ethenyl)phenyl]sulfide;
(2-(and 3-)(Hydroxymethyl)-benzodioxan-6-yl)[2-trifluoramethyl-4-(*E*-((3-(pyrrolidin-2-on-1-yl)prop-1-ylamino)carbonyl)ethenyl)phenyl]sulfide;
(3-Hydroxymethyl)-benzodioxan-6-yl)[2-nitro-4-(*E*-((3-(pyrrolidin-2-on-1-yl)prop-1-ylamino)carbonyl)ethenyl)phenyl]sulfide;
(Benzodioxan-6-yl)[2-chloro-4-(*E*-((3-carboxypiperidin-1-yl)carbonyl)ethenyl)phenyl]
(2-(and 3-)(Aminomethyl)-benzodioxan-6-yl)[2-trifluoromethyl-4-(*E*-((3-(pyrrolidin-2-on-1-yl)prop-1-ylamino)carbonyl)ethenyl)phenyl]sulfide;
(2-Isopropylphenyl)[2-nitro-4-(*E*-((3-(methylaminocarbonyl)morpholin-1-yl)carbonyl)ethenyl)phenyl]sulfide;
(2-Isopropylphenyl)[2-nitro-4-(*E*-((3-(hydroxymethyl)morpholin-1-yl)carbonyl)ethenyl)phenyl]sulfide;
(2-Isopropylphenyl)[2-nitro-4-(*E*-((3-(acetoxymethyl)morpholin-1-yl)carbonyl)ethenyl)phenyl]sulfide;
(2-Isopropylphenyl)[2-nitro-4-(*E*-((3-(aminomethyl)morpholin-1-yl)carbonyl)ethenyl)phenyl]sulfide;
(2-Isopropylphenyl)[2-nitro-4-(*E*-((3-(acetamidomethyl)morpholin-1-yl)carbonyl)ethenyl)phenyl]sulfide;
(Benzodioxan-6-yl)[2-chloro-4-(*E*-((3-(pyrrolidin-2-on-1-yl)prop-1-ylamino)carbonyl)ethenyl)phenyl]sulfide;
(Benzodioxan-6-yl)[2-chloro-4-(*E*-((3-carboethoxypiperidin-1-yl)carbonyl)ethenyl)phenyl]sulfide;
(Benzodioxan-6-yl)[2-chtoro-4-(*E*-((2-carboethoxypiperidin-1-yl)carbonyl)ethenyl)phenyl]sulfide
(2-Methoxyphenyl)-[2,3-dichtoro-4(*E*-[(morpholin-1-yl)carbonyl]ethenyl)phenyl]sulfide;
(2-Methoxyphenyl)-[2,3-dimethyl-4(*E*-[(morpholin-1-yl)carbonyl]ethenyl)phenyl]sulfide;
(2-Isopropylphenyl)[2-nitro-4-(*E*-((indol-5-ylamino)carbonyl)ethenyl)phenyl]sulfide;
(Benzodioxan-6-yl)[2-chloro-4-(*E*-((3-carboxypiperidin-1-yl)carbonyl)ethenyl)phenyl]sulfide;
(Benzodioxan-6-yl)[2-chloro-4-(*E*-((3-(tetrazol-5-yl)piperidin-1-yl)carbonyl)ethenyl)phenyl]sulfide;
(Benzodioxan-6-yl)[2-chloro-4-(*E*-((4-(*tert*-butoxycarbonyl)piperazin-1-yl)carbonyl)ethenyl)phenyl]sulfide;
(Benzodioxan-6-yl)[2-chloro-4-(*E*-((2-carboxypiperidin-1-yl)carbonyl)ethenyl)phenyl]sulfide;
(Benzodioxan-6-yl)[2-chloro-4-(*E*-((3-(tetrazol-5-yl)morpholin-1-yl)carbonyl)ethenyl)phenyl]sulfide;
(Benzodioxan-6-yl)[2-chloro-4-(*E*-((4-(methylaminocarbonyl)piperazin-1-yl)carbonyl)ethenyl)phenyl]sulfide;
(2-Methoxyphenyl)-[2,3-dichloro-4(*E*-[(4-carboxypiperidin-1-yl)carbonyl]ethenyl)phenyl)sulfide;
(Benzodioxan-6-yl)[2-chloro-4-(*E*-((4-(tetrazol-5-yl)piperidin-1-yl)carbonyl)ethenyl)phenyl]sulfide;
(2-Methoxyphenyl)-[3-chloro-4(*E*-[(morpholin-1-yl)carbonyl]ethenyl)phenyl]sulfide;
(2-Isopropylphenyl)[2-nitro-4-(*E*-((4-oxopiperidin-1-yl)carbonyl)ethenyl)phenyl]sulfide;
(Benzodioxan-6-yl)[2-trifluoromethyl-4-(*E*-((3-R-carboethoxypiperidin-1-yl)carbonyl)ethenyl)phenyl]sulfide;
(Benzodioxan-6-yl)[2-trifluoromethyl-4-(*E*-((3-*R*-carboxypiperidin-1-yl)carbonyl)ethenyl)phenyl]sulfide;
(Benzodioxan-6-yl)[2,3-dichloro-4-(*E*-((3-(pyrrolidin-2-on-1-yl)prop-1-ylamino)carbonyl)ethenyl)phenyl]sulfide;
(Benzodioxan-6-yl)[2,3-dichloro-4-(*E*-((4-acetylpiperazin-1-yl)carbonyl)ethenyl)phenyl]sulfide;
(Benzodioxan-6-yl)[2,3-dichloro-4-(*E*-((3-carboethoxypiperidin-1-yl)carbonyl)ethenyl)phenyl]sulfide;
(Benzodioxan-6-yl)[2,3-dichloro-4-(*E*-((4-carboethoxypiperidin-1-yl)carbonyl)ethenyl)phenyl]sulfide;
(Benzodioxan-6-yl)[2,3-dichloro-4-(*E*-((3-carboxypiperidin-1-yl)carbonyl)ethenyl)phenyl]sulfide;
(Benzodioxan-6-yl)[2,3-dichloro-4-(*E*-((4-carboxypiperidin-1-yl)carbonyl)ethenyl)phenyl]sulfide;
(2-Isopropylphenyl)[2,3-dichloro-4-(*E*-((3-(1-pyrrolidin-2-onyl)propylamino) carbonyl)ethenyl)phenyl]sulfide;
(2-Isopropylphenyl)[2,3-dichloro-4-(*E*-((4-acetylpiperazin-1-yl)carbonyl)ethenyl)phenyl]sulfide;
(2-Isopropylphenyl)[2,3-dichloro-4-(*E*-((3-carboethoxypiperidin-1-yl)carbonyl)ethenyl)phenyl]sulfide;
(2-Isopropylphenyl)[2,3-dichloro-4-(*E*-((4-carboethoxypiperidin-1-yl)carbonyl)ethenyl)phenyl]sulfide;
(2-Isopropylphenyl)[2,3-dichloro-4-(*E*-((3-carboxypiperidin-1-yl)carbonyl)ethenyl)phenyl]sulfide;
(2-Isopropylphenyl)(2,3-dichloro-4-(*E*-((4-carboxypiperidin-1-yl)carbonyl)ethenyl)phenyl]sulfide;
(1-Methylindol-5-yl)[2,3-dichloro-4-(*E*-((3-carboethoxypiperidin-1-yl) carbonyl)ethenyl)phenyl]sulfide;
(1-Methylindol-5-yl)[2,3-dichloro-4-(*E*-((3-carboxypiperidin-1-yl)carbonyl)ethenyl)phenyl]sulfide;
(1-Methylindol-5-yl)[2,3-dichloro-4-(*E*-((4-carboethoxypiperidin-1-yl) carbonyl)ethenyl)phenyl]sulfide;
(1-Methylindol-5-yl)[2,3-dichloro-4-(*E*-((4-carboxypiperidin-1-yl)carbonyl)ethenyl)phenyl]sulfide;
(2-Ethoxyphenyl)-[2,3-dichloro-4(*E*-[(4-carboxypiperidin-1-yl)carbonyl]ethenyl)phenyl]sulfide;
(2-Ethoxyphenyl)-[2,3-dichloro-4(*E*-[(morpholin-1-yl)carbonyl]ethenyl)phenyl]sulfide;
(2-Ethoxyphenyl)-[2,3-dichloro-4(*E*-[(3-carboxypiperidin-1-yl)carbonyl]ethenyl)phenyl]sulfide;
(2-Isopropylphenyl)[2-nitro-4-(*E*-((3-carboethoxypyrrolidin-1-yl)carbonyl)ethenyl)phenyl]sulfide;
(2-Isopropylphenyl)[2-nitro-4-(*E*-((3-carboxypyrrolidin-1-yl)carbonyl)ethenyl)phenyl]sulfide;
(2-Isopropylphenyl)(2,3-difluoro-4-(*E*-((3-carboethoxypiperidin-1-yl)carbonyl)ethenyl)phenyl]sulfide;
(2-Isopropylphenyl)[2,3 -difluoro-4-(*E*-((3-carboxypiperidin-1-yl)carbonyl)ethenyl)phenyl]sulfide;
(2-Isopropylphenyl)[2,3-difluoro-4-(*E*-((4-carboxypiperidin-1-yl)carbonyl)ethenyl)phenyl]sulfide;
(Benzodioxan-6-yl)[2-trifluoromethyl-4-(*E*-((3-ethoxycarbonylpyrrolidin-1-yl)carbonyl)ethenyl)phenyl]sulfide
(Benzodioxan-6-yl)[2-trifluoromethyl-4-(*E*-((3-carboxypyrrolidin-1-yl)carbonyl)ethenyl)phenyl]sulfide;
(2-Methoxyphenyl)[2-chloro-3 -trifluoromethyl-4-(*E*-((4-carboethoxypiperidin-1-yl)carbonyl)ethenyl)phenyl]sulfide;
(2-Methoxyphenyl)[2-chloro-3-trifluoromethyl-4-(*E*-((4-carboethoxypiperidin-1-yl)carbonyl)ethenyl)phenyl)sulfide;
(2-Methoxyphenyl)[2-chloro-3-trifluoromethyl-4-(*E*-((morpholin-1-yl)carbonyl)ethenyl)phenyl]sulfide;
(Benzodioxan-6-yl) [4-(*E*-((4-carboxypiperidin-1-yl)carbonyl)ethenyt)naphthyl] sulfide;
(2-Methoxyphenyl) [2,3-dichloro-4-(*E*-((4-(spiro-hydantoin-5-yl)-piperidin-1-yl)carbonyl)ethenyl)phenyl]sulfide;
(2-Methoxyphenyl) [2,3-dichloro-4-(*E*-(4-(2-(2-hydroxyethoxy)ethyl)piperazin-1-yl)carbonyl)ethenyl)phenyl]sulfide;
(2-Methoxyphenyl)[2,3-dichloro-4-(*E*-((4-ethylpiperazin-1-yl)carbonyl)ethenyl)phenyl]sulfide;
(2-Isopropylphenyl)[ 2,3-dichloro-4-(*E*-((4-(2-(2-hydroxyethoxy)ethyl)piperazin-1-yl)carbonyl)ethenyl)phenyl]sulfide;
(Benzodioxan-6yl)[2,3-bis(trifluoromethyl)-4-(*E*-((4-carboxypiperidin-1-yl)carbonyl)ethenyl)phenyl]sulfide;
(2-Methoxyphenyl) [2,3-dichloro-4-(*E*-((4-(carboxymethylamino)carbonyl-piperidin-1-yl)carbonyl)ethenyl)phenyl]sulfide;
(2-Methoxyphenyl) [2,3-bis(trifluoromethyl)-4-(*E*-((4-carboxymethylpiperazin-1-yl) carbonyl)ethenyl)phenyl]sulfide;
(2-Methoxyphenyl)[2,3-bis(trifluoromethyl)-4-(*E*-((4-N-(2-hydroxyethyl)piperazin-1-yl)carbonyl)ethenyl)phenyl]sulfide;
(1-Methylindol-5-yl)[2,3-dichloro-4-(*E*-((4-(carbo-2,3-dihydroxypropylamino)piperidin-1-yl)carbonyl)ethenyl)phenyl]sulfide;
(2-Methoxyphenyl) [2,3-dichloro-4-(*E*-(4-(2,3-dihydroxypropionyl)piperazin-1-yl)carbonyl)ethenyl)phenyl]sulfide;
(2-Methoxyphenyl) [2,3-dichloro-4-(*E*-(4-(2,3-dihydroxy-3-carboxypropionyl)piperazin-1-yl)carbonyl)ethenyl)phenyl]sulfide;
(1-Methylindol-5-yl)[2,3-dichloro-4-(*E*-((4-(carboxymethylamino)carbonylpiperidin-1-yl)carbonyl)ethenyl)phenyl]sulfide;
(1-Methylindol-5-yl)[2,3-dichloro-4-(*E*-((4-sulfopiperidin-1-yl)carbonyl)ethenyl)phenyl]sulfide;
(1-Methylindol-5-yl)[2,3-dichloro-4-(*E*-(4-methylhomopiperazin-1-ylcarbonyl)ethenyl)phenyl]sulfide;
(1-Methylindol-5-yl)[2,3-dichloro-4-(*E*-(4-tetrohydrofuroylpiperazin-1-yl)carbonyl)ethenyl)phenyl]sulfide;
(2-Methoxyphenyl) [2,3-dichloro-4-(*E*-((4-amino-4-carboxypiperidin-1-yl)carbonyl)ethenyl)phenyl]sulfide;
(2-Methoxyphenyl)[2,3-dichloro-4-((4-furoylpiperazin-1-yl)carbonyl)ethenyl)phenyl]sulfide;
(1-Methylindol-5-yl)[2,3-dichloro-4-(*E*-(4-(carbo-3-sulfopropylamino)piperadin-1-yl)carbonyl)ethenyl]phenyl]sulfide;
(2-Methoxyphenyl)[2,3-dichloro-4-(*E*-(4-acetylamino-4-carboxypiperidin-1-ylcarbonyl)ethenyl)phenyl]sulfide;
(2-Methoxyphenyl)[2,3-bis(trifluoromethyl)-4-(*E*-((4-carboxypiperidin-1-yl)carbonyl)ethenyl)phenyl]sulfide;
(2-Methoxyphenyl) 5-[8-(*E*-((4-(aminocarbonyl)piperidin-1-yl)carbonyl)ethenyl)quinolinyl]sulfide;
(2-Methoxyphenyl) [2-trifluoromethyl-4-(*E*-((4-carboxypiperidin-1-yl)carbonyl)ethenyl)phenyl]sulfide;
(1-Methylindol-5-yl)[2,3-dichloro-4-(*E*/*Z*-((1S,4S)-2,5-diazabycyclo(2,2,1)heptan-2-ylcarbonyl)ethenyl)-2,3-dichlorophenyl]sulfide;
(1-Methylindol-5-yl)[2,3-dichloro-4-(*E*-(4-hydroxy-3-carboxypiperadin-1-ylcarbonyl)ethenyl)phenyl]sulfide;;
(1-Methylindol-5-yl)[2,3-dichloro-4-(*E*-(S-oxothiomorpholin-1-ylcarbonyl)ethenyl)phenyl]sulfide;
(2-Methoxyphenyl) [2,3-dichloro-4-(*E*-((4-sulfophenylamino)carbonyl)ethenyl)phenyl]sulfide;
(2-Methoxyphenyl) [2,3-dichloro-4-(*E*-((4-carboxyphenylamino)carbonyl)ethenyl)phenyl]sulfide; and
[3-(4-Morpholino)phenyl][2,3-dichloro-4-(*E*-((4-carboxypiperidin-1-yl)carbonyl]ethenyl)phenyl]sulfide.

13. A compound according to Claim 1 selected from:
(2-Formylphenyl)[2-nitro-4-(*E*-((4-acetylpiperazin-1-yl)carbonyl)ethenyl)phenyl]sulfide;
(2-Ethoxyphenyl)-[2-chloro-4(*E*-[(morpholin-1-yl)carbonyl]ethenyl)phenyl]sulfide;
(2-Isopropylphenyl)[2-nitro-4-(*E*-((3-dimethylaminocarbonyl-4-acetylpinerazin-1-yl)carbonyl)ethenyl)phenyl]sulfide;
(2-Isopropylphenyl)[2-nitro-4-(*E*-((4-methoxycarbonylpiperazin-1-yl)carbonyl)ethenyl)phenyl]sulfide;
(2-Ethoxyhenyl)[2-trifluoromethyl-4-(*E*-((2-carboxy-4-(methoxycarbonyl)piperazin-1-yl)carbonyl)ethenyl)phenyl]sulfide;
(2-Ethyl-4-bromophenyl)[2-nitro-4-(*E*-((4-acetylpiperazin-1-yl)carbonyl)ethenyl)phenyl]sulfide;
(3-Morpholinophenyl)[2-nitro-4-(*E*-((4-acetylpinerazin-1-yl)carbonyl)ethenyl)phenyl]sulfide;
(2-Isopropylphenyl)[2-nitro-4-(*E*-((3-carboethoxypiperidin-1-yl)carbonyl)ethenyl)phenyl]sulfide;
(2-Isopropylphenyl)[2-nitro-4-(*E*-((3-(1-pyrrolidin-2-onyl)prop-1-ylamino) carbonyl)ethenyl)phenyl]sulfide;
(2-(Hydroxymethyl)-benzodioxan-6-yl)[2-chloro-4-(*E*-((4-acetylpiperazin-1-yl)carbonyl)ethenyl)phenyl]sulfide;
(Benzodioxan-6-yl)[2-trifluoromethyl-4-(*E*-((3-carboxypyrrolidin-1-yl)carbonyl)ethenyl)phenyl]sulfide;
(Benzodioxan-6-yl)[2-trifluoromethyl-4-(*E*-((4-carboethoxypiperidin-1-yl)carbonyl)ethenyl)phenyl]sulfide;
(Benzodioxan-6-yl)[2-trifluoromethyl4-(*E*-((2-carbomethoxy-4-methoxycarbonylpiperazin-1-yl)carbonyl)ethenyl)phenyl]sulfide;
(Benzodioxan-6-yl)[2-trifluoromethyl-4-(*E*-((2-carbomethoxypiperazin-1-yl)carbonyl)ethenyl)phenyl]sulfide;
(Benzodioxan-6-yl)[2-chloro-4-(*E*-((4-(methylaminocarbonyl)piperazin-1-yl) carbonyl)ethenyl)phenyl]sulfide;
(2-Methoxyphenyl)-[2,3-dichloro-4(*E*-[(4-carboxypiperidin-1-yl)carbonyl]ethenyl)phenyl]sulfide;
(Benzodioxan-6-yl)[2-trifluoromethyl-4-(*E*-((3-*R*-carboethoxypiperidin-1-yl)carbonyl)ethenyl)phenyl]sulfide;
(Benzodioxan-6-yl)[2,3-dichloro-4-(*E*-((3-[pyrrolidin-2-on-1-yl)proo-1-ylamino)carbonyl)ethenyl)phenyl]sulfide;
(Benzodioxan-6-yl)[2,3-dichloro-4-(*E*-((4-acetylpiperazin-1-yl)carbonyl)ethenyl)phenyl]sulfide;
(Benzodioxan-6-yl)[2,3-dichloro-4-(*E*-((3-carboethoxypioeridin-1-yl) carbonyl)ethenyl)phenyl]sulfide;
(Benzodioxan-6-yl)[2,3-dichloro-4-(*E*-((4-carboethoxypiperidin-1-yl) carbonyl)ethenyl)phenyl]sulfide;
(Benzodioxan-6-yl)[2,3-dichloro-4-(*E*-((3-carboxypiperidin-1-yl)carbonyl)ethenyl)phenyl]sulfide;
(Benzodioxan-6-yl)[2,3-dichloro-4-(*E*-((4-carboxypiperidin-1-yl)carbonyl)ethenyl)phenyl]sulfide;
(2-Isopropylphenyl)[2,3-dichloro-4-(*E*-((4-acetylpiperazin-1-yl)carbonyl)ethenyl)phenyl]sulfide;
(2-Isopropylphenyl)[2,3-dichloro-4-(*E*-((3-carboethoxypiperidin-1-yl) carbonyl)ethenyl)phenyl]sulfide;
(2-Isopropylphenyl)[2,3-dichloro-4-(*E*-((3-carboxypiperidin-1-yl)carbonyl)ethenyl)phenyl]sulfide;
(2-Isopropylphenyl)[2,3-dichloro-4-(*E*-((4-carboxypiperidin-1-yl)carbonyl)ethenyl)phenyl]sulfide;
(2-Methoxyphenyl) [2,3-dichloro-4-(*E*-((4-(carboxymethylamino)carbonyl-piperidin-1-yl)carbonyl)ethenyl)phenyl]sulfide;
(2-Methoxyphenyl)[2,3-bis(trifluoromethyl)-4-(*E*-((4-carboxymethylpiperazin-1-yl) carbonyl)ethenyl)phenyl]sulfide; and
(2-Methoxyphenyl)[2,3-bis(trifluoromethyl)-4-(*E*-((4-N-(2-hydroxyethyl)piperazin-1-yl)carbonyl)ethenyl)phenyl]sulfide.

14. A compound according to Claim 1 wherein Ar is selected from the group consisting of methoxyphenyl and isopropylphenyl.

15. A compound according to Claim 1 wherein Ar is benzodioxan or substituted benzodioxan.

16. A compound according to Claim 1 wherein R₃ is a "trans-cinnamide"; and Ar is selected from 1,3-benzimidazol-2-one, 1,4-benzodioxane, 1,3-benzodioxole, 1-benzopyr-2-en-4-one, indole, isatin, phenyl, 1,3-quinazolin-4-one, and quinoline.

17. A compound according to Claim 1 wherein R₁₀ and R₁₁ are independently selected from hydrogen, alkyl, cycloalkyl, alkoxycarbonylalkyl, hydroxyalkyl, and heterocyclylalkyl.

18. A compound according to Claim 1 wherein NR₁₀R₁₁ is heterocyclyl or substituted heterocyclyl.

19. A pharmaceutical composition comprising a compound of Claim 1 in a pharmaceutically-acceptable carrier.

20. Use of a compound of claim 1 or 12 for preparing a medicament for inhibiting inflammation in a mammal.

21. Use of a compound of claim 1 or 12 for preparing a medicament for suppressing immune response in a mammal.

22. A process for preparing a compound of claim 1, which process comprises activating the acid group in a compound of the formula 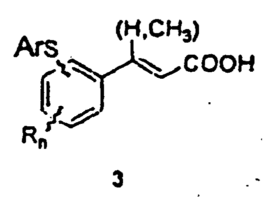 and subsequently reacting it with a primary or secondary amine of the formula NR₁₀ R₁₁, where all the variables are as defined in claim 1.

## Patentansprüche

1. Verbindung der Formel I 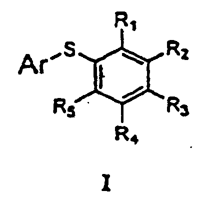 wobei R₁, R₂, R₃, R₄ und R₅ unabhängig ausgewählt sind aus
a. Wasserstoff;
b. Halogen;
c. Alkyl;
d. Halogenalkyl;
e. Alkoxy;
f. Cyano;
g. Nitro;
h. Carboxaldehyd;
mit der Maßgabe, dass wenigstens einer der Reste R₁ bis R₃ ein "cis-Cinnamid" oder ein "trans-Cinnamid" ist, die definiert sind als 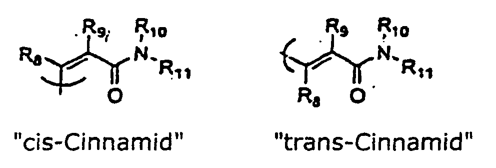 wobei
R₈ und R₉ unabhängig ausgewählt sind aus
a. Wasserstoff;
b. Alkyl;
c. Carboxyalkyl;
d. Alkylaminocarbonylalkyl; und
e. Dialkylaminocarbonylalkyl; und
R₁₀ und R₁₁ unabhängig ausgewählt sind aus
a. Wasserstoff;
b. Alkyl;
c. Cycloalkyl;
d. Alkoxycarbonylalkyl;
e. Hydroxyalkyl;
f. Heterocyclyl, das einen vier-, fünf-, sechs- oder siebengliedrigen Ring darstellt, der ein, zwei oder drei Heteroatome enthält, die unabhängig aus Stickstoff, Sauerstoff und Schwefel ausgewählt sind, und der mit einem oder zwei Ringen kondensiert sein kann, die unabhängig ausgewählt sind aus einem Arylring, wie er unten definiert ist, einem Cyclohexanring, einem Cyclohexenring, einem Cyclopentanring, einem Cyclopentenring oder einem anderen monocyclischen heterocyclischen Ring;
g. Heterocyclylalkyl, wobei das Heterocyclyl wie oben definiert ist;
h. Heterocyclylamino, wobei das Heterocyclyl wie oben definiert ist;
i. einer Heterocyclylgruppe, wie sie oben unter f. definiert ist und die Substituenten aufweist, die unabhängig aus Alkyl, Halogen, Hydroxy und Alkoxy ausgewählt sind;
j. einer Heterocyclylalkylgruppe, wie sie oben unter g. definiert ist und die Substituenten aufweist, die unabhängig aus Alkyl, Halogen, Hydroxy und Alkoxy ausgewählt sind; oder
wobei NR₁₀R₁₁ eine Heterocyclyl- oder substituierte Heterocyclylgruppe ist,
wobei die Heterocyclylgruppe wie oben unter f. definiert ist und die Substituenten unabhängig ausgewählt sind aus
1) Alkyl;
2) Alkoxy;
3) Alkoxyalkyl;
4) Cycloalkyl;
5) einer Arylgruppe, bei der es sich um ein mono- oder bicyclisches carbocyclisches Ringsystem handelt, das einen oder zwei aromatische Ringe aufweist, die mit einem Cyclohexan-, Cyclohexen-, Cyclopentanoder Cyclopentenring kondensiert sein können;
6) einer Heterocyclylgruppe, wie sie oben definiert ist;
7) Heterocyclylcarbonyl, wobei das Heterocyclyl wie oben definiert ist;
8) Heterocyclylalkylaminocarbonyl, wobei das Heterocyclyl wie oben definiert ist;
9) Hydroxy;
10) Hydroxyalkyl;
11) Hydroxyalkoxyalkyl;
12) Carboxy;
13) Carboxyalkyl;
14) Carboxycarbonyl;
15) Carboxaldehyd;
16) Alkoxycarbonyl;
17) Arylalkoxycarbonyl, wobei das Aryl wie oben definiert ist;
18) Aminoalkyl;
19) Aminoalkanoyl;
20) Carboxamido;
2.1) Alkoxycarbonylalkyl;
22) Carboxamidoalkyl;
23) Cyano;
24) Tetrazolyl;
25) substituiertes Tetrazolyl;
26) Alkanoyl;
27) Hydroxyalkanoyl;
28) Alkanoyloxy;
29) Alkanoylamino;
30) Alkanoyloxyalkyl;
31) Alkanoylaminoalkyl;
32) Sulfonat;
33) Alkylsulfonyl;
34) Alkylsulfonylaminocarbonyl;
35) Arylsulfonylaminocarbonyl, wobei das Aryl wie oben definiert ist; und
36) Heterocyclylsulfonylaminocarbonyl, wobei die Heterocyclylgruppe wie oben definiert ist; und
wobei Ar eine substituierte Aryl- oder substituierte Heteroarylgruppe ist, die einen oder zwei aromatische Ringe aufweist, welche mit einem Cyclohexan-, Cyclohexen-, Cyclopentan- oder Cyclopentenring kondensiert sein können, wobei Substituenten unabhängig ausgewählt sind aus:
a. Wasserstoff;
b. Halogen;
c. Alkyl;
d. einer Arylgruppe, wie sie oben definiert ist;
e. Halogenalkyl;
f. Hydroxy;
g. Alkoxy;
h. Alkoxyalkyl;
i. Alkoxycarbonyl;
j. Alkoxyalkoxy;
k. Hydroxyalkyl;
l. Aminoalkyl;
m. Aminocarbonyl;
n. Alkyl(alkoxycarbonylalkyl)aminoalkyl;
o. einer Heterocyclylgruppe, wie sie oben definiert ist;
p. einer Heterocyclylalkylgruppe, wie sie oben definiert ist;
q. einer Heterocyclylgruppe, wie sie oben definiert ist und die Substituenten aufweist, die unabhängig aus Alkyl, Halogen, Hydroxy und A)koxy ausgewählt sind;
r. Carboxaldehyd;
s. Carboxaldehydhydrazon;
t. Carboxamid;
u. Alkoxycarbonylalkyl;
v. Carboxy;
w. Carboxyalkyl;
x. Hydroxycarbonylalkyl(carboxyalkyl);
y. Hydroxyalkylaminocarbonyl;
z. Cyano;
aa. Amino;
bb. Heterocyclylalkylamino, wobei das Heterocyclyl wie oben definiert ist;
cc. Heterocyclylaminocarbonyl, wobei das Heterocyclyl wie oben definiert ist; und
dd. "trans-Cinnamid";
mit den folgenden Maßgaben:
(i) wenn Ar Phenyl ist und R₁, R₂, R₄ und R₅ gleichzeitig Wasserstoff sind, dann ist R₃ kein cis-Cinnamid oder trans-Cinnamid, bei dem R₈ Methyl ist, R₉ Wasserstoff ist und R₁₀ und R₁₁ gleichzeitig -C₂H₅ sind; und
(ii) wenn Ar Pyridyl oder C₁₋₃-alkylsubstituiertes Pyridyl ist und R₁, R₂, R₄ und R₅ gleichzeitig Wasserstoff sind, dann ist R₃ kein cis-Cinnamid oder trans-Cinnamid, bei dem R₈ Wasserstoff ist, R₉ Wasserstoff oder C₁₋₃-Alkyl ist und R₁₀ und R₁₁ unabhängig voneinander Wasserstoff, C₁₋₄-Alkyl oder C₃₋₆-Cycloalkyl sind;
oder ein pharmazeutisch annehmbares Salz davon.

2. Verbindung gemäß Anspruch 1, wobei R₁ ein "cis-Cinnamid" oder "trans-Cinnamid" ist und R₃ Wasserstoff ist.

3. Verbindung gemäß Anspruch 1, wobei R₃ ein "cis-Cinnamid" oder "trans-Cinnamid" ist und R₁ Wasserstoff ist.

4. Verbindung gemäß Anspruch 1, wobei R₃ ein "cis-Cinnamid" oder "trans-Cinnamid" ist und R₁, R₈ und R₉ Wasserstoff sind.

5. Verbindung gemäß Anspruch 4, wobei R₃ ein "cis-Cinnamid" ist.

6. Verbindung gemäß Anspruch 4, wobei R₃ ein "trans-Cinnamid" ist.

7. Verbindung gemäß Anspruch 1, wobei R₃ ein "cis-Cinnamid" oder "trans-Cinnamid" ist, R₁, R₂ und R₄ unabhängig Wasserstoff oder Alkyl sind und R₅ aus Halogen, Halogenalkyl und Nitro ausgewählt ist.

8. Verbindung gemäß Anspruch 4, wobei Ar Aryl, substituiertes Aryl, Heteroaryl oder substituiertes Heteroaryl ist.

9. Verbindung gemäß Anspruch 4, wobei R₁₀ und R₁₁ jeweils unabhängig aus Wasserstoff, Alkyl, Cycloalkyl, Alkoxycarbonylalkyl, Hydroxyalkyl und Heterocyclylalkyl ausgewählt sind.

10. Verbindung gemäß Anspruch 4, wobei NR₁ₒR₁₁ Heterocyclyl oder substituiertes Heterocyclyl ist.

11. Verbindung gemäß Anspruch 8, wobei Ar aus substituiertem Phenyl, 1,3-Benzimidazol-2-on, 1,4-Benzodioxan, 1,3-Benzodioxol, 1-Benzopyr-2-en-4-on, Indol, Isatin, 1,3-Chinazolin-4-on und Chinolin ausgewählt ist.

12. Verbindung gemäß Anspruch 1, die ausgewählt ist aus:
(2,4-Dichlorphenyl)[2-(E-((6-hydroxyhexylamino)carbonyl)ethenyl)phenyl]sulfid;
(2,4-Dichlorphenyl)[2-(E-((3-(1-imidazolyl)propylamino)carbonyl)ethenyl)phenyl]sulfid;
(2,4-Dichlorphenyl)[2-chlor-4-(E-((2-hydroxyethylamino)carbonyl)ethenyl)phenyl]sulfid;
(2,4-Dichlorphenyl)[2-chlor-4-(E-((6-hydroxyhexylamino)carbonyl)ethenyl)phenyl]sulfid;
(2,4-Dichlorphenyl)[2-chlor-4-(E-((bis(2-hydroxyethyl)amino)carbonyl)ethenyl)phenyl]sulfid;
(2,4-Dichlorphenyl)[2-chlor-4-(E-((3-(1-pyrrolidin-2-onyl)propylamino)carbonyl)ethenyl)phenyl]sulfid;
(2,4-Dichlorphenyl)[2-chlor-4-(E-((1-morpholinyl)carbonyl)ethenyl)phenyl]sulfid;
(2,4-Dichlorphenyl)[2-chlor-4-(E-((4-methylpiperazin-1-yl)carbonyl)ethenyl)phenyl]sulfid;
(2,4-Dichlorphenyl)[2-chlor-4-(E-((4-acetylpiperazin-i-yl)carbonyl)ethenyl)phenyl]sulfid;
(2,4-Dichlorphenyl)[2-chlor-4-(E-((4-(2-pyridyl)piperazin-1-yl)carbonyl)ethenyl)phenyl]sulfid;
(2-(Hydroxymethyl)phenyl)[2-chlor-4-(E-((1-morpholinyl)carbonyl)ethenyl)phenyl]sulfid;
(2-Bromphenyl)[2-chlor-4-(E-((1-morpholinyl)carbonyl)ethenyl)phenyl]sulfid;
(2,4-Dichlorphenyl)[2-chlor-4-(E-((4-(2-hydroxyethyl)piperazin-1-yl)carbonyl)ethenyl)phenyl]sulfid;
(2,4-Dichlorphenyl)[2-chlor-4-(E-((4-(2-hydroxyethoxyethyl)piperazin-1-yl)carbonyl)ethenyl)phenyl]sulfid;
(2-Bromphenyl)[2-chlor-4-(E-((3-(hydroxymethyl)piperidin-1-yl)carbonyl)ethenyl)phenyl]sulfid;
(2-Bromphenyl)[2-chlor-4-(E-((2-(hydroxymethyl)piperidin-1-yl)carbonyl)ethenyl)phenyl]sulfid;
(2-Bromphenyl)[2-chlor-4-(E-((3-acetamidopyrrolidin-1-yl)carbonyl)ethenyl)phenyl)sulfid;
(2-Bromphenyl)[2-chlor-4-(E-((4-hydroxypiperidin-1-yl)carbonyl)ethenyl)phenyl]sulfid;
(2-Bromphenyl)(2-chlor-4-(E-((piperidin-1-yl)carbonyl)ethenyl)phenyl]sulfid;
(2,4-Dichlorphenyl)[2-chlor-4-(E-((3-carboxypiperidin-1-yl)carbonyl)ethenyl)phenyl]sulfid;
(2,4-Dichlorphenyl)[2-chlor-4-(E-((4-carboxypiperidin-1-yl)carbonyl)ethenyl)phenyl]sulfid;
(2-Bromphenyl)[2-chlor-4-(E-((4-acetylhomopiperazin-1-yl)carbonyl)ethenyl)phenyl]sulfid;
(2-Bromphenyl)[2-chlor-4-(E-((thiomorpholin-1-yl)carbonyl)ethenyl)phenyl]sulfid;
(2-Bromphenyl)[2-chlor-4-(E-((4-(1-benzimidazol-2-onyl)piperidin-1-yl)carbonyl)ethenyl)phenyl]sulfid;
(2-Bromphenyl)[2-chlor-4-(E-((2-tetrahydroisochinolinyl)carbonyl)ethenyl)phenyl]sulfid;
(2-Methylphenyl)[2-trifluormethyl-4-(E-((4-acetylpiperazin-1-yl)carbonyl)ethenyl)phenyl]sulfid;
(2-Methylphenyl)[2-trifluormethyl-4-(E-((1-morpholinyl)carbonyl)ethenyl)phenyl]sulfid;
(2-Methylphenyl)[2-trifluormethyl-4-(E-((2-(1-morpholinyl)ethylamino)carbonyl)ethenyl)phenyl]sulfid;
(2-Methylphenyl)(2-trifluormethyl-4-(E-((4-phenylpiperazin-1-yl)carbonyl)ethenyl)phenyl]sulfid;
(2-Methylphenyl)(2-trifluormethyl-4-(E-((3-(1-pyrrolidin-2-onyl)propylamino)carbonyl)ethenyl)phenyl]sulfid;
(2-Methylphenyl)[2-trifluormethyl-4-(E-((cyclopropylamino)carbonyl)ethenyl)phenyl]sulfid;
(2,4-Dichlorphenyl)[2-nitro-4-(E-((4-acetylpiperazin-1-yl)carbonyl)ethenyl)phenyl)sulfid;
(2,4-Dichlorphenyl)[2-nitro-4-(E-((3-(1-pyrrolidin-2-onyl)propylamino)carbonyl)ethenyl)phenyl]sulfid;
(2,3-Dichlorphenyl)[2-nitro-4-(E-((4-acetylpiperazin-1-yl)carbonyl)ethenyl)phenyl]sulfid;
(4-Bromphenyl)[2-nitro-4-(E-((4-acetylpiperazin-1-yl)carbonyl)ethenyl)phenyl]sulfid;
(4-Methylphenyl)[2-nitro-4-(E-((4-acetylpiperazin-1-yl)carbonyl)ethenyl)phenyl]sulfid;
(2,4-Dichlorphenyl)[2-nitro-4-(E-((4-(tert-butoxycarbonyl)piperazin-1-yl)carbonyl)ethenyl)phenyl]sulfid;
(2,4-Dichlorphenyl)[2-nitro-4-(E-((4-(2-furoylcarbonyl)piperazin-1-yl)carbonyl)ethenyl)phenyl]sulfid;
(2,4-Dichlorphenyl)[2-nitro-4-(E-((4-(methansulfonyl)piperazin-1-yl)carbonyl)ethenyl)phenyl]sulfid;
(2,4-Dichlorphenyl)[2-nitro-4-(E-((4-(diethylaminocarbonylmethyl)piperazin-1-yl)carbonyl)ethenyl)phenyl]sulfid;
(2,4-Dichlorphenyl)[2-nitro-4-(E-((4-(diethylaminocarbonyl)piperazin-1yl)carbonyl)ethenyl)phenyl]sulfid;
(2,4-Dichlorphenyl)[2-nitro-4-(E-((4-(tert-butoxycarbonylmethyl)piperazin-1-yl)carbonyl)ethenyl)phenyl]sulfid;
(2,4-Dichlorphenyl)[2-nitro-4-(E-((4-(carboxycarbonyl)piperazin-1-yl])carbonyl)ethenyl)phenyl]sulfid;
(2,4-Dichlorphenyl)[2-nitro-4-(E-((4-(carboxymethyl)piperazin-1-yl)carbonyl)ethenyl)phenyl]sulfid;
(2-Methylphenyl)[2-nitro-4-(E-((4-acetylpiperazin-1-yl)carbonyl)ethenyl)phenyl]sulfid;
(2-Chlorphenyl)[2-nitro-4-(E-((4-acetylpiperazin-1-yl)carbonyl)ethenyl)phenyl]sulfid;
(2-Aminophenyl)[2-nitro-4-(E-((4-acetylpiperazin-1-yl)carbonyl)ethenyl)phenyl]sulfid;
(2-Hydroxymethylphenyl)[2-nitro-4-(E-((4-acetylpiperazin-1-yl)carbonyl)ethenyl)phenyl]sulfid;
(2-Ethylphenyl)[2-nitro-4-(E-((4-acetylpiperazin-1-yl)carbonyl)ethenyl)phenyl]sulfid;
(2-Isopropylphenyl)[2-nitro-4-(E-((4-acetylpiperazin-1-yl)carbonyl)ethenyl)phenyl]sulfid;
(2-tert-Butylphenyl)[2-nitro-4-(E-((4-acetylpiperazin-1-yl)carbonyl)ethenyl)phenyl]sulfid;
(2-Chlorphenyl)[2-chlor-4-(E-((4-acetylpiperazin-1-yl)carbonyl))-2-propenyl)phenyl]sulfid;
(2-(1-Morpholinylmethyl)phenyl)[2-chlor-4-(E-((1-morpholinyl)carbonyl)ethenyl)phenyl]sulfid;
(2-(4-(1,3-Benzodioxolyl-5-methyl)piperazin-1-ylmethyl)phenyl)[2-chlor-4-(E-((1-morpholinyl)carbonyl)ethenyl)phenyl]sulfid;
(2-(4-(Isopropylaminocarbonylmethyl)piperazin-1-ylmethyl)phenyl)[2-chlor-4-(E-((1-morpholinyl)carbonyl)ethenyl)phenyl]sulfid;
(2-((N-Ethoxycarbonylmethyl-N-methyl)aminomethyl)phenyl)[2-chlor-4-(E-((1-morpholinyl)carbonyl)ethenyl)phenyl]sulfid;
(2-Formylphenyl)[2-chlor-4-(E-((1-morpholinyl)carbonyl)ethenyl)phenyl]sulfid;
(2-(4-Formylpiperazin-1-ylmethyl)phenyl)[2-chlor-4-(E-((1-morpholinyl)carbonyl)ethenyl)phenyl]sulfid;
(2-(E-((1-Morpholinyl)carbonyl)ethenyl)phenyl)[2-chlor-4-(E-((1-morpholinyl)carbonyl)ethenyl)phenyl]sulfid;
(2-Formylphenyl)[2-nitro-4-(E-((4-acetylpiperazin-1-yl)carbonyl)ethenyl)phenyl]sulfid;
(2-Formylphenyl)[2-chlor-4-(E-((1-morpholinyl)carbonyl)ethenyl)phenyl]sulfid-N,N-dimethylhydrazon;
(2-((3-(1-Morpholinyl)propyl)-1-amino)phenyl)[2-chlor-4-(E-((1-morpholinyl)carbonyl)ethenyl)phenyl]sulfid;
(2,4-Dichlorphenyl)[2-brom-4-(E-((3-(1-pyrrolidin-2-onyl)propylamino)carbonyl)ethenyl)phenyl]sulfid;
(2,4-Dichlorphenyl)[2-formyl-4-(E-((1-morpholinyl)carbonyl)ethenyl)phenyl]sulfid;
(2-Chlor-6-formylphenyl)(2-chlor-4-(E-((4-acetylpiperazin-1-yl)carbonyl)ethenyl)phenyl]sulfid;
(2-Cyanophenyl)[2-chlor-4-(E-((4-acetylpiperazin-1-yl)carbonyl)ethenyl)phenyl]sulfid;
(2-Isopropylphenyl)[2-cyano-4-(E-((morpholin-1-yl)carbonyl)ethenyl)phenyl]sulfid;
(2-Bromphenyl)[2-nitro-4-(E-((4-acetylpiperazin-1-yl)carbonyl)ethenyl)phenyl]sulfid;
(2-(Pyrrolidin-1-yl)phenyl)[2-chlor-4-(E-((morpholin-1-yl)carbonyl)ethenyl)phenyl]sulfid;
(2-Methoxyphenyl)[2-chlor-4-(E-[(morpholin-1-yl)carbonyl]ethenyl)phenyl]sulfid;
(2-Isopropylphenyl)[2-nitro-4-(E-((3-carbomethoxypiperazin-1-yl)carbonyl)ethenyl)phenyl]sulfid;
(2-Methylphenyl)[2-nitro-4-(E-((3-carboxamido-4-carbobenzoxypiperazin-1-yl)carbonyl)ethenyl)phenyl]sulfid;
(2-Isopropylphenyl)(2-nitro-4-(E-((2-carbomethoxy-4-tert-butoxycarbonylpiperazin-1-yl)carbonyl)ethenyl)phenyl]sulfid;
(2-Isopropylphenyl)[2-nitro-4-(E-((2-carboxy-4-tert-butoxycarbonylpiperazin-1-yl)carbonyl)ethenyl)phenyl]sulfid;
(2-Isopropylphenyl)[2-trifluormethyl-4-(E-((4-acetylpiperazin-1-yl)carbonyl)ethenyl)phenyl]sulfid;
(2-Isopropylphenyl)[2-trifluormethyl-4-(E-((morpholin-1-yl)carbonyl)ethenyl)phenyl]sulfid;
(2-Isopropylphenyl)[2-trifluormethyl-4-(E-((3-(pyrrolidin-2-on-1-yl)prop-1-ylamino)carbonyl)ethenyl)phenyl]sulfid;
(2-Isopropylphenyl)[2-trifluormethyl-4-(E-((cyclobutylamino)carbonyl)ethenyl)phenyl]sulfid;
(2-Isopropylphenyl)[2-trifluormethyl-4-(E-((cyclopentylamino)carbonyl)ethenyl)phenyl]sulfid;
(2-Isopropylphenyl)[2-trifluormethyl-4-(E-((5-hydroxypent-1-ylamino)carbonyl)ethenyl)phenyl]sulfid;
(2-Isopropylphenyl)[2-nitro-4-(E-((3-carbomethoxy-4-acetylpiperazin-1-yl)carbonyl)ethenyl)phenyl]sulfid;
(2-Biphenyl)[2-chlor-4-(E-((morpholin-1-yl)carbonyl)ethenyl)phenyl]sulfid;
(3,4-Dimethylphenyl)[2-nitro-4-(E-((4-acetylpiperazin-1-yl)carbonyl)ethenyl)phenyl]sulfid;
(2-Bromphenyl)[2-trifluormethyl-4-(E-((4-acetylpiperazin-1-yl)carbonyl)ethenyl)phenyl]sulfid;
(5-Indolyl)[2-chlor-4-(E-((4-acetylpiperazin-1-yl)carbonyl)ethenyl)phenyl]sulfid;
(5-Benzodioxolyl)[2-chlor-4-(E-((4-acetylpiperazin-1-yl)carbonyl)ethenyl)phenyl]sulfid;
(2-Isopropylphenyl)[2-nitro-4-(E-((2-carbomethoxypiperazin-1-yl)carbonyl)ethenyl)phenyl]sulfid;
(2,3-Dimethoxyphenyl)[2-chlor-4-(E-[(morpholin-1-yl)carbonyl]ethenyl)phenyl]sulfid;
(2-Fluorphenyl)[2-nitro-4-(E-((4-acetylpiperazin-1-yl)carbonyl)ethenyl)phenyl]sulfid;
(2-Bromphenyl)[2-trifluormethyl-4-(E-((4-(tert-butoxycarbonyl)piperazin-1-yl)carbonyl)ethenyl)phenyl]sulfid;
(2-(Pyrrolidin-1-yl)phenyl)[2-trifluormethyl-4-(E-((4-(tert-butoxycarbonyl)piperazin-1-yl)carbonyl)ethenyl)phenyl]sulfid;
(3-Carboxamidophenyl)[2-nitro-4-(E-((4-acetylpiperazin-1-yl)carbonyl)ethenyl)phenyl]sulfid;
(3-(Hydroxymethyl)phenyl)[2-nitro-4-(E-((4-acetylpiperazin-1-yl)carbonyl)ethenyl)phenyl]sulfid;
Phenyl[2-trifluormethyl-4-(E-((4-(tert-butoxycarbonyl)piperazin-1-yl)carbonyl)ethenyl)phenyl]sulfid;
(2-Isopropylphenyl)[2-trifluormethyl-4-(E-((2-carbomethoxy-4-(tert-butoxycarbonyl)piperazin-1-yl)carbonyl)ethenyl)phenyl]sulfid;
(2-Isopropylphenyl)[2-nitro-4-(E-((3-(pyridin-4-methylaminocarbonyl)-4-tert-butoxycarbonylpiperazin-1-yl)carbonyl)ethenyl)phenyl]sulfid;
(2-Ethoxyphenyl)[2-chlor-4(E-[(morpholin-1-yl)carbonyl]ethenyl)phenyl]sulfid;
(2-Methoxyphenyl)[2-nitro-4-(E-((4-acetylpiperazin-1-yl)carbonyl)ethenyl)phenyl]sulfid;
(2-(Azetidin-1-yl)phenyl)[2-trifluormethyl-4-(E-((4-(tert-butoxycarbonyl)piperazin-1-yl)carbonyl)ethenyl)phenyl]sulfid;
(2-(Piperidin-1-yl)phenyl)[2-trifluormethyl-4-(E-((4-(tert-butoxycarbonyl)piperazin-1-yl)carbonyl)ethenyl)phenyl]sulfid;
(3-Chlor-2-formylphenyl)[2-chlor-4-(E-((4-acetylpiperazin-1-yl)carbonyl)ethenyl)phenyl]sulfid;
(2-Trifluormethylphenyl)[2-trifluormethyl-4-(E-((4-acetylpiperazin-1-yl)carbonyl)ethenyl)phenyl]sulfid;
(3-Bromphenyl)[2-trifluormethyl-4-(E-((4-acetylpiperazin-1-yl)carbonyl)ethenyl)phenyl]sulfid;
(3,5-Dimethylphenyl)[2-trifluormethyl-4-(E-((4-acetylpiperazin-1-yl)carbonyl)ethenyl)phenyl]sulfid;
(2-Isopropylphenyl)[2-nitro-4-(E-((3-dimethylaminocarbonyl-4-(pyridin-4-carbonyl)piperazin-1-yl)carbonyl)ethenyl)phenyl]sulfid;
(2-Isopropylphenyl)[2-nitro-4-(E-((3-dimethylaminocarbonyl-4-carbomethoxypiperazin-1-yl)carbonyl)ethenyl)phenyl]sulfid;
(2-Isopropylphenyl)[2-nitro-4-(E-((3-dimethylaminocarbonyl-4-acetylpiperazin-1-yl)carbonyl)ethenyl)phenyl]sulfid;
(2-Isopropylphenyl)[2-nitro-4-(E-((3-(1-morpholinocarbonyl)-4-tert-butoxycarbonylpiperazin-1-yl)carbonyl)ethenyl)phenyl]sulfid;
(2-Isopropylphenyl)[2-nitro-4-(E-((3-(pyridin-4-methylaminocarbonyl)piperazin-1-yl)carbonyl)ethenyl)phenyl]sulfid;
(2-Isopropylphenyl)[2-nitro-4-(E-(((3-dimethylaminocarbonyl)piperazin-1-yl)carbonyl)ethenyl)phenyl]sulfid;
(2-Isopropylphenyl)[2-nitro-4-(E-((3-(benzylaminocarbonyl)-4-tert-butoxycarbonylpiperazin-1-yl)carbonyl)ethenyl)phenyl]sulfid;
(2-Isopropylphenyl)[2-nitro-4-(E-((3-(dimethylaminocarbonyl)-4-tert-butoxycarbonylpiperazin-1-yl)carbonyl)ethenyl)phenyl]sulfid;
(2-Bromphenyl)[2-chlor-4-(E-((3-(5S-hydroxymethylpyrrolidin-2-on-1-yl)prop-1-ylamino)carbonyl)ethenyl)phenyl]sulfid;
(2-Bromphenyl)[2-chlor-4-(E-((3-(pyrrolidin-2-on-1-yl)prop-1-ylamino)carbonyl)ethenyl)phenyl]sulfid;
(2-Bromphenyl)[2-chlor-4-(E-(N-methyl-N-(3-(pyrrolidin-2-on-1-yl)prop-1-yl)amino)carbonyl)ethenyl)phenyl]sulfid;
(2-[2-Methoxy]ethoxyphenyl)[2-chlor-4-(E-[(morpholin-1-yl)carbonyl]ethenyl)phenyl]sulfid;
(2-Isopropylphenyl)[2-nitro-4-(E-((3-(morpholinocarbonyl)piperazin-1-yl)carbonyl)ethenyl)phenyl]sulfid;
(2-Isopropylphenyl)[2-nitro-4-(E-((4-tert-butoxycarbonylpiperazin-1-yl)carbonyl)ethenyl)phenyl]sulfid;
(2-Isopropylphenyl)[2-nitro-4-(E-((4-methoxycarbonylpiperazin-1-yl)carbonyl)ethenyl)phenyl]sulfid;
(2-Isopropylphenyl)[2-nitro-4-(E-(4-(pyridin-4-carbonyl)piperazin-1-yl)carbonyl)ethenyl)phenyl]sulfid;
(2-Isopropylphenyl)[2-nitro-4-(E-((3-(pyridin-3-methylaminocarbonyl)-4-tert-butoxycarbonylpiperazin-1-yl)carbonyl)ethenyl)phenyl]sulfid;
(2-Isopropylphenyl)[2-nitro-4-(E-((3-(pyridin-2-methylaminocarbonyl)piperazin-1-yl)carbonyl)ethenyl)phenyl]sulfid;
(2-Isopropylphenyl)[2-nitro-4-(E-((3-(pyridin-3-methylaminocarbonyl)piperazin-1-yl)carbonyl)ethenyl)phenyl]sulfid;
(4-Hydroxyphenyl)[2-nitro-4-(E-((4-acetylpiperazin-1-yl)carbonyl)ethenyl)phenyl]sulfid;
(3,5-Dichlorphenyl)[2-nitro-4-(E-((4-acetylpiperazin-1-yl)carbonyl)ethenyl)phenyl]sulfid;
(2-Bromphenyl)[2-chlor-4-(E-((3-(5S-acetoxymethylpyrrolidin-2-on-1-yl)prop-1-ylamino)carbonyl)ethenyl)phenyl]sulfid;
(2-Bromphenyl)[2-chlor-4-(E-((3-(5S-methoxymethylpyrrolidin-2-on-1-yl)prop-1-ylamino)carbonyl)ethenyl)phenyl]sulfid;
(2-Bromphenyl)[2-chlor-4-(E-((3-(4R-hydroxymethylpyrrolidin-2-on-1-yl)prop-1-ylamino)carbonyl)ethenyl)phenyl]sulfid;
Phenyl[2-nitro-4-(E-((4-acetylpiperazin-1-yl)carbonyl)ethenyl)phenyl]sulfid;
(2-Dimethylaminophenyl)[2-nitro-4-(E-((4-acetylpiperazin-1-yl)carbonyl)ethenyl)phenyl]sulfid;
(3-((2-Hydroxyethyl)aminocarbonyl)phenyl)[2-nitro-4-(E-((4-acetylpiperazin-1-yl)carbonyl)ethenyl)phenyl]sulfid;
(3-((3-(1-Imidazolyl)propyl)aminocarbonyl)phenyl)[2-nitro-4-(E-((4-acetylpiperazin-1-yl)carbonyl)ethenyl)phenyl]sulfid;
(3-((2-(1-Morpholinyl)ethyl)aminocarbonyl)phenyl)[2-nitro-4-(E-((4-acetylpiperazin-1-yl)carbonyl)ethenyl)phenyl]sulfid;
(2-Isopropylphenyl)[2-nitro-4-(E-((3-hydroxymethyl-4-tert-butoxycarbonylpiperazin-1-yl)carbonyl)ethenyl)phenyl]sulfid;
(2-Isopropylphenyl)[2-nitro-4-(E-((4-formylpiperazin-1-yl)carbonyl)ethenyl)phenyl]sulfid;
(2-Isopropylphenyl)[2-nitro-4-(E-((2-hydroxymethyl-4-tert-butoxycarbonylpiperazin-1-yl)carbonyl)ethenyl)phenyl]sulfid;
(2-Ethoxyphenyl)[2-chlor-4-(E-[(3-ethoxycarbonylpiperidin-1-yl)carbonyl]ethenyl)phenyl]sulfid;
(3-Aminophenyl)[2-nitro-4-(E-((4-acetylpiperazin-1-yl)carbonyl)ethenyl)phenyl]sulfid;
(4-Aminophenyl)[2-nitro-4-(E-((4-acetylpiperazin-1-yl)carbonyl)ethenyl)phenyl]sulfid;
(2,4-Dimethylphenyl)[2-nitro-4-(E-((4-acetylpiperazin-1-yl)carbonyl)ethenyl)phenyl]sulfid;
(2,5-Dimethylphenyl)[2-nitro-4-(E-((4-acetylpiperazin-1-yl)carbonyl)ethenyl)phenyl]sulfid;
(4-Methoxyphenyl)[2-nitro-4-(E-((4-acetylpiperazin-1-yl)carbonyl)ethenyl)phenyl]sulfid;
(3-Chlorphenyl)[2-nitro-4-(E-((4-acetylpiperazin-1-yl)carbonyl)ethenyl)phenyl]sulfid;
(2-Chlor-4,5-diaminophenyl)[2-chlor-4-(E-((4-acetylpiperazin-1-yl)carbonyl)ethenyl)phenyl]sulfid;
(3,4-Diaminophenyl)[2-chlor-4-(E-((4-acetylpiperazin-1-yl)carbonyl)ethenyl)phenyl]
(6-Chlorbenzimidazol-2-on-5-yl)[2-chlor-4-(E-((4-acetylpiperazin-1-yl)carbonyl)ethenyl)phenyl]sulfid;
(1-Methylindol-7-yl)[2-chlor-4-(E-((4-acetylpiperazin-1-yl)carbonyl)ethenyl)phenyl]sulfid;
(2-Hydroxy-4-aminophenyl)[2-chlor-4-(E-((4-acetylpiperazin-1-yl)carbonyl)ethenyl)phenyl]sulfid;
(2-Isopropylphenyl)[2-nitro-4-(E-((4-methylpiperazin-1-yl)carbonyl)ethenyl)phenyl]sulfid;
(2-Isopropylphenyl)[2-nitro-4-(E-(4-pyridin-2-carbonyl)piperazin-1-yl)carbonyl)ethenyl)phenyl]sulfid;
(2-Isopropylphenyl)[2-nitro-4-(E-((4-(pyridin-3-carbonyl)piperazin-1-yl)carbonyl)ethenyl)phenyl]sulfid;
(2-Isopropylphenyl)[2-nitro-4-(E-((2-carbomethoxy-4-methoxycarbonylpiperazin-1-yl)carbonyl)ethenyl)phenyl]sulfid;
(2-Isopropylphenyl)[2-nitro-4-(E-((2-carboxy-4-methoxycarbonylpiperazin-1-yl)carbonyl)ethenyl)phenyl]sulfid;
(2-Isopropylphenyl)[2-nitro-4-(E-((3-carbomethoxy-4-methylpiperazin-1-yl)carbonyl)ethenyl)phenyl]sulfid;
(2-Ethoxyphenyl)[2-chlor-4-(E-[(3-carboxypiperidin-1-yl)carbonyl]ethenyl)phenyl]sulfid;
(2-Ethoxyphenyl])[2-chlor-4-(E-[(3-carboxypiperidin-1-yl)carbonyl]ethenyl)phenyl]sulfid;
(2-Ethoxyphenyl)[2-chlor-4(E-[(2-ethoxycarbonylpiperidin-1-yl)carbonyl]ethenyl)phenyl]sulfid;
(2-Ethoxyphenyl)[2-trifluormethyl-4-(E-((1-(tert-butoxycarbonyl)-4-hydroxypyrrolidin-3-ylamino)carbonyl)ethenyl)phenyl]sulfid;
(2-Ethoxyphenyl)[2-chlor-4-(E-[(2-carboxypiperidin-1-yl)carbonyl]ethenyl)phenyl]sulfid;
(2-Ethoxyphenyl)[2-trifluormethyl-4-(E-(((pyrrol-3-in-1-yl)carbonyl)ethenyl)phenyl]sulfid;
(2-Ethoxyphenyl)[2-trifluormethyl-4-(E-((3-(pyrrolidin-2-on-1-yl)prop-1-ylamino)carbonyl)ethenyl)phenyl]sulfid;
(2-Ethoxyphenyl)[2-trifluormethyl-4-(E-((4-acetylpiperazin-1-yl)carbonyl)ethenyl)phenyl]sulfid;
(2-Ethoxyphenyl)[2-trifluormethyl-4-(E-((4-(ethoxycarbonyl)piperazin-1-yl)carbonyl)ethenyl)phenyl]sulfid;
(2-Ethoxyphenyl)[2-trifluormethyl-4-(E-((4-(2-furylcarbonyl)piperazin-1-yl)carbonyl)ethenyl)phenyl]sulfid;
(2-Ethoxyphenyl)[2-chlor-4-(E-[(3-ethoxycarbonylpiperidin-1-yl)carbonyl]ethenyl)phenyl]sulfid;
(2-Ethoxyphenyl)[2-chlor-4-(E-[(4-carboxypiperidin-1-yl)carbonyl]ethenyl)phenyl]sulfid;
(Benzodioxan-6-yl)[2-chlor-4-(E-((4-acetylpiperazin-1-yl)carbonyl)ethenyl)phenyl]sulfid;
(2-Isopropylphenyl)[2-nitro-4-(E-((4-ethoxycarbonylpiperazin-1-yl)carbonyl)ethenyl)phenyl]sulfid;
(2-Isopropylphenyl)[2-nitro-4-(E-((4-isopropoxycarbonylpiperazin-1-yl)carbonyl)ethenyl)phenyl]sulfid;
(2-Isopropylphenyl)[2-nitro-4-(E-((4-isobutoxycarbonylpiperazin-1-yl)carbonyl)ethenyl)phenyl]sulfid;
(2-Isopropylphenyl)[2-nitro-4-(E-((4-((1-propen-2-oxy)carbonyl)piperazin-1-yl)carbonyl)ethenyl)phenyl]sulfid;
(2-Isopropylphenyl)[2-nitro-4-(E-((4-propionylpiperazin-1-yl)carbonyl)ethenyl)phenyl]sulfid;
(2-Isopropylphenyl)[2-nitro-4-(E-((4-carboxamidopiperazin-1-yl)carbonyl)ethenyl)phenyl]sulfid;
(2-Isopropylphenyl)[2-nitro-4-(E-((4-methylaminocarbonylpiperazin-1-yl)carbonyl)ethenyl)phenyl]sulfid;
(2-Isopropylphenyl)[2-nitro-4-(E-((4-(pyrimidin-2-yl)piperazin-1-yl)carbonyl)ethenyl)phenyl]sulfid;
(2-Isopropylphenyl)[2-nitro-4-(E-((4-hydroxyacetylpiperazin-1-yl)carbonyl)ethenyl)phenyl]sulfid;
(2-Isopropylphenyl)[2-nitro-4-(E-((4-(pyrazin-2-carbonyl)piperazin-1-y1)carbonyl)ethenyl)phenyl]sulfid;
(2-Isopropylphenyl)[2-trifluormethyl-4-(E-(((2-carboxypyrrol-3-in-1-yl)carbonyl)ethenyl)phenyl]sulfid;
(2-Isopropylphenyl)[2-nitro-4-(E-((3-hydroxymethyl-4-methylpiperazin-1-yl)carbonyl)ethenyl)phenyl]sulfid;
(2-Isopropylphenyl)[2-trifluormethyl-4-(E-(((2-carboxypyrrol-3-in-1-yl)carbonyl)ethenyl)phenyl]sulfid;
(2-Isopropylphenyl)[2-trifluormethyl-4-(E-(((2-hydroxymethylpyrrolidin-1-yl)carbonyl)ethenyl)phenyl]sulfid;
(2-Isopropylphenyl)[2-nitro-4-(E-((3-methylaminocarbonyl)piperazin-1-yl)carbonyl)ethenyl)phenyl]sulfid;
(2-Isopropylphenyl)[2-nitro-4-(E-(((3-cyclopropylaminocarbonyl)piperazin-1-yl)carbonyl)ethenyl)phenyl]sulfid;
(2-Isopropylphenyl)[2-nitro-4-(E-((3-carboxamidopiperazin-1-yl)carbonyl)ethenyl)phenyl]sulfid;
(2-Isopropylphenyl)[2-nitro-4-(E-((3-carbomethoxy-4-oxopiperidin-1-yl)carbonyl)ethenyl)phenyl]sulfid;
(2-Isopropylphenyl)[2-nitro-4-(E-((3,5-dimethylpiperazin-1-yl)carbonyl)ethenyl)phenyl]sulfid;
(1-Ethylindol-7-yl)[2-chlor-4-(E-((4-acetylpiperazin-1-yl)carbonyl)ethenyl)phenyl]sulfid;
(3-[2-Methoxy]ethoxyphenyl)[2-chlor-4-(E-[(morpholin-1-yl)carbonyl]ethenyl)phenyl]sulfid;
(2-Bromphenyl)[2-chlor-4-(E-((4,4'-S-dioxythiomorpholin-1-yl)carbonyl)ethenyl)phenyl]sulfid;
(2-Bromphenyl)[2-chlor-4-(E-(N-carbomethoxymethyl-N-(3-(pyrrolidin-2-on- 1-yl)prop- 1-yl)amino)carbonyl)ethenyl)phenyl]sulfid;
(2-Bromphenyl)[2-chlor-4-(E-((4-S-oxythiomorpholin-1-yl)-2-pyrrolidinon)carbonyl)ethenyl)phenyl]sulfid;
(2-Methoxy-5-chlorphenyl)[2-nitro-4-(E-((4-acetylpiperazin-1-yl)carbonyl)ethenyl)phenyl]sulfid;
(2-Isopropylphenyl)[2-nitro-4-(E-((3-acetoxymethyl)piperazin-1-yl)carbonyl)ethenyl)phenyl]sulfid;
(2-Isopropylphenyl)[2-nitro-4-(E-((3,5-dimethyl-4-acetylpiperazin-1-yl)carbonyl)ethenyl)phenyl]sulfid;
(1-Methylindol-5-yl)[2-chlor-4-(E-((4-acetylpiperazin-1-yl)carbonyl)ethenyl)phenyl]sulfid;
(Benzodioxan-6-yl)[2-nitro-4-(E-((4-acetylpiperazin-1-yl)carbonyl)ethenyl)phenyl]sulfid;
(Benzodioxan-6-yl)[2-nitro-4-(E-((3-(pyrrolidin-2-on-1-yl)prop-1-ylamino)carbonyl)ethenyl)phenyl]sulfid;
(Benzodioxan-6-yl)[2-nitro-4-(E-((3-carboethoxypiperidin-1-yl)carbonyl)ethenyl)phenyl]sulfid;
(Benzodioxan-6-yl)[2-nitro-4-(E-((4-carboethoxypiperidin-1-yl)carbonyl)ethenyl)phenyl]sulfid;
(2-Ethoxyphenyl)[2-trifluormethyl-4-(Z-((4-acetylpiperazin-1-yl)carbonyl)ethenyl)phenyl]sulfid;
(2-Ethoxyphenyl)[2-trifluormethyl-4-(E-((6-methylpyrid-2-ylamino)carbonyl)ethenyl)phenyl]sulfid;
(2-Methyl-3-chlorphenyl)[2-nitro-4-(E-((4-acetylpiperazin-1-yl)carbonyl)ethenyl)phenyl]sulfid;
(Benzodioxan-6-yl)[2-nitro-4-(E-((3-carboxamidopiperidin-1-yl)carbonyl)ethenyl)phenyl]sulfid;
(Benzodioxan-6-yl)(2-nitro-4-(E-((2-carboethoxypiperidin-1-yl)carbonyl)ethenyl)phenyl]sulfid;
(Benzodioxan-6-yl)[2-nitro-4-(E-((4-carboxamidopiperidin-1-yl)carbonyl)ethenyl)phenyl]sulfid;
(Benzodioxan-6-yl)[2-nitro-4-(E-((4-tert-butoxycarbonylpiperazin-1-yl)carbonyl)ethenyl)phenyl]sulfid;
(2-Isopropylphenyl)[2-nitro-4-(E-((syn-3,5-dimethylmorphalin-1-yl)carbonyl)ethenyl)phenyl]sulfid;
(2-Isopropylphenyl)[2-nitro-4-(E-((anti-3,5-dimethylmorpholin-1-yl)carbonyl)ethenyl)phenyl]sulfid;
(2-Isopropylphenyl)[2-nitro-4-(E-((3-carboethoxypiperazin-1-yl)carbonyl)ethenyl)phenyl]sulfid;
(2-Isopropylphenyl)[2-nitro-4-(E-((3-isopropoxycarbonylpiperazin-1-yl)carbonyl)ethenyl)phenyl]sulfid;
(2-Isopropylphenyl)[2-nitro-4-(E-((3-(dimethylaminocarbonyl)-4-methylpiperazin-1-yl)carbonyl)ethenyl)phenyl]sulfid;
(2-Isopropylphenyl)[2-nitro-4-(E-((3-carbomethoxy-4-hydroxypiperidin-1-yl)carbonyl)ethenyl)phenyl]sulfid;
(2-Isopropylphenyl)[2-nitro-4-(E-((3-hydroxymethyl-4-hydroxypiperidin-1-yl)carbonyl)ethenyl)phenyl]sulfid;
2-Ethoxyphenyl)[2-trifluormethyl-4-(E-((2-carbomethoxy-4-(methoxycaronyl)piperazin-1-yl)carbonyl)ethenyl)phenyl]sulfid;
(2-Ethoxyphenyl)[2-trifluormethyl-4-(E-((2-carbomethoxy-4-methylpiperazin-1-yl)carbonyl)ethenyl)phenyl]sulfid;
(2-Ethoxyphenyl)[2-trifluormethyl-4-(E-((2-carboxy-4-(methoxycarbonyl)piperazin-1-yl)carbonyl)ethenyl)phenyl]sulfid;
(Indol-6-yl)[2-chlor-4-(E-((4-acetylpiperazin-1-yl)carbonyl)ethenyl)phenyl]sulfid;
(1-Ethyl-3-(dimethylaminomethyl)indol-7-yl)[2-chlor-4-(E-((4-acetylpiperazin-1-yl)carbonyl)ethenyl)phenyl]sulfid;
(5-Ethoxybenzodioxan-6-yl)[2-chior-4-(E-((4-acetylpiperazin-1-yl)carbonyl)ethenyl)phenyl]sulfid;
(2-Ethyl-4-bromphenyl)[2-nitro-4-(E-((4-acetylpiperazin-1-yl)carbonyl)ethenyl)phenyl]sulfid;
(Benzodioxan-6-yl)[2-nitro-4-(E-((2-carboxypiperidin-1-yl)carbonyl)ethenyl)phenyl]sulfid;
(Benzodioxan-6-yl)[2-nitro-4-(E-((4-carboxymethylpiperazin-1-yl)carbonyl)ethenyl)phenyl]sulfid;
(3-Morpholinophenyl)[2-nitro-4-(E-((4-acetylpiperazin-1-yl)carbonyl)ethenyl)phenyl]sulfid;
(5-Ethoxybenzodioxan-8-yl)[2-chlor-4-(E-((4-acetylpiperazin-1-yl)carbonyl)ethenyl)phenyl]sulfid;
(5-Chlor-8-ethoxychinolin-7-yl)[2-chlor-4-(E-((4-acetylpiperazin-1-yl)carbonyl)ethenyl)phenyl]sulfid;
(2-Isopropylphenyl)[2-nitro-4-(E-((3-carboethoxypiperidin-1-yl)carbonyl)ethenyl)phenyl]sulfid;
(2-Isopropylphenyl)[2-nitro-4-(E-((3-carboxypiperidin-1-yl)carbonyl)ethenyl)phenyl]sulfid;
(2-Isopropylphenyl)[2-nitro-4-(E-(((3-ethansulfonylaminocarbonyl)piperidin-1-yl)carbonyl)ethenyl)phenyl)sulfid;
(2-Isopropylphenyl)[2-nitro-4-(E-(((3-(4-methylpiperazin)sulfonylaminocarbonyl)piperidin-1-yl)carbonyl)ethenyl)phenyl]sulfid;
(2-Isopropylphenyl)[2-nitro-4-(E-(((3-p-toluolsulfonylaminocarbonyl)piperidin-1-yl)carbonyl)ethenyl)phenyl]sulfid;
(2-Isopropylphenyl)[2-nitro-4-(E-((3-methyl-4-acetylpiperazin-1-yl)carbonyl)ethenyl)phenyl]sulfid;
(2-Hydroxyphenyl)[2-chlor-4-(E-[(morpholin-1-yl)carbonyl]ethenyl)phenyl]sulfid
(1-(Carboxymethyl)indol-5-yl)[2-chlor-4-(E-((4-acetylpiperazin-1-yl)carbonyl)ethenyl)phenyl]sulfid;
(Benzodioxan-6-yl)[2-trifluormethyl-4-(E-((4-acetylpiperazin-1-yl)carbonyl)ethenyl)phenyl]sulfid;
(2-Isopropylphenyl)[2-nitro-4-(E-((3-(1-pyrrolidin-2-onyl)prop-1-ylamino)carbonyl)ethenyl)phenyl]sulfid;
(3-(2-Morpholinoethylamino)phenyl)[2-trifluormethyl-4-(E-((4-acetylpiperazin-1-yl)carbonyl)ethenyl)phenyl]sulfid;
(2-Pyrrolidin-1-ylphenyl)[2-nitro-4-(E-((4-acetylpiperazin-1-yl)carbonyl)ethenyl)phenyl]sulfid;
(3-Bromphenyl)[2-nitro-4-(E-((3-carboethoxypyrrolidin-1-yl)carbonyl)ethenyl)phenyl]sulfid;
(3-Bromphenyl)[2-nitro-4-(E-((4-carboethoxypyrrolidin-1-yl)carbonyl)ethenyl)phenyl]sulfid;
(2-(Hydroxymethyl)benzodioxan-6-yl)[2-chlor-4-(E-((4-acetylpiperazin-1-yl)carbonyl)ethenyl)phenyl]sulfid;
(Benzodioxan-6-yl)[2-trifluormethyl-4-(E-((3-(pyrrolidin-2-on-1-yl)prop-1-ylamino)carbonyl)ethenyl)phenyl]sulfid;
(3-(Dimethylaminomethyl)indol-5-yl)[2-chlor-4-(E-((4-acetylpiperazin-1-yl)carbonyl)ethenyl)phenyl]sulfid;
(2-Isopropylphenyl)[2-nitro-4-(E-((2-carboethoxypiperidin-1-yl)carbonyl)ethenyl)phenyl]sulfid;
(2-Isopropylphenyl)[2-nitro-4-(E-((2-carboxypiperidin-1-yl)carbonyl)ethenyl)phenyl]sulfid;
(2-Isopropylphenyl)[2-nitro-4-(E-((4-carboethoxypiperidin-1-yl)carbonyl)ethenyl)phenyl]sulfid;
(2-Isopropylphenyl)[2-nitro-4-(E-((4-carboxypiperidin-1-yl)carbonyl)ethenyl)phenyl]sulfid;
(2-Isopropylphenyl)[2-nitro-4-(E-(((4-p-toluolsulfonylaminocarbonyl)piperidin-1-yl)carbonyl)ethenyl)phenyl]sulfid;
(2-Isopropylphenyl)[2-nitro-4-(E-((3-carboxy-4-hydroxypiperidin-1-yl)carbonyl)ethenyl)phenyl]sulfid;
(Benzodioxan-6-yl)[2-trifluormethyl-4-(E-((3-carboethoxypiperidin-1-yl)carbonyl)ethenyl)phenyl]sulfid;
(Benzodioxan-6-yl)[2-trifluormethyl-4-(E-((2-carboethoxypiperidin-1-yl)carbonyl)ethenyl)phenyl]sulfid;
(Benzodioxan-6-yl)[2-nitro-4-(E-((4-carboxypiperidin-1-yl)carbonyl)ethenyl)phenyl]sulfid;
(Benzodioxan-6-yl)[2-trifluormethyl-4-(E-((3-carboxypyrrolidin-1-yl)carbonyl)ethenyl)phenyl]sulfid;
(Benzodioxan-6-yl)[2-trifluormethyl-4-(E-((4-carboethoxypiperidin-1-yl)carbonyl)ethenyl)phenyl]sulfid;
(Benzodioxan-6-yl)[2-trifluormethyl-4-(E-((2-carbomethoxy-4-tert-butoxycarbonylpiperazin-1-yl)carbonyl)ethenyl)phenyl]sulfid;
(Benzodioxan-6-yl)[2-trifluormethyl-4-(E-((2-carbomethoxy-4-methoxycarbonylpiperazin-1-yl)carbonyl)ethenyl)phenyl]sulfid;
(Benzodioxan-6-yl)[2-trifluormethyl-4-(E-((2-carbomethoxypiperazin-1-yl)carbonyl)ethenyl)phenyl]sulfid;
(2-Methyl-3-(carboethoxymethyl)indol-5-yl)[2-trifluormethyl-4-(E-((morpholin-1-yl)carbonyl)ethenyl)phenyl]sulfid;
(1-(2-Methoxyethyl)indol-5-yl)[2-chlor-4-(E-((4-acetylpiperazin-1-yl])carbonyl)ethenyl)phenyl]sulfid;
(2-Isopropylphenyl)[2-nitro-4-(E-((3-acetoxymethyl-4-hydroxypiperidin-1-yl)carbonyl)ethenyl)phenyl]sulfid;
(2-Isopropylphenyl)[2-nitro-4-(E-((3-(dimethylaminocarbonyl)-4-hydroxypiperidin-1-yl)carbonyl)ethenyl)phenyl]sulfid;
(2-Isopropylphenyl)[2-nitro-4-(E-((3-cyanomorpholin-1-yl])carbonyl)ethenyl)phenyl]sulfid;
(2-Isopropylphenyl)[2-nitro-4-(E-((3-carboethoxymorpholin-1-yl)carbonyl)ethenyl)phenyl]sulfid;
(2-Isopropylphenyl)[2-nitro-4-(E-((3-(tetrazol-5-yl)morpholin-1-yl)carbonyl)ethenyl)phenyl]sulfid;
(Benzodioxan-6-yl)[2-trifluormethyl-4-(E-((4-carboxypiperidin-1-yl)carbonyl)ethenyl)phenyl]sulfid;
(Benzodioxan-6-yl)[2-trifluormethyl-4-(E-((2-carboxypiperidin-1-yl)carbonyl)ethenyl)phenyl]sulfid;
(Benzodioxan-6-yl)[2-trifluormethyl-4-(E-((4-carbomethoxypiperazin-1-yl)carbonyl)ethenyl)phenyl]sulfid;
(Benzodioxan-6-yl)[2-trifluormethyl-4-(E-((3-aza-6,9-diooxaspiro[5.4]decan-1-yl)carbonyl)ethenyl)phenyl]sulfid;
(Benzodioxan-6-yl)[2-trifluor-4-(E-((4-(benzimidazolon-1-yl)piperidin-1-yl)carbonyl)ethenyl)phenyl]sulfid;
(Benzodioxan-6-yl)[2-trifluormethyl]-4-(E-((4-(methylaminocarbonyl)piperidin-1-yl)carbonyl)ethenyl)phenyl]sulfid;
(Benzodioxan-6-yl)[2-trifluormethyl-4-(E-((3-carbomethoxy-4-methoxycarbonylpiperazin-1-yl)carbonyl)ethenyl)phenyl]sulfid;
(2-Isopropylphenyl)[2-nitro-4-(E-((3-carboxymorpholin-1-yl)carbonyl)ethenyl)phenyl]sulfid;
(Benzodioxan-6-yl)[2-trifluormethyl-4-(E-((2-carboxy-4-methoxycarbonylpiperazin-1-yl)carbonyl)ethenyl)phenyl]sulfid;
(Benzodioxan-6-yl)[2-trifluormethyl-4-(E-((morpholin-1-yl)carbonyl)ethenyl)phenyl]sulfid;
(Benzodioxan-6-yl)[2-trifluormethyl-4-(E-((4-(pyrrolidin-1-yl)piperidin-1-yl)carbonyl)ethenyl)phenyl]sulfid;
(2-Isopropylphenyl)[2-nitro-4-(E-((3-aza-6,9-diooxaspiro[5.4]decan-1-yl)carbonyl)ethenyl)phenyl]sulfid;
(2-Isopropylphenyl)[2-nitro-4-(E-((2-(dimethylaminomethyl)piperidin-1-yl)carbonyl)ethenyl)phenyl]sulfid;
(2-Isopropylphenyl)[2-nitro-4-(E-((piperidin-1-ylamino)carbonyl)ethenyl)phenyl]sulfid;
(Benzodioxan-6-yl)[2-trifluormethyl-4-(E-((3-carboxy-4-methoxycarbonylpiperazin-1-yl)carbonyl)ethenyl)phenyl]sulfid;
(2-(Dimethylaminocarbonyl)benzodioxan-6-yl)[2-chlor-4-(E-((4-acetylpiperazin-1-yl)carbonyl)ethenyl)phenyl]sulfid;
(2-lsopropylphenyl)[2-nitro-4-(E-((3-(2-(methoxymethyl)tetrazol-5-yl)piperidin-1-yl)carbonyl)ethenyl)phenyl]sulfid;
(2-Isopropylphenyl)[2-nitro-4-(E-((3-(1-(methoxymethyl)tetrazol-5-yl)piperidin-1-yl)carbonyl)ethenyl)phenyl]sulfid;
(1-Methylindol-5-yl)[2-chlor-4-(E-((3-(1-pyrrolidin-2-onyl)propylamino)carbonyl)ethenyl)phenyl]sulfid;
(2-Isopropylphenyl)[2-nitro-4-(E-((3-(tetrazol-5-yl)piperidin-1-yl)carbonyl)ethenyl)phenyl]sulfid;
(1-Methylindol-5-yl)[2-chlor-4-(E-((3-carboethoxypiperidin-1-yl)carbonyl)ethenyl)phenyl]sulfid;
(1-Methylindol-5-yl)[2-chlor-4-(E-((3-carboxypiperidin-1-yl)carbonyl)ethenyl)phenyl]sulfid;
(1-Methylindol-5-yl)[2-chlor-4-(E-((4-carboethoxypiperidin-1-yl)carbonyl)ethenyl)phenyl]sulfid;
(1-Methylindol-5-yl)[2-chlor-4-(E-((3-carboxypiperidin-1-yl)carbonyl)ethenyl)phenyl]sulfid;
(2-Isopropylphenyl)[2-nitro-4-(E-((2-(1-methylpyrrolidin-2-yl)ethylamino)carbonyl)ethenyl)phenyl]sulfid;
(2-Isopropylphenyl)[2-nitro-4-(E-((4-(pyrrolidin-1-yl)piperidin-1-yl)carbonyl)ethenyl)phenyl]sulfid;
(2-Isopropylphenyl)[2-nitro-4-(E-((4-sulfopiperidin-1-yl)carbonyl)ethenyl)phenyl]sulfid;
(2-Isopropylphenyl)[2-nitro-4-(E-((3-hydroxypiperidin-1-yl)carbonyl)ethenyl)phenyl]sulfid;
(Benzodioxan-6-yl)[2-trifluormethyl-4-(E-((3-((ethansulfonylamino)carbonyl)piperidin-1-yl)carbonyl)ethenyl)phenyl]sulfid;
(Benzodioxan-6-yl)[2-trifluormethyl-4-(E-((3-((p-toluolsulfonylamino)carbonyl)piperidin-1-yl)carbonyl)ethenyl)phenyl]sulfid;
(Benzodioxan-6-yl)[2-trifluormethyl-4-(E-((4-((ethansulfonylamino)carbonyl)piperidin-1-yl)carbonyl)ethenyl)phenyl]sulfid;
(Benzodioxan-6-yl)[2-trifluormethyl-4-(E-((2(tetrazol-5-yl)morpholin-1-yl)carbonyl)ethenyl)phenyl]sulfid;
(2-Isopropylphenyl)[2-nitro-4-(E-((2-butyl-5-(tetrazol-5-yl)morpholin-1-yl)carbonyl)ethenyl)phenyl]sulfid;
(2-(und 3-)(Hydroxymethyl)benzodioxan-6-yl)[2-nitro-4-(E-((4-acetylpiperazin-1-yl)carbonyl)ethenyl)phenyl]sulfid;
(2-(und 3-)(Hydroxymethyl)benzodioxan-6-yl)[2-nitro-4-(E-((3-(pyrrolidin-2-on-1-yl)prop-1-ylamino)carbonyl)ethenyl)phenyl]sulfid;
(2-(und 3-)(Hydroxymethyl)benzodioxan-6-yl)[2-trifluormethyl-4-(E-((3-(pyrrolidin-2-on-1-yl)prop-1-ylamino)carbonyl)ethenyl)phenyl]sulfid;
(3-Hydroxymethyl)benzodioxan-6-yl)[2-nitro-4-(E-((3-(pyrrolidin-2-on-1-yl)prop-1-ylamino)carbonyl)ethenyl)phenyl]sulfid;
(Benzodioxan-6-yl)[2-chlor-4-(E-((3-carboxypiperidin-1-yl)carbonyl)ethenyl)phenyl]
(2-(und 3-)(Aminomethyl)benzodioxan-6-yl)[2-trifluormethyl-4-(E-((3-(pyrrolidin-2-on-1-yl)prop-1-ylamino)carbonyl)ethenyl)phenyl]sulfid;
(2-Isopropylphenyl)[2-nitro-4-(E-((3-(methylaminocarbonyl)morpholin-1-yl)carbonyl)ethenyl)phenyl]sulfid;
(2-Isopropylphenyl)[2-nitro-4-(E-((3-(hydroxymethyl)morpholin-1-yl)carbonyl)ethenyl)phenyl]sulfid;
(2-Isopropylphenyl)[2-nitro-4-(E-((3-(acetoxymethyl)morpholin-1-yl])carbonyl)ethenyl)phenyl]sulfid;
(2-Isopropylphenyl)[2-nitro-4-(E-((3-(aminomethyl)morpholin-1-yl)carbonyl)ethenyl)phenyl]sulfid;
(2-Isopropylphenyl)[2-nitro-4-(E-((3-(acetamidomethyl)morpholin-1-yl)carbonyl)ethenyl)phenyl]sulfid;
(Benzodioxan-6-yl)[2-chlor-4-(E-((3-(pyrrolidin-2-on-1-yl)prop-1-ylamino)carbonyl)ethenyl)phenyl]sulfid;
(Benzodioxan-6-yl)[2-chlor-4-(E-((3-carboethoxypiperidin-1-yl)carbonyl)ethenyl)phenyl]sulfid;
(Benzodioxan-6-yl)[2-chlor-4-(E-((2-carboethoxypiperidin-1-yl)carbonyl)ethenyl)phenyl]sulfid;
(2-Methoxyphenyl)[2,3-dichlor-4-(E-[(morpholin-1-yl)carbonyl]ethenyl)phenyl]sulfid;
(2-Methoxyphenyl)[2,3-dimethyl-4-(E-[(morpholin-1-yl)carbonyl]ethenyl)phenyl]sulfid;
(2-Isopropylphenyl)[2-nitro-4-(E-((indol-5-ylamino)carbonyl)ethenyl)phenyl]sulfid;
(Benzodioxan-6-yl)(2-chlor-4-(E-((3-carboxypiperidin-1-yl)carbonyl)ethenyl)phenyl]sulfid;
(Benzodioxan-6-yl)[2-chlor-4-(E-((3-(tetrazol-5-yl)piperidin-1-yl)carbonyl)ethenyl)phenyl]sulfid;
(Benzodioxan-6-yl)[2-chlor-4-(E-((4-(tert-butoxycarbonyl)piperazin-1-yl)carbonyl)ethenyl)phenyl]sulfid;
(Benzodioxan-6-yl)[2-chlor-4-(E-((2-carboxypiperidin-1-yl)carbonyl)ethenyl)phenyl]sulfid;
(Benzodioxan-6-yl)[2-chlor-4-(E-((3-(tetrazol-5-yl)morpholin-1-yl)carbonyl)ethenyl)phenyl]sulfid;
(Benzodioxan-6-yl)[2-chlor-4-(E-((4-(methylaminocarbonyl)piperazin-1-yl)carbonyl)ethenyl)phenyl]sulfid;
(2-Methoxyphenyl)[2,3-dichlor-4-(E-[(4-carboxypiperidin-1-yl)carbonyl]ethenyl)phenyl]sulfid;
(Benzodioxan-6-yl)[2-chlor-4-(E-((4-(tetrazol-5-yl)piperidin-1-yl)carbonyl)ethenyl)phenyl]sulfid;
(2-Methoxyphenyl)[3-chlor-4-(E-[(morpholin-1-yl)carbonyl]ethenyl)phenyl]sulfid;
(2-Isopropylphenyl)[2-nitro-4-(E-((4-oxapiperidin-1-yl)carbonyl)ethenyl)phenyl]sulfid;
(Benzodioxan-6-yl)[2-trifluormethyl-4-(E-((3-R-carboethoxypiperidin-1-yl)carbonyl)ethenyl)phenyl]sulfid;
(Benzodioxan-6-yl)[2,3-dichlor-4-(E-((3-(pyrrolidin-2-on-1-yl)prop-1-ylamino)carbonyl)ethenyl)phenyl]sulfid;
(Benzodioxan-6-yl)[2,3-dichlor-4-(E-((4-acetylpiperazin-1-yl)carbonyl)ethenyl)phenyl]sulfid;
(Benzodioxan-6-yl)[2,3-dichlor-4-(E-((3-carboethoxypiperidin-1-yl)carbonyl)ethenyl)phenyl]sulfid;
(Benzodioxan-6-yl)(2,3-dichlor-4-(E-((4-carboethoxypiperidin-1-yl)carbonyl)ethenyl)phenyl]sulfid;
(Benzodioxan-6-yl)[2,3-dichlor-4-(E-((3-carboxypiperidin-1-yl)carbonyl)ethenyl)phenyl]sulfid;
(Benzodioxan-6-yl)[2,3-dichlor-4-(E-((4-carboxypiperidin-1-yl)carbonyl)ethenyl)phenyl]sulfid;
(2-Isopropylphenyl)[2,3-dichlor-4-(E-((3-(1-pyrrolidin-2-onyl)propylamino)carbonyl)ethenyl)phenyl]sulfid;
(2-Isopropylphenyl)[2,3-dichlor-4-(E-((4-acetylpiperazin-1-yl)carbonyl)ethenyl)phenyl]sulfid;
(2-Isopropylphenyl)[2,3-dichlor-4-(E-((3-carboethoxypiperidin-1-yl)carbonyl)ethenyl)phenyl]sulfid;
(2-Isopropylphenyl)[2,3-dichlor-4-(E-((4-carboethoxypiperidin-1-yl)carbonyl)ethenyl)phenyl]sulfid;
(2-Isopropylphenyl)[2,3-dichlor-4-(E-((3-carboxypiperidin-1-yl)carbonyl)ethenyl)phenyl]sulfid;
(2-Isopropylphenyl)[2,3-dichlor-4-(E-((4-carboxypiperidin-1-yl)carbonyl)ethenyl)phenyl]sulfid;
(1-Methylindol-5-yl)[2,3-dichlor-4-(E-((3-carboethoxypiperidin-1-yl)carbonyl)ethenyl)phenyl]sulfid;
(1-Methylindol-5-yl)[2,3-dichlor-4-(E-((3-carboxypiperidin-1-yl)carbonyl)ethenyl)phenyl]sulfid;
(1-Methylindol-5-yl)[2,3-dichlor-4-(E-((4-carboethoxypiperidin-1-yl)carbonyl)ethenyl)phenyl]sulfid;
(1-Methylindol-5-yl)[2,3-dichlor-4-(E-((4-carboxypiperidin-1-yl)carbonyl)ethenyl)phenyl]sulfid;
(2-Ethoxyphenyl)[2,3-dichlor-4-(E-[(4-carboxypiperidin-1-yl)carbonyl]ethenyl)phenyl]sulfid;
(2-Ethoxyphenyl)[2,3-dichlor-4-(E-[(morpholin-1-yl)carbonyl]ethenyl)phenyl]sulfid;
(2-Ethoxyphenyl)[2,3-dichlor-4-(E-[(3-carboxypiperidin-1-yl)carbonyl]ethenyl)phenyl]sulfid;
(2-Isopropylphenyl)[2-nitro-4-(E-((3-carboethoxypyrrolidin-1-yl)carbonyl)ethenyl)phenyl]sulfid;
(2-Isopropylphenyl)[2-nitro-4-(E-((3-carboxypyrrolidin-1-yl)carbonyl)ethenyl)phenyl]sulfid;
(2-Isopropylphenyl)[2,3-difluor-4-(E-((3-carboethoxypiperidin-1-yl)carbonyl)ethenyl)phenyl]sulfid;
(2-Isopropylphenyl)[2,3-difluor-4-(E-((3-carboxypiperidin-1-yl)carbonyl)ethenyl)phenyl]sulfid;
(2-Isopropylphenyl)[2,3-difluor-4-(E-((4-carboxypiperidin-1-yl)carbonyl)ethenyl)phenyl]sulfid;
(Benzodioxan-6-yl)[2-trifluormethyl-4-(E-((3-ethoxycarbonylpyrrolidin-1-yl)carbonyl)ethenyl)phenyl]sulfid
(Benzodioxan-6-yl)[2-trifluormethyl-4-(E-((3-carboxypyrrolidin-1-yl)carbonyl)ethenyl)phenyl]sulfid;
(2-Methoxyphenyl)[2-chlor-3-trifluormethyl-4-(E-((4-carboethoxypiperidin-1-yl)carbonyl)ethenyl)phenyl]sulfid;
(2-Methoxyphenyl)[2-chlor-3-trifluormethyl-4-(E-((4-carboethoxypiperidin-1-yl)carbonyl)ethenyl)phenyl]sulfid;
(2-Methoxyphenyl)(2-chlor-3-trifluormethyl-4-(E-((morpholin-1-yl)carbonyl)ethenyl)phenyl]sulfid;
(Benzodioxan-6-yl)[4-(E-((4-carboxypiperidin-1-yl)carbonyl)ethenyl)naphthyl]sulfid;
(2-Methoxyphenyl)[2,3-dichlor-4-(E-((4-(spiro-hydantoin-5-yl)-piperidin-1-yl)carbonyl)ethenyl)phenyl]sulfid;
(2-Methoxyphenyl)[2,3-dichlor-4-(E-(4-(2-(2-hydroxyethoxy)ethyl)piperazin-1-yl)carbonyl)ethenyl)phenyl]sulfid;
(2-Methoxyphenyl)[2,3-dichlor-4-(E-((4-ethylpiperazin-1-yl)carbonyl)ethenyl)phenyl]sulfid;
(2-Isopropylphenyl)[2,3-dichlor-4-(E-((4-(2-(2-hydroxyethoxy)ethyl)piperazin-1-yl)carbonyl)ethenyl)phenyl]sulfid;
(Benzodioxan-6-yl)[2,3-bis(trifluormethyl)-4-(E-((4-carboxypiperidin-1-yl)carbonyl)ethenyl)phenyl]sulfid;
(2-Methoxyphenyl)[2,3-dichlor-4-(E-((4-(carboxymethylamino)carbonylpiperidin-1-yl)carbonyl)ethenyl)phenyl]sulfid;
(2-Methoxyphenyl) [2,3-bis(trifluormethyl)-4-(E-((4-carboxymethylpiperazin-1-yl)carbonyl)ethenyl)phenyl]sulfid;
(2-Methoxyphenyl)[2,3-bis(trifluormethyl)-4-(E-((4-N-(2-hydroxyethyl)piperazin-1-yl)carbonyl)ethenyl)phenyl]sulfid;
(1-Methylindol-5-yl)[2,3-dichlor-4-(E-((4-(carbo-2,3-dihydroxypropylamino)piperidin-1-yl)carbonyl)ethenyl)phenyl]sulfid;
(2-Methoxyphenyl)[2,3-dichlor-4-(E-(4-(2,3-dihydroxypropionyl)piperazin-1-yl)carbonyl)ethenyl)phenyl)sulfid;
(2-Methoxyphenyl)[2,3-dichlor-4-(E-(4-(2,3-dihydroxy-3-carboxypropionyl)piperazin-1-yl)carbonyl)ethenyl)phenyl]sulfid;
(1-Methylindol]-5-yl)[2,3-dichlor-4-(E-((4-(carboxymethylamino)carbonylpiperidin-1-yl)carbonyl)ethenyl)phenyl]sulfid;
(1-Methylindol-5-yl)[2,3-dichlor-4-(E-((4-sulfopiperidin-1-yl)carbonyl)ethenyl)phenyl]sulfid;
(1-Methylindol-5-yl)[2,3-dichlor-4-(E-(4-methylhomopiperazin-1-ylcarbonyl)ethenyl)phenyl]sulfid;
(1-Methylindol-5-yl)[2,3-dichlor-4-(E-(4-tetrahydrofuroylpiperazin-1-yl)carbonyl)ethenyl)phenyl]sulfid;
(2-Methoxyphenyl)[2,3-dichlor-4-(E-((4-amino-4-carboxypiperidin-1-yl)carbonyl)ethenyl)phenyl]sulfid;
(2-Methoxyphenyl)[2,3-dichlor-4-((4-furoylpiperazin-1-yl)carbonyl)ethenyl)phenyl]sulfid;
(1-Methylindol-5-yl)[2,3-dichlor-4-(E-(4-(carbo-3-sulfopropylamino)piperadin-1-yl)carbonyl)ethenyl)phenyl]sulfid;
(2-Methoxyphenyl)[2,3-dichlor-4-(E-(4-acetylamino-4-carboxypiperidin-1-ylcarbonyl)ethenyl)phenyl]sulfid;
(2-Methoxyphenyl)[2,3-bis(trifluormethyl)-4-(E-((4-carboxypiperidin-1-yl)carbonyl)ethenyl)phenyl]sulfid;
(2-Methoxyphenyl)-5-[8-(E-((4-(aminocarbonyl)piperidin-1-yl)carbonyl)ethenyl)chinolinyl]sulfid;
(2-Methoxyphenyl)[2-trifluormethyl-4-(E-((4-carboxypiperidin-1-yl)carbonyl)ethenyl)phenyl]sulfid;
(1-Methylindol-5-yl)[2,3-dichlor-4-(E/Z-((1S,4S)-2,5-diazabicyclo[2.2.1]heptan-2-ylcarbonyl)ethenyl)-2,3-dichlorphenyl]sulfid;
(1-Methylindol-5-yl)[2,3-dichlor-4-(E-(4-hydroxy-3-carboxypiperadin-1-ylcarbonyl)ethenyl)phenyl]sulfid;
(1-Methylindol-5-yl)[2,3-dichlor-4-(E-(5-oxothiomorpholin-1-ylcarbonyl)ethenyl)phenyl]sulfid;
(2-Methoxyphenyl)[2,3-dichlor-4-(E-((4-sulfophenylamino)carbonyl)ethenyl)phenyl]sulfid;
(2-Methoxyphenyl)[2,3-dichlor-4-(E-((4-carboxyphenylamino)carbonyl)ethenyl)phenyl]sulfid; und
[3-(4-Morpholino)phenyl][2,3-dichlor-4-(E'-[(4-carboxypiperidin-1-yl)carbonyl]ethenyl)phenyl]sulfid.

13. Verbindung gemäß Anspruch 1, die ausgewählt ist aus:
(2-Formylphenyl)[2-nitro-4-(E-((4-acetylpiperazin-1-yl)carbonyl)ethenyl)phenyl]sulfid;
(2-Ethoxyphenyl)[2-chlor-4(E-[(morpholin-1-yl)carbonyl]ethenyl)phenyl]sulfid;
(2-Isopropylphenyl)[2-nitro-4-(E-((3-dimethylaminocarbonyl-4-acetylpiperazin-1-yl)carbonyl)ethenyl)phenyl]sulfid;
(2-Isopropylphenyl)[2-nitro-4-(E-((4-methoxycarbonylpiperazin-1-yl)carbonyl)ethenyl)phenyl]sulfid;
(2-Ethoxyphenyl)[2-trifluormethyl-4-(E-((2-carboxy-4-(methoxycarbonyl)piperazin-1-yl)carbonyl)ethenyl)phenyl]sulfid;
(2-Ethyl-4-bromphenyl)[2-nitro-4-(E-((4-acetylpiperazin-1-yl)carbonyl)ethenyl)phenyl]sulfid;
(3-Morpholinophenyl)[2-nitro-4-(E-((4-acetylpiperazin-1-yl)carbonyl)ethenyl)phenyl]sulfid;
(2-Isopropylphenyl)[2-nitro-4-(E-((3-carboethoxypiperidin-1-yl)carbonyl)ethenyl)phenyl]sulfid;
(2-Isopropylphenyt)[2-nitro-4-(E-((3-(1-pyrrolidin-2-onyl)prop-1-ylamino)carbonyl)ethenyl)phenyl]sulfid;
(2-(Hydroxymethyl)benzodioxan-6-yl)[2-chlor-4-(E-((4-acetyfpiperazin-1-yl)carbonyl)ethenyl)phenyl]sulfid;
(Benzodioxan-6-yl)[2-trifluormethyl-4-(E-((3-carboxypyrrolidin-1-yl)carbonyl)ethenyl)phenyl]sulfid;
(Benzodioxan-6-yl)[2-trifluormethyl-4-(E-((4-carboethoxypiperidin-1-yl)carbonyl)ethenyl)phenyl]sulfid;
(Benzodioxan-6-yl)[2-trifluormethyl-4-(E-((2-carbomethoxy-4-methoxycarbonylpiperazin-1-yl)carbonyl)ethenyl)phenyl]sulfid;
(Benzodioxan-6-yl)[2-trifluormethyl-4-(E-((2-carbomethoxypiperazin-1-yl)carbonyl)ethenyl)phenyl]sulfid;
(Benzodioxan-6-yl)[2-chlor-4-(E-((4-(methylaminocarbonyl)piperazin-1-yl)carbonyl)ethenyl)phenyl]sulfid;
(2-Methoxyphenyl)[2,3-dichlor-4-(E-[(4-carboxypiperidin-1-yl)carbonyl]ethenyl)phenyl]sulfid;
(Benzodioxan-6-yl)[2-trifluormethyl-4-(E-((3-R-carboethoxypiperidin-1-yl)carbonyl)ethenyl)phenyl]sulfid;
(Benzodioxan-6-yl)[2,3-dichlor-4-(E-((3-(pyrrofidin-2-on-1-yl)prop-1-ylamino)carbonyl)ethenyl)phenyl]sulfid;
(Benzodioxan-6-yl)[2,3-dichlor-4-(E-((4-acetylpiperazin-1-yl)carbonyl)ethenyl)phenyl]sulfid;
(Benzodioxan-6-yl)[2,3-dichlor-4-(E-((3-carboethoxypiperidin-1-yl)carbonyl)ethenyl)phenyl]sulfid;
(Benzodioxan-6-yl)(2,3-dichlor-4-(E-((4-carboethoxypiperidin-1-yl)carbonyl)ethenyl)phenyl]sulfid;
(Benzodioxan-6-yl)[2,3-dichlor-4-(E-((3-carboxypiperidin-1-yl)carbonyl)ethenyl)phenyl]sulfid;
(Benzodioxan-6-yl)[2,3-dichlor-4-(E-((4-carboxypiperidin-1-yl)carbonyl)ethenyl)phenyl]sulfid;
(2-Isopropylphenyl)[2,3-dichlor-4-(E-((4-acetylpiperazin-1-yl)carbonyl)ethenyl)phenyl]sulfid;
(2-Isopropylphenyl)[2,3-dichlor-4-(E-((3-carboethoxypiperidin-1-yl)carbonyl)ethenyl)phenyl]sulfid;
(2-Isopropylphenyl)[2,3-dichlor-4-(E-((3-carboxypiperidin-1-yl)carbonyl)ethenyl)phenyl]sulfid;
(2-Isopropylphenyl)[2,3-dichlor-4-(E-((4-carboxypiperidin-1-yl)carbonyl)ethenyl)phenyl]sulfid;
(2-Methoxyphenyl)[2,3-dichlor-4-(E-((4-(carboxymethylamino)carbonylpiperidin-1-yl)carbonyl)ethenyl)phenyl]sulfid;
(2-Methoxyphenyl)[2,3-bis(trifluormethyl)-4-(E-((4-carboxymethylpiperazin-1-yl)carbonyl)ethenyl)phenyl]sulfid; und
(2-Methoxyphenyl)[2,3-bis(trifluormethyl)-4-(E-((4-N-(2-hydroxyethyl)piperazin-1-yl)carbonyl)ethenyl)phenyl]sulfid.

14. Verbindung gemäß Anspruch 1, wobei Ar aus der Gruppe ausgewählt ist, die aus Methoxyphenyl und Isopropylphenyl besteht.

15. Verbindung gemäß Anspruch 1, wobei Ar Benzodioxan oder substituiertes Benzodioxan ist.

16. Verbindung gemäß Anspruch 1, wobei R₃ ein "trans-Cinnamid" ist und Ar aus 1,3-Benzimidazol-2-on, 1,4-Benzodioxan, 1,3-Benzodioxol, 1-Benzopyr-2-en-4-on, Indol, Isatin, Phenyl, 1,3-Chinazolin-4-on und Chinolin ausgewählt ist.

17. Verbindung gemäß Anspruch 1, wobei R₁₀ und R₁₁ unabhängig aus Wasserstoff, Alkyl, Cycloalkyl, Alkoxycarbonylalkyl, Hydroxyalkyl und Heterocyclylalkyl ausgewählt sind.

18. Verbindung gemäß Anspruch 1, wobei NR₁₀R₁₁ Heterocyclyl oder substituiertes Heterocyclyl ist.

19. Pharmazeutische Zusammensetzung, die eine Verbindung gemäß Anspruch 1 in einem pharmazeutisch annehmbaren Träger umfasst.

20. Verwendung einer Verbindung gemäß Anspruch 1 oder 12 zur Herstellung eines Medikaments zur Entzündungshemmung bei einem Säuger.

21. Verwendung einer Verbindung gemäß Anspruch 1 oder 12 zur Herstellung eines Medikaments zur Unterdrückung der Immunantwort bei einem Säuger.

22. Verfahren zur Herstellung einer Verbindung gemäß Anspruch 1, wobei das Verfahren das Aktivieren der Säuregruppe in einer Verbindung der Formel 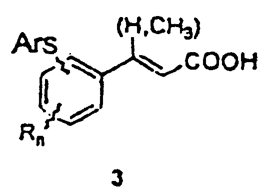 und das anschließende Umsetzen der aktivierten Verbindung mit einem primären oder sekundären Amin der Formel NR₁₀R₁₁ umfasst, wobei alle Variablen wie in Anspruch 1 definiert sind.

## Revendications

1. Un composé de formule 1 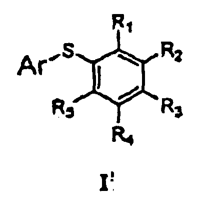 dans laquelle R₁, R₂, R₃, R₄ et R₅ sont, indépendamment les uns des autres, choisis dans le groupe comprenant:
a. hydrogène,
b. halogène,
c. alkyle,
d. haloalkyle,
e. alcoxy,
f. cyano,
g. nitro,
h. carboxaldéhyde, et
à la condition qu'au moins un des radicaux R₁ à R₃ consiste en un "cis-cinnamide" ou un "trans-cinnamide", défini par les formules: 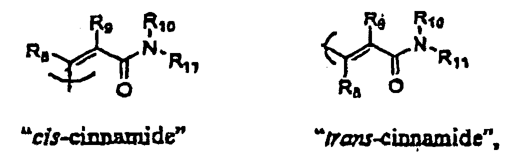 dans lesquelles R₈ et R₉ sont, indépendamment les uns des autres, choisis dans le groupe comprenant:
a. hydrogène,
b. alkyle,
c. carboxyalkyle,
d. alkyl-amino-carbonyl alkyle, et
e. dialkyl-amino-carbonyl alkyle,
et R₁₀ et R₁₁ sont, indépendamment les uns des autres, choisis dans le groupe comprenant:
a. hydrogène,
b. alkyle,
c. cycloalkyle,
d. alcoxycarbonylalkyle,
e. hydroxyalkyle,
f. hétérocyclyle comportant un noyau à 4, 5, 6 ou 7 atomes, contenant deux ou trois hétéroatomes choisis, indépendamment les uns des autres, parmi azote, oxygène et soufre, lesquels peuvent être fusionnés pour former un ou deux noyaux choisis, indépendamment les uns des autres, dans le groupe comprenant les noyau aryle, tels que définis ci-dessous, cyclohexane, cyclohexène, cyclopentane, cyclopentène et autres noyaux hétérocycliques monocyclique,
g. hétérocyclylalkyle où le radical hétérocyclyle est tel que défini ci-dessus,
h. hétérocyclyl-amino, où le radical hétérocyclyle est tel que défini ci-dessus,
i. un groupe hétérocyclyle tel que défini dans f ci-dessus comportant des substituants choisis, indépendamment les uns des autres, parmi les groupes alkyle, halogène, hydroxy et alcoxy,
j. un radical hétérocyclyl alkyle tel que défini dans g ci-dessus comportant des substituants choisis, indépendamment les uns des autres, parmi les groupes alkyle, halogène, hydroxy et alkyloxy,
où lorsque NR₁₀R₁₁ est un radical hétérocyclyle ou hétérocyclyle substitué où le groupe hétérocyclyle est tel que défini dans f ci-dessus et les substituants sont choisis indépendamment les uns des autres parmi les radicaux:
1. alkyle,
2. alcoxy,
3. alkoxyalkyle,
4. cycloalkyle,
5. aryle consistant en un système de noyau carboxyclique mono ou bicycliquecomportant un ou deux noyaux aromatiques qui peuvent être fusionnés pour former un noyau cyclohexane, cyclohexène, cyclopentane ou cyclopentène,
6. hétérocyclique tel que défini ci-dessus,
7. hétérocyclylcarbonyle dans lequel l'hétérocyclyle est tel que défini ci-dessus,
8. hétérocyclylalkyl-amino-carbonyle, où le radical hétérocyclyle est tel que défini ci-dessus,
9. hydroxy,
10. hydroxyalkyle,
11. hydroxyalcoxyalkyle,
12. carboxy,
13. carboxyalkyle,
14. carboxycarbonyle,
15. carboxyaldéhyde,
16. alcoxycarbonyle,
17. arylalcoxycarbonyle, où le radical aryle est tel que défini ci-dessus,
18, aminoalkyle,
19. aminoalcanoyle,
20. carboxamido,
21. alcoxycarbonylakyle,
22. carboxamidoalkyle,
23. cyano,
24. tétrazolyle,
25. tétrazolyle substitué,
26. alcanoyle,
27. hydroxyalcanoyle,
28. alcanoyloxy,
29. alcanoyl-amino,
30. alcanoyl-aminoalkyle,
31. alcanoyl-aminoalkyle,
32. sulfonate,
33. alkylsulfonyle,
34. alkylsulfonyl-amino-carbonyle,
35. arylsulfonyl-amino-carbonyle, où le radical aryle est tel que défini ci-dessus,
et
36. hétérocyclylsulfonyl-amino-carbonyle, où le radical hétérocyclyle est tel que défini ci-dessus,
et où Ar est un groupe aryle ou hétéroaryle substitué qui comporte un ou deux noyaux aromatiques qui peuvent être fusionnés pour former un noyau cyclohexane, cyclohexène, cyclopentane ou cyclopentène, les substituants étant, indépendamment les uns des autres, choisis parmi les radicaux:
a. hydrogène,
b. halogène,
c, alkyle,
d. aryle tel que défini ci-dessus,
e. halocalkyle,
f. hydroxy,
g. alcoxy,
h. alcoxyalkyle,
i. alcoxycarbonyle,
j. alcoxyalcoxy,
k. hydroxyalkyle,
l. aminoalkyle,
m. amino-carbonyle,
n. alkyl(alcoxycarbonylalkyl)-aminoalkyle,
o. hétérocyclyle tel que défini ci-dessus,
p. hétérocyclylallcyle tel que défini ci-dessus,
q. hétérocyclylalkyle tel que défini ci-dessus compotrant des substituants choisis, indépendamment 1'un' l'autre, dans le groupe comprenant alkyle, halogène, hydroxy et alcoxy,
r. carboxaldéhyde,
s. carboxaldéhyde hydrazone,
t. caiboxamide,
u. alcoxycarbonylalkyle,
v. carboxy,
w. carboxyalkyle,
x. hydroxycarbonylalkyl (carboxyalkyl)·,
y. hydroxyalkyl-amino-carbonyle,
z. a. cyano,
aa. amino,
bb. hétérocyclylalkyl-amino, où le radical hétérocyclyle est tel que défini ci-dessus,
cc. hétérocyclyl-amino-carbonyle, où le radical hétérocyclyle est tel que défini ci-dessus, et
dd. "trans-cinnamide",
étant enntendu que
(i) Si Ar est un radical phényle et R₁, R₂, R₄ et R₅ sont simultanément de l'hydrogène, alors R₃ n'est pas un radical cis-cinnamide ni trans-cinnamide où R₈ est un radical méthyle, R₉ est un atome d'hydrogène et R₁₀ et R₁₁ sont simultanément -C₂H₅, et
(ii) Si Ar est un groupe pyridyle ou pyridyle substitué par un radical C₁₋₃ alkyle, et R₁, R₂, R₄ et R₅ sont simultanément un atome d'hydrogène, alors R₃ n'est pas un groupe cis-cinnamide ou trans-cinnamide où R₈ est un atome d'hydrogène, R₉ un atome d'hydrogène ou un radical alkyle en C₁₋₃, et R₁₀ et R₁₁ sont, indépendamment l'un de l'autre, choisis dans le groupe comprenant l'hydrogène, C₁₋₄ alkyle ou C₃₋₆ cycloalkyle,
ou un sel pharmaceutiquement acceptable ou en dérivant.

2. Un composé selon la revendication 1, où R₁ est un radical "cis-cinnamide" ou "trans-cinnamide" et R₃ est un atome d'hydrogène.

3. Un composé selon la revendication 1, où R₃ est un radical "cis-cinnamide" ou un "trans-cinnamide", et R₁ est un atome d'hydrogène.

4. Un composé selon la revendication 1, où R₃ est un un radical "cis-cinnamide" ou un "trans-cinnamide", et R₁, R₈ et R₉ sont des atomes d'hydrogène.

5. Un composé selon la revendication 4, où R₃ est un radical "cis-cinnamide".

6. Un composé selon la revendication 4, où R₃ est un radical "trans-cinnamide".

7. Un composé selon la revendication 1 où R₃ est un radical "cis-cinnamide'' ou "trans-cinnamide", R₁, R₂ et R₄ sont indépendamment les uns des autres des atomes d'hydrogène ou un radical alkyle, et R₅ est choisi dans le groupe comprenant halogène, haloalkyle et nitro.

8. Un composé selon la revendication 4, où Ar est un aryle, un aryle substitué, un hétéroaryle, ou un hétéroaryle substitué.

9. Un composé selon la revendication 4 où R₁₀ et R₁₁ sont indépendamment les uns des autres choisis dans le groupe comprenant hydrogène, alkyle, cycloalkyle, alcoxycarbonylalkyle, hydroxyalkyle, et hétérocyclylalkyle.

10. Un composé selon la revendication 4 où NR₁₀R₁₁ est un groupe hétérocyclyle ou hétérocyclyle substitué.

11. Un composé selon la revendication 8 où Ar est choisi dans le groupe comprenant les radicaux phényle substitué, 1,3-benzimidazol-2-one, 1,4-benzodioxane, 1,3-benzodioxole, 1-benzopyr-2-èn-4-one, indole, isatine, 1,3-quinazoline-4-one, et quinoline,

12. Un composé selon la revendication 1 choisi dans le groupe comprenant:
sulfure de (2,4-dichlorophényl)-[2-(*E*-((6-hydroxy-hexyl-amino)-carbonyl)-éthényl)-phényle] ;
sulfure de (2,4-dichlorophényl)-[2-(*E*-((3-(1-imidazolyl)-propyl-amino)-carbonyl)-éthényl)-phényle)-;
sulfure de (2,4-dichlorophényl)-[2-chloro-4-(*E*-((2-hydroxy-éthyl-amino)-carbonyl)-éthényl)-phényle] ;
sulfure de (2,4-dichlorophényl)-[2-chloro-4-(*E*-((6-hydroxy-hexyl-amino)-carbonyl)-éthényl)-phényle] ;
sulfure de (2,4-dichlorophényl)-[2-chloro-4-(*E*-((bis-(2-hydroxyéthyl)-amino)-carbonyl)-éthényl)-phényle] ;
sulfure de (2,4-dichlorophényl)-[2-chloro-4-(*E*-((3-(1-pyrrolidin-2-only)-propyl-amino)-carbonyl)-éthényl)-phényle] ;
sulfure de (2,4-dichlorophényl)-[2-chloro-4-(*E*-((1-morpholinyl)-carbonyl)-éthényl)-phényle] ;
sulfure de (2,4-dichlorophényl)-[2-chloro-4-(*E*-((4-méthyl-pipérazine-1-yl)-carbonyl)-éthenyl)-phényle] ;
sulfure de (2,4-dichlorophényl)-[2-chloro-4-(*E*-((4-acétyl-pipérazine-1-yl)-carbonyl)-éthényl)-phényle] ;
sulfure de (2,4-dichlorophényl)-[2-chloro-4-(*E*-((4-(2-pyridyl)-pipérazine-1-yl)-carbonyl)-éthényl)-phényle] ;
sulfure de (2-(hydroxy-méthyl)-phényl)-[2-chloro-4-(*E*-((1-morpholinyl)-carbonyl)-éthényl)-phényle] ;
sulfure de (2-bromophényl)-[2-chloro-4-(*E*-((1-morpholinyl)-carbonyl)-éthényl)-phényle] ;
sulfure de (2,4-dichlorophényl)-[2-chloro-4-(*E*-((4-(2-hydroxyéthyl)-pipérazine-1-yl)-carbonyl)-éthényl)-phényle] ;
sulfure de (2-bromophényl)-[2-chloro-4-(*E*-((3-hydroxy-méthyl)-pipéridine-1-yl)-carbonyl)-éthényl)-phényle] ;
sulfure de (2-bromophényl)-[2-chloro-4-(*E*-((2-(hydroxy-méthyl)-pipéridine-1-yl)-carbonyl)-éthényl)-phényle] ;
sulfure de (2-bromophényl)-[2-chloro-4-(*E*-((3-acétamidopyrrolidine-1-yl)-carbonyl)-éthényl)-phényle] ;
sulfure de (2-bromophényl)-[2-chloro-4-(*E*-((4-hydroxypipéridine-1-yl)-carbonyl)-éthényl)-phényle] ;
sulfure de (2-bromophényl)-[2-chloro-4-(*E*-((pipéridine-1-yl)-carbonyl)-éthényl)-phényle] ;
sulfure de (2,4-dichlorophényl)-[2-chloro-4-(*E*-((3-carboxypipéridine-1-yl)-carbonyl)-éthényl)-phényle] ;
sulfure de (2,4-dichlorophényl)-[2-chloro-4-(*E*-((4-carboxypipéridine-1-yl)-carbonyl)-éthényl)-phényle] ;
sulfure de (2-bromophényl)-[2-chloro-4-(*E*-((4-acétylhomopipérazine-1-yl)-carbonyl)-éthényl)-phényle] ;
sulfure de (2-bromophényl)-[2-chloro-4-(*E*-((thiomorpholine-1-yl)-carbonyl)-éthényl)-phényle] ;
sulfure de (2-bromophényl)-[2-chloro-4-(*E*-((4-(1-benzimidazol-2-only)-pipéridine-1-yl)-carbonyl)-éthényl)-phényle] ;
sulfure de (2-bromophényl)-[2-chloro-4-(*E*-((2-tétrahydroisoquinolinyl)-carbonyl)-éthényl)-phényle] ;
sulfure de (2-méthylphenyl)-[2-trifluorométhyl-4-(*E*-((4-acétyl-pipérazine-1-yl)-carbonyl)-éthényl)-phényle] ;
sulfure de (2-méthylphényl)-[2-trifluorométhyl-4-(*E*-((1-morpholinyl)-carbonyl)-éthényl)-phényle] ;
sulfure de (2-méthylphényl)-[2-trifluorométhyl-4-(*E*-((2-(1-morpholinyl)-éthyl-amino)-carbonyl]éthényl)-phényle] ;
sulfure de (2-méthylphényl)-[2-trifluorométhyl-4-(*E*-((4-phényl-pipérazine-1-yl)-carbonyl)-éthényl)-phényle] ;
sulfure de (2-méthylphényl)-[2-trifluorométhyl-4-(*E*-((3-(1-pyrrolidine-2-onyl)-propyl-amino)-carbonyl)-éthényl)-phényle] ;
sulfure de (2-méthylphényl)-[2-trifluorométhyl-4-(*E*-((cyclopropyl-amino)-carbonyl)-éthényl)-phényle] ;
sulfure de (2,4-(dichlorophényl)-[2-nitro-4-(*E*-((4-acétyl-pipérazine-1-yl)-carbonyl)-éthényl)-phényle] ;
sulfure de (2,4-dichlorophényl)-[2-nitro-4-(*E*-((3-(1-pyrrolidine-2-only)-propyl-amino)-carbonyl)-éthényl)-phényle] ;
sulfure de (2,3-dichlorophényl)-[2-nitro-4-(*E*-((4-acétyl-pipérazine-1-yl)-carbo-nyl)-éthényl)-phényle] ;
sulfure de (4-bromophényl)-[2-nitro-4-(*E*-((4-acétyl-pipérazine)-1-yl)-carbonyl)-éthényl)-phényle] ;
sulfure de (4-méthylphényl)-[2-nitro-4-(*E*-((4-acétyl-pipérazine-1-yl)-carbonyl)-éthényl)-phényle] ;
sulfure de (2,4-dichlorophényl)-[2-nitro-4-(*E*-((4-(*tert*-butoxycarbonyl)-pipérazine-1-yl)-carbonyl)-éthényl)-phényle] ;
sulfure de (2,4-dichlorophényl)-[2-nitro-4-(*E*-((4-(2-furoylcarbonyl)-pipérazine-1-yl)-carbonyl)-éthényl)-phényle] ;
sulfure de (2,4-dichlorophényl)-[2-nitro-4-(*E*-((4-(méthanesulfonyl)-pipérazine-1-yl)-carbonyl)-éthényl)-phényle];
sulfure de (2,4-dichlorophényl)-[2-nitro-4-(*E*-((4-diéthyl-amino-carbonylméthyl)-pipérazine-1-yl)-carbonyl)-éthényl)-phényle] ;
sulfure de (2,4-dichlorophényl)-[2-nitro-4-(*E*-((4-(diéthyl-amino-carbonyl)pipérazine-1-yl)-carbonyl)-éthényl)-phényle] ;
sulfure de (2,4-dichlorophényl)-[2-nitro-4-(*E*-((4-(*tert*-butoxycarbonylméthyl)-pipérazine-1-yl)-carbonyl)-éthényl)-phényle] ;
sulfure de (2,4-dichlorophényl)-[2-nitro-4-(*E*-((4-carboxycarbonyl)-pipérazine-1-yl)-carbonyl)-éthényl)-phényle] ;
sulfure de (2,4-dichlorophényl)-[2-nitro-4-(*E*-((4-carboxy-méthyl)-pipérazine-1-yl)-carbonyl)-éthényl)-phényle] ;
sulfure de (2-méthylphényl)-[2-nitro-4-(*E*-((4-acétyl-pipérazine-1-yl)-carbonyl)-éthényl)-phényle] ;
sulfure de (2-chlorophényl)-[2-nitro-4-(*E*-((4-acétyl-pipérazine-1-yl)-carbonyl)-éthényl)-phényle] ;
sulfure de (2-aminophényl)-[2-nitro-4-(*E*-((4-acétyl-pipérazine-1-yl)-carbonyl)-éthényl)-phényle] ;
sulfure de (2-hydroxy-méthylphényl)-[2-nitro-4-(*E*-((4-acétyl-pipérazine-1-yl)-carbonyl)-éthényl)-phényle] ;
sulfure de (2-éthylphényl)-[2-nitro-4-(*E*-((4-acétyl-pipérazine-1-yl)-carbonyl)-éthényl)-phényle] ;
sulfure de (2-*iso*-propylphényl)-[2-nitro-4-(*E*-((acétyl-pipérazine-1-yl)-carbonyl)-éthényl)-phényle] ;
sulfure de 2-*tert*-butylphényl)-[2-nitro-4-(*E*-((4-acétyl-pipérazine-1-yl)-carbonyl)-éthényl)-phényle] ;
sulfure de (2-chlorophényl)-[2-chloxo-4-(*E*6554-acétyl-pipérazine-1-yl)-carbonyl)-)-2-propényl)-phényle] ;
sulfure de (2-(1-morpholinylméthyl)-phényl)-[2-chloro-4-(*E*-((1-morpholinyl)-carbo-nyl)-éthényl)-phényle] ;
sulfure de (2-(4-(1,3-benzodioxolyl-5-méthyl)-pipérazine-1-ylméthyl)-phényl)-[2-chloro-4-(*E*-((1-morpholinyl)-carbonyl)-éthényl)-phényle] ;
sulfure de (2-(4-*iso*-propyl-amino-carbonylméthyl)-pipérazine-1-ylméthyl)-phényl)-[2-chloro-4-(*E*-((1-morpholinyl)-carbonyl)-éthényl)-phényle] ;
sulfure de (2-((*N*-éthoxycarbonylméthyl-*N*-méthyl)-aminométhyl)-phényl)-[2-chloro-4-(*E*-((1-morpholinyl)-carbonyl)-éthényl)-phényle] ;
sulfure de (2-formylphényl)-[2-chloro-4-(*E*-((1-morpholinyl)-carbonyl)-éthényl)-phényle] ;
sulfure de (2-(4-formyl-pipérazine-1-ylméthyl)-phényl)-[2-chloxo-4-(*E*-((1-morpholinyl)-carbonyl)-éthényl)-phényle] ;
sulfure de (2-(*E*-((1-morpholinyl)-carbonyl)-éthényl)-phényl)-[2-chloro-4-(*E*-((1-morpholinyl)-carbonyl)-éthényl)-phényle] ;
sulfure de (2-formylphényl)-[2-nitro-4-(*E*-((4-acétyl-pipérazine-1-yl)-carbonyl)-éthényl)-phényle] ;
sulfure de (2-formylphényl)-[2-chloro-4-(*E*-((1-morpholinyl)-carbonyl)-éthényl)-phényle] ;
N,N-diméthyl hydrazone ;
sulfure de (2-((3-(1-morpholinyl)-propyl)-1-amino)-phényl)-[2-chloro-4-(*E*-((1-morpholinyl)-carbonyl)-éthényl)-phényle] ;
sulfure de (2,4-dichlorophényl)-[2-bromo-4-(*E*-((3-(1-pyrrolidine-2-only)-propyl-amino)-carbonyl)-éthényl)-phényle] ;
sulfure de (2,4-dichlorophényl)-[2-formyl-4-(*E*-((1-morpholinyl)-carbonyl)-éthényl)-phényle] ;
sulfure de (2-chloro-6-formylphényl)-[2-chloro-4-(*E*-((4-acétyl-pipérazine-1-yl)-carbonyl)-éthényl)-phényle] ;
sulfure de (2-cyanophényl)-[2-chloro-4-(*E*-((4-acétyl-pipérazine-1-yl)-carbonyl)-éthényl)-phényle] ;
sulfure de (2-isopropylphényl)-[2-cyano-4-(*E*-((morpholine-1-yl)-carbonyl)-éthényl)-phényle] ;
sulfure de (2-bromophényl)-[2-nitro-4-(*E*-((4-acétyl-pipérazine-1-yl)-carbonyl)-éthényl)-phényle] ;
sulfure de (2-(pyrrolidine-1-yl)-phényl)-[2-chloro-4-(*E*-((morpholine-1-yl)-carbonyl)-éthényl)-phényle] ;
sulfure de (2-méthoxyphényl)-(2-chloro-4-(*E*-[(morpholine-1-yl)-carbonyl]éthényl)-phényle] ;
sulfure de (2-(isopropylphényl)-[2-nitro-4-(*E*-((3-carbométhoxypipérazine-1-yl)-carbonyl)-éthényl)-phényle] ;
sulfure de (2-méthylphényl)-[2-nitro-4-(*E*-((3-carboxamido-4-carbobenzoxypipérazine-1-yl)-carbonyl)-éthényl)-phényle] ;
sulfure de (2-isopropylphényl)-[2-nitro-4-(*E*-((2-carbométhoxy-4-*tert*-butoxy-carbonyl-pipérazino-1-yl)-carbonyl)-éthényl)-phényle] ;
sulfure de (2-isopropylphényl)-[2-nitro-4-(*E*-((2-carboxy-4-*tert*-butoxy-carbonyl-pipérazine-1-yl)-carbonyl)-éthényl)-phényle] ;
sulfure de (2-isopropylphényl)-[2-trifluorométhyl)-4-(*E*-((4-acétyl-pipérazine-1-yl)-carbonyl)-éthényl)-phényle] ;
sulfure de (2-isopropylphényl)-[2-trifluorométhyl-4-(*E*-((morpholine-1-yl)-carbonyl)-éthényl)-phényle] ;
sulfure de (2-isopropylphényl)-[2-trifluorométhyl-4-(*E*6553-pyrrolidine-2-on-1-yl)-prop-1-yl-amino)-carbonyl)-éthényl)-phényle] ;
sulfure de (2-isopropylphényl)-[2-trifluorométhyl-4-(*E*-((cyclobutyl-amino)-carbonyl)-éthényl)-phényle] ;
sulfure de (2-isopropylphényl)-[2-trifluorométhyl-4-(*E*-((cyclopentyl-amino)-carbonyl)-éthényl)-phényle] ;
sulfure de (2-isopropylphényl)-[2-trifluorométhyl-4-(*E*-((5-hydroxypent-1-yl-amino)-carbonyl)-éthényl)-phényle] ;
sulfure de (2-isopropylphényl)-[2-nitro-4-(*E*-((3-carbométhoxy-4-acétyl-pipérazine-1-yl)-carbonyl)-éthényl)-phényle] ;
sulfure de (2-biphényl)-[2-chloro-4-(*E*-((morpholine-1-yl)-carbonyl)-éthényl)-phényle] ;
sulfure de (3,4-diméthylphényl)-[2-nitro-4-(*E*-((4-acétyl-pipérazine-1-yl)-carbonyl)-éthényl)-phényle] ;
sulfure de (2-bromophényl)-[2-trifluorométhyl)-4-(*E*-((4-acétyl-pipérazine-1-yl)-carbonyl)-éthényl)-phényle] ;
sulfure de (5-indolyl)-[2-chloro-4-(*E*-((4-acétyl-pipérazine-1-yl)-carbonyl)-éthényl)-phényle] ;
sulfure de (5-benzodioxolyl)-[2-chloro-4-(*E*-((4-acétyl-pipérazine-1-yl)-carbonyl)-éthényl)-phényle] ;
sulfure de (2-isopropylphényl)-[2-nitro-4-(*e*-((2-carbométhoxypipérazine-1-yl)-carbonyl)-éthényl)-phényle] ;
sulfure de (2,3-diméthoxyphényl)-[2-chloro-4(*E*-[(morpholine-1-yl)-carbonyl]éthényl)-phényle] ;
sulfure de (2-fluorophényl)-[2-nitro-4-(*E*-((4-acétyl-pipérazine-1-yl)-carbonyl)-éthényl)-phényle] ;
sulfure de (2-bromophényl)-[2-trifluorométhyl-4-(*E*-((4-(*tert*-butoxycarbonyl)-pipérazine-1-yl)-carbonyl)-éthényl)-phényle] ;
sulfure de (2-pyrrolidine-1-yl)-phényl)-[2-trifluorométhyl-4-(*E*-((4-(*tert*-butoxycarbonyl)-pipérazine-1-yl)-carbonyl)-éthényl)-phényle] ;
sulfure de (3-carboxamidophényl)-[2-nitro-4-(*E*-((4-acétyl-pipérazine-1-yl)-carbonyl)-éthényl)-phényle] ;
sulfure de (3-(hydroxy-méthyl)-phényl)-[2-nitro-4-(*E*((4-acétyl-pipérazine-1-yl)-carbonyl)-éthényl)-phényle] ;
sulfure de phényl[2-trifluorométhyl-4-(*E*-((4-(*tert*-butoxycarbonyl)-pipérazine-1-yl)-carbonyl)-éthényl)-phényle] ;
sulfure de (2-isopropylphényl)-[2-trifluorométhyl-4-(*E*-((2-carbométhoxy-4-(*tert*-butoxycarbonyl)-pipérazine-1-yl)-carbonyl)-éthényl)-phényle] ;
sulfure de (2-isopropylphényl)-[2-nitro-4-(*E*-((3-(pyridine-4-méthyl-amino-carbonyl)-4-*tert*-butoxycarbonyl-pipérazine-1-yl)-carbonyl)-éthényl)-phényle] ;
sulfure de (2-éthoxyphényl)-[2-chloro-4(*E*-[(morpholine-1-yl)-carbonyl]-éthényl]phényle ;
sulfure de (2-méthoxyphényl)-[2-nitro-4-(*E*-((4-acétyl-pipérazine-1-yl)-carbonyl)-éthényl)-phényle] ;
sulfure de (2-(azétidine-1-yl)-phényl)-[2-trifluorométhyl-4-(*E*-((4-(*tert*-butoxycarbonyl)-pipérazine-1-yl)-carbnyl)-éthényl)-phényle] ;
sulfure de (2-(pipéridine-1-yl)-phényl)-[2-trifluorométhyl-4-(*E*-((4-(*tert*-butoxycarbonyl)-pipéridine-1-yl)-carbonyl)-éthényl)-phényle] ;
sulfure de (3-chloro-2-formylphényl)-[2-chloro-4-(*E*-((4-acétyl)-pipérazine-1-yl)-carbonyl)-éthényl)-phényle] ;
sulfure de (2-trifluorométhylphényl)-[2-trifluorométhyl-4-(*E*-((4-acétyl-pipérazine-1-yl)-carbonyl)-éthényl)-phényle] ;
sulfure de (3-bromophényl)-[2-trifluorométhyl-4-(*E*-((4-acétyl-pipérazine-1-yl)-carbonyl)-éthényl)-phényle] ;
sulfure de (3,5-diméthylphényl)-[2-trifluorométhyl-4-(*E*-((4-acétyl-pipérazine-1-yl)-carbonyl)-éthényl)-phényle] ;
sulfure de (2-isopropylphényl)-[2-nitro-4-(*E*-((3-diméthyl-amino-carbonyl)-4-(pyridine-4-carbonyl)-pipérazine-1-yl)-carbonyl)-éthényl)-phényle] ;
sulfure de (2-isopropylphényl)-[2-nitro-4-(*E*-((3-diméthyl-amino-carbonyl)-4-carbométhoxypipérazine-1-yl)-carbonyl)-éthényl)-phényle] ;
sulfure de (2-isopropylphényl)-[2-nitro-4-(*E*-((3-diméthyl-amino-carbonyl-4-acétyl-pipérazine-1-yl)-carbonyl)-éthényl)-phényle] ;
sulfure de (2-isopropylphényl)-[2-nitro-4-(*E*-((3-(1-morpholino-carbonyl)-4-*tert*-butoxycarbonyl-pipérazine-1-yl)-carbonyl)-éthényl)-phényle] ;
sulfure de (2-isopropylphényl)-[2-nitro-4-(*E*-((3-(pyridine-4-méthyl-amino-carbonyl)-pipérazine-1-yl)-carbonyl)-éthényl)-phényle] ;
sulfure de (2-isopropylphényl)-[2-nitro-4-(*E*-((3-diméthyl-amino-carbonyl)-pipérazine-1-yl)-carbonyl)-éthényl)-phényle] ;
sulfure de (2-isopropylphényl)-[2-nitro-4-(*E*-((3-(benzyl-amino-carbonyl)-4-*tert*-butoxycarbonyl-pipérazine-1-yl)-carbonyl)-éthényle)-phényle] ;
sulfure de (2-isopropylphényle)-[2-nitro-4-(*E*-((3-diméthyl-amino-carbonyl)-4-*tert*-butoxycarbonyl-pipérazine-1-yl)-carbonyl)-éthényl)-phényle] ;
sulfure de (2-bromophényl)-[2-chloro-4-(*E*-((3-(5S-hyroxy-méthyl-pyrrolidine-2-on-1-yl)-prop-1-yl-amino)-carbonyl)-éthényl)-phényle] ;
sulfure de (2-bromophényl)-[2-chloro-4-(*E*-((3-(pyrrolidin-2-on-1-yl)-prop-1-yl-amino)-carbonyl)-éthényl)-phényle] ;
sulfure de (2-bromophényl)-[2-chloro-4-(*E*-(N-méthyl-N-(3-pyrrolidine-2-on-1-yl)-prop-1-yl)-amino)-carbonyl)-éthényl)-phényle] ;
sulfure de (2-[2-méthoxy]éthoxyphényl)-[2-chloro-4(*E*-[(morpholine-1-yl)-carbonyl]-éthényl)-phényle] ;
sulfure de (2-isopropylphényl)-[2-nitro-4-(*E*-((3-morpholino-carbonyl)-pipérazine-1-yl)-carbonyl)-éthényl)-phényle] ;
sulfure de (2-isopropylphényl)-[2-nitro-4-(*E-*((4-*tert*-butoxy-carbonyl-pipérazine-1-yl)-carbonyl)-éthényl)-phényle] ;
sulfure de (2-isopropylphényl)-[2-nitro-4-(*E*-((4-méthoxycarbonyl-pipérazine-1-yl)-carbonyl)-éthényl)-phényle] ;
sulfure de (2-isopropylphényl)-[2-nitro-4-(*E*-(4-pyridine-4-carbonyl)-pipérazine-1-yl)-carbonyl)-éthényl)-phényle] ;
sulfure de (2-isopropylphényl)-[2-nitro-4-(*E*-((3-pyridine-3-méthyl-amino-carbonyl)-4-*tert*-butoxycarbonyl-pipérazine)-1-yl)-carbonyl)-éthényl)-phényle] ;
sulfure de (2-isopropylphényl)-[2-nitro-4-(*E*-((3-(pyridine-2-méthyl-amino-carbonyl)-pipérazine-1-yl)-carbonyl)-éthényl)-phényle] ;
sulfure de (2-isopropylphényl)-[2-nitro-4-(*E*-((3-(pyridine-3-méthyl-amino-carbonyl)-pipéridine-1-yl)-carbonyl)-éthényl)-phényle] ;
sulfure de (4-hydroxyphényl)-[2-nitro-4-(*E*-((4-acétyl-pipérazine-1-yl)-carbonyl)-éthényl)-phényle] ;
sulfure de (3,5-dichlorophényl)-[2-nitro-4-(*E*-((4-acétyl-pipérazine-1-yl)-carbo-nyl)-éthényl)-phényle] ;
sulfure de (2-bromophényl)-[2-chloro-4-(*E*-((3-(5S-acétoxy-méthyl-pyrrolidine-2-on-1-yl)-prop-1-yl-amino)-carbonyl)-éthényl)-phényle] ;
sulfure de (2-bromophényl)-[2-chloro-4-(*E*-((3-(5S-méthoxy-méthyl-pyrrolidine-2-on-1-yl)-prop-1-yl-amino)-carbonyl)-éthényl)-phényle] ;
sulfure de (2-bromophényl)-[2-chloro-4-(*E*-((3-(4*R*-hydroxy-méthyl-pyrrolidin-2-on-1-yl)-prop-1-yl-amino)-carbonyl)-éthényl)-phényle] ;
sulfure de phényl-[2-nitro-4-(*E*-((4-acétyl-pipérazine-1-yl)-carbonyl)-éthényl)-phényle] ;
sulfure de (2-diméthyl-aminophényl)-[2-nitro-4-(*E*-((4-acétyl-pipérazine-1-yl)-carbonyl)-éthényl)-phényle] ;
sulfure de (3-((2-hydroxyéthyl)-amino-carbonyl)-phényl)-[2-nitro-4-(*E*-((4-acétyl-pipérazine-1-yl)-carbonyl)-éthényl)-phényle] ;
sulfure de (3-((3-(1-imidazolyl)-propyl)-amino-carbonyl)-phényl)-[2-nitro-4-(*E*-((4-acétyl-pipérazine-1-yl)-carbonyl)-éthényl)-phényle] ;
sulfure de (3-((2-(1-morpholinyl)-éthyl)-amino-carbonyl)-phényl)-[2-nitro-4-(*E*-((4-acétyl-pipérazine-1-yl)-carbonyl)-éthényl)-phényle] ;
sulfure de (2-isopropylphényl)-[2-nitro-4-(*E*-((3-hydroxy-méthyl-4-*tert*-butoxycarbonyl-pipérazine-1-yl)-carbonyl)-éthényl)-phényle] ;
sulfure de (2-isopropylphényl)-[2-nitro-4-(*E*-((4-formyl-pipérazine-1-yl)-carbonyl)-éthényl)-phényle] ;
sulfure de (2-isopropylphényl)-[2-nitro-4-(*E*-((2-hydroxy-méthyl-4-*tert*-butoxy-carbonyl-pipérazine-1-yl)-carbonyl)-éthényl)-phényle] ;
sulfure de (2-éthoxyphényl)-[2-chloro-4(*E*-[(3-éthoxycarbonyl-pipéridine-1-yl)-carbonyl]éthényl)-)-phényle] ;
sulfure de (3-aminophényl)-[2-nitro-4-(*E*-((4-acétyl-pipérazine-1-yl)-carbonyl)-éthényl)-phényle] ;
sulfure de (4-aminophényl)-[2-nitro-4-(*E*-((4-acétyl-pipérazine-1-yl)-carbonyl)-éthényl)-phényle] ;
sulfure de (2,4-diméthylphényl)-[2-nitro-4-(*E*-((4-acétyl-pipérazine-1-yl)-carbonyl)-éthényl)-phényle] ;
sulfure de (2,5-diméthylphényl)-[2-nitro-4-(*E*-((4-acétyl-pipérazine-1-yl)-carbonyl)-éthenyl)-phényle] ;
sulfure de (4-méthoxyphényl)-[2-nitro-4-(*E*-((4-acétyl-pipérazine-1-yl)-carbonyl)-éthényl)-phényle] ;
sulfure de (3-chlorophényl)-[2-nitro-4-(*E*-((4-acétyl-pipérazine-1-yl)-carbonyl)-éthényl)-phényle] ;
sulfure de (2-chloro,4,5-diaminophényl)-[2-chloro-4-(*E*-((4-acétyl-pipérazine-1-yl)-carbonyl)-éthényl)-phényle] ;
sulfure de (3,4-diaminophényl)-[2-chloro-4-(*E*-((4-acétyl-pipérazine-1-yl)-carbonyl)-éthényl)-phényle] ;
sulfure de (6-chlorobenzimidazol-2-on-5-yl)-[2-chloro-4-(*E*-((4-acétyl-pipérazine-1-yl)-carbonyl)-éthényl)-phényle] ;
sulfure de (1-méthylindol-7-yl)-[2-chloro-4-(*E*-((4-acétyl-pipérazine-1-yl)-carbonyl)-éthényl)-phényle] ;
sulfure de (2-hydroxy, 4-aminophényl)-[2-chloro-4-(*E*-((4-acétyl-pipérazine-1-yl)-carbonyl)-éthényl)-phényle] ;
sulfure de (2-isopropylphényl)-[2-nitro-4-(*E*-((4-méthyl-pipérazine-1-yl)-carbonyl)-éthényl)-phényle] ;
sulfure de (2-isopropylphényl)-[2-nitro-4-(*E*-((4-pyridine-2-carbonyl)-pipérazine-1-yl)-carbonyl)-éthényl)-phényle] ;
sulfure de (2-isopropylphényl)-[2-nitro-4-(*E*-((4-(pyridine-3-carbonyl)-pipérazine-1-yl)-carbonyl)-éthényl)-phényle] ;
sulfure de (2-isopropylphényl)-[2-nitro-4-(*E*-((2-carbométhoxy-4-méthoxy-carbonyl-pipérazine-1-yl)-carbonyl)-éthényl)-phényle] ;
sulfure de (2-isopropylphényl)-[2-nitro-4-(*E*-((2-caxboxy-4-méthoxy-carbonyl-pipérazine-1-yl)-carbonyl)-éthényl)-phényle] ;
sulfure de (2-isopropylphényl)-[2-nitro-4-(*E*-((3-carbométhoxy-4-méthyl-pipérazine-1-yl)-carbonyl)-éthényl)-phényle] ;
sulfure de (2-éthoxyphényl)-[2-chloro-4-(*E*-[(3-carboxypipéridine-1-yl)-carbonyl]-éthényl)-phényle] ;
sulfure de (2-éthoxyphényl)-[2-chloro-4(*E*-[(3-carboxypipéridine-1-yl)-carbonyl]-éthényl)-phényle] ;
sulfure de (2-éthoxyphényl)-(2-chloro-4(*E*-[(2-éthoxycarbonyl-pipéridine-1-yl)-carbonyl]éthényl)-phényle] ;
sulfure de (2-éthoxyphényl)-[2-trifluorométhyl-4-(*E*-((1-(*tert*-butoxy-carbonyl)-4-hydroxypyrrolidine-3-yl-amino)-carbonyl)-éthényl)-phényle] ;
sulfure de (2-éthoxyphényl)-[2-chloro-4(*E*-[(2-carboxypipéridine-1-yl)-carbonyl]-éthényl)-phényle] ;
sulfure de (2-éthoxyphényl)-[2-trifluorométhyl-4-(*E*-((pyrrol-3-in-1-yl)-carbonyl)-éthényl)-phényle] ;
sulfure de (2-éthoxyphényl)-[2-trifluorométhyl-4-(*E*-((3-(pyrrolidin-2-on-1-yl)-prop-1-yl-amino)-carbonyl)-éthényl)-phényle] ;
sulfure de (2-éthoxyphényl)-[2-trifluorométhyl-4-(*E*-((4-acétyl-pipérazine-1-yl)-carbonyl)-éthenyl)-phényle] ;
sulfure de (2-éthoxyphényl)-[2-trifluorométhyl-4-(*E*-((4-(éthoxycarbonyl)-pipérazine-1-yl)-carbonyl)-éthényl)-phényle] ;
sulfure de (2-éthoxyphényl)-[2-trifluorométhyl-4-(*E*-((4-(2-furylcarbonyl)-pipérazine-1-yl)-carbonyl)-éthényl)-phényle] ;
sulfure de (2-éthoxyphényl)-[2-chloro-4-(*E*-[(3)-éthoxycarbonyl-pipéridine-1yl)-carbonyl)-éthényl)-phényle] ;
sulfure de (2-éthoxyphényl)-[2-chloro-4(*E*-[(4-carboxypipéridine-1-yl)-carbonyl]-éthényl)-phényle] ;
sulfure de (benzodioxan-6-yl)-[2-chloro-4-(*E*-[(4-acétyl-pipérazine-1-yl)-carbonyl)-éthényl)-phényle] ;
sulfure de (2-isopropylphényl)-[2-nitro-4-(*E*-((4-éthoxycarbonyl-pipérazine-1-yl)-carbonyl)-éthényl)-phényle] ;
sulfure de (2-isopropylphényl)-[2-nitro-4-(*E*-((4-isopropoxy-carbonyl-pipérazine-1-yl)-carbonyl)-éthényl)-phényle] ;
sulfure de (2-isopropylphényl)-[2-nitro-4-(*E*-((4-isobutoxycarbonyl-pipérazine-1-yl)-carbonyl)-éthényl)-phényle] ;
sulfure de (2-isopropylphényl)-[2-nitro-4-(*E*-((4-((1-propèn-2-oxy)-carbonyl)pipérazine-1-yl)-carbonyl)-éthényl)-phényle] ;
sulfure de (2-isopropylphényl)-[2-nitro-4-(*E*-((4-propionyl-pipérazine-1-yl)-carbonyl)-éthényl)-phényle] ;
sulfure de (2-isopropylphényl)-[2-nitro-4-(*E*-((4-carboxamidopipérazine-1-yl)-carbonyl)-éthényl)-phényle] ;
sulfure de (2-isopropylphényl)-[2-nitro-4-(*E*-((4-méthyl-amino-carbon-ylpipérazine-1-yl)-carbonyl)-éthényl)-phényle] ;
sulfure de (2-isopropylphényl)-[2-nitro-4-(*E*-((4-pyrimidine-2-yl)-pipérazine-1-yl)-carbonyl)-éthényl)-phényle] ;
sulfure de (2-isopropylphényl)-[2-nitro-4-(*E*-((4-hydroxyacétyl-pipérazine-1-yl)-carbonyl)-éthényl)-phényle] ;
sulfure de (2-isopropylphényl)-[2-nitro-4-(*E*-((4-pyrazine-2-carbonyl)-pipérazine-1-yl)-carbonyl)-éthényl)-phényle] ;
sulfure de (2-isopropylphényl)-[2-trifluorométhyl-4-(*E*-(((2-carboxypyrrol-3-in-1-yl)-carbonyl)-éthényl)-phényle] ;
sulfure de (2-isopropylphényl)-[2-nitro-4-(*E*-((3-hydroxy-méthyl-4-méthyl-pipérazine-1-yl)-carbonyl)-éthényl)-phényle] ;
sulfure de (2-isopropylphényl)-[2-trifluorométhyl-4-(*E*-(((2-carboxypyrrol-3-in-1-yl)-carbonyl)-éthényl)-phényle] ;
sulfure de (2-isopropylphényl)-[2-trifluorométhyl-4-(*E*-(((2-hydroxy-méthyl-pyrrolidine-1-yl)-carbonyl)-éthényl)-phényle] ;
sulfure de (2-isopropylphényl)-[2-nitro-4-(*E*-((3-méthyl-amino-carbonyl)-pipérazine-1-yl)-carbonyl)-éthényl)-phényle] ;
sulfure de (2-isopropylphényl)-[2-nitro-4-(*E*-(((3-cyclopropyl-amino-carbonyl)-pipérazine-1-yl)-carbonyl)-éthényl)-phényle] ;
sulfure de (2-isopropylphényl)-[2-nitro-4-(*E*-((3-carboxamidopipérazine-1-yl)-carbonyl)-éthényl)-phényle] ;
sulfure de (2-isopropylphényl)-[2-nitro-4-(*E*-((3-carbométhoxy-4-oxopipéridine-1-yl)-carbonyl)-éthényl)-phényle] ;
sulfure de (2-isopropylphényl)-[2-nitro-4-(*E*-((3,5-diméthyl-pipérazine-1-yl)-carbonyl)-éthényl)-phényle] ;
sulfure de (1-éthylindol-7-yl)-[2-chloro-4-(*E*-((4-acétyl-pipérazine-1-yl)-carbonyl)-éthényl)-phényle] ;
sulfure de (3-[2-méthoxy]éthoxyphényl)-[2-chloro-4(*E*-[(morpholine-1-yl)-carbonyl]-éthénylàphényle] ;
sulfure de (2-bromophényl)-[2-chloro-4-(*E*-((4,4'-S-dioxythiomorpholine-1-yl)-carbonyl)-éthényl)-phényle] ;
sulfure de (2-bromophényl)-[2-chloro-4-(*E*-(N-carbométhoxy-méthyl-N-(3-pyrrolidine-2-on-1-yl)-prop-1-yl)-amino)-carbonyl)-éthényl)-phényle] ;
sulfure de (2-bromophényl)-[2-chloro-4-(*E*-((4-S-oxythiomorpholine-1-yl)-2-pyrrolidinone)-carbonyl)-éthényl)-phényle] ;
sulfure de (2-méthoxy-5-chlorophényl)-[2-nitro-4-(*E*-((4-acétyl-pipérazine-1-yl)-carbonyl)-éthényl)-phényle] ;
sulfure de (2-isopropylphényl)-[2-nitro-4-(*E*-((3-acétoxy-méthyl)-pipérazine-1-yl)-carbonyl)-éthényl)-phényle] ;
sulfure de (2-isopropylphényl)-[2-nitro-4-(*E*-((3,5-diméthyl-4-acétyl-pipérazine-1-yl)-carbonyl)-éthényl)-phényle] ;
sulfure de (1-méthylindol-5-yl)-[2-chloro-4-(*E*-((4-acétyl-pipérazine-1-yl)-carbonyl)-éthényl)-phényle] ;
sulfure de (benzodioxane-6-yl)-[2-nitro-4-(*E*-((4-acétyl-pirpérazine-1-yl)-carbonyl)-éthényl)-phényle] ;
sulfure de (benzodioxane-6-yl)-[2-nitro-4-(*E*-((3-(pyrrolidin-2-on-1-yl)-prop-1-yl-amino)-carbonyl)-éthényl)-phényle] ;
sulfure de (benzodioxane-6-yl)-[2-nitro-4-(*E*-((3-carboéthoxypipéridine-1-yl)-carbonyl)-éthenyl)-phényle] ;
sulfure de (benzodioxane-6-yl)-[2-nitro-4-(*E*-((4-carboéthoxypipéridine-1-yl)-carbonyl)-éthényl)-phényle] ;
sulfure de (2-éthoxyphényl)-[2-trifluorométhyl-4-(Z-((4-acétyl-pipérazine-1-yl)-carbonyl)-éthényl)-phényle] ;
sulfure de (2-éthoxyphényl)-[2-trifluorométhyl-4-(*E*-((6-méthylpyrid-2-yl-amino)-carbonyl)-éthényl)-phényle] ;
sulfure de (2-méthyl-3-chlorophényl)-[2-nitro-4-(*E*-((4-acétyl-pipérazine-1-yl)-carbonyl)-éthényl)-phényle] ;
sulfure de (benzodioxane-6-yl)-[2-nitro-4-(*E*-((3-carboxamidopipéridine-1-yl)-carbonyl)-éthényl)-phényle] ;
sulfure de (benzodioxane-6-yl)-[2-nitro-4-(*E*-((2-carboéthoxypipéridine-1-yl)-carbonyl)-éthényl)-phényle] ;
sulfure de (benzodioxane-6-yl)-[2-nitro-4-(*E*-((4-carboxamidopipéridine-1-yl)-carbonyl)-éthényl)-phényle] ;
sulfure de (benzodioxane-6-yl)-[2-nitro-4-(*E*-((4-*tert*-butoxycarbonyl-pipérazine-1-yl)-carbonyl)-éthényl)-phényle] ;
sulfure de (2-isopropylphényl)-[2-nitro-4-(*E*-((*syn*-3,5-diméthylmorpholine-1-yl)-carbonyl)-éthényl)-phényle] ;
sulfure de (2-isopropylphényl)-[2-nitro-4-(*E*-((*anti*-3,5-diméthylmorpholine-1-yl)-carbonyl)-éthényl)-phényle] ;
sulfure de (2-isopropylphényl)-[2-nitro-4-(*E*-((3-carboéthoxypipérazine-1-yl)-carbonyl)-éthényl)-phényle] ;
sulfure de (2-isopropylphényl)-[2-nitro-4-(*E*-((3-isopropoxycarbonyl-pipérazine-1-yl)-carbonyl)-éthényl)-phényle] ;
sulfure de (2-isopropylphényl)-[2-nitro-4-(*E*-((3-(diméthyl-amino-carbonyl)-4-méthyl-pipérazine-1-yl)-carbonyl)-éthényl)-phényle] ;
sulfure de (2-isopropylphényl)-[2-nitro-4-(*E*-((3-carbométhoxy-4-hydroxy-pipéridine-1-yl)-carbonyl)-éthényl)-phényle] ;
sulfure de (2-isopropylphényl)-[2-nitro-4-(*E*-((3-hydroxy-méthyl-4-hydroxy-pipéridine-1-yl)-carbonyl)-éthényl)-phényle] ;
sulfure de (2-éthoxyphényl)-[2-trifluorométhyl-4-(*E*-((2-carbométhoxy-4-(méthoxycarbonyl)-pipérazine-1-yl)-carbonyl)-éthényl)-phényle] ;
sulfure de (2-éthoxyphényl)-[2-trifluorométhyl-4-(*E*-((2-carbométhoxy-4-méthyl pipérazine-1-yl)-carbonyl)-éthényl)-phényle] ;
sulfure de (2-éthoxyphényl)-[2-trifluorométhyl-4-(*E*-((2-carboxy-4-(méthoxy-carbonyl)-pipérazine-1-yl)-carbonyl)-éthényl)-phényle] ;
sulfure de (indol-6-yl)-[2-chloro-4-(*E*-((4-acétyl-pipérazine-1-yl)-carbonyl)-éthényl)-phényle] ;
sulfure de (1-éthy1,3-(diméthyl-aminométhl)-indol-7-yl)-[2-chloro-4-(*E*-((4-acétyl-pipérazine-1-yl)-carbonyl)-éthényl)-phényle] ;
sulfure de (5-éthoxybenzodioxane-6-yl)-[2-chloro-4-(*E*-((4-acétyl-pipérazine-1-yl)-carbonyl)-éthényl)-phényle] ;
sulfure de (2-éthyl)-4-bromophényl)-[2-nitro-4-(*E*-((4-acétyl-pipérazine-1-yl)-carbonyl)-éthényl)-phényle] ;
sulfure de (benzodioxane-6-yl)-[2-nitro-4-(*E*-((2-carboxypipéridine-1-yl)-carbonyl)-éthényl)-phényle] ;
sulfure de (benzodioxane-6-yl)-[2-nitro-4-(*E*-((4-carboxy-méthyl)-pipérazine-1-yl)-carbonyl)-éthényl)-phényle] ;
sulfure de (3-morpholinophényl)-[2-nitro-4-(*E*-((4-acétyl-pipérazine-1-yl)-carbonyl)-éthényl)-phényle] ;
sulfure de (5-éthoxybenzodioxane-8-yl)-[2-chloro-4-(*E*-((4-acétyl-pipérazine-1-yl)-carbonyl)-éthényl)-phényle] ;
sulfure de (5-chloro-8-éthoxyquinoléine-7-yl)-[2-chloro-4-(*E*-(4-acétyl-pipérazine-1-yl)-carbonyl)-éthényl)-phényle] ;
sulfure de (2-isopropylphényl)-[2-nitro-4-(*E*-((3-carboéthoxypipéridine-1-yl)-carbonyl)-éthényl)-phényle] ;
sulfure de (2-isopropylphényl)-[2-nitro-4-(*E*-((3-carboxypipéridine-1-yl)-carbonyl)-éthényl)-phényle] ;
sulfure de (2-isopropylphényl)-[2-nitro-4-(*E*-(((3-éthanesulfonyl-amino-carbonyl)-pipéridine-1-yl)-carbonyl)-éthényl)-phényle] ;
sulfure de (2-isopropylphényl)-[2-nitro-4-(*E*-(((3-(4-méthyl-pipérazine)-sulfonyl-amino-carbonyl)-pipéridine-1-yl)-carbonyl)-éthényl)-phényle ;]
sulfure de (2-isopropylphényl)-[2-nitro-4-(*E*-(((3-p-toluènesulfonyl-amino-carbonyl)-pipéridine-1-yl)-carbonyl)-éthényl)-phényle] ;
sulfure de (2-isopropylphényl)-[2-nitro-4-*E*-((3-méthyl-4-acétyl-pipérazine-1-yl)-carbonyl)-éthényl)-phényle] ;
sulfure de (2-hydroxyphényl)-[2-chloro-4-*E*-[(morpholine-1-yl)-carbonyl] éthényl)-phényle] ;
sulfure de (1-(carboxy-méthyl)-indol-5-yl)-[2-chloro-4-(*E*-((4-acétyl-pipérazine-1-yl)-carbonyl)-éthényl)-phényle] ;
sulfure de (benzodioxane-6-yl)-[2-trifluorométhyl-4-(*E*-((4-acétyl-pipérazine-1-yl)-carbonyl)-éthényl)-phényle ;
sulfure de (2-isopropylphényl)-[2-nitro-4-(*E*-((3-(1-pyrrolidin-2-onyl)-prop-1-yl-amino)-carbonyl)-éthényl)-phényle] ;
sulfure de (3-(2-morpholinoéthyl-amino)-phényl)-[2-trifluorométhyl-4-(*E*-((4-acétyl-pipéridine-1-yl)-carbonyl)-éthényl)-phényle] ;
sulfure de (2-pyrrolidine-1-ylphényl)-[2-nitro-4-(*E*-((4-acétyl-pipérazine-1-yl)-carbonyl)-éthényl)-phényle] ;
sulfure de (3-bromophényl)-[2-nitro-4-(*E*-((3-carboéthoxypyrrolidine-1-yl)-carbonyl)-éthényl)-phényle] ;
sulfure de (3-bromophényl)-[2-nitro-4-(*E*-((4-carboéthoxypyrrolidine-1-yl)-carbonyl)-éthényl)-phényle ;
sulfure de (2-hydroxy-méthyl)-benzodioxane-6-yl)-[2-chloro-4-(*E*-((4-acétyl-pipérazine-1-yl)-carbonyl)-éthényl)-phényle] ;
sulfure de (benzodioxane-6-yl)-[2-trifluorométhyl-4-(*E*-((3-(pyrrolidine-2-on-1-yl)-prop-1-yl-amino)-carbonyl)-éthényl)-phényle] ;
sulfure de (3-(diméthyl-aminométhyl)-indol-5-yl)-[2-chloro-4-(*E*-((4-acétyl-pipérazine-1-yl)-carbonyl)-éthényl)-phényle] ;
sulfure de (2-isopropylphényl)-[2-nitro-4-(*E*-((2-carboéthoxypipéridine-1-yl)-carbonyl)-éthényl)-phényle] ;
sulfure de (2-isopropylphényl)-[2-nitro-4-(*E*-((2-carboxypipéridine-1-yl)-carbonyl)-éthényl)-phényle] ;
sulfure de (2-isopropylphényl)-[2-nitro-4-(*E*-((4-carboéthoxypipéridine-1-yl)-carbonyl)-éthényl)-phényle] ;
sulfure de (2-isopropylphényl)-[2-nitro-4-(*E*-((4-carboxypipéridine-1-yl)-carbonyl)-éthényl)-phényle] ;
sulfure de (2-isopropylphényl)-[2-nitro-4-(*E*-(((4-p-toluènesulfonyl-amino-carbonyl)-pipéridine-1-yl)-carbonyl)-éthényl)-phényle] ;
sulfure de (2-isopropylphényl)-[2-nitro-4-(*E*-((3-carboxy-4-hydroxy-pipéridine-1-yl)-carbonyl)-éthényl)-phényle] ;
sulfure de (benzodioxane-6-yl)-[2-trifluorométhyl-4-(*E*-(3-carbo-éthoxy-pipécidine-1-yl)-carbonyl)-éthényl)-phényle] ;
sulfure de (benzodioxane-6-yl)-[2-trifluorométhyl-4-(*E*-((2-carboéthoxy-pipéridine-1-yl)-carbonyl)-éthényl)-phényle] ;
sulfure de (benzodioxane-6-yl)-[2-nitro-4-(*E*-((4-carboxypipéridine-1-yl)-carbonyl)-éthényl)-phényle] ;
sulfure de (benzodioxane-6-yl)-[2-trifluorométhyl-4-(*E*-((3-carboxy-pyrrolidine-1-yl)-carbonyl)-éthényl)-phényle] ;
sulfure de (benzodioxane-6-yl)-[2-trifluorométhyl-4-(*E*-((4-carboéthoxy-pipéridine-1-yl)-carbonyl)-éthényl)-phényle];
sulfure de (benzodioxane-6-yl)-[2-trifluorométhyl-4-(*E*-((2-carbométhoxy-4-*tert*-butoxycarbonyl-pipérazine-1-yl)-carbonyl)-éthényl)-phényle] ;
sulfure de (benzodioxane-6-yl)-[2-trifluorométhyl-4-(*E*-((2-carbométhoxy-4-méthoxycarbonyl-pipérazine-1-yl)-carbonyl)-éthényl)-phényle] ;
sulfure de (benzodioxane-6-yl)-[2-triflurométhyl-4-(*E*-((2-carbométhoxy-pipérazine-1-yl)-carbonyl)-éthényl)-phényle] ;
sulfure de (2-méthyl-3-(carboéthoxy-méthyl)-indol-5-yl)-(2-trifluorométhyl-4-(*E*-((morpholine-1-yl)-carbonyl)-éthényl)-phényle] ;
sulfure de (1-(2-méthoxyéthyl)-indol-5-yl)-[2-chloro-4-(*E*-((4-acétyl-pipérazine-1-yl)-carbonyl)-éthényl)-phényle] ;
sulfure de (2-isopropylphényl)-[2-nitro-4-(*E*-((3-acétoxy-méthyl-4-hydroxy-pipéridine-1-yl)-carbonyl)-éthényl)-phényle] ;
sulfure de (2-isopropylphényl)-[2-nitro-4-(*E*-((3-(diméthyl-amino-carbonyl)-4-hydroxypipéridine-1-yl)-carbonyl)-éthényle)-phényle] ;
sulfure de (2-isopropylphényl)-[2-nitro-4-(*E*-((3-cyanomorpholine-1-yl)-carbonyl)-éthényl)-phényle] ;
sulfure de (2-isopropylphényl)-[2-nitro-4-(*E*-(3-carboéthoxymorpholine-1-yl)-carbonyl)-éthényl)-phényle] ;
sulfure de (2-isopropylphényl)-[2-nitro-4-(*E*-((3-(tétrazol-5-yl)-morpholine-1-yl)-caibonyl)-cthényl)-phény!e] ;
sulfure de (benzodioxane-6-yl)-[2-trifluorométhyl-4-(*E*-((4-carboxypipéridine-1-yl)-carbonyl)-éthényl)-phényle] ;
sulfure de (benzodioxane-6-yl)-[2-trifluorométhyl)-4-(*E*-((2-carboxypipéridine-1-yl)-carbonyt)-éthényl)-phényle] ;
sulfure de (benzodioxane-6-yl)-[2-trifluorométhyl-4-(*E*-((4-carbométhoxy-pipérazine-1-yl)-carbonyl)-éthényl)-phényle] ;
sulfure de (benzodioxane-6-yl)-[2-trifluorométhyl-4-(*E*-((3-aza-6,9-dioxaspiro[5,4]décan-1-yl)-carbonyl)-éthényl)-phényle] ;
sulfure de (benzodioxane-6-yl)-[2-tcifluoro-4-(*E*-((4-benzimidazolon-1-yl)-pipéridine-1-yl)-carbonyl)-éthényl)-phényle] ;
sulfure de (benzodioxane-6-yl)-[2-trifluoromethyl-4-(*E*-((4-(méthyl-amino-carbonyl)-pipéridine-1-yl)-carbonyl)-éthényl)-phényle] ;
sulfure de (benzodioxane-6-yl)-[2-trifluorométhyl-4-(*E*-((3-carbométhoxy-4-méuioxycaibonyl-pipérazine-1 -yl)-carbonyl)-éthényl)-phényle] ;
sulfure de (2-isopropylphényl)-[2-nitro-4-(*E*-((3-carboxymozpholine-1-yl)-caibonyl)-ethényl)-phenyle] ;
sulfure de (benzodioxane-6-yl)-[2-tritluorométhyl-4-(*E*-((2-carboxy-4-méthoxy-carbonyl-pipérazine-1-yl)-carbonyl)-éthényl)-phényle] ;
sulfure de (benzodioxane-6-yl)-[2-trifluorométhyl-4-(*E*-morpholine-1-yl)-carbonyl)-éthényl)-phényle] ;
sulfure de (benzodioxane-6-yl)-[2-trifluorométhyl-4-(*E*-((4-pyrrolidine-1-yl)-pipéridine-1-yl)-carbonyl)-éthényl)-phényle] ;
sulfure de (2-isopropylphényl)-[2-nitro-4-(*E*-((3-aza-6,9-dioxaspiro[5,4]decan-1-yl)-carbonyl)-éthényl)-phényle] ;
sulfure de (2-isopropylphényl)-[2-nitro-4-(*E*-((2-(diméthyl-aminométhyl)-pipéridine-1-yl)-carbonyl)-éthényl)-phényle] ;
sulfure de (2-isopropylphényl)-[2-nitro-4-(*E*-((pipéridine-1-yl-amino)-carbonyl)-éthényl)-phényle] ;
sulfure de (benzodioxane-6-yl)-[2-trifluorométhyl-4-(*E*-((3-carboxy-4-méthoxycarbonyl-pipérazine-1-yl)-carbonyl)-éthényl)-phényle] ;
sulfure de (2-(diméthyl-amino-carbonyl)-benzodioxane-6-yl)-[2-chloro-4-(*E*-((4-acétyl-pipérazine-1-yl)-carbonyl)-éthényl)-phényle] ;
sulfure de (2-isopropylphényl)-[2-nitro-4-(*E*-((3-(2-méthoxy-méthyl)-tétrazol-5-yl)-pipéridine-1-yl)-carbonyl)-éthényl)-phényle] ;
sulfure de (2-isopropylphényl)-[2-nitro-4-(*E*-((3-(1-(méthoxy-méthyl)-tétrazol-5-yl)-pipéridine-1-yl)-carbonyl)-éthényl)-phényle] ;
sulfure de (1-méthylindol-5-yl)-[2-chloro-4-(*E*-((3-(1-pyrrolidine-2-onyl)-propyl-amino)-carbonyl)-éthényl)-phényle] ;
sulfure de (2-isopropylphényl)-[2-nitro-4-(*E*-((3-tétrazol-5-yl)-pipéridine-1-yl)-carbonyl)-éthényl)-phényle] ;
sulfure de (1-méthylindol-5-yl)-[2-chloro-4-(*E*-(3-carboéthoxypipéridine-1-yl)-carbonyl)-éthényl)-phényle] ;
sulfure de (1-méthylindol-5-yl)-[2-chloro-4-(*E*-((3-carboxypipéridine-1-yl)-carbonyl)-éthényl)-phényle] ;
sulfure de (1-méthylindol-5-yl)-[2-chloro-4-(*E*-((4-carboéthoxypipéridine-1-yl)-carbonyl)-éthényl)-phényle] ;
sulfure de (1-méthylindol-5-yl)-[2-chloro-4-(*E*-((3-carboxypipénidine-1-yl)-carbonyl)-éthényl)-phényle] ;
sulfure de (2-isopropylphényl)-[2-nitro-4-(*E*-((2-(1-méthylpyrrolidine-2-yl)-éthyl-amino)-carbonyl)-éthényl)-phényle] ;
sulfure de (2-isopropylphényl)-[2-nitro-4-(*E*-((4-pyrrolidine-1-yl)-pipéridine-1-yl)-carbonyl)-éthényl)-phényle] ;
sulfure de (2-isopropylphényl)-[2-nitro-4-(*E*-((4-sulfopipéridine-1-yl)-carbonyl)-éthényl)-phényle] ;
sulfure de (2-isopropylphényl)-[2-nitro-4-(*E*-((3-hydroxypipéridine-1-yl)-carbonyl)-éthényl)-phényle] ;
sulfure de benzodioxane-6-yl)-[2-trifluorométhyl-4-(*E*-((3((éthanesulfonyl-amino)-carbonyl)-pipéridine-1-yl)-carbonyl)-éthényl)-phényle] ;
sulfure de (benzodioxane-6-yl)-[2-trifluorométhyl-4-(*E*-((3-((*p*-toluène-sulfonyl-amino)-carbonyl)-pipéridine-1-yl)-caronyl)-éthényl)-phényle] ;
sulfure de (benzodioxane-6-yl)-[2-trifluorométhyl-4-(*E*-((4-((éthanesulfonyl-amino)-carbonyl) pipéridine-1-yl)-carbonyl)-éthényl)-phényle] ;
sulfure de (benzodioxane-6-yl)-[2-trifluorométhyl-4-(*E*-((2-(tétrazol-5-yl)-morpholine-1-yl)-carbonyl)-éthényl)-phényle] ;
sulfure de (2-isopropylphényl)-[2-nitro-4-(*E*-((2-butyl,5-(tétrazol-5-yl)-morpholine-1-yl)-carbonyl)-éthényl)-phényle] ;
sulfure de (2-(et 3-)-(hydroxy-méthyl)-benzodioxane-6-yl)-[2-nitro-4-(*E*-((4-acétyl-pipérazine-1-yl)-carbonyl)-éthényl)-phényle] ;
sulfure de (2-(et 3-)-(hydroxy-méthyl)-benzodioxane-6-yl)-[2-nitro-4-(*E*-((3-pyrrolidine-2-one-1-yl)-prop-1-yl-amino)-carbonyl)-éthényl)-phényle] ;
sulfure de (2-et 3-)-(hydroxy-méthyl)-benzodioxane-6-yl)-[2-trifluorométhyl)-4-(*E*-((3-(pyrrolidine-2-on-1-yl)-prop-1-yl-amino)-carbonyl)-éthényl)-phényle ;
sulfure de (3-hydroxy-méthyl)-benzodioxane-6-yl)-[2-nitro-4-(*E*-((3-pyrrolidine-2-on-1-yl)-prop-1-yl-amino)-carbonyl)-éthényl)-phényle] ;
sulfure de (benzodioxane-6-yl)-[2-chloro-4-(*E*-((3-carboxypipéridine-1-yl)-carbonyl)-éthényl)-phényle] ;
sulfure de (2-(et 3-)-(aminométhyl)-benzodioxane-6-yl)-[2-trifluorométhyl-4-(*E*-((3-(pyrrolidine-2-on-1-yl)-prop-1-yl-amino)-carbonyl)-éthényl)-phényle] ;
sulfure de (2-isopropylphényl)-[2-nitro-4-(*E*-((3-(méthyl-amino-carbonyl)-morpholine-1-yl)-carbonyl)-éthényl)-phényle] ;
sulfure de (2-isopropylphényl)-[2-nitro-4-(*E*-((3-(hydroxy-méthyl)-morpholine-1-yl)-carbonyl)-éthényl)-phényle] ;
sulfure de (2-isopropylphényl)-[2-nitro-4-(*E*((3-(acétoxy-méthyl)-morpholine-1-yl)-carbonyl)-éthényl)-phényle] ;
sulfure de (2-isopropylphényl)-[2-nitro-4-(*E*-((3-(aminométhyl)-morpholine-1-yl)-carbonyl)-éthényl)-phényle] ;
sulfure de (2-isopropylphényl)-[2-nitro-4-(*E*-((3-(acétamidométhyl)-morpholine-1-yl)-carbonyl)-éthényl)-phényle] ;
sulfure de (benzodioxane-6-yl)-[2-chloro-4-(*E*-((3-(pyrrolidine-2-on-1-yl)-prop-1-yl-amino)-carbonyl)-éthényl)-phényle] ;
sulfure de (benzodioxane-6-yl)-[2-chloro-4-(*E*-((3-carboéthoxypipéridine-1-yl)-carbonyl)-éthenyl)-phényle] ;
sulfure de (banzodioxane-6-yl)-[2-chloro-4-(*E*-((2-carboéthoxypipéridine-1-yl)-carbonyl)-éthényl)-phényle] ;
sulfure de (2-méthoxyphényl)-[2,3-dichloro-4(*E*-[(morpholine-1-yl)-carbonyl]-éthényl)-phényle] ;
sulfure de (2-méthoxyphényl)-[2,3-diméthyl-4-(*E*-[(morpholine-1-yl)-carbonyl]-éthanyl)-phényle] ;
sulfure de (2-isopropylphényl)-[2-nitro-4-(*E*-((indol-5-yl-amino)-carbonyl)-éthényl)-phényle] ;
sulfure de (benzodioxane-6-yl)-[2-chloro-4-(*E*-((3-carboxypipéridine-1-yl)-carbonyl)-éthényl)-phényle] ;
sulfure de (benzodioxane-6-yl)-[2-chloro-4-(*E*-((3-(tétrazol-5-yl)-pipéridine-1-yl)-carbonyl)-éthényl)-phényle] ;
sulfure de (benzodioxane-6-yl)-[2-chloro-4-(*E*-((4-(*tert*-butoxycarbonyl)-pipérazine-1-yl)-carbonyl)-éthényl)-phényle] ;
sulfure de (benzodioxane-6-yl)-[2-chloro-4-(*E*-((2-carboxypipéridine-1-yl)-carbonyl)-éthényl)-phényle] ;
sulfure de (benzodioxane-6-yl)-[2-chloro-4-(*E*-((3-(tétrazol-5-yl)-morpholine-1-yl)-carbonyl)-éthényl)-phényle] ;
sulfure de (benzodioxane-6-yl)-[2-chloro-4-(*E*-((4-(méthyl-amino-carbonyl)-pipérazine-1-yl)-carbonyl)-éthényl)-phényle] ;
sulfure de (2-méthoxyphényl)-(2,3-dichloro-4-(*E*-[(4-carboxypipéridine-1-yl)-carbonyl)-éthényl)-phényle] ;
sulfure de (benzodioxane-6-yl)-[2-chloro-(*E*-((4-(tétrazol-5-yl)-pipéridine-1-yl)-carbonyl)-éthényl)-phényle] ;
sulfure de (2-méthoxyphényl)-[3-chloro-4-*E*-[(morpholine-1-yl)-carbonyl]-éthényl)-phényle] ;
sulfure de (2-isopropylphényl)-[2-nitro-4-(*E*-((4-oxopipéridine-1-yl)-carbonyl)-éthényl)-phényle] ;
sulfure de (benzodioxane-6-yl)-[2-trifluorométhyl-4-(*E*-((3-R-carboéthoxy-pipéridine-1-yl)-carbonyl)-éthényl)-phényle] ;
sulfure de (benzodioxane-6-yl)-[2-trifluorométhyl-4-(*E*-((3-*R*-carboxy-pipéridine-1-yl)-carbonyl)-éthényl)-phényle] ;
sulfure de (benzodioxane-6-yl)-[2,3-dichloro-4-(*E*-((3-(pyrrolidine-2-on-1-yl)-prop-1-yl-amino)-carbonyl)-éthényl)-phényle] ;
sulfure de (benzodioxane-6-yl)-[2,3-dichloro-4-(*E*-((4-acétyl-pipérazine-1-yl)-carbonyl)-éthényl)-phényle] ;
sulfure de (benzodioxane-6-yl)-[2,3-dichloro-4-(*E*-((3-carboéthoxypipéridine-1-yl)-carbonyl)-éthényl)-phényle] ;
sulfure de (benzodioxane-6-yl)-[2,3-dichloro-4-(*E*-((4-carboéthoxypipéridine-1-yl)-carbonyl)-éthényl)-phényle] ;
sulfure de (benzodioxane-6-yl)-[2,3-dichloro-4-(*E*-((3-carboxypipéridine-1-yl)-carbonyl)-éthényl)-pliényle] ;
sulfure de (benzodioxane-6-yl)-[2,3-dichloro-4-(*E*-((4-carboxypipéridine-1-yl)-carbonyl)-éthényl)-phényle] ;
sulfure de (2-isopropylphényl)-[2,3-dichloro-4-(*E*-((3-(1-pyrrolidine-2-onyl)-propyl-amino)-carbonyl)-éthényl)-phényle] ;
sulfure de (2-isopropylphényl)-[2,3-dichloro-4-(*E*-((4-acétyl-pipérazine-1-yl)-carbonyl)-éthényl)-phényle] ;
sulfure de (2-isopropylphényl)-[2,3-dichloro-4-(*E*-((3-carboéthoxypipéridine-1-yl)-carbonyl)-éthényl)-phényle] ;
sulfure de (2-isopropylphényl)-[2,3-dichloro-4-(*E*-((4-carboéthoxypipéridine-1-yl)-carbonyl)-éthényl)-phényle] ;
sulfure de (2-isopropylphényl)-[2,3-dichloro-4-(*E*-((3-carboxypipéridine-1-yl)-carbonyl)-éthényl)-phényle] ;
sulfure de (2-isopropylphényl)-[2,3-dichloro-4-(*E*((4-carboxypipéridine-1-yl)-carbonyl)-étliényl)-phényle] ;
sulfure de (1-méthylindol-5-yl)-[2,3-dichloro-4-(*E*-((3-carboéthoxypipéridine-1-yl)-carbonyl)-éthényl)-phényle] ;
sulfure de (1-méthylindol-5-yl)-[2,3-dichloro-4-(*E*-((3-carboxypipéridine-1-yl)-carbonyl)-éthényl)-phényle] ;
sulfure de (1-méthylindol-5-yl)-[2,3-dichloro-4-(*E*-((4-carboéthoxypipéridine-1-yl)-carbonyl)-éthényl)-phényle] ;
sulfure de (1-méthylindol-5-yl)-[2,3-dichloro-4-(*E*-((4-carboxypipéridine-1-yl)-carbonyl)-éthényl)-phényle] ;
sulfure de (2-éthoxyphényl)-[2,3-dichloro-4-(*E*-[(4-carboxypipéridine-1-yl)-carbonyl]éthényl)-phényle] ;
sulfure de (2-éthoxyphényl)-[2,3-dichloro-4-*E*-[(morpholine-1-yl)-carbonyl]-éthényl)-phényle] ;
sulfure de (2-éthoxyphényl)-[2,3-dichloro-4-(*E*-[(3-carboxypipéridine-1-yl)-carbonyl] éthényl)-phényle] ;
sulfure de (2-isopropylphényl)-[2-nitro-4-(*E*-((3-carboéthoxypyrrolidine-1-yl)-carbonyl)-éthényl)-phényle] ;
sulfure de (2-isopropylphényl)-[2-nitro-4-(*E*-((3-carboxypyrrolidine-1-yl)-carbonyl)-éthényl)-phényle ;
sulfure de (2-isopropylphényl)-[2,3-difluoro-4-(*E*-((3-carboéthoxypipéridine-1-yl)-carbonyl)-éthényl)-phényle] ;
sulfure de (2-isopropylphényl)-[2,3-difluoro-4-(*E*-((3-carboxypipéridine-1-yl)-carbonyl)-éthényl)-phényle] ;
sulfure de (2-isopropylphényl)-[2,3-difluoro-4-(*E*-((4-carboxypipéridine-1-yl)-carbonyl)-éthényl)-phényle] ;
sulfure de (benzodioxane-6-yl)-[2-trifluorométhyl-4-(*E*-((3-éthoxycarbonyl-pyrrolidine-1-yl)-carbonyl)-éthényl)-phényle] ;
sulfure de (benzodioxane-6-yl)-[2-trifluorométhyl-4-(*E*-((3-carboxypyrrolidine-1-yl)-carbonyl)-éthényl)-phényle] ;
sulfure de (2-méthoxyphényl)-[2-chloro-3-trifluorométhyl-4-(*E*-((4-carboéthoxy-pipéridine-1-yl)-carbonyl)-éthényl)-phényle] ;
sulfure de (2-méthoxyphényl)-[2-chloro-3-trifluorométhyl-4-(*E*-((4-carboéthoxy-pipéridine-1-yl)-carbonyl)-éthényl)-phényle] ;
sulfure de (2-méthoxyphényl)-[2-chloro-3-trifluorométhyl-4-(*E*-((morpholine-1-yl)-carbonyl)-éthényl)-phényle] ;
sulfure de (benzodioxane-6-yl)-[4-(*E*-((4-carboxypipéridine-1-yl)-carbonyl)-éthényl)-naphtyle] ;
sulfure de (2-méthoxyphényl)-[2,3-dichloro-4-(*E*-((4-(spiro-hydrantoïne-5-yl)-pipérazine-1-yl)-carbonyl)-éthényl)-phényle] ;
sulfure de (2-méthoxyphényl)-[2,3-dichloro-4-(*E*-(4-(2-(2-hydroxyéthoxy)-éthyl)-pipérazine-1-yl)-carbonyl)-éthényl)-phényle ;
sulfure de (2-méthoxyphényl)-[2,3-dichloro-4-(*E*-((4-éthyl-pipérazine-1-yl)-carbonyl)-éthényl)-phényle] ;
sulfure de (2-isopropylphényl)-[2,3-dichloro-4-(*E*-((4-(2-(2-hydroxyéthoxy)-éthyl)-pipérazine-1-yl)-carbonyl)-éthéhyl)-phényle] ;
sulfure de (benzodioxane-6-yl)-[2,3-bis(trifluorométhyl)-4-(*E*-((4-carboxy-pipéridine-1-yl)-carbonyl)-éthényl)-phényle] ;
sulfure de (2-méthoxyphényl)-[2,3-dichloro-4-(*E*-((4-(carboxy-méthyl-amino)-carbonyl-pipéridine-1-yl)-carbonyl)-éthényl)-phényle] ;
sulfure de (2-méthoxyphényl)-[2,3-bis(trifluorométhyl)-4-(*E*-((4-carboxy-méthyl-pipérazine-1-yl)-carbonyl)-éthényl)-phényle] ;
sulfure de (2-méthoxyphényl)-[2,3-bis(trifluorométhyl)-4-(*E*-((4-N-(2-hydroxyéthyl)-pipérazine-1-yl)-carbonyl)-éthényl)-phényle] ;
sulfure de (1-méthylindol-5-yl)-[2,3-dichloro-4-(*E*-((4-carbo-2,3-dihydroxypropyl-amino)-pipéridine-1-yl)-carbonyl)-éthényl)-phényle] ;
sulfure de (2-méthoxyphényl)-[2,3-dichloro-4-(*E*-(4-(,3-dihydroxypropionyl)-pipérazine-1-yl)-carbonyl)-éthényl)-phényle] ;
sulfure de (2-méthoxyphényl)-[2,3-dichloro-4-(*E*-(4-(2,3-dihydroxy-3-carboxy-propionyl)-pipérazine-1-yl)-carbonyl)-éthényl)-phényle] ;
sulfure de (1-méthylindol-5-yl)-[2,3-dichloro-4-(*E*-((4-(carboxy-méthyl-amino)-carbonyl-pipéridine-1-yl)-carbonyl)-éthényl)-phényle] ;
sulfure de (1-méthylindol-5-yl)-[2,3-dichloro-4-(*E*-((4-sulfopipéridine-1-yl)-carbonyl)-éthényl)-phényle] ;
sulfure de (1-méthylindol-5-yl)-[2,3-dichloro-4-(*E*-(4-méthylhomopipérazine-1-ylcarbonyl)-éthényl)-phényle] ;
sulfure de (1-méthylindol-5-yl)-[2,3-dichloro-4-(*E*-(4-tétrahydrofuroyl-pipérazine-1-yl)-carbonyl)-éthényl)-phényle] ;
sulfure de (2-méthoxyphényl)-[2,3-dichloro-4-(*E*-((4-amino-4-carboxy-pipéridine-1-yl)-carbonyl)-éthényl)-phényle] ;
sulfure de (2-méthoxyphényl)-[2,3-dichloro-4-((4-furoyl-pipérazine-1-yl)-carbonyl)-éthényl)-phényle] ;
sulfure de (1-méthylindol-5-yl)-[2,3-dichloro-4-(*E*-(4-carbo-3-sulfopropyl-amino)-pipérazine-1-yl)-carbonyl)-éthényl)-phényle] ;
sulfure de (2-méthoxyphényl)-[2,3-dichloro-4-(*E*-(4-acétyl-amino-4-carboxypipéridine-1-ylcarbonyl)-éthényl)-phényle] ;
sulfure de (2-méthoxyphényl)-[2,3-bis(trifluorométhyl)-4-(*E*-((4-carboxy-pipéridine-1-yl)-carbonyl)-éthényl)-phényle] ;
sulfure de (2-méthoxyphényl)-5-[8-(*E*-((4-(amino-carbonyl)-pipéridine-1-yl)-carbonyl)-éthényl)-quinolinyle] ;
sulfure de (2-méthoxyphényl)-[2-trifluorométhyl-4-(*E*6554-carboxypipéridine-1-yl)-carbonyl)-éthényl)-phényle] ;
sulfure de (1-méthylindol-5-yl)-[2,3-dichloro-4-(*E*/*Z*-((1S,4S)-2,5-diaza-bicyclo-[2,2,1)-heptan-2-ylcarbonyl)-éthényl)-2,3-dichlorophényle] ;
sulfure de (1-méthylindol-5-yl)-[2,3-dichloro-4-(*E*-(4-hydroxy-3-carboxy-pipéradine-1-ylcarbonyl)-éthényl)-phényle] ;
sulfure de (1-méthylindol-5-yl)-[2,3-dichloro-4-(*E*-(S-oxothiomorpholine-1-ylcarbonyl)-éthényl)-phényle] ;
sulfure de (2-méthoxyphényl)-[2,3-dichloro-4-(*E*-((4-sulfophényl-amino)-carbonyl)-éthénylphényle] ;
sulfure de (2-méthoxyphényl)-[2,3-dichloro-4-(*E*-((4-carboxyphényl-amino)-carbonyl)-éthényl)-phényle] ; et
sulfure de [3-(4-morpholino)-phényl] [2,3-dichloro-4-(*E*-[(4-carboxypipéridine-1-yl)-carbonyl]éthényl)-phényle].

13. Un composé selon la revendication 1, choisi dans le groupe comprenant:
sulfure de (2-formylphényl)-[2-nitro-4-(*E*-((4-acétyl-pipérazine-1-yl)-carbonyl]-éthényl)-phényle] ;
sulfure de (2-éthoxyphényl)-[2-chloro-4(*E*-[(morpholine-1-yl)-carbonyl]-éthényl)-phényle] ;
sulfure de (2-isopropylphényl)-[2-nitro-4-(*E*-((3-diméthyl-amino-carbonyl-4-acétyl-pipérazine-1-yl)-carbonyl)-éthényl)-phényle] ;
sulfure de (2-isopropylphényl)-[2-nitro-4-(*E*-((4-méthoxycarbonyl-pipérazine-1-yl)-carbonyl)-éthényl)-phényle] ;
sulfure de (2-éthoxyphényl)-[2-trifluorométhyl-4-(*E*-((2-carboxy-4-(méthoxy-carbonyl)-pipérazine-1-yl)-carbonyl)-éthénylphényle] ;
sulfure de (2-éthyl-4-bromophényl)-[2-nitro-4-(*E*-((4-acétyl-pipérazine-1-yl)-carbonyl)-éthenyl)-phényle] ;
sulfure de (3-morpholinophényl)-[2-nitro-4-(*E*-((4-acétyl-pipérazine-1-yl)-carbonyl)-éthényl)-phényle] ;
sulfure de (2-isopropylphényl)-[2-nitro-4-(*E*-((3-carboéthoxypipéridine-1-yl)-carbonyl)-éthényl)-phényle] ;
sulfure de (2-isopropylphényl)-[2-nitro-4-(*E*-((3-(1-pyrrolidine-2-onyl)-prop-1-yl-amino)-carbonyl)-éthényl)-phényle] ;
sulfure de (2-hydroxy-méthyl)-benzodioxane-6-yl)-[2-chloro-4-*E*-((4-acétyl-pipérazine-1-yl)-carbonyl)-éthényl)-phényle] ;
sulfure de (benzodioxane-6-yl)-[2-trifluorométhyl-4-(*E*-((3-carboxypyrrolidine-1-yl)-carbonyl)-éthényl)-phényle] ;
sulfure de (benzodioxane-5-yl)-[2-trifluorométhyl-4-(*E*-((4-carboéthoxy-pipéridine-1-yl)-carbonyl)-éthényl)-phényle] ;
sulfure de (benzodioxane-6-yl)-[2-trifluorométhyl-4-(*E*-((2-carbométhoxy-4-méthoxycarbonyl-pipérazine-1-yl)-carbonyl)-éthénylphényle] ;
sulfure de (benzodioxane-6-yl)-[2-trifluorométhyl-4-(*E*-((2-carbométhoxy-pipérazine-1-yl)-carbonyl)-éthényl)-phényle] ;
sulfure de (benzodioxane-6-yl)-[2-chloro-4-(*E*-((4-(méthoxyamino-carbonyl)-pipérazine-1-yl)-carbonyl)-éthényl)-phényle] ;
sulfure de (2-méthoxyphényl)-[2,3-dichloro-4(*E*-[(4-carboxypipéridine-1-yl)-carbonyl]éthényl]phényle ;
sulfure de (benzodioxane-6-yl)-[2-trifluoromethyl-4-(*E*-((3-*R*-carboéthoxy-pipéridine-1-yl)-carbonyl)-éthényl)-phényle] ;
sulfure de (benzodioxane-6-yl)-[2,3-dichloro-4-(*E*-((3-(pyrrolidine-2-on-1-yl)-prop-1-yl-amino)-carbonyl)-éthényl)-phényle];
sulfure de (benzodioxane-6-yl)-[2,3-dichloro-4-(*E*-((4-acétyl-pipérazine-1-yl)-carbonyl)-éthényl)-phényle] ;
sulfure de (benzodioxane-6-yl)-[2,3-dichloro-4-(*E*-((3-carboéthoxypipéridine-1-yl)-carbonyl)-éthényl)-phényle] ;
sulfure de (benzodioxane-6-yl)-[2,3-dichloro-4-(*E*-((4-carboéthoxypipéridine-1-yl)-carbonyl)-éthényl)-phényle] ;
sulfure de (benzodioxane-6-yl)-[2,3-dichloro-4-(*E*-((3-carboxypipéridine-1-yl)-carbonyl)-éthényl)-phényle] ;
sulfure de (benzodioxane-6-yl)-[2,3-dichloro-4-(*E*-((4-carboxypipéridine-1-yl)-carbonyl)-éthényl)-phényle] ;
sulfure de (2-isopropylphényl)-[2,3-dichloro-4-(*E*-((4-acétyl-pipérazine-1-yl)-carbonyl)-éthényl)-phényle] ;
sulfure de (2-isopropylphényl)-[2,3-dichloro-4-(*E*-((3-carboéthoxypipéridine-1-yl)-carbonyl)-éthényl)-phényle] ;
sulfure de (2-isopropylphényl)-[2,3-dichloro-4-(*E*-((3-carboxypipéridine-1-yl)-carbonyl)-éthényl)-phényle] ;
sulfure de (2-isopropylphényl)-[2,3-dichloro-4-(*E*-((4-carboxypipéridine-1-yl)-carbonyl)-éthényl)-phényle] ;
sulfure de (2-méthoxyphényl)-[2,3-dichloro-4-(*E*-((4-(carboxy-méthyl-amino)-carbonyl-pipéridine-1-yl)-carbonyl)-éthényl)-phényle] ;
sulfure de (2-méthoxyphényl)-[2,3-bis(trifluorométhyl)-4-(*E*-((4-carboxy-méthyl-pipérazine-1-yl)-carbonyl)-éthényl)-phényle ; et
sulfure de (2-méthoxyphényl)-[2,3-bis(trifluorométhyl)-4-(*E*-((4-N-(2-hydroxy-éthyl)-pipérazine-1-yl)-carbonyl)-éthényl)-phényle].

14. Un composé selon la revendication 1, où Ar est choisi dans le groupe comprenant les méthoxyphényle et l'isopropylphényle.

15. Un composé selon la revendication 1, où Ar est le benzodioxane ou le benzodioxane substitué.

16. Un composé selon la revendication 1, où R₃ est un "trans-cinnamide" et Ar est choisi dans le groupe comprenant 1,3-benzimidazol-2-one, 1,4-benzodioxane, 1,3-benzodioxole, 1-benzopyr-2-èn-4-one, indole, isatine, phényle, 1,3-quinazoline-4-one, et quinoline.

17. Un composé selon la revendication 1, où R₁₀ et R₁₁ sont indépendamment choisis dans le groupe comprenant hydrogène, alkyle, cycloalkyle, alcoxycarbonylalkyle, hydroxyalkyle et hétérocyclylalkyle.

18. Un composé selon la revendication 1 où NR₁₀R₁₁ est un radical hétérocyclyle ou hétérocyclyle substitué.

19. Une composition pharmaceutique comprenant un composé selon la revendication 1 sur un support pharmaceutiquement acceptable.

20. Utilisation d'un composé selon la revendication 1 ou 12 pour la préparation d'un médicament pour inhiber les inflammations chez un mammifère.

21. L'utilisation d'un composé selon la revendication 1 ou 12 pour la préparation d'un médicament pour supprimer la réponse immune chez un mammifère.

22. Un procédé pour préparer un composé selon la revendication 1, lequel procédé consiste à activer le groupe acide de composé de formule 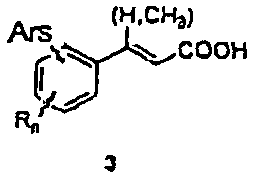 puis à le faire réagir ultérieurement avec une amine primaire ou secondaire de formule NR₁₀R₁₁, toutes les variables étant telles que définies dans la revendication 1.
